# EUROPÄISCHE PATENTANMELDUNG

(11) **EP 3 235 815 A1**
(43) Veröffentlichungstag der Anmeldung: **25.10.2017**
(21) Anmeldenummer: 16165936.2
(22) Anmeldetag: 19.04.2016
(51) Int. Cl.: C07D 403/12, C07D 405/14, A61K 31/255, A61K 31/33, C07D 215/08

(54) **WIRKSTOFFE GEGEN PARASITÄRE HELMINTHEN**

(71) Anmelder: PHILIPPS-UNIVERSITÄT MARBURG, 35037 Marburg (DE); Justus-Liebig-Universität Gießen, 35390 Gießen (DE)
(72) Erfinder: Schlitzer, Martin Prof. Dr., 35043 Marburg (DE); Mäder, Patrick, 35039 Marburg (DE); Grevelding, Christoph G. Prof. Dr., 35447 Reiskirchen (DE); Quack, Thomas Dr., Gießen (DE); Blohm, Ariane, 35398 Gießen (DE); Grünweller, Arnold PD Dr., 35094 Lahntal-Goßfelden (DE); Hartmann, Roland K. Prof. Dr., 35041 Marburg (DE); Lange-Grünweller, Kerstin Dr., 35094 Lahntal-Goßfelden (DE)
(74) Vertreter: Stumpf, Peter

(57) **Zusammenfassung**

Die vorliegende Erfindung betrifft neue chemische Wirkstoffe gegen Erkrankungen, die durch Helminthen hervorgerufen werden (z. B. Schistosomiasis). Sie umfasst im wesentlichen Verbindungen mit mindestens einer Dithiocarbamat-und/oder Dithiocarbazat-Teilstruktur.

## Beschreibung

Die vorliegende Erfindung betrifft neue chemische Wirkstoffe, die gegen parasitäre Helminthen wirksam sind und ein medizinisches Mittel zur Behandlung von Erkrankungen, die durch parasitäre Helminthen - zuvorderst Plattwürmer - hervorgerufen werden (darunter Bilharziose/Schistosomiasis).

### Beschreibung und Einleitung des allgemeinen Gebietes der Erfindung

Parasitäre Helminthen stellen weltweit ein großes medizinisches und wirtschaftliches Problem dar. Neben Arten die human- oder tierspezifisch sind, gibt es eine Reihe von parasitären Helminthen mit zoonotischem Potential. Abhängig von der Parasitenspezies und vom Wirt (der vom Parasiten befallene Organismus) können parasitäre Helminthen, die phylogenetisch (also im Hinblick auf die Evolution) nahe verwandt sind, in verschiedenen Organen eines Wirtes vorkommen.

So ergibt sich folgende Klassifizierung:
Blutegel: *Schistosoma mansoni, Schistosoma japonicum, Schistosoma haematobium, Schistosoma mekongi, Schistosoma intercalatum*
Leberegel: *Fasciola hepatica, Fasciola gigantica, Clonorchis sinensis, Clonorchis felineus, Dicrocoelium dendriticum, Dicrocoelium hospes, Echinococcus multilocularis, Echinococcus granulosus*
Hirnegel: *Echinococcus multilocularis, Echinococcus granulosus, Siprometra ssp.*
Pankreasegel: *Eurytrema pancreaticum, Eurytrema coelomaticum, Eurytrema ovis*
Lungenegel: *Paragonimus westermani, Paragonimus mexicana, Paragonimus skrjabini*
Darm- und Intestinalegel: *Fasciolopsis buski, Metagonimus yokogawai, Echinostoma ilocanum, Watsonius* watsoni, *Heterophyes heterophyes, Gastrodiscoides hominis, Taenia solium, Taenia saginata* und andere *Taenia-Arten, Moniezia spp., Diphyllobotrium*/*Spirometra ssp., Hymenolepis spp. Anoplocephala spp.* und *Mesocestoides spp.*

### Pansenegel: Paramphistomum cervi

Viele parasitäre Erkrankungen des Menschen werden von Egeln verursacht wie z.B. durch *Schistosoma-Spezies* (Blut- und Leberegel), *Echinokokken* (Leber-/Hirnegel), *Paragonimus westermani* (Lungenegel) oder *Clonorchis sinensis* (Leberegel). Weitere human- und tiermedizinisch sowie wirtschaftlich bedeutende Egel sind die Leberegel *Fasciola hepatica* und *Opisthorchis viverrini* sowie die Darm- und Intestinalegel *Fasciolopsis buski, Heterophyes heterophyes, Metagonimus yokogawai* und *Taenia*-Spezies.

Neben ihrer Bedeutung als Infektionserreger stehen viele der genannten Parasiten auch im Verdacht, Auslöser verschiedener Krebserkrankungen zu sein, wie z.B. Leber- bzw. Blasenkrebsformen, die durch *Opisthorchis viverrini,* oder *Schistosoma haematobium* ausgelöst werden können. Im ostasiatischen Raum ist *Clonorchis sinensis* der häufigste Leberegel und wurde in 2009 als "group 1 biological carcinogen" eingestuft. Vor allem in China, Russland, Korea und Vietnam tritt er mit zunehmender Tendenz als Infektionserreger auf. In China sind ca. 15 Mio. Menschen betroffen.

Helminthen der Gattung *Schistosoma* verursachen die Bilharziose (Schistosomiasis), die nach der Malaria zweithäufigste durch Parasiten verursachte Infektionskrankheit weltweit. Nach Einstufung durch die WHO ist die Schistosomiasis eine von gegenwärtig 17 als "vernachlässigt" und "Armuts-assoziiert" gelisteten Tropenkrankheiten. Außer Menschen sind auch Haus- und Wildtiere betroffen, was neben den epidemiologischen Konsequenzen zusätzlich wirtschaftliche Verluste zur Folge hat. Nach Schätzung der WHO leben weit über 600 Mio. Menschen in Endemiegebieten (Amerika, Afrika, Asien) wovon 240 Mio. infiziert sind. Die pathologischen Konsequenzen einer Erkrankung sind Entzündungen innerer Organe, vor allem Darm, Milz und Leber. Schwerwiegende Verläufe führen zur Leberzirrhose und zum Tod. Die Schistosomiasis, wie auch andere Wurmerkrankungen ist häufig mit HIV-Infektionen assoziiert. Durch Staudammbauten und andere Bewässerungssysteme nimmt das Infektionsrisiko weiter zu. Neuere Fallberichte zeigen, dass die Bilharziose auch wieder in Europa vorkommt, z.B. seit 2014 auf Korsika.

Zur Behandlung der Schistosomiasis wird derzeit Praziquantel (= 2-Cyclohexylcarbonyl-4-oxo-1,2,3,6,7,11b-hexahydro-4H-pyrazino[2,1-α]isochinolin) eingesetzt. Der Wirkstoff wurde bereits in den 1970er-Jahren von den Firmen Bayer und Merck entwickelt. Praziquantel wurde von der WHO auf die Liste der unentbehrlichen Arzneimittel gesetzt. Zu den verschiedenen Nachteilen, die dieses Medikament mit sich bringt, gehört sein regelmäßiger Einsatz in Massenbehandlungsprogrammen. Dies bringt die Gefahr einer möglichen Resistenzbildung mit sich, wofür es deutliche Hinweise aus Laboruntersuchungen aber auch aus Feldstudien in Afrika gibt. Da es bislang keine Vakzine gibt und Praziquantel zudem weltweit als Mittel der Wahl auf breiter Basis eingesetzt wird, besteht der dringende Bedarf an der Entwicklung neuer Antihelminthika.
Antihelminthika sind medizinische Mittel zur Behandlung von Erkrankungen die durch Helminthen hervorgerufen wurden (darunter die Schistosomiasis).

### Stand der Technik

Der Stand der Technik kennt verschiedene Substanzen, die als wirksam gegen verschiedene Helminthen beschrieben sind:
Die DE 69311318T2 beschreibt Benzimidazolverbindungen als Anthelmintika, die DE 2815621 Benzimidazol-Derivate, die DD298098 5-Chlor-2-nitro-benzoylchlorid. Die DE 3705227A1 nennt eine Wirkstoffkombination aus Phenylguanidinen oder Benzimidazolen und Tetrahydropyrimidinen. Die DE 2137593 offenbart S,S'-Ethylen-S,S'-bis (p-Chlorbenzyl)-diethoxyphosphinylimidodithiocarbonat. Die DE 866342 offenbart zur Bekämpfung von Helminthen Kalkstickstoff und Salpetersäure, wobei insbesondere die Eier vernichtet werden.

In DE 60126838T2 werden Amidoacetonitrile zur Bekämpfung von Endoparasiten, insbesondere Helminthen bei warmblütigem Nutzvieh und Haustieren vorgeschlagen.

### Nachteilig an diesen Verbindungen ist:

Benzimidazole sind vorrangig gegen Nematoden, aber nicht gegen Trematoden wirksam, wobei bereits seit 2011 die Möglichkeit der Resistenzbildung bekannt ist. Benzimidazole sind weiterhin aufgrund ihrer anti-mitotischen Wirkung potentiell teratogen und embryotoxisch; daher sollten sie bei Schwangerschaft nicht verwendet werden.

5-Chlor-2-nitro-benzoylchlorid wirkt nur gegen einige Nematoden (Rhabditida, Ascaridita, Spirurida, Trichocephalida), einige Cestoden (Cyclyphyllidae, Pseudophyllidae) und einige Trematoden (Digenea); es wirkt aber nicht gegen alle Helminthen, insbesondere nicht gegen Schistosoma.

Phenylguanidine stellen Prodrugs der Benzimidazole dar. Ihr Wirkspektrum ist daher analog dem der Benzimidazole. Febantel ist bei einmaliger Anwendung aufgrund der schnellen Darmpassage nicht oder nur unzureichend wirksam. Von einer Anwendung bei trächtigen und säugenden Hündinnen wird dringend abgeraten.

Pyrantel, ein bekannter Wirkstoff aus der Gruppe der Tetrahydropyrimidine, wirkt nur gegen einige Madenwürmer (*Oxyuriasis*), einige Spulwürmer *(Ascariasis),* einige Hakenwürmer (*Ancylostoma duodenale*), einige Fadenwürmer (*Trichostrongylus colubriformis*) und den Amerikanischen Hakenwurm (*Necator americanus*), aber nicht gegen alle Helminthen, insbesondere nicht gegen Schistosoma. Zudem ist das Molekül instabil; es zersetzt sich unter Einwirkung von Licht, Luft und Wärme. Tetrahydropyrimidine wie Pyrantel werden schlecht resorbiert und schnell metabolisiert, so dass die Wirkungsdauer eingeschränkt ist. Kopfschmerzen, Magen-/Darmprobleme und Mattigkeit können auftreten; Schwangeren und Kindern soll das Medikament nicht verabreicht werden. Aus der Anwendung bei Tieren sind starke Nebenwirkungen bekannt, wie u.a. Muskeltremor, Diarrhö, Tachypnoe.

S,S'-Ethylen-S,S'-bis(p-Chlorbenzyl)-diethoxyphosphinylimidodithiocarbonat ist als Insektizid und als Arachnizid beschrieben. Eine Anwendung als pharmazeutischer Wirkstoff an Mensch oder Tier ist nicht bekannt.

Kalkstickstoff (= Calciumcyanamid) wird üblicherweise als Düngemittel verwendet. Seine Wirkung gegen Helminthen ist beschrieben, aber nicht als Therapie von Mensch oder Tier, da bei Kontakt mit Cyanamid Schädigungen von Haut und Schleimhaut auftreten.

Ebenfalls zur Therapie von Mensch und Tier nicht geeignet sind Amidoacetonitril-Derivate, da sie toxisch sind und das Bindegewebe schädigen.

WO 2008037373 A2 nennt Neonikotinoide zur Bekämpfung von tierischen Schädlingen, insbesondere Insekten, Spinnentieren, Helminthen, Nematoden und Mollusken, die in der Landwirtschaft, im Gartenbau, in Forsten und in Gärten und Freizeiteinrichtungen vorkommen.

Weiterhin ist dem Fachmann die Verbindung Amoscanat als experimenteller Wirkstoff gegen Helminthen bekannt. Amoscanat ist ein Nithiocyamin:

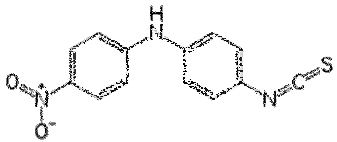

Weiterhin ist Thionocarbamat (Phenithionat) als Wirkstoff gegen Helminthen beschrieben: 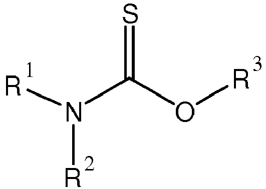 (R¹ bis R³ = aliphatische oder aromatische Reste oder Wasserstoff). Nachteilig an beiden Wirkstoffe ist eine ausgeprägte Hepatotoxizität, weshalb sie aus dem klinischen Gebrauch zurückgezogen wurden.

Carbamate werden seit den 1950er Jahren vor allem als Insektizide, Fungizide und Herbizide in der Landwirtschaft eingesetzt.
Aus der DE2629262A1 sind Dithiocarbamate und verwandte Verbindungen mit Wirksamkeit gegen Helminthen bekannt. Dort werden Mercaptoessigsäurederivate beansprucht.
Nachteilig an diesen Verbindungen ist, dass die Säurederivate jedoch nur eine sehr schwache Wirkung gegen Helminthen haben.
Es gibt weitere Wirkstoffe auf Basis von Dithiocarbamat oder Derivaten davon, die jedoch nicht für die Anwendung gegen Helminthen offenbart sind. Zum Beispiel sind die Dithiocarbamat-Verbindungen aus DE4325547C2 zur Behandlung von Hepatitis Viren beschrieben. Die DE10030781 beschreibt Dithiocarbamat-Verbindungen zur Behandlung von Bakterien, Protozoen und mehrzelligen Parasiten. Die DE69901862T2 offenbart Dithiocarbamat-Verbindungen zur Bekämpfung phytopathogener Pilze, zur Verwendung als Insektizid, Fungizid und Herbizid. Weitere Dithiocarbamat-Verbindungen sind aus DE69409611T2 als Insektizid, Fungizid, Akarizid und Nematizid bekannt.

Neben Dithiocarbamat-Verbindungen gibt es auch Wirkstoffe auf Basis von Dithiocarbazat oder Derivate davon, diese sind z.B. als Schädlingsbekämpfungsmittel gegen Schädlinge, insbesondere pflanzenparasitäre Nematoden in DE 3820628 beschrieben.

### Aufgabe

Aufgabe der vorliegenden Erfindung ist es mindestens eine chemische Verbindung sowie ein medizinisches Mittel mit Wirksamkeit gegen parasitäre Helminthen bereitzustellen, die vom Patienten gut vertragen werden, ausreichend stabil sind sowie einfach und kostengünstig hergestellt werden können.

### Lösung der Aufgabe

Diese Aufgabe wird erfindungsgemäß gelöst durch neue Dithiocarbamat-Verbindungen und Dithiocarbazat-Verbindungen und deren Anwendung als medizinisches Mittel gegen parasitäre Helminthen gemäß der Ansprüche.
Zu Parasiten der Klasse der Helminthen gehören u.a. *Ancylostoma duodenale, Ancylostoma ceylanicum, Anoplocephala spp., Acylostoma braziliensis, Ancylostoma spp., Ascaris lubricoides, Ascaris spp., Brugia malayi, Brugia timori, Bunostomum spp., Chabertia spp., Clonorchis spp., Clonorchis sinensis, Clonorchis felineus , Cooperia spp., Dicrocoelium spp, Dicrocoelium dendriticum, Dicrocoelium hospes, Dictyocaulus filaria, Diphyllobothrium latum, Dracunculus medinensis, Echinococcus granulosus, Echinococcus multilocularis, Echinostoma ilocanum, Enterobius vermicularis, Eurytrema pancreaticum, Eurytrema coelomaticum, Eurytrema ovis, Faciola spp., Fasciola hepatica, Fasciola gigantica, Fasciolopsis buski, Gastrodiscoides hominis, Haemonchus spp., Heterakis spp., Heterophyes heterophyes, Hymenolepis nana, Hyostrongulus spp., Loa Loa, Mesocestoides spp., Metagonimus yokogawai, Moniezia spp., Nematodirus spp., Oesophagostomum spp., Opisthorchis spp., Onchocerca volvulus, Opisthorchis viverrini, Ostertagia spp., Paragonimus spp., Paragonimus westermani, Paragonimus mexicana, Paragonimus skrjabini, Paramphistomum cervi, Schistosomen spp, Schistosoma mansoni, Schistosoma japonicum, Schistosoma haematobium, Schistosoma mekongi, Schistosoma intercalatum, Siprometra ssp., Strongyloides fuelleborni, Strongyloides stercoralis, Stronyloides spp., Taenia saginata, Taenia solium, Trichinella spiralis, Trichinella nativa, Trichinella britovi, Trichinella nelsoni, Trichinella pseudopsiralis, Trichostrongulus spp., Trichuris trichuria, Watsonius watsoni, Wuchereria bancrofti.*

Antihelminthika sind medizinische Mittel zur Verabreichung an einem Patienten zur Behandlung von Erkrankungen, die durch parasitäre Helminthen hervorgerufen wurden.

Der Begriff Patient umfasst dabei neben dem Menschen auch Tiere, die als End- bzw. Zwischenwirte für Parasiten dienen. Tiere meint dabei insbesondere Wirbeltiere und umfasst Nutztiere sowie Haustiere, wie z.B. Hühnervögel, Pferd, Schaf, Schwein, Rind, Ziege, Esel Hund, Kaninchen, Katze, Maus, Meerschweinchen u.v.m., die alle von parasitären Helminthen befallen werden können.
Überraschenderweise wurden neue Dithiocarbamat-Verbindungen und Dithiocarbazat-Verbindungen gemäß der Formeln I bis VIII gefunden, die eine besonders gute Wirksamkeit gegen parasitäre Helminthen aufweisen.
Insbesondere wurde in langjähriger Forschungsarbeit die Biologie der Schistosomen und die für den Menschen krankheitsauslösenden Mechanismen erforscht. Dabei wurde gefunden, dass die Schistosomen (Pärchenegel) eine typische Morphologie und physiologische Besonderheiten aufweisen. Sie kommen getrenntgeschlechtlich vor, wobei das längere Weibchen nach der Kopulation in der Bauchfalte des Männchens lebt. Vorder- und Hinterende ragen aus der Bauchfalte hervor. Eine nahezu einzigartige biologische Besonderheit der Schistosomen ist, dass die Paarung notwendige Voraussetzung für die Entwicklung der Reproduktionsorgane im Weibchen ist. Dies wiederrum ist Voraussetzung für die Eibildung und Eiablage der Schistosomen. Die Eier sind für die Aufrechterhaltung des Lebenszyklus der Schistosomen essentiell, und sie verursachen die pathologischen Konsequenzen einer Schistosomiasis. Die Eier lösen entzündliche Prozesse in verschiedenen Organen infizierter Patienten aus was u.a. zur Leberzirrhose führen kann, der wesentlichen Todesursache einer Schistosomiasis. Daher sind Wirkstoffe, die Eiproduktion der Schistosomen vermindern, bereits sehr interessant als Antihelmintika. Denn sie wirken sowohl anti-inflammatorisch (Entzündungs-hemmend), wenn auch indirekt durch Reduktion der Eiproduktion, als auch (Lebens-)Zyklusunterbrechend und vermindern so die klinisch/pathologischen Konsequenzen einer Schistosomiasis und gleichzeitig die Verbreitung der Schistosomen.

### Verwendung als medizinisches Mittel zur Behandlung von Erkrankungen, die durch Helminthen hervorgerufen wurden

Mindestens eine der erfindungsgemäßen Verbindungen der Formeln I bis VIII mit oder ohne Modifikationen wird als Bestandteil eines medizinischen Mittels verwendet zur Behandlung von Erkrankungen, die durch parasitäre Helminthen verursacht bzw. hervorgerufen werden.
Das erfindungsgemäße Mittel umfasst alternativ mindestens einen pharmakologisch verträglichen Träger, Hilfsstoff und/oder Lösungsmittel.
Das erfindungsgemäße Mittel wird in Form von Tropfen, Mundspray, Nasenspray, Tabletten, Filmtabletten, Schichttabletten, Zäpfchen, Gelen, Salben, Sirup, Inhalationspulvern, Granulaten, Suppositorien, Emulsionen, Dispersionen, Mikrokapseln, Kapseln, Puder oder Injektions- oder Infusionslösungen hergestellt und verabreicht. Dies umfasst auch Formulierungen wie Schichttabletten zur kontrollierten und/oder kontinuierlichen Freisetzung sowie Mikroverkapselungen als spezielle Darreichungsform.
Das erfindungsgemäße Mittel umfasst Verkapselungen in Vesikel wie sie in der Dermatologie und Pharmazie zum Transport in die Haut bekannt sind z.B.: anionische oder kationische Liposomen, Niosome, Nanopartikel oder multilamellare Vesikel zur Penetration durch die Haut oder in die Zellen der Haut oder Haare. Das erfindungsgemäße Mittel ist unter anderem für die Inhalation oder die intravenöse, intraperitoneale, intramuskuläre, subkutane, mucokutane, orale, rektale, transdermale, topikale, bukkale, intradermale, intragastrale, intrakutane, intranasale, intrabuccale, intrathekale, perkutane oder sublinguale Verabreichung geeignet.
Als pharmakologisch verträgliche Träger werden beispielsweise Lactose, Stärke, Sorbitol, Sucrose, Cellulose, Magnesiumstearat, Dicalciumphosphat, Calciumsulfat, Talk, Mannitol, Ethylalcohol und dergleichen eingesetzt. Puder als auch Tabletten können zu 5 bis 95% aus einem derartigen Träger bestehen.
Ferner kann dem erfindungsgemäßen Mittel noch Sprengmittel, Farbstoffe, Geschmacksstoffe und/oder Bindemittel zugesetzt werden.
Flüssige Formulierungen umfassen Lösungen, Suspensionen, Sprays und Emulsionen, beispielsweise Injektionslösungen auf Wasserbasis oder auf Wasser-Propylenglycol-Basis für parenterale Injektionen.
Kapseln werden beispielsweise aus Methylcellulose, Polyvinylalkohole oder denaturierter Gelatine oder Stärke hergestellt.
Als Sprengmittel werden Stärke, Natrium-Carboxymethylstärke, natürliche und synthetische Gummis wie beispielsweise Johannisbrotkernmehl, Karaya, Guar, Tragacanth und Agar sowie Cellulosederivate wie Methylcellulose, NatriumCarboxymethylcellulose, microkristalline Cellulose sowie Alginate, Tonerden und Bentonite verwendet. Diese Bestandteile werden in Mengen von 2 bis 30 Gew.-% eingesetzt werden.
Als Bindemittel sind dem Fachmann Zucker, Stärke aus Korn, Reis oder Kartoffeln, natürliche Gummis wie Akaziengummi, Gelatine, Traganth, Alginsäure, Natriumalginat, Ammonium-Calcium-Alginat, Methylcellulose, Wachse, NatriumCarboxymethylcellulose, Hydroxypropyl-methylcellulose, Polyethylenglycol, Polyvinylpyrrolidon sowie anorganische Verbindungen wie Magnesium-Aluminum-Silicate bekannt, diese können dem erfindungsgemäßen Mittel zugesetzt werden. Die Bindemittel werden üblicherweise in Mengen von 1 bis 30 Gew.-% zugesetzt. Als Gleitmittel sind Borsäure und Stearate wie Magnesiumstearat, Calciumstearat, Kaliumstearat, Stearinsäure, hochschmelzende Wachse als auch wasserlösliche Gleitmittel wie Natriumchlorid, Natriumbenzoat, Natriumacetat, Natriumoleat, Polyethylenglycol und Aminosäuren wie Leucin bekannt und werden eingesetzt.
Die Art der Dosierung des erfindungsgemäßen Mittels wird vom behandelnden Arzt entsprechend den klinischen Faktoren bestimmt. Es ist dem Fachmann bekannt, dass die Art der Dosierung von verschiedenen Faktoren wie z.B. Körpergröße, Gewicht, Körperoberfläche, Alter, Geschlecht oder der allgemeinen Gesundheit des Patienten abhängig ist, aber auch von dem speziell zu verabreichenden Mittel, der Dauer und Art der Verabreichung und von anderen Medikamenten, die möglicherweise parallel verabreicht werden.

Die Erfindung betrifft ein medizinisches Mittel, das als Wirkstoff mindestens eine der anthelmintisch wirksamen Verbindungen gemäß der allgemeinen Formeln I bis VIII mit oder ohne Modifikation in feinteiliger Dispersion in einem penetrationsfördernden Mittel enthält.
Es können auch Gemische der genannten Wirkstoffe eingesetzt werden.
Als penetrationsfördernde Mittel sind Öle bekannt. Es können Triglyceride gesättigter Pflanzenfettsäuren, z.B. Miglyol 812®, Triglycerindiisostearat oder Ester langkettiger Säuren, wie z.B. Oleyl-Oleat, Isopropylmyristat, Isopropylpalmitat, 2-Ethylhexylpalmitat, Isooctylstearat, Isopropylstearat, Laurinsäurehexylester oder Di-n-butyladipat eingesetzt werden. Ferner werden langkettige Alkohole, wie z.B. Hexylalkohol, Octylalkohol, Decylalkohol, Oleylalkohol, 2-Octyldodecanol, 2-Hexyldecanol oder 2-Octyldecanol eingesetzt. Diese penetrationsfördernden Mittel können allein oder untereinander gemischt eingesetzt werden. Eine besonders gute penetrationsfördernde Wirkung haben langkettige Alkohole insbesondere die 2-alkylsubstituierten Alkohole. Die Öle werden in Gewichtsanteilen von 5 - 95 % eingesetzt, bevorzugt von 30 bis 90 Gew.-%. Die feinteiligen Wirkstoffe können auch in Wasser, in welchem penetrationsfördernde Öle emulgiert sind, dispergiert sein, wobei der Wassergehalt dieser Dispersionen bis zu 50 Gew.-%, vorzugsweise bis zu 30 Gew.-% betragen kann.
Das medizinische Mittel der vorliegenden Erfindungen wird als pour-on Formulierung hergestellt. Diese Formulierung ist dem Fachmann bekannt und verschiedene Verfahren sind dazu im Stand der Technik bekannt.
Pour-on Formulierungen sind besonders zur Anwendung bei Tieren geeignet und werden sowohl bei Nutztieren, als auch Haustieren eingesetzt.

Die mindestens eine Verbindung der Formeln I bis VIII kann mit mindestens einem weiteren Wirkstoff z.B. Analgetika und Antipyretika kombiniert werden. Weiterhin ist die Kombination mit mindestens einem antiinflammatorischen Mittel, Immunmodulator, Antiasthmatikum und/oder Bronchodilatator möglich. Ebenso ist die Kombination mit mindestens einem antibakteriellen, antimykotischen, anthelmintischen und/oder antiviralen Wirkstoff möglich. Alternativ ist die Kombination mit mindestens einem dermatologischen oder kosmetischen Wirkstoff möglich.

Das Mittel wird je nach Herstellung und Applikationsform zusammen mit flüssigen oder festen Hilfsmitteln eingesetzt, die konventionell in der Formulierungstechnik verwendet werden, um verdünnbare Lösungen, Emulsionen, lösliche Pulver, Granulate oder Mikroeinkapselungen in polymere Substanzen oder Nanopartikel zu erhalten. Es wird je nach Verabreichungsform nach einem dem Fachmann bekannten Verfahren z.B. durch gründliches Mischen und/oder Mahlen der Wirkstoffe, durch Zugabe von Lösungsmitteln, festen Trägern hergestellt.

Bevorzugte Verabreichungsformen für medizinische Mittel zur Bekämpfung von parasitäre Helminthen umfassen z.B. Lösungen, Emulsionen, Suspensionen, Nahrungsmittelzusätze, Pulver, Tabletten, Brausetabletten, Kapseln, Mikrokapseln. Allen erfindungsgemäßen neuen Dithiocarbamat-Verbindungen und Dithiocarbazat-Verbindungen der Formeln I bis VIII ist gemein, dass sie entweder einzeln oder in Kombinationen beispielhaft bei Schistosomen,
a) die Eiproduktion unterbinden und/oder einen Verlust der Vitalität und/oder Absterben hervorrufen und
b) an mindestens ein Zielmolekül binden, das wegen der engen evolutionären Nähe zu den anderen genannten Parasiten in identischer oder ähnlicher Ausprägung auch in diesen vorkommen kann.
Daher ist es für den Fachmann naheliegend, und es kann davon ausgegangen werden, dass auch Kombinationen dieser Verbindungen wirken und ggf. sogar additive und somit noch potentere Effekte erzielen können, nicht nur bei Schistosomen sondern bei Helminthen allgemein.
Aufgrund der beobachteten phänotypischen Auswirkungen ist anzunehmen, dass zu den Zielmolekülen auch das (Detoxifizierungs-)Enzym ALDH gehört. Versuche mit einem bekannten ALDH-Inhibitor, Disulfiram, haben vergleichbare, teils identische Veränderungen bei behandelten Schistosomen hervorgerufen. Sequenzvergleiche mit Einträgen aus Datenbanken (BLAST- und Alignment-Analysen) haben gezeigt, dass es sehr ähnlich strukturierte ALDHs auch bei anderen parasitären Helminthen gibt wie z.B. *Clonorchis sinensis* (86% Identität auf DNA-Ebene), *Fasciola hepatica* (88% Identität auf DNA-Ebene) und *Echinococcus multilocularis* (bis zu 100% Identität auf DNA-Ebene). Auch in weiteren Helminthen kommen Aldehyddehydrogenasen (ALDHs) vor, die den genannten ebenso ähnlich sind.
Im Fall von *Schistosoma* wird dieses Enzym präferentiell in paarungserfahrenen Männchen exprimiert und hier auch im Tegument. Ein Grund dafür könnte sein, dass das Tegument physiologisch hoch aktiv ist und dem Schutz gegen das Immunsystem des Wirtes dient, aber auch der Aufnahme verschiedener Nährstoffe, wie z.B. Glucose. Das Enzym ALDH katalysiert die Oxidation von Aldehyden zu den entsprechenden Carbonsäuren unter gleichzeitiger Reduktion von NAD zu NADH als Co-Substrat. Damit ist die ALDH gewissermaßen ein Enzym mit "Entgiftungsfunktion". Die Inhibition von ALDH führt zu einer Akkumulation von Aldehyden, die toxische Wirkung entfalten können, was vor allem für die Würmer in kurzer Zeit letale Folgen hat. Auch Wirbeltiere wie der Mensch verfügen über ALDHs, die je nach Krankheitsbild Ziele von Behandlungsstrategien sind (z.B. Alkoholismus/Disulfiram). Wenn ALDH das Ziel eines (oder mehrerer) der dargestellten Substanzen ist, sind Nebenwirkungen wie sie für eine Disulfirambehandlung beschrieben sind für den Fall nicht auszuschließen, dass die eingesetzte Substanz mit ähnlicher Spezifität an die ALDH des Wirbeltierwirts bindet. Die bisher erfolgten Toxizitätstests zeigen jedoch überraschenderweise, dass die dargestellten Substanzen in diesen Systemen keine negativen Wirkungen entfalteten.
Wegen der großen Ähnlichkeit der ALDHs bei Helminthen, die man auch bei anderen potentiellen Zielmolekülen annehmen kann, ist davon auszugehen, dass die erfindungsgemäßen neuen Dithiocarbamat-Verbindungen und Dithiocarbazat-Verbindungen der Formeln I bis VIII ALDH und weitere Zielmoleküle, wie z.B. Metalloproteine, entweder direkt binden oder sonst negativ beeinflussen, so dass es zu toxischen oder sonstigen negativen Auswirkungen (z.B. Unterbrechung der Eiproduktion) bei Helminthen kommt.
Versuche an Schistosomen zeigen, dass die erfindungsgemäßen Wirkstoffe schon in sehr geringer Konzentration (10 - 1 µM) und in sehr kurzer Zeit (24-72h) zur Entpaarung und zur Reduktion der Eibildung adulter Schistosomen sowie zu deren Tod führen.
Die wissenschaftliche Analyse der beobachteten Wirkung auf Helminthen legt die Vermutung nahe, dass es neben dem Target ALDH noch weitere Targets in Helminthen geben könnte, auf die die erfindungsgemäßen neuen Dithiocarbamat-Verbindungen und Dithiocarbazat-Verbindungen der Formeln I bis VIII entweder direkt oder indirekt wirksam sind. Sie sind in jedem Fall zur wirksamen Bekämpfung von parasitären Helminthen geeignet.

Die erfindungsgemäßen Verbindungen sind nicht cytotoxisch gegenüber humanen Zelllinien, ausreichend stabil sowie einfach und kostengünstig herstellbar.

### Ausführungsbeispiele

Nachfolgend werden zunächst allgemeine Arbeitsvorschriften für die erfindungsgemäßen Substanzen beschrieben, sodann die im Rahmen dieser Offenbarung erarbeiteten Einzelbeispiele, die nicht einschränkend zu verstehen sind. Als Dezimaltrennzeichen wird bei Zahlenangaben der Dezimalpunkt verwendet.

### Allgemeine Arbeitsvorschrift für die Synthese von Carbonsäureamiden mit EDC·HCl/ HOBt (AAV4):

Eine auf 0 °C gekühlte Lösung oder Suspension des entsprechenden Amins (1.0 eq), der entsprechenden Carbonsäure (1.0 eq) und Triethylamin (2.0-3.5 eq) in Dichlormethan wurde mit EDC·HCl (1.5 eq) und HOBt (1.5 eq) versetzt. Das Reaktionsgemisch wurde 12-36 Stunden bei Raumtemperatur gerührt. Die organische Phase wurde dreimal mit 1M NaOH-Lösung und gesättigter NaCl-Lösung gewaschen, über MgSO₄ getrocknet und das Lösungsmittel am Rotavapor entfernt. Der verbleibende Rückstand wurde durch Säulenchromatographie an Kieselgel gereinigt.

### Allgemeine Arbeitsvorschrift Nr. 5 (AAV5) für die Synthese von Carbamodithioat-Derivaten:

In einem 25 mL Kolben wurden das entsprechende Alkyl(aralkyl)halogenid (1.0 eq) und Kohlenstoffdisulfid (2.0 eq) in Acetonitril (4-10 mL/mmol Alkyl(aryl)halogenid) gelöst, auf 0 °C gekühlt und 10 Minuten gerührt. Zu dem Reaktionsgemisch wurde anschließend langsam das entsprechende Amin (2.0 eq) zugegeben. Nach weiteren 30 Minuten Reaktionszeit wurde das Reaktionsgemisch auf Raumtemperatur erwärmt und weitere 1 bis 8 Stunden gerührt. Anschließend wurde das Reaktionsgemisch mit der dreifachen Menge Wasser versetzt, mit Ethylacetat extrahiert (5 x 50 mL), die vereinigten organischen Phasen über MgSO₄ getrocknet, filtriert und das Lösungsmittel am Rotavapor entfernt. Der verbleibende Rückstand wurde durch Säulenchromatographie an Kieselgel gereinigt.

### Allgemeine Arbeitsvorschrift Nr. 6 (AAV6) für die Synthese von Carbamodithioat-Derivaten:

Literaturbekannt ist die Synthese von Carbamodithioat-Derivaten **2** durch Reaktion in Acetonitril, in Ethanol unter Zusatz von Base bzw. unter Verwendung von Ethanol/Wasser- oder Ethanol/Diethylether-Gemischen. Bekannt ist ebenfalls die Reaktion mit THF unter Zusatz von Base und die Verwendung von THF/Hexan oder THF/DMF Gemischen. Überraschenderweise wurde gefunden, daß die Synthese aber auch ohne Zusatz einer separaten Base mit guten Ausbeuten wie nachfolgend beschrieben durchgeführt werden kann.
In einem 25 mL Kolben wurden das entsprechende Amin (2.0 eq) und Kohlenstoffdisulfid (2.0 eq) in Acetonitril, Tetrahydrofuran oder Ethanol (3-12 mL/mmol Amin) gelöst, auf 0 °C gekühlt und 10 Minuten gerührt. Zu dem Reaktionsgemisch wurde anschließend langsam das entsprechende Alkyl(aralkyl)halogenid (1.0 eq) zugegeben. Nach weiteren 15 Minuten Reaktionszeit wurde das Reaktionsgemisch auf Raumtemperatur erwärmt und weitere 3 bis 12 Stunden gerührt. Anschließend wurde das Reaktionsgemisch mit der dreifachen Menge Wasser versetzt, mit Ethylacetat extrahiert (5 x 50 mL), die vereinigten organischen Phasen über MgSO₄ getrocknet, filtriert und das Lösungsmittel am Rotavapor entfernt. Der verbleibende Rückstand wurde durch Säulenchromatographie an Kieselgel gereinigt.

### Allgemeine Arbeitsvorschrift Nr. 7 (AAV7) für die Synthese von Carbamodithioat-Derivaten:

Literaturbekannt ist die Synthese von Carbamodithioat-Derivaten **3** durch Reaktion in Aceton mit K₃PO₄ sowie in Acetonitril mit KOH oder K₂CO₃. Überraschenderweise wurde gefunden, daß die Synthese aber auch in Acetonitril unter Verwendung von K₃PO₄ durchgeführt werden kann.
In einem 25 mL Kolben wurden das entsprechende Amin (2.0 eq) und wasserfreies K₃PO₄ (1.5-3.5 eq) in Aceton oder Acetonitril (5-10 mL/mmol Amin) gelöst bzw. suspendiert, 10 Minuten gerührt, mit Kohlenstoffdisulfid (2.0 eq) versetzt und weitere 30 Minuten gerührt. Zu dem Reaktionsgemisch wurde anschließend langsam das entsprechende Alkyl(aralkyl)halogenid (1.0 eq) zugegeben und weitere 8 bis 12 Stunden gerührt. Das K₃PO₄ wurde abfiltriert und das Lösungsmittel am Rotavapor entfernt. Der verbleibende Rückstand wurde durch Säulenchromatographie an Kieselgel gereinigt.

### Allgemeine Arbeitsvorschrift Nr. 8 (AAV8) für die Synthese von Carbamodithioat-Derivaten:

Reaktionen in Acetonitril mit Triethylamin sind zwar bei 0grd-C bekannt, jedoch nicht bei Raumtemperatur. Die hier überraschenderweise gefundene Durchführbarkeit bei Raumtemperatur stellt daher eine deutliche Verbesserung der bekannten Verfahren dar, da auf die energieaufwendige Temperierung der Reaktionsmischung verzichtet werden kann.
In einem 25 ml Kolben wurden das entsprechende Amin (2.0 eq) und, falls nötig, Triethylamin (2.0 eq) in Acetonitril (5-10 mL/mmol Amin) gelöst bzw. suspendiert, 15 Minuten gerührt, mit Kohlenstoffdisulfid (2.0 eq) versetzt und weitere 45 Minuten gerührt. Zu dem Reaktionsgemisch wurde anschließend langsam das entsprechende Alkyl(aralkyl)halogenid (1.0 eq) zugegeben und weitere 2 bis 8 Stunden gerührt. Das Lösungsmittel wurde am Rotavapor entfernt. Der verbleibende Rückstand wurde durch Säulenchromatographie an Kieselgel gereinigt.

### Allgemeine Arbeitsvorschrift Nr. 9 (AAV9) für die Synthese von Imidazol-1-carbodithioat-Derivaten:

In einem 50 mL Kolben wurden Imidazol (1.0 eq) und wasserfreies K₃PO₄ (1.0 eq) in Aceton (10-15 mL/mmol Imidazol) gelöst bzw. suspendiert, 1 Stunde gerührt, mit Kohlenstoffdisulfid (3.0 eq) versetzt und nochmals 1 Stunde gerührt. Zu dem Reaktionsgemisch wurde anschließend langsam das entsprechende Alkyl(aralkyl)halogenid (1.0-2.0 eq) zugegeben und weitere 12 bis 18 Stunden gerührt. Das K₃PO₄ wurde abfiltriert und das Lösungsmittel am Rotavapor entfernt. Der verbleibende Rückstand wurde durch Säulenchromatographie an Kieselgel gereinigt.

### Allgemeine Arbeitsvorschrift Nr. 10 (AAV10) für die Synthese von Carbamodithioat-Derivaten:

In einem 50 mL Kolben wurden das entsprechende Amin (2.0 eq) und Natriumcarbonat bzw. Natriumhydrogencarbonat (1.5-2.5 eq) in Acetonitril (10 mL/mmol Amin) gelöst bzw. suspendiert, 30 Minuten gerührt, mit Kohlenstoffdisulfid (2.0 eq) versetzt und weitere 60 Minuten gerührt. Zu dem Reaktionsgemisch wurde anschließend langsam das entsprechende Alkyl(aralkyl)halogenid (1.0 eq) zugegeben und weitere 4 bis 10 Stunden gerührt. Das Natriumcarbonat bzw. Natriumhydrogencarbonat wurde abfiltriert und das Lösungsmittel am Rotavapor entfernt. Der verbleibende Rückstand wurde durch Säulenchromatographie an Kieselgel gereinigt.

### Allgemeine Arbeitsvorschrift Nr. 11 (AAV11) für die Entschützung von Boc-Schutzgruppen:

Eine Lösung der entsprechenden *Boc*-geschützten Verbindung in 1,4-Dioxan (3 mL/mmol *Boc*-geschützter Verbindung) wurde langsam mit 4M HCl in 1,4-Dioxan (1 mL/mmol *Boc*-geschützter Verbindung) versetzt und 1-3 Stunden bei Raumtemperatur gerührt. Der resultierende Niederschlag wurde abgesaugt, mit Pentan und 1,4-Dioxan gewaschen und getrocknet.

### Allgemeine Arbeitsvorschrift Nr. 12 (AAV12) für die Darstellung von Piperazinsulfonamiden:

Die Aminkomponente (1.0 eq) wurden in trockenem Pyridin (6-10 mL/mmol Aminkomponente) gelöst, auf 0 °C gekühlt, 15 Minuten gerührt und anschließend langsam mit dem entsprechenden Sulfonylchlorid (1.5 eq), gelöst in trockenem Pyridin (3 mL/mmol Sulfonylchlorid), versetzt. Nach 2 h Reaktionszeit wurde das Reaktionsgemisch langsam auf Raumtemperatur erwärmt und weitere 4-10 Stunden gerührt. Das Reaktionsgemisch wurde mit der 10-fachen Menge an Wasser (bezogen auf das Pyridin) verdünnt, der resultierende Niederschlag abgesaugt, mit Wasser gewaschen, getrocknet und durch Säulenchromatographie an Kieselgel gereinigt.

### Darstellung von 2-(Diethylcarbamothioylsulfanyl)essigsäure (Schl32003)

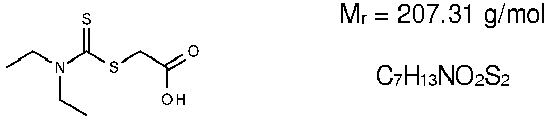

0.6 g Natriumhydroxid (15.0 mmol, 1.5 eq) und 1.0 mL Diethylamin (10.0 mmol, 1.0 eq) wurden in 10 mL Wasser gelöst, 5 Minuten bei Raumtemperatur gerührt und anschließend langsam 0.8 mL Kohlenstoffdisulfid (12.5 mmol, 1.25 eq) zugetropft. Nach 30 Minuten Reaktionszeit wurden 0.4 g Natriumhydroxid (10.0 mmol, 1.0 eq), 1.7 g Bromessigsäure (12.5 mmol, 1.25 eq) und 8 mL Wasser zugegeben und weitere 18 Stunden bei Raumtemperatur gerührt. Das Reaktionsgemisch wurde mit 6M HCl auf pH=3 eingestellt, mit Dichlormethan extrahiert (5 x 100 mL), die vereinigten organischen Phasen über MgSO₄ getrocknet, filtriert und das Lösungsmittel am Rotavapor entfernt. Der Rückstand wurde in wenig Dichlormethan gelöst und in 200 mL eisgekühltes Cyclohexan getropft. Der resultierende Niederschlag wurde abgesaugt und getrocknet. Es wurden 1.14 g von **Schl32003** (55.2%) in Form farbloser Nadeln erhalten.

| | | | | |
|---|---|---|---|---|
| **Fp.:** | 85 °C | | | |
| **¹H-NMR:** | (CDCl₃, 400 MHz), δ [ppm] = 4.18 (s, 2H, *CH₂*-COOH), 4.01 (*q*, ³J = 7.2 Hz, 2H, CH₃-*CH₂*), 3.76 (*q*, ³J = 7.2 Hz, 2H, CH₃-*CH₂*), 1.32 (*t,* ³J = 7.2 Hz, 3H, CH₂-C*H₃*), 1.27 *(t,* ³J = 7.2 Hz, 3H, CH₂-*CH₃*). | | | |
| **¹³C**-**NMR:** | (CDCl₃, 100 MHz), δ [ppm] = 193.8 (C=S), 173.2 (COOH), 50.0 (CH₂-CH₃), 47.4 (CH₂-CH₃), 38.6 (S-CH₂), 12.6 (CH₃), 11.6 (CH₃). | | | |
| **IR (ATR):** | v (cm⁻¹) = 2980 (m), 2934 (w), 2894 (w), 1709 (vs), 1573 (w), 1473 (s), 1252 (m), 1422 (s), 1380 (m), 1350 (s), 1311 (s), 1268 (s), 1212 (s), 1197 (vs), 1141 (s), 1083 (m), 1067 (s), 1003 (m), 980 (s), 911 (s), 878 (s), 828 (s), 791 (m), 773 (m), 666 (s), 595 (m), 559 (w), 456 (m), 427 (m). | | | |
| **MS** (ESI+): | m/z (%) = 116 (100, [NH₂-(C=S)-SH+Na]⁺), 208 (60, [M+H]⁺), 230 (50, [M+Na]⁺) | | | |
| **HRMS** (ESI+): | m/z für C₇H₁₃NO₂S₂ | | | |
| | ber.: | 207.0388 | | gef.: 207.0392 |
| **EA:** | ber.: | C: 40.55 | H: 6.32 | N: 6.76 |
| | gef.: | C: 40.45 | H: 6.86 | N: 6.32 |

### Darstellung von Methyl 2-(diethylcarbamothioylsulfanyl)acetat (Schl32006):

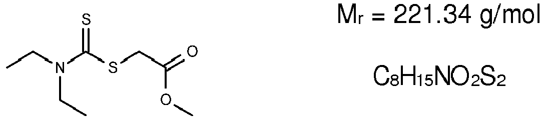

Gemäß **AAV5** wurden 0.28 mL Bromessigsäuremethylester (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Pentan/ EtOAc 5:1) ergab 539 mg von **Schl32006** (81.1 %) in Form eines blassgelben Öls.

| | | | | |
|---|---|---|---|---|
| **DC:** | R_{f} = 0.17 (Cyclohexan/ EtOAc 6:1). | | | |
| **¹H-NMR:** | (CDCl₃, 400 MHz), δ [ppm] = 4.00 (s, 2H, S-*CH₂*-COOMe), 3.85 (*q*, ³J = 7.2 Hz, 2H, CH₃-*CH₂*), 3.63 (q, ³J = 7.2 Hz, 2H, CH₃-C*H₂*), 3.59 (*s*, 3H, C(=O)-O-C*H₃*), 1.18 (*t*, ³J = 7.2 Hz, 3H, CH₂-*CH₃*), 1.10 (*t*, ³J = 7.2 Hz, 3H, CH₂-*CH₃*). | | | |
| **¹³C**-**NMR:** | (CDCl₃, 100 MHz), δ [ppm] = 193.5 (C=S), 169.1 (COOMe), 52.6 C(=O)-O-CH₃), 50.0 (CH₂-CH₃), 47.0 (CH₂-CH₃), 38.8 (S-CH₂), 12.6 (CH₂-CH₃), 11.5 (CH₂-CH₃). | | | |
| **IR (ATR):** | v (cm⁻¹) = 2977 (m), 2950 (w), 2874 (w), 2838 (w), 1739 (s), 1567 (m), 1491 (m), 1420 (m), 1379 (w), 1356 (m), 1297 (m), 1271 (s), 1209 (m), 1159 (s), 1094 (w), 1075 (w), 1008 (m), 984 (m), 919 (w), 880 (w), 831 (w), 775 (w), 688 (w), 594 (w), 568 (w), 496 (w), 432 (w). | | | |
| **MS** (ESI+): | m/z (%) = 116 (100, [NH₂-(C=S)-SH+Na]⁺), 222 (90, [M+H]⁺) | | | |
| **HRMS** (ESI+): | m/z für C₈H₁₅NO₂S₂ | | | |
| | ber.: | 221.0544 | | gef.: 221.0532 |
| **EA:** | ber.: | C: 43.41 | H: 6.83 | N: 6.33 |
| | gef.: | C: 43.26 | H: 6.82 | N: 6.21 |

### Darstellung von Benzyl-N,N-diethylcarbamodithioat (Schl32007)

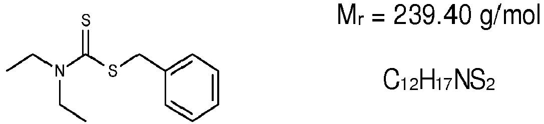

Gemäß **AAV5** wurden 0.50 mL Benzylbromid (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Pentan/ EtOAc 6:1) ergab 627 mg von **Schl32007** (87.3%) in Form eines gelben Öls.

| | | | | |
|---|---|---|---|---|
| **DC:** | R_{f} = 0.38 (Cyclohexan/ EtOAc 6:1). | | | |
| **¹H-NMR:** | (CDCl₃, 400 MHz), δ [ppm] = 7.40-7.38 (*m*, 2H, CH₂-C-*CH-*CH, CH₂-C-C*H*-CH), 7.33-7.29 (*m*, 2H, CH₂-C-CH-C*H*, CH₂-C-CH-C*H*), 7.28-7.22 (*m*, 1H, CH₂-C-CH-CH-C*H*), 4.55 (*s*, 2H, S-C*H₂*-C), 4.04 (*q,* ³J = 7.2 Hz, 2H, CH₃-C*H₂*), 3.72 (*q,* ³J = 7.2 Hz, 2H, CH₃-C*H₂*), 1.31-1.24 (m, 6H, CH₂-C*H₃*, CH₂-*CH₃*). | | | |
| **¹³C**-**NMR:** | (CDCl₃, 100 MHz), δ [ppm] = 193.3 (C=S), 136.1 (C-CH-CH), 129.5 (C-CH-CH), 128.7 (C-CH-CH), 127.6 (C-CH-CH-CH), 49.6 (CH₂-CH₃), 46.8 (CH₂-CH₃), 42.3 (S-CH₂-C), 12.6 (CH₂-CH₃), 11.7 (CH₂-CH₃). | | | |
| **IR (ATR):** | v (cm⁻¹) = 3060 (w), 2974 (m), 2931 (m), 1648 (m), 1601 (w), 1583 (w), 1485 (s), 1452 (s), 1441 (m), 1415 (vs), 1378 (m), 1354 (s), 1300 (m), 1268 (vs), 1207 (vs), 1141 (s), 1115 (m), 1092 (s), 1068 (m), 1028 (s), 1008 (s), 938 (s), 916 (s), 846 (s), 831 (m), 804 (m), 779 (m), 698 (s), 665 (s), 620 (m), 593 (w), 562 (m), 481 (m), 433 (w). | | | |
| **MS** (ESI+): | m/z (%) = 116 (100, [NH₂-(C=S)-SH+Na]⁺), 240 (60, [M+H]⁺) | | | |
| **HRMS** (ESI+): | m/z für C₁₂H₁₇NS₂ | | | |
| | ber.: | 239.0802 | | gef.: 239.0812 |
| **EA:** | ber.: | C: 60.20 | H:7.16 | N: 5.85 |
| | gef.: | C: 60.33 | H:7.19 | N: 5.83 |

### Darstellung von Methyl-N,N-diethylcarbamodithioat (Schl32008):

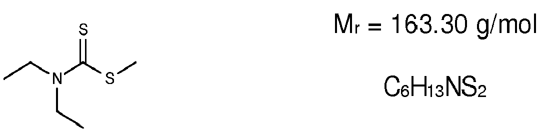

Gemäß **AAV5** wurden 0.19 mL Methyliodid (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Pentan/ EtOAc 6:1) ergab 460 mg von **Schl32008** (93.9%) in Form eines blassgelben Öls.

| | | | | |
|---|---|---|---|---|
| **DC:** | R_{f} = 0.33 (Cyclohexan/ EtOAc 6:1). | | | |
| **¹H-NMR:** | (CDCl₃, 400 MHz), δ [ppm] = 3.86-3.81 (*m*, 2H, C*H₂*-CH₃), 3.58-3.53 (*m*, 2H, C*H₂*-CH₃), 2.42 (s, 2H, S-C*H₃*), 1.11-1.05 (*m,* 6H*,* CH₂-C*H₃*, CH₂-*CH₃*). | | | |
| **¹³C**-**NMR:** | (CDCl₃, 100 MHz), δ [ppm] = 196.6 (C=S), 49.5 (CH₂-CH₃), 46.7 (CH₂-CH₃), 20.0 (S-CH₃), 12.5 (CH₂-CH₃), 11.6 (CH₂-CH₃). | | | |
| **IR (ATR):** | v (cm⁻¹) = 3150 (m), 2974 (w), 2932 (w), 2915 (w), 2872 (w), 2066 (w), 1929 (w), 1698 (w), 1619 (w), 1593 (w), 1563 (w), 1485 (s), 1457 (m), 1440 (m), 1414 (s), 1378 (m), 1354 (m), 1299 (w), 1266 (s), 1206 (s), 1142 (m), 1091 (w), 1068 (m), 1011 (m), 985 (m), 957 (m), 916 (s), 832 (m), 775 (w), 727 (w), 592 (w), 563 (m), 497 (w), 466 (m), 432 (m). | | | |
| **MS** (ESI+): | m/z (%) = 116 (90, [NH₂-(C=S)-SH+Na]⁺), 164 (100, [M+H]⁺) | | | |
| **HRMS** (ESI+): | m/z für C₆H₁₃NS₂ | | | |
| | ber.: | 163.0489 | | gef.: 163.0485 |
| **EA:** | ber.: | C: 44.13 | H: 8.02 | N: 8.58 |
| | gef.: | C: 44.45 | H: 7.98 | N: 8.38 |

### Darstellung von (2-Amino-2-oxo-ethyl)-N,N-diethylcarbamodithioat (Schl32017):

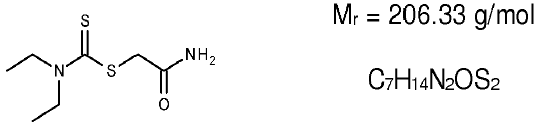

Gemäß **AAV6** wurden 0.62 mL Diethylamin (6.0 mmol, 2.0 eq), 0.36 mL Kohlenstoffdisulfid (6.0 mmol, 2.0 eq) und 414 mg Bromacetamid (3.0 mmol, 1.0 eq) umgesetzt. Säulenchromatographie an Kieselgel (DCM/ MeOH 95:5) ergab 530 mg von **Schl32017** (85.6%) in Form eines blassgelben Feststoffs.

| | | | | |
|---|---|---|---|---|
| **DC:** | R_{f} = 0.42 (DCM/ MeOH 95:5). | | | |
| **Fp.:** | 44 °C | | | |
| **¹H-NMR:** | (CDCl₃, 400 MHz), δ [ppm] = 6.77 (*sbr,* 1H, N*H*H), 6.12 (*sbr,* 1H, N*H*H), 4.04 (*s*, 2H, S-C*H₂*), 3.99-3.95 (*m,* 2H, C*H₂*-CH₃), 3.75-3.70 (*m,* 2H, C*H₂*-CH₃), 1.31-1.19 (*m*, 6H, CH₂-C*H₃*, CH₂-*CH₃*). | | | |
| **¹³C**-**NMR:** | (CDCl₃, 100 MHz), δ [ppm] = 194.0 (C=S), 171.7 (C=O), 50.6 (CH₂-CH₃), 47.3 (CH₂-CH₃), 39.5 (S-CH₂), 12.6 (CH₂-CH₃), 11.6 (CH₂-CH₃). | | | |
| **IR (ATR):** | v (cm⁻¹) =2436 (w), 3400 (w), 3307 (w), 1655 (vs), 1609 (s), 1552 (w), 1483 (s), 1449 (m), 1413 (s), 1370 (m), 1347 (vs), 1297 (m), 1266 (vs), 1245 (s), 1203 (vs), 1140 (vs), 1083 (s), 1063 (m), 1010 (s), 985 (s), 916 (m), 895 (m), 867 (m), 827 (m), 768 (m), 595 (s), 555 (s), 531 (s), 488 (s), 472 (vs), 438 (vs), 423 (vs). | | | |
| **MS** (ESI+): | m/z (%) = 207 (90, [M+H]⁺) | | | |
| **HRMS** (ESI+): | m/z für C₇H₁₄N₂OS₂ | | | |
| | ber.: | 206.0548 | | gef.: 206.0549 |
| **EA:** | ber.: | C:40.75 | H: 6.84 | N: 13.58 |
| | gef.: | C:40.53 | H: 6.78 | N: 13.18 |

### Darstellung von N,N-diethyl-1-methylsulfonylthioformamid (Schl32023):

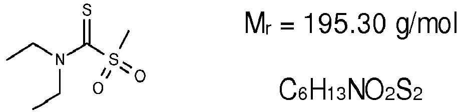

766 mg Natriummethansulfinat (7.5 mmol, 1.0 eq) und 1.14 g Diethyldithiocarbamoylchlorid (7.5 mmol, 1.0 eq) wurden in 20 mL Wasser suspendiert, mit 25 mL Toluol versetzt und anschließend 3 Stunden bei 40 °C gerührt. Die organischen Phase wurde über Calciumchlorid getrocknet, filtriert und das Lösungsmittel am Rotavapor entfernt. Es wurden 498 mg von **Schl32023** (34.1 %) in Form eines gelben Öls erhalten.

| | | | | |
|---|---|---|---|---|
| **¹H-NMR:** | (CDCl₃, 400 MHz), δ [ppm] = 4.07 (*q*, ³J= 7.2 Hz, 2H, C*H₂*-CH₃), 3.86 (*q*, ³J= 7.2 Hz, 2H, C*H₂*-CH₃), 3.45 (s, 2H, S-C*H₃*), 1.36 (*t,* ³J= 7.2 Hz, 3H, CH₂-*CH₃*), 1.29 (*t*, ³J= 7.2 Hz, 3H, CH₂-C*H₃*). | | | |
| **¹³C**-**NMR:** | (CDCl₃, 100 MHz), δ [ppm] = 188.2 (C=S), 49.8 (CH₂-CH₃), 48.0 (CH₂-CH₃), 43.6 (S-CH₃), 13.6 (CH₂-CH₃), 10.2 (CH₂-CH₃). | | | |
| **IR (ATR):** | v (cm⁻¹) = 3125 (w), 2978 (w), 2937 (w), 1579 (w), 1551 (w), 1503 (w), 1470 (s), 1471 (m), 1449 (m), 1429 (s), 1407 (w), 1357 (w), 1294 (vs), 1276 (vs), 1196 (w), 1150 (m), 1128 (vs), 1094 (m), 1075 (w), 1060 (m), 939 (m), 947 (s), 920 (m), 842 (w), 781 (s), 739 (m), 670 (w), 651 (w), 577 (m), 498 (s), 475 (m), 416 (w). | | | |
| **MS** (ESI+): | m/z (%) = 196 (90, [M+H]⁺) | | | |
| **HRMS** (ESI+): | m/z für C₆H₁₃NO₂S₂ | | | |
| | ber.: | 195.0388 | | gef.: 195.0393 |
| **EA:** | ber.: | C: 36.90 | H: 6.71 | N: 7.17 |
| | gef.: | C: 37.02 | H: 6.72 | N:7.18 |

### Darstellung von Natrium-N,N-diethylcarbamodithioat (Schl32024):

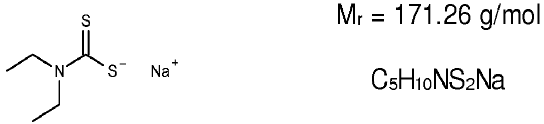

5.15 mL Diethylamin (50.0 mmol, 1.0 eq) und 4.0 mL 50%ige wässrige NaOH-Lösung wurden in 10 mL Ethanol gelöst, auf 0 °C gekühlt, 25 Minuten gerührt und anschließend 3.14 mL Kohlenstoffdisulfid (52.0 mmol, 1.04 eq) zutropft. Nach 30 Minuten Reaktionszeit wurde das Reaktionsgemisch auf Raumtemperatur erwärmt und weitere 18 Stunden gerührt. Das Lösungsmittel wurde am Rotavapor entfernt. Das Rohprodukt wurde anschließend aus Ethanol umkristallisiert, abgesaugt und mit Cyclohexan gewaschen. Es wurden 8.15 g von **Schl32024** (94.2%) in Form farbloser Nadeln erhalten.

| | | | | |
|---|---|---|---|---|
| **Fp.:** | 91 °C | | | |
| **¹H-NMR:** | (DMSO-D₆, 400 MHz), δ [ppm] = 3.97-3.91 (*m*, 4H, C*H₂*-CH₃), 1.05 (*t*, ³J=7.2 Hz, 3H, CH₂-*CH₃*). | | | |
| **¹³C**-**NMR:** | (DMSO-D₆, 100 MHz), δ [ppm] = 212.5 (C=S), 46.8 (CH₂-CH₃), 13.1 (CH₂-CH₃). | | | |
| **IR (ATR):** | v (cm⁻¹) = 3346 (m), 3238 (m), 2978 (w), 1615 (w), 1594 (w), 1562 (w), 1501 (m), 1474 (w), 1458 (w), 1434 (s), 1412 (w), 1377 (w), 1356 (w), 1343 (w), 1296 (w), 1261 (m), 1202 (s), 1129 (m), 1091 (w), 1074 (w), 1063 (m), 1062 (w), 1022 (m), 983 (s), 909 (s), 836 (m), 774 (w), 705 (w), 694 (w), 613 (w), 535 (s), 467 (s), 425 (s). | | | |
| **MS** (ESI+): | m/z (%) = 194 (100, [M+Na]+) | | | |
| **HRMS** (ESI+): | m/z für C₅H₁₀NS₂Na | | | |
| | ber.: | 171.0152 | | gef.: 171.0135 |
| **EA:** | ber.: | C: 35.07 | H: 5.89 | N: 8.18 |
| | gef.: | C: 36.48 | H: 6.09 | N: 7.91 |

### Darstellung von 2-(1,3-Dioxan-2-yl)ethyl-N,N-diethylcarbamodithioat (Schl32025):

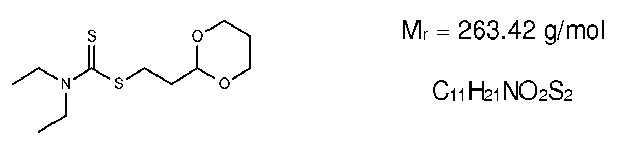

Gemäß **AAV6** wurden 0.40 mL Diethylamin (4.0 mmol, 2.0 eq), 0.24 mL Kohlenstoffdisulfid (4.0 mmol, 2.0 eq) und 0.24 mL 2-(2-Bromethyl)-1,3-dioxan (2.0 mmol, 1.0 eq) umgesetzt. Säulenchromatographie an Kieselgel (Pentan/ EtOAc 4:1) ergab 480 mg von **Schl32025** (91.1 %) in Form eines gelben Öls.

| | | | | |
|---|---|---|---|---|
| **DC:** | R_{f} = 0.19 (Cyclohexan/ EtOAc 5:1). | | | |
| **¹H-NMR:** | (CDCl₃, 400 MHz), δ [ppm] = 4.62 (*t*, ³J=5.2 Hz, 1H, CH₂-*CH-*O), 4.12-4.05 (*m*, 2H, CH-O-C*H*H-CH₂, CH-O-C*H*H-CH₂), 4.03-3.96 (*m*, 2H, CH-O-C*H*H-CH₂, CH-O-C*H*H-CH₂), 3.80-3.65 (*m*, 4H, C*H₂*-CH3, C*H₂*-CH₃), 3.36-3.32 (*m*, 2H, S-C*H₂*-CH₂), 2.11-2.01 (*m*, 1H, O-CH₂-C*H*H-CH₂-O), 2.01-1.94 (*m*, 2H, S-CH₂-C*H₂*), 1.35-1.29 (*m*, 1H, O-CH₂-C*H*H-CH₂-O), 1.28-1.19 (*m*, 6H, CH₂-C*H₃*, CH₂-*CH₃*). | | | |
| **¹³C**-**NMR:** | (CDCl₃, 100 MHz), δ [ppm] = 195.5 (C=S), 101.1 (CH₂-CH-O), 67.0 (O-CH₂-CH₂), 49.4 (CH₂-CH₃), 46.7 (CH₂-CH₃), 34.4 (S-CH₂), 31.8 (S-CH₂-CH₂), 25.9 (O-CH₂-CH₂-CH₂), 12.5 (CH₂-CH₃), 11.7 (CH₂-CH₃). | | | |
| **IR (ATR):** | v (cm⁻¹) = 2968 (m), 2930 (m), 2850 (m), 2732 (w), 2130 (w), 1997 (w), 1623 (w), 1486 (m), 1459 (m), 1441 (m), 1416 (m), 1378 (m), 1355 (m), 1301 (m), 1269 (m), 1242 (m), 1206 (m), 1145 (s), 1131 (s), 1077 (m), 1033 (m), 1003 (m), 946 (w), 920 (w), 875 (w), 841 (w), 778 (w), 640 (w), 594 (w), 565 (w), 477 (w), 444 (w). | | | |
| **MS** (ESI+): | m/z (%) = 264 (100, [M+H]⁺), 286 (85, [M+Na]⁺) | | | |
| **HRMS** (ESI+): | m/z für C₁₁H₂₁NO₂S₂ | | | |
| | ber.: | 263.1014 | | gef.: 263.1009 |
| **EA:** | ber.: | C: 50.15 | H: 8.04 | N: 5.32 |
| | gef.: | C: 49.73 | H: 7.95 | N: 5.30 |

### Darstellung von (2-Nitrophenyl)methyl-N,N-diethylcarbamodithioat (Schl32084):

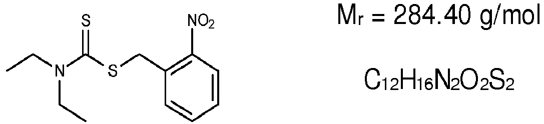

Gemäß **AAV5** wurden 648 mg 2-Nitrobenzylbromid (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 5:1) ergab 789 mg von **Schl32084** (92.5%) in Form eines gelben Öls.

| | | | | |
|---|---|---|---|---|
| **DC:** | R_{f} = 0.32 (Cyclohexan/ EtOAc 5:1). | | | |
| **¹H-NMR:** | (CDCl₃, 400 MHz), δ [ppm] = 7.97 (*d*, ³J = 8.0 Hz, 1H, CH₂-C-C(NO₂)-C*H*-CH), 7.82 (*d*, ³J = 7.6 Hz, 1H, CH₂-C-C*H-*CH), 7.54 (*t,* ³J = 7.6 Hz, 1H, CH₂-C-C(NO₂)-CH-C*H*), 7.40 (*t,* ³J = 7.6 Hz, 1H, CH₂-C-CH-C*H*), 4.96 (*s*, 2H, S-C*H₂*-C), 3.99 (*q*, ³J = 7.1 Hz, 2H, CH₃-C*H₂*), 3.70 (*q,* ³J = 7.1 Hz, 2H, CH₃-*CH₂*), 1.24 (*t*, ³J = 7.1 Hz, 6H, CH₂-C*H₃*, CH₂-*CH₃*). | | | |
| **¹³C**-**NMR:** | (CDCl₃, 100 MHz), δ [ppm] = 194.5 (C=S), 149.0 (CH₂-C-C(NO₂)-CH-CH), 148.6 (CH₂-C-CH-CH), 133.5 (CH₂-C-C(NO₂)-CH-CH), 133.0 (CH₂-C-CH-CH), 128.6 (CH₂-C-C(NO₂)-CH-CH), 125.0 (CH₂-C-CH-CH), 50.1 (CH₂-CH₃), 46.9 (CH₂-CH₃), 38.5 (S-CH₂-C), 12.6 (CH₂-CH₃), 11.6 (CH₂-CH₃). | | | |
| **IR (ATR):** | v (cm⁻¹) = 3066 (w), 2976 (w), 2932 (w), 1576 (w), 1522 (vs), 1486 (s), 1458 (m), 1442 (m), 1416 (s), 1378 (m), 1343 (vs), 1300 (m), 1269 (s), 1205 (s), 1163 (m), 1142 (m), 1092 (m), 1068 (m), 1007 (m), 982 (m), 917 (m), 884 (m), 860 (m), 831 (m), 808 (m), 785 (m), 760 (m), 712 (m), 665 (w), 581 (w), 563 (w), 499 (w), 431 (w). | | | |
| **MS** (ESI+): | m/z (%) = 285 (100, [M+H]⁺) | | | |
| **HRMS** (ESI+): | m/z für C₁₂H₁₆N₂O₂S₂ | | | |
| | ber.: | 284.0653 | | gef.: 284.0647 |
| **EA:** | ber.: | C: 50.68 | H: 5.67 | N: 9.85 |
| | gef.: | C: 50.45 | H: 5.71 | N: 9.53 |

### Darstellung von (3-Nitrophenyl)methyl-N,N-diethylcarbamodithioat (Schl32086):

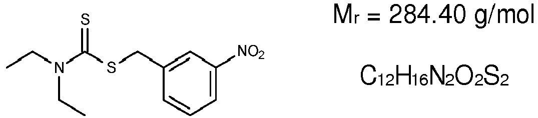

Gemäß **AAV5** wurden 648 mg 3-Nitrobenzylbromid (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 5:1) ergab 704 mg von **Schl32086** (82.5%) in Form eines gelben Öls.

| | | | | |
|---|---|---|---|---|
| **DC:** | R_{f} = 0.17 (Cyclohexan/ EtOAc 5:1). | | | |
| **¹H-NMR:** | (CDCl₃, 400 MHz), δ [ppm] = 8.19 (s, 1H, C-C*H-*C(NO₂)), 8.03 (*d,* ³J = 8.2 Hz, 1H, C-C*H*-CH-CH), 7.71 (*d*, ³J = 7.6 Hz, 1H, C-CH-CH-C*H*), 7.43 (*t,* ³J = 8.0 Hz, 1H, C-CH-C*H*-CH), 4.62 (*s*, 2H, S-C*H₂*-C), 3.99 (*q*, ³J = 7.1 Hz, 2H, CH₃-C*H₂*), 3.70 (*q,* ³J = 7.1 Hz, 2H, CH₃-*CH₂*), 1.28-1.19 (*m*, 6H, CH₂-C*H₃*, CH₂-*CH₃*). | | | |
| **¹³C**-**NMR:** | (CDCl₃, 100 MHz), δ [ppm] = 193.8 (C=S), 148.2 (CH₂-C-CH-C(NO₂)-CH), 139.5 (CH₂-C-CH-CH), 133.6 (CH₂-C-CH-CH-CH-C(NO₂)), 129.5 (CH₂-C-CH-CH-CH-C(NO₂)), 124.1 (CH₂-C-CH-C(NO₂)-CH-CH), 122.4 (CH₂-C-CH-CH-CH-C(NO₂)), 50.0 (CH₂-CH₃), 47.0 (CH₂-CH₃), 40.5 (S-CH₂-C), 12.7 (CH₂-CH₃), 11.6 (CH₂-CH₃). | | | |
| **IR (ATR):** | v (cm⁻¹) = 2976 (w), 2932 (w), 1525 (s), 1486 (m), 1459 (w), 1441 (w), 1416 (m), 1347 (s), 1316 (w), 1299 (w), 1268 (w), 1200 (s), 1142 (w), 1093 (w), 1073 (w), 1007 (w), 981 (m), 914 (w), 830 (w), 810 (m), 775 (w), 720 (m), 679 (w), 576 (w), 561 (w), 495 (w), 429 (w). | | | |
| **MS** (ESI+): | m/z (%) = 285 (100, [M+H]⁺) | | | |
| **HRMS** (ESI+): | m/z für C₁₂H₁₆N₂O₂S₂ | | | |
| | ber.: | 284.0653 | | gef.: 284.0643 |
| **EA:** | ber.: | C: 50.68 | H: 5.67 | N: 9.85 |
| | gef.: | C: 50.43 | H: 5.79 | N: 9.86 |

### Darstellung von (4-Nitrophenyl)methyl-N,N-diethylcarbamodithioat (Schl32088):

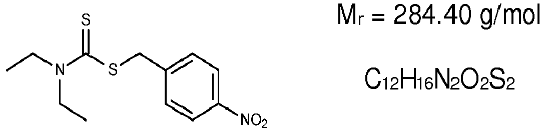

Gemäß **AAV5** wurden 1.54 g 4-Nitrobenzylbromid (7.0 mmol, 1.0 eq), 0.84 mL Kohlenstoffdisulfid (14.0 mmol, 2.0 eq) und 1.45 mL Diethylamin (14.0 mmol, 2.0 eq) umgesetzt. Säulenchromatographie an Kieselgel (Cyclohexan/ EtOAc 6:1) ergab 1.85 g von **Schl32088** (93.0%) in Form eines gelben Feststoffes.

| | | | | |
|---|---|---|---|---|
| **DC:** | R_{f} = 0.20 (Cyclohexan/ EtOAc 5:1). | | | |
| **Fp.:** | 70 °C | | | |
| **¹H-NMR:** | (CDCl₃, 400 MHz), δ [ppm] = 8.14 (*d*, ³J = 8.7 Hz, 2H, CH₂-C-CH-C*H*-C(NO₂), CH₂-C-CH-C*H-*C(NO₂)), 7.55 (*d,* ³J = 8.7 Hz, 2H, CH₂-C-C*H*-CH-C(NO₂), CH₂-C-C*H-*CH-C(NO₂)), 4.66 (s, 2H, S-C*H₂*-C), 4.02 (*q*, ³J = 7.1 Hz, 2H, CH₃-C*H₂*), 3.73 (*q*, ³J = 7.1 Hz, 2H, CH₃-C*H₂*), 1.31-1.21 (*m,* 6H, CH₂-C*H₃*, CH₂-C*H₃*). | | | |
| **¹³C**-**NMR:** | (CDCl₃, 100 MHz), δ [ppm] = 193.9 (C=S), 147.2 (C(NO₂)), 145.1 (CH₂-C-CH-CH), 130.2 (C(NO₂)-CH-CH, C(NO₂)-CH-CH), 123.8 (C-CH-CH, C-CH-CH), 50.1 (CH₂-CH₃), 47.0 (CH₂-CH₃), 40.7 (S-CH₂-C), 12.7 (CH₂-CH₃), 11.6 (CH₂-CH₃). | | | |
| **IR (ATR):** | v (cm⁻¹) = 3076 (w), 1600 (w), 1518 (m), 1493 (m), 1421 (w), 1341 (s), 1276 (m), 1195 (m), 1086 (w), 976 (m), 913 (w), 859 (m), 826 (m), 803 (m), 712 (s), 688 (m), 498 (m), 413 (w). | | | |
| **MS** (ESI+): | m/z (%) = 285 (100, [M+H]⁺) | | | |
| **HRMS** (ESI+): | m/z für C₁₂H₁₆N₂O₂S₂ | | | |
| | ber.: | 284.0653 | | gef.: 284.0646 |
| **EA:** | ber.: | C: 50.68 | H: 5.67 | N: 9.85 |
| | gef.: | C: 50.72 | H: 5.64 | N: 9.46 |

### Darstellung von (4-Aminophenyl)methyl-N,N-diethylcarbamodithioat (Schl32089):

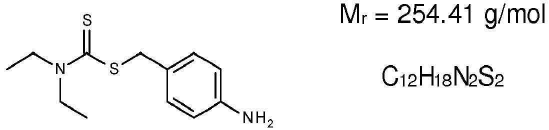

440 mg (4-Nitrophenyl)methyl-*N,N-*diethylcarbamodithioat **(Schl32088, Schl32089,** 1.55 mmol, 1.0 eq) wurden in 15 mL Methanol gelöst, mit 10 gew.-% Platin-(IV)-oxid versetzt und 4 Stunden unter H₂-Atmosphäre bei 40 °C gerührt. Die Lösung wird vom Katalysator abgetrennt und eingeengt. Säulenchromatographie an Kieselgel (Cyclohexan/ EtOAc 6:1) ergab 145 mg von **Schl32089** (36.7%) in Form eines braunen Öls.

| | | | | |
|---|---|---|---|---|
| **DC:** | R_{f} = 0.13 (Cyclohexan/ EtOAc 6:1). | | | |
| **¹H-NMR:** | (CDCl₃, 400 MHz), δ [ppm] = 7.15 (*d*, ³J = 6.2 Hz, 2H, CH₂-C-CH-C*H*-C(NH₂), CH₂-C-CH-C*H-*C(NH₂)), 6.62 (*d,* ³J = 6.2 Hz, 2H, CH₂-C-C*H*-CH-C(NH₂), CH₂-C-C*H-*CH-C(NH₂)), 4.40 (s, 2H, S-C*H₂*-C), 4.02 (*q,* ³J = 6.7 Hz, 2H, CH₃-C*H₂*), 3.69 (*q*, ³J = 6.7 Hz, 2H, CH₃-*CH₂*), 1.31-1.23 (*m*, 6H, CH₂-C*H₃*, CH₂-*CH₃*). | | | |
| **¹³C**-**NMR:** | (CDCl₃, 100 MHz), δ [ppm] = 195.8 (C=S), 145.8 (C(NH₂)), 130.6 (C(NH₂)-CH-CH, C(NH₂)-CH-CH), 125.2 (CH₂-C-CH-CH), 115.4 (C-CH-CH, C-CH-CH), 49.3 (CH₂-CH₃), 46.8 (CH₂-CH₃), 42.4 (S-CH₂-C), 12.5 (CH₂-CH₃), 11.7 (CH₂-CH₃). | | | |
| **IR (ATR):** | v (cm⁻¹) = 3341 (w), 2974 (w), 2930 (w), 1620 (m), 1514 (s), 1485 (s), 1457 (w), 1439 (w), 1414 (s), 1377 (w), 1353 (s), 1298 (w), 1266 (vs), 1237 (w), 1206 (s), 1177 (m), 1140 (m), 1091 (w), 1067 (m), 1005 (m), 981 (s), 915 (m), 827 (w), 775 (w), 746 (w), 674 (w), 638 (w), 593 (w), 565 (w), 528 (m), 492 (m), 429 (w). | | | |
| **MS** (ESI+): | m/z (%) = 255 (100, [M+H]⁺) | | | |
| **HRMS** (ESI+): | m/z für C₁₂H₁₈N₂S₂ | | | |
| | ber.: | 254.0911 | | gef.: 254.0906 |
| **EA:** | ber.: | C:56.65 | H:7.13 | N: 11.01 |
| | gef.: | C: 56.40 | H: 7.34 | N: 11.36 |

### Darstellung von Phenethyl-N,N-diethylcarbamodithioat (Schl32096):

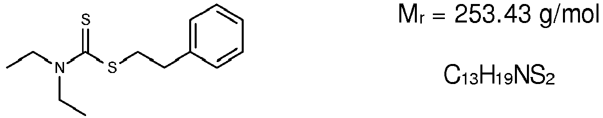

Gemäß **AAV5** wurden 0.41 mL (2-Bromethyl)benzen (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 6:1) ergab 643 mg von **Schl32096** (84.6%) in Form eines gelben Öls.

| | | | | |
|---|---|---|---|---|
| **DC:** | R_{f} = 0.39 (Cyclohexan/ EtOAc 6:1). | | | |
| **¹H-NMR:** | (CDCl₃, 400 MHz), δ [ppm] = 7.35-7.21 (*m*, 5H, CH₂-C-C*H*-C*H*-CH, CH₂-C-C*H*-C*H*-CH, CH₂-C-CH-CH-C*H*), 4.05 (*q,* ³J = 7.1 Hz, 2H, CH₃-C*H₂*), 3.73 (*q*, ³J = 6.9 Hz, 2H, CH₃-*CH₂*), 3.59-3.51 (*m*, 2H, S-C*H₂*-CH₂-C), 3.05-2.98 (*m*, 2H, S-CH₂-C*H₂*-C), 1.29 (*t,* ³J = 7.1 Hz, 6H, CH₂-C*H₃*, CH₂-*CH₃*). | | | |
| **¹³C**-**NMR:** | (CDCl₃, 100 MHz), δ [ppm] = 195.5 (C=S), 140.4 (C-CH-CH-CH), 128.8 (C-CH-CH, C-CH-CH), 128.6 (C-CH-CH, C-CH-CH), 126.5 (C-CH-CH-CH), 49.5 (CH₂-CH₃), 46.8 (CH₂-CH₃), 38.2 (S-CH₂-CH₂), 35.4 (S-CH₂-CH₂), 12.6 (CH₂-CH₃), 11.8 (CH₂-CH₃). | | | |
| **IR (ATR):** | v (cm⁻¹) = 3061 (w), 3025 (w), 2974 (m), 2931 (m), 1486 (s), 1454 (m), 1441 (m), 1416 (s), 1378 (w), 1355 (w), 1301 (w), 1268 (s), 1207 (s), 1139 (m), 1092 (w), 1073 (w), 1010 (w), 985 (w), 916 (w), 832 (w), 733 (w), 699 (w), 593 (w), 561 (w), 494 (w), 433 (w). | | | |
| **MS** (ESI+): | m/z (%) = 254 (100, [M+H]⁺) | | | |
| **HRMS** (ESI+): | m/z für C₁₃H₁₉NS₂ | | | |
| | ber.: | 253.0959 | | gef.: 253.0952 |
| **EA:** | ber.: | C: 61.61 | H: 7.56 | N: 5.53 |
| | gef.: | C: 61.97 | H: 7.68 | N: 5.64 |

### Darstellung von [(E)-Styryl]-N,N-diethylcarbamodithioat (Schl32097):

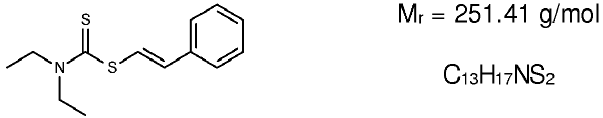

Gemäß **AAV5** wurden 0.39 mL *β*-Bromstyrol (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 3:1) ergab 147 mg von **Schl32097** (19.6%) in Form eines braunen Öls.

| | | | | |
|---|---|---|---|---|
| **DC:** | R_{f} = 0.31 (Cyclohexan/ EtOAc 3:1). | | | |
| **¹H-NMR:** | (CDCl₃, 400 MHz), δ [ppm] = 7.39-7.24 (m, 5H, CH₂-C-C*H*-C*H*-CH, CH₂-C-C*H*-C*H*-CH, CH₂-C-CH-CH-C*H*), 7.09 (*d*, ³J = 14.0 Hz, 1H, S-CH=C*H*), 6.76 (*d*, ³J = 14.0 Hz, 1H, S-C*H*=CH), 4.00 *(q,* ³J = 7.1 Hz, 4H, CH₃-C*H₂*, CH₃-*CH₂*), 1.46 (*t,* ³J = 6.9 Hz, 3H, CH₂-*CH₃*), 1.29 (*t,* ³J = 7.1 Hz, 3H, CH₂-*CH₃*). | | | |
| **¹³C**-**NMR:** | (CDCl₃, 100 MHz), δ [ppm] = 192.7 (C=S), 137.3 (S-CH=CH), 136.0 (C-CH-CH-CH), 128.9 (C-CH-CH, C-CH-CH), 128.4 (C-CH-CH, C-CH-CH), 126.2 (C-CH-CH-CH), 106.7 (S-CH=CH), 52.2 (CH₂-CH₃), 47.7 (CH₂-CH₃), 13.6 (CH₂-CH₃), 11.6 (CH₂-CH₃). | | | |
| **IR (ATR):** | v (cm⁻¹) = 2974 (w), 1488 (s), 1442 (m), 1416 (vs), 1379 (w), 1351 (m), 1268 (vs), 1198 (vs), 1145 (m), 1065 (m), 1004 (m), 967 (s), 913 (s), 817 (m), 733 (m), 691 (m), 565 (w), 428 (m). | | | |
| **MS** (ESI+): | m/z (%) = 252 (100, [M+H]⁺) | | | |
| **HRMS** (ESI+): | m/z für C₁₃H₁₇NS₂ | | | |
| | ber.: | 251.0802 | | gef.: 251.0812 |
| **EA:** | ber.: | C: 62.11 | H: 6.82 | N: 5.57 |
| | gef.: | C: 61.96 | H: 6.54 | N: 5.51 |

### Darstellung von 3-Phenylpropyl-N,N-diethylcarbamodithioat (Schl32098):

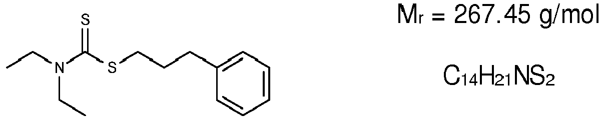

Gemäß **AAV5** wurden 0.46 mL 1-Brom-3-phenylpropan (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 6:1) ergab 764 mg von **Schl32098** (95.2%) in Form eines gelben Öls.

| | | | | |
|---|---|---|---|---|
| **DC:** | R_{f} = 0.43 (Cyclohexan/ EtOAc 9:1). | | | |
| **¹H-NMR:** | (CDCl₃, 400 MHz), δ [ppm] = 7.31-7.16 (*m*, 5H, CH₂-C-C*H*-C*H*-CH, CH₂-C-C*H*-C*H*-CH, CH₂-C-CH-CH-C*H*), 4.04 (*q,* ³J = 7.1 Hz, 2H, CH₃-C*H₂*), 3.75 (*q*, ³J = 7.1 Hz, 2H, CH₃-*CH₂*), 3.34 (*t,* ³J = 7.3 Hz, 2H, S-C*H₂*-CH₂-CH₂-C), 2.76 (*t,* ³J = 7.6 Hz, 2H, S-CH₂-CH₂-C*H₂*-C), 2.05 (*q*, ³J = 7.6 Hz, 2H, S-CH₂-C*H₂*-CH₂-C), 1.29 (*t*, ³J = 7.0 Hz, 6H, CH₂-C*H₃*, CH₂-C*H₃*). | | | |
| **¹³C**-**NMR:** | (CDCl₃, 100 MHz), δ [ppm] = 195.8 (C=S), 141.5 (C-CH-CH-CH), 128.6 (C-CH-CH, C-CH-CH), 128.5 (C-CH-CH, C-CH-CH), 126.1 (C-CH-CH-CH), 49.5 (CH₂-CH₃), 46.8 (CH₂-CH₃), 36.6 (S-CH₂-CH₂-CH₂), 35.2 (S-CH₂-CH₂-CH₂), 30.5 (S-CH₂-CH₂-CH₂), 12.6 (CH₂-CH₃), 11.8 (CH₂-CH₃). | | | |
| **IR (ATR):** | v (cm⁻¹) = 2931 (w), 1484 (s), 1454 (m), 1414 (vs), 1354 (m), 1267 (vs), 1205 (s), 1140 (m), 1069 (m), 982 (s), 916 (m), 832 (m), 742 (s), 697 (vs), 656 (w), 494 (w). | | | |
| **MS** (ESI+): | m/z (%) = 268 (100, [M+H]⁺) | | | |
| **HRMS** (ESI+): | m/z für C₁₄H₂₁NS₂ | | | |
| | ber.: | 267.1115 | | gef.: 267.1119 |
| **EA:** | ber.: | C: 62.87 | H: 7.91 | N: 5.24 |
| | gef.: | C: 62.81 | H: 7.99 | N: 5.35 |

### Darstellung von 4-Phenylbutyl-N,N-diethylcarbamodithioat (Schl32099):

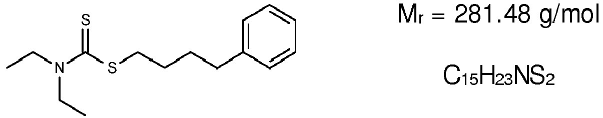

Gemäß **AAV5** wurden 0.53 mL 1-Brom-4-phenylbutan (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 6:1) ergab 553 mg von **Schl32099** (65.5%) in Form eines gelben Öls.

| | | | | |
|---|---|---|---|---|
| **DC:** | R_{f} = 0.13 (Cyclohexan). | | | |
| **¹H-NMR:** | (CDCl₃, 400 MHz), δ [ppm] = 7.31-7.18 (*m*, 5H, CH₂-C-C*H*-C*H*-CH, CH₂-C-C*H*-C*H*-CH, CH₂-C-CH-CH-C*H*), 4.03 (*q,* ³J = 6.9 Hz, 2H, CH₃-C*H₂*), 3.74 (*q*, ³J = 6.9 Hz, 2H, CH₃-C*H₂*), 3.32 (*t,* ³J = 6.9 Hz, 2H, S-C*H₂*-CH₂-CH₂-CH₂-C), 2.66 (*t,* ³J = 6.9 Hz, 2H, S-CH₂-CH₂-CH₂-C*H₂*-C), 1.80-1.69 (*m,* 4H, S-CH₂-C*H₂*-C*H₂*-CH₂-C), 1.28 (*t,* ³J = 6.6 Hz, 6H, CH₂-C*H₃*, CH₂-*CH₃*). | | | |
| **¹³C**-**NMR:** | (CDCl₃, 100 MHz), δ [ppm] = 196.0 (C=S), 142.3 (C-CH-CH-CH), 128.5 (C-CH-CH, C-CH-CH), 128.4 (C-CH-CH, C-CH-CH), 125.9 (C-CH-CH-CH), 49.5 (CH₂-CH₃), 46.8 (CH₂-CH₃), 37.2 (S-CH₂-CH₂-CH₂-CH₂), 35.6 (S-CH₂-CH₂-CH₂-CH₂), 30.9 (S-CH₂-CH₂-CH₂-CH₂), 28.4 (S-CH₂-CH₂-CH₂-CH₂), 12.6 (CH₂-CH₃), 11.8 (CH₂-CH₃). | | | |
| **IR (ATR):** | v (cm⁻¹) = 2931 (w), 1484 (s), 1454 (m), 1413 (vs), 1354 (m), 1266 (vs), 1206 (s), 1141 (m), 1070 (m), 983 (s), 916 (m), 832 (m), 743 (m), 698 (s), 565 (w). | | | |
| **MS** (ESI+): | m/z (%) = 282 (100, [M+H]⁺) | | | |
| **HRMS** (ESI+): | m/z für C₁₅H₂₃NS₂ | | | |
| | ber.: | 281.1272 | | gef.: 281.1274 |
| **EA:** | ber.: | C: 64.00 | H: 8.24 | N: 4.98 |
| | gef.: | C: 64.01 | H: 8.31 | N: 4.85 |

### Darstellung von 5-Phenylpentyl-N,N-diethylcarbamodithioat (Schl32102):

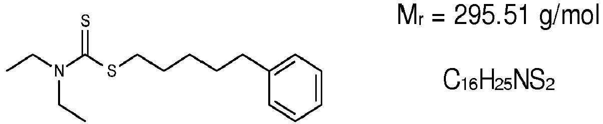

Gemäß **AAV5** wurden 0.33 mL 1-Brom-4-phenylbutan (1.76 mmol, 1.0 eq), 0.27 mL Kohlenstoffdisulfid (3.52 mmol, 2.0 eq) und 0.26 mL Diethylamin (3.52 mmol, 2.0 eq) umgesetzt. Säulenchromatographie an Kieselgel (Cyclohexan/ EtOAc 9:1) ergab 498 mg von **Schl32102** (95.7%) in Form eines gelben Öls.

| | | | | |
|---|---|---|---|---|
| **DC:** | R_{f} = 0.41 (Cyclohexan/ EtOAc 6:1). | | | |
| **¹H-NMR:** | (CDCl₃, 400 MHz), δ [ppm] = 7.34-7.18 (m, 5H, CH₂-C-C*H*-C*H*-CH, CH₂-C-C*H*-C*H*-CH, CH₂-C-CH-CH-C*H*), 4.05 (*q,* ³J = 7.1 Hz, 2H, CH₃-C*H₂*), 3.75 (*q*, ³J = 7.1 Hz, 2H, CH₃-C*H₂*), 3.31 (*t,* ³J = 7.6 Hz, 2H, S-C*H₂-*), 2.65 (*t,* ³J = 7.6 Hz, 2H, S-CH₂-CH₂-CH₂-CH₂-C*H₂*-C), 1.81-1.65 *(m,* 4H, S-CH₂-C*H₂*-CH₂-C*H₂*-CH₂-C), 1.56-1.44 (*m*, 2H, S-CH₂-CH₂-C*H₂*-CH₂-CH₂-C), 1.30 (*t,* ³J = 7.2 Hz, 6H, CH₂-C*H₃*, CH₂-*CH₃*). | | | |
| **¹³C**-**NMR:** | (CDCl₃, 100 MHz), δ [ppm] = 196.0 (C=S), 142.6 (C-CH-CH-CH), 128.6 (C-CH-CH, C-CH-CH), 128.4 (C-CH-CH, C-CH-CH), 125.8 (C-CH-CH-CH), 49.5 (CH₂-CH₃), 46.8 (CH₂-CH₃), 37.2 (S-CH₂-CH₂-CH₂-CH₂-CH₂), 35.9 (S-CH₂-CH₂-CH₂-CH₂-CH₂), 31.2 (S-CH₂-CH₂-CH₂-CH₂-CH₂), 28.8 (S-CH₂-CH₂-CH₂-CH₂-CH₂), 28.7 (S-CH₂-CH₂-CH₂-CH₂-CH₂), 12.6 (CH₂-CH₃), 11.8 (CH₂-CH₃). | | | |
| **IR (ATR):** | v (cm⁻¹) = 2930 (w), 1484 (m), 1453 (m), 1414 (s), 1355 (m), 1267 (s), 1206 (s), 1141 (m), 1069 (w), 984 (m), 916 (m), 832 (m), 745 (m), 698 (vs), 565 (m). | | | |
| **MS** (ESI+): | m/z (%) = 296 (100, [M+H]⁺) | | | |
| **HRMS** (ESI+): | m/z für C₁₆H₂₅NS₂ | | | |
| | ber.: | 295.1428 | | gef.: 295.1439 |
| **EA:** | ber.: | C: 65.03 | H: 8.53 | N: 4.74 |
| | gef.: | C: 64.84 | H: 8.84 | N: 4.88 |

### Darstellung von 2-(1,3-Dioxolan-2-yl)ethyl-N,N-diethylcarbamodithioat (Schl32116):

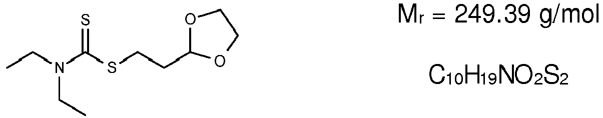

Gemäß **AAV5** wurden 0.36 mL 2-(2-Bromethyl)-1,3-dioxolan (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 6:1) ergab 598 mg von **Schl32116** (78.9%) in Form eines gelbbraunes Öls.

| | | | | |
|---|---|---|---|---|
| **DC:** | R_{f} = 0.20 (Cyclohexan/ EtOAc 6:1). | | | |
| **¹H-NMR:** | (CDCl₃, 400 MHz), δ [ppm] = 4.91 (*t*, ³J = 4.6 Hz, 1H, S-CH₂-CH₂-C*H*-O), 4.04-3.66 (*m*, 8H, O-C*H₂*C*H₂*-O, CH₃-*CH₂*, CH₃-C*H₂),* 3.34 (*t*, ³J = 6.4 Hz, 2H, S-C*H₂*-CH₂-CH-O), 2.05 (*q*, ³J = 7.1 Hz, 2H, S-CH₂-C*H₂-*CH-O), 1.22 (*t*, ³J = 7.2 Hz, 6H, CH₂-C*H₃*, CH₂-C*H₃*). | | | |
| **¹³C-NMR:** | (CDCl₃, 100 MHz), δ [ppm] = 195.4 (C=S), 103.4 (S-CH₂-CH₂-CH-O), 65.1 (O-CH₂-CH₂-O), 49.7 (CH₂-CH₃), 46.7 (CH₂-CH₃), 33.1 (S-CH₂-CH₂-CH), 31.5 (S-CH₂-CH₂-CH), 12.5 (CH₂-CH₃), 11.7 (CH₂-CH₃). | | | |
| **IR (ATR):** | v (cm⁻¹) = 2974 (w), 1486 (m), 1414 (vs), 1355 (m), 1267 (vs), 1205 (s), 1131 (vs), 981 (m), 916 (m), 864 (m), 831 (m), 563 (w). | | | |
| **MS** (ESI+): | m/z (%) = 250 (100, [M+H]⁺), 272 (30, [M+Na]⁺) | | | |
| **HRMS** (ESI+): | m/z für C₁₀H₁₉NO₂S₂ | | | |
| | ber.: | 249.0857 | | gef.: 249.0878 |
| **EA:** | ber.: | C:48.16 | H:7.68 | N: 5.62 |
| | gef.: | C: 48.14 | H:7.73 | N:5.47 |

### Darstellung von (4-MethylsuIfonylphenyl)methyl-N,N-diethylcarbamodithioat (Schl32118):

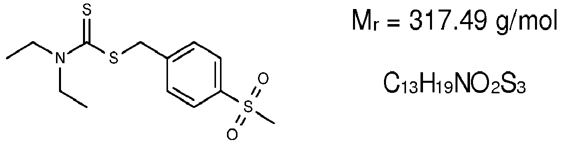

Gemäß **AAV5** wurden 747 mg 4-(Methylsulfonyl)benzylbromid (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 1:1) ergab 449 mg von **Schl32118** (47.2%) in Form eines gelbbraunen Feststoffes.

| | | | | |
|---|---|---|---|---|
| **DC:** | R_{f} = 0.30 (Cyclohexan/ EtOAc 1:1). | | | |
| **Fp.:** | 86°C | | | |
| **¹H-NMR:** | (CDCl₃, 400 MHz), δ [ppm] = 7.82 (*d*, ³J = 8.5 Hz, 2H, CH₂-C-CH-C*H-*C-SO₂Me, CH₂-C-CH-C*H*-C-SO₂Me), 7.56 (*d*, ³J = 8.2 Hz, 2H, CH₂-C-C*H-*CH-C-SO₂Me, CH₂-C-C*H-*CH-C-SO₂Me), 4.62 (*s*, 2H, S-C*H₂*-C), 3.99 (*q*, ³J = 7.1 Hz, 2H, CH₃-C*H₂*)*,* 3.70 (*q*, ³J = 7.1 Hz, 2H, CH₃-C*H₂*), 3.00 (*s*, 3H, SO₂-C*H₃*), 1.32-1.20 (*m*, 6H, CH₂-C*H₃*, CH₂-C*H₃*). | | | |
| **¹³C-NMR:** | (CDCl₃, 100 MHz), δ [ppm] = 194.0 (C=S), 143.8 (C-SO₂Me), 139.3 (CH₂-C-CH-CH), 130.3 (C-SO₂Me-CH-CH, C-SO₂Me-CH-CH), 127.6 (C-CH-CH, C-CH-CH), 50.1 (CH₂-CH₃), 47.0 (CH₂-CH₃), 44.6 (SO₂-CH₃), 40.8 (S-CH₂-C), 12.7 (CH₂-CH₃), 11.6 (CH₂-CH₃). | | | |
| **IR (ATR):** | v (cm⁻¹) = 2993 (w), 2933 (w), 1486 (m), 1456 (w), 1422 (m), 1381 (w), 1352 (w), 1312 (w), 1293 (s), 1268 (s), 1206 (s), 1186 (w), 1143 (vs), 1086 (s), 1071 (s), 1011 (m), 982 (m), 958 (m), 918 (s), 850 (m), 833 (m), 820 (m), 768 (s), 745 (s), 700 (w), 651 (m), 545 (s), 518 (vs), 502 (w), 484 (w), 436 (w), 412 (w). | | | |
| **MS** (ESI+): | m/z (%) = 318 (100, [M+H]⁺) | | | |
| **HRMS** (ESI+): | m/z für C₁₃H₁₉NO₂S₃ | | | |
| | ber.: | 317.0577 | | gef.: 317.0590 |
| **EA:** | ber.: | C: 49.18 | H: 6.03 | N: 4.41 |
| | gef.: | C: 49.14 | H: 6.05 | N: 4.34 |

### Darstellung von 1-Naphthylmethyl-N,N-diethylcarbamodithioat (Schl32120):

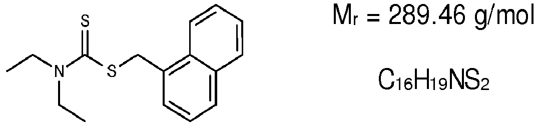

Gemäß **AAV5** wurden 530 mg 1-(Chlormethyl)naphthalen (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 6:1) ergab 795 mg von **Schl32120** (91.6%) in Form eines blassgelben Feststoffes.

| | | | | |
|---|---|---|---|---|
| **DC:** | R_{f}= 0.30 (Cyclohexan/ EtOAc 6:1). | | | |
| **Fp.:** | 68°C | | | |
| **¹H-NMR:** | (CDCl₃, 400 MHz), δ [ppm] = 8.14-8.08 (*m*, 1H, CH_{(Naphthyl)}), 7.89-7.78 (*m*, 2H, CH_{(Naphthyl)}), 7.62-7.39 (*m*, 4H, CH_{(Naphthyl)}), 5.00 (*s*, 2H, S-C*H₂*-C), 4.07 (*q,* ³J = 6.9 Hz, 2H, CH₃-C*H₂*), 3.66 (*q*, ³J = 7.1 Hz, 2H, CH₃-C*H₂*), 1.34-1.18 (*m*, 6H, CH₂-C*H₃*, CH₂-C*H₃*). | | | |
| **¹³C-NMR:** | (CDCl₃, 100 MHz), δ [ppm] = 195.3 (C=S), 134.0 (C_{q(Naphthyl)}), 132.0 (C_{q(Naphthyl)}), 131.5 (C_{q(Naphthyl)}), 128.9 (C_{t(Naphthyl)}), 128.8 (C_{t(Naphthyl)}), 128.4 (C_{t(Naphthyl)}), 126.5 (C_{t(Naphthyl)}), 126.1 (C_{t(Naphthyl)}), 125.6 (C_{t(Naphthyl)}), 124.2 (C_{t(Naphthyl)}), 49.5 (CH₂-CH₃), 46.9 (CH₂-CH₃), 40.6 (S-CH₂-C), 12.6 (CH₂-CH₃), 11.8 (CH₂-CH₃). | | | |
| **IR (ATR):** | v (cm⁻¹) = 2976 (w), 1484 (s), 1416 (s), 1377 (m), 1355 (m), 1300 (m), 1269 (s), 1199 (s), 1137 (s), 1070 (m), 978 (m), 917 | | | |
| | (m), 829 (m), 801 (m), 774 (vs), 632 (m), 561 (m), 475 (m), 423 (s). | | | |
| **MS** (ESI+): | m/z (%) = 290 (100, [M+H]⁺) | | | |
| **HRMS** (ESI+): | m/z für C₁₆H₁₉NS₂ | | | |
| | ber.: | 289.0959 | | gef.: 289.0957 |
| **EA:** | ber.: | C: 66.39 | H: 6.62 | N: 4.84 |
| | gef.: | C: 66.36 | H: 6.68 | N: 4.69 |

### Darstellung von 2-Naphthylmethyl-N,N-diethylcarbamodithioat (Schl32121):

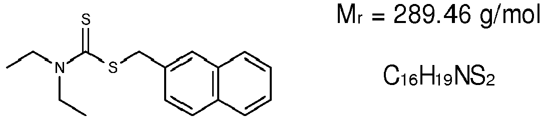

Gemäß **AAV5** wurden 530 mg 2-(Brommethyl)naphthalen (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 9:1) ergab 699 mg von **Schl32121** (80.5%) in Form eines blassgelben Feststoffes.

| | | | | |
|---|---|---|---|---|
| **DC:** | R_{f}= 0.24 (Cyclohexan/ EtOAc 9:1). | | | |
| **Fp.:** | 58°C | | | |
| **¹H-NMR:** | (CDCl₃, 400 MHz), δ [ppm] = 7.86-7.75 (*m*, 4H, CH_{(Naphthyl)}), 7.52-7.40 (*m*, 3H, CH_{(Naphthyl)}), 4.72 (*s*, 2H, S-C*H₂*-C), 4.05 *(q,* ³J = 7.1 Hz, 2H, CH₃-C*H₂),* 3.72 (*q*, ³J = 7.1 Hz, 2H, CH₃-C*H₂),* 1.32-1.21 (*m*, 6H, CH₂-C*H₃*, CH₂-C*H₃*). | | | |
| **¹³C-NMR:** | (CDCl₃, 100 MHz), δ [ppm] = 195.2 (C=S), 133.7 (C_{q(Naphthyl)}), 133.4 (C_{q(Naphthyl)}), 132.8 (C_{q(Naphthyl)}), 128.4 (C_{t(Naphthyl)}), 128.3 (C_{t(Naphthyl)}), 127.9 (C_{t(Naphthyl)}), 127.8 (C_{t(Naphthyl)}), 127.5 (C_{t(Naphthyl)}), 126.3 (C_{t(Naphthyl)}), 126.0 (C_{t(Naphthyl)}), 49.6 (CH₂-CH₃), 46.9 (CH₂-CH₃), 42.6 (S-CH₂-C), 12.6 (CH₂-CH₃), 11.8 (CH₂-CH₃). | | | |
| **IR (ATR):** | v (cm⁻¹) = 2974 (w), 1486 (s), 1454 (w), 1414 (s), 1377 (m), 1350 (m), 1297 (m), 1266 (s), 1201 (s), 1141 (m), 1068 (m), 1005 (m), 980 (m), 910 (m), 860 (m), 820 (s), 759 (s), 566 (w), 473 (vs). | | | |
| **MS** (ESI+): | m/z (%) = 290 (100, [M+H]⁺) | | | |
| **HRMS** (ESI+): | m/z für C₁₆H₁₉NS₂ | | | |
| | ber.: | 289.0959 | | gef.: 289.0946 |
| **EA:** | ber.: | C: 66.39 | H: 6.62 | N: 4.84 |
| | gef.: | C: 66.47 | H: 6.64 | N: 4.79 |

### Darstellung von 2-Pyridylmethyl-N,N-diethylcarbamodithioat (Schl32124):

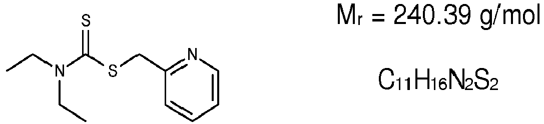

Gemäß **AAV7** wurden 0.62 mL Diethylamin (6.0 mmol, 2.0 eq), 2289 mg Kaliumphosphat (10.5 mmol, 3.5 eq), 0.36 mL Kohlenstoffdisulfid (6.0 mmol, 2.0 eq) und 759 mg 2-(Brommethyl)pyridinhydrobromid (3.0 mmol, 1.0 eq) umgesetzt. Säulenchromatographie an Kieselgel (Cyclohexan/ EtOAc 1:1) ergab 699 mg von **Schl32124** (96.5%) in Form eines gelbbraunen Öls.

| | | | | |
|---|---|---|---|---|
| **DC:** | R_{f}= 0.33 (Cyclohexan/ EtOAc 1:1). | | | |
| **¹H-NMR:** | (CDCl₃, 400 MHz), δ [ppm] = 8.40 (*d*, ³J = 4.1 Hz, 1H, C-N-C*H*), 7.50 (*t,* ³J = 7.1 Hz, 1H, C-N-CH-CH-C*H*), 7.34 (*d*, ³J = 6.7 Hz, 1H, C-C*H*-CH), 7.02 (*t*, ³J = 7.1 Hz, 1H, C-N-CH-C*H*-CH), 4.61 (*s*, 2H, S-C*H₂*-C), 3.89 (*q*, ³J = 7.1 Hz, 2H, CH₃-C*H₂*), 3.62 (*q*, ³J = 7.1 Hz, 2H, CH₃-C*H₂*), 1.18-1.08 (*m*, 6H, CH₂-C*H₃*, CH₂-C*H₃*). | | | |
| **¹³C-NMR:** | (CDCl₃, 100 MHz), δ [ppm] = 194.6 (C=S), 156.9 (CH₂-C-N-CH), 149.3 (C-N-CH), 136.7 (C-CH-CH-CH), 123.8 (C-CH-CH-CH), 122.3 (CH₂-C-N-CH-CH), 49.8 (CH₂-CH₃), 46.8 (CH₂-CH₃), 43.4 (S-CH₂-C), 12.6 (CH₂-CH₃), 11.6 (CH₂-CH₃). | | | |
| **IR (ATR):** | v (cm⁻¹) = 2974 (w), 1590 (w), 1485 (m), 1415 (vs), 1377 (w), 1354 (m), 1300 (w), 1267 (s), 1200 (s), 1141 (m), 1068 (m), 982 (s), 916 (s), 589 (m), 402 (m). | | | |
| **MS** (ESI+): | m/z (%) = 241 (100, [M+H]⁺) | | | |
| **HRMS** (ESI+): | m/z für C₁₁H₁₆N₂S₂ | | | |
| | ber.: | 240.0755 | | gef.: 240.0735 |
| **EA:** | ber.: | C: 54.96 | H: 6.71 | N: 11.65 |
| | gef.: | C: 54.62 | H: 6.79 | N: 11.60 |

### Darstellung von 3-Pyridylmethyl-N,N-diethylcarbamodithioat (Schl32125):

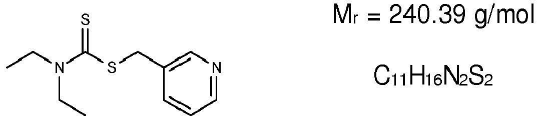

Gemäß **AAV7** wurden 0.62 mL Diethylamin (6.0 mmol, 2.0 eq), 2289 mg Kaliumphosphat (10.5 mmol, 3.5 eq), 0.36 mL Kohlenstoffdisulfid (6.0 mmol, 2.0 eq) und 759 mg 3-(Brommethyl)pyridinhydrobromid (3.0 mmol, 1.0 eq) umgesetzt. Säulenchromatographie an Kieselgel (Cyclohexan/ EtOAc 1:1) ergab 647 mg von **Schl32125** (89.7%) in Form eines gelbbraunen Öls.

| | | | | |
|---|---|---|---|---|
| **DC:** | R_{f}= 0.24 (Cyclohexan/ EtOAc 1:1). | | | |
| **¹H-NMR:** | (CDCl₃, 400 MHz), δ [ppm] = 8.50 (*s*, 1H, C-C*H*-N), 8.36 (*d*, ³J = 5.9 Hz, 1H, C-CH-N-C*H*), 7.61 (*d*, ³J = 7.9 Hz, 1H, C-C*H*-CH-CH-N), 7.11 (*t*, ³J = 7.7 Hz, 1H, C-CH-C*H*-CH-N), 4.45 (*s*, 2H, S-C*H₂*-C), 3.90 (*q*, ³J = 7.1 Hz, 2H, CH₃-C*H₂),* 3.60 (*q*, ³J = 7.1 Hz, 2H, CH₃-C*H₂*)*,* 1.15 (*t*, ³J = 7.1 Hz, 6H, CH₂-C*H₃*, CH₂-C*H₃*). | | | |
| **¹³C-NMR:** | (CDCl₃, 100 MHz), δ [ppm] = 194.1 (C=S), 150.3 (C-CH-N), 148.5 (C-CH-CH-CH-N), 136.9 (C-CH-CH-CH-N), 132.8 (S-CH₂-C), 123.4 (C-CH-CH-CH-N), 49.8 (CH₂-CH₃), 46.9 (CH₂-CH₃), 38.7 (S-CH₂-C), 12.6 (CH₂-CH₃), 11.6 (CH₂-CH₃). | | | |
| **IR (ATR):** | v (cm⁻¹) = 2975 (w), 1574 (w), 1486 (s), 1416 (vs), 1353 (m), 1300 (m), 1268 (vs), 1202 (vs), 1141 (s), 1068 (m), 981 (s), 916 (s), 824 (m), 709 (vs), 628 (m), 562 (w). | | | |
| **MS** (ESI+): | m/z (%) = 241 (100, [M+H]⁺) | | | |
| **HRMS** (ESI+): | m/z für C₁₁H₁₆N₂S₂ | | | |
| | ber.: | 240.0755 | | gef.: 240.0744 |
| **EA:** | ber.: | C:54.96 | H: 6.71 | N:11.65 |
| | gef.: | C:54.73 | H: 6.77 | N:11.38 |

### Darstellung von 4-Pyridylmethyl-N,N-diethylcarbamodithioat (Schl32126):

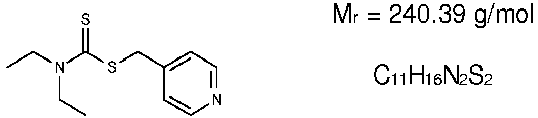

Gemäß **AAV7** wurden 0.62 mL Diethylamin (6.0 mmol, 2.0 eq), 2289 mg Kaliumphosphat (10.5 mmol, 3.5 eq), 0.36 mL Kohlenstoffdisulfid (6.0 mmol, 2.0 eq) und 759 mg 4-(Brommethyl)pyridinhydrobromid (3.0 mmol, 1.0 eq) umgesetzt. Säulenchromatographie an Kieselgel (Cyclohexan/ EtOAc 1:1) ergab 705 mg von **Schl32126** (97.8%) in Form eines gelbbraunen Öls.

| | | | | |
|---|---|---|---|---|
| **DC:** | R_{f} = 0.31 (Cyclohexan/ EtOAc 1:1). | | | |
| **¹H-NMR:** | (CDCl₃, 400 MHz), δ [ppm] = 7.78 (*d*, ³J = 7.1 Hz, 2H, C-CH-C*H*-N, C-CH-C*H*-N), 7.40 (*d*, ³J = 6.8 Hz, 2H, C-C*H*-CH-N, C-C*H*-CH-N), 4.51 (s, 2H, S-C*H₂*-C), 3.99 (q, ³J = 6.9 Hz, 2H, CH₃-C*H₂*), 3.70 (*q*, ³J = 7.1 Hz, 2H, CH₃-C*H₂*), 1.28-1.20 (*m*, 6H, CH₂-C*H₃*, CH₂-C*H₃*). | | | |
| **¹³C-NMR:** | (CDCl₃, 100 MHz), δ [ppm] = 194.0 (C=S), 149.2 (C-CH-CH-N), 146.2 (C-CH-CH-N, C-CH-CH-N), 124.2 (C-CH-CH-N, C-CH-CH-N), 50.1 (CH₂-CH₃), 47.0 (CH₂-CH₃), 40.3 (S-CH₂-C), 12.7 (CH₂-CH₃), 11.6 (CH₂-CH₃). | | | |
| **IR (ATR):** | v (cm⁻¹) = 2975 (w), 1597 (m), 1487 (s), 1412 (vs), 1353 (m), 1268 (s), 1204 (s), 1141 (m), 1067 (m), 981 (s), 916 (m), 869 (w), 829 (m), 705 (w), 569 (m), 475 (m), 430 (w). | | | |
| **MS** (ESI+): | m/z (%) = 241 (100, [M+H]⁺) | | | |
| **HRMS** (ESI+): | m/z für C₁₁H₁₆N₂S₂ | | | |
| | ber.: | 240.0755 | | gef.: 240.0738 |
| **EA:** | ber.: | C: 54.96 | H: 6.71 | N: 11.65 |
| | gef.: | C: 54.58 | H: 6.81 | N: 11.68 |

### Darstellung von Phenyl-N,N-diethylcarbamothioat (Schl32130):

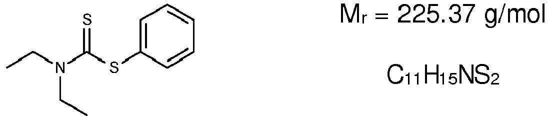

0.31 mL Diethylamin (3.0 mmol, 1.0 eq) und 0.26 mL Triethylamin (6.0 mmol, 2.0 eq) wurden in 20 mL Dichlormethan gelöst, auf 0 °C gekühlt, 30 Minuten gerührt und anschließend wurde langsam 0.47 mL Phenylchlormethandithioat (3.6 mmol, 1.2 eq) zugetropft. Das Reaktionsgemisch wurde auf Raumtemperatur erwärmt und 10 Stunden gerührt. Anschließend wurde das Lösungsmittel am Rotavapor entfernt. Säulenchromatographie an Kieselgel (Cyclohexan/ EtOAc 6:1) ergab 605 mg von **Schl32130** (88.6%) in Form eines blassgelben Öls.

| | | | | |
|---|---|---|---|---|
| **DC:** | R_{f}= 0.33 (Cyclohexan/ EtOAc 6:1). | | | |
| **¹H-NMR:** | (CDCl₃, 400 MHz), δ [ppm] = 7.55-7.37 (*m*, 5H, CH₂-C-C*H-*CH, C-C*H*-CH, C-CH-C*H*, C-CH-C*H*, C-CH-CH-C*H*), 4.03 (*q*, ³J = 6.9 Hz, 2H, CH₃-C*H₂*), 3.85 (*q*, ³J = 6.9 Hz, 2H, CH₃-C*H₂*), 1.40 (*t*, ³J = 7.1 Hz, 3H, C*H₃*-CH₂), 1.29 (*t,* ³J = 7.0 Hz, 3H, C*H₃*-CH₂). | | | |
| **¹³C-NMR:** | (CDCl₃, 100 MHz), δ [ppm] = 196.0 (C=S), 137.3 (C-CH-CH), 130.1 (C-CH-CH, C-CH-CH), 129.2 (C-CH-CH, C-CH-CH), 129.1 (C-CH-CH-CH), 50.0 (CH₂-CH₃), 47.4 (CH₂-CH₃), 12.9 (CH₂-CH₃), 11.8 (CH₂-CH₃). | | | |
| **IR (ATR):** | v (cm⁻¹) = 2974 (w), 2931 (w), 1665 (w), 1484 (m), 1456 (w), 1439 (m), 1413 (vs), 1377 (m), 1353 (s), 1299 (m), 1266 (vs), 1203 (vs), 1141 (s), 1090 (w), 1066 (s), 1007 (m), 976 (s), 916 (s), 825 (m), 773 (w), 742 (vs), 708 (m), 685 (vs), 614 (w), 590 (w), 562 (w), 504 (m), 443 (w), 420 (w). | | | |
| **MS** (ESI+): | m/z (%) = 226 (100, [M+H]⁺) | | | |
| **HRMS** (ESI+): | m/z für C₁₁H₁₅NS₂ | | | |
| | ber.: | 225.0646 | | gef.: 225.0635 |
| **EA:** | ber.: | C: 58.62 | H: 6.71 | N: 6.21 |
| | gef.: | C: 58.79 | H: 6.71 | N: 6.13 |

### Darstellung von 2-(1,3-Dithian-2-yl)ethyl-N,N-diethylcarbamodithioat (Schl32132):

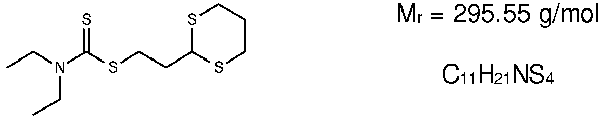

790 mg 2-(1,3-Dioxan-2-yl)ethyl-*N*,*N-*diethylcarbamodithioat (**Schl32025**, 3.0 mmol, 1.0 eq) und 0.33 mL Propandithiol (3.3 mmol, 1.1 eq) wurden in 25 mL Chloroform gelöst, mit 130 mg Iod (1.0 mmol, 0.33 eq) versetzt und 24 Stunden bei Raumtemperatur gerührt. Die Reaktion wurde mit je 25 mL 0.1M Natriumthiosulfatlösung und 1M NaOH-Lösung abgebrochen, mit Chloroform extrahiert (5 x 50 mL), die vereinigten organischen Phasen über MgSO₄ getrocknet, filtriert und das Lösungsmittel am Rotavapor entfernt. Säulenchromatographie an Kieselgel (EtOAc) ergab 574 mg von **Schl32132** (64.7%) in Form eines gelben Öls.

| | | | | |
|---|---|---|---|---|
| **DC:** | R_{f} = 0.56 (EtOAc). | | | |
| **¹H-NMR:** | (CDCl₃, 400 MHz), δ [ppm] = 4.56 (*t*, ³J=5.2 Hz, 1H, CH₂-C*H-*S), 4.17-4.09 (*m*, 2H, CH-S-C*H*H-CH₂, CH-S-C*H*H-CH₂), 4.03-3.88 (*m*, 2H, CH-S-C*H*H-CH₂, CH-S-C*H*H-CH₂), 3.74-3.62 (*m*, 4H, C*H₂-*CH3, C*H₂-*CH3), 3.36-3.30 (*m*, 2H, S-C*H₂*-CH₂), 2.89-2.70 (*m*, 2H, S-CH₂-C*H₂*), 2.11-1.81 (*m*, 2H, S-CH₂-C*H*H-CH₂-S, S-CH₂-C*H*H-CH₂-S), 1.25-1.15 (*m*, 6H, CH₂-C*H₃*, CH₂-C*H₃*). | | | |
| **¹³C-NMR:** | (CDCl₃, 100 MHz), δ [ppm] = 195.4 (C=S), 101.0 (CH₂-CH-S), 66.9 (S-CH₂-CH₂-CH₂-S), 49.4 (CH₂-CH₃), 46.7 (CH₂-CH₃), 34.3 (S-CH₂-CH₂-CH), 31.7 (S-CH₂-CH₂-CH), 25.8 (S-CH₂-CH₂-CH₂-S), 12.5 (CH₂-CH₃), 11.7 (CH₂-CH₃). | | | |
| **IR (ATR):** | v (cm⁻¹) = 2971 (w), 1646 (m), 1486 (m), 1414 (s), 1377 (m), 1355 (m), 1267 (s), 1205 (s), 1129 (vs), 1075 (s), 1032 (m), 1002 (s), 917 (m), 832 (m), 777 (w), 663 (w). | | | |
| **MS** (ESI+): | m/z (%) = 296 (100, [M+H]⁺) | | | |
| **HRMS** (ESI+): | m/z für C₁₁H₂₁NS₄ | | | |
| | ber.: | 295.0557 | | gef.: 295.0566 |
| **EA:** | ber.: | C: 44.70 | H: 7.16 | N: 4.74 |
| | gef.: | C: 44.96 | H: 7.24 | N: 4.51 |

### Darstellung von 2-[4-(Diethylcarbamothioylsulfanylmethyl)phenyl]essigsäure (Schl32136):

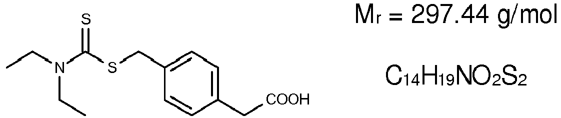

500 mg 4-(Brommethyl)phenylessigsäure (2.18 mmol, 1.0 eq) und 0.54 mL Kohlenstoffdisulfid (8.73 mmol, 4.0 eq) wurden in 15 mL Acetonitril gelöst, auf 0 °C gekühlt, 5 Minuten gerührt und anschließend langsam 0.90 mL Diethylamin (8.73 mmol, 4.0 eq) versetzt. Nach 30 Minuten Reaktionszeit wurde das Reaktionsgemisch auf Raumtemperatur erwärmt, weitere 18 Stunden gerührt, mit EtOAc extrahiert (5 x 50 mL), die vereinigten organischen Phasen über MgSO₄ getrocknet, filtriert und das Lösungsmittel am Rotavapor entfernt. Säulenchromatographie an Kieselgel (DCM/MeOH/HCOOH 95:5:0.1) ergab 625 mg von **Schl32136** (96.7%) in Form von farblosen Kristallen.

| | | | | |
|---|---|---|---|---|
| **DC:** | R_{f} = 0.37 (DCM/ MeOH/ HCOOH 95:5:0.1). | | | |
| **Fp.:** | 94 °C | | | |
| **¹H-NMR:** | (DMSO-D₆, 400 MHz), δ [ppm] = 12.28 (*sbr*, 1 H, COO*H*), 7.29 (*d*, ³J = 8.0 Hz, 2H, C*H*-C-CH₂-COOH, C*H*-C-CH₂-COOH), 7.16 (*d*, ³J = 8.0 Hz, 2H, S-CH₂-C-C*H*, S-CH₂-C-C*H*-CH), 4.44 (*s*, 2H, S-C*H₂*-C), 3.94 (*q*, ³J = 7.1 Hz, 2H, CH₃-C*H₂),* 3.69 (*q*, ³J = 7.1 Hz, 2H, CH₃-C*H₂),* 3.50 (*s*, 2H, S-CH₂-C-CH-CH-C-C*H₂*-COOH), 1.16 (*t*, ³J = 6.9 Hz, 6H, CH₂-C*H₃*, CH₂-C*H₃*). | | | |
| **¹³C-NMR:** | (DMSO-D₆, 100 MHz), δ [ppm] = 194.1 (C=S), 173.1 (COOH), 135.1 (C-CH-CH-C-CH₂-COOH), 134.7 (C-CH-CH-C-CH₂-COOH), 130.0 (S-CH₂-C-CH-CH-C-CH₂-COOH, S-CH₂-C-CH-CH-C-CH₂-COOH), 129.6 (C-CH-CH-C-CH₂-COOH, C-CH-CH-C-CH₂-COOH), 49.6 (CH₂-CH₃), 47.0 (CH₂-CH₃), 44.3 (S-CH₂-C-CH-CH-C-CH₂-COOH), 39.9 (S-CH₂-C-CH-CH-C-CH₂-COOH), 12.9 (CH₂-CH₃), 11.9 (CH₂-CH₃). | | | |
| **IR (ATR):** | v (cm⁻¹) = 2932 (w), 1700 (s), 1484 (m), 1414 (vs), 1335 (m), 1267 (s), 1243 (s), 1196 (s), 1141 (m), 1085 (m), 981 (m), 915 (s), 822 (m), 726 (w), 674 (s). | | | |
| **MS** (ESI+): | m/z (%) = 298 (100, [M+H]⁺), 320 (30, [M+Na]⁺) | | | |
| **HRMS** (ESI+): | m/z für C₁₄H₁₉NO₂S₂ | | | |
| | ber.: | 297.0857 | | gef.: 297.0859 |
| **EA:** | ber.: | C: 56.53 | H: 6.44 | N: 4.71 |
| | gef.: | C: 56.62 | H: 6.61 | N: 4.66 |

### Darstellung von 2-(1,3-Dithiolan-2-yl)ethyl-N,N-diethylcarbamodithioat (Schl32138):

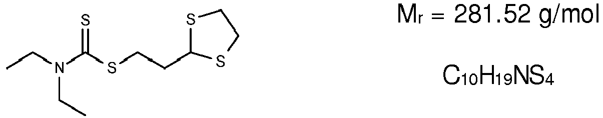

468 mg 2-(1,3-Dioxan-2-yl)ethyl-*N*,*N*-diethylcarbamodithioat (**Schl32116**, 1.78 mmol, 1.0 eq) und 0.16 mL Propandithiol (1.96 mmol, 1.1 eq) wurden in 25 mL Chloroform gelöst, mit 130 mg Iod (1.0 mmol, 0.33 eq) und 24 Stunden bei Raumtemperatur gerührt. Die Reaktion wurde anschließend mit je 25 mL 0.1M Natriumthiosulfatlösung und 1M NaOH-Lösung abgebrochen, mit Chloroform extrahiert (5 x 50 mL), die vereinigten organischen Phasen über MgSO₄ getrocknet, filtriert und das Lösungsmittel am Rotavapor entfernt. Säulenchromatographie an Kieselgel (Cyclohexan/EtOAc 5:1) ergab 232 mg von **Schl32138** (46.3%) in Form eines gelben Öls.

| | | | | |
|---|---|---|---|---|
| **DC:** | R_{f} = 0.40 (Cyclohexan/ EtOAc 5:1). | | | |
| **¹H-NMR:** | (CDCl₃, 400 MHz), δ [ppm] = 4.59 (*t*, ³J = 7.1 Hz, 1H, S-CH₂-CH₂-C*H*-S), 4.01 (*q*, ³J = 6.9 Hz, CH₃-C*H₂*), 3.72 (*q*, ³J = 7.1 Hz, CH₃-C*H₂*), 3.41 (*t*, ³J = 7.3 Hz, 2H, S-C*H₂*-CH₂-CH-O), 3.26-3.16 (*m*, 4H, S-C*H₂*-C*H₂*-S), 2.19 (*q*, ³J = 7.1 Hz, 2H, S-CH₂-C*H₂*-CH-S), 1.32-1.19 (*m*, 6H, CH₂-C*H₃*, CH₂-C*H₃*). | | | |
| **¹³C-NMR:** | (CDCl₃, 100 MHz), δ [ppm] = 195.1 (C=S), 52.5 (S-CH₂-CH₂-CH-S), 49.6 (CH₂-CH₃), 46.8 (CH₂-CH₃), 38.6 (S-CH₂-CH₂-CH), 38.5 (S-CH₂-CH₂-S), 35.7 (S-CH₂-CH₂-CH), 12.6 (CH₂-CH₃), 11.7 (CH₂-CH₃). | | | |
| **IR (ATR):** | v (cm⁻¹) = 2972 (w), 2927 (w), 1485 (s), 1457 (w), 1439 (m), 1414 (vs), 1377 (m), 1353 (m), 1300 (m), 1267 (vs), 1204 (s), 1140 (m), 1092 (m), 1068 (m), 1008 (m), 982 (m), 916 (m), 851 (m), 831 (m), 774 (w), 742 (w), 680 (w), 592 (w), 563 (w), 497 (w), 465 (w), 429 (w). | | | |
| **MS** (ESI+): | m/z (%) = 282 (100, [M+H]⁺) | | | |
| **HRMS** (ESI+): | m/z für C₁₀H₁₉NS₄ | | | |
| | ber.: | 281.0400 | | gef.: 281.0390 |
| **EA:** | ber.: | C:42.66 | H: 6.80 | N: 4.98 |
| | gef.: | C:42.67 | H: 6.84 | N: 4.96 |

### Darstellung von Methyl-4-(diethylcarbamothioylsulfanylmethyl)benzoat (Schl32150):

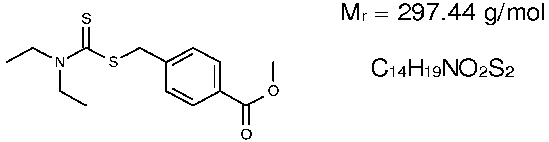

Gemäß **AAV5** wurden 1215 mg 2-Brom-4-tolylsäuremethylester (5.0 mmol, 1.0 eq), 0.60 mL Kohlenstoffdisulfid (10.0 mmol, 2.0 eq) und 1.04 mL Diethylamin (10.0 mmol, 2.0 eq) umgesetzt. Säulenchromatographie an Kieselgel (Cyclohexan/ EtOAc 5:1) ergab 1385 mg von **Schl32150** (93.1 %) in Form eines farblosen Feststoffes.

| | | | | |
|---|---|---|---|---|
| **DC:** | R_{f} = 0.24 (Cyclohexan/ EtOAc 5:1). | | | |
| **Fp.:** | 65°C | | | |
| **¹H-NMR:** | (CDCl₃, 400 MHz), δ [ppm] = 7.96 (*d*, ³J = 8.2 Hz, 2H, CH₂-C-CH-C*H*-C-COOMe, CH₂-C-CH-C*H*-C-COOMe), 7.45 (*d*, ³J = 8.5 Hz, 2H, CH₂-C-C*H*-CH-C-COOMe, CH₂-C-C*H*-CH-C-COOMe), 4.60 (*s*, 2H, S-C*H₂*-C), 4.02 (*q*, ³J = 6.9 Hz, 2H, CH₃-C*H₂*), 3.89 (*s*, 3H C(=O)-O-C*H₃*), 3.72 (*q*, ³J = 7.1 Hz, 2H, CH₃-C*H₂*), 1.27 (*t*, ³J = 7.1 Hz, 6H, CH₂-C*H₃*, CH₂-C*H₃*). | | | |
| **¹³C-NMR:** | (CDCl₃, 100 MHz), δ [ppm] = 193.9 (C=S), 166.9 (C=O), 142.0 (CH₂-C-CH-CH), 129.9 (CH-C-COOMe, CH-C-COOMe) 129.4 (CH-CH-C-COOMe, CH-CH-C-COOMe), 129.2 (C-COOMe), 52.2 (C(=O)-O-CH₃), 49.8 (CH₂-CH₃), 46.9 (CH₂-CH₃), 41.6 (S-CH₂-C), 12.6 (CH₂-CH₃), 11.7 (CH₂-CH₃). | | | |
| **IR (ATR):** | v (cm⁻¹) = 2982 (w), 1712 (s), 1490 (w), 1415 (m), 1353 (w), 1280 (s), 1205 (m), 1146 (w), 1109 (m), 986 (m), 916 (m), 863 (m), 836 (w), 783 (w), 732 (s), 696 (w), 507 (w), 438 (w). | | | |
| **MS** (ESI+): | m/z (%) = 298 (100, [M+H]⁺) | | | |
| **HRMS** (ESI+): | m/z für C₁₄H₁₉NO₂S₂ | | | |
| | ber.: | 297.0857 | | gef.: 297.0864 |
| **EA:** | ber.: | C: 56.53 | H: 6.44 | N: 4.71 |
| | gef.: | C: 56.36 | H: 6.48 | N: 4.99 |

### Darstellung von 4-(Diethylcarbamothioylsulfanylmethyl)benzoesäure (Schl32153):

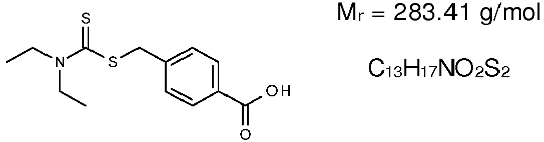

700 mg Methyl-4-(diethylcarbamothioylsulfanylmethyl)benzoat (**Schl32150**, 2.35 mmol, 1.0 eq) wurden in 10 mL Methanol gelöst, auf 40 °C erwärmt, mit 5 mL 1M NaOH-Lösung versetzt und 4 Stunden gerührt. Anschließend wurde das Reaktionsgemisch mit 200 mL Dichlormethan verdünnt und fünfmal gegen je 75 mL 1M NaOH-Lösung ausgeschüttelt. Die vereinigten wässrigen Phasen wurden mit 6M HCl auf pH=1 eingestellt. Der entstehende Niederschlag wurde abgesaugt, mit Wasser gewaschen und getrocknet. Es wurden 599 mg von **Schl32153** (70.5%) als farbloser Feststoff erhalten.

| | | | | |
|---|---|---|---|---|
| **Fp.:** | 198 °C | | | |
| **¹H-NMR:** | (DMSO-D₆, 400 MHz), δ [ppm] = 12.85 (*s*, 1 H, COO*H*), 7.84 (*d*, ³J = 7.8 Hz, 2H, CH₂-C-CH-C*H*-C-COOH, CH₂-C-CH-C*H*-C-COOH), 7.46 (*d*, ³J = 7.8 Hz, 2H, CH₂-C-C*H*-CH-C-COOH, CH₂-C-C*H*-CH-C-COOH), 4.57 (*s*, 2H, S-C*H₂*-C), 3.93 (*q*, ³J = 6.4 Hz, 2H, CH₃-C*H₂),* 3.70 (*q*, ³J = 6.4 Hz, 2H, CH₃-C*H₂),* 1.22-1.11 (*m*, 6H, CH₂-C*H₃*, CH₂-C*H₃*). | | | |
| **¹³C-NMR:** | (DMSO-D₆, 100 MHz), δ [ppm] = 193.6 (C=S), 167.6 (C=O), 142.6 (CH₂-C-CH-CH), 130.2 (C-COOH), 123.0 (CH-C-COOH, CH-C-COOH), 129.8 (CH-CH-C-COOH, CH-CH-C-COOH), 49.9 (CH₂-CH₃), 47.1 (CH₂-CH₃), 39.9 (S-CH₂-C), 13.0 (CH₂-CH₃), 11.9 (CH₂-CH₃). | | | |
| **IR (ATR):** | v (cm⁻¹) = 2974 (w), 1683 (s), 1608 (w), 1490 (m), 1416 (s), 1269 (s), 1208 (m), 1146 (w), 1118 (w), 1072 (w), 1006 (m), 917 (m), 864 (m), 789 (w), 737 (s), 541 (m), 507 (w), 441 (w). | | | |
| **MS** (ESI+): | m/z (%) = 284 (100, [M+H]⁺) | | | |
| **HRMS** (ESI+): | m/z für C₁₃H₁₇NO₂S₂ | | | |
| | ber.: | 283.0701 | | gef.: 283.0688 |
| **EA:** | ber.: | C: 55.09 | H: 6.05 | N: 4.94 |
| | gef.: | C: 54.67 | H: 6.04 | N: 4.96 |

### Darstellung von Phenacyl-N,N-diethylcarbamodithioat (Schl32154):

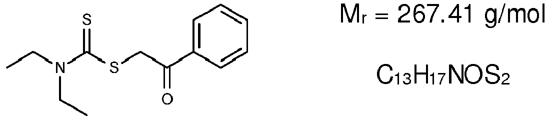

Gemäß **AAV5** wurden 597 mg 2-Bromacetophenon (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 6:1) ergab 756 mg von **Schl32154** (95.7%) in Form eines farblosen Feststoffes.

| | | | | |
|---|---|---|---|---|
| **DC:** | R_{f} = 0.24 (Cyclohexan/ EtOAc 6:1). | | | |
| **Fp.:** | 105 °C | | | |
| **¹H-NMR:** | (CDCl₃, 400 MHz), δ [ppm] = 8.06 (*d*, ³J = 7.3 Hz, 2H, C(=O)-C-C*H*-CH-CH, C(=O)-C-C*H*-CH-CH), 7.61-7.55 (*m*, 1H, C(=O)-C-C*H*-CH-C*H*), 7.48 (*t*, ³J = 7.5 Hz, 2H, C(=O)-C-CH-C*H*-CH, C(=O)-C-CH-C*H*-CH), 4.90 (*s*, 2H, S-C*H₂*-C(=O)), 4.00 (*q*, ³J = 6.9 Hz, 2H, CH₃-C*H₂*), 3.82 (*q*, ³J = 6.9 Hz, 2H, CH₃-C*H₂*), 1.33 (*t*, ³J = 7.1 Hz, 3H, CH₂-C*H₃*)*,* 1.25 (*t*, ³J = 7.1 Hz, 3H, CH₂-C*H₃*). | | | |
| **¹³C-NMR:** | (CDCl₃, 100 MHz), δ [ppm] = 194.0 (C=S), 193.2 (C=O), 136.2 (C(=O)-C-CH-CH-CH), 133.6 (C(=O)-C-CH-CH-CH), 128.8 (C(=O)-C-CH, C(=O)-C-CH), 128.6 (C(=O)-C-CH-CH-CH, C(=O)-C-CH-CH-CH), 50.2 (CH₂-CH₃), 47.2 (CH₂-CH₃), 45.2 (S-CH₂-C(=O)), 12.7 (CH₂-CH₃), 11.6 (CH₂-CH₃). | | | |
| **IR (ATR):** | v (cm⁻¹) = 2979 (w), 1684 (s), 1594 (w), 1492 (s), 1444 (m), 1421 (s), 1357 (m), 1296 (w), 1271 (s), 1203 (vs), 1141 (m), 1064 (m), 996 (s), 980 (s), 916 (m), 833 (m), 773 (w), 751 (vs), 689 (s), 647 (s), 562 (m), 496 (w), 428 (m). | | | |
| **MS** (ESI+): | m/z (%) = 268 (100, [M+H]⁺) | | | |
| **HRMS** (ESI+): | m/z für C₁₃H₁₇NOS₂ | | | |
| | ber.: | 267.0752 | | gef.: 267.0763 |
| **EA:** | ber.: | C: 58.39 | H: 6.41 | N: 5.24 |
| | gef.: | C: 58.29 | H: 6.47 | N: 5.07 |

### Darstellung von (4-Cyanophenyl)methyl-N,N-diethylcarbamodithioat (Schl32155):

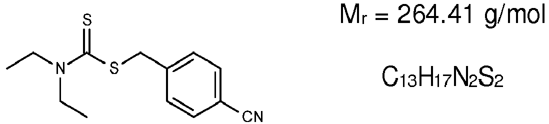

Gemäß **AAV5** wurden 1764 mg 4-(Brommethyl)benzonitril (9.0 mmol, 1.0 eq), 1.08 mL Kohlenstoffdisulfid (19.0 mmol, 2.0 eq) und 1.86 mL Diethylamin (18.0 mmol, 2.0 eq) umgesetzt. Säulenchromatographie an Kieselgel (Cyclohe-xan/ EtOAc 6:1) ergab 2177 mg von **Schl32155** (91.5%) in Form eines farblosen Feststoffes.

| | | | | |
|---|---|---|---|---|
| **DC:** | R_{f} = 0.17 (Cyclohexan/ EtOAc 6:1). | | | |
| **Fp.:** | 65 °C | | | |
| **¹H-NMR:** | (CDCl₃, 400 MHz), δ [ppm] = 7.56 (*d*, ³J = 8.2 Hz, 2H, C-CH-C*H*-C-CN, C-CH-C*H*-C-CN), 7.49 (*d*, ³J = 8.2 Hz, 2H, C-C*H*-CH-C-CN, C-C*H*-CH-C-CN), 4.61 (*s*, 2H, S-C*H₂*-C), 4.02 (*q*, ³J = 7.1 Hz, 2H, CH₃-C*H₂*), 3.72 (*q*, ³J = 7.1 Hz,2H, CH₃-C*H₂*), 1.27 (*t*, ³J = 7.1 Hz, 6H, CH₂-C*H₃*, CH₂-C*H₃*). | | | |
| **¹³C-NMR:** | (CDCl₃, 100 MHz), δ [ppm] = 194.1 (C=S), 142.8 (C-CH-CH-C-CN), 132.4 (C-CH-CH-C-CN, C-CH-CH-C-CN) 130.1 (C-CH-CH-C-CN, C-CH-CH-C-CN), 118.9 (C-CN), 111.1 (C-CN), 50.1 (CH₂-CH₃), 47.0 (CH₂-CH₃), 41.1 (S-CH₂-C), 12.7 (CH₂-CH₃), 11.6 (CH₂-CH₃). | | | |
| **IR (ATR):** | v (cm⁻¹) = 2971 (w), 2228 (w), 1605 (w), 1499 (m), 1459 (w), 1419 (m), 1353 (w), 1298 (w), 1273 (m), 1204 (m), 1148 (w), 1069 (m), 1005 (m), 915 (w), 891 (w), 827 (m), 756 (w), 549 (s), 500 (w), 437 (w). | | | |
| **MS** (ESI+): | m/z (%) = 265 (100, [M+H]⁺) | | | |
| **HRMS** (ESI+): | m/z für C₁₃H₁₇N₂S₂ | | | |
| | ber.: | 264.0755 | | gef.: 264.0766 |
| **EA:** | ber.: | C: 59.05 | H: 6.10 | N: 10.59 |
| | gef.: | C: 58.97 | H: 6.10 | N: 10.68 |

### Darstellung von (4-Phenylphenyl)methyl-N,N-diethylcarbamodithioat (Schl32156):

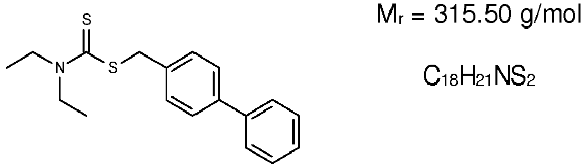

Gemäß **AAV5** wurden 741 mg 4-(Brommethyl)biphenyl (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 7:1) ergab 734 mg von **Schl32156** (77.5%) in Form eines blassgelben Feststoffes.

| | | | | |
|---|---|---|---|---|
| **DC:** | R_{f} = 0.34 (Cyclohexan/ EtOAc 7:1) | | | |
| **Fp.:** | 61 °C | | | |
| **¹H-NMR:** | (CDCl₃, 400 MHz), δ [ppm] = 7.62-7.54 (*m*, 4H, Ph-C-C*H*-C*H*-CH, Ph-C-C*H*-C*H*-CH), 7.49-7.41 (*m*, 4H, CH₂-C-C*H*-C*H*-C-Ph, CH₂-C-C*H*-C*H*-C-Ph), 7.38-7.32 (*m*, 1H, Ph-C-CH-CH-C*H*), 4.62 (*s*, 2H, S-C*H₂*-C), 4.07 (*q*, ³J = 7.1 Hz, 2H, CH₃-C*H₂*), 3.74 (*q*, ³J = 6.9 Hz, 2H, CH₃-C*H₂*), 1.34-1.25 (*m*, 6H, CH₂-C*H₃*, CH₂-C*H₃*). | | | |
| **¹³C-NMR:** | (CDCl₃, 100 MHz), δ [ppm] = 195.2 (C=S), 140.8 (CH₂-C-CH-CH-C-C-CH), 140.5 (CH₂-C-CH-CH-C-C-CH), 135.2 (C-CH-CH-C-C-CH), 130.0 (Ph-C-CH-CH-CH, Ph-C-CH-CH-CH), 128.9 (CH₂-C-CH-CH-C, CH₂-C-CH-CH-C), 127.5 (Ph-C-CH-CH-CH, Ph-C-CH-CH-CH, CH₂-C-CH-CH-C, CH₂-C-CH-CH-C), 127.2 (Ph-C-CH-CH-CH), 49.7 (CH₂-CH₃), 46.9 (CH₂-CH₃), 42.0 (S-CH₂-C), 12.6 (CH₂-CH₃), 11.8 (CH₂-CH₃). | | | |
| **IR (ATR):** | v (cm⁻¹) = 2971 (w), 1486 (s), 1453 (w), 1440 (w), 1415 (s), 1379 (w), 1352 (w), 1297 (w), 1265 (m), 1207 (m), 1142 (m), 1074 (w), 1066 (w), 1006 (m), 980 (m), 919 (m), 851 (m), 833 (m), 770 (m), 744 (vs), 695 (s), 554 (w), 499 (m). | | | |
| **MS** (ESI+): | m/z (%) = 316 (100, [M+H]⁺) | | | |
| **HRMS** (ESI+): | m/z für C₁₈H₂₁NS₂ | | | |
| | ber.: | 315.1115 | | gef.: 315.1108 |
| **EA:** | ber.: | C: 68.52 | H: 6.71 | N: 4.44 |
| | gef.: | C: 68.84 | H: 6.75 | N: 4.32 |

### Darstellung von Benzhydryl-N,N-diethylcarbamodithioat (Schl32157):

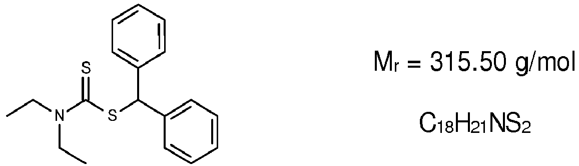

Gemäß **AAV5** wurden 741 mg Bromdiphenylmethan (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 5:1) ergab 845 mg von **Schl32157** (89.3%) in Form eines gelben Feststoffes.

| | | | | |
|---|---|---|---|---|
| **DC:** | R_{f} = 0.44 (Cyclohexan/ EtOAc 5:1). | | | |
| **Fp.:** | 76 °C | | | |
| **¹H-NMR:** | (CDCl₃, 400 MHz), δ [ppm] = 7.43-7.20 (*m*, 10H, C*H*_{(Phenyl)}), 6.61 (*s*, 1H, S-C*H*-C), 4.62 (*s*, 2H, S-C*H₂*-C), 3.99 (*q*, ³J = 6.9 Hz, 2H, CH₃-C*H₂*), 3.76 (*q*, ³J = 6.9 Hz, 2H, CH₃-C*H₂*), 1.33-1.22 (*m*, 6H, CH₂-C*H₃*, CH₂-C*H₃*). | | | |
| **¹³C-NMR:** | (CDCl₃, 100 MHz), δ [ppm] = 193.2 (C=S), 140.9 (CH-C-CH-CH-CH, CH-C-CH-CH-CH), 128.5 (C-CH-CH-CH, C-CH-CH-CH, C-CH-CH-CH, C-CH-CH-CH), 127.2 (CH-C-CH-CH-CH, CH-C-CH-CH-CH, CH-C-CH-CH-CH, CH-C-CH-CH-CH), 126.2 (C-CH-CH-CH, C-CH-CH-CH), 59.9 (C-CH-C), 49.7 (CH₂-CH₃), 46.8 (CH₂-CH₃), 12.8 (CH₂-CH₃), 11.7(CH₂-CH₃). | | | |
| **IR (ATR):** | v (cm⁻¹) = 3054 (w), 3022 (w), 2983 (w), 2931 (w), 1582 (w), 1486 (m), 1449 (m), 1416 (s), 1381 (w), 1353 (w), 1295 (w), 1266 (m), 1203 (s), 1141 (m), 1093 (w), 1074 (w), 1028 (w), 1007 (w), 985 (m), 915 (w), 855 (w), 827 (w), 747 (w), 730 (m), 696 (vs), 626 (m), 618 (m), 584 (m), 569 (w), 511 (w), 472 (w), 431 (w). | | | |
| **MS** (ESI+): | m/z (%) = 316 (100, [M+H]⁺) | | | |
| **HRMS** (ESI+): | m/z für C₁₈H₂₁NS₂ | | | |
| | ber.: | 315.1115 | | gef.: 315.1111 |
| **EA:** | ber.: | C: 68.52 | H: 6.71 | N: 4.44 |
| | gef.: | C: 68.27 | H: 6.72 | N: 4.45 |

### Darstellung von (2-Hydroxy-5-nitrophenyl)methyl-N,N-diethylcarbamodithioat (Schl32158):

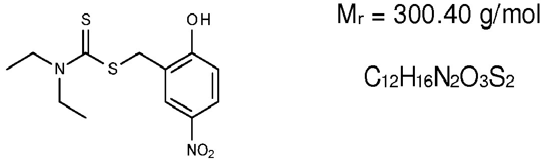

Gemäß **AAV5** wurden 696 mg 2-Hydroxy-5-nitrobenzylbromid (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 1:1) ergab 629 mg von **Schl32158** (69.8%) in Form eines gelben Öls.

| | | | | |
|---|---|---|---|---|
| **DC:** | R_{f} = 0.26 (Cyclohexan/ EtOAc 1:1). | | | |
| **¹H-NMR:** | (CDCl₃, 400 MHz), δ [ppm] = 8.84 (*sbr,* 1H, O*H*), 8.10 (*s*, 1H, C-C*H-*C(NO₂)), 8.03 (*d*, ³J = 9.0 Hz, 1H, C(NO₂)-C*H*-CH-C-OH), 6.93 (*d*, ³J = 8.9 Hz, 1H, C(NO₂)-CH-C*H*-C-OH), 4.78 (*s*, 2H, S-C*H₂-*C), 4.02 (*q*, ³J = 7.1 Hz, 2H, CH₃-C*H₂*), 3.73 (*q*, ³J = 7.1 Hz, 2H, CH₃-C*H₂*), 1.28 (*t*, ³J = 7.1 Hz, 6H, CH₂-C*H₃*, CH₂-C*H₃*). | | | |
| **¹³C-NMR:** | (CDCl₃, 100 MHz), δ [ppm] = 195.2 (C=S), 161.4 (C-OH), 140.7 (C-NO₂), 127.2 (C(NO₂)-CH-CH-C), 125.5 (C-CH-C(NO₂)), 124.1 (S-CH₂-C), 117.5 (C(NO₂)-CH-CH-C), 51.0 (CH₂-CH₃), 47.5 (CH₂-CH₃), 38.0 (S-CH₂-C), 14.2 (CH₂-CH₃), 11.6(CH₂-CH₃). | | | |
| **IR (ATR):** | v (cm⁻¹) = 2977 (w), 1699 (w), 1583 (w), 1488 (s), 1418 (s), 1333 (vs), 1268 (vs), 1142 (s), 1075 (s), 981 (m), 911 (m), 831 (m), 748 (m), 639 (m), 430 (m). | | | |
| **MS** (ESI+): | m/z (%) = 301 (100, [M+H]⁺) | | | |
| **HRMS** (ESI+): | m/z für C₁₂H₁₆N₂O₃S₂ | | | |
| | ber.: | 300.0602 | | gef.: 300.0612 |
| **EA:** | ber.: | C: 47.98 | H: 5.37 | N: 9.33 |
| | gef.: | C: 47.59 | H: 5.61 | N: 8.96 |

### Darstellung von [4-(Trifluormethoxy)phenyl]methyl-N,N-diethylcarbamodithioat (Schl32160):

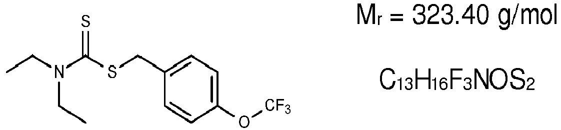

Gemäß **AAV5** wurden 500 mg 4-(Trifluormethoxy)benzylbromid (1.96 mmol, 1.0 eq), 0.24 mL Kohlenstoffdisulfid (3.92 mmol, 2.0 eq) und 0.41 mL Diethylamin (3.92 mmol, 2.0 eq) umgesetzt. Säulenchromatographie an Kieselgel (Cyclohexan/ EtOAc 5:1) ergab 557 mg von **Schl32160** (87.9%) in Form eines blassgelben Öls.

| | | | | |
|---|---|---|---|---|
| **DC:** | R_{f} = 0.38 (Cyclohexan/ EtOAc 5:1). | | | |
| **¹H-NMR:** | (CDCl₃, 400 MHz), δ [ppm] = 7.39 (*d*, ³J = 8.7 Hz, 2H, C-CH-C*H*-C-OCF₃, C-CH-C*H*-C-OCF₃), 7.11 (*d*, ³J = 8.0 Hz, 2H, C-C*H*-CH-C-OCF₃, C-C*H*-CH-C-OCF₃), 4.54 (*s*, 2H, S-C*H₂-*C), 4.01 (*q*, ³J = 7.1 Hz, 2H, CH₃-C*H₂*), 3.69 (*q*, ³J = 7.1 Hz, 2H, CH₃-C*H₂*), 1.24 (*t*, ³J = 7.0 Hz, 6H, CH₂-C*H₃*, CH₂-C*H₃*). | | | |
| **¹³C-NMR:** | (CDCl₃, 100 MHz), δ [ppm] = 194.6 (C=S), 148.4 (C-OCF₃), 135.6 (C-CH-CH-C-OCF₃), 130.8 (C-CH-CH-C-OCF₃, C-CH-CH-C-OCF₃), 121.8 (C-CH-CH-C-OCF₃, C-CH-CH-C-OCF₃), 120.5 (*q*, ¹J_{C,F} = 257.2 Hz, C-CH-CH-C-O-CF₃), 49.7 (CH₂-CH₃), 46.9 (CH₂-CH₃), 40.9 (S-CH₂-C), 12.5 (CH₂-CH₃), 11.6 (CH₂-CHs). | | | |
| **IR (ATR):** | v (cm⁻¹) = 2979 (w), 1507 (w), 1487 (m), 1459 (w), 1443 (w), 1416 (m), 1380 (w), 1355 (w), 1299 (w), 1252 (vs), 1206 (s), 1190 (vs), 1156 (vs), 1093(m), 1068 (m), 1008 (m), 982 (m), 917 (m), 867 (w), 830 (m), 777(w), 522 (w), 433 (w). | | | |
| **MS** (ESI+): | m/z (%) = 324 (100, [M+H]⁺) | | | |
| **HRMS** (ESI+): | m/z für C₁₃H₁₆F₃NOS₂ | | | |
| | ber.: | 323.0625 | gef.: 323.0625 | |
| **EA:** | ber.: | C: 48.28 | H: 4.99 | N: 4.33 |
| | gef.: | C: 48.04 | H: 5.10 | N: 4.57 |

### Darstellung von (7-Methoxy-2-oxochromen-4-yl)methyl-N,N-diethylcarbamodithioat (Schl32175):

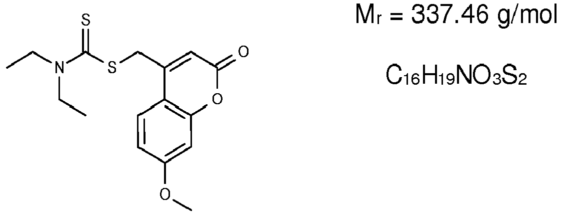

Gemäß **AAV5** wurden 404 mg 4-(Brommethyl)-7-methoxychromen-2-on (1.5 mmol, 1.0 eq), 0.18 mL Kohlenstoffdisulfid (3.0 mmol, 2.0 eq) und 0.18 mL Diethylamin (3.0 mmol, 2.0 eq) umgesetzt. Säulenchromatographie an Kieselgel (Pentan/ EtOAc 5:1) ergab 169 mg von **Schl32175** (25.1 %) in Form eines braunen Feststoffes.

| | | | | |
|---|---|---|---|---|
| **DC:** | R_{f} = 0.19 (Pentan/ EtOAc 5:1). | | | |
| **Fp.:** | 98 °C | | | |
| **¹H-NMR:** | (CDCl₃, 400 MHz), δ [ppm] = 7.63 (*d*, ³J = 8.7 Hz, 1H, C-C*H*-CH-C-O-CH₃, 6.86 (*dd*, ³J = 8.9 Hz, ³J = 8.9 Hz, 1H, C-CH-C*H*-C-O-CH₃,), 6.82-6.79 (m, 1H, C-C*H*-C-O-CH₃), 6.39 (s, 1H, C-CH-C=O), 4.68 (s, 2H, S-C*H₂-*C), 4.02 *(q,* ³J = 7.1 Hz, 2H, CH₃-C*H*₂), 3.86 (s, 3H, O-C*H₃*), 3.74 *(q,* ³J = 7.1 Hz, 2H, CH₃-C*H*₂), 1.28 (*t,* ³J = 7.2 Hz, 6H, CH₂-C*H*₃, CH₂-C*H*₃). | | | |
| **¹³C-NMR:** | (CDCl₃, 100 MHz), δ [ppm] = 192.9 (C=S), 162.9 (C(=O)-O), 161.1 (C-OMe), 155.7 (S-CH₂-C), 150.7 (C(=O)-O-C), 125.8 (C-CH-CH-C-OMe), 112.8 (C-CH-C(=O)), 112.2 (C-CH-CH-C-OMe), 101.2 (C-CH-C-OMe), 55.9 (O-CH₃), 50.4 (CH₂-CH₃), 47.1 (CH₂-CH₃), 37.7 (S-CH₂-C), 12.7 (CH₂-CH₃), 11.6 (CH₂-CH₃). | | | |
| **IR (ATR):** | v (cm⁻¹) = 3082 (w), 2967 (w), 2923 (w), 1705 (vs), 1608 (m), 1557 (vs), 1490 (m), 1454 (m), 1417 (s), 1386 (s), 1346 (m), 1283 (m), 1268 (s), 1224 (vs), 1208 (m), 1199 (m), 1146 (m), 1051 (m), 1022 (m), 986 (s), 975 (m), 907 (m), 867 (vs), 844 (vs), 824 (vs), 817 (m), 797 (m), 773 (m), 736 (m), 713 (m), 692 (m), 682 (m), 641 (m), 613 (m), 590 (m), 576 (m), 558 (m), 531 (m), 522 (m), 487 (m), 454 (s), 431 (m). | | | |
| **MS** (ESI+): | m/z (%) = 338 (100, [M+H]⁺) | | | |
| **HRMS** (ESI+): | m/z für C₁₆H₁₉NO₃S₂ | | | |
| | ber.: | 337.0806 | | gef.: 337.0811 |
| **EA:** | ber.: | C: 56.95 | H: 5.68 | N: 4.15 |
| | gef.: | C: 57.41 | H: 5.85 | N: 4.27 |

### Darstellung von Cyclohexylmethyl-N,N-diethylcarbamodithioat (Schl32178):

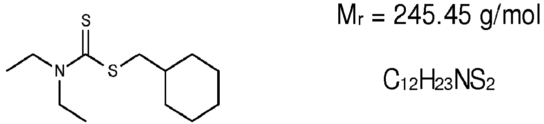

Gemäß **AAV5** wurden 0.42 mL Brommethylcyclohexan (3.0 mmol, 1.0 eq), 0.36 mL Kohlenstoffdisulfid (6.0 mmol, 2.0 eq) und 0.62 mL Diethylamin (6.0 mmol, 2.0 eq) umgesetzt. Säulenchromatographie an Kieselgel (Cyclohexan/ EtOAc 5:1) ergab 330 mg von **Schl32178** (44.8%) in Form eines gelben Öls.

| | | | | |
|---|---|---|---|---|
| **DC:** | R_{f} = 0.51 (Cyclohexan/ EtOAc 5:1). | | | |
| **¹H-NMR:** | (CDCl₃, 400 MHz), δ [ppm] = 4.01 *(q,* ³J = 7.1 Hz, 2H, CH₃-C*H₂*), 3.74 (*q*, ³J = 7.1 Hz, 2H, CH₃-C*H*₂), 3.27-3.24 (m, 1H, S-CH₂-C*H*), 3.18 (*d*, ³J = 6.9 Hz, 2H, S-C*H*₂-CH), 1.89-1.80 (m, 2H, C*H*_{(Cyclohexyl)}), 1.74-1.56 (m, 6H, C*H*_{(Cyclohexyl)}), 1.29-1.20 (m, 6H, CH₂-CH₃, CH₂-C*H₃*), 1.06-0.91 (m, 2H, C*H*_{(Cyclohexyl)}). | | | |
| **¹³C-NMR:** | (CDCl₃, 100 MHz), δ [ppm] = 196.4 (C=S), 49.5 (CH₂-CH₃), 46.7 (CH₂-CH₃), 44.5 (S-CH₂-CH), 37.3 (S-CH₂-CH), 33.0 (CH_{(Cyclohexyl)}), 31.8 (CH_{(Cyclohexyl)}), 26.4 (CH_{(Cyclohexyl)}), 26.1 (CH_{(Cyclohexyl)}), 25.9 (CH_{(Cyclohexyl)}), 12.5 (CH₂-CH₃), 11.7 (CH₂-CH₃). | | | |
| **IR (ATR):** | v (cm⁻¹) = 2974 (w), 2920 (s), 2849 (w), 1482 (s), 1445 (m), 1412 (vs), 1377 (m), 1354 (m), 1300 (m), 1264 (vs), 1206 (s), 1140 (m), 1115 (m), 1092 (m), 1068 (m), 1008 (m), 983 (s), 961 (w), 916 (m), 902 (w), 831 (m), 775 (w), 737 (w), 591 (w), 564 (w), 498 (w), 461 (w), 432 (w). | | | |
| **MS** (ESI+): | m/z (%) = 246 (100, [M+H]⁺) | | | |
| **HRMS** (ESI+): | m/z für C₁₂H₂₃NS₂ | | | |
| | ber.: | 245.1272 | | gef.: 245.1275 |
| **EA:** | ber.: | C: 58.72 | H: 9.45 | N: 5.71 |
| | gef.: | C: 58.71 | H: 9.38 | N: 5.67 |

### Darstellung von 2-(1H-lndol-3-yl)ethyl-N,N-diethylcarbamodithioat (Schl32189):

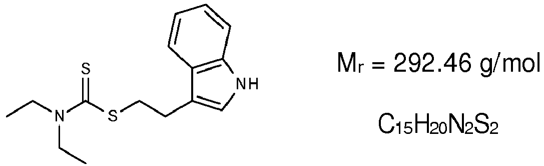

Gemäß **AAV6** wurden 0.78 mL Diethylamin (7.5 mmol, 2.1 eq), 0.45 mL Kohlenstoffdisulfid (7.5 mmol, 2.1 eq) und 785 mg 3-(2-Bromethyl)indol (3.5 mmol, 1.0 eq) umgesetzt. Säulenchromatographie an Kieselgel (Cyclohexan/ EtOAc 5:1) ergab 915 mg von **Schl32189** (89.5%) in Form eines blassgelben Feststoffes.

| | | | | |
|---|---|---|---|---|
| **DC:** | R_{f} = 0.41 (Cyclohexan/ EtOAc 6:1). | | | |
| **Fp.:** | 91 °C | | | |
| **¹H-NMR:** | (CDCl₃, 400 MHz), δ [ppm] = 8.13 (*sbr,* 1H, N*H*), 7.76 (*d,* ³J = 7.8 Hz, CH₂-C-C-C*H*), 7.34 (*d*, ³J = 8.0 Hz, N-C-C*H*), 7.23-7.11 (m, 2H, C-CH-C*H*-C*H*-CH), 7.04 (s, 1H, C-C*H*-NH), 4.06 (*q,* ³J = 7.1 Hz, 2H, CH₃-C*H*₂), 3.73 (*q*, ³J = 7.1 Hz, 2H, CH₃-C*H₂*), 3.65 (*t,* ³J = 8.0 Hz, 2H, S-C*H₂*-CH₂-C), 3.20 (*t,* ³J = 8.0 Hz, 2H, S-CH₂-C*H*₂-C), 1.33-1.23 (*m,* 6H, CH₂-CH₃, CH₂-C*H*₃). | | | |
| **¹³C-NMR:** | (CDCl₃, 100 MHz), δ [ppm] = 195.9 (C=S), 136.4 (NH-C-CH), 127.5 (NH-C-C), 122.2 (C-CH-NH), 122.1 (NH-C-CH-CH), 119.4 (NH-C-CH-CH-CH), 119.2 (NH-C-C-CH), 114.8 (NH-CH-C), 111.3 (NH-C-CH), 49.6 (CH₂-CH₃), 46.9 (CH₂-CH₃), 37.6 (S-CH₂-CH₂), 25.2 (S-CH₂-CH₂), 12.6 (CH₂-CH₃), 11.8 (CH₂-CH₃). | | | |
| **IR (ATR):** | v (cm⁻¹) = 3323 (w), 3055 (w), 2976 (w), 2931 (w), 1486 (s), 1455 (w), 1415 (vs), 1372 (m), 1353 (m), 1339 (m), 1299 (m), 1266 (vs), 1242 (s), 1203 (vs), 1129 (m), 1092 (m), 1068 (m), 1043 (m), 1007 (m), 982 (s), 915 (w), 892 (w), 832 (m), 811 (m), 779 (w), 740 (vs), 633 (w), 590 (w), 495 (m), 483 (m), 462 (m), 449 (m), 424 (s). | | | |
| **MS** (ESI+): | m/z (%) = 293 (100, [M+H]⁺) | | | |
| **HRMS** (ESI+): | m/z für C₁₅H₂₀N₂S₂ | | | |
| | ber.: | 292.1068 | | gef.: 292.1058 |
| **EA:** | ber.: | C: 61.60 | H: 6.89 | N: 9.58 |
| | gef.: | C: 61.51 | H: 6.90 | N: 9.37 |

### Darstellung von p-Tolylmethyl-N,N-diethylcarbamodithioat (Schl32229):

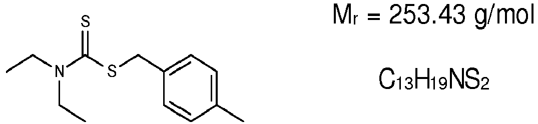

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 555 mg 1-(Brommethyl)-4-methylbenzen (3.0 mmol, 1.0 eq) umgesetzt. Säulenchromatographie an Kieselgel (Cyclohexan/ EtOAc 9:1) ergab 683 mg von **Schl32229** (89.9%) in Form eines blassgelben Öls.

| | | | | |
|---|---|---|---|---|
| **DC:** | R_{f} = 0.42 (Cyclohexan/ EtOAc 6:1) | | | |
| **¹H-NMR:** | (CDCl₃, 400 MHz), δ [ppm] = 7.28 (*d*, ³J = 7.8 Hz, 2H, C-C*H*-CH-C-CH₃, C-C*H*-CH-C-CH₃), 7.12 (*d*, ³J = 8.0 Hz, 2H, C-CH-C*H*-C-CH₃, C-CH-C*H*-C-CH₃), 4.51 (*s,* 2H, S-C*H₂*-C), 4.04 (*q,* ³J = 6.9 Hz, 2H, CH₃-C*H*₂), 3.71 (*q,* ³J = 7.1 Hz, 2H, CH₃-C*H₂*), 2.33 (s, 3H, C-C*H₃*), 1.33-1.22 *(m,* 6H, CH₂-CH₃, CH₂-C*H*₃). | | | |
| **¹³C-NMR:** | (CDCl₃, 100 MHz), δ [ppm] = 195.5 (C=S), 137.3 (C-CH₃), 132.9 (S-CH₂-C), 129.4 (C-CH-CH-C, C-CH-CH-C), 49.5 (CH₂-CH₃), 46.8 (CH₂-CH₃), 42.2 (S-CH₂-C), 21.3 (C-CH₃), 12.6 (CH₂-CH₃), 11.7 (CH₂-CH₃). | | | |
| **IR (ATR):** | v (cm⁻¹) = 2974 (w), 2931 (w), 1513 (w), 1483 (vs), 1456 (m), 1440 (m), 1413 (vs), 1377 (m), 1353 (m), 1300 (m), 1266 (vs), 1205 (vs), 1140 (s), 1091 (w), 1068 (m), 1007 (s), 982 (s), 916 (m), 860 (w), 820 (m), 775 (w), 743 (w), 725 (w), 523 (m), 478 (m), 430 (w). | | | |
| **MS** (ESI+): | m/z (%) = 254 (100, [M+H]⁺) | | | |
| **HRMS** (ESI+): | m/z für C₁₃H₁₉NS₂ | | | |
| | ber.: | 253.0959 | | gef.: 253.0969 |
| **EA:** | ber.: | C: 61.61 | H: 7.56 | N: 5.53 |
| | gef.: | C: 61.67 | H: 7.54 | N: 5.53 |

### Darstellung von m-Tolylmethyl-N,N-diethylcarbamodithioat (Schl32230):

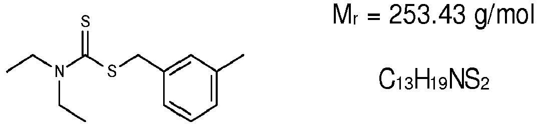

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 555 mg 1-(Brommethyl)-3-methylbenzen (3.0 mmol, 1.0 eq) umgesetzt. Säulenchromatographie an Kieselgel (Cyclohexan/ EtOAc 9:1) ergab 700 mg von **Schl32230** (92.1 %) in Form eines blassgelben Öls.

| | | | | |
|---|---|---|---|---|
| **DC:** | R_{f} = 0.51 (Cyclohexan/ EtOAc 6:1). | | | |
| **¹H-NMR:** | (CDCl₃, 400 MHz), δ [ppm] = 7.22-7.15 (*m,* 3H, CH₂-C-C*H*-C*H*-C*H*), 7.11-7.05 (CH₂-C-C*H*-C(CH₃)), 4.51 *(s,* 2H, S-C*H₂*-C), 4.05 (*q,* ³J = 6.9 Hz, 2H, CH₃-C*H*₂), 3.72 (*q,* ³J = 7.1 Hz, 2H, CH₃-C*H*₂), 2.35 *(s,* 3H, C-C*H₃*), 1.33-1.24 (*m,* 6H, CH₂-CH₃, CH₂-C*H*₃). | | | |
| **¹³C-NMR:** | (CDCl₃, 100 MHz), δ [ppm] = 195.4 (C=S), 138.4 (C-CH₃), 135.9 (CH₂-C-CH), 130.2 (C-CH-CH-CH), 128.6 (C-CH-C(CH₃)), 128.4 (C-CH-CH-CH), 126.6 (C-CH-CH-CH), 49.5 (CH₂-CH₃), 46.9 (CH₂-CH₃), 42.4 (S-CH₂-C), 21.5 (C-CH₃), 12.6 (CH₂-CH₃), 11.7 (CH₂-CH₃). | | | |
| **IR (ATR):** | v (cm⁻¹) = 2974 (w), 2931 (w), 1483 (vs), 1456 (w), 1440 (w), 1413 (vs), 1377 (m), 1353 (s), 1299 (w), 1266 (vs), 1203 (vs), 1140 (s), 1089 (w), 1066 (w), 1008 (s), 982 (s), 915 (s), 882 (w), 830 (m), 789 (m), 736 (m), 712 (m), 691 (m), 586 (w), 431 (w). | | | |
| **MS** (ESI+): | m/z (%) = 254 (100, [M+H]⁺) | | | |
| **HRMS** (ESI+): | m/z für C₁₃H₁₉NS₂ | | | |
| | ber.: | 253.0959 | | gef.: 253.0949 |
| **EA:** | ber.: | C: 61.61 | H: 7.56 | N: 5.53 |
| | gef.: | C: 61.40 | H: 7.56 | N: 5.51 |

### Darstellung von o-Tolylmethyl-N,N-diethylcarbamodithioat (Schl32231):

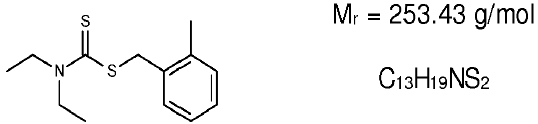

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 555 mg 1-(Brommethyl)-2-methylbenzen (3.0 mmol, 1.0 eq) umgesetzt. Säulenchromatographie an Kieselgel (Cyclohexan/ EtOAc 9:1) ergab 737 mg von **Schl32231** (96.0%) in Form eines blassgelben Öls.

| | | | | |
|---|---|---|---|---|
| **DC:** | R_{f} = 0.49 (Cyclohexan/ EtOAc 6:1). | | | |
| **¹H-NMR:** | (CDCl₃, 400 MHz), δ [ppm] = 7.39-7.33 (*m,* 1H, CH₂-C-C(CH₃)-C*H*-CH), 7.22-7.11 (*m,* 3H, CH₂-C-C*H*-C*H*-C*H*,) 4.50 *(s,* 2H, S-C*H₂*-C), 4.05 (*q*, ³J = 7.1 Hz, 2H, CH₃-C*H*₂), 3.71 (*q,* ³J = 7.1 Hz, 2H, CH₃-C*H*₂), 2.41 (*s,* 3H, C-C*H₃*), 1.33-1.23 (*m,* 6H, *CH₂-CH₃,* CH₂-C*H*₃). | | | |
| **¹³C-NMR:** | (CDCl₃, 100 MHz), δ [ppm] = 195.5 (C=S), 137.6 (CH₂-C-CH), 133.5 (C-CH₃), 130.6 (C-CH-CH-CH-CH), 128.1 (C-CH-CH-CH), 126.4 (C-CH-CH-CH-CH), 49.4 (CH₂-CH₃), 46.8 (CH₂-CH₃), 40.9 (S-CH₂-C), 19.5 (C-CH₃), 12.6 (CH₂-CH₃), 11.8 (CH₂-CH₃). | | | |
| **IR (ATR):** | v (cm⁻¹) = 2974 (w), 2931 (w), 1483 (vs), 1457 (m), 1439 (m), 1413 (vs), 1377 (m), 1353 (m), 1299 (m), 1266 (vs), 1204 (vs), 1140 (s), 1091 (w), 1068 (w), 1007 (s), 982 (s), 916 (s), 829 (m), 779 (m), 730 (vs), 699 (w), 596 (w), 565 (w), 446 (w), 432 (w). | | | |
| **MS** (ESI+): | m/z (%) = 254 (100, [M+H]⁺) | | | |
| **HRMS** (ESI+): | m/z für C₁₃H₁₉NS₂ | | | |
| | ber.: | 253.0959 | | gef.: 253.0960 |
| **EA:** | ber.: | C: 61.61 | H: 7.56 | N: 5.53 |
| | gef.: | C: 61.76 | H: 7.62 | N: 5.57 |

### Darstellung von (4-Bromphenyl)methyl-N,N-diethylcarbamodithioat (Schl32232):

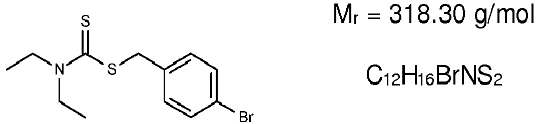

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 750 mg 1-Brom-4-(brommethyl)benzen (3.0 mmol, 1.0 eq) umgesetzt. Säulenchromatographie an Kieselgel (Cyclohexan/ EtOAc 9:1) ergab 933 mg von **Schl32232** (97.8%) in Form eines blassgelben Feststoffes.

| | | | | |
|---|---|---|---|---|
| **DC:** | R_{f} = 0.49 (Cyclohexan/ EtOAc 6:1). | | | |
| **Fp.:** | 48 °C | | | |
| **¹H-NMR:** | (CDCl₃, 400 MHz), δ [ppm] = 7.42 (*d*, ³J = 8.5 Hz, 2H, C-CH-C*H*-C-Br, C-CH-C*H*-C-Br), 7.26 (*d*, ³J = 8.5 Hz, 2H, C-C*H*-CH-C-Br, C-C*H*-CH-C-Br), 4.50 (*s,* 2H, S-C*H₂*-C), 4.03 (*q,* ³J = 7.1 Hz, 2H, CH₃-C*H*₂), 3.71 *(q,* ³J = 7.1 Hz, 2H, CH₃-C*H₂*), 1.27 (*t,* ³J = 7.1 Hz, 6H, *CH₂-CH₃,* CH₂-C*H*₃). | | | |
| **¹³C-NMR:** | (CDCl₃, 100 MHz), δ [ppm] = 194.8 (C=S), 135.6 (C-CH-CH-C-Br), 131.7 (C-CH-CH-C-Br, C-CH-CH-C-Br), 131.1 (C-CH-CH-C-Br, C-CH-CH-C-Br), 121.4 (C-Br), 49.7 (CH₂-CH₃), 46.9 (CH₂-CH₃), 41.3 (S-CH₂-C), 12.6 (CH₂-CH₃), 11.7 (CH₂-CH₃). | | | |
| **IR (ATR):** | v (cm⁻¹) = 2974 (w), 2933 (w), 1483 (s), 1458 (w), 1445 (w), 1436 (w), 1414 (s), 1399 (w), 1378 (w), 1351 (w), 1339 (w), 1298 (w), 1266 (s), 1205 (w), 1187 (s), 1175 (w), 1145 (w), 1066 (s), 1006 (s), 985 (m), 917 (m), 829 (vs), 800 (w), 791 (w), 748 (s), 506 (vs), 496 (w), 439 (w). | | | |
| **MS** (ESI+): | m/z (%) = 318 (95, [M^{79Br}+H]⁺), 320 (100, [M^{81Br}+H]⁺) | | | |
| **HRMS** (ESI+): | m/z für C₁₂H₁₆BrNS₂ | | | |
| | ber.: | 318.9908 | | gef.: 318.9922 |
| **EA:** | ber.: | C: 45.28 | H: 5.07 | N: 4.40 |
| | gef.: | C: 45.29 | H: 5.07 | N: 4.57 |

### Darstellung von (3-Bromphenyl)methyl-N,N-diethylcarbamodithioat (Schl32233):

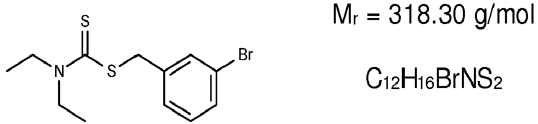

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 750 mg 1-Brom-3-(brommethyl)benzen (3.0 mmol, 1.0 eq) umgesetzt. Säulenchromatographie an Kieselgel (Cyclohexan/ EtOAc 9:1) ergab 644 mg von **Schl32233** (67.4%) in Form eines blassgelben Öls.

| | | | | |
|---|---|---|---|---|
| **DC:** | R_{f} = 0.44 (Cyclohexan/ EtOAc 6:1). | | | |
| **¹H-NMR:** | (CDCl₃, 400 MHz), δ [ppm] = 7.51 *(s,* 1H, CH₂-C-C*H-*C(Br)), 7.35 (*d*, ³J = 7.8 Hz, 1H, C-CH-CH-C*H*-C), 7.30 (*d*, ³J = 7.6 Hz, 1H, C-C*H*-CH-CH-C), 7.15 (*t,* ³J = 7.9 Hz, 1H, C-CH-C*H*-CH-C), 4.51 (*s*, 2H, S-C*H₂*-C), 4.01 (*q,* ³J = 7.1 Hz, 2H, CH₃-C*H*₂), 3.70 *(q,* ³J = 7.1 Hz, 2H, CH₃-C*H₂*), 1.26 (*t,* ³J = 7.0 Hz, 6H, CH₂-C*H*₃, CH₂-C*H*₃). | | | |
| **¹³C-NMR:** | (CDCl₃, 100 MHz), δ [ppm] = 194.6 (C=S), 139.0 (CH₂-C-CH), 132.3 (C-CH-C-Br), 130.6 (C-CH-CH-CH), 130.2 (C-CH-CH-CH), 128.1 (C-CH-CH-CH), 122.5 (C-Br), 49.8 (CH₂-CH₃), 46.9 (CH₂-CH₃), 41.3 (S-CH₂-C), 12.7 (CH₂-CH₃), 11.7 (CH₂-CH₃). | | | |
| **IR (ATR):** | v (cm⁻¹) = 2978 (w), 2935 (w), 1486 (m), 1447 (w), 1416 (s), 1380 (w), 1355 (w), 1327 (vs), 1380 (w), 1355 (w), 1268 (s), 1240 (w), 1205 (w), 1161 (s), 1118 (vs), 1091 (s), 1070 (vs), 1008 (m), 981 (m), 905 (m), 885 (w), 830 (w), 748 (w), 722 (w), 698 (s), 658 (m), 562 (w), 499 (w). | | | |
| **MS** (ESI+): | m/z (%) = 318 (95, [M^{79Br}+H]⁺), 320 (100, [M^{81Br}+H]⁺) | | | |
| **HRMS** (ESI+): | m/z für C₁₂H₁₆BrNS₂ | | | |
| | ber.: | 318.9908 | | gef.: 318.9909 |
| **EA:** | ber.: | C: 45.28 | H: 5.07 | N: 4.40 |
| | gef.: | C: 45.19 | H: 5.05 | N: 4.64 |

### Darstellung von (2-Bromphenyl)methyl-N,N-diethylcarbamodithioat (Schl32234):

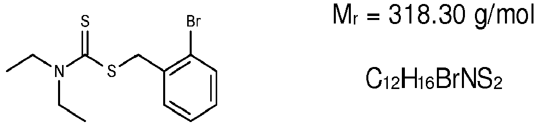

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 750 mg 1-Brom-2-(brommethyl)benzen (3.0 mmol, 1.0 eq) umgesetzt. Säulenchromatographie an Kieselgel (Cyclohexan/ EtOAc 9:1) ergab 802 mg von **Schl32234** (84.0%) in Form eines blassgelben Feststoffes.

| | | | | |
|---|---|---|---|---|
| **DC:** | R_{f} = 0.39 (Cyclohexan/ EtOAc 6:1). | | | |
| **Fp.:** | 45 °C | | | |
| **¹H-NMR:** | (CDCl₃, 400 MHz), δ [ppm] = 7.57-7.50 (m, 2H, CH₂-C-C(Br)-C*H*-CH, CH₂-C-C*H*-CH), 7.24 (*t,* ³J = 7.0 Hz, CH₂-C-CH-C*H*-CH), 7.11 (*t,* ³J = 7.1 Hz, CH₂-C-CH-CH-C*H*), 4.69 *(s,* 2H, S-C*H₂*-C), 4.02 (*q*, ³J = 7.1 Hz, 2H, CH₃-C*H*₂), 3.71 (*q,* ³J = 7.1 Hz, 2H, CH₃-C*H*₂), 1.26 *(t,* ³J = 7.0 Hz, 6H, CH₂-C*H₃*, CH₂-C*H*₃). | | | |
| **¹³C-NMR:** | (CDCl₃, 100 MHz), δ [ppm] = 194.9 (C=S), 136.3 (CH₂-C-CH), 133.0 (C-CH-CH-CH-CH), 131.7 (C-CH-CH-CH-CH), 129.3 (C-CH-CH-CH-CH), 127.7 (C-CH-CH-CH-CH), 125.1 (C-Br), 49.8 (CH₂-CH₃), 46.9 (CH₂-CH₃), 42.3 (S-CH₂-C), 12.7 (CH₂-CH₃), 11.7 (CH₂-CH₃). | | | |
| **IR (ATR):** | v (cm⁻¹) = 2980 (w), 2915 (w), 1489 (s), 1464 (w), 1438 (w), 1417 (s), 1398 (m), 1373 (w), 1337 (w), 1269 (s), 1198 (m), 1139 (w), 1092 (w), 1066 (w), 1043 (w), 999 (s), 978 (w), 845 (m), 825 (m), 775 (w), 760 (vs), 733 (vs), 687 (m), 574 (w), 559 (w), 490 (w), 443 (m), 430 (m), 406 (m). | | | |
| **MS** (ESI+): | m/z (%) = 318 (95, [M^{79Br}+H]⁺), 320 (100, [M^{81Br}+H]⁺) | | | |
| **HRMS** (ESI+): | m/z für C₁₂H₁₆BrNS₂ | | | |
| | ber.: | 318.9908 | | gef.: 318.9893 |
| **EA:** | ber.: | C: 45.28 | H: 5.07 | N: 4.40 |
| | gef.: | C: 45.23 | H: 5.05 | N: 4.24 |

### Darstellung von (4-Fluorphenyl)methyl-N,N-diethylcarbamodithioat (Schl32235):

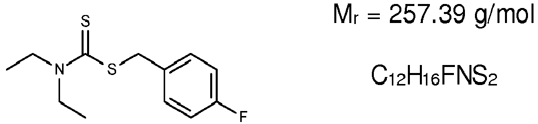

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 0.37 mL 1-Fluor-4-(brommethyl)benzen (3.0 mmol, 1.0 eq) umgesetzt. Säulenchromatographie an Kieselgel (Cyclohexan/ EtOAc 9:1) ergab 709 mg von **Schl32235** (91.8%) in Form eines blassgelben Öls.

| | | | | |
|---|---|---|---|---|
| **DC:** | R_{f} = 0.40 (Cyclohexan/ EtOAc 6:1). | | | |
| **¹H-NMR:** | (CDCl₃, 400 MHz), δ [ppm] = 7.38-7.31 (*m,* 2H, C*-CH*-CH-C-F, C-C*H*-CH-C-F), 7.01-6.93 (*m,* 2H, C-CH-C*H*-C-F, C-CH-C*H*-C-F), 4.51 (*s,* 2H, S-C*H₂*-C), 4.02 (*q,* ³J = 7.1 Hz, 2H, CH₃-C*H*₂), 3.70 (*q,* ³J = 7.1 Hz, 2H, CH₃-C*H*₂), 1.29-1.22 (*m,* 6H, CH₂-C*H₃,* CH₂-C*H*₃). | | | |
| **¹³C-NMR:** | (CDCl₃, 100 MHz), δ [ppm] = 195.0 (C=S), 162.2 (*d*, ¹J_{C,F} = 245.6 Hz, C-F), 132.1 (*d,* ⁴J_{C,F} = 2.4 Hz, C-CH-CH-C-F), 131.1 (*d,* ³J_{C,F} = 8.7 Hz, C-CH-CH-C-F, C-CH-CH-C-F), 115.5 (*d*, ²J_{C,F} = 21.2 Hz, C-CH-CH-C-F, C-CH-CH-C-F), 49.7 (CH₂-CH₃), 46.9 (CH₂-CH₃), 41.3 (S-CH₂-C), 12.6 (CH₂-CH₃), 11.7 (CH₂-CH₃). | | | |
| **IR (ATR):** | v (cm⁻¹) = 2976 (w), 2932 (w), 1507 (vs), 1484 (vs), 1458 (m), 1441 (m), 1414 (vs), 1378 (m), 1354 (m), 1267 (vs), 1219 (vs), 1206 (vs), 1156 (m), 1140 (m), 1089 (m), 1068 (m), 1007 (s), 982 (s), 916 (m), 828 (vs), 774 (w), 754 (w), 730 (w), 677 (w), 564 (w), 527 (S), 486 (s), 418 (w). | | | |
| **MS** (ESI+): | m/z (%) = 258 (100, [M+H]⁺) | | | |
| **HRMS** (ESI+): | m/z für C₁₂H₁₆FNS₂ | | | |
| | ber.: | 257.0708 | | gef.: 257.0696 |
| **EA:** | ber.: | C: 56.00 | H: 6.27 | N: 5.44 |
| | gef.: | C: 56.16 | H: 6.35 | N: 5.53 |

### Darstellung von (3-Fluorphenyl)methyl-N,N-diethylcarbamodithioat (Schl32236):

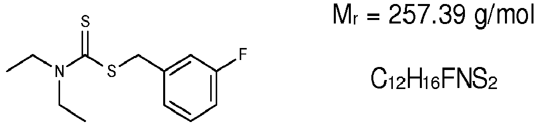

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 0.37 mL 1-Fluor-3-(brommethyl)benzen (3.0 mmol, 1.0 eq) umgesetzt. Säulenchromatographie an Kieselgel (Cyclohexan/ EtOAc 9:1) ergab 733 mg von **Schl32236** (95.0%) in Form eines blassgelben Öls.

| | | | | |
|---|---|---|---|---|
| **DC:** | R_{f} = 0.47 EtOAc | | | |
| **¹H-NMR:** | (CDCl₃, 400 MHz), 5 [ppm] = 7.28-7.22 (*m*, 1H, CH₂-C-CH-C*H*-CH), 7.15 (*d*, ³J = 7.6 Hz, 1 H, C-C*H*-CH-CH-C), 7.09 (*d*, ³J = 8.2 Hz, 1 H, C-CH-CH-C*H*-C), 6.96-6.92 (m, 1 H, C-C*H*-C), 4.54 (s, 2H, S-C*H₂-*C), 4.03 (*q,* ³J = 7.1 Hz, 2H, CH₃-C*H₂*), 3.72 (*q*, ³J = 7.1 Hz, 2H, CH₃-C*H*₂), 1.27 (*t,* ³J = 7.1 Hz, 6H, CH₂-C*H*₃, CH₂-C*H*₃). | | | |
| **¹³C-NMR:** | (CDCl₃, 100 MHz), δ [ppm] = 194.7 (C=S), 162.8 (*d*, ¹J_{C,F} = (CDCl₃, 100 MHz), δ [ppm] 245.6 Hz, C-F), 139.0 (*d*, ³J_{C,F} = 7.7 Hz, C-CH-CH-C-F), 130.1 (*d,* ³J_{C,F} = 8.7 Hz, C-CH-CH-CH-C-F), 125.1 (*d*, ⁴J_{C,F} = 1.9 Hz, C-CH-CH-CH-C-F), 116.3 (*d*, ²J_{C,F} = 22.2 Hz, C-CH-C-F), 114.4 (*d,* ²J_{C,F} = 21.2 Hz, C-CH-CH-CH-C-F), 49.8 (CH₂-CH₃), 46.9 (CH₂-CH₃), 41.5 (S-CH₂-C), 12.6 (CH₂-CH₃), 11.7 (CH₂-CH₃). | | | |
| **IR (ATR):** | v (cm⁻¹) = 2976 (w), 2932 (w), 1614 (w), 1567 (m), 1485 (vs), 1458 (m), 1443 (s), 1415 (vs), 1378 (m), 1354 (m), 1266 (vs), 1202 (vs), 1138 (s), 1091 (w), 1069 (m), 1008 (m), 981 (s), 942 (s), 916 (s), 878 (m), 830 (m), 784 (s), 742 (m), 715 (m), 681 (s), 596 (w), 562 (w), 543 (w), 438 (m). | | | |
| **MS** (ESI+): | m/z (%) = 258 (100, [M+H]⁺) | | | |
| **HRMS** (ESI+): | m/z für C₁₂H₁₆FNS₂ | | | |
| | ber.: | 257.0708 | | gef.: 257.0691 |
| **EA:** | ber.: | C:56.00 | H: 6.27 | N:5.44 |
| gef.: | C: 56.10 | | H: 6.34 | N: 5.37 |

### Darstellung von (2-Fluorphenyl)methyl-N,N-diethylcarbamodithioat (Schl32237):

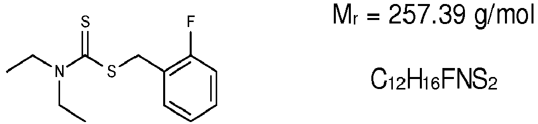

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 0.37 mL 1-Fluor-2-(brommethyl)benzen (3.0 mmol, 1.0 eq) umgesetzt. Säulenchromatographie an Kieselgel (Cyclohexan/ EtOAc 9:1) ergab 714 mg von **Schl32237** (92.0%) in Form eines blassgelben Öls.

| | | | | |
|---|---|---|---|---|
| **DC:** | R_{f} = 0.47 (Cyclohexan/ EtOAc 6:1). | | | |
| **¹H-NMR:** | (CDCl₃, 400 MHz), δ [ppm] = 7.50-7.42 (*m,* 1H, CH₂-C-C(F)-C*H*-CH), 7.26-7.14 (*m,* 1H, CH₂-C-C(F)-CH-C*H*), 7.09-6.95 *(m,* 2H, CH₂-C-C*H*-C*H*), 4.58 *(s,* 2H, S-C*H₂*-C), 4.03 (*q,* ³J = 6.9 Hz, 2H, CH₃-C*H*₂), 3.71 (*q,* ³J = 6.9 Hz, 2H, CH₃-C*H₂*), 1.29-1.19 (*m,* 6H, CH₂-C*H₃,* CH₂-C*H*₃). | | | |
| **¹³C-NMR:** | (CDCl₃, 100 MHz), δ [ppm] = 195.0 (C=S), 161.2 (*d*, ¹J_{C,F} = 247.6 Hz, C-F), 131.6 (*d,* ³J_{C,F} = 7.9 Hz, CH-CH-CH-C-F), 129.4 (*d,* ³J_{C,F} = 7.7 Hz, C-CH-CH-CH-CH-C-F), 124.3 (*d,* ⁴J_{C,F} = 2.9 Hz, C-CH-CH-CH-CH-C-F), 123.6 (*d*, ²J_{C,F} = 14.5 Hz, C-C-F), 115.5 (*d*, ²J_{C,F} = 21.2 Hz, C-CH-CH-CH-CH-C-F), 49.7 (CH₂-CH₃), 46.9 (CH₂-CH₃), 35.1 (S-CH₂-C), 12.6 (CH₂-CH₃), 11.7 (CH₂-CH₃). | | | |
| **IR (ATR):** | v (cm⁻¹) = 2975 (w), 2932 (w), 1585 (w), 1486 (vs), 1454 (m), 1414 (vs), 1378 (w), 1354 (m), 1267 (vs), 1229 (s), 1204 (vs), 1140 (s), 1090 (m), 1068 (m), 1007 (m), 982 (m), 941 (w), 916 (m), 889 (w), 866 (w), 830 (m), 751 (vs), 697 (w), 685 (w), 587 (w), 564 (w), 526 (w), 437 (w). | | | |
| **MS** (ESI+): | m/z (%) = 258 (100, [M+H]⁺) | | | |
| **HRMS** (ESI+): | m/z für C₁₂H₁₆FNS₂ | | | |
| | ber.: | 257.0708 | | gef.: 257.0714 |
| **EA:** | ber.: | C: 56.00 | H: 6.27 | N: 5.44 |
| | gef.: | C: 56.24 | H: 6.33 | N: 5.52 |

### Darstellung von [4-(Trifluormethyl)phenyl]methyl-N,N-diethylcarbamodithioat (Schl32238):

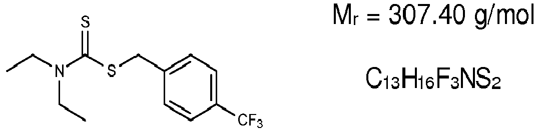

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 717 mg 1-(Brommethyl)-4-(trifluormethyl)benzen (3.0 mmol, 1.0 eq) umgesetzt. Säulenchromatographie an Kieselgel (Cyclohexan/ EtOAc 9:1) ergab 769 mg von **Schl32238** (86.4%) in Form eines blassgelben Öls.

| | | | | |
|---|---|---|---|---|
| **DC:** | R_{f} = 0.39 (Cyclohexan/ EtOAc 6:1). | | | |
| **¹H-NMR:** | (CDCl₃, 400 MHz), δ [ppm] = 7.55 (*d*, ³J = 8.2 Hz, 2H, C-CH-C*H*-C-CF₃, C-CH-C*H*-C-CF₃), 7.50 (*d*, ³J = 8.2 Hz, 2H, C-CH-C*H*-C-CF₃, C-CH-C*H*-C-CF₃), 4.61 (*s,* 2H, S-C*H₂*-C), 4.03 (*q,* ³J = 7.1 Hz, 2H, CH₃-C*H*₂), 3.72 (*q*, ³J = 7.1 Hz, 2H, CH₃-C*H₂*), 1.27 (*t,* ³J = 7.1 Hz, 6H, *CH₂-CH₃,* CH₂-C*H*₃). | | | |
| **¹³C-NMR:** | (CDCl₃, 100 MHz), δ [ppm] = 194.5 (C=S), 141.1 (S-CH₂-C), 129.7 (C-CH-CH-C-CF₃, C-CH-CH-C-CF₃), 129.2 (*q*, ²J_{C,F} = 31.8 Hz, C-CF₃), 125.5 (*q*, ³J_{C,F} = 8.8 Hz, C-CH-CH-C-CF₃, C-CH-CH-C-CF₃), 121.5 (*q*, ¹J_{C,F} = 271.7 Hz, CF₃), 49.9 (CH₂-CH₃), 46.9 (CH₂-CH₃), 41.2 (S-CH₂-C), 12.6 (CH₂-CH₃), 11.6 (CH₂-CH₃). | | | |
| **IR (ATR):** | v (cm⁻¹) = 2978 (w), 1616 (w), 1486 (m), 1458 (w), 1442 (w), 1415 (s), 1379 (w), 1355 (w), 1320 (vs), 1301 (w), 1268 (s), 1207 (m), 1161 (s), 1117 (vs), 1064 (vs), 1008 (s), 981 (s), 916 (m), 870 (w), 850 (w), 828 (m), 753 (w), 699 (w), 616 (w), 591 (w), 563 (w), 503 (w), 411 (w). | | | |
| **MS** (ESI+): | m/z (%) = 308 (100, [M+H]⁺) | | | |
| **HRMS** (ESI+): | m/z für C₁₃H₁₆F₃NS₂ | | | |
| | ber.: | 307.0676 | | gef.: 307.0673 |
| **EA:** | ber.: | C: 50.79 | H: 5.25 | N: 4.56 |
| | gef.: | C: 50.81 | H: 5.28 | N: 4.83 |

### Darstellung von [3-(Trifluormethyl)phenyl]methyl-N,N-diethylcarbamodithioat (Schl32239):

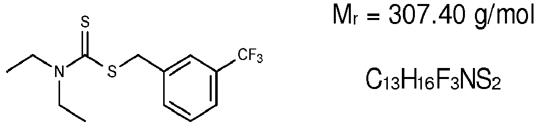

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 717 mg 1-(Brommethyl)-3-(trifluormethyl)benzen (3.0 mmol, 1.0 eq) umgesetzt. Säulenchromatographie an Kieselgel (Cyclohexan/ EtOAc 9:1) ergab 873 mg von **Schl32239** (94.8%) in Form eines blassgelben Öls.

| | | | | |
|---|---|---|---|---|
| **DC:** | R_{f} = 0.43 (Cyclohexan/ EtOAc 6:1). | | | |
| **¹H-NMR:** | (CDCl₃, 400 MHz), δ [ppm] = 7.63 (s, 1H, CH₂-C-C*H-*C(CF₃)), 7.58 (*d*, ³J = 7.6 Hz, 1H, C-CH-CH-C*H*-C), 7.49 (*d*, ³J = 7.8 Hz, 1H, C-C*H*-CH-CH-C), 7.41 (*t,* ³J = 7.8 Hz, 1H, C-CH-C*H*-CH-C), 4.61 (*s*, 2H, S-C*H₂*-C), 4.03 (*q*, ³J = 7.1 Hz, 2H, CH₃-C*H*₂), 3.72 (*q*, ³J = 7.1 Hz, 2H, CH₃-C*H₂*), 1.27 (*t,* ³J = 7.0 Hz, 6H, CH₂-CH₃, CH₂-C*H*₃). | | | |
| **¹³C-NMR:** | (CDCl₃, 100 MHz), δ [ppm] = 194.4 (C=S), 137.9 (CH₂-C), 132.9 (CH₂-C-CH-CH-CH-C), 130.8 (*q*, ²J_{C,F} = 31.8 Hz, C-CF₃), 129.1 (CH₂-C-CH-CH-CH-C), 126.1 (*q*, ³J_{C,F} = 3.9 Hz, C-CH-C-CF₃), 124.3 (*q*, ³J_{C,F} = 3.9 Hz, C-CH-CH-CH-C-CF₃), 124.1 (*q*, ¹J_{C,F} = 272.6 Hz, CF₃), 49.9 (CH₂-CH₃), 46.9 (CH₂-CH₃), 41.3 (S-CH₂-C), 12.6 (CH₂-CH₃), 11.6 (CH₂-CH₃). | | | |
| **IR (ATR):** | v (cm⁻¹) = 2974 (w), 2931 (w), 1567 (w), 1484 (s), 1458 (m), 1440 (vs), 1414 (m), 1377 (s), 1352 (w), 1299 (m), 1266 (vs), 1204 (vs), 1140 (s), 1090 (w), 1068 (s), 1007 (s), 981 (s), 916 (m), 885 (m), 855 (m), 829 (s), 784 (m), 736 (m), 716 (w), 683 (s), 665 (s), 580 (w), 495 (w), 429 (m). | | | |
| **MS** (ESI+): | m/z (%) = 308 (100, [M+H]⁺) | | | |
| **HRMS** (ESI+): | m/z für C₁₃H₁₆F₃NS₂ | | | |
| | ber.: | 307.0676 | | gef.: 307.0682 |
| **EA:** | ber.: | C: 50.79 | H: 5.25 | N: 4.56 |
| | gef.: | C: 50.81 | H: 5.29 | N: 4.50 |

### Darstellung von [2-(Trifluormethyl)phenyl]methyl-N,N-diethylcarbamodithioat (Schl32240):

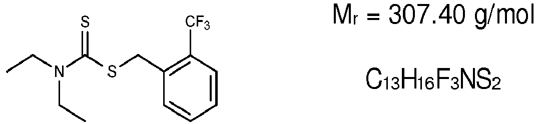

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 717 mg 1-(Brommethyl)-2-(trifluormethyl)benzen (3.0 mmol, 1.0 eq) umgesetzt. Säulenchromatographie an Kieselgel (Cyclohexan/ EtOAc 9:1) ergab 860 mg von **Schl32240** (93.3%) in Form eines blassgelben Öls.

| | | | | |
|---|---|---|---|---|
| **DC:** | R_{f} = 0.40 (Cyclohexan/ EtOAc 6:1). | | | |
| **¹H-NMR:** | (CDCl₃, 400 MHz), δ [ppm] = 7.69 (*d*, ³J = 7.6 Hz, 1H, C*H*-C-CF₃), 7.61 (*d*, ³J = 8.0 Hz, 1H, C-C*H*-CH-CH-CH-C-CF₃), 7.47 (*t,* ³J = 7.6 Hz, 1H, C-CH-C*H*-CH-CH-C-CF₃), 7.34 (*t,* ³J = 7.6 Hz, 1H, C*H*-CH-C-CF₃), 4.80 (s, 2H, S-C*H₂*-C), 4.04 (*q,* ³J = 6.9 Hz, 2H, CH₃-C*H*₂), 3.71 (*q,* ³J = 6.9 Hz, 2H, CH₃-C*H₂*), 1.31-1.22 (*m,* 6H, CH₂-C*H₃,* CH₂-C*H*₃). | | | |
| **¹³C-NMR:** | (CDCl₃, 100 MHz), δ [ppm] = 194.8 (C=S), 135.6 (CH₂-C), 132.2 (CH₂-C-CH-CH-CH-CH-C-CF₃), 132.1 (CH₂-C-CH-CH-CH-CH-C-CF₃), 128.9 (*q*, ²J_{C,F} = 29.9 Hz, C-CF₃), 127.6 (CH₂-C-CH-CH-CH-CH-C-CF₃), 129.1 (CH₂-C-CH-CH-CH-C), 126.0 (*q*, ³J_{C,F} = 5.8 Hz, CH-C-CF₃), 124.4 (*q*, ¹J_{C,F} = 274.1 Hz, CF₃), 50.0 (CH₂-CH₃), 46.9 (CH₂-CH₃), 38.3 (S-CH₂-C), 12.6 (CH₂-CH₃), 11.6 (CH₂-CH₃). | | | |
| **IR (ATR):** | v (cm⁻¹) = 2978 (w), 2934 (w), 1486 (m), 1416 (s), 1379 (w), 1355 (w), 1312 (s), 1296 (s), 1267 (s), 1206 (m), 1173 (m), 1113 (vs), 1058 (s), 1035 (vs), 1007 (m), 981 (m), 916 (m), 889 (w), 858 (w), 828 (w), 764 (vs), 710 (w), 652 (m), 596 (w), 561 (w), 506 (w), 471 (w), 435 (w). | | | |
| **MS** (ESI+): | m/z (%) = 308 (100, [M+H]⁺) | | | |
| **HRMS** (ESI+): | m/z für C₁₃H₁₆F₃NS₂ | | | |
| | ber.: | 307.0676 | | gef.: 307.0656 |
| **EA:** | ber.: | C: 50.79 | H: 5.25 | N: 4.56 |
| | gef.: | C: 50.72 | H: 5.33 | N: 4.68 |

### Darstellung von (3-Cyanophenyl)methyl-N,N-diethylcarbamodithioat (Schl32241):

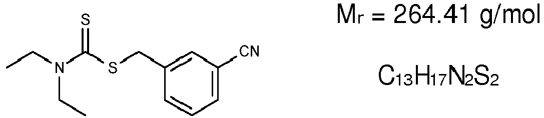

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 588 mg 3-(Brommethyl)benzonitril (3.0 mmol, 1.0 eq) umgesetzt. Säulenchromatographie an Kieselgel (Cyclohexan/ EtOAc 9:1) ergab 742 mg von **Schl32241** (93.6%) in Form eines gelben Öls.

| | | | | |
|---|---|---|---|---|
| **DC:** | R_{f} = 0.29 (Cyclohexan/ EtOAc 6:1). | | | |
| **¹H-NMR:** | (CDCl₃, 400 MHz), δ [ppm] = 7.65 *(s,* 1 H, CH₂-C-C*H*-C-CN), 7.60 (*d*, ³J = 8.9 Hz, 1H, C-CH-CH-C*H*-C), 7.49 (*d*, ³J = 7.8 Hz, 1H, C-C*H*-CH-CH-C), 7.37 (*t,* ³J = 7.8 Hz, 1H, C-CH-C*H*-CH-C), 4.56 (*s*, 2H, S-C*H₂*-C), 3.99 (*q*, ³J = 6.9 Hz, 2H, CH₃-C*H*₂), 3.70 (*q*, ³J = 7.1 Hz, 2H, CH₃-C*H₂*), 1.29-1.20 *(m,* 6H, *CH₂-CH₃,* CH₂-C*H*₃). | | | |
| **¹³C-NMR:** | (CDCl₃, 100 MHz), δ [ppm] = 194.0 (C=S), 138.9 (CH₂-C-CH), 133.9 (C-CH-C-CN), 132.8 (C-CH-CH-CH), 131.0 (C-CH-CH-CH), 129.4 (C-CH-CH-CH), 118.8 (C-CN), 112.5 (C-CN), 50.0 (CH₂-CH₃), 47.0 (CH₂-CH₃), 40.6 (S-CH₂-C), 12.7 (CH₂-CH₃), 11.6 (CH₂-CH₃). | | | |
| **IR (ATR):** | v (cm⁻¹) = 2975 (w), 2932 (w), 2228 (w), 1464 (s), 1457 (w), 1440 (vs), 1415 (m), 1378 (m), 1353 (w), 1299 (m), 1267 (vs), 1201 (vs), 1141 (s), 1091 (w), 1067 (m), 1007 (m), 980 (s), 913 (s), 829 (m), 799 (m), 744 (w), 706 (w), 683 (s), 606 (w), 561 (w), 539 (w), 496 (w), 461 (w), 429 (w). | | | |
| **MS** (ESI+): | m/z (%) = 265 (100, [M+H]⁺) | | | |
| **HRMS** (ESI+): | m/z für C₁₃H₁₇N₂S₂ | | | |
| | ber.: | 264.0755 | | gef.: 264.0757 |
| **EA:** | ber.: | C: 59.05 | H: 6.10 | N: 10.59 |
| | gef.: | C: 59.18 | H: 6.18 | N: 10.63 |

### Darstellung von (2-Cyanophenyl)methyl-N,N-diethylcarbamodithioat (Schl32242):

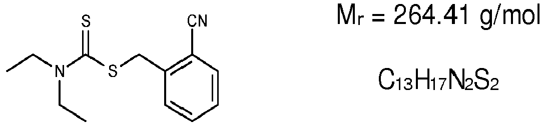

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 588 mg 2-(Brommethyl)benzonitril (3.0 mmol, 1.0 eq) umgesetzt. Säulenchromatographie an Kieselgel (Cyclohexan/ EtOAc 9:1) ergab 755 mg von **Schl32242** (95.2%) in Form eines gelben Öls.

| | | | | |
|---|---|---|---|---|
| **DC:** | R_{f} = 0.31 (Cyclohexan/ EtOAc 6:1). | | | |
| **¹H-NMR:** | (CDCl₃, 400 MHz), δ [ppm] = 7.68 (*d*, ³J = 7.8 Hz, 1H, C*H*-C-CN), 7.61 (*d*, ³J = 7.6 Hz, 1 H, C-C*H*-CH-CH-CH-C-CN), 7.51 (*t,* ³J = 7.7 Hz, 1H, CH-C*H*-CH-CH-C-CN), 7.33 (*t,* ³J = 7.6 Hz, 1 H, CH-CH-C*H*-CH-C-CN), 4.79 (s, 2H, S-C*H₂*-C), 4.01 *(q,* ³J = 7.1 Hz, 2H, CH₃-C*H*₂), 3.72 (*q*, ³J = 7.1 Hz, 2H, CH₃-C*H₂*), 1.26 (*t,* ³J = 7.0 Hz, 6H, *CH₂-CH₃,* CH₂-C*H*₃). | | | |
| **¹³C-NMR:** | (CDCl₃, 100 MHz), δ [ppm] = 193.9 (C=S), 141.2 (C-C-CN), 133.0 (C-CH-CH-CH-CH-C-CN), 132.9 (C-CH-CH-CH-CH-C-CN), 130.8 (C-CH-CH-CH-CH-C-CN), 127.9 (C-CH-CH-CH-CH-C-CN), 117.6 (C-CN), 113.0 (C-CN), 50.0 (CH₂-CH₃), 47.0 (CH₂-CH₃), 39.4 (S-CH₂-C), 12.7 (CH₂-CH₃), 11.6 (CH₂-CH₃). | | | |
| **IR (ATR):** | v (cm⁻¹) = 2975 (w), 2932 (w), 2224 (w), 1485 (vs), 1444 (m), 1415 (vs), 1378 (w), 1353 (m), 1299 (m), 1267 (vs), 1202 (vs), 1141 (s), 1090 (w), 1067 (m), 1007 (m), 981 (s), 916 (s), 851 (w), 828 (m), 754 (vs), 702 (w), 686 (w), 600 (w), 556 (w), 496 (w), 459 (w), 429 (w). | | | |
| **MS** (ESI+): | m/z (%) = 265 (100, [M+H]⁺) | | | |
| **HRMS** (ESI+): | m/z für C₁₃H₁₇N₂S₂ | | | |
| | ber.: | 264.0755 | | gef.: 264.0749 |
| **EA:** | ber.: | C: 59.05 | H: 6.10 | N: 10.59 |
| | gef.: | C: 58.72 | H: 6.10 | N: 10.54 |

### Darstellung von (3-Methoxyphenyl)methyl-N,N diethylcarbamodithioat (Schl32243):

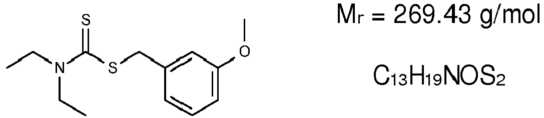

Gemäß AAV8 wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 603 mg 1-(Brommethyl)-3-methoxybenzen (3.0 mmol, 1.0 eq) umgesetzt. Säulenchromatographie an Kieselgel (Cyclohexan/EtOAc 9:1) ergab 635 mg von **Schl32243** (78.6%) in Form eines blassgelben Öls.

| | | | | |
|---|---|---|---|---|
| **DC:** | R_{f} = 0.35 (Cyclohexan/ EtOAc 6:1). | | | |
| **¹H-NMR:** | (CDCl₃, 400 MHz), δ [ppm] = 7.22 (*t*, ³J = 7.8 Hz, 1H, C-CH-C*H*-CH-C-OMe), 6.97 (s, 1H, C-C*H*-C-OMe), 6.96-6.92 (*m,* 1H, C-C*H*-CH-CH-C-OMe), 6.82-6.77 (*m,* 1H, C-CH-CH-C*H*-C-OMe), 4.51 (*s,* 2H, S-C*H*₂-C), 4.03 (*q,* ³J = 7.1 Hz, 2H, CH₃-C*H*₂), 3.78 (*s,* O-C*H*₃), 3.71 (*q*, ³J = 7.1 Hz, 2H, CH₃-C*H*₂), 1.30-1.21 (*m*, 6H, CH₂-C*H*₃, CH₂-C*H*₃). | | | |
| **¹³C-NMR:** | (CDCl₃, 100 MHz), δ [ppm] = 195.2 (C=S), 159.8 (C-OMe), 137.6 (CH₂-C-CH), 129.7 (C-CH-CH-CH-C-OMe), 121.8 (C-CH-CH-CH-C-OMe), 114.9 (C-CH-C-OMe), 113.2 (C-CH-CH-CH-C-OMe), 55.3 (O-CH₃), 49.6 (CH₂-CH₃), 46.9 (CH₂-CH₃), 42.3 (S-CH₂-C), 12.6 (CH₂-CH₃), 11.7 (CH₂-CH₃). | | | |
| **IR (ATR):** | v (cm⁻¹) = 2974 (w), 2932 (w), 1598 (m), 1583 (w), 1485 (s), 1453 (m), 1437 (m), 1414 (vs), 1377 (w), 1353 (w), 1296 (w), 1262 (vs), 1203 (vs), 1141 (s), 1089 (w), 1068 (w), 1042 (m), 1007 (m), 981 (s), 914 (w), 870 (w), 830 (m), 783 (m), 736 (w), 714 (w), 689 (m), 561 (w), 431 (w). | | | |
| **MS** (ESI+): | m/z (%) = 270 (100, [M+H]⁺) | | | |
| **HRMS** (ESI+): | m/z für C₁₃H₁₉NOS₂ | | | |
| | ber.: | 269.0908 | | gef.: 269.0918 |
| **EA:** | ber.: | C: 57.95 | H: 7.11 | N: 5.20 |
| gef.: | C: 57.89 | | H: 7.06 | N: 5.15 |

### Darstellung von (3,5-Dimethoxyphenyl)methyl-N,N-diethylcarbamodithioat (Schl32244):

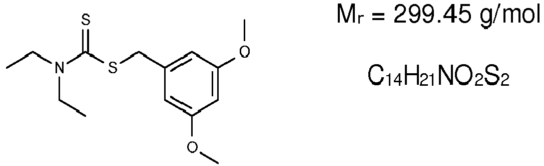

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 693 mg 1-(Brommethyl)-3,5-dimethoxybenzen (3.0 mmol, 1.0 eq) umgesetzt. Säulenchromatographie an Kieselgel (Cyclohexan/EtOAc 9:1) ergab 829 mg von **Schl32244** (92.1 %) in Form eines blassgelben Öls.

| | | | | |
|---|---|---|---|---|
| DC: | R_{f} = 0.32 (Cyclohexan/ EtOAc 6:1). | | | |
| **¹H-NMR:** | (CDCl₃, 400 MHz), δ [ppm] = 6.55-6.51 (*m,* 2H, CH₂-C-C*H-*C-OMe, CH₂-C-C*H*-C-OMe), 6.35-6.32 (*m,* 1H, C(-OMe)-C*H*-C(-OMe)), 4.54 (s, 2H, S-C*H₂*-C), 4.02 (*q,* ³J = 7.1 Hz, 2H, CH₃-C*H*₂), 3.76 (s, 6H, O*-*C*H₃,* O-C*H₃*), 3.70 *(q,* ³J = 7.0 Hz, 2H, CH₃-C*H*₂), 1.29-1.20 (*m,* 6H, CH₂-C*H*₃, CH₂-C*H*₃). | | | |
| **¹³C-NMR:** | (CDCl₃, 100 MHz), δ [ppm] = 195.2 (C=S), 160.9 (C-OMe, C-OMe), 138.3 (CH₂-C-CH), 107.4 (C-CH-C-OMe, C-CH-C-OMe), 99.6 C(-OMe)-CH-C(-OMe)), 55.4 (O-CH₃), 49.6 (CH₂-CH₃), 46.9 (CH₂-CH₃), 42.5 (S-CH₂-C), 12.6 (CH₂-CH₃), 11.7 (CH₂-CH₃). | | | |
| IR (ATR): | v (cm⁻¹) = 2973 (w), 2932 (w), 2835 (w), 1592 (vs), 1485 (m), 1456 (s), 1414 (vs), 1377 (w), 1345 (m), 1321 (m), 1297 (m), 1266 (vs), 1202 (vs), 1142 (vs), 1091 (w), 1057 (vs), 1007 (s), 982 (s), 916 (s), 828 (vs), 775 (w), 747 (w), 724 (w), 686 (m), 592 (w), 561 (w), 495 (w), 429 (w). | | | |
| **MS** (ESI+): | m/z (%) = 300 (100, [M+H]⁺) | | | |
| **HRMS** (ESI+): | m/z für C₁₄H₂₁NO2S₂ | | | |
| | ber.: | 299.1014 | | gef.: 299.1021 |
| **EA:** | ber.: | C: 56.15 | H: 7.07 | N: 4.68 |
| | gef.: | C: 55.67 | H: 7.02 | N: 4.78 |

### Darstellung von (4-Isopropylphenyl)methyl-N,N-diethylcarbamodithioat (Schl32245):

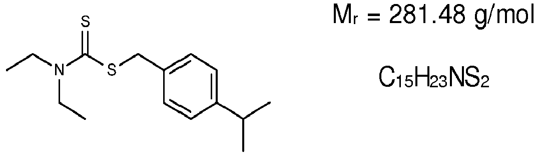

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 639 mg 4-(Isopropyl)benzylbromid (3.0 mmol, 1.0 eq) umgesetzt. Säulenchromatographie an Kieselgel (Cyclohexan/EtOAc 7:1) ergab 697 mg von **Schl32245** (82.6%) in Form eines blassgelben Öls.

| | | | | |
|---|---|---|---|---|
| **DC:** | R_{f} = 0.47 (Cyclohexan/ EtOAc 6:1). | | | |
| **¹H-NMR:** | (CDCl₃, 400 MHz), δ [ppm] = 7.31 (*d,* ³J = 6.4 Hz, 2H, C-CH-C*H*-C-CH, C-CH-C*H*-C-CH), 7.17 (*d*, ³J = 8.2 Hz, 2H, C-C*H*-CH-C-CH, C-C*H*-CH-C-CH), 4.50 (*s,* 2H, S-CH₂-C), 4.04 (*q,* ³J = 7.1 Hz, 2H, CH₃-C*H*₂), 3.71 *(q,* ³J = 7.1 Hz, 2H, CH₃-C*H*₂), 2.96-2.83 (*m,* 1H, C-C*H*-(CH₃)₂), 1.35-1.20 (*m,* 12H, CH₂-C*H*₃, CH₂-C*H*₃, C-CH-(C*H*₃)₂). | | | |
| **¹³C-NMR:** | (CDCl₃, 100 MHz), δ [ppm] = 195.5 (C=S), 148.3 (C-CH-(CH₃)₂), 133.1 (S-CH₂-C), 129.5 (CH₂-C-CH-CH-C, CH₂-C-CH-CH-C), 126.8 (CH₂-C-CH-CH-C, CH₂-C-CH-CH-C), 49.5 (CH₂-CH₃), 46.8 (CH₂-CH₃), 42.2 (S-CH₂-C), 33.9 (C-CH), 24.1 (C-CH-(CH₃)₂), 12.6 (CH₂-CH₃), 11.7 (CH₂-CH₃). | | | |
| **IR (ATR):** | v (cm⁻¹) = 2958 (m), 2931 (w), 2870 (w), 1510 (w), 1484 (s), 1458 (w), 1440 (w), 1413 (vs), 1378 (m), 1353 (m), 1300 (w), 1266 (vs), 1207 (s), 1140 (m), 1092 (w), 1068 (m), 1053 (w), 1008 (s), 983 (s), 916 (m), 864 (w), 829 (s), 775 (w), 717 (w), 555 (m), 497 (w), 431 (w), 410 (w). | | | |
| **MS** (ESI+): | m/z (%) = 282 (100, [M+H]⁺) | | | |
| **HRMS** (ESI+): | m/z für C₁₅H₂₃NS₂ | | | |
| | ber.: | 281.1272 | | gef.: 281.1281 |
| **EA:** | ber.: | C: 64.00 | H: 8.24 | N: 4.98 |
| | gef.: | C: 63.95 | H: 8.23 | N: 5.05 |

### Darstellung von (4-Benzoylphenyl)methyl-N,N-diethylcarbamodithioat (Schl32246):

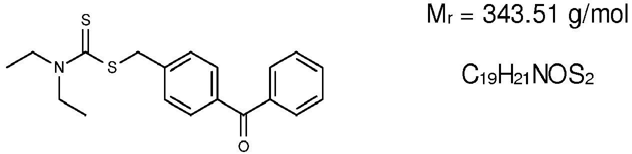

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 825 mg 4-(Brommethyl)benzophenon (3.0 mmol, 1.0 eq) umgesetzt. Säulenchromatographie an Kieselgel (Cyclohexan/ EtOAc 9:1) ergab 911 mg von **Schl32246** (88.5%) in Form eines blassgelben Öls.

| | | | | |
|---|---|---|---|---|
| DC: | R_{f}= 0.32 (Cyclohexan/ EtOAc 6:1). | | | |
| **¹H-NMR:** | (CDCl₃, 400 MHz), δ [ppm] = 7.76-7.64 (*m,* 4H, C*H*_{Benzophenon}), 7.55-7.49 (*m,* 1H, C*H*_{Benzophenon}), 7.47-7.35 (*m,* 4H, C*H*_{Benzo- phenon}), 4.60 (*s,* 2H, S-C*H*₂-C), 3.99 (*q,* ³J = 7.0 Hz, 2H, CH₃-C*H*₂), 3.69 (*q,* ³J = 6.9 Hz, 2H, CH₃-C*H*₂), 1.26-1.18 (*m,* 6H, CH₂-C*H*₃, CH₂-C*H*₃). | | | |
| **¹³C-NMR:** | (CDCl₃, 100 MHz), δ [ppm] = 196.2 (C=S), 194.5 (C=O), 141.7 (C_{q (Benzophenon)}), 137.6 (C_{q (Benzophenon)}), 136.6 (C_{q (Benzophenon)}), 132.5 (C_{t (Benzophenon)}), 130.4 (Ct _{(Benzophenon)}), 130.0 (C_{t (Benzophenon)}), 129.3 (C_{t (Benzophenon)}), 128.4 (C_{t (Benzophenon)}), 49.9 (CH₂-CH₃), 46.9 (CH₂-CH₃), 41.4 (S-CH₂-C), 12.6 (CH₂-CH₃), 11.7 (CH₂-CH₃). | | | |
| **IR (ATR):** | v (cm⁻¹) = 2974 (w), 2931 (w), 1653 (s), 1604 (m), 1577 (w), 1485 (m), 1444 (w), 1413 (s), 1376 (w), 1353 (w), 1314 (m), 1300 (m), 1267 (vs), 1206 (s), 1175 (w), 1141 (m), 1091 (w), 1069 (w), 1007 (w), 981 (m), 937 (m), 916 (s), 872 (w), 829 (m), 791 (w), 751 (w), 717 (w), 698 (vs), 634 (w), 615 (w), 562 (w), 496 (w), 429 (w). | | | |
| **MS** (ESI+): | m/z (%) = 344 (100, [M+H]⁺) | | | |
| **HRMS** (ESI+): | m/z für C₁₉H₂₁NOS₂ | | | |
| | ber.: | 343.1065 | | gef.: 343.1041 |
| **EA:** | ber.: | C: 66.43 | H: 6.16 | N: 4.08 |
| | gef.: | C: 66.52 | H: 6.26 | N: 4.34 |

### Darstellung von (4-Methylsulfanylphenyl)methyl-N,N-diethylcarbamodithioat (Schl32247) :

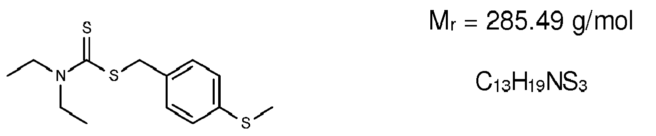

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 651 mg 1-(Brommethyl)-4-methylsulfanylbenzen (3.0 mmol, 1.0 eq) umgesetzt. Säulenchromatographie an Kieselgel (Cyclohexan/ EtOAc 9:1) ergab 783 mg von **Schl32247** (91.4%) in Form eines farblosen Feststoffes.

| | | | | |
|---|---|---|---|---|
| **DC:** | R_{f} = 0.44 (Cyclohexan/ EtOAc 6:1). | | | |
| **Fp**.: | 72°C | | | |
| **¹H-NMR:** | (CDCl₃, 400 MHz), δ [ppm] = 7.30 (*d*, ³J = 8.0 Hz, 2H, C-CH-C*H*-C-S-CH₃, C-CH-C*H*-C-S-CH₃), 7.18 (*d*, ³J = 7.6 Hz, 2H, C-C*H*-CH-C-S-CH₃, C-C*H*-CH-C-S-CH₃), 4.49 (*s,* 2H, S-C*H*₂-C), 4.03 (*q,* ³J = 6.9 Hz, 2H, CH₃-C*H*₂), 3.71 (*q,* ³J = 7.1 Hz, 2H, CH₃-C*H*₂), 2.45 (s, 1H, S-C*H*₃), 1.33-1.21 (*m*, 6H, CH₂-C*H*₃, CH₂-C*H*₃). | | | |
| **¹³C-NMR:** | (CDCl₃, 100 MHz), δ [ppm] = 195.2 (C=S), 137.8 (C-S-CH₃), 133.0 (CH₂-C-CH), 130.0 (C-CH-CH-C-S-CH₃, C-CH-CH-C-S-CH₃), 126.8 (C-CH-CH-C-S-CH₃, C-CH-CH-C-S-CH₃), 49.6 (CH₂-CH₃), 46.8 (CH₂-CH₃), 41.8 (S-CH₂-C), 15.9 (C-CH-CH-C-S-CH₃), 12.6 (CH₂-CH₃), 11.7 (CH₂-CH₃). | | | |
| **IR (ATR):** | v (cm⁻¹) = 2972 (w), 2929 (w), 1485 (vs), 1456 (w), 1435 (w), 1415 (vs), 1379 (w), 1351 (m), 1296 (m), 1266 (vs), 1205 (vs), 1140 (m), 1122 (w), 1093 (w), 1070 (s), 1005 (s), 983 (s), 915 (s), 825 (vs), 789 (w), 774 (w), 746 (s), 699 (w), 567 (w), 511 (vs), 494 (w), 444 (w), 413 (w). | | | |
| **MS** (ESI+): | m/z (%) = 286 (100, [M+H]⁺) | | | |
| **HRMS** (ESI+): | m/z für C₁₃H₁₉NS₃ | | | |
| | ber.: | 285.0680 | | gef.: 285.0683 |
| **EA:** | ber.: | C:54.69 | H: 6.71 | N:4.91 |
| | gef.: | C:54.90 | H:6.70 | N:4.79 |

### Darstellung von (4-Methoxyphenyl)methyl-N,N-diethylcarbamodithioat (Schl32248):

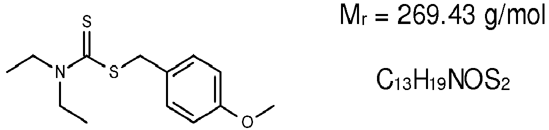

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 470 mg 1-(Chlormethyl)-4-methoxybenzen (3.0 mmol, 1.0 eq) umgesetzt. Säulenchromatographie an Kieselgel (Cyclohexan/EtOAc 9:1) ergab 728 mg von **Schl32248** (90.6%) in Form eines farblosen Feststoffes.

| | | | | |
|---|---|---|---|---|
| **DC:** | R_{f} = 0.34 (Cyclohexan/ EtOAc 6:1) | | | |
| **Fp**.: | 35°C | | | |
| **¹H-NMR:** | (CDCl₃, 400 MHz), δ [ppm] = 7.30 (*d,* ³J = 6.9 Hz, 2H, C-CH-C*H*-C-O-CH₃, C-CH-C*H*-C-O-CH₃), 6.83 (*d*, ³J = 6.6 Hz, 2H, C-C*H*-CH-C-O-CH₃, C-C*H*-CH-C-O-CH₃), 4.46 (*s,* 2H, S-C*H*₂-C), 4.02 *(q,* ³J = 6.9 Hz, 2H, CH₃-C*H*₂), 3.76 (*s,* 1H, O-C*H*₃), 3.71 (*q*, ³J = 7.1 Hz, 2H, CH₃-C*H₂*), 1.30-1.18 (*m,* 6H, CH₂-C*H*₃, CH₂-C*H*₃). | | | |
| **¹³C-NMR:** | (CDCl₃, 100 MHz), δ [ppm] = 195.4 (C=S), 159.1 (C-O-CH₃), 130.7 (C-CH-CH-C-O-CH₃, C-CH-CH-C-O-CH₃), 127.8 (CH₂-C-CH), 114.1 (C-CH-CH-C-O-CH₃, C-CH-CH-C-O-CH₃), 55.4 (O-CH₃), 49.4 (CH₂-CH₃), 46.8 (CH₂-CH₃), 41.9 (S-CH₂-C), 12.6 (CH₂-CH₃), 11.7 (CH₂-CH₃). | | | |
| **IR (ATR):** | v (cm⁻¹) = 2977 (w), 2931 (w), 1507 (w), 1485 (s), 1451 (w), 1413 (w), 1374 (s), 1344 (w), 1299 (w), 1266 (m), 1247 (vs), 1231 (w), 1208 (s), 1190 (m), 1181 (m), 1171 (w), 1140 (w), 1116 (w), 1093 (w), 1071 (w), 1031 (m), 1011 (m), 981 (m), 917 (m), 845 (w), 831 (vs), 791 (w), 752 (m), 736 (w), 699 (w), 569 (w), 542 (s), 523 (s), 501 (w), 453 (w), 424 (w). | | | |
| **MS** (ESI+): | m/z (%) = 270 (100, [M+H]⁺) | | | |
| **HRMS** (ESI+): | m/z für C₁₃H₁₉NOS₂ | | | |
| | ber.: | 269.0908 | | gef.: 269.0911 |
| **EA:** | ber.: | C:57.95 | H:7.11 | N:5.20 |
| | gef.: | C: 58.15 | H: 7.16 | N: 5.13 |

### Darstellung von [4-(Thiadiazol-4-yl)phenyl]methyl-N,N-diethylcarbamodithioat (Schl32249):

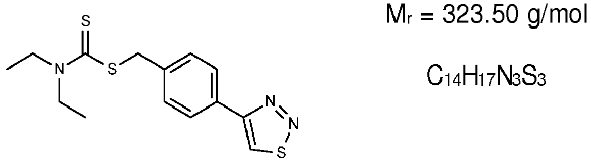

Gemäß **AAV8** wurden 0.62 mL Diethylamin (6.0 mmol, 2.0 eq), 0.43 mL Triethyl-amin (6.0 mmol, 2.0 eq), 0.36 mL Kohlenstoffdisulfid (6.0 mmol, 2.0 eq) und 765 mg 4-[4-(Brommethyl)phenyl]thiadiazol (3.0 mmol, 1.0 eq) umgesetzt. Säulenchromatographie an Kieselgel (Cyclohexan/EtOAc 6:1) ergab 500 mg von **Schl32249** (51.6%) in Form eines braunen Feststoffes.

| | | | | |
|---|---|---|---|---|
| **DC:** | R_{f} = 0.16 (Cyclohexan/ EtOAc 6:1). | | | |
| **Fp**.: | 144 °C | | | |
| **¹H-NMR:** | (DMSO-D₆, 400 MHz), δ [ppm] = 9.55 (*s,* 1H, C-C*H*-C), 8.05 (*d,* ³J = 8.5 Hz, 2H, C-CH-C*H*-C-C, C-CH-C*H*-C-C), 7.53 (*d*, ³J = 8.2 Hz, 2H, C-C*H*-CH-C-C, C-C*H*-CH-C-C), 4.57 (*s,* 2H, S-C*H*₂-C), 3.95 (*q*, ³J = 6.9 Hz, 2H, CH₃-C*H*₂), 3.71 (*q,* ³J = 7.1 Hz, 2H, CH₃-C*H*₂), 1.21-1.13 (*m,* 6H, CH₂-C*H*₃, CH₂-C*H*₃). | | | |
| **¹³C-NMR:** | (DMSO-D₆, 100 MHz), δ [ppm] = 193.8 (C=S), 162.1 (C-CH-CH-C-C), 138.5 (CH₂-C-CH), 133.7 (C-CH-S), 130.5 (C-CH-CH-C-C, C-CH-CH-C-C), 130.2 (C-CH-CH-C-C), 127.7 (C-CH-CH-C-C, C-CH-CH-C-C), 49.8 (CH₂-CH₃), 47.1 (CH₂-CH₃), 40.8 (S-CH₂-C), 13.0 (CH₂-CH₃), 11.9 (CH₂-CH₃). | | | |
| **IR (ATR):** | v (cm⁻¹) = 3076 (w), 2973 (w), 2962 (w), 1487 (s), 1453 (m), 1417 (w), 1375 (vs), 1353 (w), 1341 (w), 1297 (m), 1267 (vs), 1225 (w), 1206 (vs), 1194 (w), 1124 (s), 1093 (w), 1070 (w), 1041 (m), 1006 (w), 979 (s), 916 (s), 889 (m), 852 (m), 820 (w), 790 (vs), 773 (m), 756 (s), 709 (w), 682 (w), 636 (w), 626 (w), 592 (w), 565 (m), 541 (vs), 503 (m), 431 (m). | | | |
| **MS** (ESI+): | m/z (%) = 324 (100, [M+H]⁺) | | | |
| **HRMS** (ESI+): | m/z für C₁₄H₁₇N₃S₃ | | | |
| | ber.: | 323.0585 | | gef.: 323.0594 |
| **EA:** | ber.: | C: 51.98 | H: 5.30 | N: 12.99 |
| | gef.: | C: 51.94 | H: 5.54 | N: 12.98 |

### Darstellung von (4-Phenoxyphenyl)methyl-N,N-diethylcarbamodithioat (Schl32253):

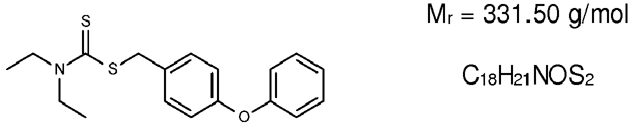

Gemäß **AAV8** wurden 0.62 mL Diethylamin (6.0 mmol, 2.0 eq), 0.43 mL Triethylamin (6.0 mmol, 2.0 eq), 0.36 mL Kohlenstoffdisulfid (6.0 mmol, 2.0 eq) und 825 mg 4-(Brommethyl)benzophenon (3.0 mmol, 1.0 eq) umgesetzt. Säulenchromatographie an Kieselgel (Cyclohexan/ EtOAc 8:1) ergab 850 mg von **Schl32253** (85.5%) in Form eines blassgelben Öls.

| | | | | |
|---|---|---|---|---|
| **DC:** | R_{f}= 0.42 (Cyclohexan/ EtOAc 6:1). | | | |
| **¹H-NMR:** | (CDCl₃, 400 MHz), δ [ppm] = 7.38-7.25 (*m,* 4H, C*H*_{Phenyl}), 7.10-6.88 (*m,* 5H, C*H*_{Phenyl}), 4.49 (*s*, 2H, S-C*H*₂-C), 4.06-3.96 (*m,* 2H, CH₃-C*H*₂), 3.77-3.64 (*m,* 2H, CH₃-C*H*₂), 1.32-1.20 (*m,* 6H, CH₂-C*H*₃, CH₂-C*H*₃). | | | |
| **¹³C-NMR:** | (CDCl₃, 100 MHz), δ [ppm] = 195.2 (C=S), 157.0 (C_{q} (_{Phenyl)}), 156.7 (C_{q (Phenyl)}), 133.6 (C_{q (Phenyl)}), 130.8 (C_{t (Phenyl)}), 129.8 (C_{t (Phenyl)}), 123.4 (C_{t (Phenyl)}), 119.0 (C_{t (Phenyl)}), 118.8 (C_{t (Phenyl)}), 49.5 (CH₂-CH₃), 46.8 (CH₂-CH₃), 41.6 (S-CH₂-C), 12.6 (CH₂-CH₃), 11.7 (CH₂-CH₃). | | | |
| **IR (ATR):** | v (cm⁻¹) = 2975 (w), 2931 (w), 1588 (m), 1504 (m), 1484 (vs), 1455 (w), 1440 (w), 1414 (s), 1353 (w), 1300 (w), 1267 (m), 1230 (vs), 1206 (vs), 1164 (m), 1140 (m), 1068 (m), 1007 (m), 982 (m), 916 (m), 869 (s), 829 (m), 748 (m), 690 (s), 564 (w), 547 (w), 504 (m), 432 (w), 411 (w). | | | |
| **MS** (EI): | m/z (%) = 331 (100, [M]⁺) | | | |
| **HRMS** (ESI+): | m/z für C₁₈H₂₁NOS₂ | | | |
| | ber.: | 331.1065 | | gef.: 331.1064 |
| **EA:** | ber.: | C: 65.22 | H: 6.39 | N: 4.23 |
| | gef.: | C: 65.38 | H: 6.43 | N: 4.15 |

### Darstellung von 2-(1-Piperidyl)ethyl-N,N-diethylcarbamodithioat (Schl32274):

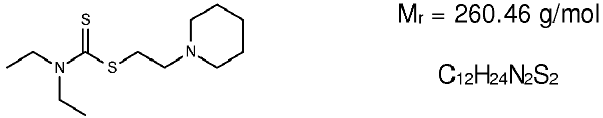

676 mg Diethylcarbamodithioat-Natrium (3.0 mmol, 1.0 eq) und 0.65 mL Triethylamin (9.0 mmol, 3.0 eq) wurden in 15 mL Acetonitril gelöst, 15 Minuten gerührt, anschließend mit 829 mg 1-(2-Chlorethyl)piperidinhydrochlorid (4.5 mmol, 1.5 eq) versetzt und 10 Stunden bei Raumtemperatur gerührt. Das Lösungsmittel wurde am Rotavapor entfernt. Säulenchromatographie an Kieselgel (Cyclohexan/ EtOAc 1:3) ergab 732 mg von **Schl32274** (93.7%) in Form eines blassgelben Öls.

| | | | | |
|---|---|---|---|---|
| **DC:** | R_{f} = 0.29 (Cyclohexan/ EtOAc 1:3). | | | |
| **¹H-NMR:** | (CDCl₃, 400 MHz), δ [ppm] = 4.00 (*q,* ³J = 7.1 Hz, 2H, CH₃-C*H*₂), 3.72 (*q,* ³J = 7.1 Hz, 2H, CH₃-C*H*₂), 3.43 (*t,* ³J = 7.6 Hz, 2H, S-C*H*₂-CH₂-N), 2.61 (*t,* ³J = 7.6 Hz, 2H, S-CH₂-C*H*₂*-*N), 2.49-2.38 (*m,* 4H, S-CH₂-CH₂-N-C*H*_{2,} S-CH₂-CH₂-N-C*H*₂), 1.60-1.51 (*m,* 4H, S-CH₂-CH₂-N-CH₂-C*H*₂, S-CH₂-CH₂-N-CH₂-C*H₂*), 1.44-1.36 (*m*, 2H, S-CH₂-CH₂-N-CH₂-CH₂-C*H*₂), 1.25 (*t,* ³J = 7.1 Hz, 6H, CH₂-C*H*₃, CH₂-C*H*₃). | | | |
| **¹³C-NMR:** | (CDCl₃, 100 MHz), δ [ppm] = 195.8 (C=S), 57.9 (S-CH₂-CH₂), 54.7 (S-CH₂-CH₂-N-CH₂-CH₂-CH₂), 54.4 (S-CH₂-CH₂-N-CH₂-CH₂-CH₂), 49.6 (CH₂-CH₃), 46.8 (CH₂-CH₃), 34.2 (S-CH₂-CH₂), 26.6 (S-CH₂-CH₂-N-CH₂-CH₂-CH₂), 26.0 (S-CH₂-CH₂-N-CH₂-CH₂-CH₂), 24.5 (S-CH₂-CH₂-N-CH₂-CH₂-CH₂), 12.5 (CH₂-CH₃), 11.7 (CH₂-CH₃). | | | |
| **IR (ATR):** | v (cm⁻¹) = 2977 (w), 2934 (s), 2854 (w), 2794 (w), 2757 (w), 2097 (w), 2003 (w), 1485 (s), 1456 (m), 1441 (m), 1414 (vs), 1378 (m), 1354 (s), 1303 (w), 1267 (vs), 1206 (vs), 1141 (s), 1117 (w), 1107 (w), 1094 (m), 1069 (m), 1040 (m), 1013 (m), 982 (vs), 917 (s), 861 (w), 832 (w), 793 (s), 776 (w), 745 (w), 592 (w), 563 (w), 498 (w), 463 (w), 430 (m). | | | |
| **MS (ESI+):** | m/z (%) = 261 (100, [M+H]⁺) | | | |
| **HRMS** (ESI+): | m/z für C₁₂H₂₄N₂S₂ | | | |
| | ber.: | 260.1381 | | gef.: 260.1398 |
| **EA:** | ber.: | C: 55.34 | H: 9.29 | N: 10.76 |
| | gef.: | C:55.14 | H: 9.24 | N: 10.58 |

### Darstellung von 2-Morpholinoethyl-N,N-diethylcarbamodithioat (Schl32275):

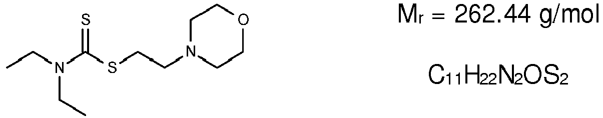

676 mg Diethylcarbamodithioat-Natrium (3.0 mmol, 1.0 eq) und 0.65 mL Triethylamin (9.0 mmol, 3.0 eq) wurden in 15 mL Acetonitril gelöst, 15 Minuten gerührt, anschließend mit 837 mg 1-(2-Chlorethyl)morpholinhydrochlorid (4.5 mmol, 1.5 eq) versetzt und 10 Stunden bei Raumtemperatur gerührt. Das Lösungsmittel wurde am Rotavapor entfernt. Säulenchromatographie an Kieselgel (Cyclohexan/ EtOAc 1:3) ergab 573 mg von **Schl32275** (72.8%) in Form eines blassgelben Öls.

| | | | | |
|---|---|---|---|---|
| **DC:** | R_{f} = 0.30 (Cyclohexan/ EtOAc 1:3). | | | |
| **¹H-NMR:** | (CDCl₃, 400 MHz), δ [ppm] = 4.00 (*q,* ³J = 7.1 Hz, 2H, CH₃-C*H*₂), 3.78-3.65 (*m,* 4H, S-CH₂-CH₂-N-CH₂-C*H*₂, S-CH₂-CH₂-N-CH₂-C*H*₂), 3.43 (*q*, ³J = 7.1 Hz, 2H, CH₃-C*H*₂), 2.72-2.63 (*m,* 2H, S-C*H*₂-CH₂-N), 2.55-2.44 (*m,* 6H, S-CH₂-C*H₂*-N, S-CH₂-CH₂-N-C*H*₂, S-CH₂-CH₂-N-C*H*₂), 1.24 (*t*, ³J = 7.1 Hz, 6H, CH₂-C*H*₃, CH₂-C*H*₃). | | | |
| **¹³C-NMR:** | (CDCl₃, 100 MHz), δ [ppm] = 195.5 (C=S), 67.0 (S-CH₂-CH₂-N-CH₂-CH₂-O), 66.9 (S-CH₂-CH₂-N-CH₂-CH₂-O), 57.5 (S-CH₂-CH₂-N-CH₂-CH₂-O, S-CH₂-CH₂-N-CH₂-CH₂-O), 53.5 (S-CH₂-CH₂), 49.6 (CH₂-CH₃), 46.8 (CH₂-CH₃), 33.9 (S-CH₂-CH₂), 12.6 (CH₂-CH₃), 11.7 (CH₂-CH₃). | | | |
| **IR (ATR):** | v (cm⁻¹) = 2962 (w), 2935 (w), 2856 (w), 2808 (w), 2103 (w), 1486 (m), 1455 (m), 1442 (m), 1416 (s), 1379 (w), 1355 (m), 1302 (m), 1267 (vs), 1205 (s), 1140 (s), 1115 (vs), 1094 (w), 1069 (m), 1034 (w), 1003 (m), 981 (s), 915 (s), 866 (w), 832 (m), 808 (m), 775 (w), 751 (w), 666 (w), 624 (w), 609 (w), 594 (w), 564 (w), 495 (w), 432 (w), 410 (w). | | | |
| **MS** (ESI+): | m/z (%) = 263 (100, [M+H]⁺) | | | |
| **HRMS** (ESI+): | m/z für C₁₁H₂₂N₂OS₂ | | | |
| | ber.: | 262.1174 | | gef.: 262.1198 |
| **EA:** | ber.: | C:50.34 | H: 8.45 | N: 10.67 |
| | gef.: | C:49.93 | H: 8.45 | N: 10.63 |

### Darstellung von Benzylimidazol-1-carbodithioat (Schl32027):

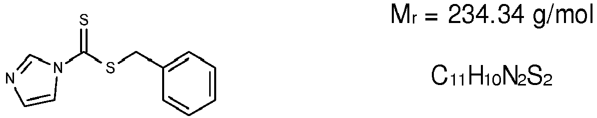

Gemäß **AAV9** wurden 170 mg Imidazol (2.5 mmol, 1.0 eq), 531 mg Kaliumphosphat (2.5 mmol, 1.0 eq), 0.45 mL Kohlenstoffdisulfid (7.5 mmol, 3.0 eq) und 0.30 mL Benzylbromid (2.5 mmol, 1.0 eq) umgesetzt. Säulenchromatographie an Kieselgel (Pentan/ EtOAc 5:1) ergab 202 mg von **Schl32027** (34.5%) in Form eines gelbbraunen Öls.

| | | | | |
|---|---|---|---|---|
| **DC:** | R_{f} = 0.19 (Cyclohexan/ EtOAc 4:2) | | | |
| **¹H-NMR:** | (CDCl₃, 400 MHz), δ [ppm] = 8.57 (s, 1H, N-CH=N), 7.76-7.71 (*m,* 1H, N-CH=C*H*-N), 7.42-7.29 (*m,* 5H, C*H*_{(Benzyl)}), 7.08-7.03 (*m,* 1H, N-CH=C*H*-N). | | | |
| **¹³C-NMR:** | (CDCl₃, 100 MHz), δ [ppm] = 197.0 (C=S), 133.4 (S-CH₂-C), 130.2 (N-CH=N-CH-CH), 129.5 (C-CH-CH), 129.1 (C-CH-CH), 128.5 (C-CH-CH-CH), 117.6 (N-CH=N-CH-CH), 42.0 (S-CH₂-C). | | | |
| **IR (ATR):** | v (cm⁻¹) = 3124 (w), 3024 (w), 2912 (w), 1669 (w), 1578 (w), 1541 (m), 1495 (w), 1479 (w), 1448 (m), 1389 (w), 1367 (w), 1325 (m), 1261 (w), 1243 (w), 1180 (w), 1147 (w), 1099 (w), 1056 (s), 933 (s), 894 (m), 839 (s), 826 (vs), 750 (vs), 737 (vs), 698 (m), 656 (vs), 618 (vs), 475 (w). | | | |
| **MS** (ESI+): | m/z (%) = 235 (100, [M+H]⁺), 257 (55, [M+Na]⁺) | | | |
| **HRMS** (ESI+): | m/z für C₁₁H₁₀N₂S₂ | | | |
| | ber.: | 234.0285 | | gef.: 234.0295 |
| **EA:** | ber.: | C: 56.38 | H: 4.30 | N: 11.95 |
| | gef.: | C: 56.41 | H:4.57 | N: 11.82 |

### Darstellung von Methylimidazol-1-carbodithioat (Schl32028):

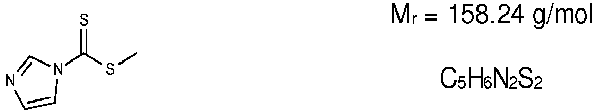

Gemäß **AAV9** wurden 204 mg Imidazol (3.0 mmol, 1.0 eq), 637 mg Kaliumphosphat (3.0 mmol, 1.0 eq), 0.55 mL Kohlenstoffdisulfid (9.0 mmol, 3.0 eq) und 0.19 mL Methyliodid (3.0 mmol, 1.0 eq) umgesetzt. Säulenchromatographie an Kieselgel (Pentan/ EtOAc 70:30) ergab 270 mg von **Schl32028** (68.3%) in Form eines gelbbraunen Öls.

| | | | | |
|---|---|---|---|---|
| **DC:** | R_{f} = 0.20 (Cyclohexan/ EtOAc 3:1). | | | |
| **¹H-NMR:** | (CDCl₃, 400 MHz), δ [ppm] = 8.55 (*s,* 1H, N-C*H*=N), 7.87-7.81 (*m,* 1H, N-C*H*=CH-N), 7.18-7.14 (*m,* 1H, N-CH=C*H*-N), 2.80 (*s,* 3H, S-C*H*₃). | | | |
| **¹³C-NMR:** | (CDCl₃, 100 MHz), δ [ppm] = 198.9 (C=S), 131.2 (N-CH=N-CH-CH), 117.6 (N-CH=N-CH-CH), 20.0 (S-CH₃). | | | |
| **IR (ATR):** | v (cm⁻¹) = 3119 (w), 3027 (w), 2852 (w), 1600 (w), 1529 (w), 1494 (w), 1470 (m), 1453 (m), 1366 (m), 1323 (w), 1273 (s), 1239 (s), 1220 (vs), 1132 (w), 1107 (m), 1092 (m), 1056 (s), 1003 (s), 969 (m), 925 (w), 925 (w), 890 (w), 832 (vs), 745 (m), 709 (s), 696 (s), 662 (s), 642 (s), 614 (m), 561 (m), 536 (m), 517 (m), 482 (m), 437 (m), 411 (m). | | | |
| **MS** (ESI+): | m/z (%) = 159 (100, [M+H]⁺), 181 (55, [M+Na]⁺) | | | |
| **HRMS** (ESI+): | m/z für C₅H₆N₂S₂ | | | |
| | ber.: | 157.9972 | | gef.: 157.9979 |
| **EA:** | ber.: | C: 37.95 | H: 3.82 | N: 17.70 |
| | gef.: | C:38.15 | H:4.03 | N: 17.32 |

### Darstellung von Methyl-2-(imidazol-1-carbothioylsulfanyl)acetat (Schl32031):

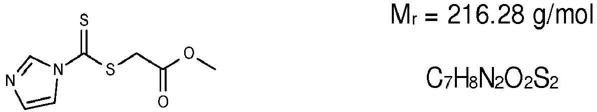

Gemäß **AAV9** wurden 170 mg Imidazol (2.5 mmol, 1.0 eq), 531 mg Kaliumphosphat (2.5 mmol, 1.0 eq), 0.45 mL Kohlenstoffdisulfid (7.5 mmol, 3.0 eq) und und 0.23 mL Methyl-2-bromacetat (2.5 mmol, 1.0 eq) umgesetzt. Säulenchromatographie an Kieselgel (DCM/ MeOH 90:10) ergab 267 mg von **Schl32031** (49.4%) in Form eines dunkelbraunen Öls.

| | | | | |
|---|---|---|---|---|
| **DC:** | R_{f}= 0.23 (DCM/ MeOH 95:5). | | | |
| **¹H-NMR:** | (CDCl₃, 400 MHz), δ [ppm] = 8.47 (*s,* 1H, N-C*H*=N), 7.77 (*m,* 1H, N-C*H*=CH-N), 7.11 (*m,* 1H, N-CH=C*H*-N), 4.19 (*s,* 2H, S-C*H*₂-COOMe), 3.76 (*s,* 3H, O-C*H*₃). | | | |
| **¹³C-NMR:** | (CDCl₃, 100 MHz), δ [ppm] = 196.2 (C=S), 167.1 (C=O), 131.9 (N-CH=N-CH-CH), 118.2 (N-CH=N-CH-CH), 53.3 (O-CH₃), 38.5 (S-CH₂-COOMe). | | | |
| **IR (ATR):** | v (cm⁻¹) = 3120 (w), 3000 (w), 2950 (w), 2571 (w), 1972 (w), 1730 (m), 1677 (m), 1583 (s), 1434 (s), 1368 (s), 1323 (m), 1253 (s), 1193 (s), 1127 (m), 1062 (s), 1007 (m), 932 (w), 827 (m), 756 (s), 697 (m), 661 (m), 636 (m), 480 (w). | | | |
| **MS** (ESI+): | m/z (%) = 217 (100, [M+H]⁺) | | | |
| **HRMS** (ESI+): | m/z für C₁₁H₁₀N₂O₂S₂ | | | |
| | ber.: | 216.0027 | | gef.: 216.0020 |
| **EA:** | ber.: | C: 38.87 | H: 3.73 | N: 12.95 |
| | gef.: | C:38.62 | H:4.08 | N: 12.46 |

### Darstellung von 2-(1,3-Dioxan-2-yl)ethylimidazol-1-carbodithioat (Schl32032):

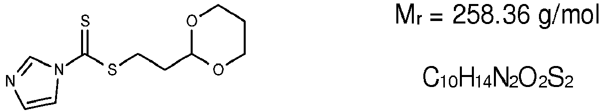

Gemäß **AAV9** wurden 170 mg Imidazol (2.5 mmol, 1.0 eq), 531 mg Kaliumphosphat (2.5 mmol, 1.0 eq), 0.45 mL Kohlenstoffdisulfid (7.5 mmol, 3.0 eq) und 0.34 mL 2-(2-Bromethyl)-1,3-dioxan (2.5 mmol, 1.0 eq) umgesetzt. Säulenchromatographie an Kieselgel (DCM/ MeOH 90:10) ergab 438 mg von **Schl32032** (67.8%) in Form eines gelben Feststoffes.

| | | | | |
|---|---|---|---|---|
| **DC:** | R_{f} = 0.11 (Cyclohexan/EtOAc 9:2). | | | |
| **Fp**.: | 43°C | | | |
| **¹H-NMR:** | (CDCl₃, 400 MHz), δ [ppm] = 8.51 (*s,* 1H, N-C*H*=N), 7.80 (*m,* 1H, N-C*H*=CH-N), 7.12 (*m,* 1H, N-CH=C*H*-N), 4.65 (*t*, ³J=4.9 Hz, 1H, CH₂-C*H*-O), 4.13-4.07 (*m,* 2H, CH-O-C*H*H-CH₂, CH-O-C*H*H-CH₂), 3.79-3.72 (*m,* 2H, CH-O-C*H*H-CH₂, CH-O-C*H*H-CH₂), 3.49-3.44 (*m,* 2H, S-C*H*₂-CH₂), 2.13-2.08 (*m,* 1H, O-CH₂-C*H*H-CH₂-O), 2.07-2.00 (*m,* 2H, S-CH₂-C*H*₂), 1.38-1.31 (*m*, 1 H, O-CH₂-C*H*H-CH₂-O). | | | |
| **¹³C-NMR:** | (CDCl₃, 100 MHz), δ [ppm] = 198.1 (C=S), 131.2 (N-CH=N-CH-CH), 118.0 (N-CH=N-CH-CH), 100.3 (CH₂-CH-O), 67.0 (O-CH₂-CH₂), 32.8 (S-CH₂), 31.6 (S-CH₂-CH₂), 25.6 (O-CH₂-CH₂-CH₂). | | | |
| **IR (ATR):** | v (cm⁻¹) = 3101 (w), 2963 (w), 1620 (w), 1525 (w), 1466 (w), 1456 (w), 1436 (w), 1402 (w), 1368 (m), 1323 (m), 1291 (m), 1273 (m), 1255 (m), 1227 (m), 1218 (m), 1140 (m), 1121 (m), 1110 (s), 1089 (m), 1062 (s), 1046 (s), 1026 (s), 998 (vs), 942 (m), 925 (m), 878 (m), 830 (vs), 779 (m), 745 (s), 639 (s), 611 (m), 501 (w), 487 (w), 472 (w), 401 (s). | | | |
| **MS** (ESI+): | m/z (%) = 259 (100, [M+H]⁺) | | | |
| **HRMS** (ESI+): | m/z für C₁₀H₁₄N₂O₂S₂ | | | |
| | ber.: | 258.0497 | | gef.: 258.0519 |
| **EA:** | ber.: | C: 46.49 | H: 5.46 | N: 10.84 |
| | gef.: | C: 46.53 | H: 5.49 | N: 10.85 |

### Darstellung von (2-Nitrophenyl)methylimidazol-1-carbodithioat (Schl32090):

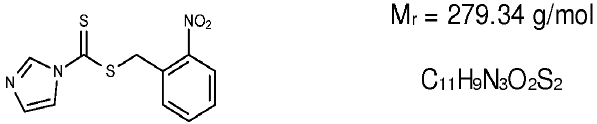

Gemäß **AAV9** wurden 408 mg Imidazol (6.0 mmol, 1.0 eq), 1272 mg Kaliumphosphat (6.0 mmol, 1.0 eq), 1.08 mL Kohlenstoffdisulfid (18.0 mmol, 3.0 eq) und 1296 mg 2-Nitrobenzylbromid (6.0 mmol, 1.0 eq) umgesetzt. Säulenchromatographie an Kieselgel (EtOAc) ergab 630 mg von **Schl32090** (42.7%) in Form eines gelben Öls.

| | | | | |
|---|---|---|---|---|
| **DC:** | R_{f}= 0.51 (EtOAc). | | | |
| **¹H-NMR:** | (CDCl₃, 400 MHz), δ [ppm] = 8.41 (*s,* 1H, N-C*H*=N), 8.08 (*d,* ³J = 8.2 Hz, 1H, C-C(NO₂)-C*H*), 7.74 (*d*, ³J = 8.2 Hz, 1H, C-C*H*-CH-CH-CH-C(NO₂)), 7.71-7.69 (*m*, 1H, N-C*H*=CH-N), 7.59 (*t,* ³J = 7.6 Hz, 1H, C-CH-C*H-*CH-CH-C(NO₂)), 7.48 (*t*, ³J = 7.3 Hz, 1H, C-CH-CH-C*H*-CH-C(NO₂)), 7.08-7.03 (*m,* 1H, N-CH=C*H*-N), 4.99 (*s,* 2H, S-C*H*₂-C). | | | |
| **¹³C-NMR:** | (CDCl₃, 100 MHz), δ [ppm] = 197.2 (C=S), 148.3 (C-NO₂), 134.0 (C(NO₂)-CH), 133.0 (C-CH-CH-CH-CH), 131.8 (N-CH=N-CH-CH), 130.7 (S-CH₂-C), 129.6 (C-CH-CH-CH-CH), 125.7 (C-CH-CH-CH-CH), 117.8 (N-CH=N-CH-CH), 38.5 (S-CH₂-C). | | | |
| **IR (ATR):** | v (cm⁻¹) = 3122 (w), 2857 (w), 2139 (w), 1845 (w), 1675 (w), 1608 (w), 1577 (w), 1519 (vs), 1464 (s), 1417 (w), 1366 (s), 1340 (s), 1267 (vs), 1237 (s), 1216 (vs), 1101 (m), 1087 (s), 1040 (s), 999 (s), 889 (m), 845 (m), 824 (s), 807 (m), 785 (m), 746 (m), 707 (s), 662 (m), 639 (s), 611 (m), 581 (m), 534 (m), 484 (m). | | | |
| **MS** (ESI+): | m/z (%) = 280 (100, [M+H]⁺) | | | |
| **HRMS** (ESI+): | m/z für C₁₁H₉N₃O₂S₂ | | | |
| | ber.: | 279.0136 | | gef.: 279.0177 |
| **EA:** | ber.: | C: 47.30 | H: 3.25 | N: 15.04 |
| | gef.: | C: 47.06 | H: 3.41 | N: 14.70 |

### Darstellung von (3-Nitrophenyl)methylimidazol-1-carbodithioat (Schl32092):

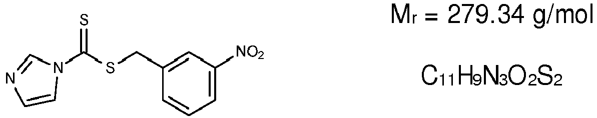

Gemäß **AAV9** wurden 408 mg Imidazol (6.0 mmol, 1.0 eq), 1272 mg Kaliumphosphat (6.0 mmol, 1.0 eq), 1.08 mL Kohlenstoffdisulfid (18.0 mmol, 3.0 eq) und 1296 mg 3-Nitrobenzylbromid (6.0 mmol, 1.0 eq) umgesetzt. Säulenchromatographie an Kieselgel (Cyclohexan/ EtOAc 1:1) ergab 364 mg von **Schl32092** (21.7%) in Form eines gelben Feststoffes.

| | | | | |
|---|---|---|---|---|
| **DC:** | R_{f} = 0.19 (Cyclohexan/ EtOAc 1:1). | | | |
| **Fp**.: | 98°C | | | |
| **¹H-NMR:** | (CDCl₃, 400 MHz), δ [ppm] = 8.46 (*s*, 1H, N-C*H*=N), 8.27 (*s*, 1H, C-C*H-*C(NO₂)), 8.17 (*d,* ³J = 8.2 Hz, 1H, C-CH-CH-C*H-*C(NO₂)), 7.78-7.76 (*m*, 1 H, N-C*H*=CH-N), 7.75-7.72 (*m,* 1 H, C-C*H*-CH-CH-C), 7.54 (*t*, ³J = 7.6 Hz, 1 H, C-CH-C*H*-CH-C(NO₂)), 7.13-7.09 (*m,* 1 H, N-CH=C*H*-N), 4.71 (s, 2H, S-C*H*₂-C). | | | |
| **¹³C-NMR:** | (CDCl₃, 100 MHz), δ [ppm] = 196.1 (C=S), 148.5 (C-NO₂), 136.5 (S-CH₂-C), 135.5 (C-CH-CH-CH-C), 131.9 (N-CH=N-CH-CH), 129.9 (C-CH-CH-CH-C), 124.4 (C-CH-C), 123.3 (C-CH-CH-CH-C), 117.9 (N-CH=N-CH-CH), 40.1 (S-CH₂-C). | | | |
| **IR (ATR):** | v (cm⁻¹) = 3743 (w), 3152 (w), 3133 (w), 3064 (w), 2916 (w), 1665 (w), 1581 (w), 1520 (s), 1477 (w), 1464 (w), 1366 (vs), 1317 (m), 1295 (m), 1273 (vs), 1219 (vs), 1102 (s), 1050 (vs), 1010 (m), 920 (w), 888 (w), 870 (w), 835 (s), 806 (s), 754 (w), 713 (s), 681 (m), 670 (m), 640 (s), 604 (w), 587 (w), 539 (w), 529 (w), 492 (w), 420 (w), 400 (m). | | | |
| **MS** (ESI+): | m/z (%) = 280 (100, [M+H]⁺) | | | |
| **HRMS** (ESI+): | m/z für C₁₁H₉N₃O₂S₂ | | | |
| | ber.: | 279.0136 | | gef.: 279.0153 |
| **EA:** | ber.: | C: 47.30 | H: 3.25 | N: 15.04 |
| | gef.: | C: 47.15 | H: 3.34 | N: 14.50 |

### Darstellung von (4-Nitrophenyl)methylimidazol-1-carbodithioat (Schl32094):

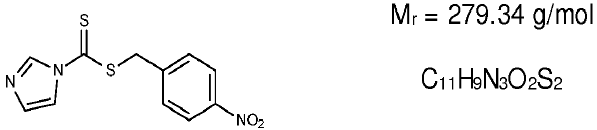

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 648 mg 4-Nitrobenzylbromid (3.0 mmol, 1.0 eq) umgesetzt. Säulenchromatographie an Kieselgel (Cyclohexan/ EtOAc 2:1) ergab 369 mg von **Schl32094** (44.0%) in Form eines orangen Feststoffes.

| | | | | |
|---|---|---|---|---|
| **DC:** | R_{f} = 0.10 (Cyclohexan/ EtOAc 2:1). | | | |
| **Fp**.: | 87°C | | | |
| **¹H-NMR:** | (CDCl₃, 400 MHz), δ [ppm] = 8.53 (*s,* 1H, N-C*H*=N), 8.21 (*d*, ³J = 8.5 Hz, 2H, CH₂-C-CH-C*H*-C(NO₂), CH₂-C-CH-C*H-*C(NO₂)), 7.79-7.77 (*m,* 1H, N-C*H*=CH-N), 7.58 (*d,* ³J = 8.5 Hz, 2H, CH₂-C-C*H*-CH-C(NO₂), CH₂-C-C*H-*CH-C(NO₂)), 7.16-7.13 (*m,* 1H, N-CH=C*H*-N), 4.71 (*s,* 2H, S-C*H*₂-C). | | | |
| **¹³C-NMR:** | (CDCl₃, 100 MHz), δ [ppm] = 194.9 (C=S), 146.9 (C-NO₂), 143.4 (S-CH₂-C), 130.2 (N-CH=N-CH-CH), 129.5 (C-CH-CH), 124.1 (C-CH-CH), 117.6 (N-CH=N-CH-CH), 40.2 (S-CH₂-C). | | | |
| **IR (ATR):** | v (cm⁻¹) = 3469 (w), 3136 (w), 3118 (w), 2923 (w), 1594 (w), 1491 (m), 1460 (m), 1416 (m), 1389 (m), 1368 (m), 1343 (m), 1310 (m), 1295 (m), 1266 (s), 1223 (vs), 1167 (w), 1103 (w), 1086 (w), 1069 (w), 1046 (s), 1028 (m), 991 (s), 905 (w), 887 (w), 974 (w), 858 (w), 823 (s), 801 (w), 777 (m), 758 (m), 734 (w), 704 (s), 639 (s), 569 (w), 483 (w), 411 (w). | | | |
| **MS** (ESI+): | m/z (%) = 280 (100, [M+H]⁺) | | | |
| **HRMS** (ESI+): | m/z für C₁₁H₉N₃O₂S₂ | | | |
| | ber.: | 279.0136 | | gef.: 279.0129 |
| **EA:** | ber.: | C: 47.30 | H: 3.25 | N: 15.04 |
| | gef.: | C:46.92 | H: 3.29 | N: 14.45 |

### Darstellung von Phenethylimidazol-1-carbodithioat (Schl32103):

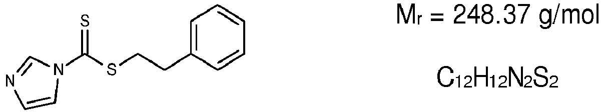

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 0.41 mL Phenethylbromid (3.0 mmol, 1.0 eq) umgesetzt. Säulenchromatographie an Kieselgel (Cyclohexan/EtOAc 2:1) ergab 497 mg von **Schl32103** (66.7%) in Form eines gelben Öls.

| | | | | |
|---|---|---|---|---|
| **DC:** | R_{f} = 0.35 (Cyclohexan/ EtOAc 4:2). | | | |
| **¹H-NMR:** | (CDCl₃, 400 MHz), δ [ppm] = 8.48 (*s,* 1H, N-CH=N), 7.79-7.77 (*m,* 1H, N-C*H*=CH-N), 7.37-7.24 (*m,* 5H, C*H*_{(Phenyl)}), 7.11-7.09 (*m*, 1H, N-CH=C*H*-N), 3.64 (*t*, ³J = 7.7 Hz, 2H, S-C*H₂*-CH₂-C), 3.08 (*t,* ³J = 7.7 Hz, 2H, S-CH₂-C*H₂*-C). | | | |
| **¹³C-NMR:** | (CDCl₃, 100 MHz), δ [ppm] = 197.9 (C=S), 139.0 (S-CH₂-CH₂-C), 131.5 (N-CH=N-CH-CH), 128.9 (C-CH-CH), 128.7 (C-CH-CH), 127.1 (N-CH=N-CH-CH), 127.0 (C-CH-CH-CH), 117.9 (N-CH=N-CH-CH), 38.1 (S-CH₂-CH₂), 33.8 (S-CH₂-CH₂). | | | |
| **IR (ATR):** | v (cm⁻¹) = 3119 (w), 3061 (w), 3026 (w), 2918 (w), 1584 (w), 1527 (w), 1495 (w), 1464 (m), 1454 (m), 1395 (m), 1366 (vs), 1267 (vs), 1239 (vs), 1215 (vs), 1101 (m), 1087 (s), 1042 (vs), 1001 (vs), 914 (w), 888 (w), 828 (vs), 728 (s), 695 (vs), 640 (vs), 611 (m), 535 (m), 507 (m), 490 (m). | | | |
| **MS** (ESI+): | m/z (%) = 249 (100, [M+H]⁺) | | | |
| **HRMS** (ESI+): | m/z für C₁₂H₁₂N₂S₂ | | | |
| | ber.: | 248.0442 | | gef.: 248.0449 |
| **EA:** | ber.: | C: 58.03 | H: 4.87 | N: 11.28 |
| | gef.: | C: 57.80 | H: 4.99 | N: 11.51 |

### Darstellung von 3-Phenylpropylimidazol-1-carbodithioat (Schl32105):

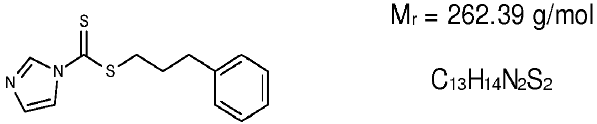

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 0.46 mL 1-Brom-3-phenylpropan (3.0 mmol, 1.0 eq) umgesetzt. Säulenchromatographie an Kieselgel (Cyclohexan/EtOAc 5:1) ergab 511 mg von **Schl32105** (64.9%) in Form eines gelben Öls.

| | | | | |
|---|---|---|---|---|
| **DC:** | R_{f} = 0.15 (Cyclohexan/ EtOAc 4:2). | | | |
| **¹H-NMR:** | (CDCl₃, 400 MHz), δ [ppm] = 8.48 (s, 1H, N-C*H*=N), 7.80-7.78 (*m,* 1H, N-C*H*=CH-N), 7.35-7.14 (*m,* 5H, C*H*_{(Phenyl)}), 7.13-7.08 (*m,* 1H, N-CH=C*H*-N), 3.39 (*t*, ³J = 7.5 Hz, 2H, S-C*H₂*-CH₂-CH₂-C), 2.88 *(t,* ³J = 7.5 Hz, 2H, S-CH₂-CH₂-C*H*₂-C), 2.16-2.07 (m*,* 2H, S-CH₂-C*H*₂-CH₂-C). | | | |
| **¹³C-NMR:** | (CDCl₃, 100 MHz), δ [ppm] = 195.1 (C=S), 140.5 (S-CH₂-CH₂-C), 131.5 (N-CH=N-CH-CH), 128.7 (C-CH-CH), 128.6 (C-CH-CH), 126.4 (N-CH=N-CH-CH), 126.4 (C-CH-CH-CH), 117.9 (N-CH=N-CH-CH), 36.2 (S-CH₂-CH₂-CH₂), 36.1 (S-CH₂-CH₂-CH₂), 35.0 (S-CH₂-CH₂-CH₂). | | | |
| **IR (ATR):** | v (cm⁻¹) = 3120 (w), 3084 (w), 3061 (w), 3025 (w), 2921 (w), 1527 (w), 1495 (w), 1464 (s), 1454 (m), 1397 (w), 1366 (vs), 1268 (vs), 1237 (vs), 1216 (vs), 1100 (m), 1088 (s), 1043 (vs), 1001 (vs), 909 (w), 889 (w), 828 (vs), 741 (vs), 697 (vs), 640 (vs), 611 (m), 591 (m), 535 (m), 490 (m). | | | |
| **MS** (ESI+): | m/z (%) = 263 (100, [M+H]⁺) | | | |
| **HRMS** (ESI+): | m/z für C₁₃H₁₄N₂S₂ | | | |
| | ber.: | 262.0598 | | gef.: 262.0569 |
| **EA:** | ber.: | C: 59.51 | H: 5.38 | N: 10.68 |
| | gef.: | C: 59.73 | H: 5.36 | N: 10.71 |

### Darstellung von 4-Phenylbutylimidazol-1-carbodithioat (Schl32106):

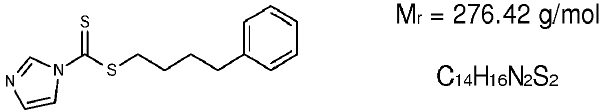

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 639 mg 1-Brom-4-phenylbutan (3.0 mmol, 1.0 eq) umgesetzt. Säulenchromatographie an Kieselgel (Cyclohexan/EtOAc 4:1) ergab 569 mg von **Schl32106** (68.6%) in Form eines gelben Öls.

| | | | | |
|---|---|---|---|---|
| **DC:** | R_{f} = 0.14 (Cyclohexan/ EtOAc 4:1). | | | |
| **¹H-NMR:** | (CDCl₃, 400 MHz), δ [ppm] = 8.47 (*s,* 1H, N-CH=N), 7.77-7.71 (*m,* 1H, N-C*H*=CH-N), 7.31-7.10 (*m,* 5H, C*H*_{(Phenyl)}), 7.10-7.05 (*m,* 1H, N-CH=C*H*-N), 3.36 (*t*, ³J = 7.1 Hz, 2H, S-C*H*₂-CH₂-CH₂-CH₂-C), 2.66 (*t,* ³J = 7.5 Hz, 2H, S-CH₂-CH₂-CH₂-C*H*₂-C), 1.78 (*quin,* ³J = 7.6 Hz, 4H, S-CH₂-C*H₂*-C*H*₂-CH₂-C). | | | |
| **¹³C-NMR:** | (CDCl₃, 100 MHz), δ [ppm] = 198.2 (C=S), 141.7 (S-CH₂-CH₂-CH₂-CH₂-C), 135.7 (N-CH=N-CH-CH), 131.5 (N-CH=N-CH-CH), 128.6 (C-CH-CH, C-CH-CH), 128.5 (C-CH-CH, C-CH-CH), 126.1 (C-CH-CH-CH), 117.9 (N-CH=N-CH-CH), 36.9 (S-CH₂-CH₂-CH₂-CH₂), 35.4 (S-CH₂-CH₂-CH₂-CH₂), 30.7 (S-CH₂-CH₂-CH₂-CH₂), 27.1 (S-CH₂-CH₂-CH₂-CH₂). | | | |
| **IR (ATR):** | v (cm⁻¹) = 3119 (w), 3083 (w), 3060 (w), 3024 (w), 2931 (w), 1527 (w), 1494 (w), 1463 (m), 1397 (w), 1365 (s), 1267 (vs), 1236 (s), 1215 (vs), 1100 (m), 1088 (s), 1042 (s), 1001 (vs), 909 (w), 889 (w), 827 (vs), 740 (s), 696 (vs), 640 (vs), 611 (w), 585 (w), 569 (w), 535 (w), 493 (w). | | | |
| **MS** (ESI+): | m/z (%) = 277 (100, [M+H]⁺) | | | |
| **HRMS** (ESI+): | m/z für C₁₄H₁₆N₂S₂ | | | |
| | ber.: | 276.0755 | | gef.: 276.0777 |
| **EA:** | ber.: | C: 60.83 | H: 5.83 | N: 10.13 |
| | gef.: | C: 60.75 | H: 5.79 | N: 10.14 |

### Darstellung von 5-Phenylpentylimidazol-1-carbodithioat (Schl32109):

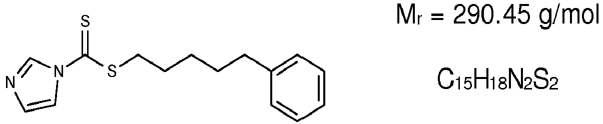

Gemäß **AAV9** wurden 150 mg Imidazol (2.2 mmol, 1.0 eq), 467 mg Kaliumphosphat (2.2 mmol, 1.0 eq), 0.40 mL Kohlenstoffdisulfid (6.6 mmol, 3.0 eq) und 0.41 mL 1-Brom-5-phenylpentan (2.2 mmol, 1.0 eq) umgesetzt. Säulenchromatographie an Kieselgel (Cyclohexan/EtOAc 4:1) ergab 355 mg von **Schl32109** (55.6%) in Form eines gelben Öls.

| | | | | |
|---|---|---|---|---|
| **DC:** | R_{f} = 0.26 (Cyclohexan/ EtOAc 4:1). | | | |
| **¹H-NMR:** | (CDCl₃, 400 MHz), δ [ppm] = 8.47 (s, 1H, N-C*H*=N), 7.82-7.74 (*m*, 1H, N-C*H*=CH-N), 7.33-7.14 (*m,* 5H, C*H*_{(Phenyl)}), 7.11-7.07 (*m*, 1H, N-CH=C*H*-N), 3.35 (*t,* ³J = 7.5 Hz, 2H, S-C*H₂*-CH₂-CH₂-CH₂-CH₂-C), 2.63 (*t,* ³J = 7.5 Hz, 2H, S-CH₂-CH₂-CH₂-CH₂-C*H*₂-C), 1.79 (*quin,* ³J = 7.6 Hz, 2H, S-CH₂-C*H₂*-CH₂-CH₂-CH₂-C), 1.68 (*quin,* ³J = 7.4 Hz, 2H, S-CH₂-CH₂-CH₂-C*H₂*-CH₂-C), 1.48 (*quin,* ³J = 7.7 Hz, 2H, S-CH₂-CH₂-C*H₂-*CH₂-CH₂-C). | | | |
| **¹³C-NMR:** | (CDCl₃, 100 MHz), δ [ppm] = 198.3 (C=S), 142.2 (S-CH₂-CH₂-CH₂-CH₂-CH₂-C), 135.7 (N-CH=N-CH-CH), 131.6 (N-CH=N-CH-CH), 128.6 (C-CH-CH, C-CH-CH), 128.5 (C-CH-CH, C-CH-CH), 125.9 (C-CH-CH-CH), 117.9 (N-CH=N-CH-CH), 37.2 (S-CH₂-CH₂-CH₂-CH₂-CH₂), 35.8 (S-CH₂-CH₂-CH₂-CH₂-CH₂), 31.0 (S-CH₂-CH₂-CH₂-CH₂-CH₂), 28.6 (S-CH₂-CH₂-CH₂-CH₂-CH₂), 27.3 (S-CH₂-CH₂-CH₂-CH₂-CH₂). | | | |
| **IR (ATR):** | v (cm⁻¹) = 3120 (w), 3084 (w), 3060 (w), 3023 (w), 2929 (w), 2854 (w), 1603 (w), 1527 (w), 1494 (w), 1463 (s), 1397 (w), 1366 (vs), 1268 (vs), 1237 (vs), 1216 (vs), 1089 (s), 1041 (vs), 1002 (vs), 889 (m), 829 (vs), 743 (s), 697 (vs), 641 (vs), 611 (m), 585 (m), 570 (m), 535 (m), 495 (m). | | | |
| **MS** (ESI+): | m/z (%) = 291 (100, [M+H]⁺) | | | |
| **HRMS** (ESI+): | m/z für C₁₅H₁₈N₂S₂ | | | |
| | ber.: | 290.0911 | | gef.: 262.0888 |
| **EA:** | ber.: | C: 62.03 | H: 6.25 | N: 9.64 |
| | gef.: | C: 62.33 | H: 6.31 | N: 9.67 |

### Darstellung von 2-(1,3-Dioxolan-2-yl)ethylimidazol-1-carbodithioat (Schl32117):

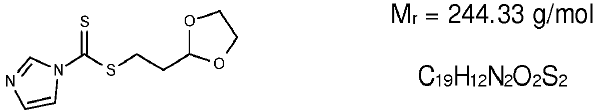

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 0.36 mL 2-(2-Bromethyl)-1,3-dioxolan (3.0 mmol, 1.0 eq) umgesetzt. Säulenchromatographie an Kieselgel (EtOAc) ergab 396 mg von **Schl32117** (54.0%) in Form eines gelben Öls.

| | | | | |
|---|---|---|---|---|
| **DC:** | R_{f}= 0.36 (EtOAc). | | | |
| **¹H-NMR:** | (CDCl₃, 400 MHz), δ [ppm] = 8.42 (*s,* 1H, N-C*H*=N), 7.77-7.69 (*m,* 1H, N-C*H*=CH-N), 7.08-7.02 (*m,* 1H, N-CH=C*H*-N), 4.95 (*t,* ³J = 4.1 Hz, 1H, S-CH₂-CH₂-C*H*-O), 3.98-3.82 (*m,* 4H, O*-*C*H₂-*C*H₂-*O), 3.43 *(t,* ³J = 6.8 Hz, 2H, S-C*H*₂-CH₂-CH-O), 2.15-2.08 *(m,* 2H, S-CH₂-C*H*₂-CH-O). | | | |
| **¹³C-NMR:** | (CDCl₃, 100 MHz), δ [ppm] = 198.1 (C=S), 135.7 (N-CH=N-CH-CH), 131.5 (N-CH=N-CH-CH), 117.8 (N-CH=N-CH-CH), 102.7 (S-CH₂-CH₂-CH-O), 65.2 (O-CH₂-CH₂-O), 31.5 (S-CH₂-CH₂-CH), 31.1 (S-CH₂-CH₂-CH). | | | |
| **IR (ATR):** | v (cm⁻¹) = 3119 (w), 2953 (w), 2881 (w), 2767 (w), 2116 (w), 1527 (w), 1464 (m), 1403 (s), 1366 (s), 1268 (vs), 1239 (s), 1216 (vs), 1178 (w), 1130 (s), 1102 (s), 1087 (vs), 1042 (m), 1000 (vs), 942 (m), 887 (m), 827 (vs), 722 (m), 640 (vs), 611 (m), 535 (m), 483 (m), 452 (m). | | | |
| **MS** (ESI+): | m/z (%) = 245 (100, [M+H]⁺) | | | |
| **HRMS** (ESI+): | m/z für C₁₉H₁₂N₂O₂S₂ | | | |
| | ber.: | 244.0340 | | gef.: 244.0346 |
| **EA:** | ber.: | C: 44.24 | H: 4.95 | N: 11.47 |
| | gef.: | C: 44.07 | H: 5.01 | N: 11.37 |

### Darstellung von (4-Methylsulfonylphenyl)methylimidazol-1-carbodithioat (Sch132119):

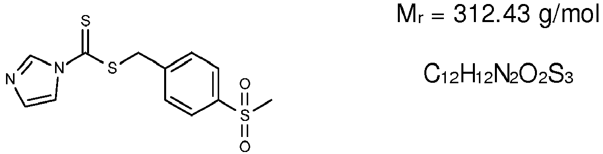

Gemäß **AAV9** wurden 137 mg Imidazol (2.0 mmol, 1.0 eq), 425 mg Kaliumphosphat (2.0 mmol, 1.0 eq), 0.36 mL Kohlenstoffdisulfid (6.0 mmol, 3.0 eq) und 500 mg 4-(Methylsulfonyl)benzylbromid (2.0 mmol, 1.0 eq) umgesetzt. Säulenchromatographie an Kieselgel (EtOAc) ergab 197 mg von **Sch132119** (31.6%) in Form eines gelben Feststoffes.

| | | | | |
|---|---|---|---|---|
| **DC:** | R_{f}= 0.55 (EtOAc). | | | |
| **Fp**.**:** | 118 °C | | | |
| **¹H-NMR:** | (CDCl₃, 400 MHz), δ [ppm] = 8.51 (*s*, 1H, N-CH=N), 7.93 (*d,* ³J = 8.2 Hz, 2H, CH₂-C-CH-C*H*-C-SO₂Me, CH₂-C-CH-C*H*-C-SO₂Me), 7.79-7.76 (*m*, 1H, N-C*H*=CH-N), 7.61 (*d,* ³J = 8.2 Hz, 2H, CH₂-C-C*H*-CH-C-SO₂Me, CH₂-C-CH CH-C-SO₂Me), 7.16-7.13 (*m*, 1H, N-CH=C*H-*N), 4.70 (*s*, 2H, S-C*H*₂₋C), 3.05 (*s*, 3H, SO₂-C*H*₃), | | | |
| **¹³C**-**NMR:** | (CDCl₃, 100 MHz), δ [ppm] = 196.4 (C=S), 141.1 (C-SO₂Me), 139.3 (CH₂-C-CH-CH), 135.7 (N-CH=N-CH-CH), 131.5 (N-CH=N-CH-CH), 130.4 (C-SO₂Me-CH-CH, C-SO₂Me-CH-CH), 128.1 (C-CH-CH, C-CH-CH), 117.8 (N-CH=N-CH-CH), 44.6 (SO₂-CH₃), 40.4 (S-CH₂-C). | | | |
| **IR (ATR):** | v (cm⁻¹) = 3142 (w), 3128 (w), 3114 (w), 3066 (w), 3009 (w), 2995 (w), 1595 (w), 1536 (w), 1489 (w), 1458 (w), 1371 (w), 1327 (w), 1317 (m), 1288 (m), 1244 (w), 1221 (m), 1215 (m), 1142 (s), 1112 (w), 1084 (w), 1050 (m), 1018 (w), 999 (m), 970 (m), 889 (w), 874 (w), 822 (s), 766 (s), 754 (m), 666 (w), 636 (s), 610 (w), 542 (s), 518 (vs), 432 (w), 409 (w). | | | |
| **MS** (ESI+): | m/z (%) = 313 (100, [M+H]⁺) | | | |
| **HRMS** (ESI+): | m/z für C₁₂H₁₂N₂O₂S₃ | | | |
| | ber.: | 312.0061 | | gef.: 312.0079 |
| **EA:** | ber.: | C: 46.13 | H: 3.87 | N: 8.97 |
| | gef.: | C: 45.80 | H: 3.93 | N: 8.56 |

### Darstellung von 1-Naphthylmethylimidazol-1-carbodithioat (Schl32122):

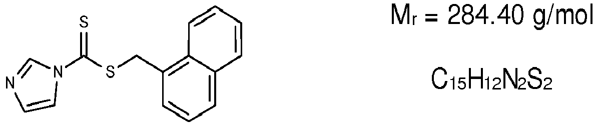

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 1.18 mL 1-Chlormethylnaphthalen (3.0 mmol, 1.0 eq) umgesetzt. Säulenchromatographie an Kieselgel (Cyclohexan/ EtOAc 3:1) ergab 491 mg von **Schl32122** (57.6%) in Form eines gelben Feststoffes.

| | | | | |
|---|---|---|---|---|
| **DC:** | R_{f} = 0.21 (Cyclohexan/ EtOAc 3:1). | | | |
| **Fp**.**:** | 79 °C | | | |
| **¹H-NMR:** | (CDCl₃, 400 MHz), δ [ppm] = 8.45 (*s*, 1H, N-C*H*=N), 7.99-7.79 (*m*, 3H, C*H*_{(Naphthyl)}), 7.74-7.71 (*m*, 1H, N-C*H*=CH-N), 7.60-7.41 (*m*, 4H, C*H*_{(Naphthyl)}), 7.09-7.06 (*m*, 1H, N-CH=C*H*-N), 5.03 (*s*, 2H, S-C*H*₂-C). | | | |
| **¹³C**-**NMR:** | (CDCl₃, 100 MHz), δ [ppm] = 197.6 (C=S), 135.8 (S-CH₂-C), 134.0 (C_{q(Naphthyl)}), 131.7 (C_{q(Naphthyl)}), 131.6 (N-CH=N-CH-CH), 129.7 (C_{t(Naphthyl)}), 129.2 (C_{t(Naphthyl)}), 128.8 (C_{t(Naphthyl)}), 127.9 (N-CH=N-CH-CH), 127.1 (C_{t(Naphthyl)}), 126.4 (C_{t(Naphthyl)}), 125.6 (C_{t(Naphthyl)}), 123.4 (C_{t(Naphthyl)}), 117.7 (N-CH=N-CH-CH), 40.3 (S-CH₂-C). | | | |
| **IR (ATR):** | v (cm⁻¹) = 3732 (w), 3152 (w), 3099 (w), 3052 (w), 1527 (w), 1488 (w), 1462 (w), 1409 (w), 1367 (m), 1282 (m), 1271 (m), 1252 (vs), 1234 (m), 1127 (m), 1104 (m), 1086 (m), 1043 (m), 1002 (vs), 969 (w), 957 (w), 898 (w), 888 (w), 875 (w), 858 (w), 839 (w), 823 (vs), 774 (w), 761 (s), 736 (s), 639 (w), 614 (w), 535 (w), 500 (w), 482 (s), 468 (w), 426 (w). | | | |
| **MS** (ESI+): | m/z (%) = 285 (100, [M+H]⁺) | | | |
| **HRMS** (ESI+): | m/z für C₁₅H₁₂N₂S₂ | | | |
| | ber.: | 284.0442 | | gef.: 284.0406 |
| **EA:** | ber.: | C: 63.35 | H: 4.25 | N: 9.85 |
| | gef.: | C: 63.47 | H: 4.35 | N: 9.63 |

### Darstellung von 2-Naphthylmethylimidazol-1-carbodithioat (Schl32123):

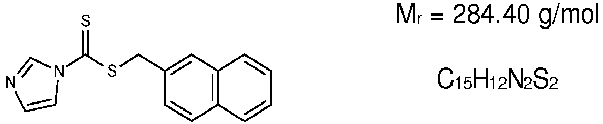

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 664 mg 2-Brommethylnaphthalen (3.0 mmol, 1.0 eq) umgesetzt. Säulenchromatographie an Kieselgel (Cyclohexan/ EtOAc 3:1) ergab 307 mg von **Schl32123** (36.0%) in Form eines gelben Feststoffes.

| | | | | |
|---|---|---|---|---|
| **DC:** | R_{f} = 0.14 (Cyclohexan/ EtOAc 3:1) | | | |
| **Fp**.**:** | 124 °C | | | |
| **¹H-NMR:** | (CDCl₃, 400 MHz), δ [ppm] = 8.57 (*s*, 1 H, N-CH=N), 7.91-7.78 (m, 4H, C*H*_{(Naphthyl)}), 7.74-7.71 (*m*, 1H, N-CH=CH-N), 7.54-7.44 (m, 3H, C*H*_{(Naphthyl)}), 7.15-7.11 (*m*, 1H, N-CH=C*H*-N), 4.79 (*s*, 2H, S-C*H*₂-C). | | | |
| **¹³C**-**NMR:** | (CDCl₃, 100 MHz), δ [ppm] = 197.3 (C=S), 135.8 (S-CH₂-C), 133.3 (C_{q(Naphthyl)}), 133.0 (C_{q(Naphthyl)}), 131.7 (N-CH=N-CH-CH), 130.9 (C_{t(Naphthyl)}), 128.9 (C_{t(Naphthyl)}), 128.7 (C_{t(Naphthyl)}), 127.9 (N-CH=N-CH-CH), 126.9 (C_{t(Naphthyl)}), 126.7 (C_{t(Naphthyl)}), 126.6 (C_{t(Naphthyl)}), 126.4 (C_{t(Naphthyl)}), 117.8 (N-CH=N-CH-CH), 42.1 (S-CH₂-C). | | | |
| **IR (ATR):** | v (cm⁻¹) = 3732 (w), 3152 (w), 3099 (w), 3052 (w), 1598 (w), 1527 (w), 1488 (w), 1462 (w), 1409 (w), 1367 (m), 1282 (m), 1271 (m), 1252 (vs), 1234 (m), 1127 (m), 1104 (m), 1086 (m), 1043 (m), 1002 (vs), 969 (w), 957 (w), 915 (w), 888 (w), 858 (w), 823 (vs), 774 (w), 761 (s), 736 (s), 639 (w), 614 (w), 535 (w), 500 (w), 482 (s), 468 (w), 426 (w). | | | |
| **MS** (ESI+): | m/z (%) = 285 (100, [M+H]⁺) | | | |
| **HRMS** (ESI+): | m/z für C₁₅H₁₂N₂S₂ | | | |
| | ber.: | 284.0442 | | gef.: 284.0449 |
| **EA:** | ber.: | C: 63.35 | H: 4.25 | N: 9.85 |
| | gef.: | C: 63.44 | H: 4.37 | N: 9.99 |

### Darstellung von Phenylimidazol-1-carbodithioat (Schl32131):

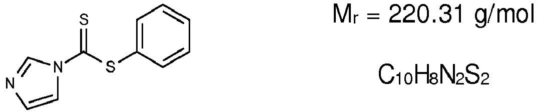

204 mg Imidazol (3.0 mmol, 1.0 eq) und 982 mg Kaliumphosphat (4.5 mmol, 1.5 eq) wurden in 20 mL Dichlormethan gelöst, auf 0 °C gekühlt, 30 Minuten gerührt und mit 0.47 mL Phenylchlormethandithioat (3.6 mmol, 1.2 eq) versetzt. Das Reaktionsgemisch wurde auf Raumtemperatur erwärmt und weitere 18 Stunden gerührt. Anschließend wurde das Lösungsmittel am Rotavapor entfernt. Säulenchromatographie an Kieselgel (Cyclohexan/ EtOAc 3:1) ergab 222 mg von **Schl32131** (33.6%) in Form eines blassgelben Öls.

| | | | | |
|---|---|---|---|---|
| **DC:** | R_{f} = 0.21 (Cyclohexan/ EtOAc 3:1). | | | |
| **¹H-NMR:** | (CDCl₃, 400 MHz), δ [ppm] = 8.58 (s, 1H, N-C*H*=N), 7.84 (*m*, 1H, N-C*H*=CH-N), 7.57-7.43 (*m*, 5H, C*H*_{(Phenyl)}), 7.16 (*m*, 1H, N-CH=C*H*-N). | | | |
| **¹³C**-**NMR:** | (CDCl₃, 100 MHz), δ [ppm] = 201.9 (C=S), 136.2 (N-CH=N-CH-CH), 135.5 (N-CH=N-CH-CH), 133.5 (S-C-CH), 131.4 (C-CH-CH), 130.0 (C-CH-CH), 128.3 (C-CH-CH-CH), 118.2 (N-CH=N-CH-CH). | | | |
| **IR (ATR):** | v (cm⁻¹) = 3122 (w), 2821 (w), 2611 (w), 1574 (m), 1540 (w), 1473 (m), 1437 (m), 1325 (w), 1255 (m), 1096 (w), 1059 (s), 1021 (w), 996 (w), 931 (m), 897 (w), 825 (s), 734 (vs), 685 (s), 656 (vs), 617 (s), 518 (w), 494 (w), 472 (w), 463 (w), 428 (w), 411 (w). | | | |
| **MS** (ESI+): | m/z (%) = 221 (100, [M+H]⁺) | | | |
| **HRMS** (ESI+): | m/z für C₁₀H₈N₂S₂ | | | |
| | ber.: | 220.0129 | | gef.: 220.0122 |
| **EA:** | ber.: | C: 54.52 | H: 3.66 | N: 12.72 |
| | gef.: | C: 54.52 | H: 3.70 | N: 12.62 |

### Darstellung von Methyl-4-(imidazol-1-carbothioylsulfanylmethyl)benzoat (Schl32164):

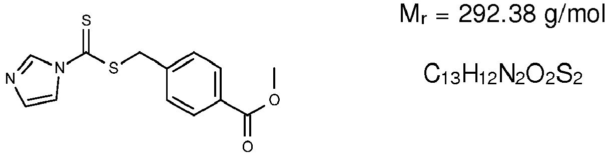

Gemäß **AAV9** wurden 408 mg Imidazol (6.0 mmol, 1.0 eq), 1272 mg Kaliumphosphat (6.0 mmol, 1.0 eq), 1.08 mL Kohlenstoffdisulfid (18.0 mmol, 3.0 eq) und 1459 mg Methyl-4-(brommethyl)benzoat (6.0 mmol, 1.0 eq) umgesetzt. Säulenchromatographie an Kieselgel (Cyclohexan/ EtOAc 4:1) ergab 431 mg von **Schl32164** (24.5%) in Form eines gelben Feststoffes.

| | | | | |
|---|---|---|---|---|
| **DC:** | R_{f} = 0.09 (Cyclohexan/ EtOAc 4:1). | | | |
| **Fp.:** | 95 °C | | | |
| **¹H-NMR:** | (CDCl₃, 400 MHz), δ [ppm] = 8.44 (*s*, 1 H, N-CH=N), 7.99 (*d*, ³J = 8.3 Hz, 2H, CH₂-C-CH-C*H*-C-COOMe, CH₂-C-CH-C*H*-C-COOMe), 7.74-7.71 (*m*, 1 H, N-C*H*=CH-N), 7.43 (*d,* 3J = 8.3 Hz, 2H, CH₂-C-C*H*-CH-C-COOMe, CH₂-C-C*H*-CH-C-COOMe), 7.09-7.06 (*m*, 1 H, N-CH=C*H*-N), 4.62 (*s*, 2H, S-C*H*₂₋C), 3.88 (*s*, 3H C(=O)-O-C*H*₃). | | | |
| **¹³C**-**NMR:** | (CDCl₃, 100 MHz), δ [ppm] = 196.6 (C=S), 166.5 (C=O), 139.0 (CH₂-C-CH-CH), 135.8 (C-COOMe), 131.8 (N-CH=N-CH-CH), 130.1 (CH-C-COOMe, CH-C-COOMe) 129.4 (CH-CH-C-COOMe, CH-CH-C-COOMe), 117.8 (N-CH=N-CH-CH), 52.3 (C(=O)-O-CH₃), 41.0 (S-CH₂-C). | | | |
| **IR (ATR):** | v (cm⁻¹) = 3155 (w), 3118 (w), 3095 (w), 3001 (w), 2950 (w), 1708 (s), 1608 (w), 1575 (w), 1531 (w), 1509 (w), 1473 (w), 1433 (w), 1416 (w), 1371 (w), 1286 (m), 1274 (m), 1257 (vs), 1191 (w), 1180 (w), 1111 (m), 1088 (s), 1018 (s), 1001 (s), 964 (w), 899 (w), 863 (m), 844 (s), 831 (w), 796 (w), 777 (w), 742 (s), 725 (s), 644 (w), 532 (w), 513 (w), 401 (m). | | | |
| **MS** (ESI+): | m/z (%) = 293 (100, [M+H]⁺) | | | |
| **HRMS** (ESI+): | m/z für C₁₃H₁₂N₂O₂S₂ | | | |
| | ber.: | 292.0340 | | gef.: 292.0321 |
| **EA:** | ber.: | C: 53.40 | H: 4.14 | N: 9.58 |
| | gef.: | C: 53.39 | H: 4.11 | N: 9.49 |

### Darstellung von 4-(Imidazol-1-carbothioylsulfanylmethyl)benzoesäure (Schl32165):

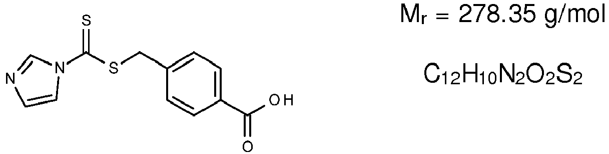

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 645 mg 4-(Brommethyl)benzoesäure (3.0 mmol, 1.0 eq) umgesetzt. Das Kaliumphosphat wurde abfiltriert, anschließend wurde das Reaktionsgemisch mit 100 mL EtOAc verdünnt und fünfmal gegen je 75 mL 1M NaOH-Lösung ausgeschüttelt. Die vereinigten wässrigen Phasen wurden mit 6M HCl auf pH=1 eingestellt. Der entstehende Niederschlag wurde abgesaugt, mit Wasser gewaschen und getrocknet. Es wurden 328 mg von **Schl32165** (39.5%) als blassgelben Feststoff erhalten.

| | | | | |
|---|---|---|---|---|
| **Fp.:** | 245 °C | | | |
| **¹H-NMR:** | (DMSO-D₆, 400 MHz), δ [ppm] = 8.48 (*s*, 1H, N-CH=N), 7.90 (*d,* 3J = 8.5 Hz, 2H, CH₂-C-CH-C*H*-C-COOH, CH₂-C-CH-CH-C-COOH), 7.78-7.74 (*m*, 1H, N-CH=CH-N), 7.34 (*d,* ³J = 8.6 Hz, 2H, CH₂-C-CH-CH-C-COOH, CH₂-C-CH-CH-C-COOH), 7.15-7.07 (*m*, 1H, N-CH=C*H*-N), 4.62 (s, 2H, S-C*H*₂-C). | | | |
| **¹³C**-**NMR:** | (DMSO-D₆, 100 MHz), δ [ppm] = 195.4 (C=S), 169.5 (C=O), 141.0 (CH₂-C-CH-CH), 137.2 (C-COOH), 131.6 (N-CH=N-CH-CH), 129.5 (CH-C-COOH, CH-C-COOH) 128.9 (CH-CH-C-COOH, CH-CH-C-COOH), 117.7 (N-CH=N-CH-CH), 40.6 (S-CH₂-C). | | | |
| **IR** (ATR): | v (cm⁻¹) = 2843 (w), 2669 (w), 2546 (w), 1682 (vs), 1607 (m), 1574 (w), 1510 (w), 1424 (m), 1371 (w), 1316 (m), 1289 (vs), 1216 (w), 1177 (w), 1125 (w), 1109 (w), 1070 (m), 935 (m), 876 (w), 838 (w), 800 (w), 740 (m), 710 (m), 634 (w), 618 (w), 543 (m), 485 (w), 472 (w), 406 (w). | | | |
| **MS** (ESI+): | m/z (%) = 279 (100, [M+H]⁺) | | | |
| **HRMS** (ESI+): | m/z für C₁₂H₁₀N₂O₂S₂ | | | |
| | ber.: | 278.0184 | | gef.: 278.0153 |
| **EA:** | ber.: | C: 51.78 | H: 3.62 | N: 10.06 |
| | gef.: | C: 51.81 | H: 3.41 | N: 10.30 |

### Darstellung von (4-Cyanophenyl)methylimidazol-1-carbodithioat (Schl32167):

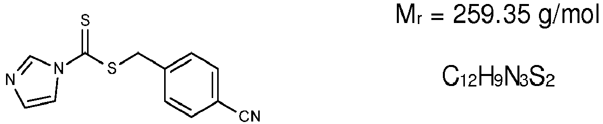

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 588 mg 4-(Brommethyl)benzonitril (3.0 mmol, 1.0 eq) umgesetzt. Säulenchromatographie an Kieselgel (Cyclohexan/ EtOAc 2:1) ergab 184 mg von **Sch132167** (23.7%) in Form eines gelben Feststoffes.

| | | | | |
|---|---|---|---|---|
| **DC:** | R_{f} = 0.11 (Cyclohexan/ EtOAc 2:1). | | | |
| **Fp**.**:** | 118 °C | | | |
| **¹H-NMR:** | (CDCl₃, 400 MHz), δ [ppm] = 8.49 (*s*, 1H, N-C*H*=N), 7.78-7.74 (*m*, 1H, N-C*H*=CH-N), 7.65 (*d*, ³J = 8.2 Hz, 2H, CH₂-C-CH-C*H*-C-C-CN, CH₂-C-CH-C*H*-C-C-CN), 7.52 (*d*, ³J = 8.2 Hz, 2H, CH₂-C-C*H*-CH-C-C-CN, CH₂-C-C*H*-CH-C-C-CN), 7.14-7.11 (*m*, 1H, N-CH=C*H*-N), 4.66 (*s*, 2H, S-C*H*₂-C). | | | |
| **¹³C**-**NMR:** | (CDCl₃, 100 MHz), δ [ppm] = 195.8 (C=S), 139.6 (CH₂-C-CH-CH), 131.8 (N-CH=N-CH-CH), 132.7 (CH-C-CN, CH-C-CN) 130.2 (CH-CH-C-CN, CH-CH-C-CN), 123.8 (C-CN), 118.4 (N-CH=N-CH-CH), 112.3 (C-CN), 40.6 (S-CH₂-C). | | | |
| **IR (ATR):** | v (cm⁻¹) = 3691 (w), 3650 (w), 3565 (w), 3230 (w), 3126 (w), 1603 (w), 1577 (w), 1531 (w), 1504 (w), 1462 (w), 1415 (w), 1369 (m), 1290 (m), 1275 (s), 1220 (s), 1113 (m), 1087 (w), 1050 (vs), 997 (s), 889 (w), 864 (w), 849 (w), 832 (vs), 753 (w), 711 (s), 640 (vs), 607 (w), 554 (vs), 503 (m), 474 (w), 461 (w), 446 (w), 423 (w), 407 (m). | | | |
| **MS** (ESI+): | m/z (%) = 260 (100, [M+H]⁺) | | | |
| **HRMS** (ESI+): | m/z für C₁₂H₉N₃S₂ | | | |
| | ber.: | 259.0238 | | gef.: 259.0236 |
| **EA:** | ber.: | C: 55.57 | H: 3.50 | N: 16.20 |
| | gef.: | C: 55.93 | H: 3.52 | N: 16.52 |

### Darstellung von (4-Phenylphenyl)methylimidazol-1-carbodithioat (Schl32168):

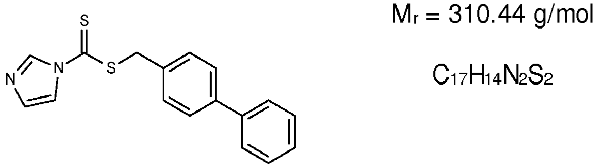

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 741 mg 4-(Brommethyl)biphenyl (3.0 mmol, 1.0 eq) umgesetzt. Säulenchromatographie an Kieselgel (Cyclohexan/ EtOAc 2:1) ergab 318 mg von **Schl32168** (34.2%) in Form eines gelben Feststoffes.

| | | | | |
|---|---|---|---|---|
| **DC:** | R_{f} = 0.29 (Cyclohexan/ EtOAc 2:1). | | | |
| **Fp**.**:** | 93 °C | | | |
| **¹H-NMR:** | (CDCl₃, 400 MHz), δ [ppm] = 8.50 (*s*, 1H, N-C*H*=N), 7.82-7.77 (*m*, 1H, N-C*H*=CH-N), 7.65-7.52 (*m*, 4H, C*H*_{(Phenyl)}), 7.59-7.40 (*m*, 4H, C*H*_{(Phenyl)}), 7.38-7.32 (*m*, 1H, C*H*_{(Phenyl)}), 7.14-7.08 (*m*, 1H, N-CH=C*H*-N), 4.65 (*s*, 2H, S-C*H*₂-C). | | | |
| **¹³C**-**NMR:** | (CDCl₃, 100 MHz), δ [ppm] = 197.3 (C=S), 141.4 (C_{q(Phenyl)}), 140.4 (C_{q(Phenyl)}), 132.6 (Cq_{(Phenyl)}), 131.6 (N-CH=N-CH-CH), 130.0 (C_{t(Phenyl)}), 129.0 (Ct_{(Phenyl)}), 127.7 (C_{t(Phenyl)}), 127.2 (Ct_{(Phenyl)}), 117.8 (N-CH=N-CH-CH), 41.6 (S-CH₂-C). | | | |
| **IR (ATR):** | v (cm⁻¹) = 3742 (w), 3130 (w), 3028 (w), 2920 (w), 1582 (w), 1562 (w), 1523 (w), 1487 (w), 1472 (w), 1449 (w), 1408 (w), 1367 (m), 1292 (m), 1273 (s), 1244 (s), 1157 (w), 1097 (m), 1086 (m), 1048 (vs), 1007 (s), 888 (w), 852 (w), 833 (s), 770 (w), 738 (s), 724 (s), 695 (s), 639 (s), 587 (w), 551 (w), 553 (w), 495 (m), 485 (m), 426 (w). | | | |
| **MS** (ESI+): | m/z (%) = 311 (100, [M+H]⁺) | | | |
| **HRMS** (ESI+): | m/z für C₁₇H₁₄N₂S₂ | | | |
| | ber.: | 310.0598 | | gef.: 310.0583 |
| **EA:** | ber.: | C: 65.77 | H: 4.55 | N: 9.02 |
| | gef.: | C: 65.33 | H: 4.72 | N: 9.05 |

### Darstellung von Benzhydrylimidazol-1-carbodithioat (Schl32169):

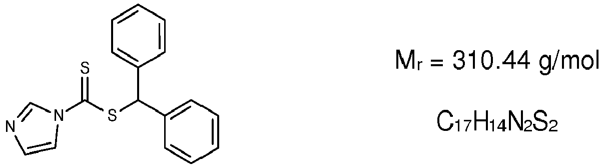

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 741 mg Bromdiphenylmethan (3.0 mmol, 1.0 eq) umgesetzt. Säulenchromatographie an Kieselgel (Cyclohexan/ EtOAc 2:1) ergab 495 mg von **Schl32169** (53.2%) in Form eines gelben Öls.

| | | | | |
|---|---|---|---|---|
| **DC:** | R_{f} = 0.26 (Cyclohexan/ EtOAc 2:1). | | | |
| **¹H-NMR:** | (CDCl₃, 400 MHz), δ [ppm] = 8.46 (*s*, 1H, N-C*H*=N), 7.77-7.75 (*m*, 1H, N-CH=CH-N), 7.45-7.39 (*m*, 4H, C*H*_{(Phenyl)}), 7.38-7.33 (*m*, 4H, C*H*_{(Phenyl)}), 7.32-7.27 (*m*, 1H, C*H*_{(Phenyl)}), 7.10-7.08 (*m*, 1H, N-CH=C*H*-N), 6.38 (*s*, 1H, S-C*H*-C). | | | |
| **¹³C**-**NMR:** | (CDCl₃, 100 MHz), δ [ppm] = 195.5 (C=S), 138.3 (Cq_{(Phenyl)}), 135.8 (C_{q(Phenyl)}), 133.8 (Cq_{(Phenyl)}), 131.7 (N-CH=N-CH-CH), 128.9 (Ct_{(Phenyl)}), 128.8 (Ct_{(Phenyl)}), 128.1 (Ct_{(Phenyl)}), 117.8 (N-CH=N-CH-CH), 60.0 (S-CH-C). | | | |
| **IR (ATR):** | v (cm⁻¹) = 3121 (w), 3058 (w), 3026 (w), 1656 (w), 1599 (w), 1583 (w), 1527 (w), 1464 (m), 1449 (m), 1365 (s), 1266 (vs), 1237 (vs), 1218 (vs), 1088 (s), 1041 (s), 999 (s), 915 (w), 888 (w), 818 (vs), 779 (w), 744 (s), 715 (m), 693 (vs), 639 (s), 614 (s), 585 (s), 533 (m), 480 (m), 407 (m). | | | |
| **MS** (ESI+): | m/z (%) = 311 (100, [M+H]⁺) | | | |
| **HRMS** (ESI+): | m/z für C₁₇H₁₄N₂S₂ | | | |
| | ber.: | 310.0598 | | gef.: 310.0589 |
| **EA:** | ber.: | C: 65.77 | H:4.55 | N:9.02 |
| | gef.: | C:65.50 | H:5.08 | N:8.80 |

### Darstellung von [4-(Trifluormethoxy)phenyl]methylimidazol-1-carbodithioat (Schl32172):

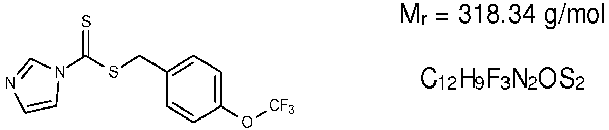

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 0.48 mL 4-(Trifluormethoxy)benzylbromid (3.0 mmol, 1.0 eq) umgesetzt. Säulenchromatographie an Kieselgel (Cyclohexan/ EtOAc 5:1) ergab 365 mg von **Schl32172** (38.3%) in Form eines gelben Öls.

| | | | | |
|---|---|---|---|---|
| **DC:** | R_{f} = 0.10 (Cyclohexan/ EtOAc 5:1). | | | |
| **¹H-NMR:** | (CDCl₃, 400 MHz), δ [ppm] = 8.43 (s, 1H, N-C*H*=N), 7.76-7.71 (*m*, 1H, N-C*H*=CH-N), 7.39 (*d*, ³J = 8.7 Hz, 2H, C-CH-C*H*-C-OCF₃, C-CH-C*H*-C-OCF₃), 7.16 (*d*, ³J = 8.0 Hz, 2H, C-C*H*-CH-C-OCF₃, C-C*H*-CH-C-OCF₃), 7.09-7.05 *(m,* 1H, N-CH=C*H*-N), 4.56 (*s*, 2H, S-C*H*₂-C). | | | |
| **¹³C**-**NMR:** | (CDCl₃, 100 MHz), δ [ppm] = 196.8 (C=S), 148.4 (C-OCF₃), 135.8 (C-CH-CH-C-OCF₃), 132.6 (N-CH=N-CH-CH), 130.9 (C-CH-CH-C-OCF₃, C-CH-CH-C-OCF₃), 121.4 (C-CH-CH-C-OCF₃, C-CH-CH-C-OCF₃), 116.6 (N-CH=N-CH-CH), 120.5 *(q,* ¹J_{C,F} = 258.2 Hz, C-CH-CH-C-O-CF₃), 40.6 (S-CH₂-C). | | | |
| **IR (ATR):** | v (cm⁻¹) = 3122 (w), 2132 (w), 1611 (w), 1594 (w), 1528 (w), 1508 (m), 1465 (m), 1419 (w), 1367 (m), 1250 (vs), 1209 (vs), 1154 (vs), 1100 (s), 1086 (s), 1041 (s), 1018 (m), 1000 (s), 921 (m), 888 (w), 823 (s), 744 (m), 719 (m), 639 (s), 610 (m), 533 (m), 522 (m), 481 (w), 408 (m). | | | |
| **MS** (ESI+): | m/z (%) = 319 (100, [M+H]⁺) | | | |
| **HRMS** (ESI+): | m/z für C₁₂H₉F₃N₂OS₂ | | | |
| | ber.: | 318.0108 | | gef.: 318.0136 |
| **EA:** | ber.: | C: 45.28 | H: 2.85 | N: 8.80 |
| | gef.: | C: 45.27 | H: 2.99 | N: 8.63 |

### Darstellung von Benzyl-4-formylimidazol-1-carbodithioat (Schl32183):

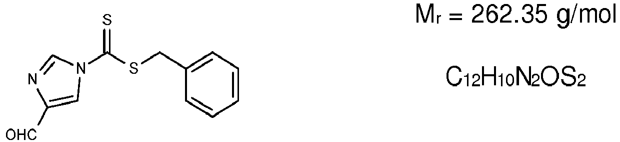

Gemäß **AAV9** wurden 288 mg Imidazol-4-carboxaldehyd (3.0 mmol, 1.0 eq), 637 mg Kaliumphosphat (3.0 mmol, 1.0 eq), 0.54 mL Kohlenstoffdisulfid (9.0 mmol, 3.0 eq) und 0.36 mL Benzylbromid (3.0 mmol, 1.0 eq) umgesetzt. Säulenchromatographie an Kieselgel (Cyclohexan/ EtOAc 1:1) ergab 260 mg von **Schl32183** (33.1 %) in Form eines hellroten Öls.

| | | | | |
|---|---|---|---|---|
| **DC:** | R_{f} = 0.61 (Cyclohexan/ EtOAc 1:1). | | | |
| **¹H-NMR:** | (CDCl₃, 400 MHz), δ [ppm] = 9.29 (*s*, 1 H, CHO), 8.50 (*s*, 1 H, N-C*H*=N), 8.38 (s, 1 H, N-C=C*H*-N), 7.42-7.31 (*m*, 5H, C*H*_{(Benzyl)}), 4.62 (*s*, 2H, C-C*H₂*-C). | | | |
| **¹³C**-**NMR:** | (CDCl₃, 100 MHz), δ [ppm] = 196.6 (C=S), 186.1 (CHO), 142.8 (S-CH₂-C), 136.6 (N-CH=N-C-CH), 132.9 (C-CHO), 129.5 (C-CH-CH), 129.1 (C-CH-CH), 128.7 (C-CH-CH-CH), 121.7 (N-CH=N-C-CH), 42.4 (S-CH₂-C). | | | |
| **IR (ATR):** | v (cm⁻¹) = 3060 (w), 3027 (w), 2917 (w), 1541 (w), 1493 (m), 1452 (m), 1418 (w), 1393 (w), 1367 (w), 1348 (w), 1285 (w), 1256 (w), 1233 (w), 1154 (m), 1059 (vs), 1027 (s), 914 (w), 885 (w), 852 (w), 830 (m), 795 (s), 765 (s), 692 (vs), 612 (m), 562 (m), 534 (w), 474 (s), 404 (w). | | | |
| **MS** (ESI+): | m/z (%) = 263 (100, [M+H]⁺) | | | |
| **HRMS** (ESI+): | m/z für C₁₂H₁₀N₂OS₂ | | | |
| | ber.: | 262.0235 | | gef.: 262.0241 |
| **EA:** | ber.: | C: 54.94 | H: 3.84 | N: 10.68 |
| | gef.: | C: 54.64 | H: 3.73 | N: 10.83 |

### Darstellung von Benzyl-4,5-dicyanoimidazol-1-carbodithioat (Schl32184):

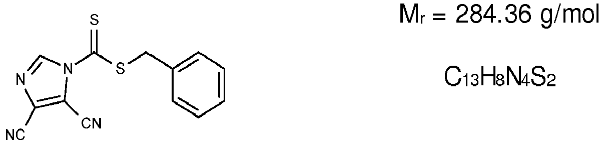

Gemäß **AAV9** wurden 354 mg 4,5-Dicyanoimidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 0.36 mL Benzylbromid (3.0 mmol, 1.0 eq) umgesetzt. Säulenchromatographie an Kieselgel (Cyclohexan/ EtOAc 1:1) ergab 466 mg von **Schl32184** (54.7%) in Form eines hellroten Feststoffes.

| | | | | |
|---|---|---|---|---|
| **DC:** | R_{f} = 0.31 (Cyclohexan/ EtOAc 1:1). | | | |
| **Fp**.**:** | 125 °C | | | |
| **¹H-NMR:** | (CDCl₃, 400 MHz), δ [ppm] = 7.67 (*s*, 1H, N-C*H*=N), 7.46-7.40 (*m*, 3H, C*H*_{(Benzyl)}), 7.30-7.23 (*m*, 2H, C*H*_{(Benzyl)}), 5.27 (*s*, 2H, C-C*H₂*-C). | | | |
| **¹³C**-**NMR:** | (CDCl₃, 100 MHz), δ [ppm] = 195.7 (C=S), 136.4 (S-CH₂-C), 135.2 (N-CH=N), 130.0 (C-CH-CH), 129.8 (C-CH-CH), 128.2 (C-CH-CH-CH), 117.6 (CN), 110.1 (C-CN), 51.9 (S-CH₂-C). | | | |
| **IR** (ATR): | v (cm⁻¹) = 3114 (m), 3068 (w), 3032 (w), 1586 (w), 1528 (w), 1497 (m), 1488 (m), 1437 (w), 1396 (w), 1363 (w), 1350 (m), 1316 (m), 1307 (m), 1259 (m), 1204 (w), 1187 (m), 1157 (w), 1076 (w), 1030 (m), 1002 (w), 952 (w), 902 (w), 880 (m), 847 (w), 793 (m), 727 (vs), 704 (vs), 687 (vs), 648 (vs), 620 (s), 605 (w), 583 (w), 504 (vs), 474 (w), 451 (s), 404 (w). | | | |
| **MS** (ESI+): | m/z (%) = 285 (100, [M+H]⁺) | | | |
| **HRMS** (ESI+): | m/z für C₁₃H₈N₄S₂ | | | |
| | ber.: | 284.0190 | | gef.: 284.0165 |
| **EA:** | ber.: | C:54.91 | H:2.84 | N:19.70 |
| | gef.: | C:55.00 | H:2.89 | N:20.01 |

### Darstellung von Benzyl-4-nitroimidazol-1-carbodithioat (Schl32214):

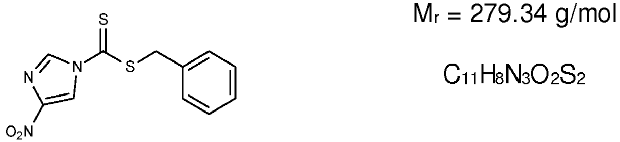

Gemäß **AAV9** wurden 905 mg 4-Nitroimidazol (8.0 mmol, 2.0 eq), 2547 mg Kaliumphosphat (12.0 mmol, 1.0 eq), 0.48 mL Kohlenstoffdisulfid (8.0 mmol, 2.0 eq) und 0.48 mL Benzylbromid (4.0 mmol, 1.0 eq) umgesetzt. Säulenchromatographie an Kieselgel (EtOAc) ergab 939 mg von **Schl32214** (84.1 %) in Form eines gelben Feststoffes.

| | | | | |
|---|---|---|---|---|
| **DC:** | R_{f}= 0.41 (EtOAc). | | | |
| **Fp**.**:** | 72 °C | | | |
| **¹H-NMR:** | (CDCl₃, 400 MHz), δ [ppm] = 7.72 (s, 1H, N-C*H*=N), 7.49 (*s*, 1H, N-C=C*H*-N), 7.42-7.25 (*m*, 5H, C*H*_{(Benzyl)}), 5.17 (*s*, 2H, C-C*H₂*-C). | | | |
| **¹³C**-**NMR:** | (CDCl₃, 100 MHz), δ [ppm] = 203.8 (C=S), 147.1 (C-NO₂), 136.3 (S-CH₂-C), 133.8 (N-CH=N-C-CH), 129.6 (C-CH-CH), 129.4 (C-CH-CH), 128.0 (C-CH-CH-CH), 119.6 (N-CH=N-C-CH), 52.2 (S-CH₂-C). | | | |
| **IR** (ATR): | v (cm⁻¹) = 3101 (w), 1545 (m), 1528 (w), 1512 (m), 1491 (m), 1446 (w), 1414 (w), 1396 (w), 1374 (m), 1338 (m), 1283 (m), 1237 (w), 1204 (w), 1168 (w), 1139 (w), 1040 (w), 1027 (w), 987 (w), 866 (w), 822 (s), 779 (w), 756 (w), 713 (vs), 655 (s), 622 (w), 574 (w), 478 (w), 423 (w). | | | |
| **MS** (ESI-): | m/z (%) = 278 (100, [M-H]⁻) | | | |
| **HRMS** (ESI+): | m/z für C₁₁H₈N₃O₂S₂ | | | |
| | ber.: | 279.0136 | | gef.: 279.0140 |
| **EA:** | ber.: | C: 47.30 | H: 3.25 | N: 15.04 |
| | gef.: | C: 47.54 | H: 3.55 | N: 15.44 |

### Darstellung von p-Tolylmethyl-imidazol-1-carbodithioat (Schl32254):

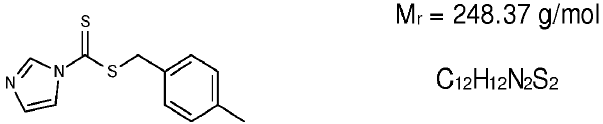

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 555 mg 1-(Brommethyl)-4-methylbenzen (3.0 mmol, 1.0 eq) umgesetzt. Säulenchromatographie an Kieselgel (Cyclohexan/ EtOAc 3:1) ergab 113 mg von **Schl32254** (15.2%) in Form eines gelben Feststoffes.

| | | | | |
|---|---|---|---|---|
| **DC:** | R_{f} = 0.14 (Cyclohexan/ EtOAc 4:1). | | | |
| **Fp**.**:** | 91 °C | | | |
| **¹H-NMR:** | (CDCl₃, 400 MHz), δ [ppm] = 8.49 (s, 1H, N-C*H*=N), 7.79-7.73 (*m*, 1H, N-CH=C*H*-N), 7.28 (*d*, ³J = 8.0 Hz, 2H, CH₂-C-CH-C*H*-C-CH₃, CH₂-C-CH-C*H*-C-CH₃), 7.16 (*d*, ³J = 8.0 Hz, 2H, CH₂-C-C*H*-CH-C-CH₃, CH₂-C-C*H*-CH-C-CH₃), 7.12-7.08 (*m*, 1H, N-CH=C*H*-N), 4.57 (*s*, 2H, S-C*H*₂₋C), 2.34 (s, 3H, C-C*H₃*). | | | |
| **¹³C**-**NMR:** | (CDCl₃, 100 MHz), δ [ppm] = 197.5 (C=S), 138.4 (C-CH₃), 131.8 (N-CH=N-CH-CH), 130.3 (CH₂-C-CH-CH), 129.7 (CH-C-CH₃, CH-C-CH₃), 129.4 (CH-CH-C-CH₃, CH-CH-C-CH₃), 117.8 (N-CH=N-CH-CH), 42.0 (S-CH₂-C), 21.5 (C-CH₃). | | | |
| **IR (ATR):** | v (cm⁻¹) = 3112 (w), 2919 (w), 2852 (w), 1511 (w), 1461 (m), 1366 (s), 1290 (vs), 1273 (m), 1240 (w), 1220 (vs), 1110 (m), 1087 (m), 1050 (vs), 1001 (vs), 948 (w), 884 (m), 866 (w), 842 (w), 819 (vs), 739 (m), 719 (vs), 639 (vs), 608 (m), 528 (m), 486 (vs), 465 (w), 403 (w). | | | |
| **MS** (ESI+): | m/z (%) = 249 (100, [M+H]⁺) | | | |
| **HRMS** (ESI+): | m/z für C₁₂H₁₂N₂S₂ | | | |
| | ber.: | 248.0442 | | gef.: 248.0444 |
| **EA:** | ber.: | C: 58.03 | H: 4.87 | N: 11.28 |
| | gef.: | C: 58.12 | H: 4.70 | N: 11.12 |

### Darstellung von m-Tolylmethylimidazol-1-carbodithioat (Schl32255):

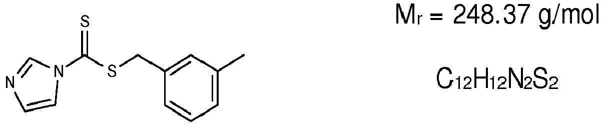

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 555 mg 1-(Brommethyl)-3-methylbenzen (3.0 mmol, 1.0 eq) umgesetzt. Säulenchromatographie an Kieselgel (Cyclohexan/ EtOAc 3:1) ergab 168 mg von **Schl32255** (22.7%) in Form eines gelben Feststoffes.

| | | | | |
|---|---|---|---|---|
| **DC:** | R_{f} = 0.20 (Cyclohexan/ EtOAc 4:1). | | | |
| **Fp**.**:** | 78 °C | | | |
| **¹H-NMR:** | (CDCl₃, 400 MHz), δ [ppm] = 8.48 (*s*, 1H, N-C*H*=N), 7.79-7.72 (*m*, 1H, N-CH=CH-N), 7.27-7.12 (*m*, CH₂-C-C*H*-C(-CH₃), CH₂-C-C*H*-C*H*-C*H*-C-(CH₃)), 7.11-7.06 (*m*, 1H, N-CH=C*H*-N), 4.57 (*s*, 2H, S-C*H*₂₋C), 2.35 (*s*, 3H, C-C*H₃*). | | | |
| **¹³C**-**NMR:** | (CDCl₃, 100 MHz), δ [ppm] = 197.5 (C=S), 138.9 (C-CH₃), 135.7 (N-CH=N-CH-CH), 133.4 (CH₂-C-CH-CH), 131.5 (N-CH=N-CH-CH), 130.2 (C-CH-CH-CH-C(-CH₃)), 129.2 (C-CH-CH-CH-C(-CH₃)), 128.9 (C-CH-C(-CH₃)), 126.6 (C-CH-CH-CH-C(-CH₃)), 117.8 (N-CH=N-CH-CH), 42.0 (S-CH₂-C), 21.5 (C-CH₃). | | | |
| **IR (ATR):** | v (cm⁻¹) = 3118 (w), 3019 (w), 2917 (w), 1607 (w), 1527 (w), 1463 (m), 1365 (s), 1267 (vs), 1238 (s), 1215 (vs), 1086 (s), 1050 (vs), 1040 (vs), 1000 (vs), 921 (w), 887 (w), 827 (vs), 789 (s), 709 (s), 689 (m), 639 (vs), 610 (w), 584 (w), 533 (w), 516 (w), 483 (m), 435 (w). | | | |
| **MS** (ESI+): | m/z (%) = 249 (100, [M+H]⁺) | | | |
| **HRMS** (ESI+): | m/z für C₁₂H₁₂N₂S₂ | | | |
| | ber.: | 248.0442 | | gef.: 248.0426 |
| **EA:** | ber.: | C: 58.03 | H: 4.87 | N: 11.28 |
| | gef.: | C: 58.11 | H: 4.91 | N: 11.33 |

### Darstellung von o-Tolylmethylimidazol-1-carbodithioat (Schl32256):

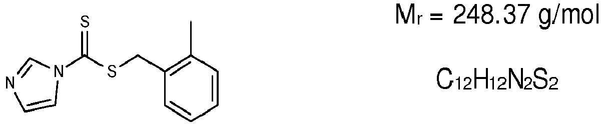

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 555 mg 1-(Brommethyl)-2-methylbenzen (3.0 mmol, 1.0 eq) umgesetzt. Säulenchromatographie an Kieselgel (Cyclohexan/ EtOAc 3:1) ergab 230 mg von **Schl32256** (30.9%) in Form eines gelben Feststoffes.

| | | | | |
|---|---|---|---|---|
| **DC:** | R_{f} = 0.18 (Cyclohexan/ EtOAc 4:1). | | | |
| **Fp**.**:** | 84 °C | | | |
| **¹H-NMR:** | (CDCl₃, 400 MHz), δ [ppm] = 8.47 (*s*, 1H, N-C*H*=N), 7.76-7.74 (*m*, 1H, N-C*H*=CH-N), 7.35 (*d*, ³J = 7.6 Hz, 1H, CH₂-C-C*H*-CH-CH-CH-C(-CH₃)), 7.28-7.15 (*m*, 3H, CH₂-C-CH-C*H*-C*H*-C*H*-C(-CH₃)), 7.11-7.07 (*m*, 1H, N-CH=C*H*-N), 4.58 (*s*, 2H, S-C*H₂*-C), 2.39 (*s*, 3H, C-C*H₃*). | | | |
| **¹³C**-**NMR:** | (CDCl₃, 100 MHz), δ [ppm] = 197.7 (C=S), 137.5 (C-CH₃), 135.7 (N-CH=N-CH-CH), 131.7 (N-CH=N-CH-CH), 131.1 (CH₂-C-CH-CH), 130.9 (CH₂-C-CH-CH-CH-CH-C(-CH₃)), 130.6 (CH₂-C-CH), 128.9 (CH₂-C-CH-CH-CH-CH-C(-CH₃)), 126.6 (CH₂-C-CH-CH), 117.7 (N-CH=N-CH-CH), 40.6 (S-CH₂-C), 19.4 (C-CH₃). | | | |
| **IR (ATR):** | v (cm⁻¹) = 3106 (w), 2921 (w), 1465 (m), 1452 (w), 1365 (s), 1279 (m), 1269 (w), 1244 (vs), 1235 (s), 1225 (s), 1106 (m), 1086 (s), 1045 (s), 1000 (vs), 945 (w), 890 (w), 844 (m), 830 (s), 813 (w), 773 (s), 749 (vs), 727 (vs), 637 (vs), 612 (w), 597 (w), 539 (w), 529 (w), 504 (w), 445 (s). | | | |
| **MS** (ESI+): | m/z (%) = 249 (100, [M+H]⁺) | | | |
| **HRMS** (ESI+): | m/z für C₁₂H₁₂N₂S₂ | | | |
| | ber.: | 248.0442 | | gef.: 248.0439 |
| **EA:** | ber.: | C: 58.03 | H: 4.87 | N: 11.28 |
| | gef.: | C: 58.27 | H: 4.83 | N: 11.00 |

### Darstellung von (4-Methoxyphenyl)methylimidazol-1-carbodithioat (Schl32257):

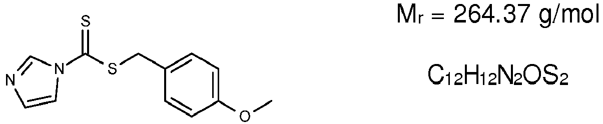

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 470 mg 1-(Chlormethyl)-4-methoxybenzen (3.0 mmol, 1.0 eq) umgesetzt. Säulenchromatographie an Kieselgel (Cyclohexan/ EtOAc 3:1) ergab 177 mg von **Schl32257** (22.3%) in Form eines gelben Feststoffes.

| | | | | |
|---|---|---|---|---|
| **DC:** | R_{f} = 0.19 (Cyclohexan/ EtOAc 4:1) | | | |
| **Fp**.**:** | 137 °C | | | |
| **¹H-NMR:** | (CDCl₃, 400 MHz), δ [ppm] = 8.45 (*s*, 1H, N-C*H*=N), 7.77-7.69 (*m*, 1H, N-C*H*=CH-N), 7.29 (*d,* ³J = 8.5 Hz, 2H, CH₂-C-CH-C*H*-C-OMe, CH₂-C-CH-C*H*-C-OMe), 7.10-7.04 (*m*, 1H, N-CH=C*H*-N), 6.86 (*d*, ³J = 8.5 Hz, 2H, CH₂-C-C*H*-CH-C-OMe, CH₂-C-C*H*-CH-C-OMe), 4.54 (*s*, 2H, S-C*H*₂₋C), 3.78 (*s*, 3H, O-C*H*₃). | | | |
| **¹³C**-**NMR:** | (CDCl₃, 100 MHz), δ [ppm] = 197.6 (C=S), 159.7 (C-OMe), 135.7 (N-CH=N-CH-CH), 131.6 (N-CH=N-CH-CH), 130.8 (CH-CH-C-OMe, CH-CH-C-OMe), 125.2 (CH₂-C-CH-CH), 117.8 (N-CH=N-CH-CH), 114.4 (CH-C-OMe, CH-C-OMe), 55.4 (O-CH₃), 41.6 (S-CH₂-C). | | | |
| **IR (ATR):** | v (cm⁻¹) = 3116 (w), 3002 (w), 2835 (w), 1606 (w), 1510 (s), 1464 (m), 1439 (w), 1366 (m), 1304 (w), 1291 (w), 1275 (s), 1251 (s), 1232 (w), 1212 (vs), 1175 (s), 1126 (m), 1108 (s), 1086 (w), 1052 (vs), 1025 (vs), 999 (vs), 882 (w), 828 (vs), 750 (w), 735 (w), 718 (vs), 696 (w), 641 (s), 607 (w), 554 (m), 526 (w), 512 (m), 438 (w), 405 (w). | | | |
| **MS** (ESI+): | m/z (%) = 265 (100, [M+H]⁺) | | | |
| **HRMS** (ESI+): | m/z für C₁₂H₁₂N₂OS₂ | | | |
| | ber.: | 264.0391 | | gef.: 264.0421 |
| **EA:** | ber.: | C:54.52 | H:4.58 | N:10.60 |
| | gef.: | C:54.12 | H:4.58 | N:10.79 |

### Darstellung von (3-Methoxyphenyl)methylimidazol-1-carbodithioat (Schl32259):

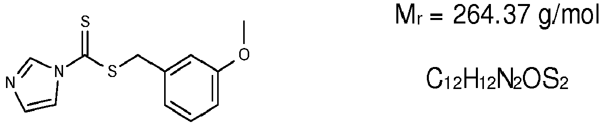

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 603 mg 1-(Brommethyl)-3-methoxybenzen (3.0 mmol, 1.0 eq) umgesetzt. Säulenchromatographie an Kieselgel (Cyclohexan/ EtOAc 3:1) ergab 210 mg von **Schl32259** (26.5%) in Form eines gelben Feststoffes.

| | | | | |
|---|---|---|---|---|
| **DC:** | R_{f} = 0.11 (Cyclohexan/ EtOAc 4:1). | | | |
| **Fp**.**:** | 144 °C | | | |
| **¹H-NMR:** | (CDCl₃, 400 MHz), δ [ppm] = 8.45 (s, 1H, N-CH=N), 7.75-7.72 (m, 1H, N-CH=CH-N), 7.24 (*t*, ³J = 7.8 Hz, 1H, C-CH-C*H*-CH-C-OMe), 7.07 (s, 1H, C-C*H*-C-OMe), 6.94 (*d*, ³J = 7.6 Hz, 1H, C-C*H*-CH-CH-C-OMe), 6.92-6.88 (m, 1H, N-CH=C*H*-N), 6.86-6.81 (m, 1H, C-CH-CH-C*H*-C-OMe), 4.54 (s, 2H, S-C*H*₂₋C), 3.78 *(s,* O-C*H*₃). | | | |
| **¹³C**-**NMR:** | (CDCl₃, 100 MHz), δ [ppm] = 197.4 (C=S), 156.0 (C-OMe), 135.8 (N-CH=N-CH-CH), 135.0 (CH₂-C-CH), 131.8 (N-CH=N-CH-CH), 130.1 (C-CH-CH-CH-C-OMe), 121.7 (C-CH-CH-CH-C-OMe), 117.8 (N-CH=N-CH-CH), 115.1 (C-CH-C-OMe), 113.8 (C-CH-CH-CH-C-OMe), 55.4 (O-CH₃), 41.9 (S-CH₂-C). | | | |
| **IR (ATR):** | v (cm⁻¹) = 3129 (w), 3116 (w), 3051 (w), 3007 (w), 2953 (w), 2925 (w), 2836 (w), 1594 (m), 1458 (m), 1429 (w), 1366 (m), 1291 (m), 1266 (vs), 1215 (vs), 1158 (m), 1108 (m), 1087 (w), 1078 (w), 1053 (s), 1032 (vs), 1005 (vs), 992 (w), 935 (w), 886 (m), 830 (vs), 796 (s), 742 (w), 724 (vs), 691 (s), 639 (vs), 608 (w), 557 (w), 544 (w), 533 (w), 487 (w), 455 (w). | | | |
| **MS** (ESI+): | m/z (%) = 265 (100, [M+H]⁺) | | | |
| **HRMS** (ESI+): | m/z für C₁₂H₁₂N₂OS₂ | | | |
| | ber.: | 264.0391 | | gef.: 264.0415 |
| **EA:** | ber.: | C: 54.52 | H: 4.58 | N: 10.60 |
| | gef.: | C: 54.11 | H: 4.53 | N: 10.41 |

### Darstellung von (3,5-Dimethoxyphenyl)methylimidazol-1-carbodithioat (Schl32260):

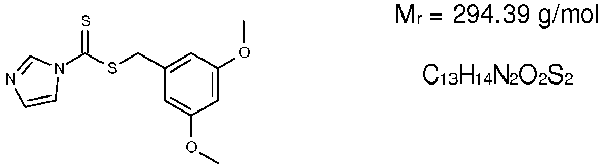

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 693 mg 1-(Brommethyl)-3,5-dimethoxybenzen (3.0 mmol, 1.0 eq) umgesetzt. Säulenchromatographie an Kieselgel (Cyclohexan/ EtOAc 3:1) ergab 175 mg von **Schl32260** (19.8%) in Form eines gelben Feststoffes.

| | | | | |
|---|---|---|---|---|
| **DC:** | R_{f} = 0.16 (Cyclohexan/ EtOAc 4:1). | | | |
| **Fp**.**:** | 151 °C | | | |
| **¹H-NMR:** | (CDCl₃, 400 MHz), δ [ppm] = 8.46 (*s*, 1H, N-C*H*=N), 7.76-7.73 (*m*, 1H, N-C*H*=CH-N), 7.10-7.07 (*m*, 1H, N-CH=C*H*-N), 6.53-6.49 (*m*, 2H, CH₂-C-C*H*-C-OMe, CH₂-C-C*H*-C-OMe), 6.41-6.38 (*m*, 1H, C(-OMe)-C*H*-C(-OMe)), 4.24 (*s*, 2H, S-C*H₂*-C), 3.77 (*s*, 6H, O-C*H₃*, O-C*H*₃). | | | |
| **¹³C**-**NMR:** | (CDCl₃, 100 MHz), δ [ppm] = 197.4 (C=S), 161.2 (C-OMe, C-OMe), 135.8 (N-CH=N-CH-CH), 135.6 (CH₂-C-CH), 131.6 (N-CH=N-CH-CH), 117.8 (N-CH=N-CH-CH), 107.5 (C-CH-C-OMe, C-CH-C-OMe), 100.2 (C(-OMe)-CH-C(-OMe)), 55.5 (O-CH₃), 42.1 (S-CH₂-C). | | | |
| **IR (ATR):** | v (cm⁻¹) = 3129 (w), 3023 (w), 2967 (w), 1592 (m), 1525 (w), 1462 (m), 1429 (w), 1370 (w), 1342 (w), 1301 (w), 1291 (w), 1276 (s), 1222 (w), 1204 (s), 1163 (w), 1146 (s), 1107 (w), 1087 (w), 1046 (vs), 1003 (m), 954 (w), 926 (w), 886 (w), 863 (w), 834 (s), 745 (w), 711 (s), 687 (m), 638 (s), 605 (w), 536 (w), 528 (w), 480 (w), 422 (w), 401 (w). | | | |
| **MS** (ESI+): | m/z (%) = 295 (100, [M+H]⁺) | | | |
| **HRMS** (ESI+): | m/z für C₁₃H₁₄N₂O₂S₂ | | | |
| | ber.: | 294.0497 | | gef.: 294.0495 |
| **EA**: | ber.: | C:53.04 | H:4.79 | N:9.52 |
| | gef.: | C:53.17 | H:4.74 | N:9.46 |

### Darstellung von Ethylimidazol-1-carbodithioat (Schl32263):

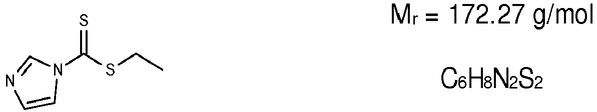

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 0.24 mL Ethyliodid (3.0 mmol, 1.0 eq) umgesetzt. Säulenchromatographie an Kieselgel (Cyclohexan/ EtOAc 5:1) ergab 493 mg von **Schl32263** (95.4%) in Form eines gelben Öls.

| | | | | |
|---|---|---|---|---|
| **DC:** | R_{f} = 0.17 (Cyclohexan/ EtOAc 6:1). | | | |
| **¹H-NMR:** | (CDCl₃, 400 MHz), δ [ppm] = 8.45 (*s*, 1H, N-CH=N), 7.77-7.74 (*m*, 1H, N-C*H*=CH-N), 7.10-7.06 (*m*, 1H, N-CH=C*H*-N), 3.37 (q, ³J = 7.3 Hz, 2H, S-C*H₂*-CH₃), 1.41 (t, ³J = 7.4 Hz, 3H, S-CH₂-C*H*₃). | | | |
| **¹³C**-**NMR:** | (CDCl₃, 100 MHz), δ [ppm] = 198.2 (C=S), 133.7 (N-CH=N-CH-CH), 131.5 (N-CH=N-CH-CH), 117.8 (N-CH=N-CH-CH), 31.4 (S-CH₂-CH₃), 12.6 (S-CH₂-CH₃). | | | |
| **IR (ATR):** | v (cm⁻¹) = 3120 (w), 2972 (w), 2930 (w), 2873 (w), 2159 (w), 1695 (w), 1528 (w), 1466 (s), 1404 (w), 1367 (vs), 1294 (s), 1272 (vs), 1238 (vs), 1221 (vs), 1092 (s), 1061 (vs), 1006 (vs), 971 (w), 890 (w), 834 (vs), 747 (m), 724 (w), 664 (w), 643 (m), 613 (w), 537 (w). | | | |
| **MS** (ESI+): | m/z (%) = 173 (100, [M+H]⁺) | | | |
| **HRMS** (ESI+): | m/z für C₆H₈N₂S₂ | | | |
| | ber.: | 172.0129 | | gef.: 172.0143 |
| **EA:** | ber.: | C:41.83 | H: 4.68 | N: 16.26 |
| | gef.: | C:41.43 | H: 4.94 | N: 16.23 |

### Darstellung von Propylimidazol-1-carbodithioat (Schl32264):

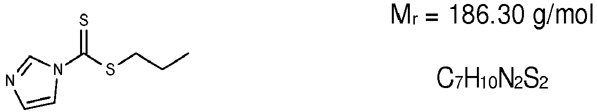

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 0.29 mL Propyliodid (3.0 mmol, 1.0 eq) umgesetzt. Säulenchromatographie an Kieselgel (Cyclohexan/ EtOAc 5:1) ergab 501 mg von **Schl32264** (89.6%) in Form eines gelben Öls.

| | | | | |
|---|---|---|---|---|
| **DC:** | R_{f} = 0.19 (Cyclohexan/ EtOAc 6:1). | | | |
| **¹H-NMR:** | (CDCl₃, 400 MHz), δ [ppm] = 8.46 (*s*, 1H, N-C*H*=N), 7.79-7.73 (*m*, 1H, N-C*H*=CH-N), 7.10-7.02 (*m*, 1H, N-CH=C*H*-N), 3.34 (*t*, ³J = 7.3 Hz, 2H, S-C*H₂*-CH₂-CH₃), 1.80 *(sept,* ³J = 7.3 Hz, 2H, S-CH₂-C*H*₂CH₃), 1.06 (t, ³J = 7.3 Hz, 3H, S-CH₂-CH₂-C*H*₃). | | | |
| **¹³C**-**NMR:** | (CDCl₃, 100 MHz), δ [ppm] = 198.4 (C=S), 135.7 (N-CH=N-CH-CH), 131.4 (N-CH=N-CH-CH), 117.8 (N-CH=N-CH-CH), 38.9 (S-CH₂-CH₂-CH₃), 21.1 (S-CH₂-CH₂-CH₃), 13.6 (S-CH₂-CH₂-CH₃). | | | |
| **IR (ATR):** | v (cm⁻¹) = 3120 (w), 2965(m), 2932 (m), 2874 (w), 2063 (w), 1662 (w), 1528 (w), 1465 (s),(s), 1399 (w), 1367 (vs), 1271 (vs), 1238 (vs), 1221 (vs), 1093m), 1050 (vs), 1006 (vs), 890 (w), 833 (vs), 745 (w), 723 (w),643 (m), 613 (w), 536 (w), 417 (w). | | | |
| **MS** (ESI+): | m/z (%) = 187 (100, [M+H]⁺) | | | |
| **HRMS** (ESI+): | m/z für C₇H₁₀N₂S₂ | | | |
| | ber.: | 186.0285 | | gef.: 186.0293 |
| **EA:** | ber.: | C: 45.13 | H: 5.41 | N: 15.04 |
| | gef.: | C: 44.76 | H: 5.52 | N: 14.84 |

### Darstellung von Butylimidazol-1-carbodithioat (Schl32265):

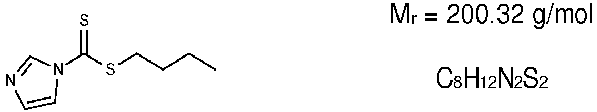

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 0.34 mL Butyliodid (3.0 mmol, 1.0 eq) umgesetzt. Säulenchromatographie an Kieselgel (Cyclohexan/ EtOAc 5:1) ergab 561 mg von **Schl32265** (93.3%) in Form eines gelben Öls.

| | | | | |
|---|---|---|---|---|
| DC: | R_{f} = 0.20 (Cyclohexan/ EtOAc 5:1). | | | |
| **¹H-NMR:** | (CDCl₃, 400 MHz), δ [ppm] = 8.45 (s, 1H, N-CH=N), 7.78-7.74 (*m*, 1H, N-CH=CH-N), 7.09-7.04 (m, 1H, N-CH=C*H*-N), 3.39-3.32 (*m*, 2H, S-C*H₂*-CH₂-CH₂-CH₃), (*t*, ³J = 7.3 Hz, 2H, S-CH₂-C*H₂*-CH₂-CH₃), 1.79-1.69 (*m*, 2H, S-CH₂-C*_{H2}*-CH₂-CH₃), 1.51-1.40 (*m*, 2H, S-CH₂-CH₂-C*H₂*-CH₃), 0.98-0.92 (*m*, 3H, S-CH₂-CH₂-CH₂-C*H₃*). | | | |
| **¹³C**-**NMR:** | (CDCl₃, 100 MHz), δ [ppm] = 198.4 (C=S), 135.7 (N-CH=N-CH-CH), 131.4 (N-CH=N-CH-CH), 117.8 (N-CH=N-CH-CH), 36.9 (S-CH₂-CH₂-CH₂-CH₃), 29.4 (S-CH₂-CH₂-CH₂-CH₃), 22.1 (S-CH₂-CH₂-CH₂-CH₃), 13.6 (S-CH₂-CH₂-CH₂-CH₃). | | | |
| **IR** (ATR): | v (cm⁻¹) = 3121 (w), 2960 (m), 2932 (m), 2873 (w), 2373 (w), 2148 (w), 1695 (w), 1528 (w), 1466 (s), 1400 (w), 1368 (vs), 1271 (vs), 1240 (vs), 1220 (vs), 1100 (m), 1098 (m), 1052 (s), 1006 (s), 890 (w), 833 (s), 745 (w), 723 (w), 664 (w), 643 (m), 613 (w), 537 (w), 400 (w). | | | |
| **MS** (ESI+): | m/z (%) = 201 (100, [M+H]⁺) | | | |
| **HRMS** (ESI+): | m/z für C₈H₁₂N₂S₂ | | | |
| | ber.: | 200.0442 | | gef.: 200.0462 |
| **EA:** | ber.: | C:47.97 | H: 6.04 | N: 13.98 |
| | gef.: | C:47.94 | H: 6.16 | N: 13.97 |

### Darstellung von Cyclohexylmethylimidazol-1-carbodithioat (Schl32276):

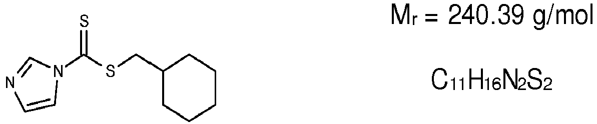

Gemäß **AAV9** wurden 204 mg Imidazol (3.0 mmol, 1.0 eq), 637 mg Kaliumphosphat (3.0 mmol, 1.0 eq), 0.54 mL Kohlenstoffdisulfid (9.0 mmol, 3.0 eq) und 0.42 mL Brommethylcyclohexan (3.0 mmol, 1.0 eq) umgesetzt. Säulenchromatographie an Kieselgel (Cyclohexan/ EtOAc 5:1) ergab 218 mg von **Schl32276** (30.2%) in Form eines gelbbraunen Öls.

| | | | | |
|---|---|---|---|---|
| **DC:** | R_{f} = 0.24 (Cyclohexan/ EtOAc 5:1). | | | |
| **¹H-NMR:** | (CDCl₃, 400 MHz), δ [ppm] = 8.45 (s, 1H, N-C*H*=N), 7.76-7.73 (*m*, 1H, N-C*H*=CH-N), 7.07-7.04 (*m*, 1H, N-CH=C*H*-N), 3.26 (*d,* ³J = 6.9 Hz, 2H, S-C*H₂*-CH), 1.86-1.78 (*m*, 2H, C*H*_{(Cyclohexyl)}), 1.75-1.59 (m, 4H, C*H*_{(Cyclohexyl)}), 1.25-0.98 (m, 5H, C*H*_{(Cyclohexyl)}, S-CH₂-C*H*). | | | |
| **¹³C**-**NMR:** | (CDCl₃, 100 MHz), δ [ppm] = 198.7 (C=S), 135.7 (N-CH=N-CH-CH), 131.4 (N-CH=N-CH-CH), 117.9 (N-CH=N-CH-CH), 44.1 (S-CH₂-CH), 36.6 (S-CH₂-CH), 33.1 (CH_{(Cyclohexyl)}), 31.0 (CH_{(Cyclohexyl)}), 26.1 (CH_{(Cyclohexyl)}). | | | |
| **IR (ATR):** | v (cm⁻¹) = 3122 (w), 2922 (m), 2851 (m), 2375 (w), 2154 (w), 1528 (w), 1466 (m), 1448 (m), 1397 (w), 1367 (s), 1270 (s), 1238 (m), 1219 (s), 1090 (m), 1053 (s), 1005 (m), 961 (w), 926 (w), 891 (w), 831 (s), 744 (w), 722 (w), 643 (m), 613 (w), 536 (w), 446 (w). | | | |
| **MS** (ESI+): | m/z (%) = 241 (100, [M+H]⁺) | | | |
| **HRMS** (ESI+): | m/z für C₁₁H₁₆N₂S₂ | | | |
| | ber.: | 240.0755 | | gef.: 240.0766 |
| **EA:** | ber.: | C: 54.96 | H: 6.71 | N: 11.65 |
| | gef.: | C:55.11 | H: 6.79 | N: 11.37 |

### Darstellung von 3-Benzyl-2-cyano-1,1-diethylguanidin (Schl32145):

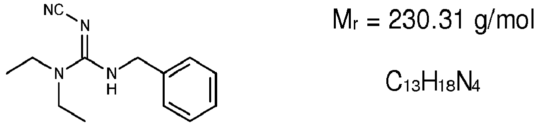

513 mg 3-Cyano-1,1-diethyl-2-methylisothioharnstoff (**Schl32148**, 3.0 mmol, 1.0 eq) wurden in 40 mL trockenem Tetrahydrofuran gelöst, auf -80 °C gekühlt, mit 0.37 mL Benzylamin (3.3 mmol, 1.1 eq) und 1.50 mL DIPEA (9.0 mmol, 3.0 eq) versetzt und innerhalb von 4 Stunden wieder auf Raumtemperatur erwärmt. Nach 2 Stunden bei Raumtemperatur wurde das Reaktionsgemisch 5 Stunden unter Rückfluss erhitzt. Das Lösungsmittel wurde am Rotavapor entfernt. Säulenchromatographie an Kieselgel (Cyclohexan/ EtOAc 1:2) ergab 329 mg von **Schl32145** (47.7%) in Form eines farblosen Feststoffes.

| | | | | |
|---|---|---|---|---|
| **DC:** | R_{f} = 0.15 (Cyclohexan/ EtOAc 1:2). | | | |
| **Fp**.**:** | 174 °C | | | |
| **¹H-NMR:** | (DMSO-D₆, 400 MHz), δ [ppm] = 8.89 (*sbr*, 1H, N*H*), 7.39-7.22 (*m*, 5H, C*H*_{(Phenyl)}), 4.39 (*s*, 2H, NH-C*H*₂), 3.52 (*q*, ³J = 7.0 Hz, 4H, CH₃-CH₂), 1.29 (*t,* ³J = 7.1 Hz, 6H, *CH₃*-CH₂). | | | |
| **¹³C**-**NMR:** | (DMSO-D₆, 100 MHz), δ [ppm] = 161.3 (S-C-N), 137.7 (C-CH-CH), 129.0 (C-CH-CH, C-CH-CH), 128.0 (C-CH-CH, C-CH-CH), 127.6 (C-CH-CH-CH), 116.9 (CN), 44.9 (CH₂-C), 42.3 (CH₂-CH₃), 13.6 (CH₂-CH₃). | | | |
| **IR (ATR):** | v (cm⁻¹) = 3277 (w), 3055 (w), 3033 (w), 3004 (w), 2157 (s), 1958 (w), 1603 (w), 1541 (s), 1511 (vs), 1454 (w), 1421 (m), 1356 (w), 1330 (w), 1315 (w), 1283 (s), 1253 (s), 1195 (w), 1114 (m), 1089 (m), 1069 (w), 1030 (w), 1020 (w), 983 (w), 960 (m), 910 (w), 810 (w), 744 (w), 736 (w), 696 (s), 675 (w), 625 (s), 583 (m), 549 (w), 504 (w), 448 (w). | | | |
| **MS** (ESI+): | m/z (%) = 231 (100, [M+H]⁺) | | | |
| **HRMS** (ESI+): | m/z für C₁₃H₁₈N₄ | | | |
| | ber.: | 230.1531 | | gef.: 230.1536 |
| **EA:** | ber.: | C: 67.80 | H: 7.88 | N: 24.33 |
| | gef.: | C: 68.01 | H: 7.84 | N: 24.57 |

### Darstellung von 1-Benzyl-3-cyano-2-methylisothioharnstoff (Schl32148):

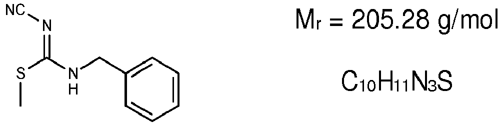

0.55 mL Benzylamin (5.0 mmol, 1.0 eq) und 529 mg Natriumcarbonat (5.0 mmol, 1.0 eq) wurden in 40 mL trockenem Tetrahydrofuran gelöst, 15 Minuten bei Raumtemperatur gerührt und anschließend auf -80 °C gekühlt, 804 mg *Bis*(methylsulfanyl)methylencyanamid (5.5 mmol, 1.1 eq) zugegeben, auf Raumtemperatur erwärmt und weitere 12 Stunden gerührt. Anschließend wurde das Lösungsmittel am Rotavapor entfernt. Säulenchromatographie an Kieselgel (Cyclohexan/ EtOAc 4:3) ergab 740 mg von **Schl32148** (72.1 %) in Form eines farblosen Feststoffes.

| | | | | |
|---|---|---|---|---|
| **DC:** | R_{f} = 0.17 (Cyclohexan/ EtOAc 4:3). | | | |
| **Fp**.**:** | 159 °C | | | |
| **¹H-NMR:** | (DMSO-D₆, 400 MHz), δ [ppm] = 8.87 (*sbr*, 1H, N*H*), 7.35-7.20 (*m*, 5H, C*H*_{(Phenyl)}), 4.46 (*s*, 2H, NH-C*H*₂), 2.59 (*s*, 3H, S-C*H₃*). | | | |
| **¹³C**-**NMR:** | (DMSO-D₆, 100 MHz), δ [ppm] = 164.1 (S-C-N), 138.3 (C-CH-CH), 129.0 (C-CH-CH, C-CH-CH), 127.9 (C-CH-CH, C-CH-CH), 127.7 (C-CH-CH-CH), 116.3 (CN), 46.7 (CH₂-C), 14.6 (S-CH₃). | | | |
| **IR (ATR):** | v (cm⁻¹) = 3277 (w), 3055 (w), 3033 (w), 3004 (w), 2157 (s), 1541 (s), 1511 (vs), 1454 (w), 1421 (m), 1356 (w), 1330 (w), 1315 (w), 1283 (s), 1253 (s), 1195 (w), 1114 (m), 1089 (m), 1069 (w), 1030 (w), 960 (m), 910 (w), 810 (w), 744 (w), 736 (w), 696 (s), 675 (w), 625 (s), 583 (m), 549 (w), 504 (w), 448 (w). | | | |
| **MS** (ESI+): | m/z (%) = 206 (100, [M+H]⁺) | | | |
| **HRMS** (ESI+): | m/z für C₁₀H₁₁N₃S | | | |
| | ber.: | 205.0674 | | gef.: 205.0659 |
| **EA:** | ber.: | C: 58.51 | H: 5.40 | N: 20.47 |
| | gef.: | C: 58.90 | H: 5.49 | N: 20.20 |

### Darstellung von 3-Cyano-1,1-diethyl-2-methylisothioharnstoff (Schl32149):

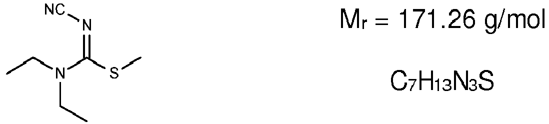

0.52 mL Diethylamin (5.0 mmol, 1.0 eq) und 529 mg Natriumcarbonat (5.0 mmol, 1.0 eq) wurden in 40 mL trockenem Tetrahydrofuran gelöst, 15 Minuten bei Raumtemperatur gerührt und anschließend auf -80 °C gekühlt. 804 mg *Bis*(methylsulfanyl)methylencyanamid (5.5 mmol, 1.1 eq) wurden zugegeben, auf Raumtemperatur erwärmt und weitere 12 Stunden gerührt. Anschließend wurde das Lösungsmittel am Rotavapor entfernt. Säulenchromatographie an Kieselgel (Cyclohexan/ EtOAc 1:1) ergab 383 mg von **Schl32149** (44.8%) Form eines blassgelben Öls.

| | | | | |
|---|---|---|---|---|
| **DC:** | R_{f} = 0.24 (Cyclohexan/ EtOAc 1:1). | | | |
| **¹H-NMR:** | (CDCl₃, 400 MHz), δ [ppm] = 3.51 (*q*, ³J = 7.1 Hz, 4H, CH₃-C*H₂*), 2.69 (s, 3H, S-C*H₃*), 1.36 (*t*, ³J = 7.1 Hz, 6H, C*H*₃-CH₂). | | | |
| **¹³C**-**NMR:** | (CDCl₃, 100 MHz), δ [ppm] = 168.4 (S-C-N), 115.6 (CN), 46.1 (CH₂-CH₃), 16.2 (S-CH₃), 16.1 (CH₂-CH₃). | | | |
| **IR (ATR):** | v (cm⁻¹) = 2976 (w), 2174 (s), 1540 (vs), 1422 (s), 1380 (m), 1357 (w), 1307 (w), 1271 (w), 1216 (m), 1145 (w), 1095 (m), 1075 (w), 1044 (w), 1002 (w), 979 (w), 852 (w), 782 (w), 634 (w), 582 (w), 545 (w). | | | |
| **MS** (ESI+): | m/z (%) = 172 (100, [M+H]⁺) | | | |
| **HRMS** (ESI+): | m/z für C₇H₁₃N₃S | | | |
| | ber.: | 171.0830 | | gef.: 171.0818 |
| **EA:** | ber.: | C:49.09 | H:7.65 | N:24.54 |
| | gef.: | C:49.17 | H:7.84 | N:24.57 |

### Darstellung von 3-Benzyl-1,1-diethylthioharnstoff (Schl32151):

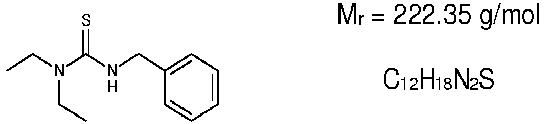

0.39 mL Benzylamin (3.6 mmol, 1.2 eq) und 1.05 mL Triethylamin (7.5 mmol, 2.5 eq) wurden in 15 mL Tetrahydrofuran gelöst und 15 Minuten bei Raumtemperatur gerührt. 455 mg *N*,*N* Diethylthiocarbamoylchlorid (3.0 mmol, 1.0 eq) wurden zugegeben, weitere 18 Stunden bei Raumtemperatur gerührt, anschließend mit EtOAc extrahiert (5 x 50 mL), die vereinigten organischen Phasen über MgSO₄ getrocknet, filtriert und das Lösungsmittel am Rotavapor entfernt. Säulenchromatographie an Kieselgel (Cyclohexan/ EtOAc 3:1) ergab 254 mg von **Schl32151** (38.1 %) in Form eines blassgelben Öls.

| | | | | |
|---|---|---|---|---|
| **DC:** | R_{f} = 0.22 (Cyclohexan/ EtOAc 3:1). | | | |
| **¹H-NMR:** | (CDCl₃, 400 MHz), δ [ppm] = 7.36-7.23 (m, 5H, CH₂-C-C*H-*CH, CH₂-C-C*H*-CH, CH₂-C-CH-C*H*, CH₂-C-CH-C*H*, CH₂-C-CH-CH-C*H*), 5.52 *(sbr,* 1 H, N*H*), 4.86 (*d*, ³J = 4.8 Hz, 2H, NH-C*H₂-C),* 3.66 (*q*, ³J = 7.1 Hz, 4H, CH₃-C*H₂*, CH₃-C*H₂*), 1.24-1.18 (m, 6H, CH₂-C*H₃*, CH₂-C*H₃*). | | | |
| **¹³C-NMR:** | (CDCl₃, 100 MHz), δ [ppm] = 180.4 (C=S), 138.3 (C-CH-CH), 128.9 (C-CH-CH, C-CH-CH), 128.0 (C-CH-CH, C-CH-CH), 127.7 (C-CH-CH-CH), 50.4 (CH₂-CH₃, CH₂-CH₃), 45.3 (S-CH₂-C), 12.8 (CH₂-CH₃, CH₂-C*H₃*). | | | |
| **IR** (ATR): | v (cm⁻¹) = 3301 (w), 2974 (w), 2930 (w), 1526 (vs), 1495 (m), 1453 (w), 1441 (w), 1407 (w), 1373 (m), 1345 (m), 1323 (m), 1272 (s), 1240 (m), 1211 (m), 1135 (m), 1093 (m), 1074 (m), 1045 (w), 1028 (w), 1001 (w), 961 (w), 926 (w), 861 (w), 794 (w), 732 (m), 695 (s), 646 (w), 591 (w), 541 (w), 448 (w), 404 (w). | | | |
| **MS** (ESI+): | m/z (%) = 223 (100, [M+H]⁺) | | | |
| **HRMS** (ESI+): | m/z für C₁₂H₁₈N₂S | | | |
| | ber.: | 222.1191 | | gef.: 222.1184 |
| **EA:** | ber.: | C:64.82 | H:8.16 | N:12.60 |
| | gef.: | C:64.56 | H:7.94 | N:12.26 |

### Darstellung von Benzyl-N,N-diethylcarbamat (Schl32161):

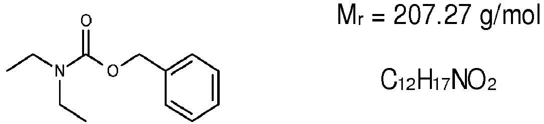

0.66 mL Benzylalkohol (7.5 mmol, 1.0 eq) wurden in 25 mL Pyridin gelöst und 15 Minuten bei Raumtemperatur gerührt. 1.05 mL *N*,*N-*Diethylcarbamoylchlorid (8.25 mmol, 1.1 eq) wurden zugetropft, weitere 12 Stunden bei 110 °C gerührt, anschließend mit 50 mL Wasser, 30 mL 1M HCL und 50 mL EtOAc versetzt, mit EtOAc extrahiert (5 x 75 mL), die vereinigten organischen Phasen mit 100 mL ges. NaHCO₃-Lösung gewaschen, über MgSO₄ getrocknet, filtriert und das Lösungsmittel am Rotavapor entfernt. Säulenchromatographie an Kieselgel (EtOAc) ergab 577 mg von **Schl32161** (37.9%) in Form eines blassgelben Öls.

| | | | | |
|---|---|---|---|---|
| **DC:** | R_{f}= 0.49 (EtOAc). | | | |
| **¹H-NMR:** | (CDCl₃, 400 MHz), δ [ppm] = 7.37-7.22 (*m*, 5H, CH₂-C-C*H-*CH, CH₂-C-C*H*-CH, CH₂-C-CH-C*H*, CH₂-C-CH-C*H*, CH₂-C-CH-CH-C*H*), 5.12 (s, 2H, O-C*H₂*-C), 3.29 (*q*, ³J = 7.1 Hz, 4H, CHs-C*H₂*, CHs-C*H₂*), 1.10 (*t,* ³J = 6.6 Hz, 6H, C*H3*-CH₂, C*H₃*-CH₂). | | | |
| **¹³C-NMR:** | (CDCl₃, 100 MHz), δ [ppm] = 155.9 (C=O), 137.2 (C-CH-CH), 128.5 (C-CH-CH, C-CH-CH), 127.9 (C-CH-CH, C-CH-CH), 127.8 (C-CH-CH-CH), 66.8 (O-CH₂-C), 42.0 (CH₂-CH₃), 41.4 (CH₂-CH₃), 14.2 (CH₂-CH₃), 13.6 (CH₂-CH₃). | | | |
| **IR (ATR):** | v (cm⁻¹) = 3033 (w), 2974 (w), 2934 (w), 1694 (vs), 1587 (w), 1536 (w), 1498 (w), 1477 (m), 1455 (m), 1422 (s), 1379 (w), 1365 (m), 1315 (m), 1269 (vs), 1212 (w), 1164 (vs), 1064 (s), 997 (s), 928 (w), 905 (w), 799 (w), 767 (m), 734 (m), 696 (vs), 626 (m), 579 (w), 561 (w), 452 (w). | | | |
| **MS** (ESI+): | m/z (%) = 208 (100, [M+H]⁺) | | | |
| **HRMS** (ESI+): | m/z für C₁₂H₁₇NO₂ | | | |
| | ber.: | 207.1259 | | gef.: 207.1246 |
| **EA:** | ber.: | C:69.54 | H:8.27 | N: 6.76 |
| | gef.: | C:69.50 | H:8.18 | N:6.80 |

### Darstellung von 3-Benzyl-1,1-diethylharnstoff (Schl32162):

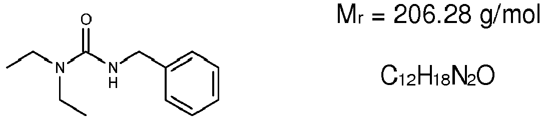

0.66 mL Benzylamin (6.0 mmol, 2.0 eq) und 1.25 mL Triethylamin (9.0 mmol, 3.0 eq) wurden in 15 mL Dichlormethan gelöst und 15 Minuten bei Raumtemperatur gerührt. 0.38 mL *N*,*N-*Diethylcarbamoylchlorid (3.0 mmol, 1.0 eq) wurden langsam zugetropft, weitere 12 Stunden bei Raumtemperatur gerührt und das Lösungsmittel wurde am Rotavapor entfernt. Säulenchromatographie an Kieselgel (EtOAc) ergab 580 mg von **Schl32162** (93.9%) in Form eines blassgelben Öls.

| | | | | |
|---|---|---|---|---|
| **DC:** | R_{f} = 0.39 (EtOAc). | | | |
| **¹H-NMR:** | (CDCl₃, 400 MHz), δ [ppm] = 7.29-7.18 (m, 5H, CH₂-C-C*H-*CH, CH₂-C-C*H*-CH, CH₂-C-CH-C*H*, CH₂-C-CH-C*H*, CH₂-C-CH-CH-C*H*), 4.92 (*sbr,* 1H, N*H*), 4.36 (s, 2H, N-C*H₂-*C), 3.24 (*q,* ³J = 7.1 Hz, 2H, CH₃-C*H₂*), 3.18 (*q*, ³J = 7.1 Hz, 2H, CH₃-C*H₂*)*,* 1.08 (*t,* ³J = 6.6 Hz, 3H, C*H₃*-CH₂), 1.01 (*t,* ³J = 7.1 Hz, 3H, C*H*₃-CH₂). | | | |
| **¹³C-NMR:** | (CDCl₃, 100 MHz), δ [ppm] = 157.4 (C=O), 140.2 (C-CH-CH), 128.6 (C-CH-CH, C-CH-CH), 127.6 (C-CH-CH, C-CH-CH), 127.1 (C-CH-CH-CH), 44.8 (N-CH₂-C), 41.2 (CH₂-CHs, CH₂-CH₃), 14.0 (CH₂-CH₃, CH₂-C*H₃*). | | | |
| **IR (ATR):** | v (cm⁻¹) = 2248 (w), 2975 (w), 1621 (s), 1526 (vs), 1493 (s), 1451 (m), 1408 (m), 1377 (m), 1358 (m), 1278 (s), 1212 (m), 1076 (m), 1027 (w), 727 (w), 697 (s), 576 (m), 481 (m). | | | |
| **MS** (ESI+): | m/z (%) = 207 (100, [M+H]⁺) | | | |
| **HRMS** (ESI+): | m/z für C₁₂H₁₈N₂O | | | |
| | ber.: | 206.1419 | | gef.: 206.1419 |
| **EA:** | ber.: | C:69.87 | H:8.80 | N:13.58 |
| | gef.: | C:70.11 | H: 8.76 | N:13.34 |

### Darstellung von S-Benzyl-N,N diethylcarbamothioat (Schl32163):

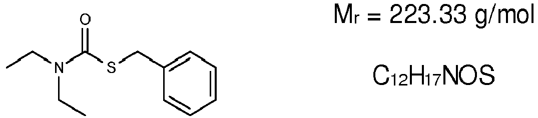

0.88 mL Benzylmercaptan (7.5 mmol, 1.0 eq) und 421 mg Kaliumhydroxid (7.5 mmol, 1.0 eq) wurden in 15 mL Aceton gelöst und 15 Minuten bei Raumtemperatur gerührt. 1.05 mL *N*,*N-*Diethylcarbamoylchlorid (8.75 mmol, 1.1 eq) wurden langsam zugetropft, weitere 36 Stunden bei Raumtemperatur gerührt und das Lösungsmittel wurde am Rotavapor entfernt. Säulenchromatographie an Kieselgel (Cyclohexan/ EtOAc 4:1) ergab 1163 mg von **Schl32163** (69.4%) in Form eines blassgelben Öls.

| | | | | |
|---|---|---|---|---|
| **DC:** | R_{f} = 0.54 (Cyclohexan/ EtOAc 4:1) | | | |
| **¹H-NMR:** | (CDCl₃, 400 MHz), δ [ppm] = 7.39-7.18 (m, 5H, CH₂-C-C*H-*CH, CH₂-C-C*H*-CH, CH₂-C-CH-C*H*, CH₂-C-CH-C*H*, CH₂-C-CH-CH-C*H*), 4.16 (s, 2H, S-C*H₂*-C), 3.36 (q, ³J = 7.1 Hz, 4H, CHs-C*H₂*, CHs-C*H₂*), 1.15 (*t*, ³J = 7.1 Hz, 6H, C*H3*-CH₂, C*H₃*-CH₂). | | | |
| **¹³C-NMR:** | (CDCl₃, 100 MHz), δ [ppm] = 166.7 (C=O), 138.5 (C-CH-CH), 129.1 (C-CH-CH, C-CH-CH), 128.6 (C-CH-CH, C-CH-CH), 127.2 (C-CH-CH-CH), 42.1 (N-CH₂-C), 34.7 (CH₂-CHs, CH₂-CH₃), 13.8 (CH₂-CH₃, CH₂-C*H₃*). | | | |
| **IR (ATR):** | v (cm⁻¹ ) = 3062 (w), 3029 (w), 2974 (w), 1643 (vs), 1603 (w), 1584 (w), 1495 (w), 1454 (m), 1404 (vs), 1379 (m), 1362 (m), 1307 (m), 1249 (vs), 1218 (s), 1114 (vs), 1097 (m), 1071 (m), 1029 (w), 1003 (w), 941 (w), 858 (s), 817 (w), 771 (w), 697 (vs), 661 (s), 506 (w), 428 (w), 405 (w). | | | |
| **MS** (ESI+): | m/z (%) = 224 (100, [M+H]⁺) | | | |
| **HRMS** (ESI+): | m/z für C₁₂H₁₇NOS | | | |
| | ber.: | 223.1031 | | gef.: 223.1044 |
| **EA:** | ber.: | C:64.53 | H:7.67 | N: 6.27 |
| | gef.: | C:64.22 | H:7.56 | N: 6.28 |

### Darstellung von N,N Diethylbenzencarbothioamid (Schl32352):

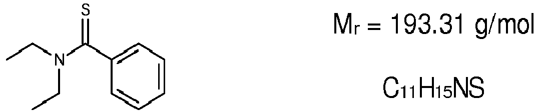

0.36 mL Thiobenzoesäure (3.0 mmol, 1.0 eq) und 0.31 mL Diethylamin (3.0 mmol, 1.0 eq) wurden in 25 mL Dichlormethan gelöst, auf 0 °C gekühlt, 15 Minuten gerührt und mit 2.03 mL Triethylamin (10.5 mmol, 3.5 eq) versetzt. Nach weiteren 15 Minuten Reaktionszeit wurde das Reaktionsgemisch mit 1021 mg HOBt (4.5 mmol, 1.5 eq) und 1447 mg EDC-HCl (4.5 mmol, 1.5 eq) versetzt, weitere 60 Minuten bei 0 °C gerührt und anschließend auf Raumtemperatur erwärmt. Nach 24 Stunden Reaktionszeit wurde das Lösungsmittel am Rotavapor entfernt. Säulenchromatographie an Kieselgel (Cyclohexan/ EtOAc 1:1) ergab 393 mg von **Schl32352** (67.8%) in Form eines blassgelben Öls.

| | | | | |
|---|---|---|---|---|
| **DC:** | R_{f} = 0.35 (Cyclohexan/ EtOAc 1:1). | | | |
| **¹H-NMR:** | (CDCl₃, 400 MHz), δ [ppm] = 7.37-7.31 (*m*, 5H, C*H*_{(Phenyl)}), 3.52 (q, ³J = 6.7 Hz, 2H, CH₃-C*H₂*), 3.22 (*q*, ³J = 6.7 Hz, 2H, CHs-C*H₂*), 1.21 (*q*, ³J = 7.1 Hz, 3H, CH₂-C*H₃*), 1.08 (*q*, ³J = 7.2 Hz, 3H, CH₂-C*H₃*). | | | |
| **¹³C-NMR:** | (CDCl₃, 100 MHz), δ [ppm] = 171.4 (C=S), 137.3 (C-CH-CH-CH), 129.2 (C-CH-CH-CH, C-CH-CH-CH), 128.5 (C-CH-CH-CH, C-CH-CH-CH), 43.4 (CH₂-CH₃), 39.3 (CH₂-CH₃), 14.3 (CH₂-CH₃), 13.0 (CH₂-CH₃). | | | |
| **IR (ATR):** | v (cm⁻¹) = 3060 (w), 2974 (w), 2936 (w), 1627 (vs), 1578 (w), 1497 (w), 1457 (m), 1427 (vs), 1382 (m), 1365 (m), 1314 (w), 1287 (s), 1195 (w), 1097 (s), 1072 (m), 1002 (w), 921 (w), 872 (w), 786 (m), 706 (s), 629 (w), 565 (w), 480 (w). | | | |
| **MS** (ESI+): | m/z (%) = 194 (100, [M+H]⁺) | | | |
| **HRMS** (ESI+): | m/z für C₁₁H₁₅NS | | | |
| | ber.: | 193.0925 | | gef.: 193.0913 |
| **EA:** | ber.: | C: 68.35 | H:7.82 | N:7.25 |
| | gef.: | C: 68.45 | H:7.82 | N:7.44 |

### Darstellung von Benzylbenzencarbodithioat (Schl32353):

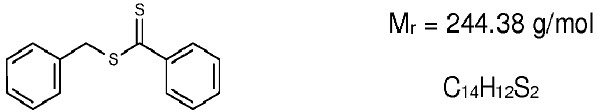

0.24 mL Thiobenzoesäure (2.0 mmol, 1.0 eq) und 0.24 mL Benzylmercaptan (2.0 mmol, 1.0 eq) wurden in 25 mL Dichlormethan gelöst, auf 0 °C gekühlt, 15 Minuten gerührt und mit 0.97 mL Triethylamin (7.0 mmol, 3.5 eq) versetzt. Nach weiteren 15 Minuten Reaktionszeit wurden 405 mg HOBt (3.0 mmol, 1.5 eq) und 575 mg EDC*HCl (3.0 mmol, 1.5 eq) zugegeben, weitere 60 Minuten bei 0 °C gerührt und anschließend auf Raumtemperatur erwärmt. Nach 24 Stunden Reaktionszeit wurde das Lösungsmittel am Rotavapor entfernt. Säulenchromatographie an Kieselgel (Cyclohexan/ EtOAc 15:1) ergab 407 mg von **Schl32353** (83.2%) in Form eines blassgelben Öls.

| | | | | |
|---|---|---|---|---|
| **DC:** | R_{f} = 0.51 (Cyclohexan/ EtOAc 15:1). | | | |
| **¹H-NMR:** | (CDCl₃, 400 MHz), δ [ppm] = 7.98 (*d*, ³J = 7.1Hz, 2H, C(=S)-C-C*H*, C(=S)-C-C*H*), 7.57 (*t,* ³J = 7.3Hz, 1H, C(=S)-C-CH-CH-C*H*), 7.44 (*t,* ³J = 7.8 Hz, 2H, C(=S)-C-CH-C*H*, C(=S)-C-CH-C*H*), 7.39 (*d,* ³J = 7.8 Hz, 2H, CH₂-C-C*H*, CH₂-C-C*H*), 7.32 (*t,* ³J = 7.3Hz, 2H, CH₂-C-CH-C*H*, CH₂-C-CH-C*H*), 7.26 (*t*, ³J = 7.2Hz, 1H, CH₂-C-CH-CH-C*H*), 4.33 (*s*, 2H, S-C*H₂*-C). | | | |
| **¹³C-NMR:** | (CDCl₃, 100 MHz), δ [ppm] = 191.4 (C=S), 144.6 (C(=S)-C), 136.0 (CH₂-C), 133.6 (C(=S)-C-CH), 129.1 (C(=S)-C-CH-CH), 128.8 (CH₂-C-CH), 128.7 (C(=S)-C-CH-CH-CH), 127.5 (CH₂-C-CH-CH), 127.4 (CH₂-C-CH-CH-CH), 33.4 (S-CH₂-C). | | | |
| **IR (ATR):** | v (cm⁻¹) = 3064 (w), 3031 (w), 1658 (vs), 1598 (w), 1522 (w), 1496 (w), 1449 (s), 1343 (w), 1241 (w), 1205 (vs), 1174 (s), 1100 (w), 1028 (w), 907 (vs), 817 (w), 770 (s), 685 (vs), 646 (vs), 617 (w), 565 (w), 474 (w). | | | |
| **MS** (ESI+): | m/z (%) = 245 (100, [M+H]⁺) | | | |
| **HRMS** (ESI+): | m/z für C₁₄H₁₂S₂ | | | |
| | ber.: | 244.0380 | | gef.: 244.0381 |
| **EA:** | ber.: | C: 68.81 | H:4.95 | N:0.00 |
| | gef.: | C: 68.64 | H: 4.91 | N: 0.21 |

### Darstellung von Dibenzylpiperazin-1,4-dicarbodithioat (Schl32142):

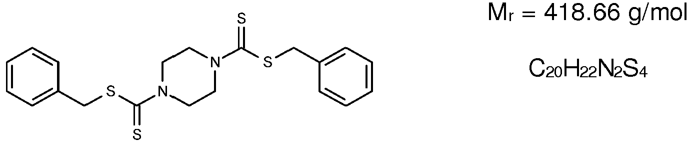

258 mg Piperazin (3.0 mmol, 1.0 eq) und 1000 mg Natriumhydroxid (25.0 mmol, 8.3 eq) wurden bei Raumtemperatur in 20 mL Wasser und 20 mL Ethanol gelöst, 30 Minuten gerührt und anschließend mit 0.45 mL Kohlenstoffdisulfid (7.5 mmol, 3.0 eq) versetzt. Nach weiteren 60 Minuten Reaktionszeit wurden 1.1 mL Benzylbromid (9.0 mmol, 3.0 eq) zugegeben und 18 Stunden gerührt. Anschließend wurde das Lösungsmittel am Rotavapor entfernt. Säulenchromatographie an Kieselgel (Cyclohexan/ EtOAc 12:1) ergab 477 mg von **Schl32142** (38.9%) in Form eines blassgelben Feststoffs.

| | | | | |
|---|---|---|---|---|
| **DC:** | R_{f} = 0.21 (Cyclohexan/ EtOAc 12:1). | | | |
| **Fp.:** | 119 °C | | | |
| **¹H-NMR:** | (CDCl₃, 400 MHz), δ [ppm] = 7.44-7.24 (*m*, 10H, C*H*_{(Phenyl)}), 4.57 (s, 4H, S-C*H₂*-C, S-C*H₂*-C), 4.51-3.99 (*m*, 8H, N-C*H₂*-C*H₂-*N, N-C*H₂*-C*H₂*-N). | | | |
| **¹³C-NMR:** | (CDCl₃, 100 MHz), δ [ppm] = 196.6 (C=S, C=S), 135.6 (S-CH₂-C-CH, S-CH₂-C-CH), 129.5 (C-CH-CH-CH, C-CH-CH-CH, C-CH-CH-CH, C-CH-CH-CH), 128.8 (C-CH-CH-CH, C-CH-CH-CH, C-CH-CH-CH, C-CH-CH-CH), 127.8 (C-CH-CH-CH, C-CH-CH-CH), 57.7 (N-CH₂-CH₂-N, N-CH₂-CH₂-N), 42.2 (S-CH₂-C, S-CH₂-C). | | | |
| **IR (ATR):** | v (cm⁻¹) = 3026 (w), 1466 (w), 1450 (m), 1436 (w), 1404 (s), 1375 (w), 1346 (m), 1275 (s), 1209 (s), 1156 (s), 1084 (w), 1040 (s), 1027 (m), 993 (s), 919 (m), 849 (m), 802 (m), 769 (w), 718 (m), 709 (m), 692 (vs), 564 (w), 473 (s). | | | |
| **MS** (ESI+): | m/z (%) = 419 (100, [M+H]⁺) | | | |
| **HRMS** (ESI+): | m/z für C₂₀H₂₀N₂S₄ | | | |
| | ber.: | 418.0666 | | gef.: 418.0654 |
| **EA:** | ber.: | C:57.38 | H: 5.30 | N: 6.69 |
| | gef.: | C:57.57 | H: 5.44 | N: 6.59 |

### Darstellung von Bis[2-(1,3-dioxan-2-yl)ethyl]piperazin-1,4-dicarbodithioat (Schl32143):

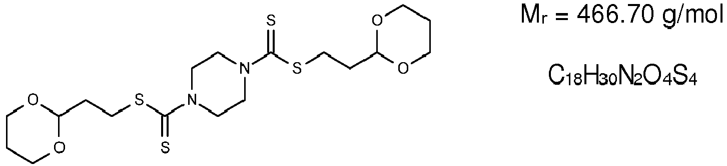

258 mg Piperazin (3.0 mmol, 1.0 eq) und 1000 mg Natriumhydroxid (25.0 mmol, 8.3 eq) wurden bei Raumtemperatur in 20 mL Wasser und 20 mL Ethanol gelöst, 30 Minuten gerührt und anschließend mit 0.45 mL Kohlenstoffdisulfid (7.5 mmol, 3.0 eq) versetzt. Nach weiteren 60 Minuten Reaktionszeit wurden 1.2 mL 2-(2-Bromethyl)-1,3-dioxan (9.0 mmol, 3.0 eq) zugegeben und 18 Stunden gerührt. Anschließend wurde das Lösungsmittel am Rotavapor entfernt. Säulenchromatographie an Kieselgel (DCM/ MeOH 80:20) ergab 732 mg von **Schl32143** (52.3%) in Form eines blassgelben Feststoffs.

| | | | | |
|---|---|---|---|---|
| **DC:** | Rf = 0.65 (DCM/ MeOH 80:20). | | | |
| **Fp.:** | 137 °C | | | |
| **¹H-NMR:** | (CDCl₃, 400 MHz), δ [ppm] = 4.64 (*t*, ³J=5.2 Hz, 2H, CH₂-C*H-*O, CH₂-C*H*-O), 4.10-4.05 (*m*, 4H, CH-O-C*H*H-CH₂, CH-O-C*H*H-CH₂, CH-O-C*H*H-CH₂, CH-O-C*H*H-CH₂), 3.99-3.91 (*m*, 4H, CH-O-C*H*H-CH₂, CH-O-C*H*H-CH₂, CH-O-C*H*H-CH₂, CH-O-C*H*H-CH₂), 3.26-3.22 (*m*, 4H, S-C*H₂*-CH₂, S-C*H₂*-CH₂), 2.88-3.69 (*m*, 8H, N-C*H₂*-C*H₂*-N, N-C*H₂-*C*H₂-*N), 2.01-1.93 (*m*, 2H, O-CH₂-C*H*H-CH₂-O, O-CH₂-C*H*H-CH₂-O), 1.92-1.90 (*m*, 4H, S-CH₂-C*H₂*, S-CH₂-C*H₂*), 1.22-1.17 (*m*, 2H, O-CH₂-C*H*H-CH₂-O, O-CH₂-C*H*H-CH₂-O). | | | |
| **¹³C-NMR:** | (CDCl₃, 100 MHz), δ [ppm] = 196.3 (C=S, C=S), 101.1 (CH₂-CH-O, CH₂-CH-O), 67.0 (O-CH₂-CH₂, O-CH₂-CH₂), 52.6 (N-CH₂-CH₂-N, N-CH₂-CH₂-N), 34.4 (S-CH₂ S-CH₂), 31.8 (S-CH₂-CH₂ S-CH₂-CH₂), 25.9 (O-CH₂-CH₂-CH₂ O-CH₂-CH₂-CH₂). | | | |
| **IR (ATR):** | v (cm⁻¹) = 2968 (w), 2951 (w), 2922 (w), 1609 (w), 1455 (m), 1415 (s), 1399 (s), 1380 (m), 1351 (w), 1335 (w), 1317 (w), 1268 (m), 1246 (m), 1246 (w), 1216 (w), 1198 (s), 1189 (s), 1143 (m), 1124 (m), 1073 (m), 1037 (m), 1020 (m), 989 (vs), 922 (s), 894 (s), 871 (s), 846 (s), 835 (s), 753 (m), 636 (w), 572 (w), 544 (m), 485 (m), 474 (m). | | | |
| **MS** (ESI+): | m/z (%) = 467 (100, [M+H]⁺) | | | |
| **HRMS** (ESI+): | m/z für C₁₈H₂₀N₂O₄S₄ | | | |
| | ber.: | 466.1088 | | gef.: 466.1102 |
| EA: | ber.: | C: 46.32 | H: 6.48 | N:6.00 |
| | gef.: | C:46.10 | H: 6.49 | N:6.20 |

### Darstellung von [4-(Diethylcarbamothioylsulfanylmethyl)phenyl]methyl-N,N-diethylcarbamodithioat (Schl32144):

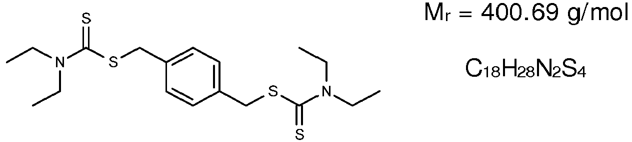

790 mg *α*,*α*'-Dibromxylen (3.0 mmol, 1.0 eq) und 0.72 mL Kohlenstoffdisulfid (12.0 mmol, 4.0 eq) wurden in 25 mL Acetonitril gelöst, auf 0 °C gekühlt und 5 Minuten gerührt. Zu dem Reaktionsgemisch wurde anschließend langsam 1.24 mL Diethylamin (12.0 mmol, 4.0 eq) zugetropft. Nach 30 Minuten wurde auf Raumtemperatur erwärmt, 18 Stunden bei Raumtemperatur gerührt, mit EtOAc extrahiert (5 x 50 mL), die vereinigten organischen Phasen über MgSO₄ getrocknet, filtriert und das Lösungsmittel am Rotavapor entfernt. Säulenchromatographie an Kieselgel (Cyclohexan/ EtOAc 6:1) ergab 1070 mg von **Schl32144** (89.0%) in Form eines blassgelben Feststoffes.

| | | | | |
|---|---|---|---|---|
| **DC:** | R_{f} = 0.23 (Cyclohexan/ EtOAc 6:1). | | | |
| **Fp.:** | 74 °C | | | |
| **¹H-NMR:** | (CDCl₃, 400 MHz), δ [ppm] = 7.32 (*d*, ³J = 8.2 Hz, 4H, C-C*H*-C*H*-C, C-C*H*-C*H*-C), 4.51 (s, 4H, S-C*H₂*-C, S-C*H₂*-C), 4.03 (*q,* ³J = 6.9 Hz, 4H, CH₃-C*H₂*, CH₃-C*H₂*), 3.71 (*q,* ³J = 6.9 Hz, 6H, CHs-C*H₂*, CHs-C*H₂*), 1.27 (*t*, ³J = 6.9 Hz, 12H, CH₂-C*H₃*, CH₂-C*H₃*, CH₂-C*H₃*, CH₂-C*H₃*). | | | |
| **¹³C-NMR:** | (CDCl₃, 100 MHz), δ [ppm] = 195.2 (C=S, C=S), 135.4 (C-CH-CH-C), 129.7 (C-CH-CH-C, C-CH-CH-C), 49.6 (CH₂-CH₃, CH₂-CH₃), 46.8 (CH₂-CH₃, CH₂-CH₃), 41.9 (S-CH₂-C, S-CH₂-C), 12.6 (CH₂-CH₃, CH₂-CH₃), 11.7 (CH₂-CH₃, CH₂-C*H₃*). | | | |
| **IR (ATR):** | v (cm⁻¹) = 2972 (w), 1486 (m), 1454 (w), 1414 (s), 1352 (m), 1299 (m), 1269 (s), 1197 (m), 1141 (m), 1067 (m), 981 (m), 915 (m), 861 (m), 827 (m), 761 (m), 685 (m), 527 (m), 428 (m). | | | |
| **MS** (ESI+): | m/z (%) = 401 (100, [M+H]⁺) | | | |
| **HRMS** (ESI+): | m/z für C₁₈H₂₈N₂S₄ | | | |
| | ber.: | 400.1135 | | gef.: 400.1112 |
| **EA:** | ber.: | C:53.96 | H:7.04 | N: 6.99 |
| | gef.: | C:53.95 | H:7.20 | N: 6.92 |

### Darstellung von Benzyl-N-[2-[benzylsulfanylcarbothioyl(ethyl)amino]ethyl]-N-ethylcarbamodithioat (Schl32173):

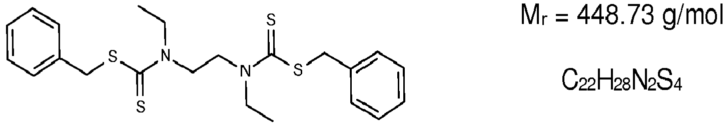

0.43 mL *N*,*N*'-Diethylethan-1,2-diamin (3.0 mmol, 1.0 eq) und 1000 mg Natriumhydroxid (25.0 mmol, 8.3 eq) wurden bei Raumtemperatur in 20 mL Wasser und 20 mL Ethanol gelöst, 30 Minuten gerührt und anschließend mit 0.45 mL Kohlenstoffdisulfid (7.5 mmol, 3.0 eq) versetzt. Nach weiteren 60 Minuten Reaktionszeit wurden 1.1 mL Benzylbromid (9.0 mmol, 3.0 eq) zugegeben und 18 Stunden gerührt. Anschließend wurde das Lösungsmittel am Rotavapor entfernt. Säulenchromatographie an Kieselgel (Cyclohexan/ EtOAc 10:1) ergab 792 mg von **Schl32173** (58.8%) in Form eines blassgelben Feststoffs.

| | | | | |
|---|---|---|---|---|
| **DC:** | R_{f} = 0.39 (Cyclohexan/ EtOAc 10:1). | | | |
| **Fp.:** | 113 °C | | | |
| **¹H-NMR:** | (DMSO-D₆, 400 MHz), δ [ppm] = 7.39-7.21 (*m*, 10H, C*H*_{(Phenyl)}), 4.46 (*S*, 2H, S-C*H₂*-C, S-C*H₂*-C), 4.01 (q, ³J = 6.8 Hz, 2H, CHs-C*H₂*), 3.69 (*q*, ³J = 6.9 Hz, 2H, CHs-C*H₂*), 2.71 (*t,* ³J = 7.0Hz, 4H, N-C*H₂*-C*H₂*-N), 1.24 (*t,* ³J = 7.0 Hz, 6H, CH₂-C*H₃*, CH₂-C*H₃*). | | | |
| **¹³C-NMR:** | (DMSO-D₆, 100 MHz), δ [ppm] = 195.4 (C=S, C=S), 138.2 (C-CH-CH, C-CH-CH), 129.1 (C-CH-CH, C-CH-CH, C-CH-CH, C-CH-CH), 127.9 (C-CH-CH, C-CH-CH, C-CH-CH, C-CH-CH), 127.0 (C-CH-CH-CH, C-CH-CH-CH), 51.8 (N-CH₂-CH₂-N), 41.2 (CH₂-CHs, CH₂-CH₃), 39.9 (S-CH₂-C), 14.0 (CH₂-CH₃, CH₂-C*H₃*). | | | |
| **IR (ATR):** | v (cm⁻¹) = 1974 (w), 1478 (s), 1452 (m), 1399 (s), 1313 (m), 1293 (m), 1232 (s), 1179 (vs), 1095 (m), 1048 (s), 995 (s), 941 (m), 863 (m), 766 (s), 695 (vs), 592 (m), 553 (m), 484 (s), 459 (m), 441 (m), 405 (m). | | | |
| **MS** (ESI+): | m/z (%) = 449 (100, [M+H]⁺) | | | |
| **HRMS** (ESI+): | m/z für C₂₂H₂₈N₂S₄ | | | |
| | ber.: | 448.1135 | | gef.: 448.1142 |
| **EA:** | ber.: | C: 58.89 | H: 6.29 | N: 6.24 |
| | gef.: | C: 58.95 | H: 6.36 | N: 5.80 |

### Darstellung von Benzylpiperidin-1-carbodithioat (Schl32012):

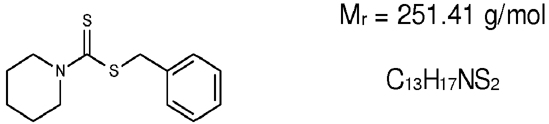

Gemäß **AAV5** wurden 0.24 mL Benzylbromid (2.0 mmol, 1.0 eq), 0.25 mL Kohlenstoffdisulfid (4.0 mmol, 2.0 eq) und 0.40 mL Piperidin (4.0 mmol, 2.0 eq) umgesetzt. Säulenchromatographie an Kieselgel (Pentan/ EtOAc 5:1) ergab 430 mg von **Schl32012** (85.5%) in Form eines gelben Feststoffs.

| | | | | |
|---|---|---|---|---|
| **DC:** | R_{f} = 0.40 (Cyclohexan/ EtOAc 5:1). | | | |
| **Fp.:** | 45 °C | | | |
| **¹H-NMR:** | (CDCl₃, 400 MHz), δ [ppm] = 7.40-7.38 (*m*, 2H, CH₂-C-C*H-*CH, CH₂-C-CH-C*H*), 7.32-7.29 (*m*, 2H, CH₂-C-CH-C*H*, CH₂-C-CH-C*H*), 7.27-7.24 (*m*, 1H, CH₂-C-CH-CH-C*H*), 4.57 (s, 2H, S-C*H₂*-C), 4.40-4.20 (*m*, 2H, N-C*H₂*), 3.96-3.78 (*m*, 2H, N-C*H₂*), 1.72-1.62 (*m*, 6H, N-CH₂-C*H₂*-C*H₂*-C*H₂*-CH₂). | | | |
| **¹³C-NMR:** | (CDCl₃, 100 MHz), δ [ppm] = 195.2 (C=S), 136.2 (CH₂-C-CH), 129.3 (C-CH-CH), 128.7 (C-CH-CH), 127.6 (C-CH-CH-CH), 42.3 (N-CH₂-CH₂), 24.4 (N-CH₂-CH₂-CH₂-CH₂-CH₂). | | | |
| **IR (ATR):** | v (cm⁻¹) = 3082 (w), 2994 (w), 2933 (m), 1637 (w), 1601 (w), 1582 (w), 1494 (w), 1475 (s), 1451 (m), 1422 (s), 1400 (s), 1358 (s), 1280 (w), 1244 (m), 1223 (vs), 1201 (m), 1162 (w), 1132 (m), 1108 (s), 1020 (m), 997 (s), 970 (s), 949 (m), 921 (m), 889 (s), 849 (s), 800 (m), 775 (m), 707 (s), 694 (vs), 620 (w), 515 (m), 490 (m), 480 (m), 433 (w), 403 (m). | | | |
| **MS (ESI+):** | m/z (%) = 276 (50, [M+Na]⁺) | | | |
| **HRMS** (ESI+): | m/z für C₁₃H₁₇NS₂ | | | |
| | ber.: | 251.0802 | | gef.: 251.0810 |
| **EA:** | ber.: | C:61.11 | H: 6.82 | N: 5.57 |
| | gef.: | C:61.39 | H: 6.86 | N: 5.47 |

### Darstellung von Methylpiperidin-1-carbodithioat (Schl32013):

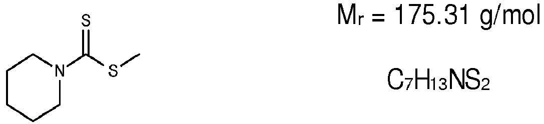

Gemäß **AAV5** wurden 0.13 mL Methyliodid (2.0 mmol, 1.0 eq), 0.25 mL Kohlenstoffdisulfid (4.0 mmol, 2.0 eq) und 0.40 mL Piperidin (4.0 mmol, 2.0 eq) umgesetzt. Säulenchromatographie an Kieselgel (Pentan/ EtOAc 5:1) ergab 332 mg von **Schl32013** (70.5%) in Form eines gelben Feststoffs.

| | | | | |
|---|---|---|---|---|
| **DC:** | R_{f} = 0.42 (Cyclohexan/ EtOAc 5:1). | | | |
| **Fp.:** | 57 °C | | | |
| **¹H-NMR:** | (CDCl₃, 400 MHz), δ [ppm] = 4.40-3.82 (*m*, 4H, N-C*H₂*, N-C*H₂*), 2.62 (*s*, 3H, S-C*H₃*), 1.72-1.62 (*m*, 6H, N-CH₂-C*H₂*-C*H₂*-C*H₂*-CH₂). | | | |
| **¹³C-NMR:** | (CDCl₃, 100 MHz), δ [ppm] = 196.9 (C=S), 25.8 (N-CH₂-CH₂), 24.4 (N-CH₂-CH₂-CH₂-CH₂-CH₂), 20.1 (S-CHs). | | | |
| **IR (ATR):** | v (cm⁻¹) = 2995 (w), 2935 (m), 2915 (m), 2853 (m), 1643 (w), 1499 (m), 1469 (w), 1427 (s), 1365 (w), 1351 (w), 1312 (w), 1280 (m), 1262 (w), 1229 (vs), 1159 (w), 1118 (vs), 1073 (m), 1009 (s), 981 (s), 955 (s), 892 (vs), 853 (s), 804 (m), 725 (w), 613 (w), 552 (w), 508 (m), 457 (w), 411 (s). | | | |
| **MS** (ESI+): | m/z (%) = 176 (100, [M+H]⁺) | | | |
| **HRMS** (ESI+): | m/z für C₇H₁₃NS₂ | | | |
| | ber.: | 175.0489 | | gef.: 175.0508 |
| **EA:** | ber.: | C:47.96 | H:7.47 | N:7.99 |
| | gef.: | C:47.77 | H:7.43 | N:8.07 |

### Darstellung von Methyl-2-(piperidin-1-carbothioylsulfanyl)acetat (Schl32014):

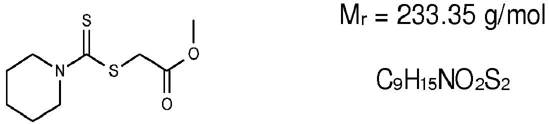

Gemäß **AAV5** wurden 0.19 mL Bromessigsäuremethylester (2.0 mmol, 1.0 eq), 0.25 mL Kohlenstoffdisulfid (4.0 mmol, 2.0 eq) und 0.40 mL Piperidin (4.0 mmol, 2.0 eq) umgesetzt. Säulenchromatographie an Kieselgel (Pentan/ EtOAc 5:1) ergab 486 mg von **Schl32014** (76.4%) in Form eines gelben Feststoffs.

| | | | | |
|---|---|---|---|---|
| **DC:** | R_{f} = 0.18 (Cyclohexan/ EtOAc 5:1). | | | |
| **Fp.:** | 59 °C | | | |
| **¹H-NMR:** | (CDCl₃, 400 MHz), δ [ppm] = 4.30-4.19 (*m*, 2H, N-C*H₂*), 4.17 (s, 2H, S-C*H₂*-COOMe), 3.95-3.83 (*m*, 2H, N-C*H₂*), 3.74 (s, 3H, C*H₃*), 1.72-1.65 (m, 6H, N-CH₂-C*H₂*-C*H₂-*C*H₂-*CH₂). | | | |
| **¹³C-NMR:** | (CDCl₃, 100 MHz), δ [ppm] = 193.7 (C=S), 169.4 (C=O), 52.9 (C(=O)-O-CH₃), 38.9 (N-CH₂-CH₂), 24.3 (N-CH₂-CH₂-CH₂-CH₂-CH₂). | | | |
| **IR (ATR):** | v (cm⁻¹) = 3027 (w), 3003 (w), 2954 (w), 1740 (m), 1568 (w), 1474 (w), 1438 (m), 1427 (m), 1365 (w), 1347 (w), 1291 (m), 1281 (m), 1258 (w), 1244 (m), 1229 (m), 1193 (m), 1145 (s), 1113 (s), 1070 (w), 1024 (w), 997 (m), 974 (s), 897 (w), 884 (m), 874 (m), 853 (m), 806 (w), 690 (m), 612 (w), 558 (w), 509 (w), 464 (w), 420 (m). | | | |
| **MS** (ESI+): | m/z (%) = 234 (100, [M+H]⁺) | | | |
| **HRMS** (ESI+): | m/z für C₉H₁₅NO₂S₂ | | | |
| | ber.: | 233.0544 | | gef.: 233.0551 |
| **EA:** | ber.: | C: 46.32 | H: 6.48 | N:6.00 |
| | gef.: | C: 46.38 | H: 6.52 | N:6.05 |

### Darstellung von 2-(Piperidin-1-carbothioylsulfanyl)essigsäure (Schl32015):

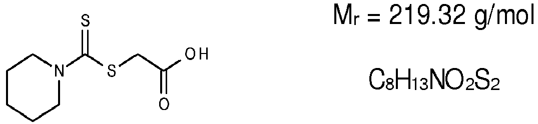

600 mg Natriumhydroxid (15.0 mmol, 1.5 eq) und 1.0 mL Piperidin (10.0 mmol, 1.0 eq) wurden in 10 mL Wasser gelöst, 5 Minuten bei Raumtemperatur gerührt und langsam mit 0.8 mL Kohlenstoffdisulfid (12.5 mmol, 1.25 eq) versetzt. Nach 30 Minuten Reaktionszeit wurden 400 mg Natriumhydroxid (10.0 mmol, 1.0 eq), 1700 mg Bromessigsäure (12.5 mmol, 1.25 eq) und 8 mL Wasser zugegeben und 18 Stunden bei Raumtemperatur gerührt. Das Reaktionsgemisch wurde mit 6M HCl auf pH=3 eingestellt, mit Dichlormethan extrahiert (5 x 100 mL), die vereinigten organischen Phasen über MgSO₄ getrocknet, filtriert und das Lösungsmittel am Rotavapor entfernt. Der verbleibende Rückstand wurde in wenig Dichlormethan gelöst und in 200 mL eisgekühltes Cyclohexan getropft. Der resultierende Niederschlag wurde abgesaugt und getrocknet. Es wurden 1830 g von **Schl32015** (83.6%) in Form farbloser Nadeln erhalten.

| | | | | |
|---|---|---|---|---|
| **Fp.:** | 131 °C | | | |
| **¹H-NMR:** | (DMSO-D₆, 400 MHz), δ [ppm] = 12.63 (s, 1H, COO*H*), 4.21-4.12 (*m*, 2H, N-C*H₂*), 4.08 (*s*, 2H, S-C*H₂*-COOH), 3.95-3.83 (*m*, 2H, N-C*H₂*), 1.66-1.53 (*m*, 6H, N-CH₂-C*H₂*-C*H₂*-*H₂*-CH₂). | | | |
| **¹³C-NMR:** | (DMSO-D₆, 100 MHz), δ [ppm] = 193.6 (C=S), 169.8 (C=O), 52.9 (C(=O)-O-CH₃), 39.2 (N-CH₂-CH₂), 24.0 (N-CH₂-CH₂-CH₂-CH₂-CH₂). | | | |
| **IR (ATR):** | v (cm⁻¹) = 2991 (w), 2920 (w), 2858 (w), 1695 (s), 1648 (m), 1475 (m), 1448 (w), 1430 (m), 1414 (m), 1353 (w), 1302 (m), 1274 (m), 1258 (w), 1241 (m), 1224 (m), 1204 (s), 1177 (s), 1129 (m), 1109 (m), 1067 (w), 1001 (m), 978 (s), 906 (m), 891 (m), 872 (m), 849 (m), 789 (w), 738 (w), 670 (s), 605 (w), 557 (w), 513 (w), 474 (w), 450 (w), 409 (m). | | | |
| **MS** (ESI+): | m/z (%) = 220 (100, [M+H]⁺) | | | |
| **HRMS** (ESI+): | m/z für C₈H₁₃NO₂S₂ | | | |
| | ber.: | 219.0388 | | gef.: 219.0404 |
| **EA:** | ber.: | C:43.81 | H: 5.97 | N: 6.39 |
| | gef.: | C:43.66 | H: 5.89 | N: 6.30 |

### Darstellung von (2-Amino-2-oxo-ethyl)piperidin-1-carbodithioat (Schl32018):

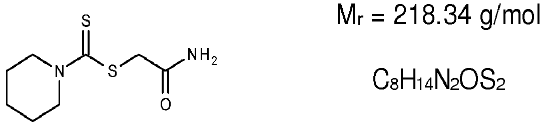

Gemäß **AAV6** wurden 0.59 mL Piperidin (6.0 mmol, 2.0 eq), 0.36 mL Kohlenstoffdisulfid (6.0 mmol, 2.0 eq) und 414 mg Bromacetamid (3.0 mmol, 1.0 eq) umgesetzt. Säulenchromatographie an Kieselgel (DCM/ MeOH 95:5) ergab 516 mg von **Schl32018** (78.8%) in Form eines blassgelben Feststoffs.

| | | | | |
|---|---|---|---|---|
| **DC:** | R_{f} = 0.42 (DCM/ MeOH 95:5). | | | |
| **Fp.:** | 142 °C | | | |
| **¹H-NMR:** | (CDCl₃, 400 MHz), δ [ppm] = 6.74 (*sbr,* 1H, NHH), 5.81 (*sbr,* 1H, NHH), 4.31-4.22 (*m*, 2H, N-C*H₂*), 4.09 (*s*, 2H, S-C*H₂*), 3.97-3.82 (*m*, 2H, N-C*H₂*), 3.75-3.70 (*m*, 2H, C*H₂-*CH3), 1.76-1.63 (*m*, 6H, N-CH₂-C*H₂*-C*H₂*-C*H₂*-CH₂). | | | |
| **¹³C-NMR:** | (CDCl₃, 100 MHz), δ [ppm] = 193.9 (C=S), 171.5 (C=O), 54.1 (N-CH₂-CH₂), 51.9 (N-CH₂-CH₂), 38.4 (S-CH2-C_{(Amid)}), 26.2 N-CH₂-CH₂-CH₂), 25.7 (N-CH₂-CH₂-CH₂), 24.3 (N-CH₂-CH₂-CH₂-CH₂-CH₂). | | | |
| **IR (ATR):** | v (cm⁻¹) = 3195 (w), 2941 (w), 1651 (s), 1604 (m), 1478 (s), 1455 (m), 1431 (s), 1343 (m), 1268 (m), 1258 (m), 1243 (m), 1219 (s), 1189 (m), 1133 (m), 1106 (s), 1065 (m), 1020 (w), 1001 (s), 978 (s), 948 (m), 886 (m), 847 (m), 778 (w), 617 (m), 605 (m), 547 (s), 520 (s), 468 (s). | | | |
| MS (ESI+): | m/z (%) = 219 (90, [M+H]⁺) | | | |
| **HRMS** (ESI+): | m/z für C₈H₁₄N₂OS₂ | | | |
| | ber.: | 218.0548 | | gef.: 218.0549 |
| **EA:** | ber.: | C:44.01 | H: 6.46 | N: 12.83 |
| | gef.: | C:43.91 | H: 6.49 | N: 12.71 |

### Darstellung von 2-(1,3-Dioxan-2-yl)ethylpiperidin-1-carbodithioat (Schl32026):

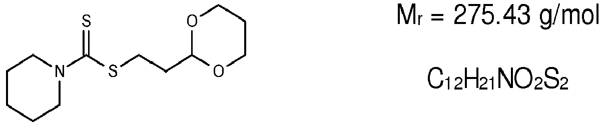

Gemäß **AAV6** wurden 0.40 mL Piperidin (4.0 mmol, 2.0 eq), 0.24 mL Kohlenstoffdisulfid (4.0 mmol, 2.0 eq) und 0.24 mL 2-(2-Bromethyl)-1,3-dioxan (2.0 mmol, 1.0 eq) umgesetzt. Säulenchromatographie an Kieselgel (Pentan/ EtOAc 4:1) ergab 501 mg von **Schl32026** (90.9%) in Form eines gelben Öls.

| | | | | |
|---|---|---|---|---|
| **DC:** | R_{f} = 0.19 (Cyclohexan/ EtOAc 5:2). | | | |
| **Fp.:** | 77 °C | | | |
| **¹H-NMR:** | (CDCl₃, 400 MHz), δ [ppm] = 4.61 (*t*, ³J=5.2 Hz, 1H, CH₂-C*H-*O), 4.30-4.19 (*m*, 2H, N-C*H*H-CH₂, N-C*H*H-CH₂), 4.11-4.03 (m, 2H, CH-O-C*H*H-CH₂, CH-O-C*H*H-CH₂), 3.94-3.79 (*m*, 2H, N-C*H*H-CH₂, N-C*H*H-CH₂), 3.78-3.69 (*m*, 2H, CH-O-C*H*H-CH₂, CH-O-C*H*H-CH₂), 3.37-3.32 (*m*, 2H, S-C*H₂*-CH₂), 2.12-2.04 (m, 2H, O-CH₂-C*H₂*-CH₂-O), 2.03-1.93 (*m*, 2H, S-CH₂-C*H₂*), 1.71-1.60 (*m*, 6H, N-CH₂-C*H₂*-C*H₂*-C*H₂*-CH₂). | | | |
| **¹³C-NMR:** | (CDCl₃, 100 MHz), δ [ppm] = 195.6 (C=S), 101.1 (CH₂-CH-O), 67.0 (O-CH₂-CH₂), 34.4 (S-CH₂), 31.8 (S-CH₂-CH₂), 25.8 (O-CH₂-CH₂-CH₂), 24.4 (CH_{2(Piperidin)}). | | | |
| **IR (ATR):** | v (cm⁻¹ ) = 3147 (w), 3077 (w), 2946 (w), 1618 (w), 1593 (w), 1562 (w), 1500 (m), 1479 (w), 1442 (m), 1425 (m), 1379 (w), 1349 (w), 1281 (w), 1266 (w), 1209 (m), 1180 (s), 1165 (m), 1120 (s), 1108 (s), 1075 (m), 1036 (w), 1020 (m), 995 (s), 974 (s), 950 (m), 924 (m), 880 (s), 805 (s), 695 (w), 575 (w), 558 (w), 522 (w), 478 (w), 466 (w), 428 (m), 408 (w). | | | |
| **MS** (ESI+): | m/z (%) = 276 (100, [M+H]⁺), 298 (85, [M+Na]⁺) | | | |
| **HRMS** (ESI+): | m/z für C₁₂H₂₁NO₂S₂ | | | |
| | ber.: | 275.1014 | | gef.: 275.0971 |
| **EA:** | ber.: | C:52.33 | H:7.68 | N:5.09 |
| | gef.: | C:52.15 | H:7.70 | N: 5.19 |

### Darstellung von Benzyl-N,N-diisopropylcarbamodithioat (Schl32188):

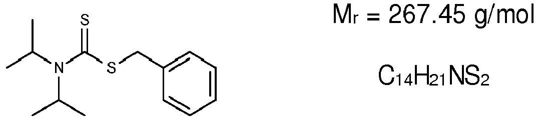

0.85 mL Diisopropylamin (6.0 mmol, 1.5 eq) und 505 mg Kaliumhydroxid (9.0 mmol, 2.25 eq) wurden bei Raumtemperatur in 40 mL Acetonitril gelöst, 15 Minuten gerührt und anschließend mit 1.26 mL Kohlenstoffdisulfid (12.0 mmol, 3.0 eq) versetzt. Nach 30 Minuten Reaktionszeit wurden 1.43 mL Benzylbromid (4.0 mmol, 1.0 eq) zugegeben, 18 Stunden gerührt, mit EtOAc extrahiert (5 x 50 mL), die vereinigten organischen Phasen über MgSO₄ getrocknet, filtriert und das Lösungsmittel am Rotavapor entfernt. Säulenchromatographie an Kieselgel (Cyclohexan) ergab 670 mg von **Schl32188** (62.6%) in Form eines gelbbraunen Öls.

| | | | | |
|---|---|---|---|---|
| **DC:** | R_{f} = 0.61 (Cyclohexan). | | | |
| **¹H-NMR:** | (CDCl3, 400 MHz), δ [ppm] = 7.43-7.22 (*m*, 5H, C*H*_{(Phenyl)}), 4.57 (*s*, 2H, S-C*H₂*-C), 1.93-1.77 (m, 2H, N-C*H*, N-C*H*), 1.28-1.16 (*m*, 12H, *CH₃).* | | | |
| **¹³C-NMR:** | (CDCl₆, 100 MHz), δ [ppm] = 195.5 (C=S), 136.0 (S-CH₂-C-CH), 129.6 (C-CH-CH-CH, C-CH-CH-CH), 128.6 (C-CH-CH-CH, C-CH-CH-CH), 127.5 (C-CH-CH-CH), 41.6 (S-CH₂-C), 34.6 (N-CH), 20.8 (CH-CH₃). | | | |
| **IR (ATR):** | v (cm⁻¹) = 3061 (w), 3027 (w), 2970 (w1601 (w), 1494 (w), 1474 (w), 1452 (m), 1438 (m), 1421 (w), 1369 (w), 1308 (vs), 1282 (s), 1235 (w), 1189 (s), 1138 (s), 1115 (m), 1069 (w), 1029 (s), 961 (w), 944 (w), 913 (w), 896 (w), 850 (w), 818 (w), 769 (m), 694 (vs), 666 (w), 640 (w), 622 (w), 584 (m), 564 (w), 548 (w), 509 (w), 470 (m), 406 (w). | | | |
| **MS** (ESI+): | m/z (%) = 268 (100, [M+H]⁺) | | | |
| **HRMS** (ESI+): | m/z für C₁₄H₂₁NS₂ | | | |
| | ber.: | 267.1115 | | gef.: 267.1107 |
| **EA:** | ber.: | C:62.87 | H:7.91 | N:5.24 |
| | gef.: | C:63.26 | H:7.94 | N:5.00 |

### Darstellung von Benzyl-N,N-dipropylcarbamodithioat (Schl32201):

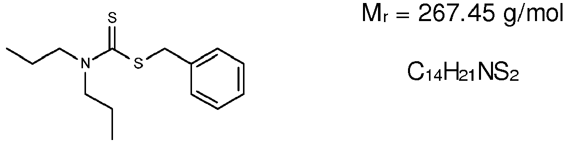

Gemäß **AAV8** wurden 0.82 mL Dipropylamin (6.0 mmol, 2.0 eq), 0.36 mL Kohlenstoffdisulfid (6.0 mmol, 2.0 eq) und 0.36 mL Benzylbromid (6.0 mmol, 1.0 eq) umgesetzt. Säulenchromatographie an Kieselgel (Cyclohexan) ergab 732 mg von **Schl32201** (91.3%) in Form eines gelben Öls.

| | | | | |
|---|---|---|---|---|
| **DC:** | R_{f} = 0.15 (Cyclohexan). | | | |
| **¹H-NMR:** | (CDCl₃, 400 MHz), δ [ppm] = 7.41-7.37 (*m*, 2H, CH₂-C-C*H-*CH, CH₂-C-CH-C*H*), 7.34-7.24 (*m*, 3H, CH₂-C-CH-C*H*, CH₂-C-CH-C*H*, CH₂-C-CH-CH-C*H*), 4.56 (s, 2H, S-C*H₂*-C), 3.94 (*t*, ³J = 7.6 Hz, 2H, CHs-CH₂-C*H₂*), 3.61 (*t,* ³J = 7.8 Hz, 2H, CH₃-CH₂-C*H₂*), 1.85-1.69 (*m*, 4H, CH₃-C*H₂*-CH₂, CH₃-C*H₂*-CH₂), 1.00-0.89 (*m*, 6H, CH₂-CH₂-C*H₃*, CH₂-CH₂-C*H₃*). | | | |
| **¹³C-NMR:** | (CDCl₃, 100 MHz), δ [ppm] = 195.9 (C=S), 136.2 (C-CH-CH), 129.5 (C-CH-CH), 128.7 (C-CH-CH), 127.6 (C-CH-CH-CH), 56.9 (N-CH₂-CH₂-CHs, N-CH₂-CH₂-CHs), 42.5 (S-CH₂-C), 19.9 (N-CH₂-CH₂-CH₃, N-CH₂-CH₂-CH₃), 11.4 (N-CH₂-CH₂-CH₃, N-CH₂-CH₂-CH₃). | | | |
| **IR (ATR):** | v (cm⁻¹) = 3060 (w), 3028 (w), 1479 (s), 1462 (m), 1452 (m), 1436 (m), 1409 (vs), 1379 (m), 1363 (m), 1342 (m), 1303 (m), 1270 (m), 1233 (vs), 1195 (s), 1144 (s), 1089 (m), 1071 (w), 1028 (w), 989 (s), 921 (w), 889 (w), 854 (w), 804 (w), 768 (w), 747 (w), 695 (vs), 618 (w), 591 (w), 563 (w), 530 (w), 479 (w), 431 (w). | | | |
| **MS** (ESI+): | m/z (%) = 268 (100, [M+H]⁺) | | | |
| **HRMS** (ESI+): | m/z für C₁₄H₂₁NS₂ | | | |
| | ber.: | 267.1115 | | gef.: 267.1106 |
| **EA:** | ber.: | C:62.87 | H:7.91 | N:5.24 |
| | gef.: | C:62.96 | H:7.77 | N:5.27 |

### Darstellung von Benzyl-N,N-dibutylcarbamodithioat (Schl32202):

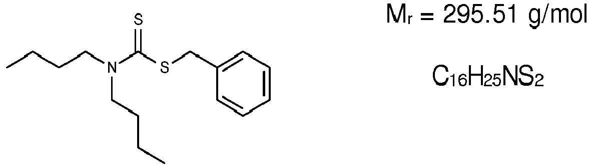

Gemäß **AAV8** wurden 1.02 mL Dibutylamin (6.0 mmol, 2.0 eq), 0.36 mL Kohlenstoffdisulfid (6.0 mmol, 2.0 eq) und 0.36 mL Benzylbromid (6.0 mmol, 1.0 eq) umgesetzt. Säulenchromatographie an Kieselgel (Cyclohexan) ergab 834 mg von **Schl32202** (94.1 %) in Form eines gelben Öls.

| | | | | |
|---|---|---|---|---|
| **DC:** | R_{f} = 0.29 (Cyclohexan). | | | |
| **¹H-NMR:** | (CDCl₃, 400 MHz), δ [ppm] = 7.42-7.37 (*m*, 2H, CH₂-C-C*H-*CH, CH₂-C-CH-C*H*), 7.35-7.23 (*m,* 3H, CH₂-C-CH-C*H*, CH₂-C-CH-C*H*, CH₂-C-CH-CH-C*H*), 4.57 (*s*, 2H, S-C*H₂*-C), 3.98 (*t,* ³J = 7.8 Hz, 2H, CHs-CH₂-CH₂-C*H₂*), 3.65 (*t,* ³J = 7.8 Hz, 2H, CH₃-CH₂-CH₂-C*H₂*), 1.80-1.64 (*m*, 4H, CH₃-CH₂-C*H₂-*CH₂, CH₃-CH₂-C*H₂*-CH2), 1.44-1.28 (*m*, 4H, CH₃-C*H₂*-CH₂-CH₂, CH₃-C*H₂*-CH₂-CH₂), 1.02-0.91 *(m,* 6H, C*H₃*-CH₂-CH₂-CH₂, C*H₃*-CH₂-CH₂-CH₂). | | | |
| **¹³C-NMR:** | (CDCl₃, 100 MHz), δ [ppm] = 195.7 (C=S), 136.3 (C-CH-CH), 129.5 (C-CH-CH), 128.7 (C-CH-CH), 127.6 (C-CH-CH-CH), 52.6 (N-CH₂-CH₂-CH₂-CH₃, N-CH₂-CH₂-CH₂-CH₃), 42.5 (S-CH₂-C), 29.5 (N-CH₂-CH₂, N-CH₂-CH₂), 20.3 (N-CH₂-CH₂-CH₂, N-CH₂-CH₂-CH₂), 14.1 (N-CH₂-CH₂-CH₂-CH₃, N-CH₂-CH₂-CH₂-CH₃). | | | |
| **IR (ATR):** | v (cm⁻¹) = 3061 (w), 3028 (w), 2956 (m), 1602 (w), 1481 (m), 1452 (s), 1410 (vs), 1366 (m), 1288 (m), 1250 (m), 1216 (vs), 1182 (m), 1142 (m), 1110 (m), 1093 (m), 1070 (m), 1051 (m), 1002 (m), 982 (m), 943 (m), 845 (w), 777 (w), 712 (w), 695 (vs), 614 (w), 563 (w), 479 (w), 433 (w). | | | |
| **MS** (ESI+): | m/z (%) = 296 (100, [M+H]⁺) | | | |
| **HRMS** (ESI+): | m/z für C₁₆H₂₅NS₂ | | | |
| | ber.: | 295.1428 | | gef.: 295.1455 |
| **EA:** | ber.: | C:65.03 | H:8.53 | N:4.74 |
| | gef.: | C:64.87 | H:8.35 | N:4.75 |

### Darstellung von Benzyl-N,N-dipentylcarbamodithioat (Schl32203):

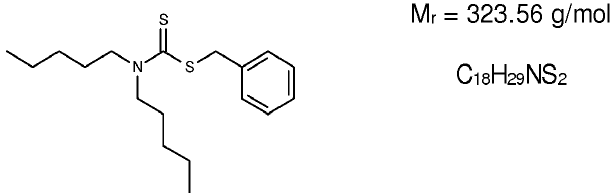

Gemäß **AAV8** wurden 1.84 mL Dibutylamin (6.0 mmol, 2.0 eq), 0.36 mL Kohlenstoffdisulfid (6.0 mmol, 2.0 eq) und 0.36 mL Benzylbromid (6.0 mmol, 1.0 eq) umgesetzt. Säulenchromatographie an Kieselgel (Cyclohexan/ EtOAc 95:5) ergab 790 mg von **Schl32203** (81.4%) in Form eines gelben Öls.

| | | | | |
|---|---|---|---|---|
| **DC:** | R_{f} = 0.29 (Cyclohexan/ EtOAc 95:5) | | | |
| **¹H-NMR:** | (CDCl₃, 400 MHz), δ [ppm] = 7.43-7.37 (*m*, 2H, CH₂-C-C*H-*CH, CH₂-C-CH-C*H*), 7.35-7.23 (*m*, 3H, CH₂-C-CH-C*H*, CH₂-C-CH-C*H*, CH₂-C-CH-CH-C*H*), 4.57 (*s*, 2H, S-C*H₂*-C), 4.09-3.82 (*m*, 2H, CH₃-CH₂-CH₂-CH₂-C*H₂*), 3.72-3.49 (*m*, 2H, CH₃-CH₂-CH₂-CH₂-C*H₂*), 1.81-1.69 (*m*, 2H, CH₃-CH₂-CH₂-C*H₂*-CH₂), 1.51-1.24 (*m*, 6H, CH₃-CH₂-CH₂-C*H₂*, CH₃-CH₂-C*H₂*-CH₂, CH₃-CH₂-C*H₂*-CH₂), 0.99-0.88 (*m*, 10H, C*H₃*-C*H₂*-CH₂-CH₂-CH₂, CH3-C*H₂*-CH₂-CH₂-CH₂). | | | |
| **¹³C-NMR:** | (CDCl₃, 100 MHz), δ [ppm] = 196.5 (C=S), 136.3 (C-CH-CH), 129.5 (C-CH-CH), 128.7 (C-CH-CH), 127.5 (C-CH-CH-CH), 53.6 (N-CH₂-CH₂-CH₂-CH₂-CH₃, N-CH₂-CH₂-CH₂-CH₂-CH₃), 42.5 (S-CH₂-C), 28.9 (N-CH₂-CH₂-CH₂-CH₂-CH₃, N-CH₂-CH₂-CH₂-CH₂-CH₃), 24.3 (N-CH₂-CH₂-CH₂-CH₂-CH₃, N-CH₂-CH₂-CH₂-CH₂-CH₃), 20.2 (N-CH₂-CH₂-CH₂-CH₂-CH₃, N-CH₂-CH₂-CH₂-CH₂-CH₃), 14.1 (N-CH₂-CH₂-CH₂-CH₂-CH₃, N-CH₂-CH₂-CH₂-CH₂-CH₃), | | | |
| **IR (ATR):** | v (cm⁻¹) = 3061 (w), 3028 (w), 2957 (m), 2928 (m), 2871 (w), 2093 (w), 1602 (w), 1478 (s), 1453 (s), 1409 (s), 1378 (w), 1357 (m), 1303 (w), 1269 (w), 1253 (w), 1226 (m), 1182 (m), 1140 (m), 1093 (m), 1070 (w), 1028 (w), 992 (m), 966 (m), 914 (w), 843 (w), 815 (w), 803 (w), 768 (w), 713 (s), 695 (vs), 620 (w), 594 (w), 564 (w), 533 (w), 479 (w), 432 (w). | | | |
| **MS** (ESI+): | m/z (%) = 324 (100, [M+H]⁺) | | | |
| **HRMS** (ESI+): | m/z für C₁₈H₂₉NS₂ | | | |
| | ber.: | 323.1741 | | gef.: 323.1739 |
| **EA:** | ber.: | C: 66.82 | H:9.03 | N:4.33 |
| | gef.: | C: 66.56 | H:8.74 | N:4.54 |

### Darstellung von Benzyl-N,N-dihexylcarbamodithioat (Schl32204):

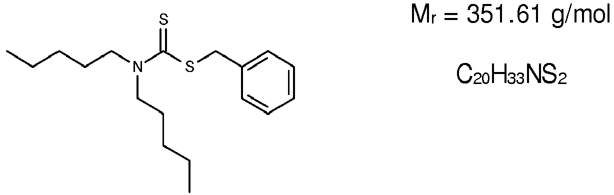

Gemäß **AAV8** wurden 2.11 mL Dihexylamin (6.0 mmol, 2.0 eq), 0.36 mL Kohlenstoffdisulfid (6.0 mmol, 2.0 eq) und 0.36 mL Benzylbromid (6.0 mmol, 1.0 eq) umgesetzt. Säulenchromatographie an Kieselgel (Cyclohexan) ergab 453 mg von **Schl32204** (42.9%) in Form eines gelben Öls.

| | | | | |
|---|---|---|---|---|
| **DC**: | R_{f} = 0.25 (Cyclohexan). | | | |
| **¹H-NMR:** | (CDCl₃, 400 MHz), δ [ppm] = 7.41-7.36 (*m*, 2H, CH₂-C-C*H-*CH, CH₂-C-CH-C*H*), 7.34-7.23 (*m*, 3H, CH₂-C-CH-C*H*, CH₂-C-CH-C*H*, CH₂-C-CH-CH-C*H*), 4.55 (*s*, 2H, S-C*H₂*-C), 3.96 *(t,* ³J = 7.8 Hz, 2H, CH₃-CH₂-CH₂-CH₂-CH₂-C*H₂*), 3.63 *(t,* ³J = 7.8 Hz, 2H, CH₃-CH₂-CH₂-CH₂-CH₂-C*H₂*), 1.79-1.64 (*m*, 4H, CH₃-CH₂-CH₂-CH₂-C*H*_{*2*,} CH₃-CH₂-CH₂-CH₂-C*H₂*), 1.37-1.25 (*m*, 12H, CH₃-C*H₂*-C*H₂*-C*H₂*-CH₂-CH₂, CH₃-C*H₂*-C*H₂*-C*H₂*-CH₂-CH₂), 0.94-0.82 (*m*, 6H, C*H₃*-CH₂-CH₂-CH₂-CH₂-CH₂, C*H₃*-CH₂-CH₂-CH₂-CH₂-CH₂). | | | |
| **¹³C-NMR:** | (CDCl₃, 100 MHz), δ [ppm] = 195.6 (C=S), 136.2 (C-CH-CH), 129.5 (C-CH-CH), 128.7 (C-CH-CH), 127.5 (C-CH-CH-CH), 55.4 (N-CH₂), 52.8 (N-CH₂), 42.4 (S-CH₂-C), 31.7 (N-CH₂-CH₂), 31.5 (N-CH₂-CH₂), 27.4 (N-CH₂-CH₂-CH₂), 26.6 (N-CH₂-CH₂-CH₂-CH₂), 22.7 (N-CH₂-CH₂-CH₂-CH₂-CH₂), 22.5 (N-CH₂-CH₂-CH₂-CH₂-CH₂), 14.2 (N-CH₂-CH₂-CH₂-CH₂-CH₂-CH₃), 14.0 N-CH₂-CH₂-CH₂-CH₂-CH₂-CH₃). | | | |
| **IR (ATR):** | v (cm⁻¹) = 3061 (w), 3028 (w), 2954 (m), 2925 (m), 2855 (m), 2093 (w), 2032 (w), 1602 (w), 1480 (s), 1453 (m), 1410 (vs), 1367 (m), 1296 (w), 1255 (w), 1223 (w), 1993 (m), 1179 (w), 1141 (w), 1118 (w), 1099 (w), 1070 (w), 1028 (w), 982 (m), 913 (w), 845 (w), 768 (w), 713 (s), 695 (vs), 619 (w), 594 (w), 564 (w), 536 (w), 479 (w), 431 (w). | | | |
| **MS** (ESI+): | m/z (%) = 352 (100, [M+H]⁺) | | | |
| **HRMS** (ESI+): | m/z für C₂₀H₃₃NS₂ | | | |
| | ber.: | 351.2054 | | gef.: 351.2043 |
| **EA:** | ber.: | C: 68.32 | H: 9.46 | N: 3.98 |
| | gef.: | C: 68.25 | H: 9.19 | N: 4.08 |

### Darstellung von S-Benzyl-N,N-dimethylcarbamodithioat (Schl32261):

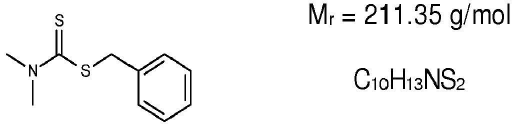

0.47 mL Benzylmercaptan (4.0 mmol, 1.0 eq) und 0.43 mL Triethylamin (6.0 mmol, 1.5 eq) wurden in 15 mL Acetonitril gelöst, 15 Minuten bei Raumtemperatur gerührt und langsam mit 989 mg *N,N-*Dimethylthiocarbamoylchlorid (8.0 mmol, 2.0 eq) versetzt. Das Reaktionsgemisch wurde 36 Stunden bei Raumtemperatur gerührt und das Lösungsmittel wurde am Rotavapor entfernt. Säulenchromatographie an Kieselgel (Cyclohexan/ EtOAc 6:1) ergab 593 mg von **Schl32261** (93.7%) in Form eines gelben Öls.

| | | | | |
|---|---|---|---|---|
| **DC**: | R_{f} = 0.36 (Cyclohexan/ EtOAc 6:1). | | | |
| **¹H-NMR:** | (CDCl₃, 400 MHz), δ [ppm] = 7.41-7.22 (*m*, 5H, CH₂-C-C*H-*CH, CH₂-C-C*H*-CH, CH₂-C-C*H*-CH, CH₂-C-C*H*-CH, CH₂-C-CH-CH-C*H*), 4.55 (s, 2H, S-C*H₂*-C), 3.55 (*s*, 3H, N-C*H₃*), 3.37 (s, 3H, N-C*H₃*). | | | |
| **¹³C-NMR:** | (CDCl₃, 100 MHz), δ [ppm] = 196.9 (C=S), 136.2 (C-CH-CH), 129.4 (C-CH-CH, C-CH-CH), 128.7 (C-CH-CH, C-CH-CH), 127.6 (C-CH-CH-CH), 45.5 (N-CH₃), 42.7 (S-CH₂-C), 41.5 (N-CH₃). | | | |
| **IR (ATR):** | v (cm⁻¹) = 3026 (w), 2923 (w), 1492 (s), 1451 (m), 1403 (w), 1369 (vs), 1141 (m), 1069 (w), 1051 (w), 1028 (w), 977 (vs), 915 (w), 870 (w), 844 (w), 804 (w), 770 (w), 694 (vs), 619 (w), 576 (w), 562 (w), 481 (w), 444 (m), 425 (m). | | | |
| **MS** (ESI+): | m/z (%) = 212 (100, [M+H]⁺) | | | |
| **HRMS** (ESI+): | m/z für C₁₀H₁₃NS₂ | | | |
| | ber.: | 211.0489 | | gef.: 211.0479 |
| **EA:** | ber.: | C: 56.83 | H: 6.20 | N: 6.63 |
| | gef.: | C:56.52 | H: 6.20 | N: 6.71 |

### Darstellung von Benzyl-N,N-bis(4-bromphenyl)carbamodithioat (Schl32011):

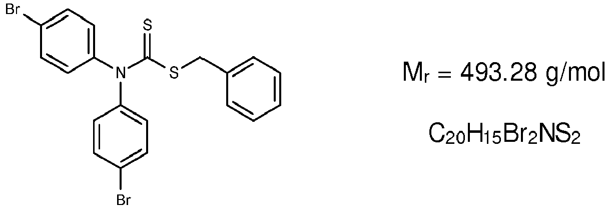

60 mg Natriumhydrid (60%ige Dispersion in Mineralöl, 1.5 mmol, 1.5 eq) wurden in 5 mL trockenem Tetrahydrofuran gelöst und unter Rühren auf 0 °C gekühlt. Anschließend wurden 327 mg 4-Brom-*N*-(4-bromphenyl)anilin (1.0 mmol, 1.0 eq) in 10 mL trockenem Tetrahydrofuran gelöst, langsam zur Natriumhydridlösung zugetropft und 60 Minuten bei 0 °C gerührt. Es wurden 0.14 mL Kohlenstoffdisulfid (2.3 mmol, 2.3 eq) zugetropft, 30 Minuten gerührt, 0.14 mL Benzylbromid (1.2 mmol, 1.2 eq) zugetropft und weitere 60 Minuten gerührt und anschließend auf Raumtemperatur erwärmt. Nach 24 Stunden wurden 20 mL Wasser zugegeben und mit Ethylacetat extrahiert (5 x 150 mL). Die vereinigten organischen Phasen wurden über MgSO₄ getrocknet, filtriert und das gewünschte Produkt wurde anschließend mit Methanol ausgefällt. Es wurden 53 mg von **Schl32011** (10.7%) in Form eines beigen Feststoffes erhalten.

| | | | | |
|---|---|---|---|---|
| **DC:** | R_{f} = 0.47 (Cyclohexan/EtOAC 6:1). | | | |
| **Fp.:** | 185 °C | | | |
| **¹H-NMR:** | (DMSO-D₆, 400 MHz), δ [ppm] = 7.39-7.35 (*m*, 4H, C*H*-C(Br)-C*H*), 7.31-7.23 (*m*, 4H, C*H*_{(Benzyl)}), 7.22-7.16 (*m*, 1H, C*H*_{(Benzyl)}), 7.00-6.96 (*m,* 4H, C*H*-CH-C(Br)-CH-C*H*), 4.95 (*s*, 2H, C*H*₂). | | | |
| **¹³C-NMR:** | (DMSO-D₆, 100 MHz), δ [ppm] = 201.8 (C=S), 135.3 (C-Br), 133.0 (CH-C-Br), 129.7 (N-C-CH), 129.6 (C_{(Benzyl)}-CH-CH), 128.7 (C_{(Benzy)}-CH-CH), 127.7 (C_{(Benzy)}-CH-CH-CH), 122.5 (CH-C-N, CH-C-CH₂), 43.5 (CH₂). | | | |
| **IR (ATR):** | v (cm⁻¹) = 3027 (w), 2979 (w), 1667 (w), 1629 (w), 1579 (w), 1551 (w), 1513 (w), 1481 (w), 1450 (w), 1418 (w), 1325 (m), 1273 (m), 1227 (w), 1192 (w), 1142 (w), 1094 (w), 1067 (w), 1049 (m), 1028 (w), 1008 (m), 937 (w), 894 (w), 842 (w), 814 (w), 799 (w), 782 (m), 771 (m), 728 (w), 707 (w), 689 (w), 650 (w), 620 (w), 526 (m), 495 (m), 479 (w), 448 (m). | | | |
| **MS** (ESI+): | m/z (%) = 495 (80, [M^{81Br, 81Br}+H]⁺), 493 (100, [M^{79Br, 81Br}+H]⁺), 491 (70, [M^{79Br, 79Br}+H]⁺) | | | |
| **HRMS** (ESI+): | m/z für C₂₀H₁₅Br₂NS₂ | | | |
| | ber.: | 490.9013 | | gef.: 490.9023 |
| **EA:** | ber.: | C:48.70 | H:3.07 | N:2.84 |
| | gef.: | C: 48.64 | H:3.11 | N:2.74 |

### Darstellung von Benzylmorpholin-4-carbodithioat (Schl32019):

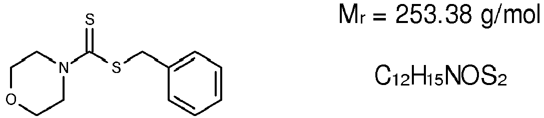

Gemäß **AAV5** wurden 0.50 mL Benzylbromid (3.0 mmol, 1.0 eq), 0.36 mL Kohlenstoffdisulfid (6.0 mmol, 2.0 eq) und 0.52 mL Morpholin (6.0 mmol, 2.0 eq) umgesetzt. Säulenchromatographie an Kieselgel (Cyclohexan/ EtOAc 5:2) ergab 651 mg von **Schl32019** (85.6%) in Form eines gelben Feststoffes.

| | | | | |
|---|---|---|---|---|
| **DC:** | R_{f} = 0.37 (Cyclohexan/ EtOAc 5:2). | | | |
| **Fp.:** | 64 °C | | | |
| **¹H-NMR:** | (CDCl₃, 400 MHz), δ [ppm] = 7.42-7.23 (*m*, 5H, CH₂-C-CH-CH-C*H*, CH₂-C-C*H*-CH, CH₂-C-C*H*-C*H*), 4.58 (*s*, 2H, S-C*H₂*-C), 4.44-3.78 (*m*, 8H, O-C*H₂*-C*H₂*-N, O-C*H₂*-C*H₂*-N). | | | |
| **¹³C-NMR:** | (CDCl₃, 100 MHz), δ [ppm] = 197.2 (C=S), 135.8 (C-CH-CH), 129.5 (C-CH-CH), 128.7 (C-CH-CH), 127.7 (C-CH-CH-CH), 66.6 (O-CH₂, O-CH₂), 42.1 (S-CH₂-C), 34.7 (N-CH₂, N-CH₂). | | | |
| **IR (ATR):** | v (cm⁻¹) = 2856 (w), 1648 (s), 1451 (s), 1420 (vs), 1299 (w), 1267 (vs), 1211 (vs), 1111 (vs), 1063 (m), 1024 (m), 993 (vs), 910 (m), 867 (s), 784 (s), 719 (vs), 699 (vs), 569 (m), 538 (m), 491 (s), 404 (m). | | | |
| **MS** (ESI+): | m/z (%) = 254 (100, [M+H]⁺) | | | |
| **HRMS** (ESI+): | m/z für C₁₂H₁₅NOS₂ | | | |
| | ber.: | 253.0595 | | gef.: 253.0606 |
| **EA:** | ber.: | C: 56.88 | H:5.97 | N:5.53 |
| | gef.: | C:56.49 | H:5.99 | N:5.68 |

### Darstellung von Benzyl-4-methylpiperazin-1-carbodithioat (Schl32140):

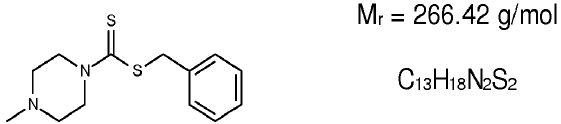

Gemäß **AAV5** wurden 0.36 mL Benzylbromid (3.0 mmol, 1.0 eq), 0.36 mL Kohlenstoffdisulfid (6.0 mmol, 2.0 eq) und 0.67 mL *N*-Methylpiperazin (6.0 mmol, 2.0 eq) umgesetzt. Säulenchromatographie an Kieselgel (DCM/ MeOH 90:10) ergab 552 mg von **Schl32140** (69.1 %) in Form eines blassgelben Feststoffes.

| | | | | |
|---|---|---|---|---|
| **DC:** | R_{f}= 0.88 (DCM/ MeOH 90:10). | | | |
| **Fp.:** | 27 °C | | | |
| **¹H-NMR:** | (CDCl₃, 400 MHz), δ [ppm] = 7.44-7.22 (*m*, 5H, CH_{(Phenyl)}), 4.55 (s, 2H, S-C*H*_{*2*-}C), 4.42-4.26 (*m*, 2H, N-CH*H*-CH₂-N-CH₃, N-CH*H*-CH₂-N-CH₃), 4.02-3.86 (*m*, 2H, N-CH*H*-CH₂-N-CH₃, N-CH*H*-CH₂-N-CH₃), 2.55-2.41 (*m*, 4H, N-CH₂-C*H₂*-N-CH₃, N-CH₂-C*H₂*-N-CH₃), 2.30 (*s*, 3H, N-C*H₃*). | | | |
| **¹³C-NMR:** | (CDCl₃, 100 MHz), δ [ppm] = 196.6 (C=S), 135.9 (S-CH₂-C-CH), 129.5 (C-CH-CH-CH, C-CH-CH-CH), 128.7 (C-CH-CH-CH, C-CH-CH-CH), 127.7 (C-CH-CH-CH), 54.5 (N-CH₂-CH₂-N-CH₃, N-CH₂-CH₂-N-CH₃), 50.8 (N-CH₂-CH₂-N-CH₃, N-CH₂-CH₂-N-CH₃), 45.7 (N-CH₂-CH₂-N-CH₃), 42.3 (S-CH₂-C). | | | |
| **IR (ATR):** | v (cm⁻¹) = 3083 (w), 3058 (w), 3028 (w), 3000 (w), 2967 (w), 1492 (s), 1472 (m), 1451 (s), 1423 (w), 1408 (m), 1376 (w), 1356 (w), 1286 (s), 1247 (m), 1230 (s), 1199 (w), 1141 (vs), 1071 (m), 1043 (w), 1021 (s), 979 (vs), 911 (s), 859 (m), 831 (m), 762 (s), 705 (s), 691 (vs), 633 (m), 618 (w), 570 (w), 553 (w), 537 (w), 490 (w), 472 (s), 434 (w). | | | |
| **MS** (ESI+): | m/z (%) = 267 (100, [M+H]⁺) | | | |
| **HRMS** (ESI+): | m/z für C₁₃H₁₈N₂S₂ | | | |
| | ber.: | 266.0911 | | gef.: 266.0918 |
| **EA:** | ber.: | C: 58.61 | H: 6.81 | N:10.51 |
| | gef.: | C:58.73 | H:6.90 | N:10.30 |

### Darstellung von Benzyl-4-acetylpiperazin-1-carbodithioat (Schl32146):

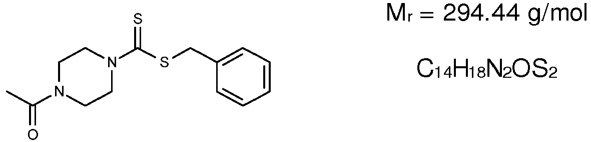

Gemäß **AAV5** wurden 0.36 mL Benzylbromid (3.0 mmol, 1.0 eq), 0.36 mL Kohlenstoffdisulfid (6.0 mmol, 2.0 eq) und 385 mg *N*-Acetylpiperazin (3.0 mmol, 1.0 eq) umgesetzt. Säulenchromatographie an Kieselgel (DCM/MeOH 90:10) ergab 756 mg von **SchL32146** (85.6%) in Form eines blassgelben Feststoffes.

| | | | | |
|---|---|---|---|---|
| **DC:** | R_{f}= 0.67 (DCM/ MeOH 90:10). | | | |
| **Fp.:** | 65 °C | | | |
| **¹H-NMR:** | (CDCl₃, 400 MHz), δ [ppm] = 7.40-7.24 (*m*, 5H, CH_{(Phenyl)}), 4.56 (*s*, 2H, S-C*H*_{*2*-}C), 4.32-4.11 (*m*, 4H, N-CH₂-C*H₂*-N-C(=O)-CH₃, N-CH₂-C*H₂*-N-C(=O)-CH₃), 3.88-3.60 (*m*, 4H, N-C*H₂*-CH₂-N-C(=O)-CH₃, N-C*H₂*-CH₂-N-C(=O)-CH₃), 2.12 (s, 3H, N-C(=O)-C*H₃*). | | | |
| **¹³C-NMR:** | (CDCl₃, 100 MHz), δ [ppm] = 197.5 (C=S), 169.5 (C=O), 135.6 (S-CH₂-C-CH), 129.5 (C-CH-CH-CH, C-CH-CH-CH), 128.8 (C-CH-CH-CH, C-CH-CH-CH), 127.8 (C-CH-CH-CH), 45.3 (N-CH₂-CH₂-N-C(=O)-CH₃, N-CH₂-CH₂-N-C(=O)-CH₃), 42.3 (S-CH₂-C), 40.8 (N-CH₂-CH₂-N-C(=O)-CH₃, N-CH₂-CH₂-N-C(=O)-CH₃), 21.5 (N-CH₂-CH₂-N-C(=O)-CH₃). | | | |
| **IR (ATR):** | v (cm⁻¹) = 2915 (w), 1642 (s), 1494 (w), 1451 (m), 1406 (vs), 1358 (m), 1280 (m), 1252 (m), 1216 (vs), 1159 (m), 1112 (w), 1069 (w), 1027 (w), 983 (s), 962 (m), 914 (w), 847 (w), 772 (w), 696 (s), 629 (w), 589 (w), 568 (w), 517 (w), 480 (w), 455 (w), 412 (w). | | | |
| **MS** (ESI+): | m/z (%) = 295 (100, [M+H]⁺) | | | |
| **HRMS** (ESI+): | m/z für C₁₄H₁₈N₄OS₂ | | | |
| | ber.: | 294.0861 | | gef.: 294.0859 |
| **EA:** | ber.: | C:57.11 | H:6.16 | N:9.51 |
| | gef.: | C:57.05 | H:5.99 | N:9.58 |

### Darstellung von Benzyl-4-(p-tolylmethyl)piperazin-1-carbodithioat (Schl32174):

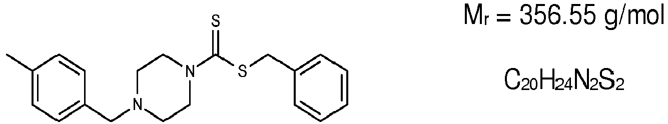

571 mg 1-(*p*-Tolylmethyl)piperazin (3.0 mmol, 1.0 eq) und 1000 mg Natriumhydroxid (25.0 mmol, 8.3 eq) wurden bei Raumtemperatur in 20 mL Wasser und 20 mL Ethanol gelöst, 30 Minuten gerührt und anschließend mit 0.45 mL Kohlenstoffdisulfid (7.5 mmol, 3.0 eq) versetzt. Nach weiteren 60 Minuten Reaktionszeit wurden 1.1 mL Benzylbromid (9.0 mmol, 3.0 eq) zugegeben und weitere 10 Stunden gerührt. Anschließend wurde das Lösungsmittel am Rotavapor entfernt. Säulenchromatographie an Kieselgel (Cyclohexan/ EtOAc 2:1) ergab 338 mg von **Schl32174** (31.6%) in Form eines blassgelben Feststoffs.

| | | | | |
|---|---|---|---|---|
| **DC:** | R_{f} = 0.39 (Cyclohexan/ EtOAc 2:1). | | | |
| **Fp.:** | 98 °C | | | |
| **¹H-NMR:** | (CDCl₃, 400 MHz), δ [ppm] = 7.40-7.14 (m, 9H, CH_{(Phenyl)}), 4.57 (s, 2H, S-C*H₂*-C), 4.42-4.30 (*m*, 2H, N-CH₂-CH*H*-N-C-S, N-CH₂-CH*H*-N-C-S), 3.99-3.86 (*m*, 2H, N-CH₂-CH*H*-N-C-S, N-CH₂-CH*H*-N-C-S), 3.51 (*s*, 2H, C-C*H₂*-N), 2.60-2.45 (*m*, 4H, N-C*H₂*-CH₂-N-C-S, N-C*H₂*-CH₂-N-C-S), 2.35 (s, 3H, C-C*H₃*). | | | |
| **¹³C-NMR:** | (CDCl₃, 100 MHz), δ [ppm] = 196.4 (C=S), 135.9 (C_{q(Phenyl)}), 129.5 (C_{t(Phenyl)}), 129.2 (C_{t(Phenyl)}), 129.1 (C_{t (Phenyl)}), 128.7 (C_{t (Phenyl)}), 127.7 (C_{t (Phenyl)}), 62.3 (C-CH₂-N), 52.4 (N-CH₂-CH₂-N, N-CH₂-CH₂-N), 42.3 (S-CH₂-C), 21.2 (C-CH₃). | | | |
| **IR (ATR):** | v (cm⁻¹) = 3086 (w), 2994 (w), 2937 (w), 2921 (w), 2863 (w), 1615 (w), 1513 (w), 1494 (w), 1468 (w), 1452 (w), 1442 (m), 1418 (m), 1383 (m), 1341 (w), 1316 (w), 1285 (w), 1248 (m), 1215 (w), 1180 (w), 1133 (m), 1116 (w), 1096 (w), 1049 (m), 1016 (m), 988 (s), 970 (s), 852 (w), 811 (w), 778 (m), 757 (m), 697 (s), 616 (w), 532 (w), 491 (m), 476 (s). | | | |
| **MS** (ESI+): | m/z (%) = 357 (100, [M+H]⁺) | | | |
| **HRMS** (ESI+): | m/z für C₂₀H₂₄N₂S₂ | | | |
| | ber.: | 356.1381 | | gef.: 356.1382 |
| **EA:** | ber.: | C:67.37 | H: 6.78 | N:7.86 |
| | gef.: | C:67.63 | H: 6.85 | N:7.52 |

### Darstellung von 1-Benzylsulfanylcarbothioylpiperidin-4-carbonsäure (Schl32176):

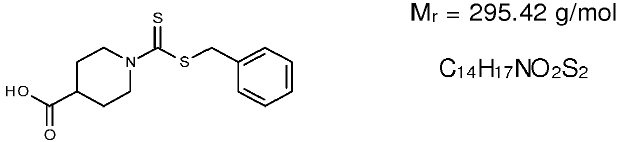

969 mg Piperidin-4-carbonsäure (7.5 mmol, 1.0 eq) und 1000 mg Natriumhydroxid (25.0 mmol, 3.3 eq) wurden bei Raumtemperatur in 20 mL Wasser und 20 mL Ethanol gelöst, 30 Minuten gerührt und anschließend mit 0.91 mL Kohlenstoffdisulfid (15.0 mmol, 2.0 eq) versetzt. Nach weiteren 60 Minuten Reaktionszeit wurden 1.79 mL Benzylbromid (15.0 mmol, 2.0 eq) zugegeben und 10 Stunden gerührt. Anschließend wurde das Reaktionsgemisch durch Zugabe von 100 mL 10%iger HCl abgebrochen, 3 Mal gegen je 100 mL Dichlormethan ausgeschüttelt und die vereinigten organischen Phasen wiederum 5 Mal gegen je 75 mL 1M NaOH-Lösung ausgeschüttelt. Die vereinigten wässrigen Phasen wurden mit 6M HCl auf pH=1 eingestellt. Der entstehende Niederschlag wurde abgesaugt, mit Wasser gewaschen und getrocknet. Es wurden 1314 mg von **Schl32176** (59.3%) als farbloser Feststoff erhalten.

| | | | | |
|---|---|---|---|---|
| **Fp.:** | 113 °C | | | |
| **¹H-NMR:** | (DMSO-D₆, 400 MHz), δ [ppm] = 7.38-7.21 (*m*, 5H, CH_{(Phenyl)}), 5.08-4.93 (*m*, 1H, N-CH*H*-CH₂-CH), 4.49 (*s*, 2H, S-C*H*_{*2*-}C), 4.38-4.25 (*m*, 1H, N-CH*H*-CH₂-CH), 3.50-3.42 (*m*, 2H, N-CH*H*-CH₂-CH, N-CH*H*-CH₂-CH), 2.66-2.58 (*m*, 1H, CH₂-C*H*-COOH), 1.95-1.84 (*m*, 2H, N-CH₂-CH*H*-CH, N-CH₂-CH*H*-CH), 1.58-1.44 (*m*, 2H, N-CH₂-CH*H*-CH, N-CH₂-CH*H*-CH). | | | |
| **¹³C-NMR:** | (DMSO-D₆, 100 MHz), δ [ppm] = 194.6 (C=S), 175.6 (C=O), 136.8 (S-CH₂-C-CH), 129.7 (C-CH-CH-CH, C-CH-CH-CH), 129.0 (C-CH-CH-CH, C-CH-CH-CH), 127.9 (C-CH-CH-CH), 48.8 (N-CH₂, N-CH₂), 41.4 (S-CH₂-C), 39.9 (CH-COOH), 28.2 (CH-CH₂-CH₂-N, CH-CH₂-CH₂-N). | | | |
| **IR (ATR):** | v (cm⁻¹) = 3742 (w), 3733 (w), 3648 (w), 3018 (w), 2988 (w), 2958 (w), 1693 (vs), 1600 (w), 1507 (w), 1493 (w), 1473 (m), 1425 (s), 1359 (w), 1307 (w), 1261 (w), 1230 (m), 1207 (s), 1170 (s), 1157 (s), 1132 (w), 1087 (w), 1069 (w), 1025 (w), 999 (w), 965 (s), 911 (s), 845 (m), 817 (w), 765 (w), 702 (vs), 651 (w), 617 (w), 563 (w), 481 (m), 447 (w), 406 (w). | | | |
| **MS** (ESI+): | m/z (%) = 296 (100, [M+H]⁺) | | | |
| **HRMS** (ESI+): | m/z für C₁₄H₁₇NO₂S₂ | | | |
| | ber.: | 295.0701 | | gef.: 295.0712 |
| **EA:** | ber.: | C: 56.92 | H:5.80 | N: 4.74 |
| | gef.: | C:56.60 | H:5.76 | N: 4.82 |

### Darstellung von Benzyl-4-[(4-phenoxyphenyl)carbamoyl]piperidin-1-carbodithioat (Schl32177) :

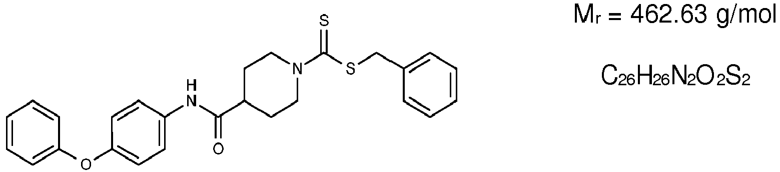

500 mg *N*-(4-Phenoxyphenyl)piperidin-4-carboxamidhydrochlorid (1.5 mmol, 1.0 eq) wurden bei Raumtemperatur in 25 mL 1M NaOH-Lösung und 25 mL Aceton gelöst, 10 Minuten gerührt und anschließend mit 0.18 mL Kohlenstoffdisulfid (3.0 mmol, 2.0 eq) versetzt. Nach weiteren 30 Minuten Reaktionszeit wurden 0.36 mL Benzylbromid (3.0 mmol, 2.0 eq) zugegeben, weitere 8 Stunden gerührt, mit EtOAc extrahiert (5 x 50 mL), die vereinigten organischen Phasen über MgSO₄ getrocknet, filtriert und das Lösungsmittel am Rotavapor entfernt. Säulenchromatographie an Kieselgel (Cyclohexan/ EtOAc 3:1) ergab 552 mg von **Schl32177** (79.4%) in Form eines blassgelben Feststoffes.

| | | | | |
|---|---|---|---|---|
| **DC:** | R_{f} = 0.22 (Cyclohexan/ EtOAc 3:1). | | | |
| **Fp.:** | 186 °C | | | |
| **¹H-NMR:** | (DMSO-D₆, 400 MHz), δ [ppm] = 9.96 (*sbr,* 1H, N*H*), 7.60-7.55 (*m*, 2H, CH_{(Phenyl)}), 7.39-7.21 (*m*, 7H, CH_{(Phenyl)}), 7.09-7.03 (*m*, 1 H, CH_{(Phenyl)}), 6.96-6.88 (m, 4H, CH_{(Phenyl)}), 5.31-5.15 (*m*, 1 H, N-CH*H*-CH₂-CH), 4.51 (*s*, 2H, S-C*H*_{*2*-}C), 4.50-4.39 (*m*, 2H, N-CH*H*-CH₂-CH, N-CH*H*-CH₂-CH), 3.46-3.32 (*m*, 1H, N-CH*H*-CH₂-CH), 2.78-2.65 (*m,* 1H, CH₂-C*H*-C(=O)), 1.95-1.84 (*m*, 2H, N-CH₂-CH*H*-CH, N-CH₂-CH*H*-CH), 1.68-1.55 (*m*, 2H, N-CH₂-CH*H*-CH, N-CH₂-CH*H*-CH). | | | |
| **¹³C-NMR:** | (DMSO-D₆, 100 MHz), δ [ppm] = 208.9 (C=S), 172.7 (C=O), 152.1 (C_{q (Phenyl)}), 135.7 (C_{q (Phenyl)}), 131.0 (C_{q (Phenyl)}), 130.5 (C_{t (Phenyl)}), 129.8 (Ct _{(Phenyl)}), 129.0 (C_{t (Phenyl)}), 127.9 (C_{t (Phenyl)}), 123.5 (Ct _{(Phenyl)}), 121.5 (C_{t (Phenyl)}), 121.4 (Ct _{(Phenyl)}), 120.1 (C_{t (Phenyl)}), 120.0 (C_{t (Phenyl)}), 118.3 (C_{t (Phenyl)}), 118.2 (C_{t (Phenyl)}), 69.0 (CH-C(=O)), 56.3 (N-CH₂, N-CH₂), 42.4 (S-CH₂-C), 30.1 (CH-CH₂-CH₂-N, CH-CH₂-CH₂-N). | | | |
| **IR (ATR):** | v (cm⁻¹) = 3030 (w), 2988 (w), 2942 (w), 2920 (w), 1607 (vs), 1528 (w), 1505 (w), 1489 (m), 1466 (s), 1427 (m), 1409 (m), 1385 (vs), 1361 (w), 1338 (w), 1326 (w), 1287 (s), 1267 (m), 1233 (m), 1176 (vs), 1101 (vs), 1087 (w), 1071 (w), 1020 (w), 965 (m), 953 (m), 921 (m), 892 (m), 851 (w), 832 (w), 807 (w), 759 (s), 724 (w), 710 (s), 693 (vs), 652 (w), 602 (w), 561 (w), 515 (m), 502 (m), 485 (m), 466 (w), 435 (w), 415 (w). | | | |
| **MS** (ESI+): | m/z (%) = 463 (100, [M+H]⁺) | | | |
| **HRMS** (ESI+): | m/z für C₂₆H₂₆N₂O₂S₂ | | | |
| | ber.: | 462.1436 | | gef.: 462.1442 |
| **EA:** | ber.: | C:67.50 | H:5.66 | N:6.06 |
| | gef.: | C:67.34 | H:5.73 | N:5.83 |

### Darstellung von Benzyl 2-(4-hydroxyphenyl)-4,5-dihydroimidazol-1-carbodithioat (Schl32185):

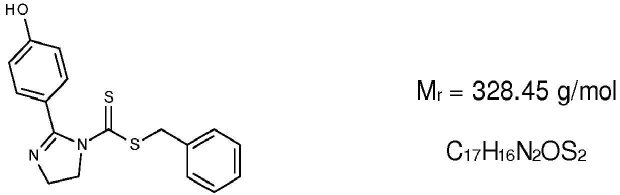

973 mg 4-(4,5-Dihydro-1*H*-imidazol-2-yl)phenol (6.0 mmol, 2.0 eq) wurden bei Raumtemperatur in 20 mL Dichlormethan gelöst, 10 Minuten gerührt und anschließend mit 0.36 mL Kohlenstoffdisulfid (6.0 mmol, 2.0 eq) versetzt. Nach weiteren 30 Minuten Reaktionszeit wurden 0.36 mL Benzylbromid (3.0 mmol, 1.0 eq) zugetropft, 18 Stunden gerührt und das Lösungsmittel am Rotavapor entfernt. Säulenchromatographie an Kieselgel (DCM/ MeOH 4:1) ergab 527 mg von **Schl32185** (53.5%) in Form eines blassgelben Feststoffes.

| | | | | |
|---|---|---|---|---|
| **DC:** | R_{f} = 0.38 (DCM/ MeOH 4:1). | | | |
| **Fp.:** | 151-160 °C | | | |
| **¹H-NMR:** | (CDCl₃, 400 MHz), δ [ppm] = 10.04 (*s*, 1H, O*H*), 8.24 (*d*, ³J = 8.9 Hz, 2H, N-C-C*H*-CH-C-OH, N-C-C*H*-CH-C-OH), 7.41-7.25 (*m*, 5H, C*H*_{(Benzyl)}), 6.95 (*d*, ³J = 8.7 Hz, 2H, N-C-CH-C*H*-C-OH, N-C-CH-C*H*-C-OH), 5.02 (*m*, 2H, N-C*H₂*-CH2-N-C(=S)), 4.07 (s, 2H, C-C*H₂*-C), 3.88 (*m*, 2H, N-CH₂-C*H₂*-N-C(=S)). | | | |
| **¹³C-NMR:** | (CDCl₃, 100 MHz), δ [ppm] = 210.6 (C=S), 163.1 (C-OH), 145.5 (N-C-N), 133.4 (N-C-C-CH₂, N-C-C-CH₂), 132.1 (S-CH₂-C), 132.0 (C-CH-CH-CH), 128.8 (C-CH-CH-CH), 127.3 (C-CH-CH-CH), 122.7 (N-C-C), 117.1 (CH₂-C-OH, CH₂-C-OH), 44.2 (N-CH₂-CH₂-N-C(=S)), 42.6 (S-CH₂-C), 40.9 (N-CH₂-CH₂-N-C(=S)). | | | |
| **IR (ATR):** | v (cm⁻¹) = 3066 (w), 2962 (w), 1604 (s), 1554 (w), 1508 (s), 1452 (w), 1365 (w), 1317 (w), 1273 (m), 1238 (m), 1189 (s), 1130 (w), 1035 (m), 1002 (m), 834 (s), 735 (w), 720 (s), 691 (s), 641 (vs), 611 (w), 534 (w), 514 (w), 457 (w). | | | |
| **MS** (ESI+): | m/z (%) = 329 (100, [M+H]⁺) | | | |
| **HRMS** (ESI+): | m/z für C₁₇H₁₆N₂OS₂ | | | |
| | ber.: | 328.0704 | | gef.: 328.0795 |
| **EA:** | ber.: | C:62.16 | H: 4.91 | N: 8.53 |
| | gef.: | C:62.35 | H: 4.89 | N: 8.64 |

### Darstellung von Ethyl-1-benzylsulfanylcarbothioylpiperidin-4-carboxylat (Schl32186):

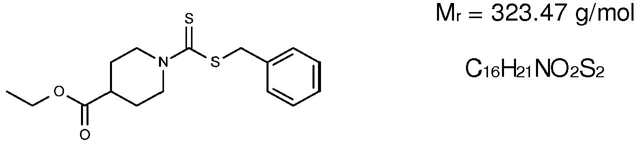

Gemäß **AAV10** wurden 472 mg Ethylpiperidin-4-carboxylat (3.0 mmol, 1.0 eq), 635 mg Natriumcarbonat (6.0 mmol, 2.0 eq), 0.32 mL Kohlenstoffdisulfid (6.0 mmol, 2.0 eq) und 0.72 mL Benzylbromid (6.0 mmol, 2.0 eq) umgesetzt. Säulenchromatographie an Kieselgel (Cyclohexan/ EtOAc 5:1) ergab 657 mg von **Schl32186** (67.8%) in Form eines gelben Öls.

| | | | | |
|---|---|---|---|---|
| **DC**: | R_{f} = 0.39 (Cyclohexan/ EtOAc 5:1). | | | |
| **¹H-NMR:** | (DMSO-D₆, 400 MHz), δ [ppm] = 7.39-7.20 (*m*, 5H, CH_{(Phenyl)}), 5.24-5.03 (*m*, 1H, N-CH*H*-CH₂-CH), 4.53 (*s*, 2H, S-C*H*_{*2*-}C), 4.45-4.30 (*m*, 1H, N-CH*H*-CH₂-CH), 4.13 (*q*, ³J = 7.1 Hz, 2H, CH₃-C*H₂*-O), 3.50-3.38 (*m*, 2H, N-CH*H*-CH₂-CH, N-CH*H*-CH₂-CH), 2.66-2.56 (*m*, 1H, CH₂-C*H*-COOEt), 2.02-1.90 (*m*, 2H, N-CH₂-CH*H*-CH, N-CH₂-CH*H*-CH), 1.82-1.71 (*m*, 2H, N-CH₂-CH*H*-CH, N-CH₂-CH*H*-CH), 1.24 (*t*, ³J = 6.9 Hz, 3H, C*H₃*-CH₂-O). | | | |
| **¹³C-NMR:** | (DMSO-D₆, 100 MHz), δ [ppm] = 194.0 (C=S), 174.0 (C=O), 136.0 (S-CH₂-C-CH), 129.5 (C-CH-CH-CH, C-CH-CH-CH), 128.7 (C-CH-CH-CH, C-CH-CH-CH), 127.6 (C-CH-CH-CH), 60.7 (CH₃-CH₂-O), 50.7 (N-CH₂), 49.2 (N-CH₂), 42.4 (S-CH₂-C), 40.5 (CH-COOEt), 27.9 (CH-CH₂-CH₂-N, CH-CH₂-CH₂-N), 14.4 (CH₃-CH₂-O). | | | |
| **IR (ATR):** | v (cm⁻¹) = 3060 (w), 3027 (w), 2978 (w), 2955 (w), 2929 (w), 1725 (vs), 1649 (w), 1494 (m), 1473 (m), 1446 (s), 1423 (s), 1374 (m), 1310 (m), 1263 (s), 1170 (vs), 1095 (m), 1070 (w), 1036 (vs), 1006 (s), 970 (m), 919 (s), 851 (w), 773 (w), 695 (s), 601 (w), 565 (w), 481 (w), 409 (w). | | | |
| **MS** (ESI+): | m/z (%) = 324 (100, [M+H]⁺) | | | |
| **HRMS** (ESI+): | m/z für C₁₆H₂₁NO₂S₂ | | | |
| | ber.: | 323.1014 | | gef.: 323.1009 |
| **EA:** | ber.: | C:59.41 | H: 6.54 | N:4.33 |
| | gef.: | C:59.17 | H: 6.44 | N: 4.52 |

### Darstellung von Benzyl-N-(2,2-dimethoxyethyl)-N-[3-oxo-3-(phenethylamino)ethyl]-carbamodithioat (Schl32187):

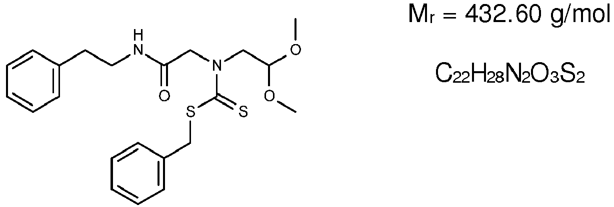

Gemäß **AAV10** wurden 602 mg 3-(2,2-Dimethoxyethylamino)-*N-*phenethylpropanamid **(Schl32180,** 2.27 mmol, 1.0 eq), 285 mg Natriumhydrogencarbonat (3.39 mmol, 1.5 eq), 0.27 mL Kohlenstoffdisulfid (4.52 mmol, 2.0 eq) und 0.32 mL Benzylbromid (2.71 mmol, 1.2 eq) umgesetzt. Säulenchromatographie an Kieselgel (Cyclohexan/ EtOAc 1:1) ergab 312 mg von **Schl32187** (44.8%) in Form eines farblosen Feststoffes.

| | | | | |
|---|---|---|---|---|
| **DC**: | R_{f} = 0.31 (Cyclohexan/ EtOAc 1:1). | | | |
| **Fp.:** | 74 °C | | | |
| **¹H-NMR:** | (CDCl₃, 400 MHz), δ [ppm] = 7.39-7.14 (*m*, 10H, C*H*_{(Phenyl)}), 6.30 (*sbr,* 1H, N*H*), 4.74-4.59 (*m*, 2H, C(=O)-C*H₂*-N), 4.50 (*s*, 2H, S-C*H*_{*2*-}C), 3.88-3.80 (*m*, 1H, N-CH₂-C*H*-OMe), 3.54-3.46 (*m*, 2H, C-CH₂-C*H₂*-NH), 3.31 (*s*, 6H, O-C*H₃,* O-C*H₃*), 2.82-2.71 (*m*, 2H, C-C*H₂*-CH₂-NH), 2.04-2.01 (*m*, 2H, O-CH-C*H₂*-N). | | | |
| **¹³C-NMR:** | (CDCl₃, 100 MHz), δ [ppm] = 196.9 (C=S), 171.3 (C=O), 138.7 (Cq_{(Phenyl)}), 135.3 (Cq_{(Phenyl)}), 129.5 (C_{t(Phenyl)}), 129.0 (C_{t(Phenyl)}), 128.8 (C_{t(Phenyl)}), 127.9 (C_{t(Phenyl)}), 126.6 (C_{t(Phenyl)}), 101.9 (NH-CH₂-CH-O), 60.3 (C(=O)-CH₂-N), 55.4 (O-CH₃, O-CH₃), 54.8 (N-CH₂-CH), 43.1 (C-CH₂-CH₂-NH), 40.8 (C-CH₂-CH₂-NH), 35.7 (S-CH₂-C). | | | |
| **IR (ATR):** | v (cm⁻¹) = 3925 (w), 3061 (w), 3026 (w), 2933 (w), 1657 (s), 1602 (w), 1544 (m), 1495 (w), 1465 (m), 1453 (m), 1425 (w), 1393 (m), 1293 (m), 1235 (m), 1195 (s), 1173 (m), 1120 (vs), 1083 (vs), 1030 (s), 998 (s), 953 (s), 915 (m), 846 (w), 748 (m), 696 (vs), 566 (m), 535 (m), 495 (m), 479 (m), 435 (m), 406 (m). | | | |
| **MS** (ESI+): | m/z (%) = 433 (100, [M+H]⁺) | | | |
| **HRMS** (ESI+): | m/z für C₂₂H₂₈N₂O₃S₂ | | | |
| | ber.: | 432.1541 | | gef.: 432.1564 |
| **EA:** | ber.: | C:61.08 | H: 6.52 | N: 6.48 |
| | gef.: | C:61.28 | H: 6.52 | N: 5.99 |

### Darstellung von tert-Butyl-4-benzylsulfanylcarbothioylpiperazin-1-carboxylat (Schl32190):

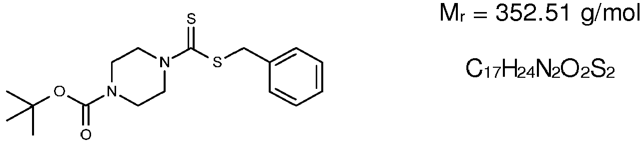

Gemäß **AAV6** wurden 1676 mg *Boc*-Piperazin (9.0 mmol, 1.0 eq), 1.60 mL Kohlenstoffdisulfid (27.0 mmol, 3.0 eq) und 1.10 mL Benzylbromid (9.0 mmol, 1.0 eq) umgesetzt. Säulenchromatographie an Kieselgel (Cyclohexan/ EtOAc 5:1) ergab 1346 mg von **Schl32190** (42.4%) in Form eines farblosen Feststoffes.

| | | | | |
|---|---|---|---|---|
| **DC:** | R_{f} = 0.38 (Cyclohexan/ EtOAc 5:1) | | | |
| **Fp.:** | 84 °C | | | |
| **¹H-NMR:** | (CDCl₃, 400 MHz), δ [ppm] = 7.41-7.23 (*m*, 5H, CH_{(Phenyl)}), 4.56 (*s*, 2H, S-C*H₂*-C), 4.42-4.26 (*m*, 2H, N-CH*H*-CH₂-N-CH₃, N-CH*H*-CH₂-N-CH₃), 4.09-3.81 (*m*, 2H, N-CH*H*-CH₂-N-Boc, N-CH*H*-CH₂-N-Boc), 3.57-3.50 (*m*, 4H, N-CH₂-C*H₂*-N-Boc, N-CH₂-C*H₂*-N-Boc), 1.46 (*s*, 9H, C-C*H₃*, C-C*H₃,* C-C*H₃).* | | | |
| **¹³C-NMR:** | (CDCl₃, 100 MHz), δ [ppm] = 192.6 (C=S), 154.6 (C=O), 135.7 (S-CH₂-C-CH), 129.5 (C-CH-CH-CH, C-CH-CH-CH), 128.7 (C-CH-CH-CH, C-CH-CH-CH), 127.7 (C-CH-CH-CH), 80.8 (O-C-(CH₃)₃), 53.6 (N-CH₂-CH₂-N-Boc, N-CH₂-CH₂-N-Boc), 53.1 (N-CH₂-CH₂-N-Boc, N-CH₂-CH₂-N-Boc), 42.2 (S-CH₂-C), 28.5 (CH₃). | | | |
| **IR (ATR):** | v (cm⁻¹) = 3022 (m), 2855 (m), 1617 (w), 1489 (w), 1454 (s), 1454 (s), 1416 (vs), 1378 (m), 1369 (m), 1352 (m), 1338 (m), 1298 (m), 1267 (s), 1246 (w), 1224 (m), 1206 (vs), 1140 (m), 1095 (m), 1071 (s), 1003 (m), 980 (m), 917 (s), 892 (w), 937 (w), 811 (w), 792 (w), 780 (w), 744 (vs), 717 (w), 632 (m), 594 (m), 531 (s), 499 (m), 484 (m), 427 (s), 405 (m). | | | |
| **MS** (ESI+): | m/z (%) = 353 (100, [M+H]⁺) | | | |
| **HRMS** (ESI+): | m/z für C₁₇H₂₄N₂O₂S₂ | | | |
| | ber.: | 352.1279 | | gef.: 352.1292 |
| **EA:** | ber.: | C:57.92 | H: 6.86 | N:7.95 |
| | gef.: | C:57.85 | H: 6.82 | N:7.90 |

### Darstellung von Benzylpiperazin-1-carbodithioathydrochlorid (Schl32191):

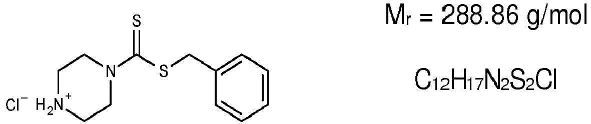

Gemäß **AAV11** wurden 500 mg *tert*-Butyl-4-benzylsulfanylcarbothioylpiperazin-1-carboxylat **(Schl32190,** 1.42 mmol, 1.0 eq) umgesetzt. Es wurden 266 mg von **Schl32191** (64.9%) in Form farbloser Nadeln erhalten.

| | | | | |
|---|---|---|---|---|
| **Fp.:** | 172 °C | | | |
| **¹H-NMR:** | (DMSO-D₆, 400 MHz), δ [ppm] = 9.39 (*sbr,* 2H, N*H₂*⁺), 7.37-7.22 (*m*, 5H, CH_{(Phenyl)}), 4.52 (*s*, 2H, S-C*H₂*-C), 4.44-4.11 (*m*, 4H, N-C*H₂*-CH₂-NH₂⁺, N-C*H₂*-CH₂-NH₂⁺), 3.22-3.14 (*m*, 4H, N-CH₂-C*H₂*-NH₂⁺, N-CH₂-C*H₂*-NH₂⁺). | | | |
| **¹³C-NMR:** | (DMSO-D₆, 100 MHz), δ [ppm] = 197.0 (C=S), 136.4 (S-CH₂-C-CH), 129.8 (C-CH-CH-CH, C-CH-CH-CH), 129.0 (C-CH-CH-CH, C-CH-CH-CH), 128.0 (C-CH-CH-CH), 51.3 (N-CH₂-CH₂-NH₂⁺, N-CH₂-CH₂-NH₂⁺), 48.3 (N-CH₂-CH₂-NH₂⁺, N-CH₂-CH₂-NH₂⁺), 42.6 (S-CH₂-C). | | | |
| **IR (ATR):** | v (cm⁻¹) = 3057 (w), 2938 (w), 2783 (m), 2754 (m), 1591 (w), 1493 (w), 1461 (w), 1451 (w), 1417 (vs), 1391 (m), 1361 (w), 1333 (w), 1314 (w), 1260 (s), 1231 (vs), 1186 (w), 1167 (m), 1140 (s), 1087 (w), 1070 (w), 1040 (m), 1025 (s), 989 (vs), 923 (w), 904 (m), 863 (w), 840 (w), 815 (w), 762 (w), 697 (s), 613 (w), 569 (m), 538 (w), 482 (w), 465 (w), 415 (vs). | | | |
| **MS** (ESI+): | m/z (%) = 253 (100, [M-Cl]⁺) | | | |
| **HRMS** (ESI+): | m/z für C₁₂H₁₆N₂S₂ | | | |
| | ber.: | 252.0755 | | gef.: 252.0744 |
| **EA:** | ber.: | C:49.90 | H:5.93 | N:9.70 |
| | gef.: | C:49.54 | H: 5.99 | N: 9.62 |

### Darstellung von Benzyl-4-ethylpiperazin-1-carbodithioat (Schl32192):

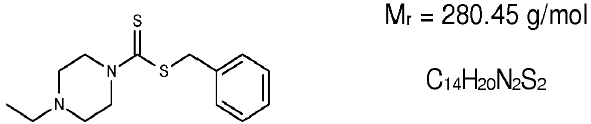

Gemäß **AAV6** wurden 0.76 mL *N*-Ethylpiperazin (6.0 mmol, 2.0 eq), 0.36 mL Kohlenstoffdisulfid (6.0 mmol, 2.0 eq) und 0.36 mL Benzylbromid (3.0 mmol, 1.0 eq) umgesetzt. Säulenchromatographie an Kieselgel (DCM/MeOH/NEt₃ 90:10:0.1) ergab 638 mg von **Schl32192** (75.7%) in Form eines braunen Öls.

| | | | | |
|---|---|---|---|---|
| **DC:** | R_{f} = 0.33 (DCM/ MeOH/ NEt₃ 90:10:0.1). | | | |
| **¹H-NMR:** | (CDCl₃, 400 MHz), δ [ppm] = 7.40-7.20 (*m*, 5H, CH_{(Phenyl)}), 4.55 (*s*, 2H, S-C*H₂*-C), 4.44-4.22 (*m*, 2H, N-CH*H*-CH₂-N-Et, N-CH*H*-CH₂-N-Et), 4.00-3.81 (*m*, 2H, N-CH*H*-CH₂-N-Et, N-CH*H*-CH₂-N-Et), 2.60-2.39 (*m*, 6H, N-C*H₂*-CH3, N-CH₂-C*H₂*-N-Et, N-CH₂-C*H₂*-N-Et), 1.11-1.05 (*m*, 3H, N-CH₂-C*H₃*). | | | |
| **¹³C-NMR:** | (CDCl₃, 100 MHz), δ [ppm] = 196.4 (C=S), 136.0 (S-CH₂-C-CH), 129.5 (C-CH-CH-CH, C-CH-CH-CH), 128.7 (C-CH-CH-CH, C-CH-CH-CH), 127.6 (C-CH-CH-CH), 52.3 (N-CH₂-CH₂-N-Et, N-CH₂-CH₂-N-Et), 52.0 (N-CH₂-CH₃), 42.2 (S-CH₂-C), 12.1 (N-CH₂-CH₃). | | | |
| **IR (ATR):** | v (cm⁻¹) = 3060 (w), 3027 (w), 2968 (w), 2930 (w), 2808 (w), 1601 (w), 1494 (m), 1467 (m), 1451 (vs), 1420 (m), 1379 (w), 1344 (w), 1330 (m), 1307 (m), 1286 (s), 1268 (s), 1228 (vs), 1217 (vs), 1156 (s), 1123 (vs), 1070 (m), 1016 (s), 994 (vs), 924 (m), 834 (w), 765 (w), 695 (vs), 631 (w), 568 (w), 549 (w), 536 (m), 477 (m), 421 (w). | | | |
| **MS** (ESI+): | m/z (%) = 281 (100, [M+H]⁺) | | | |
| **HRMS** (ESI+): | m/z für C₁₄H₂₀N₂S₂ | | | |
| | ber.: | 280.1068 | | gef.: 280.1060 |
| **EA:** | ber.: | C:59.96 | H:7.19 | N:9.99 |
| | gef.: | C:59.90 | H:7.18 | N:10.04 |

### Darstellung von Benzyl-4-propylpiperazin-1-carbodithioat (Schl32193):

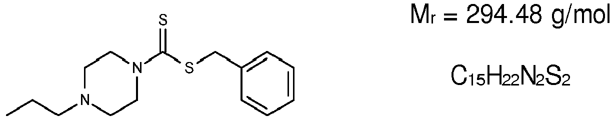

Gemäß **AAV7** wurden 871 mg 1-*N*-Propylpiperazindihydrobromid (3.0 mmol, 1.0 eq), 2230 mg Kaliumphosphat (10.5 mmol, 3.5 eq), 0.54 mL Kohlenstoffdisulfid (9.0 mmol, 3.0 eq) und 0.36 mL Benzylbromid (3.0 mmol, 1.0 eq) umgesetzt. Säulenchromatographie an Kieselgel (DCM/ MeOH/ NEt₃ 90:10:0.1) ergab 364 mg von **Schl32193** (41.2%) in Form eines gelben Öls.

| | | | | |
|---|---|---|---|---|
| **DC:** | R_{f} = 0.58 (DCM/ MeOH/ NEt₃ 90:10:0.1). | | | |
| **¹H-NMR:** | (CDCl₃, 400 MHz), δ [ppm] = 7.38-7.22 (*m*, 5H, CH_{(Phenyl)}), 4.55 (*s*, 2H, S-C*H*_{*2*-}C), 4.44-4.24 (*m*, 2H, C(=S)-N-CH*H*-CH₂-N, C(=S)-N-CH*H*-CH₂-N), 3.97-3.81 (*m*, 2H, C(=S)-N-CH*H*-CH₂-N, C(=S)-N-CH*H*-CH₂-N), 2.56-2.41 (m, 4H, C(=S)-N-CH₂-C*H₂*-N, C(=S)-N-CH₂-C*H₂*-N), 2.32 (*t*, ³J = 7.6 Hz, 2H, N-C*H₂*-CH₂-CH₃), 1.56-1.44 (*m*, 2H, N-C*H₂*-CH₂-CH₃), 0.91 (*t*, ³J = 7.5 Hz, 3H, N-CH₂-CH₂-C*H₃*). | | | |
| **¹³C-NMR:** | (CDCl₃, 100 MHz), δ [ppm] = 196.4 (C=S), 136.0 (S-CH₂-C-CH), 129.5 (C-CH-CH-CH, C-CH-CH-CH), 128.7 (C-CH-CH-CH, C-CH-CH-CH), 127.6 (C-CH-CH-CH), 60.2 (N-CH₂-CH₂-CH₃), 52.8 (C(=S)-N-CH₂-CH₂-N, C(=S)-N-CH₂-CH₂-N), 42.3 (S-CH₂-C), 20.1 (N-CH₂-CH₂-CH₃), 12.0 (N-CH₂-CH₂-CH₃). | | | |
| **IR (ATR):** | v (cm⁻¹) = 3061 (w), 3027 (w), 2956 (w), 2930 (w), 2872 (w), 2808 (w), 2774 (w), 1650 (w), 1601 (w), 1494 (m), 1465 (m), 1419 (s), 1375 (w), 1331 (w), 1300 (w), 1273 (m), 1260 (m), 1224 (vs), 1156 (s), 1141 (m), 1099 (w), 1070 (w), 1027 (s), 994 (vs), 935 (m), 915 (w), 898 (w), 844 (w), 803 (w), 771 (w), 695 (vs), 656 (w), 563 (w), 549 (w), 477 (w), 419 (w). | | | |
| **MS** (ESI+): | m/z (%) = 295 (100, [M+H]⁺) | | | |
| **HRMS** (ESI+): | m/z für C₁₅H₂₂N₂S₂ | | | |
| | ber.: | 294.1224 | | gef.: 294.1247 |
| **EA:** | ber.: | C:61.18 | H: 7.53 | N: 9.51 |
| | gef.: | C:61.05 | H: 7.66 | N: 9.37 |

### Darstellung von Benzyl-4-[2-(isopropylamino)-2-oxo-ethyl]piperazin-1-carbodithioat (Schl32194):

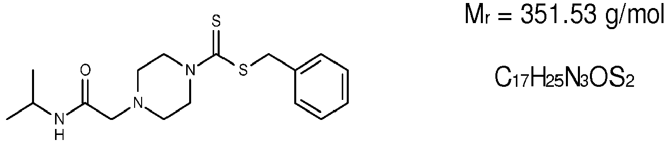

Gemäß **AAV7** wurden 559 mg *N*-Isopropyl-2-piperazin-1-yl-acetamid (3.0 mmol, 1.0 eq), 955 mg Kaliumphosphat (4.5 mmol, 1.5 eq), 0.54 mL Kohlenstoffdisulfid (9.0 mmol, 3.0 eq) und 0.36 mL Benzylbromid (3.0 mmol, 1.0 eq) umgesetzt. Säulenchromatographie an Kieselgel (DCM/ MeOH/ NEt₃ 90:10:0.1) ergab 686 mg von **Schl32194** (65.2%) in Form eines farblosen Feststoffes.

| | | | | |
|---|---|---|---|---|
| **DC:** | R_{f} = 0.36 (DCM/ MeOH/ NEt₃ 90:10:0.1). | | | |
| **Fp.:** | 86 °C | | | |
| **¹H-NMR:** | (CDCl₃, 400 MHz), δ [ppm] = 7.38-7.21 (*m*, 5H, CH_{(Phenyl)}), 6.76 (*sbr,* 1H, N*H*), 4.53 (*s*, 2H, S-C*H₂*-C), 4.44-4.24 (*m*, 2H, N-CH*H*-CH₂-N-CH₂), 4.16-4.02 (*m*, 1H, NH-C*H*), 4.00-3.84 (*m*, 2H, N-CH*H*-CH₂-N-CH₂), 2.99 (*s*, 2H, N-C*H₂*-C(=O)), 2.64-2.53 (*m*, 4H, N-CH₂-C*H₂*-N-CH₂, N-CH₂-C*H₂*-N-CH₂), 1.15 (*d*, ³J = 6.4 Hz, 6H, C*H₃*-CH-C*H₃*). | | | |
| **¹³C-NMR:** | (CDCl₃, 100 MHz), δ [ppm] = 197.2 (C=S), 168.3 (C=O), 135.7 (S-CH₂-C-CH), 129.5 (C-CH-CH-CH, C-CH-CH-CH), 128.7 (C-CH-CH-CH, C-CH-CH-CH), 127.7 (C-CH-CH-CH), 61.2 (N-CH₂-C), 52.9 (N-CH₂-CH₂-N-CH₂, N-CH₂-CH₂-N-CH₂), 45.8 (N-CH-CH₃), 41.0 (S-CH₂-C), 22.9 (CH-CH₃). | | | |
| **IR (ATR):** | v (cm⁻¹) = 3285 (m), 3067 (w), 3031 (w), 2971 (w), 2919 (w), 1644 (s), 1543 (s), 1493 (w), 1468 (s), 1453 (m), 1431 (m), 1418 (m), 1395 (s), 1353 (w), 1322 (w), 1290 (w), 1263 (w), 1241 (m), 1220 (m), 1203 (s), 1156 (m), 1142 (s), 1129 (s), 1107 (w), 1071 (w), 1054 (w), 1022 (s), 1005 (s), 980 (s), 966 (s), 940 (m), 909 (w), 842 (m), 805 (w), 757 (m), 719 (m), 693 (vs), 643 (m), 601 (s), 566 (m), 550 (m), 469 (w), 419 (w). | | | |
| **MS** (ESI+): | m/z (%) = 352 (100, [M+H]⁺) | | | |
| **HRMS** (ESI+): | m/z für C₁₇H₂₃N₃OS₂ | | | |
| | ber.: | 351.1439 | | gef.: 351.1445 |
| **EA:** | ber.: | C:58.08 | H:7.17 | N:11.95 |
| | gef.: | C:57.86 | H:7.32 | N:11.53 |

### Darstellung von Benzyl-4-hydroxypiperidin-1-carbodithioat (Schl32195):

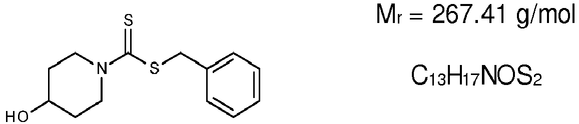

Gemäß **AAV7** wurden 304 mg 4-Hydroxypiperidin (3.0 mmol, 1.0 eq), 764 mg Kaliumphosphat (3.6 mmol, 1.2 eq), 0.54 mL Kohlenstoffdisulfid (9.0 mmol, 3.0 eq) und 0.36 mL Benzylbromid (3.0 mmol, 1.0 eq) umgesetzt. Säulenchromatographie an Kieselgel (EtOAc) ergab 621 mg von **Schl32195** (77.5%) in Form eines gelben Öls.

| | | | | |
|---|---|---|---|---|
| **DC:** | R_{f}= 0.44 (EtOAc). | | | |
| **¹H-NMR:** | (CDCl₃, 400 MHz), δ [ppm] = 7.38-7.21 (*m*, 5H, CH_{(Phenyl)}), 4.70-4.55 (*m*, 2H, N-CH*H*-CH₂-CH-OH, N-CH*H*-CH₂-CH-OH), 4.53 (s, 2H, S-C*H*_{*2*-}C), 4.05-3.97 (m, 1H, C*H*-OH), 3.68 (*sbr,* 1H, O*H*), 2.69-2.56 (*m*, 2H, N-CH*H*-CH₂-CH-OH, N-CH*H*-CH₂-CH-OH), 1.95-1.81 (*m*, 2H, N-CH₂-CH*H*-CH-OH, N-CH₂-CH*H*-CH-OH), 1.68-1.55 (*m*, 2H, N-CH₂-CH*H*-CH-OH, N-CH₂-CH*H*-CH-OH). | | | |
| **¹³C-NMR:** | (CDCl₃, 100 MHz), δ [ppm] = 195.8 (C=S), 135.9 (S-CH₂-C-CH), 129.5 (C-CH-CH-CH, C-CH-CH-CH), 128.7 (C-CH-CH-CH, C-CH-CH-CH), 127.7 (C-CH-CH-CH), 66.1 (CH-OH), 48.5 (N-CH₂-CH₂-CH-OH, N-CH₂-CH₂-CH-OH), 42.5 (S-CH₂-C), 33.6 (N-CH₂-CH₂-CH-OH, N-CH₂-CH₂-CH-OH). | | | |
| **IR (ATR):** | v (cm⁻¹) =3377 (w), 3059 (w), 3026 (w), 2995 (w), 1601 (w), 1493 (w), 1474 (m), 1422 (vs), 1358 (w), 1327 (w), 1257 (s), 1209 (vs), 1173 (m), 1109 (w), 1062 (s), 1000 (s), 968 (s), 946 (s), 911 (s), 892 (m), 847 (m), 803 (w), 772 (w), 694 (vs), 632 (w), 565 (m), 533 (s), 476 (m), 427 (m). | | | |
| **MS** (ESI+): | m/z (%) = 268 (100, [M+H]⁺) | | | |
| **HRMS** (ESI+): | m/z für C₁₃H₁₇NOS₂ | | | |
| | ber.: | 267.0752 | | gef.: 267.0755 |
| **EA:** | ber.: | C: 58.39 | H: 6.41 | N:5.24 |
| | gef.: | C: 58.69 | H: 6.29 | N: 5.11 |

### Darstellung von Benzyl-4-oxopiperidin-1-carbodithioat (Schl32196):

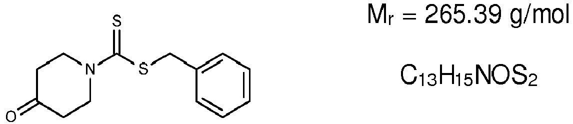

Gemäß **AAV7** wurden 461 mg 4-Piperidinonhydrochlorid (3.0 mmol, 1.0 eq), 1592 mg Kaliumphosphat (7.5 mmol, 2.5 eq), 0.54 mL Kohlenstoffdisulfid (9.0 mmol, 3.0 eq) und 0.36 mL Benzylbromid (3.0 mmol, 1.0 eq) umgesetzt. Säulenchromatographie an Kieselgel (Cyclohexan/ EtOAc 3:1) ergab 736 mg von **Schl32196** (92.5%) in Form eines gelben Öls.

| | | | | |
|---|---|---|---|---|
| **DC:** | R_{f} = 0.35 (Cyclohexan/ EtOAc 3:1). | | | |
| **¹H-NMR:** | (CDCl₃, 400 MHz), δ [ppm] = 7.38-7.23 (*m*, 5H, CH_{(Phenyl)}), 4.59 (s, 2H, S-C*H*_{*2*-}C), 4.33-4.20 (*m*, 2H, N-CH*H*-CH₂-C, N-CH*H*-CH₂-C), 2.67-2.47 (*m*, 6H, N-CH*H*-CH₂-C, N-CH*H*-CH₂-C, N-CH₂-C*H₂*-C, N-CH₂-C*H₂*-C). | | | |
| **¹³C-NMR:** | (CDCl₃, 100 MHz), δ [ppm] = 205.9 (C=O), 197.5 (C=S), 135.6 (S-CH₂-C-CH), 129.5 (C-CH-CH-CH, C-CH-CH-CH), 128.8 (C-CH-CH-CH, C-CH-CH-CH), 127.8 (C-CH-CH-CH), 52.5 (N-CH₂-CH₂-C, N-CH₂-CH₂-C), 42.5 (S-CH₂-C), 39.9 (N-CH₂-CH₂-C, N-CH₂-CH₂-C). | | | |
| **IR (ATR):** | v (cm⁻¹) = 3059 (w), 3026 (w), 2963 (w), 2876 (w), 1901 (w), 1717 (vs), 1493 (w), 1475 (w), 1451 (m), 1398 (vs), 1356 (w), 1309 (w), 1259 (vs), 1203 (vs), 1084 (w), 1070 (w), 1008 (s), 975 (m), 931 (vs), 828 (m), 771 (m), 695 (vs), 619 (w), 565 (m), 522 (m), 480 (m), 424 (s). | | | |
| **MS** (ESI+): | m/z (%) = 266 (100, [M+H]⁺) | | | |
| **HRMS** (ESI+): | m/z für C₁₃H₁₅NOS₂ | | | |
| | ber.: | 265.0595 | | gef.: 265.0614 |
| **EA:** | ber.: | C:58.83 | H:5.70 | N:5.28 |
| | gef.: | C:50.03 | H:5.73 | N:5.45 |

### Darstellung von 2-[Benzylsulfanylcarbothioyl(methyl)amino]ethansulfonsäure (Schl32197):

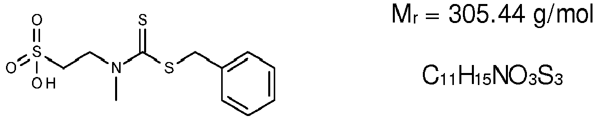

Gemäß **AAV7** wurden 967 mg Natrium-*N*-Methyltaurin (6.0 mmol, 2.0 eq), 956 mg Kaliumphosphat (4.5 mmol, 1.5 eq), 0.36 mL Kohlenstoffdisulfid (6.0 mmol, 2.0 eq) und 0.36 mL Benzylbromid (3.0 mmol, 1.0 eq) umgesetzt. Anschließend wurde das Reaktionsgemisch mit 100 mL EtOAc verdünnt und 5 Mal gegen je 75 mL 1M NaOH-Lösung ausgeschüttelt. Die vereinigten wässrigen Phasen wurden mit 6M HCl auf pH=1 eingestellt. Der entstehende Niederschlag wurde abgesaugt, mit Wasser gewaschen und getrocknet. Es wurden 735 mg von **Schl32197** (80.3%) als gelber Feststoff erhalten.

| | | | | |
|---|---|---|---|---|
| **Fp.:** | 197 °C | | | |
| **¹H-NMR:** | (DMSO-D₆, 400 MHz), δ [ppm] = 7.44-7.20 (*m*, 5H, CH_{(Phenyl)}), 4.45 (*s*, 2H, S-C*H₂*-C), 3.41-3.37 (*m*, 2H, S-C*H₂*-CH₂-N), 3.29-3.25 (*m,* 2H, S-CH₂-C*H₂*-N), 1.94 (N-C*H₃*). | | | |
| **¹³C-NMR:** | (DMSO-D₆, 100 MHz), δ [ppm] = 195.9 (C=S), 135.9 (S-CH₂-C-CH), 129.6 (C-CH-CH-CH, C-CH-CH-CH), 128.9 (C-CH-CH-CH, C-CH-CH-CH), 128.0 (C-CH-CH-CH), 52.5 (S-CH₂-CH₂-N), 44.1 (S-CH₂-C), 43.6 (S-CH₂-CH₂-N), 38.9 (N-CH₃). | | | |
| **IR (ATR):** | v (cm⁻¹) = 3027 (w), 1719 (w), 1640 (w), 1601 (w), 1537 (w), 1492 (w), 1453 (w), 1359 (w), 1322 (w), 1214 (s), 1174 (s), 979 (vs), 876 (w), 798 (w), 763 (w), 735 (w), 695 (vs), 601 (w), 560 (w), 519 (w), 479 (w). | | | |
| **MS** (ESI-): | m/z (%) = 304 (100, [M-H]⁻) | | | |
| **HRMS** (ESI+): | m/z für C₁₁H₁₅NO₃S₃ | | | |
| | ber.: | 305.0214 | | gef.: 305.0212 |
| **EA:** | ber.: | C:43.26 | H: 4.95 | N: 4.59 |
| | gef.: | C:43.52 | H: 4.74 | N: 4.62 |

### Darstellung von Benzyl-1,2,4-triazol-1-carbodithioat (Schl32198):

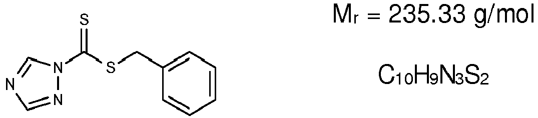

Gemäß **AAV7** wurden 345 mg 1,2,4-1*H*-Triazol (5.0 mmol, 1.0 eq), 1592 mg Kaliumphosphat (7.5 mmol, 1.5 eq), 0.90 mL Kohlenstoffdisulfid (15.0 mmol, 3.0 eq) und 0.61 mL Benzylbromid (5.0 mmol, 1.0 eq) umgesetzt. Säulenchromatographie an Kieselgel (Cyclohexan/ EtOAc 5:1) ergab 939 mg von **Schl32198** (79.9%) in Form eines gelben Feststoffes.

| | | | | |
|---|---|---|---|---|
| **DC:** | R_{f} = 0.41 (Cyclohexan/ EtOAc 5:1) | | | |
| **Fp.:** | 54 °C | | | |
| **¹H-NMR:** | (CDCl₃, 400 MHz), δ [ppm] = 8.11 (s, 1H, N-C*H*-N-N-C(=S)), 8.09 (*s*, 1H, N-C*H*-N-C(=S)), 7.40-7.21 (*m*, 5H, CH_{(Phenyl)}), 4.47 (s, 2H, S-C*H₂*-C). | | | |
| **¹³C-NMR:** | (CDCl₃, 100 MHz), δ [ppm] = 197.5 (C=S), 153.5 (N-CH-N-N-C(=S)), 143.2 (N-CH-N-C(=S)), 133.6 (S-CH₂-C-CH), 129.6 (C-CH-CH-CH, C-CH-CH-CH), 128.9 (C-CH-CH-CH, C-CH-CH-CH), 128.2 (C-CH-CH-CH), 41.4 (S-CH₂-C). | | | |
| **IR (ATR):** | v (cm⁻¹) = 3136 (w), 3123 (w), 3062 (w), 2978 (w), 1530 (w), 1504 (s), 1493 (w), 1454 (w), 1419 (w), 1383 (w), 1366 (s), 1316 (w), 1282 (s), 1262 (m), 1247 (m), 1189 (s), 1157 (w), 1117 (w), 1072 (vs), 1026 (w), 993 (s), 941 (w), 899 (w), 854 (vs), 812 (m), 800 (m), 776 (s), 705 (vs), 693 (s), 657 (vs), 627 (s), 573 (m), 532 (w), 489 (w), 478 (s), 409 (w). | | | |
| **MS** (ESI+): | m/z (%) = 236 (100, [M+H]⁺) | | | |
| **HRMS** (ESI+): | m/z für C₁₀H₉N₃S₂ | | | |
| | ber.: | 235.0238 | | gef.: 235.0216 |
| **EA:** | ber.: | C:51.04 | H:3.85 | N:17.86 |
| | gef.: | C:51.32 | H:4.08 | N:17.71 |

### Darstellung von Benzylindol-1-carbodithioat (Schl32199):

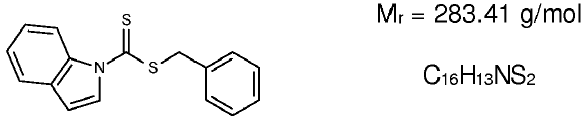

Gemäß **AAV7** wurden 352 mg Indol (3.0 mmol, 1.0 eq), 1274 mg Kaliumphosphat (6.0 mmol, 2.0 eq), 0.54 mL Kohlenstoffdisulfid (9.0 mmol, 3.0 eq) und 0.36 mL Benzylbromid (3.0 mmol, 1.0 eq) umgesetzt. Säulenchromatographie an Kieselgel (Cyclohexan) ergab 343 mg von **Schl32199** (40.4%) in Form eines gelben Feststoffes.

| | | | | |
|---|---|---|---|---|
| **DC:** | R_{f} = 0.15 (Cyclohexan). | | | |
| **Fp.:** | 60 °C | | | |
| **¹H-NMR:** | (CDCl₃, 400 MHz), δ [ppm] = 8.97 (*d*, ³J = 8.5 Hz, 1H, C*H*_{(Indol)}), 8.10 (*d*, ³J = 5.8 Hz, 1H, C*H*_{(Indol)}), 7.55 (*d*, ³J = 7.8 Hz, 1H, C*H*_{(Indol)}), 7.44 (*d*, ³J = 7.1 Hz, 2H, C*H*_{(Indol)}), 7.39-7.26 (*m*, 5H, C*H*_{(Pnenyl)}), 6.66 (*d*, ³J = 7.7 Hz, 1H, C*H*_{(Indol)}), 4.64 (*s*, 2H, S-C*H₂*-C). | | | |
| **¹³C-NMR:** | (CDCl₃, 100 MHz), δ [ppm] = 198.4 (C=S), 139.8 (C_{q (Indol)}), 133.7 (S-CH₂-C-CH), 132.1 (C_{q (Indol)}), 129.6 (C-CH-CH-CH, C-CH-CH-CH), 128.9 (C-CH-CH-CH, C-CH-CH-CH), 128.0 (C-CH-CH-CH), 125.4 (Ct _{(Indol)}), 124.4 (Ct _{(Indol)}), 121.4 (C_{t (Indol)}), 117.1 (C_{t (Indol)}), 108.8 (C_{t (Indol)}), 42.0 (S-CH₂-C). | | | |
| **IR (ATR):** | v (cm⁻¹) = 3411 (w), 3059 (w), 1584 (w), 1537 (w), 1493 (w), 1449 (s), 1347 (m), 1313 (s), 1229 (w), 1201 (s), 1154 (m), 1125 (w), 1097 (w), 1072 (m), 1028 (w), 1015 (w), 938 (w), 913 (w), 881 (m), 849 (s), 802 (w), 740 (vs), 694 (vs), 652 (m), 620 (w), 561 (m), 516 (w), 462 (w), 420 (w). | | | |
| **MS** (ESI+): | m/z (%) = 284 (100, [M+H]⁺) | | | |
| **HRMS** (ESI+): | m/z für C₁₆H₁₃NS₂ | | | |
| | ber.: | 283.0489 | | gef.: 283.0478 |
| **EA:** | ber.: | C:67.81 | H: 4.62 | N: 4.94 |
| | gef.: | C:67.39 | H: 4.87 | N: 4.61 |

### Darstellung von Benzyl-4-(furan-2-carbonyl)piperazin-1-carbodithioat (Schl32200):

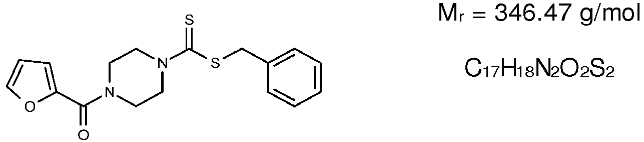

Gemäß **AAV7** wurden 1621 mg 1-(2-Furoyl)piperazin (9.0 mmol, 1.0 eq), 2865 mg Kaliumphosphat (13.5 mmol, 1.5 eq), 1.60 mL Kohlenstoffdisulfid (27.0 mmol, 3.0 eq) und 1.10 mL Benzylbromid (9.0 mmol, 1.0 eq) umgesetzt. Säulenchromatographie an Kieselgel (Cyclohexan/ EtOAc 1:1) ergab 2865 mg von **Schl32200** (91.3%) in Form eines gelben Öls.

| | | | | |
|---|---|---|---|---|
| **DC:** | R_{f} = 0.32 (Cyclohexan/ EtOAc 1:1). | | | |
| **¹H-NMR:** | (CDCl₃, 400 MHz), δ [ppm] = 7.47-7.40 (*m*, 1H, C-O-C*H*), 7.35-7.17 (*m*, 5H, CH_{(Pheny)}), 7.02-6.96 (*m*, 1H, C(=O)-C-C*H*, 6.44-6.39 (*m*, 1H, C-O-CH-C*H*), 4.48 (s, 2H, S-C*H₂*-C), 4.38-4.21 (*m*, 2H, N-CH*H*-CH₂-N-C(=O), N-CH*H*-CH₂-C(=O)), 3.90-3.74 (*m*, 2H, N-CH*H*-CH₂-C(=O), N-CH*H*-CH₂-C(=O)), 2.00-1.91 (*m*, 2H N-CH₂-CH*H*-C(=O), N-CH₂-CH*H*-C(=O)), 1.30-1.18 (*m*, 2H N-CH₂-CH*H*-C(=O), N-CH₂-CH*H*-C(=O)). | | | |
| **¹³C**-**NMR:** | (CDCl₃, 100 MHz), δ [ppm] = 197.1 (C=S), 159.1 (C=O), 147.5 (C(=O)-C), 144.3 (C-O-CH), 135.8 (S-CH₂-C-CH), 129.4 (C-CH-CH-CH, C-CH-CH-CH), 128.7 (C-CH-CH-CH, C-CH-CH-CH), 127.7 (C-CH-CH-CH), 117.4 (C(=O)-C-CH), 111.7 (C(=O)-C-CH-CH), 60.4 (N-CH₂-CH₂-N-(=O), N-CH₂-CH₂-N-(C=O)), 59.9 (N-CH₂-CH₂-N-C(=O), N-CH₂-CH₂-N-C(=O)), 42.1 (S-CH₂-C). | | | |
| **IR (ATR):** | v (cm⁻¹) = 3118 (w), 3028 (w), 2981 (w), 2912 (w), 2107 (w), 1732 (s), 1622 (s), 1573 (w), 1481 (m), 1452 (w), 1407 (vs), 1371 (s), 1284 (m), 1268 (m), 1236 (vs), 1217 (vs), 1180 (s), 1159 (s), 1088 (w), 1072 (w), 1042 (s), 995 (vs), 949 (m), 928 (m), 884 (w), 857 (w), 752 (vs), 697 (vs), 633 (w), 608 (w), 595 (w), 569 (w), 548 (w), 463 (s), 414 (w). | | | |
| **MS** (ESI+): | m/z (%) = 347 (100, [M+H]⁺) | | | |
| **HRMS** (ESI+): | m/z für C₁₇H₁₈N₂O₂S₂ | | | |
| | ber.: 346.0810 | | | gef.: 346.0813 |
| **EA:** | ber.: | C: 58.93 | H: 5.24 | N:8.09 |
| | gef.: | C:59.12 | H: 5.29 | N:8.14 |

### Darstellung von Benzyl-3,4-dihydro-1H-isochinolin-2-carbodithioat (Schl32205):

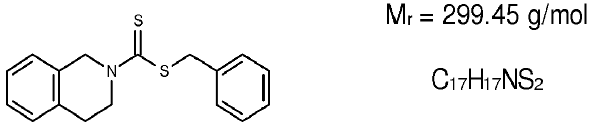

Gemäß **AAV7** wurden 0.76 mL Tetrahydroisochinolin (6.0 mmol, 2.0 eq), 1911 mg Kaliumphosphat (9.0 mmol, 3.0 eq), 0.36 mL Kohlenstoffdisulfid (6.0 mmol, 2.0 eq) und 0.36 mL Benzylbromid (3.0 mmol, 1.0 eq) umgesetzt. Säulenchromatographie an Kieselgel (Cyclohexan/ EtOAc 6:1) ergab 849 mg von **Schl32205** (94.4%) in Form eines blassgelben Feststoffes.

| | | | | |
|---|---|---|---|---|
| **DC**: | R_{f} = 0.47 (Cyclohexan/ EtOAc 6:1). | | | |
| **Fp**.**:** | 59 °C | | | |
| **¹H-NMR:** | (CDCl₃, 400 MHz), δ [ppm] = 7.46-7.40 (*m*, 2H, C*H*_{(Aromat)}), 7.37-7.15 (*m*, 7H, C*H*_{(Aromat)}), 5.40-5.32 (*m*, 1H, C-C*H*H-N), 5.06-4.94 (*m*, 1H, C-C*H*H-N), 4.64 (s, 2H, S-C*H*₂C), 4.52-4.42 (m, 1H, C-CH₂-C*H*H-N), 4.08-3.99 (*m*, 1H, C-CH₂-C*H*H-N), 3.03-2.94 (m, 2H, C-C*H₂*-CH₂-N). | | | |
| **¹³C**-**NMR:** | (CDCl₃, 100 MHz), δ [ppm] = 197.4 (C=S), 136.0 (C_{q (Benzyl)}), 134.1 (C_{q (Isochinolin)}), 129.5 (C_{t (Benzyl)}), 129.0 (C_{q (Isochinolin)}), 128.7 (C_{t (Benzyl)}), 127.5 (C_{t (Benzyl)}), 126.9 (C_{t (Isochinolin)}), 126.8 (C_{t (Isochinolin)}), 125.9 (C_{t (Isochinolin)}), 125.0 (C_{t (Isochinolin)}), 51.4 (C-CH₂-N), 48.0 (C-CH₂-CH₂-N), 41.9 (S-CH₂-C), 29.2 (C-CH₂-CH₂-N). | | | |
| **IR (ATR):** | v (cm⁻¹) = 3024 (w), 2966 (w), 2880 (w), 1492 (w), 1472 (s), 1451 (w), 1441 (vs), 1426 (m), 1405 (w), 1351 (w), 1292 (m), 1274 (w), 1246 (w), 1239 (w), 1214 (vs), 1148 (m), 1069 (w), 1000 (m), 980 (w), 948 (s), 927 (m), 911 (m), 853 (m), 813 (w), 751 (vs), 704 (vs), 695 (vs), 653 (s), 567 (w), 553 (w), 543 (w), 508 (w), 485 (m), 456 (m), 433 (w). | | | |
| **MS** (ESI+): | m/z (%) = 300 (100, [M+H]⁺) | | | |
| **HRMS** (ESI+): | m/z für C₁₇H₁₇NS₂ | | | |
| | ber.: 299.0802 | | gef.: 299.0788 | |
| **EA:** | ber.: | C:68.18 | H: 5.72 | N: 4.68 |
| | gef.: | C: 68.52 | H: 5.74 | N: 4.62 |

### Darstellung von Benzyl-3,4-dihydro-2H-chinolin-1-carbodithioat (Schl32206):

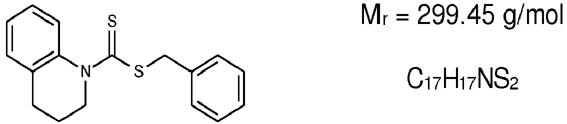

Gemäß **AAV7** wurden 0.75 mL Tetrahydrochinolin (6.0 mmol, 2.0 eq), 1911 mg Kaliumphosphat (9.0 mmol, 3.0 eq), 0.36 mL Kohlenstoffdisulfid (6.0 mmol, 2.0 eq) und 0.36 mL Benzylbromid (3.0 mmol, 1.0 eq) umgesetzt. Säulenchromatographie an Kieselgel (Cyclohexan/ EtOAc 6:1) ergab 361 mg von **Schl32206** (40.2%) in Form eines blassgelben Feststoffes.

| | | | | |
|---|---|---|---|---|
| **DC**: | R_{f}= 0.59 (Cyclohexan/ EtOAc 6:1). | | | |
| **Fp**.**:** | 64 °C | | | |
| **¹H-NMR:** | (CDCl₃, 400 MHz), δ [ppm] = 7.49-7.33 (*m*, 5H, C*H*_{(Benzyl)}), 7.13-7.06 (*m*, 2H, C*H*_{(Chinolin)}), 6.74-6.67 (*m*, 1H, C*H*_{(Chinolin)}), 6.65-6.59 (*m*, 1H, C*H*_{(Chinolin)}), 4.58 (*s*, 2H, S-C*H₂*-C), 3.47 *(t,* 3_{J} = 5.1 Hz, 2H, C-CH₂-CH₂-C*H₂*-N), 2.93 *(t,* 3_{J} = 6.0 Hz, 2H, C-C*H₂*-CH₂-CH₂-N), 2.17-2.08 *(m,* 2H, C-CH₂-C*H₂*-CH₂-N). | | | |
| **¹³C**-**NMR:** | (CDCl₃, 100 MHz), δ [ppm] = 203.4 (C=S), 145.8 (C_{q (Chinolin)}), 136.7 (C_{q (Benzyl)}), 135.9 (C_{q (Chinolin)}), 129.3 (C_{t (Isochinolin)}), 128.8 (C_{t (Benzyl)}), 128.0 (C_{t (Isochinolin)}), 127.4 (C_{t (Benzyl)}), 126.7 (C_{t (Benzyl)}), 126.3 (C_{t (Isochinolin)}), 122.4 (C_{t (Isochinolin)}), 55.4 (C-CH₂-CH₂-CH₂-N), 50.1 (S-CH₂-C), 28.5 (C-CH₂-CH₂-CH₂-N), 22.6 (C-CH₂-CH₂-CH₂-N). | | | |
| **IR (ATR):** | v (cm⁻¹) = 3061 (w), 3025 (w), 2925 (w), 1600 (s), 1573 (w), 1504 (vs), 1494 (vs), 1450 (s), 1398 (w), 1343 (s), 1329 (m), 1299 (m), 1245 (m), 1211 (m), 1154 (w), 1115 (w), 1092 (w), 1074 (w), 1059 (w), 1027 (w), 1001 (w), 969 (w), 868 (w), 801 (w), 740 (vs), 730 (vs), 693 (vs), 627 (w), 615 (w), 591 (w), 556 (w), 540 (w), 489 (w), 455 (w), 436 (w). | | | |
| **MS** (ESI+): | m/z (%) = 300 (100, [M+H]⁺) | | | |
| **HRMS** (ESI+): | m/z für C₁₇H₁₇NS₂ | | | |
| | ber.: | 299.0802 | | gef.: 299.0768 |
| **EA:** | ber.: | C: 68.18 | H: 5.72 | N: 4.68 |
| | gef.: | C: 68.50 | H: 5.88 | N: 4.78 |

### Darstellung von Benzyl-4-pyrimidin-2-ylpiperazin-1-carbodithioat (Schl32207):

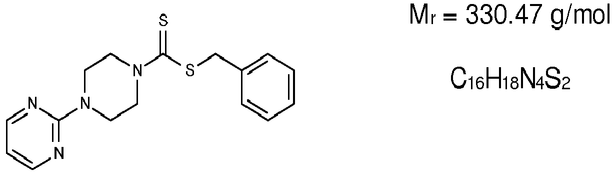

Gemäß **AAV7** wurden 0.85 mL 2-Piperazin-1-ylpyrimidin (6.0 mmol, 2.0 eq), 1911 mg Kaliumphosphat (9.0 mmol, 3.0 eq), 0.36 mL Kohlenstoffdisulfid (6.0 mmol, 2.0 eq) und 0.36 mL Benzylbromid (3.0 mmol, 1.0 eq) umgesetzt. Säulenchromatographie an Kieselgel (Cyclohexan/ EtOAc 3:1) ergab 918 mg von **Schl32207** (92.6%) in Form eines blassgelben Feststoffes.

| | | | | |
|---|---|---|---|---|
| **DC**: | R_{f} = 0.31 (Cyclohexan/ EtOAc 3:1). | | | |
| **Fp**.**:** | 135 °C | | | |
| **¹H-NMR:** | (CDCl₃, 400 MHz), δ [ppm] = 8.33 (*d*, ³J = 4.8 Hz, 2H, N=C*H*=CH=CH=N), 7.41-7.37 (*m*, 2H, C*H*_{(Benzyl)}), 7.34-7.23 (*m*, 3H, C*H*_{(Benzyl)}), 6.56 (*t*, 3_{J} = 4.8 Hz, 1H, N=CH=C*H*=CH=N), 4.59 (s, 2H, S-C*H₂*-C), 4.52-4.33 (*m*, 2H, N=C-N-CH*H*-CH₂-N, N=C-N-CH*H*-CH₂-N), 4.15-4.01 (*m*, 2H, N=C-N-CH*H*-CH₂-N, N=C-N-CH*H*-CH₂-N), 3.99-3.86 (*m*, 4H, N=C-N-CH₂-C*H₂*-N, N=C-N-CH₂-C*H₂*-N). | | | |
| **¹³C**-**NMR:** | (CDCl₃, 100 MHz), δ [ppm] = 197.0 (C=S), 157.9 (N=CH=CH=CH=N), 135.8 (C-CH-CH-CH), 129.5 (C-CH-CH-CH, C-CH-CH-CH), 128.8 (C-CH-CH-CH, C-CH-CH-CH), 127.7 (C-CH-CH-CH), 110.8 (N=CH=CH=CH=N), 43.1 (N-CH₂-CH₂-N, N-CH₂-CH₂-N), 42.2 (S-CH₂-C). | | | |
| **IR (ATR):** | v (cm⁻¹) = 2904 (w), 1585 (s), 1548 (s), 1494 (s), 1453 (w), 1414 (m), 1386 (m), 1366 (m), 1354 (m), 1304 (w), 1261 (m), 1225 (w), 1214 (w), 1177 (s), 1152 (m), 1121 (w), 1090 (w), 1066 (w), 1046 (w), 1017 (s), 981 (vs), 939 (w), 915 (s), 908 (s), 863 (w), 819 (w), 794 (vs), 784 (s), 766 (s), 693 (vs), 638 (m), 563 (w), 517 (w), 478 (s), 443 (w), 409 (w). | | | |
| **MS** (ESI+): | m/z (%) = 331 (100, [M+H]⁺) | | | |
| **HRMS** (ESI+): | m/z für C₁₆H₁₈N₄S₂ | | | |
| | ber.: | 330.0973 | | gef.: 330.0999 |
| **EA:** | ber.: | C:58.15 | H: 5.49 | N:16.95 |
| | gef.: | C: 58.17 | H: 5.89 | N:16.66 |

### Darstellung von Benzyl-3-oxopiperazin-1-carbodithioat (Schl32208):

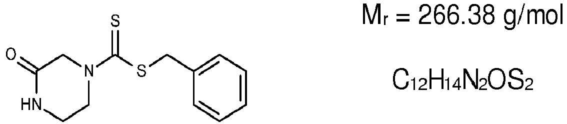

Gemäß **AAV7** wurden 601 mg Piperazin-2-on (6.0 mmol, 2.0 eq), 1274 mg Kaliumphosphat (6.0 mmol, 2.0 eq), 0.36 mL Kohlenstoffdisulfid (6.0 mmol, 2.0 eq) und 0.36 mL Benzylbromid (3.0 mmol, 1.0 eq) umgesetzt. Säulenchromatographie an Kieselgel (EtOAc) ergab 753 mg von **Schl32208** (94.2%) in Form eines blassgelben Feststoffes.

| | | | | |
|---|---|---|---|---|
| **DC:** | R_{f} = 0.20 (EtOAc). | | | |
| **Fp**.**:** | 178 °C | | | |
| **¹H-NMR:** | (DMSO-D₆, 400 MHz), δ [ppm] = 8.29 (*sbr*, 1H, N*H*), 7.39-7.34 (m, 2H, C*H*_{(Benzyl)}), 7.32-7.20 (*m*, 3H, C*H*_{(Benzyl)}), 4.61-4.48 (*m*, 4H, C(=O)-C*H₂*-N, S-C*H₂*-C), 4.38-4.20 (*m*, 2H, NH-C*H₂-*CH₂-N), 4.07-3.92 (*m*, 2H, NH-CH₂-C*H₂*-N). | | | |
| **¹³C**-**NMR:** | (DMSO-D₆, 100 MHz), δ [ppm] = 205.4 (C=S), 171.1 (C=O), 136.6 (C-CH-CH-CH), 129.7 (C-CH-CH-CH, C-CH-CH-CH), 129.0 (C-CH-CH-CH, C-CH-CH-CH), 127.9 (C-CH-CH-CH), 58.4 (C(=O)-CH₂-N), 55.8 (N-CH₂-CH₂-NH), 39.7 (S-CH₂-C), 34.4 (N-CH₂-CH₂). | | | |
| **IR (ATR):** | v (cm⁻¹) = 3178 (w), 3058 (w), 3029 (w), 2898 (w), 1661 (vs), 1491 (w), 1410 (m), 1383 (s), 1343 (s), 1276 (m), 1223 (s), 1203 (m), 1122 (m), 1070 (w), 1022 (s), 979 (w), 967 (w), 936 (w), 916 (w), 815 (m), 776 (m), 713 (m), 691 (vs), 599 (w), 566 (w), 516 (m), 490 (m), 477 (m), 418 (s). | | | |
| **MS** (ESI+): | m/z (%) = 267 (100, [M+H]⁺) | | | |
| **HRMS** (ESI+): | m/z für C₁₂H₁₄N₂OS₂ | | | |
| | ber.: | 266.0548 | | gef.: 266.0545 |
| **EA:** | ber.: | C:54.11 | H:5.30 | N:10.52 |
| | gef.: | C:53.99 | H: 5.33 | N:10.61 |

### Darstellung von Benzyl-4-(cyclopropancarbonyl)piperazin-1-carbodithioat (Schl32209):

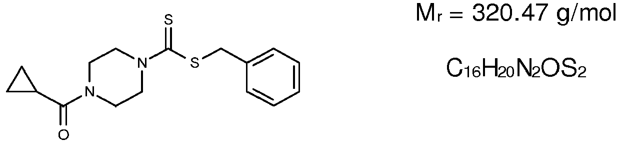

Gemäß **AAV10** wurden 0.85 mL Cyclopropyl(piperazin-1-yl)methanon (6.0 mmol, 2.0 eq), 636 mg Natriumcarbonat (6.0 mmol, 2.0 eq), 0.36 mL Kohlenstoffdisulfid (6.0 mmol, 2.0 eq) und 0.36 mL Benzylbromid (3.0 mmol, 1.0 eq) umgesetzt. Säulenchromatographie an Kieselgel (Cyclohexan/ EtOAc 1:1) ergab 899 mg von **Schl32209** (93.5%) in Form eines blassgelben Feststoffes.

| | | | | |
|---|---|---|---|---|
| **DC:** | R_{f} = 0.20 (Cyclohexan/ EtOAc 1:1). | | | |
| **Fp**.**:** | 88 °C | | | |
| **¹H-NMR:** | (CDCl₃, 400 MHz), δ [ppm] = 7.38-7.33 (*m*, 2H, C*H*_{(Benzyl)}), 7.32-7.21 (*m*, 3H, C*H*_{(Benzyl)}), 4.55 (*s*, 2H, S-C*H₂*-C), 4.46-4.29 (*m*, 2H, C(=O)-N-CH*H*-CH₂-N, C(=O)-N-CH*H*-CH₂-N), 4.05-3.88 (*m*, 2H, C(=O)-N-CH*H*-CH₂-N, C(=O)-N-CH*H*-CH₂-N), 3.78-3.66 (*m*, 4H, C(=O)-N-CH₂-C*H₂*-N, C(=O)-N-CH₂-C*H₂*-N), 1.73-1.64 (*m*, 1 H, C*H*_{(Cyclopropyl)}), 1.01-0.96 (*m*, 2H, CH*H*_{(Cyclopropyl)}-CH*H*_{(Cyclopropyl)}), 0.82-0.75 (*m*, 2H, CH*H*_{(Cyclopropyl)}-CH*H*_{(Cyclopropyl)}). | | | |
| **¹³C**-**NMR:** | (CDCl₃, 100 MHz), δ [ppm] = 197.4 (C=S), 172.6 (C=O), 135.7 (C-CH-CH-CH), 129.5 (C-CH-CH-CH, C-CH-CH-CH), 128.8 (C-CH-CH-CH, C-CH-CH-CH), 127.8 (C-CH-CH-CH), 60.5 (N-CH₂-CH₂-N, N-CH₂-CH₂-N), 42.2 (S-CH₂-C), 11.2 (CH_{2(Cyclopropyl)}-CH_{2(Cyclopropyl)}), 8.0 (CH_{(Cyclopropyl)}). | | | |
| **IR (ATR):** | v (cm⁻¹) = 3013 (w), 1632 (s), 1477 (m), 1447 (w), 1426 (s), 1409 (vs), 1377 (w), 1352 (w), 1338 (w), 1282 (w), 1263 (m), 1231 (w), 1202 (s), 1179 (s), 1093 (w), 1081 (w), 1068 (w), 1035 (w), 1018 (s), 1002 (s), 928 (vs), 890 (w), 859 (w), 833 (w), 820 (w), 804 (m), 791 (w), 781 (w), 718 (vs), 700 (m), 629 (w), 571 (w), 510 (m), 485 (s), 452 (w), 407 (w). | | | |
| **MS** (ESI+): | m/z (%) = 321 (100, [M+H]⁺) | | | |
| **HRMS** (ESI+): | m/z für C₁₆H₂₀N₂OS₂ | | | |
| | ber.: | 320.1017 | | gef.: 320.1015 |
| **EA:** | ber.: | C:59.96 | H: 6.29 | N: 8.74 |
| | gef.: | C:59.61 | H: 6.30 | N: 8.78 |

### Darstellung von Benzyl-4-[(E)-cinnamyl]piperazin-1-carbodithioat (Schl32210):

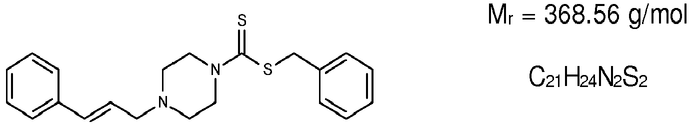

Gemäß **AAV7** wurden 1214 mg 1-[(*E*)-Cinnamyl]piperazin (6.0 mmol, 2.0 eq), 1592 mg Kaliumphosphat (6.0 mmol, 2.0 eq), 0.36 mL Kohlenstoffdisulfid (6.0 mmol, 2.0 eq) und 0.36 mL Benzylbromid (3.0 mmol, 1.0 eq) umgesetzt. Säulenchromatographie an Kieselgel (Cyclohexan/ EtOAc 5:1) ergab 991 mg von **Schl32210** (89.6%) in Form eines gelben Feststoffes.

| | | | | |
|---|---|---|---|---|
| **DC:** | R_{f} = 0.20 (Cyclohexan/ EtOAc 5:1). | | | |
| **Fp**.**:** | 69 °C | | | |
| **¹H-NMR:** | (CDCl₃, 400 MHz), δ [ppm] = 7.41-7.36 (m, 4H, C*H*_{(Aromat)}), 7.33-7.22 (*m*, 6H, C*H*_{(Aromat)}), 6.54 (*d*, ³J = 15.8 Hz, 1H, N-CH₂-CH=C*H*), 6.25 (*dt,* ³J = 13.5 Hz, 1H, N-CH₂-C*H*=CH), 4.58 (*s*, 2H, S-C*H₂*-C), 4.46-4.29 (*m,* 2H, CH₂-N-CH₂-CH*H*-N, CH₂-N-CH₂-CH*H*-N), 4.05-3.84 (*m,* 2H, CH₂-N-CH₂-CH*H*-N, CH₂-N-CH₂-CH*H*-N), 3.19 (*d*, ³J = 6.9 Hz, 2H, N-C*H*₂-CH=CH), 2.66-2.52 (m, 4H, CH₂-N-C*H₂*-CH₂-N, CH₂-N-C*H₂*-CH₂-N). | | | |
| **¹³C**-**NMR:** | (CDCl₃, 100 MHz), δ [ppm] = 196.6 (C=S), 136.7 (C_{q (Aromat)}), 135.9 (C_{q (Aromat)}), 133.9 (C-CH=CH-CH₂), 129.5 (C_{t (Aromat)}), 128.8 (C_{t (Aromat)}), 128.7 (C_{t (Aromat)}), 127.9 (C_{t (Aromat)}), 127.7 (C_{t (Aromat)}), 126.5 (C_{t (Aromat)}), 125.7 (C-CH=CH-CH₂), 60.5 (C-CH=CH-CH₂), 52.6 (N-CH₂-CH₂-N, N-CH₂-CH₂-N), 42.3 (S-CH₂-C). | | | |
| **IR (ATR):** | v (cm⁻¹) = 2890 (w), 2804 (w), 1458 (w), 1422 (m), 1365 (s), 1354 (w), 1329 (w), 1299 (w), 1284 (w), 1268 (m), 1245 (w), 1218 (vs), 1197 (m), 1146 (m), 1132 (m), 1105 (w), 1086 (w), 1068 (w), 1036 (m), 1015 (m), 995 (m), 969 (vs), 916 (w), 857 (w), 785 (m), 739 (s), 720 (s), 688 (vs), 646 (w), 616 (w), 570 (w), 560 (w), 550 (w), 489 (m), 419 (w), 403 (w). | | | |
| **MS** (ESI+): | m/z (%) = 369 (100, [M+H]⁺) | | | |
| **HRMS** (ESI+): | m/z für C₂₁H₂₄N₂S₂ | | | |
| | ber.: | 368.1381 | | gef.: 368.1389 |
| **EA:** | ber.: | C: 68.44 | H: 6.56 | N:7.60 |
| | gef.: | C:67.85 | H: 6.65 | N:7.12 |

### Darstellung von Benzylthiazolidin-3-carbodithioat (Schl32211):

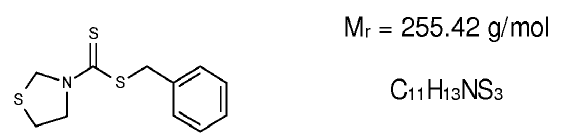

Gemäß **AAV8** wurden 0.47 mL Thiazolidin (6.0 mmol, 2.0 eq), 0.36 mL Kohlenstoffdisulfid (6.0 mmol, 2.0 eq) und 0.36 mL Benzylbromid (3.0 mmol, 1.0 eq) umgesetzt. Säulenchromatographie an Kieselgel (Cyclohexan/ EtOAc 7:1) ergab 705 mg von **Schl32211** (92.0%) in Form eines gelben Feststoffes.

| | | | | |
|---|---|---|---|---|
| **DC:** | R_{f} = 0.41 (Cyclohexan/ EtOAc 5:1). | | | |
| **Fp**.**:** | 61 °C | | | |
| **¹H-NMR:** | (CDCl₃, 400 MHz), δ [ppm] = 7.41-7.36 (*m*, 2H, C*H*_{(Benzyl)}), 7.34-7.23 (*m*, 3H, C*H*_{(Benzyl)}), 5.04 (*s*, 1H, S-CH*H*-N), 4.67 (*s*, 1H, S-CH*H*-N), 4.57 (s, 2H, S-C*H₂*-C), 4.31 (*t*, 3_{J} = 6.0 Hz, 1H, S-CH₂-CH*H*), 3.93 (*t*, 3_{J} = 6.0 Hz, 1H, S-CH₂-CH*H*), 3.16 (*t*, ³J = 6.2 Hz, 1H, S-CH*H*-CH₂), 3.07 (*t*, ³J = 6.1 Hz, 1H, S-CH*H*-CH₂). | | | |
| **¹³C**-**NMR:** | (CDCl₃, 100 MHz), δ [ppm] = 193.6 (C=S), 136.0 (C-CH-CH-CH), 129.4 (C-CH-CH-CH, C-CH-CH-CH), 128.8 (C-CH-CH-CH, C-CH-CH-CH), 127.7 (C-CH-CH-CH), 56.5 (S-CH₂-N), 52.7 (N-CH₂-CH₂-S), 42.0 (S-CH₂-C), 31.3 (N-CH₂-CH₂-S). | | | |
| **IR (ATR):** | v (cm⁻¹) = 3083 (w), 3056 (w), 3027 (w), 1597 (w), 1580 (w), 1493 (w), 1444 (w), 1344 (vs), 1330 (w), 1301 (m), 1255 (s), 1243 (m), 1197 (w), 1180 (m), 1142 (s), 1068 (w), 1014 (s), 1000 (s), 984 (s), 965 (m), 941 (w), 916 (w), 867 (m), 842 (m), 804 (w), 775 (m), 717 (vs), 692 (vs), 668 (w), 620 (w), 572 (w), 551 (w), 499 (m), 480 (s), 442 (m), 431 (m). | | | |
| **MS** (ESI+): | m/z (%) = 256 (100, [M+H]⁺) | | | |
| **HRMS** (ESI+): | m/z für C₁₁H₁₃NS₃ | | | |
| | ber.: | 255.0210 | | gef.: 255.0202 |
| **EA:** | ber.: | C:51.73 | H:5.13 | N: 5.48 |
| | gef.: | C:51.48 | H:5.10 | N: 5.54 |

### Darstellung von Benzylthiomorpholin-4-carbodithioat (Schl32212):

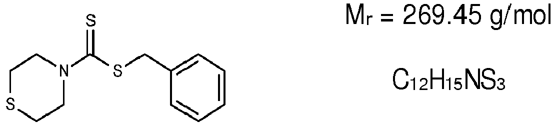

Gemäß **AAV5** wurden 0.50 mL Benzylbromid (3.0 mmol, 1.0 eq), 0.36 mL Kohlenstoffdisulfid (6.0 mmol, 2.0 eq) und 0.60 mL Thiomorpholin (6.0 mmol, 2.0 eq) umgesetzt. Säulenchromatographie an Kieselgel (Cyclohexan/ EtOAc 9:1) ergab 698 mg von **Schl32212** (86.4%) in Form eines gelben Feststoffes.

| | | | | |
|---|---|---|---|---|
| **DC:** | R_{f} = 0.45 (Cyclohexan/ EtOAc 5:1 ). | | | |
| **Fp**.**:** | 103 °C | | | |
| **¹H-NMR:** | (CDCl₃, 400 MHz), δ [ppm] = 7.40-7.23 (*m*, 5H, CH₂-C-CH-CH-C*H*, CH₂-C-C*H*-CH, CH₂-C-C*H*-C*H*), 4.70-4.56 (*m*, 2H, S-CH₂-CH*H*-N, S-CH₂-CH*H*-N), 4.55 (s, 2H, S-C*H₂*-C), 4.35-4.18 (*m*, 2H, S-CH₂-CH*H*-N, S-CH₂-CH*H*-N), 2.80-2.66 (m, 4H, S-C*H₂*-CH₂-N, S-CH₂-C*H₂*-N). | | | |
| **¹³C**-**NMR:** | (CDCl₃, 100 MHz), δ [ppm] = 194.0 (C=S), 135.7 (C-CH-CH), 129.5 (C-CH-CH), 128.7 (C-CH-CH), 127.8 (C-CH-CH-CH), 52.6 (N-CH₂, N-CH₂), 42.4 (S-CH₂-C), 27.4 (S-CH₂, S-CH₂). | | | |
| **IR (ATR):** | v (cm⁻¹) = 1895 (w), 1492 (w), 1451 (w), 1435 (w), 1410 (s), 1352 (w), 1277 (m), 1244 (m), 1220 (m), 1187 (m), 1135 (m), 1068 (w), 1026 (w), 992 (m), 983 (w), 965 (w), 928 (vs), 913 (m), 856 (m), 820 (w), 805 (w), 767 (s), 697 (vs), 596 (w), 567 (m), 476 (s), 452 (w), 437 (w), 400 (m). | | | |
| **MS** (ESI+): | m/z (%) = 270 (100, [M+H]⁺) | | | |
| **HRMS** (ESI+): | m/z für C₁₂H₁₅NS₃ | | | |
| | ber.: | 269.0367 | | gef.: 269.0355 |
| **EA:** | ber.: | C:53.49 | H: 5.61 | N: 5.20 |
| | gef.: | C: 53.16 | H: 5.58 | N: 5.20 |

### Darstellung von Benzyl-6,7-dihydro-4H-thieno[3,2-c]pyridin-5-carbodithioat (Schl32215):

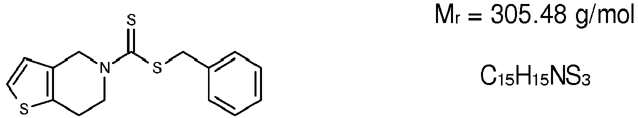

Gemäß **AAV8** wurden 835 mg 4,5,6,7-Tetrahydrothieno[3,2-*c*]pyridinhydrochlorid (6.0 mmol, 2.0 eq), 0.36 mL Kohlenstoffdisulfid (6.0 mmol, 2.0 eq) und 0.36 mL Benzylbromid (3.0 mmol, 1.0 eq) umgesetzt. Säulenchromatographie an Kieselgel (Cyclohexan/ EtOAc 10:1) ergab 378 mg von **Schl32215** (41.3%) in Form eines braunen Feststoffes.

| | | | | |
|---|---|---|---|---|
| **DC:** | R_{f} = 0.41 (Cyclohexan/ EtOAc 7:1). | | | |
| **Fp**.**:** | 72 °C | | | |
| **¹H-NMR:** | (CDCl₃, 400 MHz), δ [ppm] = 7.43-7.36 (*m*, 2H, C*H*_{(Benzyl)}), 7.34-7.23 (*m*, 3H, C*H*_{(Benzyl)}), 7.18-7.11 (*m*, 1H, S-C*H*=CH-C), 6.84-6.72 (*m*, 1H, S-CH=C*H*-C), 5.40-5.28 (*m*, 1H, C-CH*H*-N), 5.07-4.88 (*m*, 1H, C-CH*H*-N), 4.72-4.62 (*m*, 1H, C-CH₂-CHH-N), 4.61 (*s*, 2H, S-C*H₂*-C), 4.26-4.13 (*m*, 1H, C-CH₂-CHH-N), 3.05-2.93 (*m*, 2H, C-C*H₂*-CH₂-N). | | | |
| **¹³C**-**NMR:** | (CDCl₃, 100 MHz), δ [ppm] = 194.2 (C=S), 135.9 (C-CH-CH-CH), 134.9,134.4 (S-C-C), 129.5 (C-CH-CH-CH, C-CH-CH-CH), 128.8 (C-CH-CH-CH, C-CH-CH-CH), 127.7 (C-CH-CH-CH), 124.5 (S-CH=CH), 124.2 (S-CH=CH), 52.7 (C-CH₂-CH₂-N), 44.3 (C-CH₂-S), 40.9 (S-CH₂-C), 25.5 (N-CH₂-CH₂-C). | | | |
| **IR (ATR):** | v (cm⁻¹) = 1923 (w), 2880 (w), 1650 (w), 1492 (w), 1451 (m), 1443 (w), 1420 (s), 1404 (s), 1344 (w), 1327 (w), 1255 (s), 1219 (w), 1207 (m), 1177 (m), 1153 (w), 1144 (w), 1082 (w), 1044 (w), 1015 (m), 990 (s), 933 (m), 910 (m), 896 (w), 846 (m), 821 (w), 764 (w), 703 (vs), 693 (vs), 654 (m), 593 (w), 565 (w), 551 (w), 531 (w), 497 (m), 469 (m), 458 (m). | | | |
| **MS** (ESI+): | m/z (%) = 306 (100, [M+H]⁺) | | | |
| **HRMS** (ESI+): | m/z für C₁₅H₁₅NS₃ | | | |
| | ber.: | 305.0367 | | gef.: 305.0361 |
| **EA:** | ber.: | C: 58.98 | H: 4.95 | N: 4.59 |
| | gef.: | C:59.11 | H: 5.08 | N: 4.77 |

### Darstellung von Benzyl-4-[[4-(4-Nitrophenyl)sulfanylphenyl]carbamoyl]piperidin-1-carbodithioat (Schl32222):

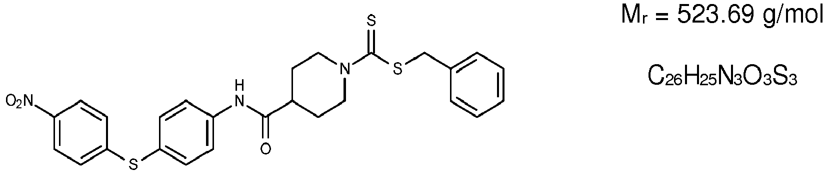

493 mg 4-(Nitrophenylthio)anilin (2.0 mmol, 1.0 eq), 591 mg 1-Benzylsulfanylcarbothioyl-piperidin-4-carbonsäure **(Schl32176,** 2.0 mmol, 1.0 eq), und 0.55 mL NEt₃ (4.0 mmol, 2.0 eq) wurden bei 0 °C in 50 mL Dichlormethan gelöst, mit 910 mg HBTU (2.4 mmol, 1.2 eq) versetzt und 12 Stunden bei Raumtemperatur gerührt. Die organische Phase wird 3x mit 1 M HCl-Lösung, 3x mit 1 M NaOH-Lösung. und ges. NaCl-Lösung. gewaschen, über MgSO₄ getrocknet und das Lösungsmittel am Rotavapor entfernt. Säulenchromatographie an Kieselgel (Cyclohexan/ EtOAc 2:1) und anschließende Umkristallisation aus Cyclohexan/ EtOAc 2:1 ergab 402 mg von **Schl32222** (38.4%) in Form eines blassgelben Feststoffes.

| | | | | |
|---|---|---|---|---|
| **DC:** | R_{f} = 0.22 (Cyclohexan/ EtOAc 2:1). | | | |
| **Fp**.**:** | 193 °C | | | |
| **¹H-NMR:** | (DMSO-D₆, 400 MHz), δ [ppm] = 10.27 (*sbr,* 1H, N*H*), 8.08 (*d*, 3_{J}=8.9 Hz, 2H, (NO₂)-C-C*H*-CH, (NO₂)-C-C*H*-CH), 7.75 (*d*, 3_{J}=8.5 Hz, 2H, NH-C-C*H*-CH, NH-C-C*H*-CH), 7.51 (*d*, 3_{J}=8.7 Hz, 2H, (NO₂)-C-CH-C*H*, (NO₂)-C-CH-C*H*), 7.37 (*d*, 3_{J}=8.3 Hz, 2H, NH-C-CH-C*H*, NH-C-CH-C*H*), 7.33-7.16 (m, 5H, C*H*_{(Benzyl)}), 5.35-5.19 (*m*, 1H, N-CH*H*-CH₂-CH), 4.51 (*s*, 2H, S-C*H₂*-C), 4.50-4.42 (*m*, 1H, N-CH*H*-CH₂-CH), 3.46-3.32 (*m*, 2H, N-CH*H*-CH₂-CH, N-CH*H*-CH₂-CH), 2.81-2.70 (*m*, 1H, CH₂-C*H*-C(=O)), 1.98-1.86 (*m*, 2H, N-CH₂-CH*H*-CH, N-CH₂-CH*H-*CH), 1.70-1.55 (*m*, 2H, N-CH₂-CH*H-CH,* N-CH₂-CH*H*-CH). | | | |
| **¹³C**-**NMR:** | (DMSO-D₆, 100 MHz), δ [ppm] = 194.7 (C=S), 173.4 (C=O), 149.3 (C_{q (Phenyl)}), 141.5 (C_{q (Phenyl)}), 136.8 (C_{q (Phenyl)}), 136.5 (C_{t (Phenyl)}), 129.8 (C_{t (Phenyl)}), 129.0 (C_{t (Phenyl)}), 127.9 (C_{t (Phenyl)}), 126.5 (C_{t (Phenyl)}), 124.8 (C_{t (Phenyl)}), 122.8 (C_{t (Phenyl)}), 121.1 (C_{t (Phenyl)}), 120.0 (C_{t (Phenyl)}), 118.3 (C_{t (Phenyl)}), 118.2 (C_{t (Phenyl)}), 71.9 (CH-C(=O)), 57.3 (N-CH₂, N-CH₂), 42.5 (S-CH₂-C), 30.1 (CH-CH₂-CH₂-N, CH-CH₂-CH₂-N). | | | |
| **IR (ATR):** | v (cm⁻¹) = 3295 (w), 3031 (w), 2946 (w), 1660 (w), 1596 (w), 1581 (w), 1494 (s), 1468 (w), 1431 (w), 1362 (w), 1333 (vs), 1261 (w), 1214 (w), 1175 (s), 1084 (w), 1023 (w), 1002 (w), 957 (w), 945 (w), 920 (w), 851 (w), 827 (w), 776 (w), 738 (w), 713 (w), 693 (m), 681 (w), 570 (w), 542 (w), 524 (w), 508 (w), 487 (w), 476 (w), 462 (w), 410 (w). | | | |
| **MS** (ESI-): | m/z (%) = 522 (100, [M-H]⁻) | | | |
| **HRMS** (ESI+): | m/z für C₂₆H₂₅N₃O₃S₃ | | | |
| | ber.: | 523.1058 | | gef.: 523.1046 |
| **EA:** | ber.: | C:59.63 | H: 4.81 | N:8.02 |
| | gef.: | C:59.37 | H: 4.91 | N:8.09 |

### Darstellung von (4-Nitrophenyl)methyl-4-(furan-2-carbonyl)piperazin-1-carbodithioat (Schl32277):

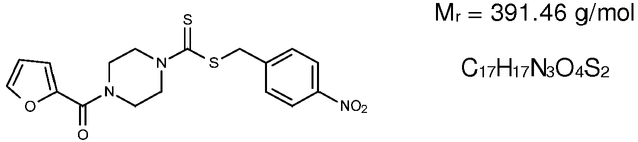

Gemäß **AAV7** wurden 360 mg 1-(2-Furoyl)piperazin (2.0 mmol, 1.0 eq), 637 mg Kaliumphosphat (3.0 mmol, 1.5 eq), 0.22 mL Kohlenstoffdisulfid (6.0 mmol, 3.0 eq) und 432 mg 1-(Brommethyl)-4-nitrobenzen (2.0 mmol, 1.0 eq) umgesetzt. Säulenchromatographie an Kieselgel (Cyclohexan/ EtOAc 1:1) ergab 707 mg von **Schl32277** (90.3%) in Form eines gelben Feststoffes.

| | | | | |
|---|---|---|---|---|
| **DC:** | R_{f} = 0.26 (Cyclohexan/ EtOAc 1:1). | | | |
| **Fp**.**:** | 178 °C | | | |
| **¹H-NMR:** | (CDCl₃, 400 MHz), δ [ppm] = 8.13 (*d*, ³J = 8.7 Hz, 2H, C-CH-C*H*-C-NO₂, C-CH-C*H*-C-NO₂), 7.55 (*d*, ³J = 8.7 Hz, 2H, C-C*H*-CH-C-NO₂, C-C*H*-CH-C-NO₂), 7.49-7.45 (*m*, 1H, C-O-C*H*), 7.09-7.01 (*m*, 1H, C(=O)-C-C*H*), 6.50-6.43 (*m*, 1H, C-O-CH-C*H*), 4.68 (*s*, 2H, S-C*H₂*-C), 4.48-4.29 (*m*, 4H, N-C*H₂*-CH₂-N-C(=O), N-C*H₂*CH₂-C(=O)), 4.00-3.83 (*m*, 4H, N-CH₂-C*H*₂C(=O), N-CH₂-C*H₂*-C(=O)). | | | |
| **¹³C**-**NMR:** | (CDCl₃, 100 MHz), δ [ppm] = 196.0 (C=S), 163.5 (C=O), 147.2 (C(NO₂)), 144.5 (C(=O)-C), 144.3 (C-O-CH), 140.6 (CH₂-C-CH-CH), 130.2 (C(NO₂)-CH-CH, C(NO₂)-CH-CH), 123.8 (C-CH-CH, C-CH-CH), 117.7 (C(=O)-C-CH), 111.8 (C(=O)-C-CH-CH), 60.4 (N-CH₂-CH₂-N-(C=O), N-CH₂-CH₂-N-(=O)), 57.7 (N-CH₂-CH₂-N-C(=O), N-CH₂-CH₂-N-C(=O)), 40.5 (S-CH₂-C). | | | |
| **IR (ATR):** | v (cm⁻¹) = 3074 (w), 2986 (w), 1613 (s), 1577 (w), 1513 (w), 1488 (w), 1454 (s), 1444 (m), 1421 (w), 1383 (s), 1343 (m), 1292 (s), 1238 (w), 1185 (s), 1171 (w), 1159 (m), 1143 (m), 1084 (s), 1070 (s), 1046 (s), 1028 (w), 994 (s), 962 (vs), 925 (w), 886 (m), 876 (s), 855 (m), 837 (w), 754 (vs), 717 (vs), 618 (w), 592 (m), 471 (m), 431 (w), 418 (w), 403 (w). | | | |
| **MS** (ESI+): | m/z (%) = 392 (10, [M+H]⁺), 409 (100, [M+NH₄]⁺) | | | |
| **HRMS** (ESI+): | m/z für C₁₇H₁₇N₃O₄S₂ | | | |
| | ber.: | 391.0660 | | gef.: 391.0667 |
| **EA:** | ber.: | C:52.16 | H: 4.38 | N:10.73 |
| | gef.: | C:52.26 | H:4.43 | N:10.67 |

### Darstellung von (4-Methylsulfonylphenyl)methyl-4-(furan-2-carbonyl)piperazin-1-carbodithioat (Schl32278):

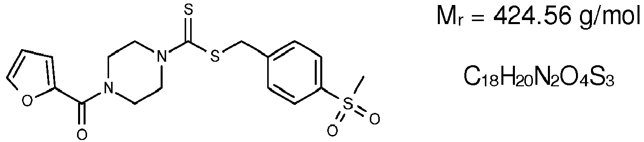

Gemäß **AAV7** wurden 360 mg 1-(2-Furoyl)piperazin (2.0 mmol, 1.0 eq), 637 mg Kaliumphosphat (3.0 mmol, 1.5 eq), 0.22 mL Kohlenstoffdisulfid (6.0 mmol, 3.0 eq) und 498 mg 1-(Brommethyl)-4-methylsulfonylbenzen (2.0 mmol, 1.0 eq) umgesetzt. Säulenchromatographie an Kieselgel (Cyclohexan/ EtOAc 1:2) ergab 541 mg von **Schl32278** (63.7%) in Form eines blassgelben Feststoffes.

| | | | | |
|---|---|---|---|---|
| **DC:** | R_{f} = 0.22 (Cyclohexan/ EtOAc 1:2). | | | |
| **Fp**.**:** | 170 °C | | | |
| **¹H-NMR:** | (CDCl₃, 400 MHz), δ [ppm] = 7.86 (*d*, ³J = 8.2 Hz, 2H, CH₂-C-CH-C*H*-C-SO₂Me, CH₂-C-CH-C*H*-C-SO₂Me), 7.58 (*d*, ³J = 8.2 Hz, 2H, CH₂-C-C*H*-CH-C-SO₂Me, CH₂-C-C*H*-CH-C-SO₂Me), 7.50-7.47 (*m*, 1H, C-O-C*H*), 7.07 (*d*, 3_{J} = 3.4 Hz, 1H, C(=O)-C-C*H*), 6.51-6.48 (*m*, 1H, C-O-CH-C*H*), 4.67 (s, 2H, S-C*H₂*-C), 4.51-4.27 (*m*, 4H, N-C*H₂*-CH₂-N-C(=O), N-C*H₂*CH₂-C(=O)), 4.11-3.87 (*m*, 4H N-CH₂-C*H₂*-C(=O), N-CH₂-C*H*₂-C(=O)), 3.02 (s, 3H, SO₂-C*H₃*). | | | |
| **¹³C**-**NMR:** | (CDCl₃, 100 MHz), δ [ppm] = 192.6 (C=S), 162.8 (C=O), 144.6 (C(=O)-C), 144.3 (C-O-CH), 143.2 (C-SO₂Me), 139.6 (CH₂-C-CH-CH), 130.3 (C-SO₂Me-CH-CH, C-SO₂Me-CH-CH), 127.7 (C-CH-CH, C-CH-CH), 117.7 (C(=O)-C-CH), 111.7 (C(=O)-C-CH-CH), 56.9 (N-CH₂-CH₂-N-(C=O), N-CH₂-CH₂-N-(=O)), 53.4 (N-CH₂-CH₂-N-C(=O), N-CH₂-CH₂-N-C(=O)), 44.6 (SO₂-CH₃), 40.8 (S-CH₂-C). | | | |
| **IR (ATR):** | v (cm⁻¹) = 3142 (w), 3120 (w), 3031 (w), 1631 (s), 1595 (w), 1581 (w), 1486 (m), 1466 (w), 1439 (w), 1406 (s), 1380 (s), 1319 (w), 1267 (vs), 1254 (s), 1229 (m), 1214 (s), 1183 (m), 1155 (m), 1139 (vs), 1113 (w), 1072 (m), 1016 (s), 997 (s), 966 (s), 951 (m), 928 (s), 873 (s), 855 (m), 757 (vs), 624 (w), 595 (w), 539 (vs), 518 (vs), 471 (s), 458 (m), 416 (w). | | | |
| **MS** (ESI+): | m/z (%) = 425 (10, [M+H]⁺), 442 (100, [M+NH₄]⁺) | | | |
| **HRMS** (ESI+): | m/z für C₁₈H₂₀N₂O₄S₃ | | | |
| | ber.: | 424.0585 | | gef.: 424.0570 |
| **EA:** | ber.: | C: 50.92 | H: 4.75 | N: 6.60 |
| | gef.: | C: 50.70 | H: 4.81 | N: 6.57 |

### Darstellung von (4-Cyanophenyl)methyl-4-(furan-2-carbonyl)piperazin-1-carbodithioat (Schl32279):

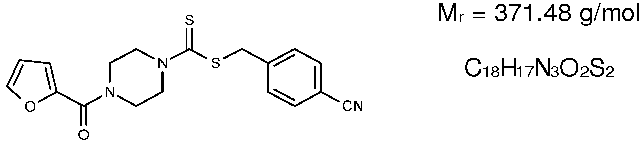

Gemäß **AAV7** wurden 360 mg 1-(2-Furoyl)piperazin (2.0 mmol, 1.0 eq), 637 mg Kaliumphosphat (3.0 mmol, 1.5 eq), 0.22 mL Kohlenstoffdisulfid (6.0 mmol, 3.0 eq) und 392 mg 4-(Brommethyl)benzonitril (2.0 mmol, 1.0 eq) umgesetzt. Säulenchromatographie an Kieselgel (Cyclohexan/ EtOAc 1:1) ergab 720 mg von **Schl32279** (97.0%) in Form eines blassgelben Feststoffes.

| | | | | |
|---|---|---|---|---|
| **DC:** | R_{f} = 0.24 (Cyclohexan/ EtOAc 1:1). | | | |
| **Fp**.**:** | 115 °C | | | |
| **¹H-NMR:** | (CDCl₃, 400 MHz), δ [ppm] = 7.57 (d, ³J = 8.2 Hz, 2H, C-CH-C*H*-C-CN, C-CH-C*H*-C-CN), 7.50 (*d*, ³J = 8.2 Hz, 2H, C-C*H*-CH-C-CN, C-C*H*-CH-C-CN), 7.49-7.44 (*m*, 1H, C-O-C*H*), 7.08 (*d*, 3_{J} = 3.4 Hz, 1H, C(=O)-C-C*H*), 6.51-6.47 (m, 1H, C-O-CH-C*H*), 4.64 (s, 2H, S-C*H₂*-C), 4.49-4.24 (*m*, 4H, N-C*H₂*-CH₂-N-C(=O), N-C*H₂*CH₂-C(=O)), 4.02-3.85 (*m*, 4H N-CH₂-C*H₂*-C(=O), N-CH₂-C*H₂*-C(=O)). | | | |
| **¹³C**-**NMR:** | (CDCl₃, 100 MHz), δ [ppm] = 196.2 (C=S), 164.9 (C=O), 144.4 (C(=O)-C), 142.3 (C-O-CH), 142.2 (C-CH-CH-C-CN), 132.4 (C-CH-CH-C-CN, C-CH-CH-C-CN), 130.1 (C-CH-CH-C-CN, C-CH-CH-C-CN), 118.8 (C-CN), 117.7 (C(=O)-C-CH), 111.8 (C(=O)-C-CH-CH), 111.4 (C-CN), 62.2 (N-CH₂-CH₂-N-(C=O), N-CH₂-CH₂-N-(=O)), 60.5 (N-CH₂-CH₂-N-C(=O), N-CH₂-CH₂-N-C(=O)), 40.9 (S-CH₂-C). | | | |
| **IR (ATR):** | v (cm⁻¹) = 3064 (w), 2998 (w), 1603 (vs), 1569 (w), 1480 (m), 1450 (m), 1424 (vs), 1386 (w), 1283 (s), 1249 (w), 1223 (s), 1182 (m), 1163 (m), 1111 (w), 1075 (w), 1024 (w), 1013 (s), 996 (w), 936 (m), 882 (w), 866 (m), 846 (w), 838 (w), 820 (w), 761 (vs), 738 (m), 671 (w), 632 (w), 612 (w), 598 (w), 551 (s), 483 (w), 464 (w), 409 (w). | | | |
| **MS** (ESI+): | m/z (%) = 371 (10, [M+H]⁺), 389 (100, [M+NH₄]⁺) | | | |
| **HRMS** (ESI+): | m/z für C₁₈H₁₇N₃O₂S₂ | | | |
| | ber.: | 371.0762 | | gef.: 371.0758 |
| **EA:** | ber.: | C:58.20 | H: 4.61 | N:11.31 |
| | gef.: | C:58.21 | H:4.71 | N:11.21 |

### Darstellung von (2-Hydroxy-5-nitrophenyl)methyl-4-(furan-2-carbonyl)piperazin-1-carbodithioat (Schl32280):

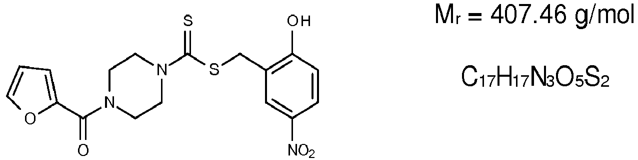

Gemäß **AAV7** wurden 360 mg 1-(2-Furoyl)piperazin (2.0 mmol, 1.0 eq), 637 mg Kaliumphosphat (3.0 mmol, 1.5 eq), 0.22 mL Kohlenstoffdisulfid (6.0 mmol, 3.0 eq) und 464 mg 2-Hydroxy-5-nitrobenzylbromid (2.0 mmol, 1.0 eq) umgesetzt. Säulenchromatographie an Kieselgel (Cyclohexan/ EtOAc 1:3) ergab 485 mg von **Schl32280** (59.5%) in Form eines blassgelben Feststoffes.

| | | | | |
|---|---|---|---|---|
| **DC:** | R_{f} = 0.27 (Cyclohexan/ EtOAc 1:3). | | | |
| **Fp**.**:** | 200 °C | | | |
| **¹H-NMR:** | (DMSO-D₆, 400 MHz), δ [ppm] = 8.26 (s, 1H, C-C*H*-C(NO₂)), 8.02 (*d*, 3_{J} = 9.0 Hz, 1H, C(NO₂)-C*H*-CH-C-OH), 7.83-7.81 *(m,* 1H, C-O-C*H*), 7.05-7.00 (*m*, 1H, C(=O)-C-C*H*), 6.96 (d, ³J = 8.9 Hz, 1H, C(NO₂)-CH-C*H*-C-OH), 6.62-6.58 (*m*, 1H, C-O-CH-C*H*), 4.53 (s, 2H, S-C*H₂*-C), 4.36-4.21 (*m*, 2H, N-C*H₂*CH₂-N-C(=O)), 4.09-3.92 (*m*, 2H, N-C*H₂*CH₂-C(=O)), 3.88-3.71 (*m*, 4H N-CH₂-C*H₂*-C(=O), N-CH₂-C*H₂*-C(=O)). | | | |
| **¹³C**-**NMR:** | (DMSO-D₆, 100 MHz), δ [ppm] = 195.7 (C=S), 162.8 (C=O), 158.9 (C-OH), 147.2 (C-NO₂), 141.9 (C(=O)-C), 139.6 (C-O-CH), 126.9 (C(NO₂)-CH-CH-C), 125.8 (C-CH-C(NO₂)), 124.4 (S-CH₂-C), 116.6 (C(NO₂)-CH-CH-C), 115.9 (C(=O)-C-CH), 111.9 (C(=O)-C-CH-CH), 61.4 (N-CH₂-CH₂-N-(C=O), N-CH₂-CH₂-N-(=O)), 60.0 (N-CH₂-CH₂-N-C(=O), N-CH₂-CH₂-N-C(=O)), 35.5 (S-CH₂-C). | | | |
| **IR (ATR):** | v (cm⁻¹) = 2975 (w), 2913 (w), 1586 (m), 1521 (m), 1479 (w), 1464 (s), 1424 (w), 1388 (vs), 1336 (w), 1296 (vs), 1270 (m), 1234 (w), 1212 (vs), 1190 (m), 1158 (m), 1127 (w), 1084 (m), 1046 (w), 1028 (s), 1011 (w), 954 (w), 928 (w), 884 (m), 858 (m), 815 (w), 759 (vs), 746 (s), 640 (w), 624 (w), 613 (w), 591 (w), 550 (w), 532 (w), 481 (w), 432 (w), 416 (w). | | | |
| **MS** (ESI+): | m/z (%) = 407 (10, [M+H]⁺), 426 (100, [M+NH₄]⁺) | | | |
| **HRMS** (ESI+): | m/z für C₁₇H₁₇N₃O₅S₂ | | | |
| | ber.: | 407.0610 | | gef.: 407.0586 |
| **EA:** | ber.: | C:50.11 | H:4.21 | N:10.31 |
| | gef.: | C:49.81 | H:4.38 | N:10.01 |

### Darstellung von Cyclohexylmethyl-4-(furan-2-carbonyl)piperazin-1-carbodithioat (Schl32281):

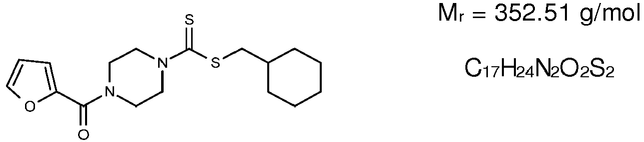

Gemäß **AAV7** wurden 360 mg 1-(2-Furoyl)piperazin (2.0 mmol, 1.0 eq), 637 mg Kaliumphosphat (3.0 mmol, 1.5 eq), 0.22 mL Kohlenstoffdisulfid (6.0 mmol, 3.0 eq) und 0.28 mL Brommethylcyclohexan (2.0 mmol, 1.0 eq) umgesetzt. Säulenchromatographie an Kieselgel (Cyclohexan/ EtOAc 1:1) ergab 519 mg von **Schl32281** (73.7%) in Form eines farblosen Feststoffes.

| | | | | |
|---|---|---|---|---|
| **DC:** | R_{f} = 0.48 (Cyclohexan/ EtOAc 1:1). | | | |
| **Fp**.**:** | 111 °C | | | |
| **¹H-NMR:** | (CDCl₃, 400 MHz), δ [ppm] = 7.51-7.49 (*m*, 1H, C-O-C*H*), 7.07 (*d*, ³J = 3.4 Hz, 1H, C(=O)-C-C*H*), 6.51-6.47 (*m*, 1H, C-O-CH-C*H*), 4.50-4.11 (*m*, 4H, N-C*H₂*-CH₂-N-C(=O), N-C*H₂*CH₂-C(=O)), 4.00-3.79 (*m*, 5H, N-CH₂-C*H₂*-C(=O), N-CH₂-C*H₂*-C(=O), S-CH₂-C*H*), 3.22 (*d*, ³J = 6.6 Hz, 2H, S-C*H₂*-CH), 1.91-1.79 (*m*, 2H, C*H*_{(Cyclohexyl)}), 1.75-1.54 (*m*, 4H, C*H*_{(Cyclohexyl)}), 1.30-0.97 (*m*, 4H, C*H*_{(Cyclohexyl)}). | | | |
| **¹³C**-**NMR:** | (CDCl₃, 100 MHz), δ [ppm] = 198.8 (C=S), 159.2 (C=O), 147.6 (C(=O)-C), 144.3 (C-O-CH), 117.5 (C(=O)-C-CH), 111.7 (C(=O)-C-CH-CH), 60.4 (N-CH₂-CH₂-N-(C=O), N-CH₂-CH₂-N-(=O)), 57.7 (N-CH₂-CH₂-N-C(=O), N-CH₂-CH₂-N-C(=O)), 44.5 (S-CH₂-CH), 37.3 (S-CH₂-CH), 32.9 (CH_{(Cyclohexyl)}), 26.4 (CH_{(Cyclohexyl)}), 26.1 (CH_{(Cyclohexyl)}). | | | |
| **IR (ATR):** | v (cm⁻¹) = 3118 (w), 2922 (m), 2851 (w), 1613 (s), 1565 (m), 1481 (s), 1450 (w), 1408 (vs), 1363 (w), 1351 (w), 1280 (s), 1269 (m), 1248 (w), 1211 (s), 1186 (m), 1163 (m), 1153 (m), 1090 (w), 1034 (w), 1016 (m), 998 (vs), 952 (m), 934 (w), 895 (w), 885 (w), 863 (w), 837 (w), 821 (w), 793 (m), 755 (vs), 627 (w), 596 (w), 552 (w), 507 (w), 469 (m), 414 (w). | | | |
| **MS** (ESI+): | m/z (%) = 353 (25, [M+H]⁺), 370 (100, [M+NH₄]⁺) | | | |
| **HRMS** (ESI+): | m/z für C₁₇H₂₄N₂O₂S₂ | | | |
| | ber.: | 352.1279 | | gef.: 352.1267 |
| **EA:** | ber.: | C: 57.92 | H: 6.86 | N: 7.95 |
| | gef.: | C: 58.01 | H: 6.89 | N: 7.99 |

### Darstellung von (7-Methoxy-2-oxochromen-4-yl)methyl-4-(furan-2-carbonyl)piperazin-1-carbodithioat (Schl32282):

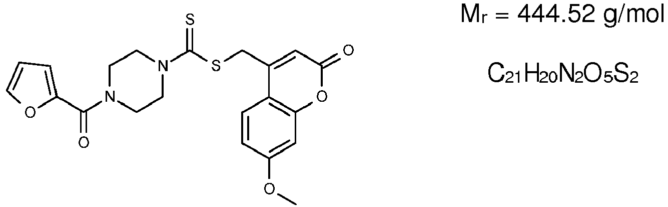

Gemäß **AAV7** wurden 360 mg 1-(2-Furoyl)piperazin (2.0 mmol, 1.0 eq), 637 mg Kaliumphosphat (3.0 mmol, 1.5 eq), 0.22 mL Kohlenstoffdisulfid (6.0 mmol, 3.0 eq) und 538 mg 4-(Brommethyl)-7-methoxychromen-2-on (2.0 mmol, 1.0 eq) umgesetzt. Säulenchromatographie an Kieselgel (Cyclohexan/ EtOAc 1:1) ergab 595 mg von **Schl32282** (73.7%) in Form eines gelben Feststoffes.

| | | | | |
|---|---|---|---|---|
| **DC:** | R_{f} = 0.15 (Cyclohexan/ EtOAc 1:1). | | | |
| **Fp**.**:** | 153 °C | | | |
| **¹H-NMR:** | (CDCl₃, 400 MHz), δ [ppm] = 7.59 (*d*, ³J = 8.7 Hz, 1H, C-C*H*-CH-C-O-CH₃), 7.51-7.46 (*m*, 1H, C-O-C*H*), 7.08 (d, ³J = 3.4 Hz, 1H, C(=O)-C-C*H*), 6.86 (*dd*, ³J = 8.7 Hz, ²J = 2.5 Hz, 1H, C-CH-C*H*-C-O-CH₃), 6.82-6.79 (*m*, 1H, C-C*H*-C-O-CH₃), 6.51-6.47 (*m*, 1H, C-O-CH-C*H*), 6.39 (*s*, 1H, C-C*H*-C=O), 4.71 (s, 2H, S-C*H₂*-C), 4.49-4.33 (*m*, 4H, N-C*H₂*-CH₂-N-C(=O), N-C*H₂*CH₂-C(=O)), 4.10-3.89 (*m*, 4H N-CH₂-C*H₂*-C(=O), N-CH₂-C*H₂*-C(=O)), 3.85 (s, 3H, O-C*H₃*). | | | |
| **¹³C**-**NMR:** | (CDCl₃, 100 MHz), δ [ppm] = 195.0 (C=S), 163.0 (C(=O)-O), 161.0 (C-OMe), 159.2 (S-CH₂-C), 155.7 (C(=O)-O-C), 147.5 (C(=O)-C), 144.4 (C-O-CH), 125.6 (C-CH-CH-C-OMe), 117.8 (C(=O)-C-CH), 112.9 (C-CH-C(=O)), 112.6 (C-CH-CH-C-OMe), 111.8 (C(=O)-C-CH-CH), 101.2 (C-CH-C-OMe), 60.1 (N-CH₂-CH₂-N-(C=O), N-CH₂-CH₂-N-(=O)), 58.2 (N-CH₂-CH₂-N-C(=O), N-CH₂-CH₂-N-C(=O)), 55.9 (O-CH₃), 37.5 (S-CH₂-C). | | | |
| **IR (ATR):** | v (cm⁻¹) = 3142 (w), 3068 (w), 2914 (w), 1613 (vs), 1573 (w), 1511 (w), 1423 (s), 1390 (m), 1289 (w), 1263 (s), 1212 (vs), 1180 (w), 1164 (m), 1154 (m), 1136 (m), 989 (s), 951 (vs), 927 (w), 848 (w), 821 (w), 797 (s), 763 (m), 736 (s), 715 (m), 630 (w), 615 (w), 597 (m), 574 (w), 481 (m). | | | |
| **MS** (ESI+): | m/z (%) = 445 (15, [M+H]⁺), 462 (100, [M+NH₄]⁺) | | | |
| **HRMS** (ESI+): | m/z für C₂₁H₂₀N₂O₅S₂ | | | |
| | ber.: | 444.0814 | | gef.: 444.0808 |
| **EA:** | ber.: | C: 56.74 | H: 4.53 | N: 6.30 |
| | gef.: | C: 57.11 | H: 4.54 | N: 6.40 |

### Darstellung von 2-(1H-Indol-3-yl)ethyl-4-(furan-2-carbonyl)piperazin-1-carbodithioat (Schl32283):

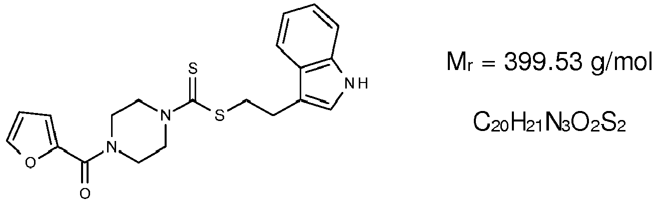

Gemäß **AAV7** wurden 360 mg 1-(2-Furoyl)piperazin (2.0 mmol, 1.0 eq), 637 mg Kaliumphosphat (3.0 mmol, 1.5 eq), 0.22 mL Kohlenstoffdisulfid (6.0 mmol, 3.0 eq) und 448 mg 3-(2-Bromethyl)indol (2.0 mmol, 1.0 eq) umgesetzt. Säulenchromatographie an Kieselgel (Cyclohexan/ EtOAc 1:1) ergab 653 mg von **Schl32283** (81.7%) in Form eines blassgelben Feststoffes.

| | | | | |
|---|---|---|---|---|
| **DC:** | R_{f} = 0.27 (Cyclohexan/ EtOAc 1:1) | | | |
| **Fp**.**:** | 146 °C | | | |
| **¹H-NMR:** | (CDCl₃, 400 MHz), δ [ppm] = 8.18 (*sbr,* 1H, N*H*), 7.73 (d, ³J = 7.8 Hz, CH₂-C-C-C*H*), 7.53-7.48 (*m*, 1H, C-O-C*H*), 7.35 (*d*, ³J = 7.6 Hz, N-C-C*H*), 7.23-7.11 (*m*, 2H, C-CH-C*H-*C*H*-CH), 7.09-7.01 (*m*, 1H, C(=O)-C-C*H*), 7.07 (s, 1H, C-C*H*-NH), 6.52-6.47 (*m*, 1H, C-O-CH-C*H*), 4.58-4.29 (*m*, 4H, N-C*H₂*-CH₂-N-C(=O), N-C*H₂*CH₂-C(=O)), 4.09-3.85 (*m*, 4H N-CH₂-C*H₂*-C(=O), N-CH₂-C*H₂*-C(=O)), 3.67 (*t*, ³J = 7.8 Hz, 2H, S-C*H₂*-CH₂-C), 3.19 (*t*, ³J = 7.8 Hz, 2H, S-CH₂-C*H₂*-C). | | | |
| **¹³C**-**NMR:** | (CDCl₃, 100 MHz), δ [ppm] = 198.2 (C=S), 147.5 (C(=O)-C), 144.4 (C-O-CH), 136.4 (NH-C-CH), 127.4 (NH-C-C), 122.2 (C-CH-NH), 122.1 (NH-C-CH-CH), 119.5 (NH-C-CH-CH-CH), 119.1 (NH-C-C-CH), 117.6 (C(=O)-C-CH), 114.5 (NH-CH-C), 111.8 (C(=O)-C-CH-CH), 111.3 (NH-C-CH), 60.4 (N-CH₂-CH₂-N-(C=O), N-CH₂-CH₂-N-(=O)), 57.7 (N-CH₂-CH₂-N-C(=O), N-CH₂-CH₂-N-C(=O)), 40.5 (S-CH₂-C), 37.7 (S-CH₂-CH₂), 25.0 (S-CH₂-CH₂). | | | |
| **IR (ATR):** | v (cm⁻¹) = 3050 (w), 3005 (w), 2909 (w), 1597 (m), 1577 (w), 1492 (w), 1457 (w), 1436 (m), 1388 (w), 1340 (w), 1293 (w), 1247 (m), 1229 (m), 1218 (w), 1188 (w), 1155 (w), 1119 (w), 1090 (w), 1005 (m), 991 (m), 946 (w), 920 (w), 886 (w), 854 (w), 825 (w), 739 (vs), 720 (m), 635 (w), 616 (m), 591 (w), 579 (w), 563 (w), 541 (w), 482 (w), 463 (w), 426 (s). | | | |
| **MS** (ESI+): | m/z (%) = 400 (25, [M+H]⁺), 417 (100, [M+NH₄]⁺) | | | |
| **HRMS** (ESI+): | m/z für C₂₀H₂₁N₃O₂S₂ | | | |
| | ber.: | 399.1075 | | gef.: 399.1065 |
| **EA:** | ber.: | C: 60.12 | H:5.30 | N: 10.52 |
| | gef.: | C:60.13 | H:5.30 | N: 10.67 |

### Darstellung von (4-Nitrophenyl)methyl-4-ethylpiperazin-1-carbodithioat (SchI32284):

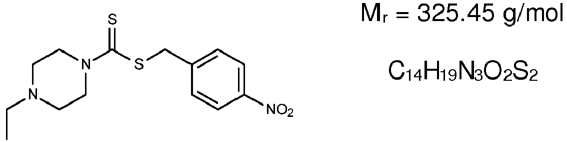

Gemäß **AAV8** wurden 0.51 mL *N*-Ethylpiperazin (4.0 mmol, 2.0 eq), 0.84 mL Tri-ethylamin (6.0 mmol, 3.0 eq), 0.22 mL Kohlenstoffdisulfid (6.0 mmol, 3.0 eq) und 432 mg 1-(Brommethyl)-4-nitrobenzen (2.0 mmol, 1.0 eq) umgesetzt. Säulenchromatographie an Kieselgel (Cyclohexan/ EtOAc 1:1) ergab 575 mg von **SchI32284** (88.5%) in Form eines gelben Feststoffes.

| | | | | |
|---|---|---|---|---|
| **DC**: | R_{f} = 0.28 (Cyclohexan/ EtOAc 1:1). | | | |
| **Fp.:** | 117 °C | | | |
| **¹H**-**NMR:** | (CDCl₃, 400 MHz), δ [ppm] = 8.14 (*d*, ³J = 8.7 Hz, 2H, C-CH-C*H*-C-NO₂, C-CH-C*H*-C-NO₂), 7.55 (*d*, ³J = 8.5 Hz, 2H, C-CH-C*H*-C-NO₂, C-C*H*-CH-C-NO₂), 4.68 (s, 2H, S-C*H₂*-C), 4.41-4.29 (m, 2H, N-CH*H*-CH₂-N-Et, N-CH*H*-CH₂-N-Et), 4.01-3.88 (m, 2H, N-CH*H*-CH₂-N-Et, N-CH*H*-CH₂-N-Et), 2.55-2.49 (m, 4H, N-CH₂-C*H₂*-N-Et, N-CH₂-C*H₂*-N-Et), 2.45 (q, ³J = 7.2 Hz, 2H, N-C*H₂-*CH₃), 1.09 (*t,* ³J = 7.2 Hz, 3H, N-CH₂-C*H₃*). | | | |
| **¹³C**-**NMR:** | (CDCl₃, 100 MHz), δ [ppm] = 195.0 (C=S), 147.2 (C(NO₂)), 134.9 (CH₂-C-CH-CH), 130.2 (C(NO₂)-CH-CH, C(NO₂)-CH-CH), 123.8 (C-CH-CH, C-CH-CH), 52.2 (N-CH₂-CH₂-N-Et, N-CH₂-C*H₂*-N-Et), 52.0 (N-CH₂-CH₃), 40.6 (S-CH₂-C), 12.0 (N-CH₂-CH₃). | | | |
| **IR (ATR):** | v (cm⁻¹ ) = 3386 (w), 3104 (w), 3046 (w), 1596 (w), 1520 (s), 1469 (w), 1456 (w), 1429 (w), 1376 (m), 1344 (w), 1331 (vs), 1308 (m), 1268 (w), 1236 (m), 1219 (m), 1180 (m), 1154 (w), 1124 (s), 1107 (w), 1089 (w), 1065 (w), 1053 (w), 1032 (m), 996 (vs), 919 (w), 893 (w), 857 (s), 818 (w), 769 (w), 723 (s), 686 (w), 636 (w), 547 (w), 496 (w), 470 (m). | | | |
| **MS** (ESI+): | m/z (%) = 326 (100, [M+H]⁺) | | | |
| **HRMS** (ESI+): | m/z für C₁₄H₁₉N₃O₂S₂ | | | |
| | ber.: | 325.0919 | | gef.: 325.0921 |
| **EA:** | ber.: | C: 51.67 | H: 5.88 | N: 12.91 |
| | gef.: | C: 51.57 | H: 5.92 | N: 12.75 |

### Darstellung von (4-Methylsulfonylphenyl)methyl-4-ethylpiperazin-1-carbodithioat (Schl32285):

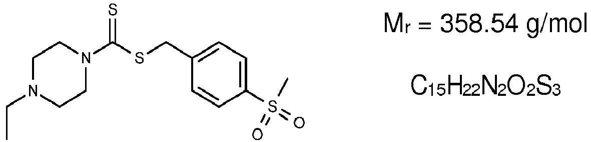

Gemäß **AAV8** wurden 0.51 mL *N*-Ethylpiperazin (4.0 mmol, 2.0 eq), 0.84 mL Tri-ethylamin (6.0 mmol, 3.0 eq), 0.22 mL Kohlenstoffdisulfid (6.0 mmol, 3.0 eq) und 498 mg 1-(Brommethyl)-4-methylsulfonylbenzen (2.0 mmol, 1.0 eq) umgesetzt. Säulenchromatographie an Kieselgel (Cyclohexan/ EtOAc 1:4) ergab 508 mg von **Schl32285** (70.9%) in Form eines blassgelben Feststoffes.

| | | | | |
|---|---|---|---|---|
| **DC**: | R_{f} = 0.21 (Cyclohexan/ EtOAc 1:4). | | | |
| **Fp.:** | 113 °C | | | |
| **¹H**-**NMR:** | (CDCl₃, 400 MHz), δ [ppm] = 7.85 (*d*, ³J = 8.0 Hz, 2H, CH₂-C-CH-C*H-*C-SO₂Me, CH₂-C-CH-C*H*-C-SO₂Me), 7.57 (*d*, ³J = 8.0 Hz, 2H, CH₂-C-C*H*-CH-C-SO₂Me, CH₂-C-C*H*-CH-C-SO₂Me), 4.66 (s, 2H, S-C*H₂-*C), 4.49-4.29 (m, 2H, N-CH*H*-CH₂-N-Et, N-CH*H*-CH₂-N-Et), 4.09-3.87 (m, 2H, N-CH*H*-CH₂-N-Et, N-CH*H*-CH₂-N-Et), 3.02 (s, 3H, SO₂-C*H₃*), 2.62-2.49 (m, 4H, N-CH₂-C*H₂*-N-Et, N-CH₂-C*H₂*-N-Et), 2.44 (q, ³J = 7.2 Hz, 2H, N-C*H₂-*CH₃), 1.08 (*t,* ³J = 7.2 Hz, 3H, N-CH₂-C*H₃*). | | | |
| **¹³C**-**NMR:** | (CDCl₃, 100 MHz), δ [ppm] = 195.1 (C=S), 143.5 (C-SO₂Me), 139.5 (CH₂-C-CH-CH), 130.3 (C-SO₂Me-CH-CH, C-SO₂Me-CH-CH), 127.6 (C-CH-CH, C-CH-CH), 52.2 (N-CH₂-CH₂-N-Et, N-CH₂-CH*₂*-N-Et), 51.9 (N-CH₂-CH₃), 44.6 (SO₂-CH₃), 40.8 (S-CH₂-C), 12.1 (N-CH₂-CH₃). | | | |
| **IR (ATR):** | v (cm⁻¹ ) = 3043 (w), 3023 (w), 2995 (w), 2972 (w), 2932 (w), 1597 (s), 1466 (w), 1423 (w), 1412 (w), 1383 (w), 1371 (w), 1351 (w), 1312 (w), 1296 (m), 1271 (m), 1235 (m), 1184 (w), 1158 (w), 1141 (vs), 1126 (s), 1087 (m), 1036 (w), 1014 (m), 997 (s), 960 (m), 920 (m), 850 (m), 769 (s), 746 (m), 701 (w), 652 (m), 547 (m), 521 (vs), 488 (s), 427 (m), 411 (w). | | | |
| **MS** (ESI+): | m/z (%) = 359 (100, [M+H]⁺) | | | |
| **HRMS** (ESI+): | m/z für C₁₅H₂₃N₂O₂S₃ | | | |
| | ber.: | 358.0843 | | gef.: 358.0876 |
| **EA:** | ber.: | C:50.25 | H:6.18 | N: 7.81 |
| | gef.: | C:50.11 | H: 6.20 | N: 7.81 |

### Darstellung von (4-Cyanophenyl)methyl-4-ethylpiperazin-1-carbodithioat (Schl32286):

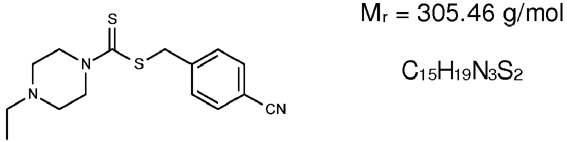

Gemäß **AAV8** wurden 0.51 mL *N*-Ethylpiperazin (4.0 mmol, 2.0 eq), 0.84 mL Tri-ethylamin (6.0 mmol, 3.0 eq), 0.22 mL Kohlenstoffdisulfid (6.0 mmol, 3.0 eq) und 392 mg 4-(Brommethyl)benzonitril (2.0 mmol, 1.0 eq) umgesetzt. Säulenchromatographie an Kieselgel (Cyclohexan/ EtOAc 1:1) ergab 538 mg von **Schl32286** (88.2%) in Form eines blassgelben Feststoffes.

| | | | | |
|---|---|---|---|---|
| **DC:** | R_{f} = 0.14 (Cyclohexan/ EtOAc 1:1). | | | |
| **Fp.:** | 104 °C | | | |
| **¹H**-**NMR:** | (CDCl₃, 400 MHz), δ [ppm] = 7.58 (*d*, ³J = 8.2 Hz, 2H, C-CH-C*H*-C-CN, C-CH-C*H*-C-CN), 7.49 (*d*, ³J = 8.2 Hz, 2H, C-C*H*-CH-C-CN, C-C*H*-CH-C-CN), 4.63 (*s*, 2H, S-C*H₂*-C), 4.43-4.26 (*m*, 2H, N-CH*H*-CH₂-N-Et, N-CH*H*-CH₂-N-Et), 4.05-3.85 (*m*, 2H, N-CH*H*-CH₂-N-Et, N-CH*H*-CH₂-N-Et), 2.58-2.47 (*m*, 4H, N-CH₂-C*H₂*-N-Et, N-CH₂-C*H₂*-N-Et), 2.45 (*q*, ³J = 7.2 Hz, 2H, N-C*H₂-*CH₃), 1.09 (*t*, ³J = 7.2 Hz, 3H, N-CH₂-C*H₃*). | | | |
| **¹³C**-**NMR:** | (CDCl₃, 100 MHz), δ [ppm] = 195.1 (C=S), 142.6 (C-CH-CH-C-CN), 132.3 (C-CH-CH-C-CN, C-CH-CH-C-CN), 130.1 (C-CH-CH-C-CN, C-CH-CH-C-CN), 118.9 (C-CN), 111.8 (C-CN), 52.2 (N-CH₂-CH₂-N-Et, N-CH₂-C*H₂*-N-Et), 52.0 (N-CH₂-CH₃), 41.0 (S-CH₂-C), 12.1 (N-CH₂-CH₃). | | | |
| **IR (ATR):** | v (cm⁻¹) = 3088 (w), 3046 (w), 2975 (w), 2937 (w), 1503 (m), 1472 (w), 1457 (s), 1427 (w), 1382 (w), 1353 (w), 1341 (w), 1329 (w), 1310 (w), 1267 (s), 1233 (m), 1218 (s), 1153 (vs), 1124 (m), 1097 (w), 1065 (w), 1053 (w), 1027 (m), 993 (vs), 938 (m), 920 (m), 891 (w), 853 (s), 837 (m), 826 (m), 754 (s), 644 (w), 551 (s), 539 (s), 482 (w), 467 (w), 423 (w). | | | |
| **MS** (ESI+): | m/z (%) = 306 (100, [M+H]⁺) | | | |
| **HRMS** (ESI+): | m/z für C₁₅H₁₉N₃S₂ | | | |
| | ber.: | 305.1020 | | gef.: 305.1047 |
| **EA:** | ber.: | C: 58.98 | H: 6.27 | N: 13.76 |
| | gef.: | C:58.99 | H: 6.33 | N: 14.11 |

### Darstellung von (2-Hydroxy-5-nitrophenyl)methyl-4-ethylpiperazin-1-carbodithioat (Schl32287):

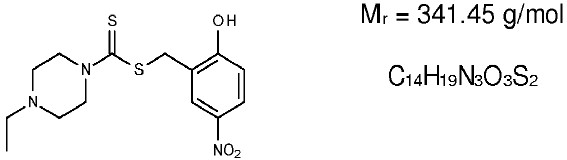

Gemäß **AAV8** wurden 0.51 mL *N*-Ethylpiperazin (4.0 mmol, 2.0 eq), 0.84 mL Tri-ethylamin (6.0 mmol, 3.0 eq), 0.22 mL Kohlenstoffdisulfid (6.0 mmol, 3.0 eq) und 464 mg 2-Hydroxy-5-nitrobenzylbromid (2.0 mmol, 1.0 eq) umgesetzt. Säulenchromatographie an Kieselgel (DCM/ MeOH 7:1) ergab 569 mg von **Schl32287** (83.4%) in Form eines braunen Feststoffes.

| | | | | |
|---|---|---|---|---|
| **DC:** | R_{f} = 0.66 (DCM/ MeOH 7:1). | | | |
| **Fp.:** | 165 °C | | | |
| **¹H**-**NMR:** | (DMSO-D₆, 400 MHz), δ [ppm] = 8.23 (*s*, 1 H, C-C*H*-C(NO₂)), 8.00 *(dd,* ³J = 8.9 Hz, ²J = 2.8 Hz, 1 H, C(NO₂)-C*H*-CH-C-OH), 6.93 (*d*, ³J = 8.9 Hz, 1 H, C(NO₂)-CH-C*H*-C-OH), 4.50 (*s*, 2H, S-C*H₂*-C), 4.29-4.11 *(m*, 2H, N-CH*H*-CH₂-N-Et, N-CH*H*-CH₂-N-Et), 3.94-3.73 (*m*, 2H, N-CH*H*-CH₂-N-Et, N-CH*H*-CH₂-N-Et), 2.44-2.37 (*m*, 4H, N-CH₂-C*H₂*-N-Et, N-CH₂-C*H₂*-N-Et), 2.33 (q, ³J = 7.1 Hz, 2H, N-C*H₂-*CH₃), 0.97 (*t,* ³J = 7.2 Hz, 3H, N-CH₂-C*H₃*). | | | |
| **¹³C**-**NMR:** | (DMSO-D₆, 100 MHz), δ [ppm] = 195.1 (C=S), 163.3 (C-OH), 139.3 (C-NO₂), 126.9 (C(NO₂)-CH-CH-C), 125.7 (C-CH-C(NO₂)) 124.5 (S-CH₂-C), 116.0 (C(NO₂)-CH-CH-C), 52.3 (N-CH₂-CH₂-N-Et, N-CH₂-CH*₂*-N-Et), 51.5 (N-CH₂-CH₃), 35.6 (S-CH₂-C), 12.4 (N-CH₂-CH₃). | | | |
| **IR (ATR):** | v (cm⁻¹) = 3065 (w), 2974 (w), 2934 (w), 2877 (w), 1590 (w), 1524 (w), 1471 (m), 1441 (w), 1424 (m), 1361 (w), 1333 (s), 1285 (m), 1264 (m), 1237 (m), 1225 (m), 1151 (w), 1132 (w), 1086 (w), 1012 (m), 990 (s), 870 (s), 824 (m), 815 (m), 753 (s), 739 (vs), 695 (m), 660 (m), 641 (m), 631 (m), 601 (m), 539 (m), 525 (m), 488 (s), 467 (s), 444 (s), 415 (s). | | | |
| **MS** (ESI+): | m/z (%) = 342 (100, [M+H]⁺) | | | |
| **HRMS** (ESI+): | m/z für C₁₄H₁₉N₃O₃S₂ | | | |
| | ber.: | 341.0868 | | gef.: 341.0894 |
| **EA:** | ber.: | C:49.25 | H: 5.61 | N: 12.31 |
| | gef.: | C:49.11 | H: 5.60 | N: 12.36 |

### Darstellung von Cyclohexylmethyl-4-ethylpiperazin-1-carbodithioat (Schl32288):

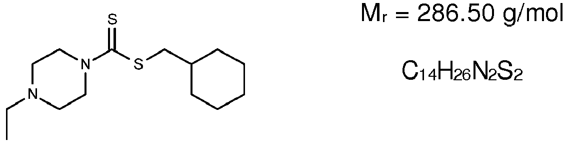

Gemäß **AAV8** wurden 0.51 mL *N*-Ethylpiperazin (4.0 mmol, 2.0 eq), 0.84 mL Tri-ethylamin (6.0 mmol, 3.0 eq), 0.22 mL Kohlenstoffdisulfid (6.0 mmol, 3.0 eq) und 0.28 mL Brommethylcyclohexan (2.0 mmol, 1.0 eq) umgesetzt. Säulenchromatographie an Kieselgel (Cyclohexan/ EtOAc 1:1) ergab 203 mg von **Schl32288** (35.5%) in Form eines blassgelben Öls.

| | | | | |
|---|---|---|---|---|
| **DC:** | R_{f} = 0.17 (Cyclohexan/ EtOAc 1:1). | | | |
| **¹H**-**NMR:** | (CDCI₃, 400 MHz), δ [ppm] = 4.39-4.20 *(m,* 2H, N-CH*H*-CH₂-N-Et, N-CH*H*-CH₂-N-Et), 4.03-3.86 (*m*, 2H, N-CH*H*-CH₂-N-Et, N-CH*H*-CH₂-N-Et), 3.15 (*d*, ³J = 6.9 Hz, 2H, S-C*H₂*-CH), 2.46 (*t,* ³J = 4.8 Hz, 4H, N-CH₂-C*H₂*-N-Et, N-CH₂-C*H₂*-N-Et), 2.39 (q, ³J = 7.2 Hz, 2H, N-C*H₂-*CH₃), 1.85-1.77 (*m*, 2H, C*H*_{(Cyclohexyl)}), 1.69-1.54 (*m*, 3H C*H*(_{Cyclohexyl)}, S-CH₂-C*H*), 1.25-1.08 (*m*, 4H, C*H*_{(Cyclohexyl)}), 1.05 *(t,* ³J = 7.1 Hz, 3H, N-CH₂-C*H₃*), 1.01-0.89 (*m*, 4H, C*H*_{(Cyclohexyl)}). | | | |
| **¹³C**-**NMR:** | (CDCI₃, 100 MHz), δ [ppm] = 197.6 (C=S), 52.3 (N-CH₂-CH₂-N-Et, N-CH₂-C*H₂*-N-Et), 52.0 (N-CH₂-CH₃), 44.4 (S-CH₂-CH), 37.3 (S-CH₂-CH), 32.9 (CH_{(Cyclohexyl)}), 26.4 (CH_{(Cyclohexyl)}), 26.1 (CH_{(Cyclohexyl)}), 12.1 (N-CH₂-CH₃). | | | |
| **IR (ATR):** | v (cm⁻¹) = 2971 (w), 2923 (m), 2851 (w), 1467 (m), 1449 (m), 1420 (vs), 1380 (w), 1346 (w), 1332 (w), 1308 (w), 1286 (w), 1269 (m), 1228 (vs), 1218 (vs), 1158 (s), 1145 (m), 1124 (m), 1091 (w), 1017 (s), 996 (vs), 962 (w), 922 (w), 902 (m), 833 (w), 766 (w), 737 (w), 630 (w), 595 (w), 552 (w), 539 (w), 487 (w), 467 (w), 419 (w). | | | |
| **MS** (ESI+): | m/z (%) = 287 (100, [M+H]⁺) | | | |
| **HRMS** (ESI+): | m/z für C₁₄H₂₆N₂S₂ | | | |
| | ber.: | 286.1537 | | gef.: 286.1561 |
| **EA:** | ber.: | C: 58.69 | H:9.15 | N: 9.78 |
| | gef.: | C: 58.70 | H: 9.10 | N: 9.66 |

### Darstellung von (7-Methoxy-2-oxochromen-4-yl)methyl-4-ethylpiperazin-1-carbodithioat (SchI32289):

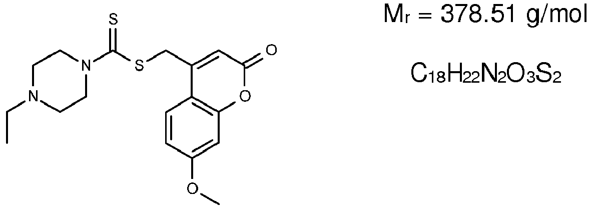

Gemäß **AAV8** wurden 0.51 mL *N*-Ethylpiperazin (4.0 mmol, 2.0 eq), 0.84 mL Tri-ethylamin (6.0 mmol, 3.0 eq), 0.22 mL Kohlenstoffdisulfid (6.0 mmol, 3.0 eq) und 538 mg 4-(Brommethyl)-7-methoxychromen-2-on (2.0 mmol, 1.0 eq) umgesetzt. Säulenchromatographie an Kieselgel (Cyclohexan/ EtOAc 1:3) ergab 713 mg von **Schl32289** (97.2%) in Form eines blassgelben Feststoffes.

| | | | | |
|---|---|---|---|---|
| **DC**: | R_{f} = 0.23 (Cyclohexan/ EtOAc 1:1). | | | |
| **Fp.:** | 146 °C | | | |
| **¹H**-**NMR:** | (CDCI₃, 400 MHz), δ [ppm] = 7.60 (*d*, ³J = 8.9 Hz, 1H, C-C*H*-CH-C-O-CH₃), 6.85 (*dd*, ³J = 8.7 Hz, ²J = 2.5 Hz, 1H, C-CH-C*H*-C-O-CH₃), 6.82-6.78 (*m*, 1H, C-C*H*-C-O-CH₃), 6.38 (s, 1 H, C-C*H*-C=O), 4.69 (s, 2H, S-C*H₂*-C), 4.42-4.28 (*m*, 2H, N-CH*H*-CH₂-N-Et, N-CH*H*-CH₂-N-Et), 4.02-3.91 (*m*, 2H, N-CH*H*-CH₂-N-Et, N-CH*H*-CH₂-N-Et), 3.85 (s, 3H, O-C*H₃*), 2.64-2.51 (*m*, 4H, N-CH₂-C*H₂*-N-Et, N-CH₂-C*H₂*-N-Et), 2.47 (q, ³J = 7.1 Hz, 2H, N-C*H₂-*CH₃), 1.10 (*t*, ³J = 6.2 Hz, 3H, N-CH₂-C*H₃*). | | | |
| **¹³C**-**NMR:** | (CDCl₃, 100 MHz), δ [ppm] = 194.0 (C=S), 162.9 (C(=O)-O), 161.0 (C-OMe), 159.2 (S-CH₂-C), 155.6 (C(=O)-O-C), 150.4 (C(=O)-C), 125.7 (C-CH-CH-C-OMe), 112.8 (C-CH-C(=O)), 112.2 (C-CH-CH-C-OMe), 101.2 (C-CH-C-OMe), 55.9 (O-CH₃), 52.2 (N-CH₂-CH₂-N-Et, N-CH₂-CH*₂*-N-Et), 51.9 (N-CH₂-CH₃), 37.5 (S-CH₂-C), 12.0 (N-CH₂-CH₃). | | | |
| **IR (ATR):** | v (cm⁻¹) = 3105 (w), 2972 (w), 2931 (w), 1711 (m), 1614 (s), 1557 (w), 1510 (w), 1461 (w), 1431 (m), 1379 (w), 1343 (w), 1330 (m), 1297 (s), 1271 (m), 1227 (w), 1181 (w), 1145 (vs), 1126 (s), 1048 (w), 1016 (m), 995 (s), 972 (w), 928 (w), 886 (m), 849 (w), 841 (m), 809 (w), 767 (w), 727 (w), 702 (w), 567 (w), 550 (w), 537 (w), 486 (w), 453 (w), 425 (w). | | | |
| **MS** (ESI+): | m/z (%) = 379 (100, [M+H]⁺) | | | |
| **HRMS** (ESI+): | m/z für C₁₈H₂₂N₂O₃S₂ | | | |
| | ber.: | 378.1072 | | gef.: 378.1096 |
| **EA:** | ber.: | C: 57.12 | H: 5.86 | N: 7.40 |
| | gef.: | C: 57.55 | H: 5.94 | N: 7.59 |

### Darstellung von 2-(1H-Indol-3-yl)ethyl-4-ethylpiperazin-1-carbodithioat (Schl32290):

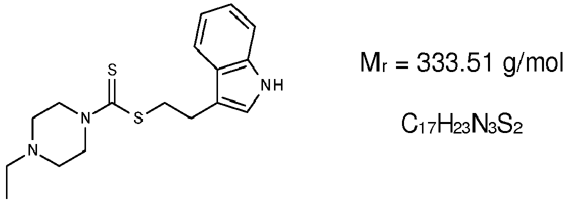

Gemäß **AAV8** wurden 0.51 mL *N*-Ethylpiperazin (4.0 mmol, 2.0 eq), 0.84 mL Tri-ethylamin (6.0 mmol, 3.0 eq), 0.22 mL Kohlenstoffdisulfid (6.0 mmol, 3.0 eq) und 448 mg 3-(2-Bromethyl)indol (2.0 mmol, 1.0 eq) umgesetzt. Säulenchromatographie an Kieselgel (Cyclohexan/ EtOAc 1:1) ergab 612 mg von **Schl32290** (91.8%) in Form eines braungelben Feststoffes.

| | | | | |
|---|---|---|---|---|
| **DC:** | R_{f} = 0.21 (Cyclohexan/ EtOAc 1:1). | | | |
| **Fp.:** | 106 °C | | | |
| **¹H**-**NMR:** | (CDCI₃, 400 MHz), δ [ppm] = 8.44 (*sbr,* 1H, N*H*), 7.72 (*d*, ³J = 7.8 Hz, CH₂-C-C-C*H*), 7.30 (*d*, ³J = 7.6 Hz, N-C-C*H*), 7.17 (*t,* ³J = 7.5 Hz, 1H, C-CH-C*H*-CH-CH), 7.11 (*t,* ³J = 7.6 Hz, 1H, C-CH-CH-C*H*-CH), 7.08 (s, 1 H, C-C*H*-NH), 4.46-4.27 (*m*, 2H, N-CH*H*-CH₂-N-Et, N-CH*H*-CH₂-N-Et), 4.11 (*q*, ³J = 7.2 Hz, 2H, N-C*H₂-*CH₃), 4.00-3.80 (*m*, 2H, N-CH*H*-CH₂-N-Et, N-CH*H*-CH₂-N-Et), 3.65 (*t,* ³J = 7.6 Hz, 2H, S-C*H₂₋*CH₂-C), 3.17 (*t*, ³J = 7.7 Hz, 2H, S-CH₂-C*H₂*-C), 2.54-2.37 (*m*, 4H, N-CH₂-C*H₂*-N-Et, N-CH₂-C*H₂*-N-Et), 1.08 (*t*, ³J = 7.2 Hz, 3H, N-CH₂-C*H₃*). | | | |
| **¹³C**-**NMR:** | (CDCl₃, 100 MHz), δ [ppm] = 197.1 (C=S), 136.4 (NH-C-CH), 127.4 (NH-C-C), 122.2 (C-CH-NH), 122.0 (NH-C-CH-CH), 119.4 (NH-C-CH-CH-CH), 119.1 (NH-C-C-CH), 114.5 (NH-CH-C), 111.3 (NH-C-CH), 52.2 (N-CH₂-CH₂-N-Et, N-CH₂-CH*₂*-N-Et), 52.0 (N-CH₂-CH₃), 37.6 (S-CH₂-CH₂), 25.1 (S-CH₂-CH₂), 12.1 (N-CH₂-CH₃). | | | |
| **IR (ATR):** | v (cm⁻¹) = 3150 (w), 3108 (w), 3056 (w), 2972 (w), 1456 (m), 1425 (s), 1379 (w), 1345 (w), 1311 (w), 1284 (w), 1268 (m), 1227 (s), 1213 (s), 1156 (w), 1142 (s), 1119 (m), 1106 (m), 1094 (w), 1033 (s), 1013 (vs), 998 (s), 939 (w), 921 (s), 834 (w), 795 (w), 763 (w), 738 (s), 633 (w), 607 (w), 592 (w), 577 (w), 560 (w), 537 (m), 458 (m), 421 (s). | | | |
| **MS** (ESI+): | m/z (%) = 334 (100, [M+H]⁺) | | | |
| **HRMS** (ESI+): | m/z für C₁₇H₂₃N₃S₂ | | | |
| | ber.: | 333.1333 | | gef.: 333.1360 |
| **EA:** | ber.: | C: 61.22 | H: 6.95 | N: 12.60 |
| | gef.: | C: 61.16 | H: 6.94 | N: 12.47 |

### Darstellung von (4-Nitrophenyl)methyl-4-(p-tolylmethyl)piperazin-1-carbodithioat (Schl32291):

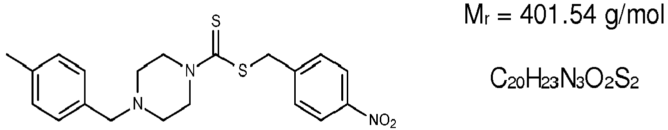

Gemäß **AAV8** wurden 381 mg 1-(*p*-Tolylmethyl)piperazin (2.0 mmol, 1.0 eq), 0.84 mL Triethylamin (6.0 mmol, 3.0 eq), 0.22 mL Kohlenstoffdisulfid (6.0 mmol, 3.0 eq) und 432 mg 1-(Brommethyl)-4-nitrobenzen (2.0 mmol, 1.0 eq) umgesetzt. Säulenchromatographie an Kieselgel (Cyclohexan/ EtOAc 2:1) ergab 706 mg von **Schl32291** (87.9%) in Form eines blassgelben Feststoffes.

| | | | | |
|---|---|---|---|---|
| **DC:** | R_{f} = 0.39 (Cyclohexan/ EtOAc 2:1). | | | |
| **Fp.:** | 147 °C | | | |
| **¹H**-**NMR:** | (CDCl₃, 400 MHz), δ [ppm] = 8.15 (*d*, ³J = 8.0 Hz, 2H, C-CH-C*H*-C-NO₂, C-CH-C*H*-C-NO₂), 7.55 (*d*, ³J = 8.7 Hz, 2H, C-CH-C*H*-C-NO₂, C-CH-C*H*-C-NO₂), 7.19 (*d*, ³J = 7.8 Hz, 2H, C-CH-C*H*-C-CH₂-N, C-CH-C*H*-C-CH₂-N), 7.13 (*d*, ³J = 7.8 Hz, 2H, C-CH-C*H*-C-CH₂-N, C-CH-C*H*-C-CH₂-N), 4.68 (s, 2H, S-C*H₂*-C), 4.46-4.27 *(m*, 2H, N-CH₂-CH*H*-N-C-S, N-CH₂-CH*H*-N-C-S), 4.04-3.81 (*m*, 2H, N-CH₂-CH*H*-N-C-S, N-CH₂-CH*H*-N-C-S), 3.51 (*s*, 2H, C-C*H₂*-N), 2.64-2.45 (*m*, 4H, N-C*H₂*-CH₂-N-C-S, N-C*H₂*-CH₂-N-C-S), 2.34 (*s*, 3H, C-C*H₃*). | | | |
| **¹³C**-**NMR:** | (CDCI₃, 100 MHz), δ [ppm] = 194.9 (C=S), 147.2 (C(NO₂)), 144.8 (CH₂-C-CH-CH-C(NO₂)), 137.3 (N-CH₂-C), 134.8 (N-CH₂-C-CH-CH-C), 130.2 (C(NO₂)-CH-CH, C(NO₂)-CH-CH), 129.3 (N-CH₂-C-CH-CH-C, N-CH₂-C-CH-CH-C), 129.2 (N-CH₂-C-CH-CH-C, N-CH₂-C-CH-CH-C), 123.8 (C-CH-CH-C(NO₂), C-CH-CH-C(NO₂)), 62.3 (C-CH₂-N), 52.4 (N-CH₂-CH₂-N, N-CH₂-CH₂-N), 40.6 (S-CH₂-C), 21.2 (C-CH₃). | | | |
| **IR (ATR):** | v (cm⁻¹) = 3081 (w), 3001 (w), 2949 (w), 2938 (w), 1521 (s), 1495 (m), 1469 (m), 1455 (m), 1443 (w), 1383 (w), 1345 (vs), 1298 (w), 1285 (m), 1269 (m), 1256 (m), 1181 (s), 1134 (w), 1111 (s), 1098 (s), 1052 (s), 1017 (vs), 990 (vs), 971 (vs), 918 (w), 877 (s), 857 (m), 848 (m), 813 (s), 801 (vs), 714 (vs), 679 (m), 644 (m), 631 (m), 493 (s), 481 (m), 471 (w). | | | |
| **MS** (ESI+): | m/z (%) = 402 (100, [M+H]⁺) | | | |
| **HRMS** (ESI+): | m/z für C₂₀H₂₃N₃O₂S₂ | | | |
| | ber.: | 401.1232 | | gef.: 401.1251 |
| **EA:** | ber.: | C: 59.82 | H: 5.77 | N: 10.46 |
| | gef.: | C: 59.98 | H: 5.88 | N: 10.29 |

### Darstellung von (4-Methylsulfonylphenyl)methyl-4-(p-tolylmethyl)-piperazin-1-carbodithioat (SchI32292):

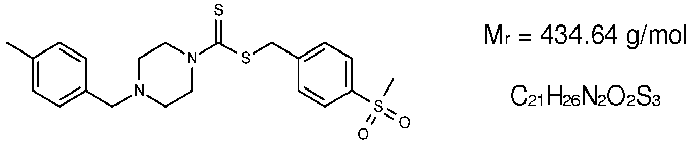

Gemäß **AAV8** wurden 381 mg 1-(*p*-Tolylmethyl)piperazin (2.0 mmol, 1.0 eq), 0.84 mL Triethylamin (6.0 mmol, 3.0 eq), 0.22 mL Kohlenstoffdisulfid (6.0 mmol, 3.0 eq) und 498 mg 1-(Brommethyl)-4-methylsulfonylbenzen (2.0 mmol, 1.0 eq) umgesetzt. Säulenchromatographie an Kieselgel (Cyclohexan/ EtOAc 1:1) ergab 659 mg von **Schl32292** (75.8%) in Form eines farblosen Feststoffes.

| | | | | |
|---|---|---|---|---|
| **DC:** | R_{f} = 0.27 (Cyclohexan/ EtOAc 1:1). | | | |
| **Fp.:** | 138 °C | | | |
| **¹H**-**NMR:** | (CDCl₃, 400 MHz), δ [ppm] = 7.86 (*d*, ³J = 8.2 Hz, 2H, CH₂-C-CH-C*H*-C-SO₂Me, CH₂-C-CH-C*H*-C-SO₂Me), 7.58 (*d*, ³J = 8.0 Hz, 2H, CH₂-C-C*H-*CH-C-SO₂Me, CH₂-C-CH-C*H*-C-SO₂Me), 7.19 (*d,* ³J = 7.8 Hz, 2H, C-CH-C*H*-C-CH₂-N, C-CH-C*H*-C-CH₂-N), 7.13 (*d*, ³J = 8.0 Hz, 2H, C-CH-C*H*-C-CH₂-N, C-CH-C*H*-C-CH₂-N), 4.67 (s, 2H, S-C*H₂*-C), 4.41-4.26 (*m*, 2H, N-CH₂-CH*H*-N-C-S, N-CH₂-CH*H*-N-C-S), 4.03-3.83 (*m*, 2H, N-CH₂-CH*H*-N-C-S, N-CH₂-CH*H*-N-C-S), 3.51 (*s*, 2H, C-C*H*₂-N), 3.03 (s, 3H, SO₂-C*H*₃), 2.60-2.43 (*m*, 4H, N-C*H₂*-CH₂-N-C-S, N-C*H₂*-CH₂-N-C-S), 2.33 (s, 3H, C-C*H₃*). | | | |
| **¹³C**-**NMR:** | (CDCl₃, 100 MHz), δ [ppm] = 195.1 (C=S), 143.5 (C-SO₂Me), 139.5 (CH₂-C-CH-CH), 137.3 (N-CH₂-C), 133.5 (N-CH₂-C-CH-CH-C), 130.3 (C-SO₂Me-CH-CH, C-SO₂Me-CH-CH), 129.3 (N-CH₂-C-CH-CH-C, N-CH₂-C-CH-CH-C), 129.2 (N-CH₂-C-CH-CH-C, N-CH₂-C-CH-CH-C), 127.7 (C-CH-CH, C-CH-CH), 62.2 (C-CH₂-N), 52.3 (N-CH₂-CH₂-N, N-CH₂-CH₂-N), 44.6 (SO₂-CH₃), 40.8 (S-CH₂-C), 21.2 (C-CH₃). | | | |
| **IR (ATR):** | v (cm⁻¹ ) = 3004 (w), 2923 (w), 2764 (w), 1515 (w), 1469 (w), 1455 (w), 1444 (w), 1422 (m), 1407 (w), 1355 (m), 1343 (s), 1320 (m), 1269 (w), 1218 (s), 1183 (vs), 1156 (s), 1146 (m), 1135 (s), 1091 (w), 1052 (s), 1018 (s), 990 (s), 966 (w), 919 (m), 846 (m), 765 (s), 738 (m), 639 (m), 572 (s), 551 (w), 537 (w), 531 (vs), 518 (m), 492 (m), 472 (w). | | | |
| **MS** (ESI+): | m/z (%) = 435 (100, [M+H]⁺) | | | |
| **HRMS** (ESI+): | m/z für C₂₁H₂₆N₂O₂S₃ | | | |
| | ber.: | 434.1156 | | gef.: 434.1175 |
| **EA:** | ber.: | C: 58.03 | H: 6.03 | N: 6.45 |
| | gef.: | C: 58.11 | H: 6.09 | N: 6.37 |

### Darstellung von (4-Cyanophenyl)methyl-4-(p-tolylmethyl)piperazin-1-carbodithioat (Schl32293):

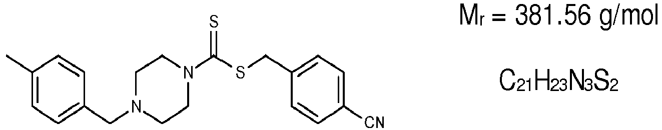

Gemäß **AAV8** wurden 381 mg 1-(*p*-Tolylmethyl)piperazin (2.0 mmol, 1.0 eq), 0.84 mL Triethylamin (6.0 mmol, 3.0 eq), 0.22 mL Kohlenstoffdisulfid (6.0 mmol, 3.0 eq) und 392 mg 4-(Brommethyl)benzonitril (2.0 mmol, 1.0 eq) umgesetzt. Säulenchromatographie an Kieselgel (Cyclohexan/ EtOAc 2:1) ergab 725 mg von **Schl32293** (95.0%) in Form eines farblosen Feststoffes.

| | | | | |
|---|---|---|---|---|
| **DC:** | R_{f} = 0.39 (Cyclohexan/ EtOAc 2:1). | | | |
| **Fp.:** | 127 °C | | | |
| **¹H**-**NMR:** | (CDCl₃, 400 MHz), δ [ppm] = 7.58 (*d*, ³J = 8.5 Hz, 2H, C-CH-C*H*-C-CN, C-CH-C*H*-C-CN), 7.49 (*d*, ³J = 8.5 Hz, 2H, C-CH-C*H*-C-CN, C-CH-C*H*-C-CN), 7.19 (*d*, ³J = 7.8 Hz, 2H, C-CH-C*H*-C-CH₂-N, C-CH-C*H*-C-CH₂-N), 7.13 (*d*, ³J = 7.8 Hz, 2H, C-CH-C*H*-C-CH₂-N, C-CH-C*H*-C-CH₂-N), 4.63 (s, 2H, S-C*H₂*-C), 4.45-4.27 (m, 2H, N-CH₂-CH*H*-N-C-S, N-CH₂-CH*H*-N-C-S), 4.02-3.82 (*m*, 2H, N-CH₂-CH*H*-N-C-S, N-CH₂-CH*H*-N-C-S), 3.51 (*s*, 2H, C-C*H₂*-N), 2.60-2.42 (*m*, 4H, N-C*H₂*-CH₂-N-C-S, N-C*H₂*-CH₂-N-C-S), 2.34 (*s*, 3H, C-C*H₃*). | | | |
| **¹³C**-**NMR:** | (CDCl₃, 100 MHz), δ [ppm] = 195.2 (C=S), 142.6 (C-CH-CH-C-CN), 136.9 (N-CH₂-C), 134.0 (N-CH₂-C-CH-CH-C), 132.4 (C-CH-CH-C-CN, C-CH-CH-C-CN), 130.1 (C-CH-CH-C-CN, C-CH-CH-C-CN), 129.3 (N-CH₂-C-CH-CH-C, N-CH₂-C-CH-CH-C), 129.2 (N-CH₂-C-CH-CH-C, N-CH₂-C-CH-CH-C), 118.9 (C-CN), 111.3 (C-CN), 62.3 (C-CH₂-N), 52.3 (N-CH₂-CH₂-N, N-CH₂-CH₂-N), 41.0 (S-CH₂-C), 21.2 (C-CH₃). | | | |
| **IR (ATR):** | v (cm⁻¹ ) = 3057 (w), 2999 (w), 2948 (w), 2938 (w), 1512 (w), 1468 (m), 1456 (m), 1442 (s), 1412 (w), 1385 (w), 1354 (w), 1320 (w), 1285 (w), 1269 (w), 1259 (s), 1250 (w), 1216 (m), 1179 (m), 1134 (w), 1113 (s), 1098 (vs), 1051 (s), 990 (m), 971 (w), 917 (s), 848 (s), 812 (w), 759 (m), 709 (m), 632 (w), 532 (vs), 493 (w), 472 (w), 450 (w), 434 (w). | | | |
| **MS** (ESI+): | m/z (%) = 382 (100, [M+H]⁺) | | | |
| **HRMS** (ESI+): | m/z für C₂₁H₂₃N₃S₂ | | | |
| | ber.: | 381.1333 | | gef.: 381.1359 |
| **EA:** | ber.: | C: 66.10 | H: 6.08 | N: 11.01 |
| | gef.: | C: 65.85 | H: 5.97 | N: 10.93 |

### Darstellung von (2-Hydroxy-5-nitro-phenyl)methyl-4-(p-tolylmethyl)piperazin-1-carbodithioat (Schl32294):

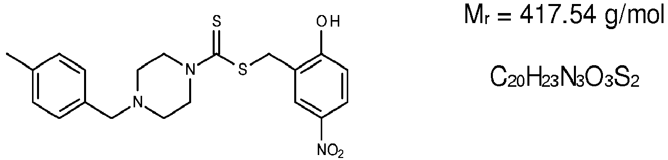

Gemäß **AAV8** wurden 381 mg 1-(*p*-Tolylmethyl)piperazin (2.0 mmol, 1.0 eq), 0.84 mL Triethylamin (6.0 mmol, 3.0 eq), 0.22 mL Kohlenstoffdisulfid (6.0 mmol, 3.0 eq) und 464 mg 2-Hydroxy-5-nitrobenzylbromid (2.0 mmol, 1.0 eq) umgesetzt. Säulenchromatographie an Kieselgel (Cyclohexan/ EtOAc 1:1) ergab 792 mg von **Schl32294** (94.5%) in Form eines gelben Feststoffes.

| | | | | |
|---|---|---|---|---|
| **DC:** | R_{f} = 0.42 (Cyclohexan/ EtOAc 1:1). | | | |
| **Fp.:** | 86 °C | | | |
| **¹H**-**NMR:** | (DMSO-D₆, 400 MHz), δ [ppm] = 8.11 (s, 1H, C-C*H*-C(NO₂)) 8.05 (*dd*, ³J = 8.9 Hz, ²J = 2.8 Hz, 1H, C(NO₂)-C*H*-CH-C-OH), 7.19 (*d,* ³J = 8.0 Hz, 2H, C-CH-C*H*-C-CH₂-N, C-CH-C*H*-C-CH₂-N), 7.13 (*d,* ³J = 7.8 Hz, 2H, C-CH-C*H*-C-CH₂-N, C-CH-C*H*-C-CH₂-N), 6.89 (*d*, ³J = 8.9 Hz, 1H, C(NO₂)-CH-C*H*-C-OH), 4.79 (s, 2H, S-C*H₂*-C), 4.45-4.28 (m, 2H, N-CH₂-CH*H*-N-C-S, N-CH₂-CH*H*-N-C-S), 4.00-3.86 (m, 2H, N-CH₂-CH*H*-N-C-S, N-CH₂-CH*H*-N-C-S), 3.53 (s, 2H, C-C*H*₂-N), 2.65-2.48 (m, 4H, N-C*H₂*-CH₂-N-C-S, N-C*H₂*-CH₂-N-C-S), 2.33 (s, 3H, C-C*H₃*). | | | |
| **¹³C**-**NMR:** | (DMSO-D₆, 100 MHz), δ [ppm] = 196.3 (C=S), 161.3 (C-OH), 140.8 (C-NO₂), 137.6 (N-CH₂-C), 133.3 (N-CH₂-C-CH-CH-C), 129.4 (N-CH₂-C-CH-CH-C, N-CH₂-C-CH-CH-C), 129.3 (N-CH₂-C-CH-CH-C, N-CH₂-C-CH-CH-C), 127.3 (C(NO₂)-CH-CH-C), 125.6 (C-CH-C(NO₂) 123.9 (S-CH₂-C), 117.4 (C(NO₂)-CH-CH-C), 62.1 (C-CH₂-N), 52.2 (N-CH₂-CH₂-N, N-CH₂-CH₂-N), 37.8 (S-CH₂-C), 21.2 (C-CH₃). | | | |
| **IR (ATR):** | v (cm⁻¹ ) = 2929 (w), 2811 (w), 1732 (w), 1590 (w), 1517 (w), 1494 (w), 1467 (w), 1421 (w), 1334 (s), 1276 (s), 1223 (s), 1136 (w), 1078 (m), 1022 (w), 985 (m), 936 (m), 909 (w), 864 (w), 813 (w), 779 (w), 748 (m), 690 (w), 640 (m), 595 (w), 575 (w), 50 (w), 530 (w), 489 (m). | | | |
| **MS** (ESI+): | m/z (%) = 418 (100, [M+H]⁺) | | | |
| **HRMS** (ESI+): | m/z für C₂₀H₂₃N₃O₃S₂ | | | |
| | ber.: | 417.1181 | | gef.: 417.1208 |
| **EA:** | ber.: | C: 57.53 | H: 5.55 | N: 10.06 |
| | gef.: | C: 57.40 | H: 5.62 | N: 10.05 |

### Darstellung von Cyclohexylmethyl-4-(p-tolylmethyl)piperazin-1-carbodithioat (Schl32295):

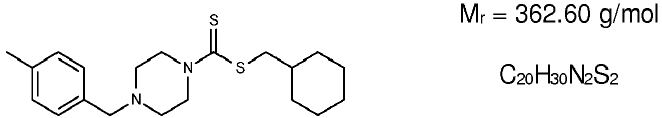

Gemäß **AAV8** wurden 381 mg 1-(*p*-Tolylmethyl)piperazin (2.0 mmol, 1.0 eq), 0.84 mL Triethylamin (6.0 mmol, 3.0 eq), 0.22 mL Kohlenstoffdisulfid (6.0 mmol, 3.0 eq) und 0.28 mL Brommethylcyclohexan (2.0 mmol, 1.0 eq) umgesetzt. Säulenchromatographie an Kieselgel (Cyclohexan/ EtOAc 2:1) ergab 588 mg von **Schl32295** (81.1 %) in Form eines gelben Feststoffes.

| | | | | |
|---|---|---|---|---|
| **DC:** | R_{f} = 0.60 (Cyclohexan/ EtOAc 2:1). | | | |
| **Fp.:** | 84 °C | | | |
| **¹H**-**NMR:** | (CDCl₃, 400 MHz), δ [ppm] = 7.20 (*d*, ³J = 8.0 Hz, 2H, C-CH-C*H*-C-CH₂-N, C-CH-C*H*-C-CH₂-N), 7.13 (*d*, ³J = 8.0 Hz, 2H, C-CH-C*H*-C-CH₂-N, C-CH-C*H*-C-CH₂-N), 4.45-4.27 (*m*, 2H, N-CH₂-CH*H*-N-C-S, N-CH₂-CH*H*-N-C-S), 4.07-3.83 (*m*, 2H, N-CH₂-CH*H*-N-C-S, N-CH₂-CH*H*-N-C-S), 3.51 (s, 2H, C-C*H*₂-N), 3.21 (*d*, ³J = 6.9 Hz, 2H, S-C*H₂*-CH), 2.58-2.44 *(m*, 4H, N-C*H₂*-CH₂-N-C-S, N-C*H₂*-CH₂-N-C-S), 2.34 (*s*, 3H, C-C*H₃*), 1.89-1.79 (*m*, 2H, C*H*_{(Cyclohexyl)}), 1.77-1.55 (*m*, 3H C*H*_{(Cyclohexyl)}, S-CH₂-C*H*), 1.31-1.07 (*m*, 4H, C*H*_{(Cyclohexyl)}), 1.05-0.92 (*m*, 4H, C*H*_{(Cyclohexyl)}). | | | |
| **¹³C**-**NMR:** | (CDCl₃, 100 MHz), δ [ppm] = 197.7 (C=S), 137.2 (N-CH₂-C), 129.3 (N-CH₂-C-CH-CH-C, N-CH₂-C-CH-CH-C), 129.2 (N-CH₂-C-CH-CH-C, N-CH₂-C-CH-CH-C), 125.0 (N-CH₂-C-CH-CH-C), 62.3 (C-CH₂-N), 52.4 (N-CH₂-CH₂-N, N-CH₂-CH₂-N), 44.5 (S-CH₂-CH), 37.3 (S-CH₂-CH), 33.0 (CH_{(Cyclohexyl)}), 26.4 (CH_{(Cyclohexyl)}), 26.1 (CH_{(Cyclohexyl)}), 21.2 (C-CH₃). | | | |
| **IR (ATR):** | v (cm⁻¹) = 2998 (w), 2926 (s), 2884 (w), 1616 (w), 1514 (m), 1470 (s), 1456 (s), 1443 (w), 1413 (w), 1387 (w), 1356 (w), 1344 (w), 1315 (m), 1298 (m), 1269 (w), 1252 (vs), 1217 (w), 1180 (w), 1134 (s), 1117 (m), 1099 (m), 1069 (w), 1052 (vs), 1016 (vs), 990 (s), 971 (w), 926 (w), 916 (w), 849 (w), 812 (m), 780 (s), 764 (w), 736 (w), 708 (w), 644 (w), 632 (w), 572 (w), 535 (w), 494 (m), 476 (m), 458 (w), 446 (w), 436 (w). | | | |
| **MS** (ESI+): | m/z (%) = 363 (100, [M+H]⁺) | | | |
| **HRMS** (ESI+): | m/z für C₂₀H₃₀N₂S₂ | | | |
| | ber.: | 362.1850 | | gef.: 362.1886 |
| **EA:** | ber.: | C: 66.25 | H: 8.34 | N: 7.73 |
| | gef.: | C: 66.21 | H: 8.38 | N: 7.60 |

### Darstellung von (7-Methoxy-2-oxochromen-4-yl)methyl-4-(p-tolylmethyl)-piperazin-1-carbodithioat (Schl32296):

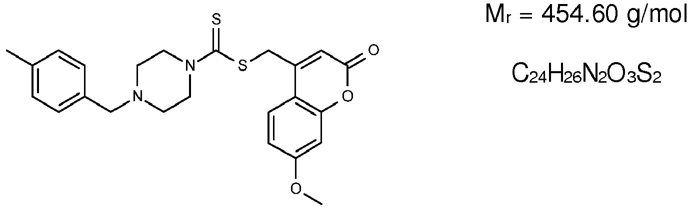

Gemäß **AAV8** wurden 381 mg 1-(*p*-Tolylmethyl)piperazin (2.0 mmol, 1.0 eq), 0.84 mL Triethylamin (6.0 mmol, 3.0 eq), 0.22 mL Kohlenstoffdisulfid (6.0 mmol, 3.0 eq) und 538 mg 4-(Brommethyl)-7-methoxychromen-2-on (2.0 mmol, 1.0 eq) umgesetzt. Säulenchromatographie an Kieselgel (Cyclohexan/ EtOAc 2:1) ergab 685 mg von **Schl32296** (75.3%) in Form eines blassgelben Feststoffes.

| | | | | |
|---|---|---|---|---|
| **DC:** | R_{f} = 0.17 (Cyclohexan/ EtOAc 2:1). | | | |
| **Fp.:** | 141 °C | | | |
| **¹H**-**NMR:** | (CDCl₃, 400 MHz), δ [ppm] = 7.61 (*d*, ³J = 8.7 Hz, 1H, C-C*H*-CH-C-O-CH₃), 7.19 (*d,* ³J = 7.6 Hz, 2H, C-CH-C*H*-C-CH₂-N, C-CH-C*H*-C-CH₂-N), 7.13 (*d*, ³J = 7.8 Hz, 2H, C-C*H*-CH-C-CH₂-N, C-C*H*-CH-C-CH₂-N), 6.89-6.85 (*m*, 1H, C-CH-C*H*-C-O-CH₃,), 6.83-6.77 (*m*, 1H, C-C*H*-C-O-CH₃), 6.39 (*s*, 1H, C-C*H*-C=O), 4.70 (*s*, 2H, S-C*H₂*-C), 4.42-4.28 (*m*, 2H, N-CH₂-CH*H*-N-C-S, N-CH₂-CH*H*-N-C-S), 3.95-3.87 (*m*, 2H, N-CH₂-CH*H*-N-C-S, N-CH₂-CH*H*-N-C-S), 3.86 (*s*, 3H, O-C*H₃*), 3.46 (*s*, 2H, C-C*H₂*-N), 2.60-2.44 (*m*, 4H, N-C*H₂*-CH₂-N-C-S, N-C*H₂*-CH₂-N-C-S), 2.33 (*s*, 3H, C-C*H₃*). | | | |
| **¹³C**-**NMR:** | (CDCl₃, 100 MHz), δ [ppm] = 194.0 (C=S), 162.9 (C(=O)-O), 161.1 (C-OMe), 155.7 (C(=O)-O-C), 150.4 (C(=O)-C), 137.3 (N-CH₂-C), 135.9 (S-CH₂-C), 134.2 (N-CH₂-C-CH-CH-C), 129.2 (N-CH₂-C-CH-CH-C, N-CH₂-C-CH-CH-C), 129.1 (N-CH₂-C-CH-CH-C, N-CH₂-C-CH-CH-C), 125.7 (C-CH-CH-C-OMe), 112.9 (C-CH-C(=O)), 112.2 (C-CH-CH-C-OMe), 101.2 (C-CH-C-OMe), 62.2 (C-CH₂-N), 55.9 (O-CH₃), 52.3 (N-CH₂-CH₂-N, N-CH₂-CH₂-N), 37.6 (S-CH₂-C), 21.2 (C-CH₃). | | | |
| **IR (ATR):** | v (cm⁻¹) = 3019 (w), 2998 (w), 2937 (w), 1732 (s), 1614 (s), 1558 (m), 1514 (s), 1473 (m), 1434 (w), 1386 (w), 1372 (w), 1349 (m), 1310 (s), 1299 (s), 1286 (w), 1204 (w), 1175 (s), 1138 (s), 1053 (w), 1034 (m), 1020 (w), 1000 (w), 984 (vs), 938 (w), 921 (w), 905 (m), 869 (vs), 835 (m), 822 (m), 806 (m), 746 (w), 697 (w), 679 (w), 611 (w), 533 (w), 486 (s). | | | |
| **MS** (ESI+): | m/z (%) = 455 (100, [M+H]⁺) | | | |
| **HRMS** (ESI+): | m/z für C₂₄H₂₆N₂O₃S₂ | | | |
| | ber.: | 454.1385 | | gef.: 454.1397 |
| **EA:** | ber.: | C: 63.41 | H: 5.76 | N: 6.16 |
| | gef.: | C: 63.01 | H: 5.78 | N: 6.21 |

### Darstellung von 2-(1H-indol-3-yl)ethyl-4-(p-tolylmethyl)piperazin-1-carbodithioat (Schl32297):

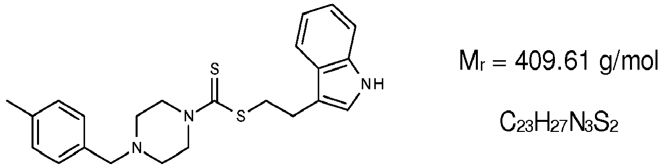

Gemäß **AAV8** wurden 381 mg 1-(*p*-Tolylmethyl)piperazin (2.0 mmol, 1.0 eq), 0.84 mL Triethylamin (6.0 mmol, 3.0 eq), 0.22 mL Kohlenstoffdisulfid (6.0 mmol, 3.0 eq) und 448 mg 3-(2-Bromethyl)indol (2.0 mmol, 1.0 eq) umgesetzt. Säulenchromatographie an Kieselgel (Cyclohexan/ EtOAc 2:1) ergab 729 mg von **Schl32297** (89.0%) in Form eines braungelben Feststoffes.

| | | | | |
|---|---|---|---|---|
| **DC:** | R_{f} = 0.21 (Cyclohexan/ EtOAc 2:1). | | | |
| **Fp.:** | 178 °C | | | |
| **¹H**-**NMR:** | (CDCl₃, 400 MHz), δ [ppm] = 8.16 (*sbr,* 1H, N*H*), 7.74 (*d*, ³J = 7.8 Hz, CH₂-C-C-C*H*), 7.34 (*d*, ³J = 8.0 Hz, N-C-C*H*), 7.23-7.11 (m, 6H, C-CH-C*H*-CH-CH, C-CH-CH-CH-CH, C-CH-C*H*-C-CH₂-N, C-CH-C*H*-C-CH₂-N, C-CH-C*H*-C-CH₂-N, C-CH-C*H*-C-CH₂-N), 7.05 (s, 1H, C-C*H*-NH), 4.46-4.32 (*m***,** 2H, N-CH₂-CH*H*-N-C-S, N-CH₂-CH*H*-N-C-S), 4.00-3.82 *(m***,** 2H, N-CH₂-CH*H*-N-C-S, N-CH₂-CH*H*-N-C-S), 3.66 (*t*, ³J = 7.7 Hz, 2H, S-C*H₂*-CH₂-C), 3.50 (s, 2H, C-C*H₂*-N), 3.19 (*t*, ³J = 7.7 Hz, 2H, S-CH₂-C*H₂*-C), 2.58-2.44 (*m***,** 4H, N-C*H₂*-CH₂-N-C-S, N-C*H₂*-CH₂-N-C-S), 2.36 (s, 3H, C-C*H₃*). | | | |
| **¹³C**-**NMR:** | (CDCl₃, 100 MHz), δ [ppm] = 197.0 (C=S), 137.2 (N-CH₂-C), 136.4 (NH-C-CH), 134.3 (N-CH₂-C-CH-CH-C), 129.4 (N-CH₂-C-CH-CH-C, N-CH₂-C-CH-CH-C), 129.2 (N-CH₂-C-CH-CH-C, N-CH₂-C-CH-CH-C), 127.4 (NH-C-C), 122.2 (C-CH-NH), 122.1 (NH-C-CH-CH), 119.5 (NH-C-CH-CH-CH), 119.2 (NH-C-C-CH), 114.7 (NH-CH-C), 111.3 (NH-C-CH), 62.3 (C-CH₂-N), 52.5 (N-CH₂-CH₂-N, N-CH₂-CH₂-N), 49.9 (N-CH₂-CH₃), 37.6 (S-CH₂-CH₂), 25.1 (S-CH₂-CH₂), 21.3 (C-CH₃). | | | |
| **IR (ATR):** | v (cm⁻¹ ) = 3052 (w), 2918 (w), 2812 (w), 2768 (w), 2115 (w), 1905 (w), 1619 (w), 1553 (w), 1514 (w), 1457 (m), 1419 (vs), 1342 (w), 1298 (w), 1274 (m), 1223 (vs), 1188 (m), 1138 (m), 1094 (w), 1064 (w), 1024 (m), 1010 (m), 991 (vs), 923 (m), 847 (w), 811 (m), 783 (m), 739 (vs), 635 (w), 578 (m), 552 (w), 530 (w), 489 (s), 475 (s), 422 (vs). | | | |
| **MS** (ESI+): | m/z (%) = 410 (100, [M+H]⁺) | | | |
| **HRMS** (ESI+): | m/z für C₂₃H₂₇N₃S₂ | | | |
| | ber.: | 409.1646 | | gef.: 409.1669 |
| **EA:** | ber.: | C: 67.44 | H: 6.64 | N: 10.26 |
| | gef.: | C: 67.40 | H: 6.62 | N: 10.05 |

### Darstellung von (4-Nitrophenyl)methyl-4-(cyclopropancarbonyl)piperazin-1-carbodithioat (Schl32298):

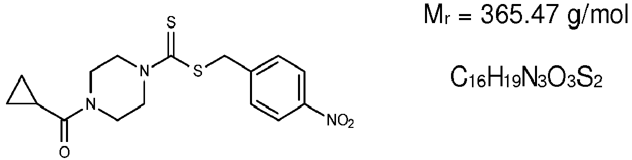

Gemäß **AAV10** wurden 381 mg Cyclopropyl(piperazin-1-yl)methanon (2.0 mmol, 1.0 eq), 424 mg Natriumcarbonat (4.0 mmol, 2.0 eq), 0.15 mL Kohlenstoffdisulfid (4.0 mmol, 2.0 eq) und 432 mg 1-(Brommethyl)-4-nitrobenzen (2.0 mmol, 1.0 eq) umgesetzt. Säulenchromatographie an Kieselgel (Cyclohexan/ EtOAc 1:1) ergab 657 mg von **Schl32298** (89.9%) in Form eines blassgelben Feststoffes.

| | | | | |
|---|---|---|---|---|
| **DC:** | R_{f} = 0.20 (Cyclohexan/ EtOAc 1:1) | | | |
| **Fp.:** | 137 °C | | | |
| **¹H**-**NMR:** | (CDCl₃, 400 MHz), δ [ppm] = 8.12 (*d*, ³J = 8.7 Hz, 2H, C-CH-C*H*-C-(NO₂), C-CH-C*H-*C-(NO₂)), 7.54 (*d*, ³J = 8.7 Hz, 2H, C-C*H*-CH-C-(NO₂), C-C*H*-CH-C-(NO₂)), 4.67 (*s*, 2H, S-C*H₂*-C), 4.49-4.25 (*m*, 2H, C(=O)-N-CH*H*-CH₂-N, C(=O)-N-CH*H*-CH₂-N), 4.07-3.87 (*m*, 2H, C(=O)-N-CH*H*-CH₂-N, C(=O)-N-CH*H*-CH₂-N), 3.81-3.68 (*m*, 4H, C(=O)-N-CH₂-C*H₂*-N, C(=O)-N-CH₂-C*H₂*-N), 1.73-1.65 (*m*, 1H, C*H*_{(Cyclopropyl)}), 1.02-0.94 (*m*, 2H, CH*H*_{(Cyclopropyl)}-CH*H*_{(Cyclopropyl)}), 0.82-0.73 (*m*, 2H, CH*H*_{(Cyclopropyl)}-CH*H*_{(Cyclopropyl)}). | | | |
| **¹³C**-**NMR:** | (CDCl₃, 100 MHz), δ [ppm] = 195.9 (C=S), 172.6 (C=O), 147.2 (C(NO₂)), 144.5 (C(=O)-C), 140.6 (CH₂-C-CH-CH), 130.2 (C(NO₂)-CH-CH, C(NO₂)-CH-CH), 123.8 (C-CH-CH, C-CH-CH), 60.5 (N-CH₂-CH₂-N, N-CH₂-CH₂-N), 40.5 (S-CH₂-C), 11.1 (CH_{2(Cyclopropyl)}-CH_{2(Cyclopropyl)}), 8.0 (CH_{(Cyclopropyl)}). | | | |
| **IR (ATR):** | v (cm⁻¹) = 3110 (w), 3079 (w), 3002 (w), 2981 (w), 1616 (m), 1601 (w), 1509 (s), 1461 (w), 1435 (w), 1421 (m), 1407 (m), 1340 (vs), 1316 (w), 1280 (w), 1237 (w), 1218 (s), 1164 (m), 1108 (w), 1068 (w), 1035 (m), 1016 (m), 998 (w), 967 (w), 927 (m), 886 (w), 862 (m), 851 (m), 819 (w), 736 (w), 705 (s), 625 (w), 565 (w), 514 (w), 495 (s), 455 (w). | | | |
| **MS** (ESI+): | m/z (%) = 366 (10, [M+H]⁺), 383 (100, [M+NH₄]⁺) | | | |
| **HRMS** (ESI+): | m/z für C₁₆H₁₉N₃O₃S₂ | | | |
| | ber.: | 365.0868 | | gef.: 365.0862 |
| **EA**: | ber.: | C: 52.58 | H: 5.24 | N: 11.50 |
| | gef.: | C: 52.88 | H: 5.30 | N: 11.65 |

### Darstellung von (4-Methylsulfonylphenyl)methyl-4-(cyclopropancarbonyl)piperazin-1-carbodithioat (Schl32299):

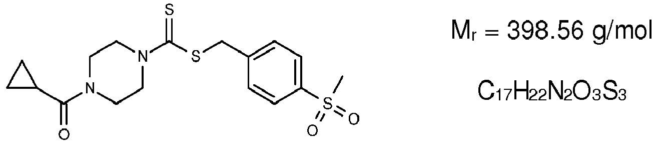

Gemäß **AAV10** wurden 381 mg Cyclopropyl(piperazin-1-yl)methanon (2.0 mmol, 1.0 eq), 424 mg Natriumcarbonat (4.0 mmol, 2.0 eq), 0.15 mL Kohlenstoffdisulfid (4.0 mmol, 2.0 eq) und 498 mg 1-(Brommethyl)-4-methylsulfonylbenzen (2.0 mmol, 1.0 eq) umgesetzt. Säulenchromatographie an Kieselgel (Cyclohexan/ EtOAc 1:4) ergab 346 mg von **Schl32299** (43.4%) in Form eines farblosen Feststoffes.

| | | | | |
|---|---|---|---|---|
| **DC:** | R_{f} = 0.17 (Cyclohexan/ EtOAc 1:4). | | | |
| **Fp**.**:** | 192 °C | | | |
| **¹H-NMR:** | (DMSO-D₆, 400 MHz), δ [ppm] = 7.84 (*d*, ³J = 8.2 Hz, 2H, CH₂-C-CH-C*H*-C-SO₂Me, CH₂-C-CH-C*H*-C-SO₂Me), 7.64 (*d*, ³J = 8.2 Hz, 2H, CH₂-C-C*H*-CH-C-SO₂Me, CH₂-C-C*H*-CH-C-SO₂Me), 4.68 *(s,* 2H, S-C*H₂*-C), 4.36-4.15 (*m,* 2H, C(=O)-N-CH*H*-CH₂-N, C(=O)-N-CH*H*-CH₂-N), 4.04-3.89 (*m*, 2H, C(=O)-N-CH*H*-CH₂-N, C(=O)-N-CH*H*-CH₂-N), 3.83-3.72 (*m*, 2H, C(=O)-N-CH₂-CH*H*-N, C(=O)-N-CH₂-CH*H*-N), 3.64-3.39 (m, 2H, C(=O)-N-CH₂-CH*H*-N, C(=O)-N-CH₂-CH*H*-N), 3.16 (s, 3H, SO₂-C*H₃*), 1.96-1.87 (m, 1H, C*H*_{(Cyclopropyl)}), 0.75-0.67 (m, 4H, C*H₂*(Cyclopropyl)-C*H*_{*2*(Cyclopropyl)}). | | | |
| **¹³C-NMR:** | (DMSO-D₆, 100 MHz), δ [ppm] = 195.0 (C=S), 172.0 (C=O), 143.6 (C-SO₂Me), 140.2 (CH₂-C-CH-CH), 130.5 (C-SO₂Me-CH-CH, C-SO₂Me-CH-CH), 127.6 (C-CH-CH, C-CH-CH), 51.4 (N-CH₂-CH₂-N, N-CH₂-CH₂-N), 44.1 (S-CH₂-C), 11.0 (CH_{2(Cyclopropyl)}-CH_{2(Cyclopropyl)}), 7.8 (CH_{(Cyclopropyl)}). | | | |
| **IR (ATR):** | v (cm⁻¹) = 3029 (w), 2996 (w), 2917 (w), 1622 (s), 1596 (w), 1516 (w), 1464 (s), 1434 (m), 1408 (w), 1319 (s), 1292 (s), 1278 (w), 1255 (s), 1238 (m), 1217 (w), 1161 (w), 1141 (vs), 1114 (w), 1087 (m), 1026 (s), 1001 (s), 921 (m), 874 (s), 763 (vs), 734 (m), 625 (w), 545 (s), 518 (vs), 510 (vs), 457 (m), 409 (w). | | | |
| **MS** (ESI+): | m/z (%) = 416 (100, [M+NH₄]⁺) | | | |
| **HRMS** (ESI+): | m/z für C₁₇H₂₂N₂O₃S₃ | | | |
| | ber.: | 398.0792 | | gef.: 398.0785 |
| **EA:** | ber.: | C: 51.23 | H: 5.56 | N: 7.03 |
| | gef.: | C: 51.25 | H: 5.61 | N: 7.14 |

### Darstellung von (4-Cyanophenyl)methyl-4-(cyclopropancarbonyl)piperazin-1-carbodithioat (Schl32300):

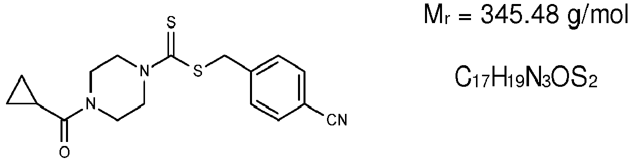

Gemäß **AAV10** wurden 381 mg Cyclopropyl(piperazin-1-yl)methanon (2.0 mmol, 1.0 eq), 424 mg Natriumcarbonat (4.0 mmol, 2.0 eq), 0.15 mL Kohlenstoffdisulfid (4.0 mmol, 2.0 eq) und 392 mg 4-(Brommethyl)benzonitril (2.0 mmol, 1.0 eq) umgesetzt. Säulenchromatographie an Kieselgel (Cyclohexan/EtOAc 1:1) ergab 641 mg von **Schl32300** (92.8%) in Form eines farblosen Feststoffes.

| | | | | |
|---|---|---|---|---|
| **DC:** | R_{f} = 0.22 (Cyclohexan/ EtOAc 1:1). | | | |
| **Fp**.**:** | 128 °C | | | |
| **¹H-NMR:** | (CDCl₃, 400 MHz), δ [ppm] = 7.58 (*d*, ³J = 8.2 Hz, 2H, C-CH-C*H*-C-CN, C-CH-C*H*-C-CN), 7.49 (*d*, ³J = 8.2 Hz, 2H, C-C*H*-CH-C-CN, C-C*H*-CH-C-CN), 4.64 *(s,* 2H, S-C*H₂*-C), 4.48-4.29 (*m*, 2H, C(=O)-N-CH*H*-CH₂-N, C(=O)-N-CH*H*-CH₂-N), 4.10-3.89 (*m*, 2H, C(=O)-N-CH*H*-CH₂-N, C(=O)-N-CH*H*-CH₂-N), 3.86-3.70 *(m,* 4H, C(=O)-N-CH₂-C*H₂*N, C(=O)-N-CH₂-C*H₂-*N), 1.74-1.65 (*m*, 1H, C*H*_{(Cyclopropyl)}), 1.04-0.96 *(m,* 2H, CH*H*_{(Cyclopropyl)}-CH*H*_{(Cyclopropyl)}), 0.84-0.77 (*m*, 2H, CH*H*_{(Cyclopropyl)}-CH*H*_{(Cyclopropyl)}). | | | |
| **¹³C-NMR:** | (CDCl₃, 100 MHz), δ [ppm] = 196.2 (C=S), 172.6 (C=O), 142.3 (C-CH-CH-C-CN), 132.4 (C-CH-CH-C-CN, C-CH-CH-C-CN), 130.1 (C-CH-CH-C-CN, C-CH-CH-C-CN), 118.8 (C-CN), 111.4 (C-CN), 54.4 (N-CH₂-CH₂-N, N-CH₂-CH₂-N), 40.9 (S-CH₂-C), 11.2 (CH₂(cyclopropyl)-CH_{2Ccyclopropyl)}), 8.0 (CH_{(Cyclopropyl)}). | | | |
| **IR (ATR):** | v (cm⁻¹) = 3051 (w), 3008 (w), 2856 (w), 1628 (s), 1606 (m), 1505 (w), 1472 (s), 1433 (s), 1416 (vs), 1367 (w), 1282 (m), 1248 (w), 1219 (vs), 1159 (s), 1055 (w), 1027 (m), 1013 (m), 995 (vs), 927 (s), 892 (w), 873 (m), 860 (m), 841 (m), 824 (m), 756 (w), 736 (w), 724 (w), 694 (w), 635 (w), 601 (w), 556 (s), 534 (w), 521 (m), 447 (w), 419 (w). | | | |
| **MS** (ESI+): | m/z (%) = 363 (100, [M+NH₄]⁺) | | | |
| **HRMS** (ESI+): | m/z für C₁₇H₁₉N₃OS₂ | | | |
| | ber.: | 345.0970 | | gef.: 345.0961 |
| **EA:** | ber.: | C: 59.10 | H: 5.54 | N: 12.16 |
| | gef.: | C: 59.26 | H: 5.66 | N: 12.05 |

### Darstellung von (2-Hydroxy-5-nitrophenyl)methyl-4-(cyclopropancarbonyl)-piperazin-1-carbodithioat (Schl32301):

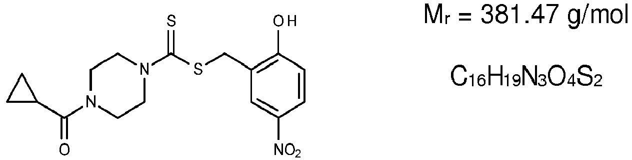

Gemäß **AAV10** wurden 381 mg Cyclopropyl(piperazin-1-yl)methanon (2.0 mmol, 1.0 eq), 424 mg Natriumcarbonat (4.0 mmol, 2.0 eq), 0.15 mL Kohlenstoffdisulfid (4.0 mmol, 2.0 eq) und 464 mg 2-Hydroxy-5-nitrobenzylbromid (2.0 mmol, 1.0 eq) umgesetzt. Säulenchromatographie an Kieselgel (Cyclohexan/ EtOAc 1:4) ergab 631 mg von **Schl32301** (82.9%) in Form eines gelber Feststoffes.

| | | | | |
|---|---|---|---|---|
| **DC:** | R_{f} = 0.29 (Cyclohexan/ EtOAc 1:4) | | | |
| **Fp**.**:** | 168 °C | | | |
| **¹H-NMR:** | (DMSO-D₆, 400 MHz), δ [ppm] = 8.25 (s, 1H, C-C*H*-C(NO₂)), 8.02 (*d*, ³J = 9.0 Hz, 1H, C(NO₂)-C*H*-CH-C-OH), 6.95 (*d*, ³J = 9.1 Hz, 1H, C(NO₂)-CH-C*H*-C-OH), 4.52 *(s,* 2H, S-C*H₂*-C), 4.33-4.14 (*m*, 2H, C(=O)-N-CH*H*-CH₂-N, C(=O)-N-CH*H*-CH₂-N), 4.02-3.81 (*m*, 2H, C(=O)-N-CH*H*-CH₂-N, C(=O)-N-CH*H*-CH₂-N), 3.75-3.50 (m, 4H, C(=O)-N-CH₂-C*H₂-*N, C(=O)-N-CH₂-C*H₂-*N), 1.95-1.84 (*m*, 1H, C*H*_{(Cyclopropyl)}), 0.75-0.63 (*m*, 4H, C*H*_{*2*(Cyclopropyl)}-C*H*_{*2*(Cyclopropyl)}). | | | |
| **¹³C-NMR:** | (DMSO-D₆, 100 MHz), δ [ppm] = 195.6 (C=S), 171.9 (C=O), 163.5 (C-OH), 139.9 (C-NO₂), 126.9 (C(NO₂)-CH-CH-C), 125.8 (C-CH-C(NO₂)), 124.4 (S-CH₂-C), 115.9 (C(NO₂)-CH-CH-C), 56.6 (N-CH₂-CH₂-N, N-CH₂-CH₂-N), 44.2 (S-CH₂-C), 10.9 (CH_{2(Cyclopropyl)}-CH_{2(Cyclopropyl)}), 7.8 (CH_{(Cyclopropyl)}). | | | |
| **IR (ATR):** | v (cm⁻¹) = 3013 (w), 2866 (w), 2731 (w), 1617 (m), 1576 (w), 1521 (m), 1470 (w), 1422 (w), 1390 (w), 1331 (vs), 1289 (vs), 1240 (s), 1216 (vs), 1159 (w), 1139 (w), 1121 (w), 1081 (m), 1032 (w), 1012 (m), 994 (s), 907 (w), 863 (w), 830 (s), 799 (w), 752 (m), 742 (w), 690 (w), 659 (w), 637 (w), 601 (w), 563 (w), 526 (m), 474 (w), 451 (w), 433 (w), 409 (w). | | | |
| **MS** (ESI+): | m/z (%) = 382 (100, [M+H]⁺) | | | |
| **HRMS** (ESI+): | m/z für C₁₆H₁₉N₃O₄S₂ | | | |
| | ber.: | 381.0817 | | gef.: 381.0830 |
| **EA:** | ber.: | C: 50.38 | H: 5.02 | N: 11.02 |
| | gef.: | C: 50.31 | H: 5.01 | N: 11.38 |

### Darstellung von Cyclohexylmethyl-4-(cyclopropancarbonyl)piperazin-1-carbodithioat (Schl32302):

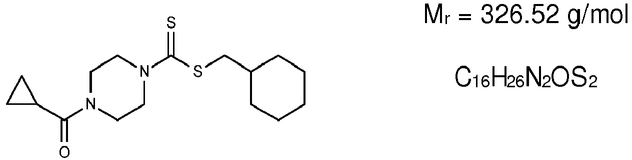

Gemäß **AAV10** wurden 381 mg Cyclopropyl(piperazin-1-yl)methanon (2.0 mmol, 1.0 eq), 424 mg Natriumcarbonat (4.0 mmol, 2.0 eq), 0.15 mL Kohlenstoffdisulfid (4.0 mmol, 2.0 eq) und 0.28 mL Brommethylcyclohexan (2.0 mmol, 1.0 eq) umgesetzt. Säulenchromatographie an Kieselgel (Cyclohexan/EtOAc 1:1) ergab 561 mg von **Schl32302** (85.9%) in Form eines farblosen Feststoffes.

| | | | | |
|---|---|---|---|---|
| **DC:** | R_{f} = 0.41 (Cyclohexan/ EtOAc 1:1). | | | |
| **Fp**.**:** | 102 °C | | | |
| **¹H-NMR:** | (CDCl₃, 400 MHz), δ [ppm] = 4.46-4.25 (*m,* 2H, C(=O)-N-CH*H*-CH₂-N, C(=O)-N-CH*H*-CH₂-N), 4.20-3.91 (*m,* 2H, C(=O)-N-CH*H*-CH₂-N, C(=O)-N-CH*H*-CH₂-N), 3.80-3.69 (*m,* 4H, C(=O)-N-CH₂-C*H₂*-N, C(=O)-N-CH₂-C*H₂*-N), 3.20 (*d*, ³J = 6.9 Hz, 2H, S-C*H₂*-CH), 2.02-1.94 (*m,* 2H, S-CH₂-C*H*, C*H*_{(Cyclopropyl)}), 1.87-1.77 (*m,* 2H, C*H*_{(Cyclohexyl)}), 1.72-1.56 (m, 4H, C*H*_{(Cyclohexyl)}), 1.26-1.11 (*m,* 3H, C*H*_{(Cyclohexyl)}), 1.01-0.92 (*m,* 3H, C*H*_{(Cyclohexyl)}, CH*H*_{(Cyclopropyl)}-CH*H*_{(Cyclopropyl)}), 0.81-0.73 (*m,* 2H, CH*H*_{(Cyclopropyl)}-CH*H*_{(Cyclopropyl)}). | | | |
| **¹³C-NMR:** | (CDCl₃, 100 MHz), δ [ppm] = 198.7 (C=S), 172.6 (C=O), 60.5 (N-CH₂-CH₂-N, N-CH₂-CH₂-N), 44.5 (S-CH₂-CH), 37.2 (S-CH₂-CH), 32.9 (CH_{(Cyclohexyl)}), 26.3 (CH_{(Cyclohexyl)}), 26.1 (CH_{(Cyclohexyl)}), 11.2 (CH_{2(Cyclopropyl)}-CH_{2(Cyclopropyl)}), 8.0 (CH_{(Cyclopropyl)}). | | | |
| **IR (ATR):** | v (cm⁻¹) = 3083 (w), 3014 (w), 2921 (m), 1628 (s), 1457 (s), 1438 (m), 1410 (vs), 1362 (w), 1345 (w), 1280 (m), 1239 (m), 1218 (vs), 1206 (s), 1163 (s), 1091 (m), 1060 (s), 1048 (s), 1027 (w), 1014 (w), 1001 (w), 963 (w), 925 (s), 893 (w), 869 (w), 822 (w), 791 (w), 735 (w), 639 (w), 559 (w), 551 (w), 511 (m), 474 (w), 447 (w), 401 (w). | | | |
| **MS** (ESI+): | m/z (%) = 327 (10, [M+H]⁺), 344 (100, [M+NH₄]⁺) | | | |
| **HRMS** (ESI+): | m/z für C₁₆H₂₆N₂OS₂ | | | |
| | ber.: | 326.1487 | | gef.: 326.1483 |
| **EA:** | ber.: | C: 58.85 | H: 8.03 | N: 8.58 |
| | gef.: | C: 59.10 | H: 8.05 | N: 8.52 |

### Darstellung von (7-Methoxy-2-oxochromen-4-yl)methyl-4-(cyclopropancarbonyl)-piperazin-1-carbodithioat (Schl32303):

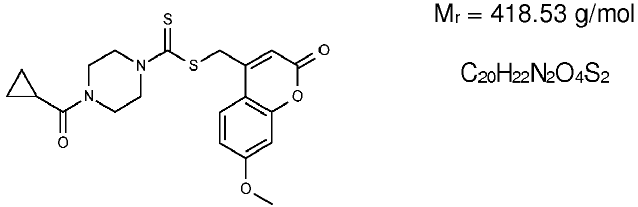

Gemäß **AAV10** wurden 381 mg Cyclopropyl(piperazin-1-yl)methanon (2.0 mmol, 1.0 eq), 424 mg Natriumcarbonat (4.0 mmol, 2.0 eq), 0.15 mL Kohlenstoffdisulfid (4.0 mmol, 2.0 eq) und 538 mg 4-(Brommethyl)-7-methoxychromen-2-on (2.0 mmol, 1.0 eq) umgesetzt. Säulenchromatographie an Kieselgel (Cyclohexan/ EtOAc 1:4) ergab 588 mg von **Schl32303** (70.3%) in Form eines gelben Feststoffes.

| | | | | |
|---|---|---|---|---|
| **DC:** | R_{f} = 0.12 (Cyclohexan/ EtOAc 1:4). | | | |
| **Fp**.**:** | 202 °C | | | |
| **¹H-NMR:** | (CDCl₃, 400 MHz), δ [ppm] = 7.60 (*d*, ³J = 8.7 Hz, 1H, C-C*H*-CH-C-O-CH₃), 6.87 (*dd*, ³J = 8.9 Hz, ²J = 2.5 Hz, 1H, C-CH-C*H*-C-O-CH₃,), 6.84-6.80 (*m,* 1H, C-C*H*-C-O-CH₃), 6.40 (*s,* 1H, C-C*H*-C=O), 4.71 *(s,* 2H, S-C*H₂*-C), 4.50-4.34 (*m,* 2H, C(=O)-N-CH*H*-CH₂-N, C(=O)-N-CH*H*-CH₂-N), 4.10-3.93 (*m,* 2H, C(=O)-N-CH*H*-CH₂-N, C(=O)-N-CH*H*-CH₂-N), 3.86 (s, 3H, O-C*H₃*), 3.81-3.71 (*m,* 4H, C(=O)-N-CH₂-C*H₂*-N, C(=O)-N-CH₂-C*H₂*-N), 1.73-1.63 (*m,* 1H, C*H*_{(Cyclopropyl)}), 1.05-0.94 (*m,* 2H, CH*H*_{(Cyclopropyl)}-CH*H*_{(Cyclopropyl)}), 0.86-0.76 *(m,* 2H, CH*H*_{(Cyclopropyl)}-CH*H*_{(Cyclopropyl)}). | | | |
| **¹³C-NMR:** | (CDCl₃, 100 MHz), δ [ppm] = 196.6 (C=S), 172.7 (C=O), 163.0 (C(=O)-O), 161.0 (C-OMe), 155.7 (S-CH₂-C), 150.1 (C(=O)-O-C), 125.6 (C-CH-CH-C-OMe), 112.9 (C-CH-C(=O)), 112.7 (C-CH-CH-C-OMe), 101.2 (C-CH-C-OMe), 55.9 (O-CH₃), 44.5 (N-CH₂-CH₂-N, N-CH₂-CH₂-N), 37.5 (S-CH₂-C), 11.2 (CH_{2(Cyclopropyl)}-CH_{2(Cyclopropyl)}), 8.1 (CH_{(Cyclopropyl)}). | | | |
| **IR (ATR):** | v (cm⁻¹) = 3104 (w), 3002 (w), 1704 (s), 1638 (m), 1611 (vs), 1557 (w), 1511 (w), 1467 (s), 1422 (w), 1393 (m), 1378 (w), 1332 (w), 1294 (w), 1278 (m), 1264 (m), 1242 (s), 1213 (w), 1143 (vs), 1065 (w), 1028 (w), 1014 (m), 992 (m), 975 (w), 906 (m), 889 (vs), 869 (m), 836 (w), 801 (w), 741 (w), 696 (w), 573 (m), 566 (m), 541 (m), 454 (w), 421 (w). | | | |
| **MS** (ESI+): | m/z (%) = 419 (25, [M+H]⁺), 436 (100, [M+NH₄]⁺) | | | |
| **HRMS** (ESI+): | m/z für C₂₀H₂₂N₂O₄S₂ | | | |
| | ber.: | 418.1021 | | gef.: 418.1016 |
| **EA:** | ber.: | C: 57.39 | H: 5.30 | N: 6.69 |
| | gef.: | C: 57.28 | H: 5.36 | N: 6.61 |

### Darstellung von 2-(1H-Indol-3-yl)ethyl-4-(cyclopropancarbonyl)piperazin-1-carbodithioat (Schl32304):

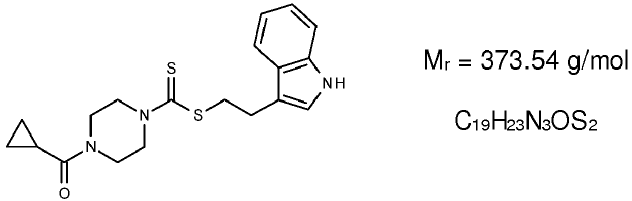

Gemäß **AAV10** wurden 381 mg Cyclopropyl(piperazin-1-yl)methanon (2.0 mmol, 1.0 eq), 424 mg Natriumcarbonat (4.0 mmol, 2.0 eq), 0.15 mL Kohlenstoffdisulfid (4.0 mmol, 2.0 eq) und 448 mg 3-(2-Bromethyl)indol (2.0 mmol, 1.0 eq) umgesetzt. Säulenchromatographie an Kieselgel (Cyclohexan/ EtOAc 1:1) ergab 712 mg von **Schl32304** (95.3%) in Form eines gelben Feststoffes.

| | | | | |
|---|---|---|---|---|
| **DC:** | R_{f} = 0.20 (Cyclohexan/ EtOAc 1:1). | | | |
| **Fp**.**:** | 150 °C | | | |
| **¹H-NMR:** | (CDCl₃, 400 MHz), δ [ppm] = 8.16 (*sbr*, 1H, N*H*), 7.73 (*d*, ³J = 7.8 Hz, CH₂-C-C-C*H*), 7.35 (*d*, ³J = 8.0 Hz, N-C-C*H*), 7.22-7.10 (*m,* 2H, C-CH-C*H*-C*H*-CH), 7.07 *(s,* 1H, C-C*H*-NH), 4.51-4.29 (*m*, 2H, C(=O)-N-CH*H*-CH₂-N, C(=O)-N-CH*H*-CH₂-N), 4.16-3.90 (*m*, 2H, C(=O)-N-CH*H*-CH₂-N, C(=O)-N-CH*H*-CH₂-N), 3.82-3.70 *(m,* 4H, C(=O)-N-CH₂-C*H₂*-N, C(=O)-N-CH₂-C*H₂-*N), 3.67 (*t*, ³J = 7.7 Hz, 2H, S-C*H₂*-CH₂-C), 3.19 (*t*, ³J = 7.5 Hz, 2H, S-CH₂-C*H₂*-C), 1.75-1.66 *(m,* 1H, C*H*_{(Cyclopropyl)}), 1.07-0.99 *(m,* 2H, CH*H*_{(Cyclopropyl)}-CH*H*_{(Cyclopropyl)}), 0.86-0.77 (*m,* 2H, CH*H*_{(Cyclopropyl)}-CH*H*_{(Cyclopropy)}). | | | |
| **¹³C-NMR:** | (CDCl₃, 100 MHz), δ [ppm] = 198.1 (C=S), 172.7 (C=O), 136.3 (NH-C-CH), 127.4 (NH-C-C), 122.2 (C-CH-NH), 122.1 (NH-C-CH-CH), 119.5 (NH-C-CH-CH-CH), 119.1 (NH-C-C-CH), 117.6 (C(=O)-C-CH), 114.6 (NH-CH-C), 111.3 (NH-C-CH), 63.3 (N-CH₂-CH₂-N, N-CH₂-CH₂-N), 37.7 (S-CH₂-CH₂), 25.0 (S-CH₂-CH₂), 11.2 (CH_{2(Cyclopropyl)}-CH_{2(Cyclopropyl)}), 8.0 (CH_{(Cyclopropyl)}). | | | |
| **IR (ATR):** | v (cm⁻¹) = 3063 (w), 3002 (w), 2915 (w), 1628 (m), 1459 (w), 1423 (s), 1387 (w), 1353 (w), 1280 (w), 1238 (w), 1220 (vs), 1205 (m), 1163 (w), 1137 (w), 1118 (w), 1100 (w), 1065 (s), 1032 (w), 1005 (w), 956 (m), 919 (w), 867 (w), 814 (w), 797 (w), 777 (w), 760 (vs), 742 (m), 729 (m), 674 (w), 639 (w), 579 (w), 563 (w), 511 (m), 464 (w), 425 (m), 405 (w). | | | |
| **MS** (ESI+): | m/z (%) = 374 (50, [M+H]⁺), 391 (100, [M+NH₄]⁺) | | | |
| **HRMS** (ESI+): | m/z für C₁₉H₂₃N₃OS₂ | | | |
| | ber.: | 373.1283 | | gef.: 371.1269 |
| **EA:** | ber.: | C: 61.09 | H: 6.21 | N: 11.25 |
| | gef.: | C: 61.28 | H: 6.25 | N: 11.25 |

### Darstellung von (4-Nitrophenyl)methyl-4-[2-(isopropylamino)-2-oxo-ethyl]piperazin-1-carbodithioat (Schl32305):

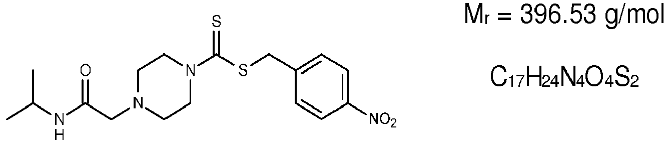

Gemäß **AAV7** wurden 373 mg *N*-Isopropyl-2-piperazin-1-yl-acetamid (2.0 mmol, 1.0 eq), 637 mg Kaliumphosphat (3.0 mmol, 1.5 eq), 0.22 mL Kohlenstoffdisulfid (6.0 mmol, 3.0 eq) und 432 mg 1-(Brommethyl)-4-nitrobenzen (2.0 mmol, 1.0 eq) umgesetzt. Säulenchromatographie an Kieselgel (DCM/ MeOH 95:5) ergab 708 mg von **Schl32305** (89.3%) in Form eines braungelben Feststoffes.

| | | | | |
|---|---|---|---|---|
| **DC:** | R_{f} = 0.60 (DCM/ MeOH 95:5). | | | |
| **Fp**.**:** | 124 °C | | | |
| **¹H-NMR:** | (CDCl₃, 400 MHz), δ [ppm] = 8.14 (*d*, ³J = 8.7 Hz, 2H, C-CH-C*H*-C-(NO₂), C-CH-C*H*-C-(NO₂)), 7.54 *(d,* ³J = 8.8 Hz, 2H, C-C*H*-CH-C-(NO₂), C-C*H*-CH-C-(NO₂)), 6.75 (*sbr*, 1H, N*H*), 4.67 (*s,* 2H, S-C*H₂*-C), 4.44-4.22 (*m,* 2H, N-CH*H*-CH₂-N-CH₂), 4.16-4.08 (*m,* 1H, NH-C*H*), 4.06-3.84 *(m,* 2H, N-CH*H*-CH₂-N-CH₂), 3.03 (*s,* 2H, N-C*H₂-*C(=O)), 2.70-2.54 (*m,* 4H, N-CH₂-C*H₂*-N-CH₂, N-CH₂-C*H₂*-N-CH₂), 1.16 (*d*, ³J = 6.4 Hz, 6H, C*H₃*-CH-C*H₃*). | | | |
| **¹³C-NMR:** | (CDCl₃, 100 MHz), δ [ppm] = 195.8 (C=S), 168.1 (C=O), 147.3 (C(NO₂)) 144.5 (CH₂-C-CH-CH), 130.2 (C(NO₂)-CH-CH, C(NO₂)-CH-CH), 123.8 (C-CH-CH, C-CH-CH), 61.2 (N-CH₂-C), 52.8 (N-CH₂-CH₂-N-CH₂, N-CH₂-CH₂-N-CH₂), 41.0 (N-CH-CH₃), 40.6 (S-CH₂-C), 22.9 (CH-CH₃). | | | |
| **IR (ATR):** | v (cm⁻¹) = 3304 (w), 3071 (w), 2975 (w), 2955 (w), 1648 (s), 1603 (w), 1544 (m), 1517 (vs), 1474 (s), 1463 (s), 1427 (s), 1382 (w), 1365 (w), 1346 (m), 1289 (w), 1265 (vs), 1208 (m), 1149 (w), 1105 (m), 1085 (m), 1054 (m), 1033 (m), 983 (w), 964 (w), 868 (m), 860 (m), 770 (w), 723 (w), 648 (w), 602 (m), 565 (w), 531 (w), 497 (w), 422 (w). | | | |
| **MS** (ESI+): | m/z (%) = 397 (100, [M+H]⁺) | | | |
| **HRMS** (ESI+): | m/z für C₁₇H₂₄N₄O₄S₂ | | | |
| | ber.: | 396.1290 | | gef.: 396.1309 |
| **EA:** | ber.: | C: 51.49 | H: 6.10 | N: 14.13 |
| | gef.: | C: 51.58 | H: 6.09 | N: 14.02 |

### Darstellung von (4-Methylsulfonylphenyl)methyl-4-[2-(isopropylamino)-2-oxo-ethyl]piperazin-1-carbodithioat (Schl32306):

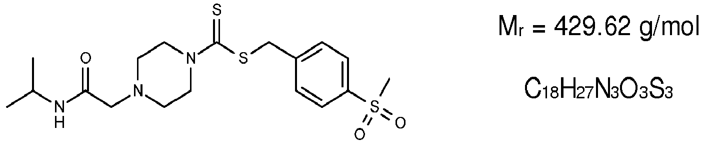

Gemäß **AAV7** wurden 373 mg *N*-Isopropyl-2-piperazin-1-yl-acetamid (2.0 mmol, 1.0 eq), 637 mg Kaliumphosphat (3.0 mmol, 1.5 eq), 0.22 mL Kohlenstoffdisulfid (6.0 mmol, 3.0 eq) und 498 mg 1-(Brommethyl)-4-methylsulfonylbenzen (2.0 mmol, 1.0 eq) umgesetzt. Säulenchromatographie an Kieselgel (DCM/ MeOH 95:5) ergab 761 mg von **Schl32306** (88.6%) in Form eines braunen Feststoffes.

| | | | | |
|---|---|---|---|---|
| **DC:** | R_{f} = 0.51 (DCM/ MeOH 95:5). | | | |
| **Fp**.**:** | 64 °C | | | |
| **¹H-NMR:** | (CDCl₃, 400 MHz), δ [ppm] = 7.85 (*d*, ³J = 7.8 Hz, 2H, CH₂-C-CH-C*H*-C-SO₂Me, CH₂-C-CH-C*H*-C-SO₂Me), 7.57 (*d*, ³J = 8.0 Hz, 2H, CH₂-C-C*H*-CH-C-SO₂Me, CH₂-C-C*H*-CH-C-SO₂Me), 6.73 (*sbr*, 1H, N*H*), 4.65 (*s,* 2H, S-C*H₂*-C), 4.47-4.21 (*m,* 2H, N-CH*H*-CH₂-N-CH₂), 4.14-4.01 (*m,* 1H, NH-C*H*), 3.99-3.82 (*m,* 2H, N-CH*H*-CH₂-N-CH₂), 3.02 (*s,* 3H, SO₂-C*H₃*), 3.01 (*s,* 2H, N-C*H₂-*C(=O)), 2.67-2.55 (*m,* 4H, N-CH₂-C*H₂*-N-CH₂, N-CH₂-C*H₂*-N-CH₂), 1.15 (*d*, ³J = 6.4 Hz, 6H, C*H*₃-CH-C*H₃*). | | | |
| **¹³C-NMR:** | (CDCl₃, 100 MHz), δ [ppm] = 195.9 (C=S), 168.1 (C=O), 142.2 (C-SO₂Me), 139.5 (CH₂-C-CH-CH), 130.3 (C-SO₂Me-CH-CH, C-SO₂Me-CH-CH), 127.7 (C-CH-CH, C-CH-CH), 61.3 (N-CH₂-C), 52.8 (N-CH₂-CH₂-N-CH₂, N-CH₂-CH₂-N-CH₂), 45.9 (N-CH-CH₃), 44.6 (SO₂-CH₃), 41.0 (S-CH₂-C), 22.9 (CH-CH₃). | | | |
| **IR (ATR):** | v (cm⁻¹) = 3342 (w), 2972 (w), 2924 (w), 2823 (w), 1656 (s), 1597 (w), 1520 (m), 1467 (w), 1424 (s), 1366 (w), 1300 (vs), 1261 (w), 1225 (s), 1184 (w), 1144 (vs), 1089 (m), 996 (m), 958 (s), 923 (w), 857 (w), 802 (w), 765 (s), 698 (w), 640 (m), 595 (w), 521 (vs), 475 (w), 419 (w). | | | |
| **MS** (ESI+): | m/z (%) = 430 (100, [M+H]⁺) | | | |
| **HRMS** (ESI+): | m/z für C₁₈H₂₇N₃O₃S₃ | | | |
| | ber.: | 429.1215 | | gef.: 429.1242 |
| **EA:** | ber.: | C: 50.32 | H: 6.33 | N: 9.78 |
| | gef.: | C: 50.08 | H: 6.51 | N: 9.95 |

### Darstellung von (4-Cyanophenyl)methyl-4-[2-(isopropylamino)-2-oxo-ethyl]piperazin-1-carbodithioat (Schl32307):

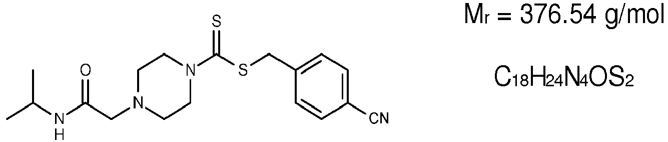

Gemäß **AAV7** wurden 373 mg *N*-Isopropyl-2-piperazin-1-yl-acetamid (2.0 mmol, 1.0 eq), 637 mg Kaliumphosphat (3.0 mmol, 1.5 eq), 0.22 mL Kohlenstoffdisulfid (6.0 mmol, 3.0 eq) und 392 mg 4-(Brommethyl)benzonitril (2.0 mmol, 1.0 eq) umgesetzt. Säulenchromatographie an Kieselgel (DCM/MeOH 95:5) ergab 705 mg von **Schl32307** (93.6%) in Form eines gelben Feststoffes.

| | | | | |
|---|---|---|---|---|
| **DC:** | R_{f} = 0.63 (DCM/MeOH 95:5) | | | |
| **Fp**.**:** | 102 °C | | | |
| **¹H-NMR:** | (CDCl₃, 400 MHz), δ [ppm] = 7.58 (*d*, ³J = 8.0 Hz, 2H, C-CH-C*H*-C-CN, C-CH-C*H*-C-CN), 7.48 (*d*, ³J = 8.0 Hz, 2H, C-C*H*-CH-C-CN, C-CH-CH-C-CN), 6.74 (*sbr*, 1 H, N*H*), 4.62 *(s,* 2H, S-C*H₂*-C), 4.43-4.21 *(m,* 2H, N-CH*H*-CH₂-N-CH₂), 4.14-4.05 *(m,* 1 H, NH-C*H*), 4.02-3.81 *(m,* 2H, N-CH*H*-CH₂-N-CH₂), 3.02 *(s,* 2H, N-C*H₂-*C(=O)), 2.65-2.51 *(m,* 4H, N-CH₂-C*H₂*-N-CH₂, N-CH₂-C*H₂*-N-CH₂), 1.16 *(d,* ³J = 6.4 Hz, 6H, C*H₃*-CH-C*H₃*). | | | |
| **¹³C-NMR:** | (CDCl₃, 100 MHz), δ [ppm] = 195.9 (C=S), 168.2 (C=O), 142.3 (C-CH-CH-C-CN), 132.4 (C-CH-CH-C-CN, C-CH-CH-C-CN), 130.1 (C-CH-CH-C-CN, C-CH-CH-C-CN), 118.8 (C-CN), 111.3 (C-CN), 61.2 (N-CH₂-C), 52.8 (N-CH₂-CH₂-N-CH₂, N-CH₂-CH₂-N-CH₂), 45.9 (N-CH-CH₃), 41.0 (S-CH₂-C), 22.9 (CH-CH₃). | | | |
| **IR (ATR):** | v (cm⁻¹) = 3269 (m), 3082 (w), 2974 (w), 1645 (vs), 1606 (w), 1553 (m), 1501 (w), 1465 (s), 1434 (s), 1423 (s), 1386 (w), 1365 (w), 1299 (m), 1250 (m), 1219 (vs), 1171 (m), 1140 (s), 1131 (s), 1056 (w), 1043 (w), 1012 (s), 993 (s), 917 (m), 854 (m), 826 (w), 756 (w), 733 (m), 659 (w), 621 (w), 556 (m), 549 (m), 535 (w), 487 (w), 433 (w). | | | |
| **MS** (ESI+): | m/z (%) = 377 (100, [M+H]⁺) | | | |
| **HRMS** (ESI+): | m/z für C₁₈H₂₄N₄OS₂ | | | |
| | ber.: | 376.1392 | | gef.: 376.1406 |
| **EA:** | ber.: | C: 57.42 | H: 6.42 | N: 14.88 |
| | gef.: | C: 57.60 | H: 6.46 | N: 14.73 |

### Darstellung von (2-Hydroxy-5-nitro-phenyl)methyl-4-[2-(isopropylamino)-2-oxo-ethyl]piperazin-1-carbodithioat (Schl32308):

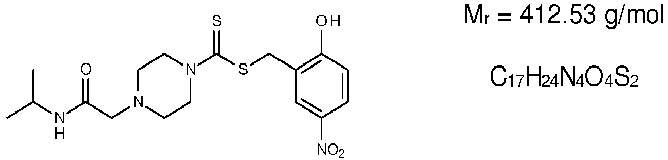

Gemäß **AAV7** wurden 373 mg *N*-Isopropyl-2-piperazin-1-yl-acetamid (2.0 mmol, 1.0 eq), 637 mg Kaliumphosphat (3.0 mmol, 1.5 eq), 0.22 mL Kohlenstoffdisulfid (6.0 mmol, 3.0 eq) und 464 mg 2-Hydroxy-5-nitrobenzylbromid (2.0 mmol, 1.0 eq) umgesetzt. Säulenchromatographie an Kieselgel (DCM/ MeOH 95:5) ergab 709 mg von **Schl32308** (85.9%) in Form eines gelben Feststoffes.

| | | | | |
|---|---|---|---|---|
| **DC:** | R_{f} = 0.34 (DCM/ MeOH 95:5). | | | |
| **Fp**.**:** | 192 °C | | | |
| **¹H-NMR:** | (DMSO-D₆, 400 MHz), δ [ppm] = 8.23 *(s,* 1H, C-C*H*-C(NO₂)), 8.01 (*d*, ³J = 7.6 Hz, 1H, C(NO₂)-C*H*-CH-C-OH), 7.53 (*sbr*, 1H, N*H*), 7.00 (*d*, ³J = 8.9 Hz, 1H, C(NO₂)-CH-C*H*-C-OH), 4.50 *(s,* 2H, S-C*H₂*-C), 4.30-4.16 *(m,* 2H, N-CH*H*-CH₂-N-CH₂), 4.02-3.78 *(m,* 3H, NH-CH, N-CH*H*-CH₂-N-CH₂), 2.92 *(s,* 2H, N-C*H₂-*C(=O)), 2.54-2.44 *(m,* 4H, N-CH₂-C*H₂*-N-CH₂, N-CH₂-C*H₂*-N-CH₂), 1.03 (*d*, ³J = 6.6 Hz, 6H, C*H₃*-CH-C*H₃*). | | | |
| **¹³C-NMR:** | (DMSO-D₆, 100 MHz), δ [ppm] = 195.2 (C=S), 168.2 (C=O), 162.8 (C-OH), 139.6 (C-NO₂), 126.8 (C(NO₂)-CH-CH-C), 125.7 (C-CH-C(NO₂)) 124.4 (S-CH₂-C), 115.9 (C(NO₂)-CH-CH-C), 60.7 (N-CH₂-C), 52.6 (N-CH₂-CH₂-N-CH₂, N-CH₂-CH₂-N-CH₂), 46.1 (N-CH-CH₃), 35.5 (S-CH₂-C), 22.9 (CH-CH₃). | | | |
| **IR (ATR):** | v (cm⁻¹) = 3395 (w), 2982 (w), 2921 (w), 1637 (m), 1612 (w), 1587 (w), 1548 (m), 1515 (w), 1489 (m), 1473 (s), 1384 (w), 1366 (m), 1331 (w), 1286 (vs), 1221 (vs), 1192 (s), 1161 (s), 1150 (w), 1102 (s), 1080 (w), 1051 (s), 1035 (m), 1013 (m), 970 (s), 909 (w), 859 (m), 830 (s), 813 (m), 797 (m), 753 (m), 638 (m), 573 (w), 545 (s), 450 (w), 436 (m), 412 (w). | | | |
| **MS** (ESI+): | m/z (%) = 413 (100, [M+H]⁺) | | | |
| **HRMS** (ESI+): | m/z für C₁₇H₂₄N₄O₄S₂ | | | |
| | ber.: | 412.1239 | | gef.: 412.1250 |
| **EA:** | ber.: | C: 49.50 | H: 5.86 | N: 13.58 |
| | gef.: | C: 49.44 | H: 6.06 | N: 13.56 |

### Darstellung von Cyclohexylmethyl-4-[2-(isopropylamino)-2-oxo-ethyl]piperazin-1-carbodithioat (Schl32309):

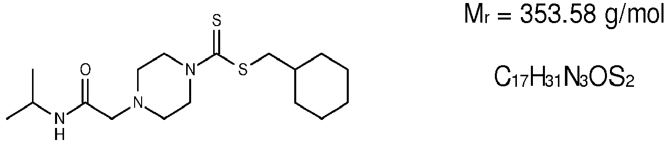

Gemäß **AAV7** wurden 373 mg *N*-Isopropyl-2-piperazin-1-yl-acetamid (2.0 mmol, 1.0 eq), 637 mg Kaliumphosphat (3.0 mmol, 1.5 eq), 0.22 mL Kohlenstoffdisulfid (6.0 mmol, 3.0 eq) und 0.28 mL Brommethylcyclohexan (2.0 mmol, 1.0 eq) umgesetzt. Säulenchromatographie an Kieselgel (DCM/ MeOH 95:5) ergab 616 mg von **Schl32309** (86.2%) in Form eines gelben Feststoffes.

| | | | | |
|---|---|---|---|---|
| **DC:** | R_{f} = 0.62 (DCM/ MeOH 95:5). | | | |
| **Fp**.**:** | 63 °C | | | |
| **¹H-NMR:** | (CDCl₃, 400 MHz), δ [ppm] = 6.81 (*sbr*, 1 H, N*H*), 4.44-4.22 *(m,* 2H, N-CH*H*-CH₂-N-CH₂), 4.12-4.03 *(m,* 1 H, NH-C*H*), 4.00-3.87 *(m,* 2H, N-CH*H*-CH₂-N-CH₂), 3.43-3.38 *(m,* 1 H, S-CH₂-C*H*), 3.17 (*d*, ³J = 6.9 Hz, 2H, S-C*H₂*-CH), 2.98 *(s,* 2H, N-C*H₂-*C(=O)), 2.60-2.52 *(m,* 4H, N-CH₂-C*H₂*-N-CH₂, N-CH₂-C*H₂*-N-CH₂), 1.84-1.76 *(m,* 2H, C*H*_{(Cyclohexyl)}), 1.70-1.54 *(m,* 4H, C*H*_{(Cyclohexyl)}), 1.22-1.14 *(m,* 2H, C*H*_{(Cyclohexyl)}), 1.13 *(d,* ³J = 6.6 Hz, 6H, C*H₃*-CH-C*H₃*), 1.05-0.92 *(m,* 2H, C*H*_{(Cyclohexyl)}). | | | |
| **¹³C-NMR:** | (CDCl₃, 100 MHz), δ [ppm] = 198.5 (C=S), 168.6 (C=O), 61.2 (N-CH₂-C), 52.8 (N-CH₂-CH₂-N-CH₂, N-CH₂-CH₂-N-CH₂), 50.6 (N-CH-CH₃), 44.5 (S-CH₂-CH), 37.2 (S-CH₂-CH), 32.9 (CH_{(Cyclohexyl)}), 26.3 (CH_{(Cyclohexyl)}), 25.9 (CH_{(Cyclohexyl)}), 22.8 (CH-CH₃). | | | |
| **IR (ATR):** | v (cm⁻¹) = 3310 (w), 3272 (w), 3078 (w), 1669 (m), 1648 (s), 1531 (m), 1478 (m), 1460 (m), 1448 (vs), 1424 (w), 1386 (w), 1337 (w), 1307 (w), 1287 (w), 1264 (w), 1217 (s), 1185 (w), 1172 (w), 1147 (s), 1134 (s), 1102 (w), 1056 (w), 1027 (m), 1013 (m), 996 (s), 975 (s), 940 (w), 917 (w), 894 (w), 804 (w), 726 (m), 656 (w), 613 (w), 549 (w), 477 (w), 419 (w). | | | |
| **MS** (ESI+): | m/z (%) = 358 (100, [M+H]⁺) | | | |
| **HRMS** (ESI+): | m/z für C₁₇H₃₁N₃OS₂ | | | |
| | ber.: | 357.1909 | | gef.: 357.1951 |
| **EA:** | ber.: | C: 57.10 | H: 8.74 | N: 11.75 |
| | gef.: | C: 56.97 | H: 8.70 | N: 11.58 |

### Darstellung von (7-Methoxy-2-oxochromen-4-yl)methyl-4-[2-(isopropylamino)-2-oxo-ethyl]piperazin-1-carbodithioat (Schl32310):

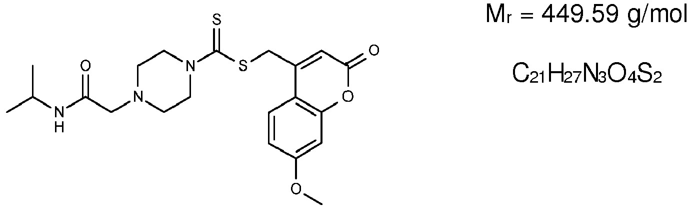

Gemäß **AAV7** wurden 373 mg *N*-Isopropyl-2-piperazin-1-yl-acetamid (2.0 mmol, 1.0 eq), 637 mg Kaliumphosphat (3.0 mmol, 1.5 eq), 0.22 mL Kohlenstoffdisulfid (6.0 mmol, 3.0 eq) und 538 mg 4-(Brommethyl)-7-methoxychromen-2-on (2.0 mmol, 1.0 eq) umgesetzt. Säulenchromatographie an Kieselgel (DCM/ MeOH 95:5) ergab 739 mg von **Schl32310** (82.2%) in Form eines gelben Feststoffes.

| | | | | |
|---|---|---|---|---|
| **DC:** | R_{f} = 0.45 (DCM/ MeOH 95:5). | | | |
| **Fp**.**:** | 70 °C | | | |
| **¹H-NMR:** | (CDCl₃, 400 MHz), δ [ppm] = 7.60 (*d*, ³J = 8.7 Hz, 1H, C-C*H*-CH-C-O-CH₃, 6.86 (*dd*, ³J = 8.7 Hz, ²J = 1.8 Hz, 1H, C-CH-C*H*-C-O-CH₃,), 6.83-6.78 (m, 1H, C-C*H*-C-O-CH₃), 6.74 (*sbr*, 1H, N*H*), 6.39 *(s,* 1H, C-C*H*-C=O), 4.70 *(s,* 2H, S-C*H₂*-C), 4.46-4.22 *(m,* 2H, N-CH*H*-CH₂-N-CH₂), 4.16-4.08 *(m,* 1H, NH-C*H*), 4.05-3.89 *(m,* 2H, N-CH*H*-CH₂-N-CH₂), 3.86 *(s,* 3H, O-C*H₃*), 3.04 *(s,* 2H, N-C*H₂-*C(=O)), 2.70-2.56 *(m,* 4H, N-CH₂-C*H₂*-N-CH₂, N-CH₂-C*H₂*-N-CH₂), 1.16 (*d*, ³J = 6.6 Hz, 6H, C*H₃*-CH-C*H₃*). | | | |
| **¹³C-NMR:** | (CDCl₃, 100 MHz), δ [ppm] = 192.0 (C=S), 168.3 (C=O), 163.0 (C(=O)-O), 161.0 (C-OMe), 159.2 (S-CH₂-C), 155.7 (C(=O)-O-C), 125.6 (C-CH-CH-C-OMe), 112.9 (C-CH-C(=O)), 112.6 (C-CH-CH-C-OMe), 101.2 (C-CH-C-OMe), 61.2 (N-CH₂-C), 55.9 (O-CH₃), 52.8 (N-CH₂-CH₂-N-CH₂, N-CH₂-CH₂-N-CH₂), 41.0 (N-CH-CH₃), 37.6 (S-CH₂-C), 22.9 (CH-CH₃). | | | |
| **IR (ATR):** | v (cm⁻¹) = 3331 (w), 3070 (w), 2969 (w), 1714 (s), 1660 (m), 1609 (vs), 1555 (w), 1512 (m), 1463 (w), 1423 (s), 1384 (m), 1363 (w), 1285 (m), 1263 (m), 1227 (s), 1207 (s), 1186 (w), 1132 (vs), 1054 (m), 1022 (s), 986 (s), 972 (w), 923 (w), 887 (w), 835 (m), 800 (w), 766 (w), 741 (w), 696 (w), 639 (w), 611 (w), 592 (w), 566 (w), 485 (w), 450 (m), 419 (w). | | | |
| **MS** (ESI+): | m/z (%) = 450 (100, [M+H]⁺) | | | |
| **HRMS** (ESI+): | m/z für C₂₁H₂₇N₃O₄S₂ | | | |
| | ber.: | 449.1443 | | gef.: 449.1399 |
| **EA:** | ber.: | C: 56.10 | H: 6.05 | N: 9.35 |
| | gef.: | C: 55.74 | H: 6.24 | N: 9.01 |

### Darstellung von 2-(1H-Indol-3-yl)ethyl-4-[2-(isopropylamino)-2-oxo-ethyl]-piperazin-1-carbodithioat (Schl32311):

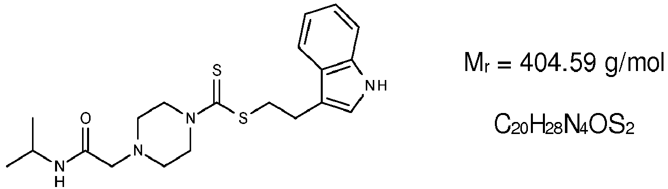

Gemäß **AAV7** wurden 373 mg *N*-Isopropyl-2-piperazin-1-yl-acetamid (2.0 mmol, 1.0 eq), 637 mg Kaliumphosphat (3.0 mmol, 1.5 eq), 0.22 mL Kohlenstoffdisulfid (6.0 mmol, 3.0 eq) und 448 mg 3-(2-Bromethyl)indol (2.0 mmol, 1.0 eq) umgesetzt. Säulenchromatographie an Kieselgel (DCM/ MeOH 95:5) ergab 757 mg von **Schl32311** (93.6%) in Form eines gelben Feststoffes.

| | | | | |
|---|---|---|---|---|
| **DC:** | R_{f} = 0.73 (DCM/ MeOH 95:5). | | | |
| **Fp**.**:** | 156 °C | | | |
| **¹H-NMR:** | (CDCl₃, 400 MHz), δ [ppm] = 8.22 (*sbr*, 1H, N*H*), 7.71 (*d*, ³J = 7.8 Hz, CH₂-C-C-C*H*), 7.35 (*d*, ³J = 8.7 Hz, N-C-C*H*), 7.22-7.10 *(m,* 2H, C-CH-C*H*-C*H*-CH), 7.06 *(s,* 1H, C-C*H*-NH), 6.81 (*sbr*, 1H, C(=O)-N*H*), 4.50-4.25 *(m,* 2H, N-CH*H*-CH₂-N-CH₂), 4.17-4.07 *(m,* 1H, NH-C*H*), 4.05-3.81 *(m,* 2H, N-CH*H*-CH₂-N-CH₂), 3.65 *(t,* ³J = 7.6 Hz, 2H, S-C*H₂*-CH₂-C), 3.17 *(t,* ³J = 7.6 Hz, 2H, S-CH₂-C*H₂*-C), 3.02 *(s,* 2H, N-C*H₂-*C(=O)), 2.68-2.50 *(m,* 4H, N-CH₂-C*H₂*-N-CH₂, N-CH₂-C*H₂*-N-CH₂), 1.18 (*d*, ³J = 6.4 Hz, 6H, C*H₃*-CH-C*H*₃). | | | |
| **¹³C-NMR:** | (CDCl₃, 100 MHz), δ [ppm] = 197.9 (C=S), 168.3 (C=O), 136.3 (NH-C-CH), 127.4 (NH-C-C), 122.2 (C-CH-NH), 122.1 (NH-C-CH-CH), 119.5 (NH-C-CH-CH-CH), 119.1 (NH-C-C-CH), 114.5 (NH-CH-C), 111.3 (NH-C-CH), 61.2 (N-CH₂-C), 52.9 (N-CH₂-CH₂-N-CH₂, N-CH₂-CH₂-N-CH₂), 41.1 (N-CH-CH₃), 41.0 (S-CH₂-C), 37.7 (S-CH₂-CH₂), 25.0 (S-CH₂-CH₂), 22.9 (CH-CH₃). | | | |
| **IR (ATR):** | v (cm⁻¹) = 3351 (w), 3252 (w), 3056 (w), 1661 (s), 1576 (m), 1518 (m), 1461 (s), 1389 (w), 1361 (w), 1339 (w), 1330 (w), 1303 (w), 1286 (s), 1232 (s), 1187 (w), 1133 (s), 1099 (w), 1035 (w), 1020 (m), 1008 (m), 994 (m), 971 (m), 952 (w), 920 (w), 862 (w), 844 (w), 774 (vs), 744 (s), 710 (m), 680 (m), 616 (w), 588 (w), 550 (w), 475 (w), 456 (m), 426 (vs). | | | |
| **MS** (ESI+): | m/z (%) = 405 (100, [M+H]⁺) | | | |
| **HRMS** (ESI+): | m/z für C₂₀H₂₈N₄OS₂ | | | |
| | ber.: | 404.1705 | | gef.: 404.1705 |
| **EA:** | ber.: | C: 59.37 | H: 6.98 | N: 13.85 |
| | gef.: | C:59.67 | H:7.16 | N: 13.57 |

### Darstellung von tert-Butyl-4-[(4-nitrophenyl)methylsulfanylcarbothioyl]piperazin-1-carboxylat (Schl32312):

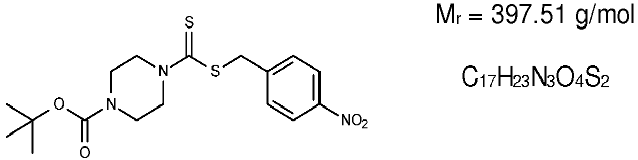

Gemäß **AAV8** wurden 745 mg *Boc*-Piperazin (4.0 mmol, 1.0 eq), 1.11 mL Triethylamin (8.0 mmol, 2.0 eq), 0.72 mL Kohlenstoffdisulfid (12.0 mmol, 3.0 eq) und 864 mg 1-(Brommethyl)-4-nitrobenzen (4.0 mmol, 1.0 eq) umgesetzt. Säulenchromatographie an Kieselgel (Cyclohexan/EtOAc 5:1) ergab 1392 mg von **Schl32312** (87.5%) in Form eines farblosen Feststoffes.

| | | | | |
|---|---|---|---|---|
| **DC:** | R_{f} = 0.15 (Cyclohexan/ EtOAc 5:1). | | | |
| **Fp**.**:** | 78 °C | | | |
| **¹H-NMR:** | (CDCl₃, 400 MHz), δ [ppm] = 8.14 (*d*, ³J = 8.7 Hz, 2H, C-CH-C*H*-C-NO₂, C-CH-C*H*-C-NO₂), 7.55 (*d*, ³J = 8.7 Hz, 2H, C-C*H*-CH-C-NO₂, C-C*H*-CH-C-NO₂), 4.68 *(s,* 2H, S-C*H₂*-C), 4.38-4.20 *(m,* 2H, N-CH*H*-CH₂-N-Boc, N-CH*H*-CH₂-N-Boc), 4.01-3.79 *(m,* 2H, N-CH*H*-CH₂-N-Boc, N-CH*H*-CH₂-N-Boc), 3.59-3.49 *(m,* 4H, N-CH₂-C*H₂-*N-Boc, N-CH₂-C*H₂*-N-Boc), 1.46 *(s,* 9H, C-C*H₃*, C-C*H₃*, C-C*H₃*). | | | |
| **¹³C-NMR:** | (CDCl₃, 100 MHz), δ [ppm] = 195.7 (C=S), 154.5 (C=O), 147.3 (C(NO₂)), 144.5 (CH₂-C-CH-CH), 130.2 (C(NO₂)-CH-CH, C(NO₂)-CH-CH), 123.8 (C-CH-CH, C-CH-CH), 80.8 (O-C-(CH₃)₃), 50.9 (N-CH₂-CH₂-N-Boc, N-CH₂-CH₂-N-Boc), 50.1 (N-CH₂-CH₂-N-Boc, N-CH₂-CH₂-N-Boc), 40.5 (S-CH₂-C), 28.4 (CH₃). | | | |
| **IR (ATR):** | v (cm⁻¹) = 2981 (w), 2938 (w), 1695 (s), 1600 (w), 1492 (s), 1452 (w), 1426 (w), 1409 (m), 1365 (vs), 1282 (vs), 1268 (m), 1158 (vs), 1120 (vs), 1080 (vs), 1057 (m), 1035 (m), 1010 (m), 993 (m), 935 (s), 892 (w), 859 (s), 815 (w), 804 (m), 758 (m), 723 (w), 686 (w), 669 (w), 632 (w), 550 (w), 500 (w), 484 (w), 460 (w), 434 (w), 419 (w). | | | |
| **MS** (ESI+): | m/z (%) = 398 (100, [M+H]⁺) | | | |
| **HRMS** (ESI+): | m/z für C₁₇H₂₃N₃O₄S₂ | | | |
| | ber.: | 397.1130 | | gef.: 397.1113 |
| **EA:** | ber.: | C: 51.36 | H: 5.83 | N: 10.57 |
| | gef.: | C: 51.36 | H: 5.92 | N: 10.74 |

### Darstellung von tert-Butyl-4-[(4-methylsulfonylphenyl)methylsulfanylcarbothioyl]-piperazin-1-carboxylat (Schl32313):

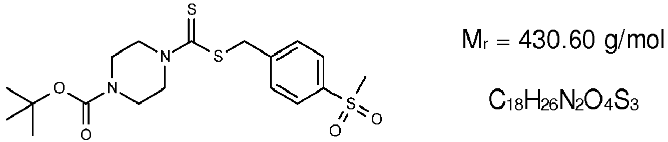

Gemäß **AAV8** wurden 745 mg *Boc*-Piperazin (4.0 mmol, 1.0 eq), 1.11 mL Triethylamin (8.0 mmol, 2.0 eq), 0.72 mL Kohlenstoffdisulfid (12.0 mmol, 3.0 eq) und 810 mg 1-(Brommethyl)-4-methylsulfonylbenzen (4.0 mmol, 1.0 eq) umgesetzt. Säulenchromatographie an Kieselgel (Cyclohexan/ EtOAc 2:1) ergab 1592 mg von **Schl32313** (92.5%) in Form eines blassgelben Feststoffes.

| | | | | |
|---|---|---|---|---|
| **DC:** | R_{f} = 0.15 (Cyclohexan/ EtOAc 2:1). | | | |
| **Fp**.**:** | 124 °C | | | |
| **¹H-NMR:** | (CDCl₃, 400 MHz), δ [ppm] = 7.85 (*d*, ³J = 8.5 Hz, 2H, CH₂-C-CH-C*H*-C-SO₂Me, CH₂-C-CH-C*H*-C-SO₂Me), 7.57 (*d*, ³J = 8.2 Hz, 2H, CH₂-C-C*H*-CH-C-SO₂Me, CH₂-C-C*H*-CH-C-SO₂Me), 4.66 *(s,* 2H, S-C*H₂*-C), 4.42-4.19 *(m,* 2H, N-CH*H*-CH₂-N-CH₃, N-CH*H*-CH₂-N-CH₃), 4.02-3.81 *(m,* 2H, N-CH*H*-CH₂-N-Boc, N-CH*H*-CH₂-N-Boc), 3.58-3.49 *(m,* 4H, N-CH₂-C*H₂*-N-Boc, N-CH₂-C*H₂*-N-Boc), 3.02 (*s*, 3H, SO₂-C*H₃*), 1.45 (s, 9H, C-C*H₃*, C-C*H₃*, C-C*H₃*). | | | |
| **¹³C-NMR:** | (CDCl₃, 100 MHz), δ [ppm] = 195.9 (C=S), 154.5 (C=O), 143.3 (C-SO₂Me), 139.5 (CH₂-C-CH-CH), 130.3 (C-SO₂Me-CH-CH, C-SO₂Me-CH-CH), 127.6 (C-CH-CH, C-CH-CH), 80.8 (O-C-(CH₃)₃), 60.5 (N-CH₂-CH₂-N-Boc, N-CH₂-CH₂-N-Boc), 60.1 (N-CH₂-CH₂-N-Boc, N-CH₂-CH₂-N-Boc), 44.6 (SO₂-CH₃), 40.7 (S-CH₂-C), 28.4 (CH₃). | | | |
| **IR (ATR):** | v (cm⁻¹) = 2980 (w), 2932 (w), 2090 (w), 1691 (s), 1597 (w), 1469 (w), 1421 (s), 1365 (w), 1318 (w), 1297 (s), 1283 (s), 1249 (m), 1219 (vs), 1160 (s), 1142 (vs), 1123 (s), 1086 (m), 1019 (m), 993 (m), 972 (w), 949 (m), 934 (m), 861 (w), 816 (w), 795 (w), 775 (s), 730 (w), 691 (w), 637 (w), 553 (m), 533 (m), 515 (s), 475 (w), 437 (w). | | | |
| **MS** (ESI+): | m/z (%) = 431 (100, [M+H]⁺) | | | |
| **HRMS** (ESI+): | m/z für C₁₈H₂₆N₂O₄S₃ | | | |
| | ber.: | 430.1055 | | gef.: 430.0983 |
| **EA:** | ber.: | C: 50.21 | H: 6.09 | N: 6.51 |
| | gef.: | C: 49.89 | H: 6.10 | N: 6.52 |

### Darstellung von tert-Butyl-4-[(4-cyanophenyl)methylsulfanylcarbothioyl]piperazin-1-carboxylat (Schl32314):

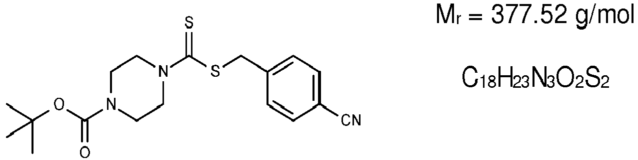

Gemäß **AAV8** wurden 745 mg *Boc*-Piperazin (4.0 mmol, 1.0 eq), 1.11 mL Triethylamin (8.0 mmol, 2.0 eq), 0.72 mL Kohlenstoffdisulfid (12.0 mmol, 3.0 eq) und 784 mg 4-(Brommethyl)benzonitril (4.0 mmol, 1.0 eq) umgesetzt. Säulenchromatographie an Kieselgel (Cyclohexan/EtOAc 5:1) ergab 1367 mg von **Schl32314** (90.5%) in Form eines farblosen Feststoffes.

| | | | | |
|---|---|---|---|---|
| **DC:** | R_{f} = 0.14 (Cyclohexan/ EtOAc 5:1). | | | |
| **Fp**.**:** | 106 °C | | | |
| **¹H-NMR:** | (CDCl₃, 400 MHz), δ [ppm] = 7.57 (*d*, ³J = 8.0 Hz, 2H, C-CH-C*H*-C-CN, C-CH-C*H*-C-CN), 7.48 (*d*, ³J = 8.2 Hz, 2H, C-C*H*-CH-C-CN, C-C*H*-CH-C-CN), 4.63 *(s,* 2H, S-C*H₂*-C), 4.40-4.19 (*m,* 2H, N-CH*H*-CH₂-N-CH₃, N-CH*H*-CH₂-N-CH₃), 4.03-3.79 (*m,* 2H, N-CH*H*-CH₂-N-Boc, N-CH*H*-CH₂-N-Boc), 3.58-3.47 (*m,* 4H, N-CH₂-C*H₂*-N-Boc, N-CH₂-C*H₂*-N-Boc), 1.45 *(s,* 9H, C-C*H₃*, C-C*H₃*, C-C*H₃*). | | | |
| **¹³C-NMR:** | (CDCl₃, 100 MHz), δ [ppm] = 195.9 (C=S), 168.3 (C=O), 142.3 (C-CH-CH-C-CN), 132.4 (C-CH-CH-C-CN, C-CH-CH-C-CN), 130.1 (C-CH-CH-C-CN, C-CH-CH-C-CN), 118.8 (C-CN), 111.3 (C-CN), 80.8 (O-C-(CH₃)₃), 53.6 (N-CH₂-CH₂-N-Boc, N-CH₂-CH₂-N-Boc), 53.1 (N-CH₂-CH₂-N-Boc, N-CH₂-CH₂-N-Boc), 40.9 (S-CH₂-C), 28.4 (CH₃). | | | |
| **IR (ATR):** | v (cm⁻¹) = 2970 (w), 2934 (w), 2229 (w), 2113 (w), 2055 (w), 1691 (vs), 1681 (vs), 1606 (w), 1503 (w), 1456 (w), 1401 (vs), 1366 (s), 1283 (m), 1230 (vs), 1161 (vs), 1124 (vs), 1083 (w), 1029 (w), 992 (m), 940 (s), 893 (w), 868 (m), 852 (s), 770 (m), 739 (w), 720 (w), 666 (w), 646 (w), 554 (s), 540 (m), 504 (w), 477 (w), 435 (w), 415 (w). | | | |
| **MS** (ESI+): | m/z (%) = 378 (100, [M+H]⁺) | | | |
| **HRMS** (ESI+): | m/z für C₁₈H₂₃N₃O₂S₂ | | | |
| | ber.: | 377.1232 | | gef.: 377.1221 |
| **EA:** | ber.: | C: 57.27 | H:6.14 | N: 11.13 |
| | gef.: | C: 57.31 | H: 6.21 | N: 11.12 |

### Darstellung von tert-Butyl-4-[(2-hydroxy-5-nitro-phenyl)methylsulfanylcarbothioyl]-piperazin-1-carboxylat (Schl32315):

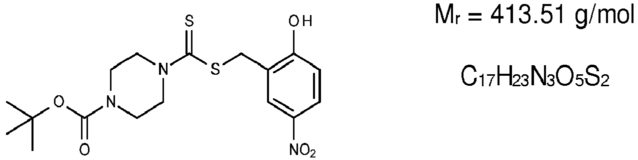

Gemäß **AAV8** wurden 745 mg *Boc*-Piperazin (4.0 mmol, 1.0 eq), 1.11 mL Triethylamin (8.0 mmol, 2.0 eq), 0.72 mL Kohlenstoffdisulfid (12.0 mmol, 3.0 eq) und 928 mg 2-Hydroxy-5-nitrobenzylbromid (4.0 mmol, 1.0 eq) umgesetzt. Säulenchromatographie an Kieselgel (Cyclohexan/EtOAc 1:1) ergab 1211 mg von **Schl32315** (73.2%) in Form eines blassgelben Feststoffes.

| | | | | |
|---|---|---|---|---|
| **DC:** | R_{f} = 0.48 (Cyclohexan/ EtOAc 1:1). | | | |
| **Fp**.**:** | 121 °C | | | |
| **¹H-NMR:** | (DMSO-D₆, 400 MHz), δ [ppm] = 11.43 (s, 1H, O*H*), 8.24 *(s,* 1H, C-C*H*-C(NO₂)), 8.02 (*dd,* ³J = 8.9 Hz, ²J = 3.0 Hz, 1H, C(NO₂)-C*H*-CH-C-OH), 6.96 (*d,* ³J = 8.9 Hz, 1H, C(NO₂)-CH-C*H*-C-OH), 4.51 (*s,* 2H, S-C*H₂*-C), 4.29-4.09 (*m,* 2H, N-CH*H*-CH₂-N-CH₃, N-CH*H*-CH₂-N-CH₃), 4.00-3.78 (*m,* 2H, N-CH*H*-CH₂-N-Boc, N-CH*H*-CH₂-N-Boc), 3.47-3.36 (*m,* 4H, N-CH₂-C*H₂*-N-Boc, N-CH₂-C*H₂*-N-Boc), 1.37 *(s,* 9H, C-C*H₃*, C-C*H₃*, C-C*H₃*). | | | |
| **¹³C-NMR:** | (DMSO-D₆, 100 MHz), δ [ppm] = 195.6 (C=S), 162.6 (C-OH), 154.2 (C=O), 139.7 (C-NO₂), 126.9 (C(NO₂)-CH-CH-C), 125.7 (C-CH-C(NO₂)), 124.4 (S-CH₂-C), 115.9 (C(NO₂)-CH-CH-C), 79.9 (O-C-(CH₃)₃), 53.6 (N-CH₂-CH₂-N-Boc, N-CH₂-CH₂-N-Boc), 53.1 (N-CH₂-CH₂-N-Boc, N-CH₂-CH₂-N-Boc), 35.5 (S-CH₂-C), 28.6 (CH₃). | | | |
| **IR (ATR):** | v (cm⁻¹ ) = 3140 (w), 2979 (w), 2920 (w), 1652 (s), 1619 (w), 1595 (w), 1525 (w), 1499 (m), 1459 (m), 1435 (s), 1421 (w), 1392 (w), 1370 (w), 1337 (vs), 1279 (vs), 1225 (vs), 1157 (vs), 1136 (s), 1078 (m), 1045 (m), 1020 (s), 992 (s), 931 (m), 917 (m), 854 (m), 828 (s), 815 (w), 763 (m), 685 (w), 639 (m), 587 (w), 542 (m), 503 (w), 480 (w), 435 (w), 411 (w). | | | |
| **MS (ESI+):** | m/z (%) = 414 (100, [M+H]⁺) | | | |
| **HRMS** (ESI+): | m/z für C₁₇H₂₃N₃O₅S₂ | | | |
| | ber.: | 413.1079 | | gef.: 413.1075 |
| **EA:** | ber.: | C:49.38 | H: 5.61 | N:10.16 |
| | gef.: | C:49.21 | H:5.48 | N:10.10 |

### Darstellung von tert-Butyl-4-(Cyclohexylmethylsulfanylcarbothioyl)-piperazin-1-carboxylat (Schl32316):

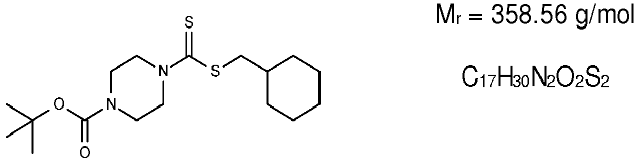

Gemäß **AAV8** wurden 745 mg *Boc*-Piperazin (4.0 mmol, 1.0 eq), 1.11 mL Triethylamin (8.0 mmol, 2.0 eq), 0.72 mL Kohlenstoffdisulfid (12.0 mmol, 3.0 eq) und 0.56 mL Brommethylcyclohexan (4.0 mmol, 1.0 eq) umgesetzt. Säulenchromatographie an Kieselgel (Cyclohexan/ EtOAc 5:1) ergab 804 mg von **Schl32316** (56.1 %) in Form eines blassgelben Feststoffes.

| | | | | |
|---|---|---|---|---|
| **DC:** | R_{f} = 0.25 (Cyclohexan/ EtOAc 5:1). | | | |
| **Fp**.**:** | 102 °C | | | |
| **¹H-NMR:** | (CDCl₃, 400 MHz), δ [ppm] = 4.40-4.19 (*m*, 2H, N-CH*H*-CH₂-N-CH₃, N-CH*H*-CH₂-N-CH₃), 4.13-3.89 (*m*, 2H, N-CH*H*-CH₂-N-Boc, N-CH*H*-CH₂-N-Boc), 3.57-3.47 (*m*, 4H, N-CH₂-C*H₂-*N-Boc, N-CH2-C*H₂-*N-Boc), 3.22 (*d,* ³J = 6.6 Hz, 2H, S-C*H*₂-CH), 1.87-1.80 (m, 2H, C*H*_{(Cyclohexyl)}), 1.74-1.59 (*m*, 4H, C*H*_{(Cyclohexyl}), S-CH₂-C*H*), 1.46 (*s*, 9H, C-C*H*₃, C-C*H*₃, C*-*C*H3),* 1.29-1.11 (*m*, 3H, C*H*(_{Cyclohexyl)}), 1.05-0.93 (*m*, 2H, C*H*_{(Cyclohexyl)}). | | | |
| **¹³C-NMR:** | (CDCl₃, 100 MHz), δ [ppm] = 198.5 (C=S), 154.6 (C=O), 80.6 (O-C-(CH₃)₃), 57.8 (N-CH₂-CH₂-N-Boc, N-CH₂-CH₂-N-Boc), 44.5 (S-CH₂-CH), 37.3 (S-CH₂-CH), 32.9 (CH_{(Cyclohexyl)}), 28.5 (CH₃), 26.4 (CH_{(Cyclohexyl)}), 26.1 (CH_{(Cyclohexyl)}). | | | |
| **IR (ATR):** | v (cm⁻¹) = 2978 (w), 2922 (m), 2852 (w), 2175 (w), 2099 (w), 1942 (w), 1684 (vs), 1463 (m), 1421 (vs), 1366 (m), 1283 (s), 1265 (m), 1238 (w), 1223 (vs), 1163 (vs), 1126 (vs), 1076 (w), 1052 (w), 1020 (s), 1002 (vs), 962 (w), 934 (vs), 894 (w), 865 (m), 839 (w), 819 (w), 770 (w), 755 (w), 652 (w), 623 (w), 557 (w), 538 (m), 505 (w), 476 (w), 440 (w). | | | |
| **MS** (ESI+): | m/z (%) = 359 (100, [M+H]⁺) | | | |
| **HRMS** (ESI+): | m/z für C₁₇H₃₀N₂O₂S₂ | | | |
| | ber.: | 358.1749 | | gef.: 358.1722 |
| **EA:** | ber.: | C: 56.94 | H:8.43 | N:7.81 |
| | gef.: | C: 57.05 | H: 8.45 | N: 7.85 |

### Darstellung von tert-Butyl-4-[(7-methoxy-2-oxochromen-4-yl)methylsulfanylcarbothioyl]-piperazin-1-carboxylat (Schl32317):

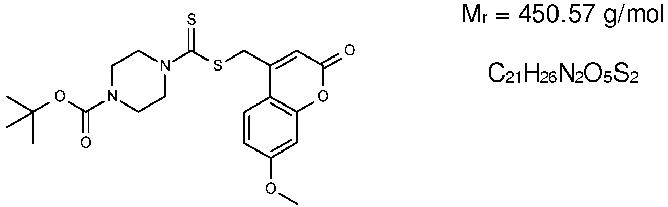

Gemäß **AAV8** wurden 745 mg *Boc*-Piperazin (4.0 mmol, 1.0 eq), 1.11 mL Triethylamin (8.0 mmol, 2.0 eq), 0.72 mL Kohlenstoffdisulfid (12.0 mmol, 3.0 eq) und 1076 mg 4-(Brommethyl)-7-methoxychromen-2-on (4.0 mmol, 1.0 eq) umgesetzt. Säulenchromatographie an Kieselgel (Cyclohexan/ EtOAc 2:1) ergab 1360 mg von **Schl32317** (75.5%) in Form eines gelben Feststoffes.

| | | | | |
|---|---|---|---|---|
| **DC:** | R_{f} = 0.25 (Cyclohexan/ EtOAc 2:1). | | | |
| **Fp**.**:** | 164 °C | | | |
| **¹H-NMR:** | (CDCl₃, 400 MHz), δ [ppm] = 7.59 (*d*, ³J = 8.9 Hz, 1H, C-C*H*-CH-C-O-CH₃), 6.86 (*dd*, ³J = 8.7 Hz, ²J = 2.6 Hz, 1H, C-C*H*-CH-C-O-CH₃), 6.82-6.79 (*m*, 1H, C-C*H*-C-O-CH₃), 6.39 (*s*, 1H, C-C*H*-C=O), 4.70 (s, 2H, S-C*H*₂-C), 4.38-4.22 (*m*, 2H, N-CH*H*-CH₂-N-CH₃, N-CH*H*-CH₂-N-CH₃), 4.05-3.87 (*m*, 2H, N-CH*H*-CH₂-N-Boc, N-CH*H*-CH₂-N-Boc), 3.86 (*s*, 3H, O-C*H*₃), 3.59-3.47 (*m*, 4H, N-CH₂-C*H₂*-N-Boc, N-CH₂-C*H₂*-N-Boc), 1.46 (*s*, 9H, C-C*H*₃, C*-*C*H₃,* C*-*C*H₃).* | | | |
| **¹³C-NMR:** | (CDCl₃, 100 MHz), δ [ppm] = 194.7 (C=S), 163.0 (C(=O)-O), 161.0 (C-OMe), 160.2 (S-CH₂-C), 155.7 (C(=O)-O-C), 154.5 (C=O), 150.2 (C(=O)-C), 125.6 (C-CH-CH-C-OMe), 112.9 (C-CH-C(=O)), 112.6 (C-CH-CH-C-OMe), 101.2 (C-CH-C-OMe), 80.8 (O-C-(CH₃)₃), 55.9 (O-CH₃), 53.9 (N-CH₂-CH₂-N-Boc, N-CH₂-CH₂-N-Boc), 53.5 (N-CH₂-CH₂-N-Boc, N-CH₂-CH₂-N-Boc), 37.5 (S-CH₂-C), 28.4 (CH₃). | | | |
| **IR (ATR):** | v (cm⁻¹) = 3082 (w), 2979 (w), 2931 (w), 1680 (vs), 1604 (m), 1512 (w), 1468 (w), 1456 (m), 1446 (m), 1421 (m), 1403 (m), 1382 (m), 1367 (m), 1357 (m), 1281 (w), 1265 (m), 1221 (vs), 1161 (vs), 1124 (vs), 1079 (m), 1043 (s), 1023 (vs), 987 (w), 937 (s), 894 (w), 858 (m), 838 (s), 813 (w), 768 (w), 750 (m), 671 (w), 639 (w), 611 (w), 539 (m), 480 (w), 456 (w). | | | |
| **MS** (ESI+): | m/z (%) = 451 (100, [M+H]⁺) | | | |
| **HRMS** (ESI+): | m/z für C₂₁H₂₆N₂O₅S₂ | | | |
| | ber.: | 450.1283 | | gef.: 450.1252 |
| **EA:** | ber.: | C: 55.98 | H:5.82 | N: 6.22 |
| | gef.: | C: 55.78 | H: 5.88 | N: 6.45 |

### Darstellung von tert-Butyl-4-[2-(1H-indol-3-yl)ethylsulfanylcarbothioyl]piperazin-1-carboxylat (Schl32318):

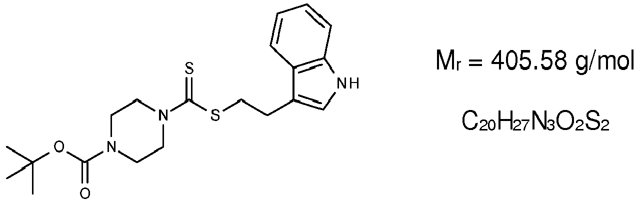

Gemäß **AAV8** wurden 745 mg *Boc*-Piperazin (4.0 mmol, 1.0 eq), 1.11 mL Triethylamin (8.0 mmol, 2.0 eq), 0.72 mL Kohlenstoffdisulfid (12.0 mmol, 3.0 eq) und 896 mg 3-(2-Bromethyl)indol (4.0 mmol, 1.0 eq) umgesetzt. Säulenchromatographie an Kieselgel (Cyclohexan/ EtOAc 1:1) ergab 1357 mg von **Schl32318** (83.7%) in Form eines gelben Feststoffes.

| | | | | |
|---|---|---|---|---|
| **DC**: | R_{f} = 0.59 (Cyclohexan/ EtOAc 1:1). | | | |
| **Fp**.**:** | 142 °C | | | |
| **¹H-NMR:** | (CDCl₃, 400 MHz), δ [ppm] = 8.05 (*sbr,* 1H, N*H*), 7.72 (d, ³J = 7.8 Hz, CH₂-C-C-C*H*), 7.36 (d, ³J = 8.0 Hz, N-C-C*H*), 7.20 (*t,* ³J = 7.5 Hz, 1H, C-CH-C*H*-CH-CH), 7.13 (*t,* ³J = 7.5 Hz, 1H, C-CH-C*H*-CH-CH), 7.08 (s, 1H, C-C*H*-NH), 4.46-4.23 (*m*, 2H, N-CH*H*-CH₂-N-CH₃, N-CH*H*-CH₂-N-CH₃), 4.04-3.81 (*m*, 2H, N-CH*H*-CH₂-N-Boc, N-CH*H*-CH₂-N-Boc), 3.66 (*t*, ³J = 7.7 Hz, 2H, S-C*H₂*-CH₂-C), 3.58-3.49 (*m*, 4H, N-CH₂-C*H*₂-N-Boc, N-CH₂-C*H*₂N-Boc), 3.18 (*t*, ³J = 7.7 Hz, 2H, S-CH₂-CH₂-C), 1.48 (s, 9H, C-C*H*₃, C-C*H*₃, C-C*H*₃). | | | |
| **¹³C-NMR:** | (CDCl₃, 100 MHz), δ [ppm] = 197.9 (C=S), 154.6 (C=O), 136.3 (NH-C-CH), 127.4 (NH-C-C), 122.2 (C-CH-NH), 122.0 (NH-C-CH-CH), 119.5 (NH-C-CH-CH-CH), 119.1 (NH-C-C-CH), 114.7 (NH-CH-C), 111.2 (NH-C-CH), 80.7 (O-C-(CH₃)₃), 55.5 (N-CH₂-CH₂-N-Boc, N-CH₂-CH₂-N-Boc), 49.3 (N-CH₂-CH₂-N-Boc, N-CH₂-CH₂-N-Boc), 37.6 (S-CH₂-CH₂), 28.5 (CH₃), 25.0 (S-CH₂-CH₂). | | | |
| **IR** (ATR): | v (cm⁻¹ ) = 3434 (w), 3057 (w), 3005 (w), 2979 (w), 2930 (w), 1942 (w), 1667 (vs), 1618 (w), 1550 (w), 1477 (w), 1450 (w), 1416 (vs), 1363 (m), 1338 (w), 1288 (w), 1248 (w), 1223 (m), 1208 (m), 1157 (s), 1119 (m), 1086 (w), 1055 (w), 1031 (m), 995 (m), 937 (s), 870 (w), 817 (w), 774 (w), 734 (vs), 707 (w), 638 (w), 618 (w), 592 (w), 535 (m), 493 (m), 462 (m). | | | |
| **MS** (ESI+): | m/z (%) = 406 (100, [M+H]⁺) | | | |
| **HRMS** (ESI+): | m/z für C₂₀H₂₇N₃O₂S₂ | | | |
| | ber.: | 405.1545 | | gef.: 405.1537 |
| **EA:** | ber.: | C: 59.23 | H: 6.71 | N: 10.36 |
| | gef.: | C: 59.40 | H: 6.59 | N: 10.47 |

### Darstellung von (4-Nitrophenyl)methylpiperazin-1-carbodithioathydrochlorid (Schl32319):

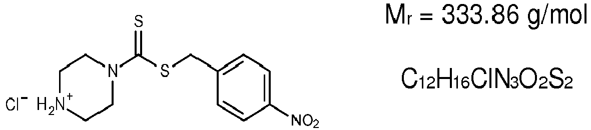

Gemäß **AAV11** wurden 1327 mg *tert*-Butyl-4-[(4-nitrophenyl)methylsulfanylcarbothioyl]piperazin-1-carboxylat **(Schl32312,** 3.33 mmol, 1.0 eq) umgesetzt. Es wurden 991 mg von **Schl32318** (89.1 %) in Form eines farblosen Feststoffes erhalten.

| | | | | |
|---|---|---|---|---|
| **Fp**.**:** | 227 °C | | | |
| **¹H-NMR:** | (DMSO-D₆, 400 MHz), δ [ppm] = 9.72 *(sbr,* 2H, N*H*₂⁺), 8.14 (*d,* ³J = 8.9 Hz, 2H, C-CH-C*H*-C-NO₂, C-CH-C*H*-C-NO₂), 7.64 (*d*, ³J = 8.7 Hz, 2H, C-CH-C*H*-C-NO₂, C-C*H*-CH-C-NO₂), 4.71 (*s*, 2H, S-C*H₂-*C), 4.48-4.11 (*m*, 4H, N-C*H₂-*CH₂-NH₂⁺, N-C*H*₂-CH₂-NH₂⁺), 3.20-3.12 (*m*, 4H, N-CH₂-C*H*₂-NH₂⁺, N-CH₂-C*H₂*-NH₂⁺). | | | |
| **¹³C-NMR:** | (DMSO-D₆, 100 MHz), δ [ppm] = 196.2 (C=S), 147.2 (C(NO₂)), 144.4 (CH₂-C-CH-CH), 130.9 (C(NO₂)-CH-CH, C(NO₂)-CH-CH), 124.0 (C-CH-CH, C-CH-CH), 66.9 (N-CH₂-CH₂-NH₂⁺, N-CH₂-CH₂-NH₂⁺), 42.5 (S-CH₂-C). | | | |
| **IR (ATR):** | v (cm⁻¹ ) = 3061 (w), 2927 (w), 2780 (w), 1595 (w), 1516 (s), 1477 (w), 1414 (m), 1376 (w), 1345 (vs), 1318 (w), 1273 (m), 1252 (m), 1190 (w), 1171 (w), 1143 (w), 1116 (w), 1086 (w), 1036 (w), 993 (m), 915 (m), 890 (s), 863 (m), 804 (w), 752 (w), 728 (m), 680 (w), 635 (w), 622 (w), 551 (w), 495 (w), 468 (w), 415 (s). | | | |
| **MS** (ESI+): | m/z (%) = 298 (100, [M-Cl]⁺) | | | |
| **HRMS** (ESI+): | m/z für C₁₂H₁₅N₃O₂S₂ | | | |
| | ber.: | 297.0606 | | gef.: 297.0633 |
| **EA:** | ber.: | C:43.17 | H:4.83 | N:12.59 |
| | gef.: | C:43.00 | H:4.62 | N:12.55 |

### Darstellung von (4-Methylsulfonylphenyl)methylpiperazin-1-carbodithioathydrochlorid (Schl32320):

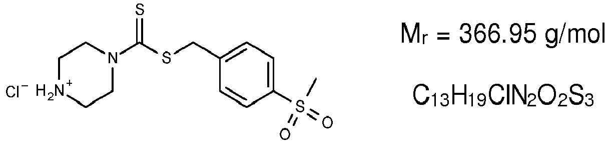

Gemäß **AAV11** wurden 1413 mg *tert*-Butyl-4-[(4-methylsulfonylphenyl)methylsulfanyl-carbothioyl]-piperazin-1-carboxylat **(Schl32313,** 3.28 mmol, 1.0 eq) umgesetzt. Es wurden 1084 mg von **Schl32320** (90.0%) in Form eines farblosen Feststoffes erhalten.

| | | | | |
|---|---|---|---|---|
| **Fp**.**:** | 211° C | | | |
| **¹H-NMR:** | (DMSO-D₆, 400 MHz), δ [ppm] = 9.75 (*sbr,* 2H, N*H*₂⁺), 7.84 (d, ³J = 8.2 Hz, 2H, CH₂-C-CH-C*H*-C-SO₂Me, CH₂-C-CH-C*H*-C-SO₂Me), 7.63 (*d,* ³J = 8.2 Hz, 2H, CH₂-C-C*H*-CH-C-SO₂Me, CH₂-C-C*H*-CH-C-SO₂Me), 4.67 (s, 2H, S-C*H*₂-C), 4.48-4.12 (*m,* 4H, N-C*H₂-*CH₂-NH₂⁺, N-C*H*₂-CH₂-NH₂⁺), 3.22-3.10 (*m*, 7H, N-CH₂-C*H*₂-NH₂⁺, N-CH₂-C*H*₂-NH₂⁺, SO₂-C*H*₃). | | | |
| **¹³C-NMR:** | (DMSO-D₆, 100 MHz), δ [ppm] = 196.4 (C=S), 143.3 (C-SO₂Me), 140.3 (CH₂-C-CH-CH), 130.6 (C-SO₂Me-CH-CH, C-SO₂Me-CH-CH), 127.6 (C-CH-CH, C-CH-CH), 66.9 (N-CH₂-CH₂-NH₂⁺, N-CH₂-CH₂-NH₂⁺), 44.1 (SO₂-CH₃), 42.5 (S-CH₂-C). | | | |
| **IR (ATR):** | v (cm⁻¹) = 2914 (w), 2785 (w), 2703 (w), 2451 (w), 1598 (w), 1472 (w), 1419 (m), 1364 (w), 1344 (w), 1317 (w), 1293 (s), 1265 (m), 1245 (m), 1198 (w), 1162 (w), 1145 (vs), 1117 (w), 1090 (w), 1038 (w), 1011 (m), 982 (s), 906 (w), 877 (m), 828 (w), 801 (w), 758 (s), 669 (w), 625 (w), 614 (w), 541 (s), 521 (vs), 475 (w), 459 (w), 418 (w). | | | |
| **MS** (ESI+): | m/z (%) = 331 (100, [M-Cl]⁺) | | | |
| **HRMS** (ESI+): | m/z für C₁₃H₁₈N₂O₂S₃ | | | |
| | ber.: | 330.0530 | | gef.: 330.0558 |
| **EA:** | ber.: | C:42.55 | H:5.22 | N:7.63 |
| | gef.: | C: 42.59 | H: 5.23 | N: 7.71 |

### Darstellung von (4-Cyanophenyl)methylpiperazin-1-carbodithioathydrochlorid (Schl32321):

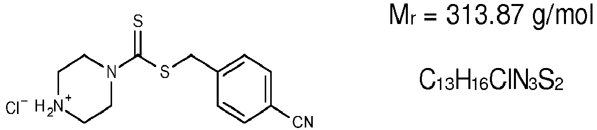

Gemäß **AAV11** wurden 1176 mg *tert-*Butyl-4-[(4-cyanophenyl)methylsulfanylcarbothioyl]piperazin-1-carboxylat **(Schl32314,** 3.12 mmol, 1.0 eq) umgesetzt. Es wurden 868 mg von **Schl32321** (88.7%) in Form eines farblosen Feststoffes erhalten.

| | | | | |
|---|---|---|---|---|
| **Fp**.**:** | 215 °C | | | |
| **¹H-NMR:** | (DMSO-D₆, 400 MHz), δ [ppm] = 9.68 (*sbr,* 2H, N*H*₂⁺), 7.76 (d, ³J = 8.5 Hz, 2H, C-CH-C*H*-C-CN, C-CH-C*H*-C-CN), 7.57 (*d,* ³J = 8.5 Hz, 2H, C-C*H*-CH-C-CN, C-C*H*-CH-C-CN), 4.65 (s, 2H, S-C*H₂-*C), 4.48-4.13 (*m*, 4H, N-C*H₂-*CH₂-NH₂⁺, N-C*H*₂-CH₂-NH₂⁺), 3.21-3.16 (*m*, 4H, N-CH₂-C*H*₂-NH₂⁺, N-CH₂-C*H*₂-NH₂⁺). | | | |
| **¹³C-NMR:** | (DMSO-D₆, 100 MHz), δ [ppm] = 196.4 (C=S), 143.2 (C-CH-CH-C-CN), 132.8 (C-CH-CH-C-CN, C-CH-CH-C-CN), 130.7 (C-CH-CH-C-CN, C-CH-CH-C-CN), 119.3 (C-CN), 110.6 (C-CN), 66.9 (N-CH₂-CH₂-NH₂⁺, N-CH₂-CH₂-NH₂⁺), 42.5 (S-CH₂-C). | | | |
| **IR (ATR):** | v (cm⁻¹ ) = 2978 (w), 2915 (w), 1789 (w), 1592 (w), 1506 (w), 1463 (w), 1429 (s), 1399 (w), 1385 (w), 1366 (w), 1316 (w), 1304 (w), 1262 (m), 1244 (s), 1187 (w), 1164 (m), 1145 (m), 1119 (w), 1088 (w), 1031 (m), 988 (vs), 962 (w), 937 (w), 905 (w), 874 (vs), 831 (w), 810 (w), 752 (w), 676 (w), 639 (w), 610 (w), 553 (s), 540 (w), 478 (w), 466 (w), 424 (m). | | | |
| **MS** (ESI+): | m/z (%) = 278 (100, [M-Cl]⁺) | | | |
| **HRMS** (ESI+): | m/z für C₁₃H₁₆N₃S₂ | | | |
| | ber.: | 277.0707 | | gef.: 277.0736 |
| **EA:** | ber.: | C:49.75 | H:5.14 | N:13.39 |
| | gef.: | C:49.62 | H:5.19 | N:13.48 |

### Darstellung von (2-Hydroxy-5-nitro-phenyl)methylpiperazin-1-carbodithioathydrochlorid (Schl32322):

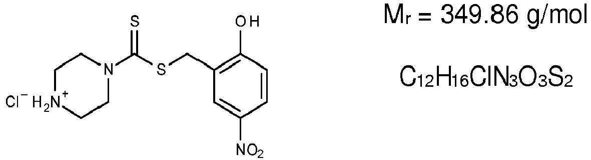

Gemäß **AAV11** wurden 1162 mg *tert-*Butyl-4-[(2-hydroxy-5-nitrophenyl)methylsulfanyl-carbothioyl]-piperazin-1-carboxylat **(Schl32315,** 2.81 mmol, 1.0 eq) umgesetzt. Es wurden 638 mg von **Schl32322** (64.9%) in Form eines farblosen Feststoffes erhalten.

| | | | | |
|---|---|---|---|---|
| **Fp**.**:** | 218 °C | | | |
| **¹H-NMR:** | (DMSO-D₆, 400 MHz), δ [ppm] = 11.67 (*sbr,* 1H, O*H*), 9.55 (*sbr,* 2H, N*H*₂+), 8.24 (*s*, 1H, C-C*H-*C(NO₂)), 8.03 (*dd,* ³J = 9.0 Hz, ²J = 2.9 Hz, 1H, C(NO₂)-C*H*-CH-C-OH), 7.08 (*d*, ³J = 8.9 Hz, 1H, C(NO₂)-CH-C*H*-C-OH), 4.52 (s, 2H, S-C*H₂*-C), 4.41-4.13 (*m*, 4H, N-CH₂-C*H*₂-NH₂⁺, N-C*H*₂-CH₂-NH₂⁺), 3.21-3.13 (*m*, 4H, N-CH₂-C*H*₂-NH₂⁺, N-CH₂-C*H*₂-NH₂⁺). | | | |
| **¹³C-NMR:** | (DMSO-D₆, 100 MHz), δ [ppm] = 197.1 (C=S), 162.8 (C-OH), 139.6 (C-NO₂), 126.9 (C(NO₂)-CH-CH-C), 125.8 (C-CH-C(NO₂)), 124.0 (S-CH₂-C), 115.9 (C(NO₂)-CH-CH-C), 42.6 (N-CH₂-CH₂-NH₂⁺, N-CH₂-CH₂-NH₂⁺), 35.9 (S-CH₂-C). | | | |
| **IR (ATR):** | v (cm⁻¹ ) = 3369 (w), 2981 (w), 1594 (w), 1524 (w), 1487 (w), 1464 (w), 1431 (m), 1362 (w), 1342 (vs), 1298 (vs), 1259 (m), 1244 (s), 1188 (w), 1161 (w), 1138 (w), 1079 (w), 1019 (m), 989 (w), 938 (w), 899 (w), 836 (w), 747 (m), 700 (w), 607 (w), 550 (w), 527 (w), 469 (w), 435 (m). | | | |
| **MS** (ESI+): | m/z (%) = 314 (100, [M-Cl]⁺) | | | |
| **HRMS** (ESI+): | m/z für C₁₂H₁₅N₃O₃S₂ | | | |
| | ber.: | 313.0555 | | gef.: 313.0580 |
| **EA:** | ber.: | C:41.20 | H:4.61 | N: 12.01 |
| | gef.: | C:41.19 | H:4.63 | N: 12.07 |

### Darstellung von Cyclohexylmethylpiperazin-1-carbodithioathydrochlorid (Schl32323):

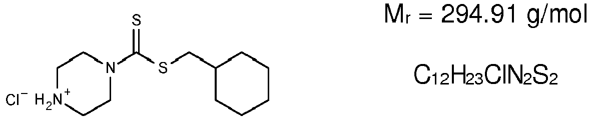

Gemäß **AAV11** wurden 618 mg *tert*-Butyl-4-(Cyclohexylmethylsulfanylcarbothioyl)-piperazin-1-carboxylat **(Schl32316,** 1.72 mmol, 1.0 eq) umgesetzt. Es wurden 143 mg von **Schl32323** (28.2%) in Form eines farblosen Feststoffes erhalten.

| | | | | |
|---|---|---|---|---|
| **Fp**.**:** | 218 °C | | | |
| **¹H-NMR:** | (DMSO-D₆, 400 MHz), δ [ppm] = 9.67 (*sbr,* 2H, N*H*₂⁺), 4.40-4.14 (*m*, 4H, N-C*H₂*-CH₂-NH₂⁺, N-C*H*₂-CH₂-NH₂⁺), 3.28 (*d,* ³J = 6.9 Hz, 2H, S-C*H₂-*CH), 3.20-3.09 (*m*, 4H, N-CH₂-C*H₂*-NH₂⁺, N-CH₂-C*H*₂-NH₂⁺), 1.79-1.69 (*m*, 2H, C*H*_{(Cyclohexyl)}), 1.67-1.49 (*m*, 3H C*H*_{(Cyclohexyl}), S-CH₂-C*H*), 1.21-1.08 (*m*, 4H, C*H*_{(Cyclohexyl)}), 1.04-0.88 (*m*, 4H, C*H*_{(Cyclohexyl)}). | | | |
| **¹³C-NMR:** | (DMSO-D₆, 100 MHz), δ [ppm] = 198.0 (C=S), 44.0 (S-CH₂-CH), 42.5 (N-CH₂-CH₂-NH₂⁺, N-CH₂-CH₂-NH₂⁺), 37.2 (S-CH₂-CH), 32.7 (CH_{(Cyclohexyl)}), 26.3 (CH_{(Cyclohexyl)}), 26.0 (CH_{(Cyclohexyl)}). | | | |
| **IR (ATR):** | v (cm⁻¹) = 3087 (w), 3009 (m), 2921 (w), 1546 (w), 1474 (w), 1463 (w), 1448 (m), 1403 (w), 1357 (w), 1332 (w), 1308 (w), 1295 (w), 1259 (s), 1251 (w), 1231 (w), 1189 (w), 1141 (m), 1078 (w), 1030 (w), 1012 (m), 1002 (m), 981 (vs), 933 (w), 894 (w), 877 (m), 851 (w), 813 (w), 765 (w), 736 (w), 557 (w), 538 (m), 487 (w), 436 (m), 422 (m), 407 (m). | | | |
| **MS** (ESI+): | m/z (%) = 259 (100, [M-Cl]⁺) | | | |
| **HRMS** (ESI+): | m/z für C₁₂H₂₂N₂S₂ | | | |
| | ber.: | 258.1224 | | gef.: 258.1236 |
| **EA:** | ber.: | C: 48.87 | H: 7.86 | N: 9.50 |
| | gef.: | C: 48.76 | H: 7.89 | N: 9.67 |

### Darstellung von (7-Methoxy-2-oxochromen-4-yl)methylpiperazin-1-carbodithioathydrochlorid (Schl32324):

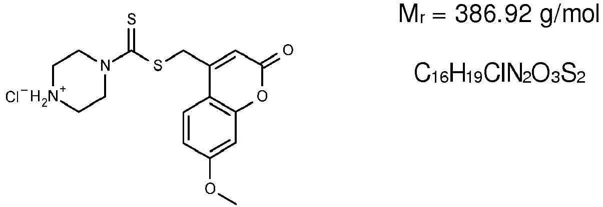

Gemäß **AAV11** wurden 1162 mg *tert*-Butyl-4-[(7-methoxy-2-oxochromen-4-yl)methylsulfanyl-carbothioyl]-piperazin-1-carboxylat **(Schl32317,** 2.58 mmol, 1.0 eq) umgesetzt. Es wurden 838 mg von **Schl32324** (84.0%) in Form eines farblosen Feststoffes erhalten.

| | | | | |
|---|---|---|---|---|
| **Fp**.**:** | 237 °C | | | |
| **¹H-NMR:** | (DMSO-D₆, 400 MHz), δ [ppm] = 9.66 (*sbr,* 2H, N*H*₂⁺), 7.76 (d, ³J = 8.7 Hz, 1H, C-C*H*-CH-C-O-CH₃), 7.01-6.90 (*m*, 2H, C-CH-C*H*-C-O-CH₃, C-C*H*-C-O-CH₃)₃ 6.38 (*s*, 1H, C-C*H*-C=O), 4.78 (*s,* 2H, S-C*H*₂-C), 4.48-4.13 (*m*, 4H, N-C*H₂-*CH₂-NH₂⁺, N-C*H*₂-CH₂-NH₂⁺), 3.83 (*s*, 3H, O-C*H*₃), 3.23-3.15 (m, 4H, N-CH₂-C*H₂*-NH₂⁺, N-CH₂-C*H₂*-NH₂⁺). | | | |
| **¹³C-NMR:** | (DMSO-D₆, 100 MHz), δ [ppm] = 195.5 (C=S), 163.1 (C(=O)-O), 160.5 (C-OMe), 159.2 (S-CH₂-C), 155.6 (C(=O)-O-C), 151.5 (C(=O)-C), 126.7 (C-CH-CH-C-OMe), 112.9 (C-CH-C(=O)), 112.2 (C-CH-CH-C-OMe), 101.6 (C-CH-C-OMe), 56.6 (O-CH₃), 42.5 (N-CH₂-CH₂-NH₂⁺ , N-CH₂-CH₂-NH₂⁺), 37.1 (S-CH₂-C). | | | |
| **IR (ATR):** | v (cm⁻¹) = 3064 (w), 3004 (w), 2909 (w), 1715 (vs), 1605 (s), 1567 (w), 1510 (w), 1469 (w), 1443 (w), 1414 (m), 1383 (m), 1359 (w), 1344 (m), 1315 (w), 1298 (w), 1191 (w), 1150 (s), 1133 (vs), 1080 (w), 1046 (w), 1027 (m), 1009 (vs), 988 (w), 893 (m), 857 (w), 842 (vs), 815 (w), 769 (w), 704 (w), 669 (w), 614 (w), 590 (w), 486 (w), 470 (m), 437 (w), 411 (w). | | | |
| **MS** (ESI+): | m/z (%) = 351 (100, [M-Cl]⁺) | | | |
| **HRMS** (ESI+): | m/z für C₁₂H₁₈N₂O₃S₂ | | | |
| | ber.: | 350.0759 | | gef.: 350.0730 |
| **EA:** | ber.: | C: 49.67 | H: 4.95 | N: 7.24 |
| | gef.: | C: 49.40 | H: 4.99 | N: 7.47 |

### Darstellung von 2-(1H-Indol-3-yl)ethylpiperazin-1-carbodithioathydrochlorid (Schl32325):

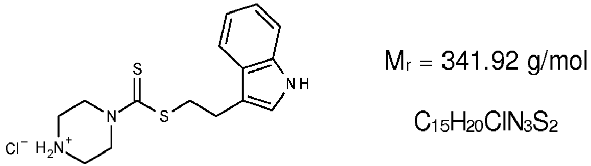

Gemäß **AAV11** wurden 1263 mg *tert-*Butyl-4-[2-(1*H-*indol-3-yl)ethylsulfanyl-carbothioyl]piperazin-1-carboxylat **(Schl32318,** 3.11 mmol, 1.0 eq) umgesetzt. Es wurden 1023 mg von **Schl32325** (96.1 %) in Form eines hellbraunen Feststoffes erhalten.

| | | | | |
|---|---|---|---|---|
| **Fp**.**:** | 240 °C | | | |
| **¹H-NMR:** | (DMSO-D₆, 400 MHz), δ [ppm] = 10.90 (*sbr,* 1H, N*H*), 9.72 (*sbr,* 2H, N*H*₂⁺), 7.59 (d, ³J = 7.7 Hz, CH₂-C-C-C*H*), 7.32 (*d*, ³J = 8.0 Hz, N-C-C*H*), 7.17 (*s*, 1H, C-C*H*-NH), 7.04 *(t,* ³J = 7.0 Hz, 1H, C-CH-C*H*-CH-CH), 6.96 (*t*, ³J = 7.0 Hz, 1H, C-CH-C*H*-CH-CH), 4.48-4.16 (*m*, 4H, N-C*H₂*-CH₂-NH₂⁺, N-C*H*₂-CH₂-NH₂⁺), 3.54 *(t,* ³J = 7.5 Hz, 2H, S-C*H*₂-CH₂-C), 3.21-3.11 (*m*, 4H, N-CH₂-C*H₂*-NH₂⁺, N-CH₂-C*H₂*-NH₂⁺), 3.04 *(t,* ³J = 7.6 Hz, 2H, S-CH₂-C*H₂*-C). | | | |
| **¹³C-NMR:** | (DMSO-D₆, 100 MHz), δ [ppm] = 197.8 (C=S), 136.8 (NH-C-CH), 127.5 (NH-C-C), 123.5 (C-CH-NH), 121.5 (NH-C-CH-CH), 118.9 (NH-C-CH-CH-CH), 118.8 (NH-C-C-CH), 113.0 (NH-CH-C), 112.0 (NH-C-CH), 66.9 (N-CH₂-CH₂-NH₂⁺, N-CH₂-CH₂-NH₂⁺), 42.5 (S-CH₂-C), 37.8 (S-CH₂-CH₂), 25.0 (S-CH₂-CH₂). | | | |
| **IR (ATR):** | v (cm⁻¹ ) = 3258 (w), 2922 (w), 2784 (w), 1597 (w), 1491 (w), 1458 (w), 1445 (s), 1407 (w), 1390 (w), 1367 (w), 1316 (w), 1301 (w), 1264 (m), 1239 (vs), 1183 (w), 1172 (s), 1134 (w), 1095 (w), 1028 (m), 993 (vs), 962 (w), 903 (w), 871 (w), 860 (w), 817 (w), 779 (w), 758 (vs), 704 (w), 655 (m), 613 (w), 581 (w), 563 (w), 549 (w), 465 (w), 429 (vs). | | | |
| **MS** (ESI+): | m/z (%) = 306 (100, [M-Cl]⁺) | | | |
| **HRMS** (ESI+): | m/z für C₁₅H₁₉N₃S₂ | | | |
| | ber.: | 305.1020 | | gef.: 305.1052 |
| **EA:** | ber.: | C:52.69 | H: 5.90 | N: 12.29 |
| | gef.: | C:52.69 | H: 5.93 | N: 12.27 |

### Darstellung von (4-Nitrophenyl)methyl-4-[2-(tert-butoxycarbonylamino)acetyl]piperazin-1-carbodithioat (Schl32336):

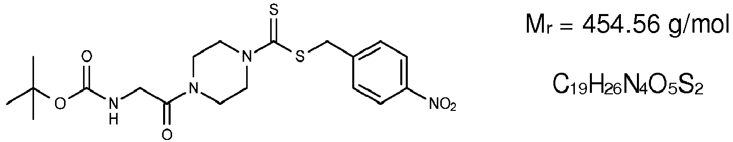

Gemäß **AAV4** wurden 263 mg *Boc-*Gly*-*OH (1.5 mmol, 1.0 eq), 505 mg (4-Nitrophenyl)methylpiperazin-1-carbodithioathydrochlorid **(Schl32319,** 1.5 mmol, 1.0 eq), 1.01 mL Triethylamin (5.25 mmol, 3.5 eq), 338 mg HOBt (2.25 mmol, 1.5 eq) und 479 mg EDC▪HCl (2.25 mmol, 1.5 eq) umgesetzt. Säulenchromatographie an Kieselgel (Cyclohexan/ EtOAc 1:1) ergab 606 mg von **Schl32336** (89.0%) in Form eines blassgelben Feststoffes.

| | | | | |
|---|---|---|---|---|
| **DC:** | R_{f} = 0.30 (Cyclohexan/ EtOAc 1:1). | | | |
| **Fp**.**:** | 58 °C | | | |
| **¹H-NMR:** | (CDCl₃, 400 MHz), δ [ppm] = 8.15 (d, ³J = 8.7 Hz, 2H, C-CH-C*H*-C-NO₂, C-C*H*-CH-C-NO₂), 7.55 (*d,* ³J = 8.9 Hz, 2H, C-CH-C*H*-C-NO₂, C-C*H*-CH-C-NO₂), 5.43 (*sbr,* 1H, N*H*), 4.68 (s, 2H, S-C*H*₂₋C), 4.47-4.27 (*m*, 2H, N-CH*H*-CH₂-N-C(=O), N-CH*H-*CH₂-N-C(=O)), 4.23-4.01 (*m*, 2H, N-CH*H*-CH₂-N-C(=O), N-CH*H-*CH₂-N-C(=O)), 3.97 (*s*, 2H, NH-C*H*₂-C(=O)), 3.75 (*t*, ³J = 5.2 Hz, 2H, N-CH₂-CH*H*-N-C(=O), N-CH₂-CH*H-*N-C(=O)), 3.55 (*t*, ³J = 5.2 Hz, 2H, N-CH₂-CH*H*-N-C(=O), N-CH₂-CH*H-*N-C(=O)), 1.43 (*s,* 9H, C*H*₃). | | | |
| **¹³C-NMR:** | (CDCl₃, 100 MHz), δ [ppm] = 196.3 (C=S), 167.6 (C(=O)-N), 155.9 (O-C(=O)-NH), 147.3 (C(NO₂)), 144.2 (CH₂-C-CH-CH), 130.2 (C(NO₂)-CH-CH, C(NO₂)-CH-CH), 123.8 (C-CH-CH, C-CH-CH), 80.1 (C(CH₃)₃), 43.5 (NH-CH₂-C(=O)), 42.4 (N-CH₂-CH₂-N-C(=O)), 41.2 (N-CH₂-CH₂-N-C(=O)), 40.6 (S-CH₂-C), 28.4 (CH₃). | | | |
| **IR (ATR):** | v (cm⁻¹) =3327 (w), 3077 (w), 2977 (w), 2931 (w), 1707 (w), 1651 (m), 1600 (w), 1518 (m), 1460 (w), 1414 (w), 1365 (w), 1342 (s), 1282 (w), 1244 (w), 1216 (s), 1158 (vs), 1109 (w), 1049 (w), 994 (m), 961 (m), 859 (m), 801 (w), 762 (w), 687 (w), 550 (w), 490 (w). | | | |
| **MS** (ESI+): | m/z (%) = 472 (100, [M+NH₄]⁺) | | | |
| **HRMS** (ESI+): | m/z für C₁₉H₂₆N₄O₅S₂ | | | |
| | ber.: | 454.1345 | | gef.: 454.1321 |
| **EA:** | ber.: | C: 50.20 | H: 5.77 | N:12.33 |
| | gef.: | C: 50.30 | H: 5.79 | N: 12.03 |

### Darstellung von (4-Nitrophenyl)methyl-4-[2-(tert-butoxycarbonylamino)propanoyl]-piperazin-1-carbodithioat (Schl32337):

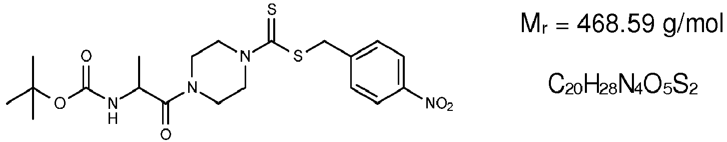

Gemäß **AAV4** wurden 284 mg *Boc*-Ala-OH (1.5 mmol, 1.0 eq), 505 mg (4-Nitrophenyl)methylpiperazin-1-carbodithioathydrochlorid **(Schl32319,** 1.5 mmol, 1.0 eq), 1.01 mL Triethylamin (5.25 mmol, 3.5 eq), 338 mg HOBt (2.25 mmol, 1.5 eq) und 479 mg EDC▪HCl (2.25 mmol, 1.5 eq) umgesetzt. Säulenchromatographie an Kieselgel (Cyclohexan/ EtOAc 1:1) ergab 648 mg von **Schl32337** (92.2%) in Form eines blassgelben Feststoffes.

| | | | | |
|---|---|---|---|---|
| **DC:** | R_{f} = 0.28 (Cyclohexan/ EtOAc 1:1). | | | |
| **Fp**.**:** | 143 °C | | | |
| **¹H-NMR:** | (CDCl₃, 400 MHz), δ [ppm] = 8.15 (*d*, ³J = 8.7 Hz, 2H, C-CH-C*H*-C-NO₂, C-C*H*-CH-C-NO₂), 7.55 (*d,* ³J = 8.7 Hz, 2H, C-CH-C*H*-C-NO₂, C-C*H*-CH-C-NO₂), 5.37 (*sbr,* 1H, N*H*), 4.68 (*s*, 2H, S-C*H*₂₋C), 4.61 (*q*, ³J = 7.1 Hz, 1H, NH-C*H-*C(=O)), 4.39-4.21 (*m*, 2H, N-CH*H*-CH₂-N-C(=O), N-CH*H-*CH₂-N-C(=O)), 4.18-4.01 (*m*, 2H, N-CH*H*-CH₂-N-C(=O), N-CH*H-*CH₂-N-C(=O)), 3.95-3.85 (*m*, 2H, N-CH₂-CH*H*-N-C(=O), N-CH₂-CH*H-*N-C(=O)), 3.63-3.51 (*m*, 2H, N-CH₂-CH*H*-N-C(=O), N-CH₂-CH*H-*N-C(=O)), 1.42 (*s*, 9H, C*H*₃), 1.30 (*d,* ³J = 6.9 Hz, 3H, CH-C*H*₃). | | | |
| **¹³C-NMR:** | (CDCl₃, 100 MHz), δ [ppm] = 196.2 (C=S), 171.9 (C(=O)-N), 155.2 (O-C(=O)-NH), 147.3 (C(NO₂)), 144.3 (CH₂-C-CH-CH), 130.2 (C(NO₂)-CH-CH, C(NO₂)-CH-CH), 123.8 (C-CH-CH, C-CH-CH), 80.0 (C(CH₃)₃), 46.1 (NH-CH-C(=O)), 44.5 (N-CH₂-CH₂-N-C(=O)), 41.4 (N-CH₂-CH₂-N-C(=O)), 40.6 (S-CH₂-C), 28.4 (C-CH₃), 19.2 (CH-CH₃). | | | |
| **IR (ATR):** | v (cm⁻¹) =3503 (w), 2980 (w), 1689 (m), 1653 (s), 1606 (w), 1524 (vs), 1462 (m), 1426 (s), 1383 (w), 1365 (vs), 1314 (w), 1275 (w), 1239 (s), 1219 (w), 1160 (vs), 1107 (w), 1065 (m), 1010 (s), 991 (s), 950 (w), 900 (w), 860 (s), 784 (w), 710 (m), 639 (w), 563 (w), 529 (w), 492 (m). | | | |
| **MS** (ESI+): | m/z (%) = 486 (100, [M+NH₄]⁺) | | | |
| **HRMS** (ESI+): | m/z für C₂oH₂₈N₄O₅S₂ | | | |
| | ber.: | 468.1501 | | gef.: 468.1498 |
| **EA:** | ber.: | C: 51.26 | H: 6.02 | N: 11.96 |
| | gef.: | C: 50.91 | H: 6.09 | N: 11.75 |

### Darstellung von (4-Nitrophenyl)methyl-4-[4-amino-2-(tert-butoxycarbonylamino)-4-oxo-butanoyl]piperazin-1-carbodithioat (Schl32338):

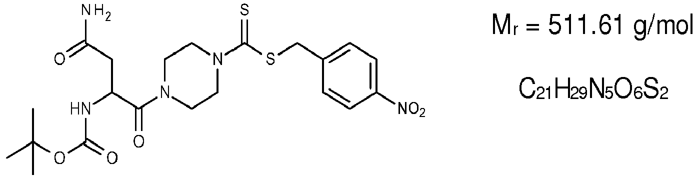

Gemäß **AAV4** wurden 406 mg Boc-Asn-OH (1.75 mmol, 1.0 eq), 590 mg (4-Nitrophenyl)methylpiperazin-1-carbodithioathydrochlorid **(Schl32319,** 1.75 mmol, 1.0 eq), 1.11 mL Triethylamin (6.13 mmol, 3.5 eq), 395 mg HOBt (2.63 mmol, 1.5 eq) und 560 mg EDC▪HCl (2.63 mmol, 1.5 eq) umgesetzt. Säulenchromatographie an Kieselgel (Cyclohexan/ EtOAc 1:1) ergab 371 mg von **Schl32338** (41.5%) in Form eines blassgelben Feststoffes.

| | | | | |
|---|---|---|---|---|
| **DC:** | R_{f} = 0.29 (Cyclohexan/ EtOAc 1:1). | | | |
| **Fp**.**:** | 77 °C | | | |
| **¹H-NMR:** | (CDCl₃, 400 MHz), δ [ppm] = 8.16 (d, ³J = 8.7 Hz, 2H, C-CH-C*H*-C-NO₂, C-C*H*-CH-C-NO₂), 7.55 (*d,* ³J = 8.9 Hz, 2H, C-CH-C*H*-C-NO₂, C-C*H*-CH-C-NO₂), 5.41 (*sbr,* 1H, N*H*), 4.92 (*t*, ³J = 6.9 Hz, 1H, NH-C*H-*C(=O)), 4.68 (s, 2H, S-C*H*₂₋C), 4.51-4.33 (*m*, 2H, N-CH*H*-CH₂-N-C(=O), N-CH*H-*CH₂-N-C(=O)), 4.27-4.01 (*m*, 2H, N-CH*H*-CH₂-N-C(=O), N-CH*H-*CH₂-N-C(=O)), 3.83-3.64 (*m*, 4H, N-CH₂-C*H*₂-N-C(=O), N-CH₂-C*H₂-*N-C(=O)), 2.80-2.74 (*m*, 2H, CH-C*H₂-*C(=O)NH₂), 1.43 (s, 9H, C*H*₃). | | | |
| **¹³C-NMR:** | (CDCl₃, 100 MHz), δ [ppm] = 196.3 (C=S), 167.9 (C(=O)-N), 154.6 (O-C(=O)-NH), 147.3 (C(NO₂)), 144.2 (CH₂-C-CH-CH), 130.2 (C(NO₂)-CH-CH, C(NO₂)-CH-CH), 123.8 (C-CH-CH, C-CH-CH), 116.5 (C(=O)NH₂), 81.5 (C(CH₃)₃), 46.9 (NH-CH-C(=O)), 44.9 (N-CH₂-CH₂-N-C(=O)), 41.9 (N-CH₂-CH₂-N-C(=O)), 40.6 (S-CH₂-C), 28.3 (CH₃), 22.0 (CH-CH₂-C(=O)NH₂). | | | |
| **IR (ATR):** | v (cm⁻¹) = 3310 (w), 2979 (w), 2932 (w), 1706 (m), 1646 (s), 1600 (w), 1519 (vs), 1456 (m), 1416 (vs), 1367 (m), 1344 (vs), 1282 (m), 1249 (w), 1218 (vs), 1157 (vs), 1109 (w), 1046 (w), 1011 (m), 995 (s), 972 (m), 943 (w), 859 (m), 803 (w), 722 (w), 686 (w), 622 (w), 526 (w), 493 (w). | | | |
| **MS** (ESI+): | m/z (%) = 512 (100, [M+H]⁺) | | | |
| **HRMS** (ESI+): | m/z für C₂₁H₂₉N₅O₆S₂ | | | |
| | ber.: | 511.1559 | | gef.: 511.1553 |
| **EA:** | ber.: | C:49.30 | H: 5.71 | N: 13.69 |
| | gef.: | C:49.25 | H: 5.60 | N: 13.83 |

### Darstellung von (4-Nitrophenyl)methyl-4-[2-(tert-butoxycarbonylamino)-4-methylsulfanyl-butanoyl]piperazin-1-carbodithioat (Schl32339):

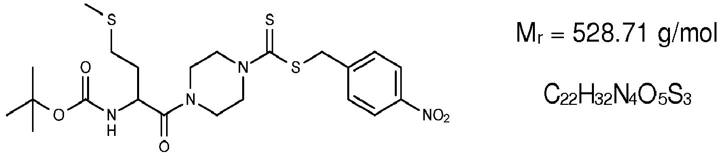

Gemäß **AAV4** wurden 374 mg *Boc*-Met-OH (1.5 mmol, 1.0 eq), 505 mg (4-Nitrophenyl)methylpiperazin-1-carbodithioathydrochlorid **(Schl32319,** 1.5 mmol, 1.0 eq), 1.01 mL Triethylamin (5.25 mmol, 3.5 eq), 338 mg HOBt (2.25 mmol, 1.5 eq) und 479 mg EDC▪HCl (2.25 mmol, 1.5 eq) umgesetzt. Säulenchromatographie an Kieselgel (Cyclohexan/ EtOAc 1:1) ergab 700 mg von **Schl32339** (88.2%) in Form eines blassgelben Feststoffes.

| | | | | |
|---|---|---|---|---|
| **DC:** | R_{f} = 0.38 (Cyclohexan/ EtOAc 1:1). | | | |
| **Fp**.**:** | 49 °C | | | |
| **¹H-NMR:** | (CDCl₃, 400 MHz), δ [ppm] = 8.16 (*d*, ³J = 8.7 Hz, 2H, C-CH-C*H*-C-NO₂, C-C*H*-CH-C-NO₂), 7.55 (*d,* ³J = 8.7 Hz, 2H, C-CH-C*H*-C-NO₂, C-C*H*-CH-C-NO₂), 5.28 (*sbr,* 1H, N*H*), 4.78 (*q*, ³J = 7.8 Hz, 1H, NH-C*H-*C(=O)), 4.68 (s, 2H, S-C*H*₂₋C), 4.47-4.27 (*m*, 2H, N-CH*H*-CH₂-N-C(=O), N-CH*H-*CH₂-N-C(=O)), 4.23-4.01 (*m*, 2H, N-CH*H*-CH₂-N-C(=O), N-CH*H-*CH₂-N-C(=O)), 3.91-3.75 (*m*, 4H, N-CH₂-C*H*₂-N-C(=O), N-CH₂-C*H₂₋*N-C(=O)), 2.56-2.50 (*m*, 2H, S-C*H₂*-CH₂-CH), 2.09 (*s*, 1H, S-C*H*₃), 1.96-1.77 (*m*, 2H, S-CH₂-C*H₂*-CH), 1.42 *(s,* 9H, C*H*₃). | | | |
| **¹³C-NMR:** | (CDCl₃, 100 MHz), δ [ppm] = 196.1 (C=S), 171.1 (C(=O)-N), 155.6 (O-C(=O)-NH), 147.3 (C(NO₂)), 144.3 (CH₂-C-CH-CH), 130.2 (C(NO₂)-CH-CH, C(NO₂)-CH-CH), 123.8 (C-CH-CH, C-CH-CH), 80.3 (C(CH₃)₃), 48.9 (NH-CH-C(=O)), 44.6 (N-CH₂-CH₂-N-C(=O)), 41.5 (N-CH₂-CH₂-N-C(=O)), 40.6 (S-CH₂-C), 32.9 (S-CH₂-CH₂-CH), 30.3 (S-CH₂-CH₂-CH), 28.4 (CH₃),15.8 (S-CH₃). | | | |
| **IR (ATR):** | v (cm⁻¹ ) = 3311 (w), 2977 (w), 2919 (w), 1703 (w), 1641 (s), 1600 (w), 1519 (s), 1416 (vs), 1366 (m), 1343 (vs), 1279 (m), 1217 (vs), 1158 (vs), 1109 (w), 1046 (w), 1011 (s), 994 (s), 961 (m), 859 (s), 802 (w), 746 (w), 721 (m), 621 (w), 550 (w), 516 (m), 490 (m), 411 (w). | | | |
| **MS** (ESI+): | m/z (%) = 529 (100, [M+H]⁺) | | | |
| **HRMS** (ESI+): | m/z für C₂₂H₃₂N₄O₅S₃ | | | |
| | ber.: | 528.1535 | | gef.: 528.1514 |
| **EA:** | ber.: | C:49.98 | H:6.10 | N: 10.60 |
| | gef.: | C: 49.98 | H:6.18 | N: 10.76 |

### Darstellung von tert-Butyl-2-[4-[(4-nitrophenyl)methylsulfanylcarbothioyl]piperazin-1-carbonyl]pyrrolidin-1-carboxylat (Schl32340):

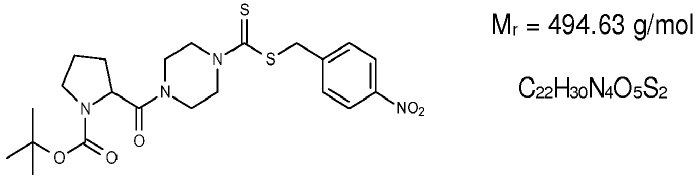

Gemäß **AAV4** wurden 371 mg Boc-Pro-OH (1.75 mmol, 1.0 eq), 590 mg (4-Nitrophenyl)methylpiperazin-1-carbodithioathydrochlorid **(Schl32319,** 1.75 mmol, 1.0 eq), 1.11 mL Triethylamin (6.13 mmol, 3.5 eq), 395 mg HOBt (2.63 mmol, 1.5 eq) und 560 mg EDC▪HCl (2.63 mmol, 1.5 eq) umgesetzt. Säulenchromatographie an Kieselgel (Cyclohexan/ EtOAc 1:1) ergab 733 mg von **Schl32340** (84.6%) in Form eines blassgelben Feststoffes.

| | | | | |
|---|---|---|---|---|
| **DC**: | R_{f} = 0.11 (Cyclohexan/ EtOAc 1:1). | | | |
| **Fp**.**:** | 139 °C | | | |
| **¹H-NMR:** | (CDCl₃, 400 MHz), δ [ppm] = 8.15 (*d*, ³J = 8.7 Hz, 2H, C-CH-C*H*-C-NO₂, C-C*H*-CH-C-NO₂), 7.55 (*d,* ³J = 8.9 Hz, 2H, C-CH-C*H*-C-NO₂, C-C*H*-CH-C-NO₂), 4.68 (*s*, 2H, S-C*H*₂₋C), 4.67-4.62 (*m*, 1H, N-C*H*-C(=O)), 4.59-4.41 (*m*, 2H, N-CH*H*-CH₂-N-C(=O), N-CH*H-*CH₂-N-C(=O)), 4.36-4.11 (m, 2H, N-CH*H*-CH₂-N-C(=O), N-CH*H*-CH₂-N-C(=O)), 3.99-3.81 (m, 2H, N-CH₂-CH*H*-N-C(=O), N-CH₂-CH*H-*N-C(=O)), 3.75-3.60 (*m*, 2H, N-CH₂-CH*H*-N-C(=O), N-CH₂-CH*H-*N-C(=O)), 3.58-3.48 (*m*, 2H, N-C*H₂*-CH₂-CH₂-CH), 2.22-2.04 (m, 2H, N-CH₂-CH₂-C*H₂*-CH), 1.90-1.77 (m, 2H, N-CH₂-C*H₂*-CH₂-CH), 1.43 (*s*, 9H, C*H*₃). | | | |
| **¹³C-NMR:** | (CDCl₃, 100 MHz), δ [ppm] = 197.3 (C=S), 171.7 (C(=O)-N), 154.6 (O-C(=O)-NH), 147.3 (C(NO₂)), 144.4 (CH₂-C-CH-CH), 130.2 (C(NO₂)-CH-CH, C(NO₂)-CH-CH), 123.8 (C-CH-CH, C-CH-CH), 79.9 (C(CH₃)₃), 56.8 (N-CH-C(=O)), 46.9 (N-CH₂-CH₂-N-C(=O)), 44.6 (N-CH₂-CH₂-CH₂-CH), 41.4 (N-CH₂-CH₂-N-C(=O)), 40.6 (S-CH₂-C), 29.9 (N-CH₂-CH₂-CH₂-CH), 28.6 (CH₃), 24.5 (N-CH₂-CH₂-CH₂-CH). | | | |
| **IR (ATR):** | v (cm⁻¹) = 2970 (w), 2892 (w), 1689 (vs), 1651 (s), 1599 (w), 1516 (m), 1455 (w), 1423 (s), 1403 (vs), 1351 (w), 1282 (vs), 1245 (w), 1211 (vs), 1189 (w), 1162 (s), 1082 (m), 1027 (w), 996 (s), 978 (m), 920 (w), 876 (w), 855 (m), 757 (w), 725 (m), 643 (w), 592 (w), 518 (w), 467 (w). | | | |
| **MS** (ESI+): | m/z (%) = 512 (100, [M+NH₄]⁺) | | | |
| **HRMS** (ESI+): | m/z für C₂₂H₃₀N₄O₅S₂ | | | |
| | ber.: | 494.1658 | | gef.: 494.1632 |
| **EA:** | ber.: | C: 53.42 | H: 6.11 | N: 11.33 |
| | gef.: | C: 53.42 | H: 6.11 | N: 11.24 |

### Darstellung von (4-Nitrophenyl)methyl-4-[2-(tert-butoxycarbonylamino)-3-(4-hydroxyphenyl)propanoyl]piperazin-1-carbodithioat (Schl32341):

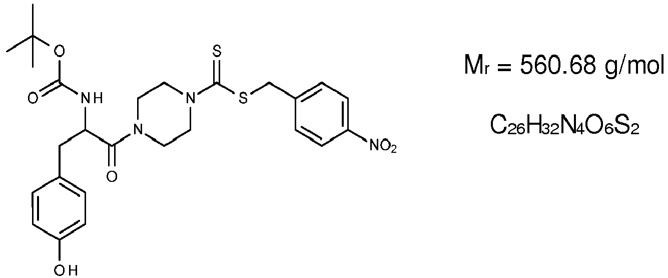

Gemäß **AAV4** wurden 492 mg *Boc*-Tyr-OH (1.75 mmol, 1.0 eq), 590 mg (4-Nitrophenyl)methylpiperazin-1-carbodithioathydrochlorid **(Schl32319,** 1.75 mmol, 1.0 eq), 1.11 mL Triethylamin (6.13 mmol, 3.5 eq), 395 mg HOBt (2.63 mmol, 1.5 eq) und 560 mg EDC▪HCl (2.63 mmol, 1.5 eq) umgesetzt. Säulenchromatographie an Kieselgel (Cyclohexan/ EtOAc 2:3) ergab 795 mg von **Schl32341** (81.0%) in Form eines blassgelben Feststoffes.

| | | | | |
|---|---|---|---|---|
| **DC**: | R_{f} = 0.49 (Cyclohexan/ EtOAc 2:3). | | | |
| **Fp**.**:** | 97 °C | | | |
| **¹H-NMR:** | (CDCl₃, 400 MHz), δ [ppm] = 8.13 (*d*, ³J = 8.7 Hz, 2H, C-CH-C*H*-C-NO₂, C-C*H*-CH-C-NO₂), 7.52 (*d,* ³J = 8.9 Hz, 2H, C-CH-C*H*-C-NO₂, C-C*H*-CH-C-NO₂), 7.02 (*d,* ³J = 8.5 Hz, 2H, OH-C-CH-C*H*-C, HO-C-CH-C*H*-C), 6.74 (*d*, ³J = 8.5 Hz, 2H, HO-C-C*H*-CH-C, HO-C-CH-C*H*-C), 5.42 (*sbr,* 1H, N*H*), 4.79-4.70 (*m,* 1H, NH-C*H*-C(=O)), 4.63 (s, 2H, S-C*H₂*-C), 4.00-3.81 (*m*, 2H, N-CH*H*-CH₂-N-C(=O), N-CH*H-*CH₂-N-C(=O)), 3.71-3.53 (*m*, 2H, N-CH*H*-CH₂-N-C(=O), N-CH*H-*CH₂-N-C(=O)), 3.44-3.23 (m, 2H, N-CH₂-CH*H*-N-C(=O), N-CH₂-CH*H*-N-C(=O)), 3.19-3.00 (*m*, 2H, N-CH₂-CH*H*-N-C(=O), N-CH₂-CH*H*-N-C(=O)), 2.91-2.83 (*m*, 2H, CH-C*H₂*-C), 1.41 *(s,* 9H, C*H₃*). | | | |
| **¹³C**-**NMR:** | (CDCl₃, 100 MHz), δ [ppm] = 195.8 (C=S), 171.2 (C(=O)-N), 155.5 (O-C(=O)-NH), 147.3 (C(NO₂)), 144.3 (S-CH₂-C-CH-CH), 130.7 (C(NO₂)-CH-CH, C(NO₂)-CH-CH), 130.2 (CH-CH-C-OH, CH-CH-C-OH), 127.6 (C-OH), 123.8 (C-CH-CH, C-CH-CH), 121.7 (CH-CH₂-C), 115.7 (CH-C-OH, CH-C-OH), 80.5 (C(CH₃)₃), 51.4 (NH-CH-C(=O)), 44.5 (N-CH₂-CH₂-N-C(=O)), 41.2 (N-CH₂-CH₂-N-C(=O)), 40.6 (S-CH₂-C), 39.5 (CH-CH₂-C), 28.4 (CH₃). | | | |
| **IR (ATR):** | v (cm⁻¹) = 2977 (w), 2932 (w), 1763 (w), 1703 (w), 1638 (w), 1598 (w), 1516 (s), 1450 (w), 1416 (m), 1366 (w), 1344 (vs), 1278 (w), 1217 (s), 1158 (vs), 1105 (w), 1045 (w), 1011 (m), 995 (m), 859 (w), 802 (w), 721 (w), 686 (w), 622 (w), 537 (m), 491 (w). | | | |
| **MS** (ESI-): | m/z (%) = 559 (100, [M-H]⁻) | | | |
| **HRMS** (ESI+): | m/z für C₂₆H₃₂N₄O₆S₂ | | | |
| | ber.: | 560.1763 | | gef.: 560.1726 |
| **EA:** | ber.: | C:55.70 | H:5.75 | N:9.99 |
| | gef.: | C:56.04 | H: 5.90 | N: 9.69 |

### Darstellung von (4-Nitrophenyl)methyl-4-[3-(tert-butoxycarbonylamino)propanoyl]-piperazin-1-carbodithioat (Schl32342):

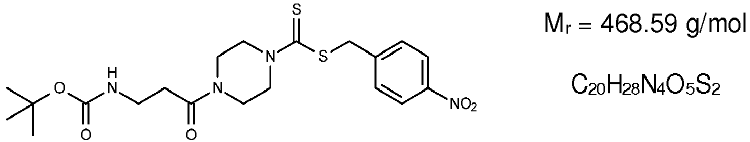

Gemäß **AAV4** wurden 284 mg *Boc-β*-Ala-OH (1.5 mmol, 1.0 eq), 505 mg (4-Nitrophenyl)methylpiperazin-1-carbodithioathydrochlorid (**Schl32319**, 1.5 mmol, 1.0 eq), 1.01 mL Triethylamin (5.25 mmol, 3.5 eq), 338 mg HOBt (2.25 mmol, 1.5 eq) und 479 mg EDC·HCl (2.25 mmol, 1.5 eq) umgesetzt. Säulenchromatographie an Kieselgel (Cyclohexan/ EtOAc 1:3) ergab 650 mg von **Schl32342** (92.5%) in Form eines blassgelben Feststoffes.

| | | | | |
|---|---|---|---|---|
| **DC**: | R_{f} = 0.34 (Cyclohexan/ EtOAc 1:3). | | | |
| **Fp.:** | 49 °C | | | |
| **¹H-NMR:** | (CDCl₃, 400 MHz), δ [ppm] = 8.16 (d, ³J = 8.7 Hz, 2H, C-CH-C*H*-C-NO₂, C-CH-C*H*-C-NO₂), 7.55 (*d*, ³J = 8.9 Hz, 2H, C-CH-C*H*-C-NO₂, C-CH-C*H*-C-NO₂), 5.20 (*sbr,* 1H, N*H*), 4.68 (*s,* 2H, S-C*H₂*-C), 4.47-4.29 (*m,* 2H, N-CH*H*-CH₂-N-C(=O), N-CH*H-*CH₂-N-C(=O)), 4.13-3.95 (*m*, 2H, N-CH*H*-CH₂-N-C(=O), N-CH*H*-CH₂-N-C(=O)), 3.74 (*t*, ³J = 5.2 Hz, 2H, N-CH₂-CH*H*-N-C(=O), N-CH₂-CH*H*-N-C(=O)), 3.58 (*t*, ³J = 5.0 Hz, 2H, N-CH₂-CH*H*-N-C(=O), N-CH₂-CH*H*-N-C(=O)), 3.43 (*t*, ³J = 4.6 Hz, 2H, NH-C*H₂*-CH₂-C(=O)), 2.53 (*t*, ³J = 5.7 Hz, 2H, NH-CH₂-C*H₂*-C(=O)), 1.42 (s, 9H, C*H₃*). | | | |
| **¹³C**-**NMR:** | (CDCl₃, 100 MHz), δ [ppm] = 196.1 (C=S), 170.6 (C(=O)-N), 156.1 (O-C(=O)-NH), 147.3 (C(NO₂)), 144.3 (CH₂-C-CH-CH), 130.2 (C(NO₂)-CH-CH, C(NO₂)-CH-CH), 123.8 (C-CH-CH, C-CH-CH), 79.5 (C(CH₃)₃), 44.3 (N-CH₂-CH₂-N-C(=O)), 40.7 (N-CH₂-CH₂-N-C(=O)), 40.6 (S-CH₂-C), 36.3 (NH-CH₂-CH₂-C(=O)), 33.6 (NH-CH₂-CH₂-C(=O)), 28.5 (CH₃). | | | |
| **IR (ATR):** | v (cm⁻¹) = 2977 (w), 2932 (w), 1703 (m), 1639 (s), 1600 (w), 1518 (s), 1458 (w), 1415 (vs), 1365 (m), 1343 (vs), 1279 (s), 1244 (m), 1216 (vs), 1160 (vs), 1108 (w), 1070 (w), 1012 (s), 994 (vs), 859 (m), 802 (w), 758 (w), 687 (w), 622 (w), 524 (m), 491 (w), 411 (w). | | | |
| **MS** (ESI+): | m/z (%) = 469 (40, [M+H]⁺), 486 (100, [M+NH₄]⁺) | | | |
| **HRMS** (ESI+): | m/z für C₂₀H₂₈N₄O₅S₂ | | | |
| | ber.: | 468.1501 | | gef.: 468.1501 |
| **EA:** | ber.: | C:51.26 | H:6.02 | N:11.96 |
| | gef.: | C:51.14 | H:6.06 | N:11.80 |

### Darstellung von (4-Nitrophenyl)methyl-4-[2-(tert-butoxycarbonylamino)-3-hydroxy-propanoyl]piperazin-1-carbodithioat (Schl32354):

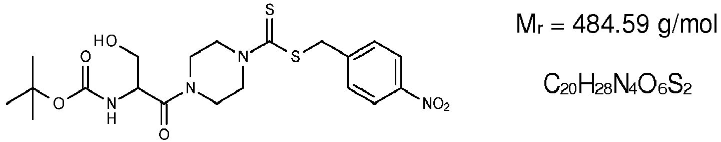

Gemäß **AAV4** wurden 308 mg Boc-Ser-OH (1.5 mmol, 1.0 eq), 505 mg (4-Nitrophenyl)methylpiperazin-1-carbodithioathydrochlorid (**Schl32319**, 1.5 mmol, 1.0 eq), 1.01 mL Triethylamin (5.25 mmol, 3.5 eq), 338 mg HOBt (2.25 mmol, 1.5 eq) und 479 mg EDC·HCl (2.25 mmol, 1.5 eq) umgesetzt. Säulenchromatographie an Kieselgel (Cyclohexan/ EtOAc 1:1) ergab 524 mg von **Schl32354** (72.1 %) in Form eines blassgelben Feststoffes.

| | | | | |
|---|---|---|---|---|
| **DC**: | R_{f} = 0.11 (Cyclohexan/ EtOAc 1:1). | | | |
| **Fp.:** | 161 °C | | | |
| **¹H-NMR:** | (CDCl₃, 400 MHz), δ [ppm] = 8.16 (*d,* ³J = 8.7 Hz, 2H, C-CH-C*H*-C-NO₂, C-CH-C*H*-C-NO₂), 7.55 (*d*, ³J = 8.7 Hz, 2H, C-CH-C*H*-C-NO₂, C-CH-C*H*-C-NO₂), 5.60 (*sbr,* 1H, N*H*), 4.68 (*s,* 2H, S*-*C*H₂-*C), 4.64-4.60 (*m,* 1H, NH-C*H*-C(=O)), 4.49-4.25 (*m,* 2H, N-CH*H*-CH₂-N-C(=O), N-CH*H-*CH₂-N-C(=O)), 4.21-3.95 (*m*, 2H, N-CH*H*-CH₂-N-C(=O), N-CH*H-*CH₂-N-C(=O)), 3.88 (*t*, ³J = 6.5 Hz, 2H, CH-C*H*₂-OH), 3.72 (*t*, ³J = 5.2 Hz, 2H, N-CH₂-CH*H*-N-C(=O), N-CH₂-CH*H*-N-C(=O)), 3.68 (*t*, ³J = 5.4 Hz, 2H, N-CH₂-CH*H*-N-C(=O), N-CH₂-CH*H*-N-C(=O)), 2.46 (*sbr,* 1H, O*H*), 1.43 (*s,* 9H, C*H₃*). | | | |
| **¹³C**-**NMR:** | (CDCl₃, 100 MHz), δ [ppm] = 196.2 (C=S), 170.4 (C(=O)-N), 155.7 (O-C(=O)-NH), 147.3 (C(NO₂)), 144.3 (CH₂-C-CH-CH), 130.2 (C(NO₂)-CH-CH, C(NO₂)-CH-CH), 123.8 (C-CH-CH, C-CH-CH), 80.8 (C(CH₃)₃), 64.1 (CH-CH₂-OH), 51.1 (NH-CH-C(=O)), 44.7 (N-CH₂-CH₂-N-C(=O)), 41.6 (N-CH₂-CH₂-N-C(=O)), 40.6 (S-CH₂-C), 28.4 (CH₃). | | | |
| **IR (ATR):** | v (cm⁻¹) = 2720 (w), 2454 (w), 1646 (s), 1609 (w), 1537 (m), 1459 (m), 1423 (m), 1398 (w), 1346 (vs), 1314 (w), 1280 (m), 1215 (s), 1161 (m), 1107 (w), 1017 (m), 993 (s), 936 (w), 863 (m), 800 (w), 705 (m), 625 (w), 550 (w), 508 (w), 492 (m). | | | |
| **MS** (ESI+): | m/z (%) = 485 (100, [M+H]⁺), 502 (50, [M+NH₄]⁺) | | | |
| **HRMS** (ESI+): | m/z für C₂₀H₂₈N₄O₆S₂ | | | |
| | ber.: | 484.1450 | | gef.: 484.1447 |
| **EA:** | ber.: | C:49.57 | H:5.82 | N:11.56 |
| | gef.: | C:49.61 | H:5.78 | N:11.47 |

### Darstellung von (4-Nitrophenyl)methyl-4-(2-aminoacetyl)piperazin-1-carbodithioathydrochlorid (Schl32343):

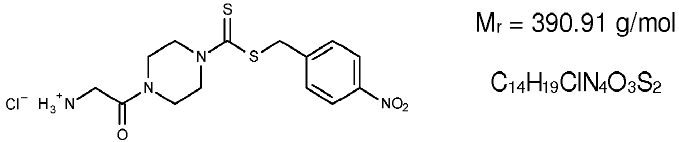

Gemäß **AAV11** wurden 350 mg (4-Nitrophenyl)methyl-4-[2-(*tert-*butoxycarbonylamino)-acetyl]piperazin-1-carbodithioat (**Schl32336**, 0.77 mmol, 1.0 eq) umgesetzt. Es wurden 286 mg von **Schl32343** (94.9%) in Form eines blassgelben Feststoffes erhalten.

| | | | | |
|---|---|---|---|---|
| **Fp.:** | 243 °C | | | |
| **¹H-NMR:** | (DMSO-D₆, 400 MHz), δ [ppm] = 8.28 (*sbr,* 3H, N*H₃*⁺), 8.14 (*d,* ³J = 8.5 Hz, 2H, C-CH-C*H*-C-NO₂, C-CH-C*H*-C-NO₂), 7.64 (*d,* ³J = 8.5 Hz, 2H, C-CH-C*H*-C-NO₂, C-CH-C*H*-C-NO₂), 4.72 (*s*, 2H, S-C*H₂*-C), 4.33-4.16 (*m,* 2H, N-CH*H*-CH₂-N-C(=O), N-CH*H-*CH₂-N-C(=O)), 4.09-3.90 (*m*, 2H, N-CH*H*-CH₂-N-C(=O), N-CH*H-*CH₂-N-C(=O)), 3.84 (*s,* 2H, C*H₂*-C(=O)), 3.62 (*t*, ³J = 5.2 Hz, 2H, N-CH₂-CH*H*-N-C(=O), N-CH₂-CH*H*-N-C(=O)), 3.56 (*t*, ³J = 4.9 Hz, 2H, N-CH₂-CH*H*-N-C(=O), N-CH₂-CH*H-*N-C(=O)). | | | |
| **¹³C**-**NMR:** | (DMSO-D₆, 100 MHz), δ [ppm] = 195.1 (C=S), 165.6 (C(=O)-NH), 147.2 (C(NO₂)), 145.7 (CH₂-C-CH-CH), 130.9 (C(NO₂)-CH-CH, C(NO₂)-CH-CH), 124.0 (C-CH-CH, C-CH-CH), 66.9 (CH₂-C(=O)), 43.3 (N-CH₂-CH₂-N-C(=O)), 41.3 (S-CH₂-C). | | | |
| **IR (ATR):** | v (cm⁻¹ ) = 3003 (w), 2886 (w), 2709 (w), 2628 (w), 1707 (w), 1651 (vs), 1600 (m), 1518 (m), 1460 (w), 1414 (w), 1365 (w), 1342 (s), 1282 (w), 1244 (w), 1216 (s), 1158 (vs), 1109 (w), 1049 (w), 994 (m), 961 (m), 924 (vs), 859 (m), 801 (w), 762 (w), 687 (w), 550 (w), 490 (w). | | | |
| **MS** (ESI+): | m/z (%) = 355 (100, [M-Cl]⁺) | | | |
| **HRMS** (ESI+): | m/z für C₁₄H₁₈N₄O₃S₂ | | | |
| | ber.: 354.0820 gef.: 354.0821 | | | |
| **EA:** | ber.: | C:43.02 | H:4.90 | N:14.33 |
| | gef.: | C:42.95 | H: 4.83 | N: 14.37 |

### Darstellung von (4-Nitrophenyl)methyl-4-(2-aminopropanoyl)piperazin-1-carbodithioathydrochlorid (Schl32344):

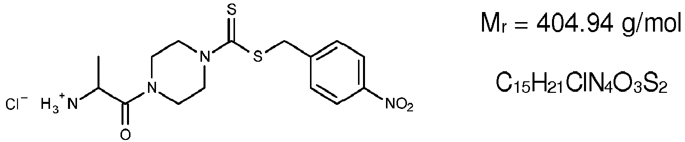

Gemäß **AAV11** wurden 468 mg (4-Nitrophenyl)methyl-4-[2-(*tert-*butoxycarbonylamino)-propanoyl]-piperazin-1-carbodithioat (**Schl32337**, 1.00 mmol, 1.0 eq) umgesetzt. Es wurden 360 mg von **Schl32344** (88.9%) in Form eines farblosen Feststoffes erhalten.

| | | | | |
|---|---|---|---|---|
| **Fp.:** | 265 °C | | | |
| **¹H-NMR:** | (DMSO-D₆, 400 MHz), δ [ppm] = 8.31 (*sbr,* 3H, N*H₃*⁺), 8.14 (*d*, ³J = 8.7 Hz, 2H, C-CH-C*H*-C-NO₂, C-CH-C*H*-C-NO₂), 7.64 (d, ³J = 8.9 Hz, 2H, C-CH-C*H*-C-NO₂, C-CH-C*H*-C-NO₂), 4.72 (*s*, 2H, S-C*H₂*-C), 4.32 (*q,* ³J = 6.9 Hz, 1H, C*H-*C(=O)), 4.19-4.00 (*m,* 2H, N-CH*H*-CH₂-N-C(=O), N-CH*H-*CH₂-N-C(=O)), 3.98-3.81 (*m*, 2H, N-CH*H*-CH₂-N-C(=O), N-CH*H-*CH₂-N-C(=O)), 3.75-3.66 (*m*, 2H, N-CH₂-CH*H*-N-C(=O), N-CH₂-CH*H*-N-C(=O)), 3.63-3.47 (*m,* 2H, N-CH₂-CH*H*-N-C(=O), N-CH₂-CH*H*-N-C(=O)), 1.31 *(d,* ³J = 6.9 Hz, 3H, CH-C*H₃*). | | | |
| **¹³C**-**NMR:** | (DMSO-D₆, 100 MHz), δ [ppm] = 195.2 (C=S), 169.0 (C(=O)-N), 147.2 (C(NO₂)), 145.7 (CH₂-C-CH-CH), 130.9 (C(NO₂)-CH-CH, C(NO₂)-CH-CH), 124.0 (C-CH-CH, C-CH-CH), 46.5 (CH-C(=O)), 44.0 (N-CH₂-CH₂-N-C(=O)), 41.7 (N-CH₂-CH₂-N-C(=O)), 40.5 (S-CH₂-C), 16.7 (CH-CH₃). | | | |
| **IR** (ATR): | v (cm⁻¹) = 3003 (w), 2886 (w), 2709 (w), 2628 (w), 1707 (w), 1651 (vs), 1600 (m), 1518 (m), 1460 (w), 1414 (w), 1365 (w), 1342 (s), 1282 (w), 1244 (w), 1216 (s), 1158 (vs), 1109 (w), 1049 (w), 994 (m), 961 (m), 924 (vs), 859 (m), 801 (w), 762 (w), 687 (w), 550 (w), 490 (w). | | | |
| **MS** (ESI+): | m/z (%) = 369 (100, [M-Cl]⁺) | | | |
| **HRMS** (ESI+): | m/z für C₁₅H₂₀N₄O₃S₂ | | | |
| | ber.: | 368.0977 | | gef.: 368.0986 |
| **EA:** | ber.: | C: 44.49 | H: 5.23 | N: 13.84 |
| | gef.: | C: 44.19 | H: 5.26 | N: 13.74 |

### Darstellung von (4-Nitrophenyl)methyl-4-(2,4-diamino-4-oxo-butanoyl)piperazin-1-carbodithioathydrochlorid (Schl32345):

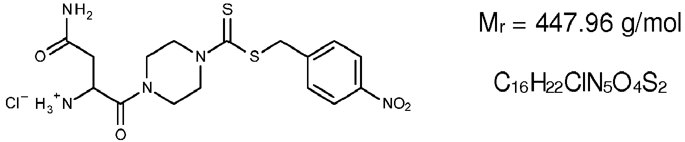

Gemäß **AAV11** wurden 349 mg (4-Nitrophenyl)methyl-4-[4-amino-2-(*tert*-butoxycarbonylamino)-4-oxo-butanoyl]piperazin-1-carbodithioat (**Schl32338**, 0.68 mmol, 1.0 eq) umgesetzt. Es wurden 251 mg von **Schl32345** (82.3%) in Form eines blassgelben Feststoffes erhalten.

| | | | | |
|---|---|---|---|---|
| **Fp.:** | 115 °C | | | |
| **¹H-NMR:** | (DMSO-D₆, 400 MHz), δ [ppm] = 8.82 *(sbr,* 3H, N*H₃*⁺), 8.14 (*d*, ³J = 8.7 Hz, 2H, C-CH-C*H*-C-NO₂, C-CH-C*H*-C-NO₂), 7.64 (d, ³J = 8.9 Hz, 2H, C-CH-C*H*-C-NO₂, C-CH-C*H*-C-NO₂), 4.72 (*s*, 2H, S-C*H₂*-C), 4.55-4.47 (*m,* 1H, C*H-*C(=O)), 4.40-4.29 (*m,* 2H, N-CH*H*-CH₂-N-C(=O), N-CH*H*-CH₂-N-C(=O)), 4.25-4.11 (*m,* 2H, N-CH*H*-CH₂-N-C(=O), N-CH*H-*CH₂-N-C(=O)), 3.90-3.79 (*m*, 2H, N-CH₂-CH*H*-N-C(=O), N-CH₂-CH*H*-N-C(=O)), 3.77-3.60 (*m*, 2H, N-CH₂-CH*H*-N-C(=O), N-CH₂-CH*H*-N-C(=O)), 3.58-3.49 (*m,* 2H, C(=O)-N*H*₂), 3.20-3.04 (*m,* 2H, CH-C*H₂-*C(=O)NH₂). | | | |
| **¹³C**-**NMR:** | (DMSO-D₆, 100 MHz), δ [ppm] = 195.3 (C=S), 166.0 (C(=O)-N), 147.2 (C(NO₂)), 145.7 (CH₂-C-CH-CH), 130.9 (C(NO₂)-CH-CH, C(NO₂)-CH-CH), 124.0 (C-CH-CH, C-CH-CH), 116.6 (C(=O)NH₂), 45.9 (CH-C(=O)), 44.4 (N-CH₂-CH₂-N-C(=O)), 42.1 (N-CH₂-CH₂-N-C(=O)), 40.4 (S-CH₂-C), 19.8 (CH-CH₂-C(=O)NH₂). | | | |
| **IR (ATR):** | v (cm⁻¹ ) = 2960 (w), 2842 (w), 1726 (w), 1652 (s), 1599 (s), 1515 (s), 1469 (vs), 1417 (w), 1344 (vs), 1282 (m), 1217 (s), 1161 (w), 1109 (m), 1015 (s), 996 (s), 950 (w), 871 (m), 860 (m), 820 (w), 802 (w), 757 (w), 714 (m), 688 (w), 623 (w), 522 (s), 492 (w). | | | |
| **MS** (ESI+): | m/z (%) = 412 (100, [M-Cl]⁺) | | | |
| **HRMS** (ESI+): | m/z für C₁₆H₂₁N₅O₄S₂ | | | |
| | ber.: | 411.1035 | | gef.: 411.1031 |
| **EA:** | ber.: | C:42.90 | H:4.95 | N: 15.63 |
| | gef.: | C:42.75 | H:5.05 | N: 15.66 |

### Darstellung von (4-Nitrophenyl)methyl-4-(2-amino-4-methylsulfanylbutanoyl)piperazin-1-carbodithioathydrochlorid (Sch32346):

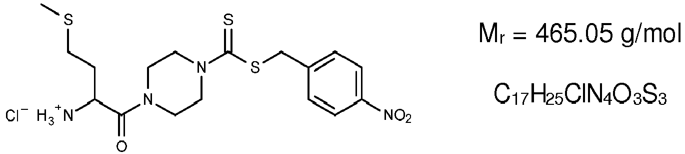

Gemäß **AAV11** wurden 354 mg (4-Nitrophenyl)methyl-4-[2-(*tert-*butoxycarbonylamino)-4-methylsulfanylbutanoyl]piperazin-1-carbodithioat (**Schl32339**, 0.67 mmol, 1.0 eq) umgesetzt. Es wurden 271 mg von **Schl32346** (86.9%) in Form eines gelben Feststoffes erhalten.

| | | | | |
|---|---|---|---|---|
| **Fp.:** | 137 °C | | | |
| **¹H-NMR:** | (DMSO-D₆, 400 MHz), δ [ppm] = 8.39 (*sbr,* 3H, N*H₃*⁺), 8.15 (*d,* ³J = 8.9 Hz, 2H, C-CH-C*H*-C-NO₂, C-CH-C*H*-C-NO₂), 7.64 (*d,* ³J = 8.7 Hz, 2H, C-CH-C*H*-C-NO₂, C-CH-C*H*-C-NO₂), 4.73 (*s*, 2H, S-C*H₂*-C), 4.40-4.33 (*m,* 1 H, C*H-*C(=O)), 4.17-4.00 (*m,* 2H, N-CH*H*-CH₂-N-C(=O), N-CH*H*-CH₂-N-C(=O)), 3.98-3.81 (*m,* 2H, N-CH*H*-CH₂-N-C(=O), N-CH*H-*CH₂-N-C(=O)), 3.77-3.53 (*m,* 4H, N-CH₂-C*H₂*-N-C(=O), N-CH₂-C*H₂*-N-C(=O)), 2.63-2.48 (*m,* 2H, S-C*H₂*-CH₂-CH), 2.03 (*s*, 1H, S-C*H₃*), 1.98-1.89 (*m,* 2H, S-CH₂-C*H₂*-CH). | | | |
| **¹³C**-**NMR:** | (DMSO-D₆, 100 MHz), δ [ppm] = 195.2 (C=S), 167.9 (C(=O)-N), 147.2 (C(NO₂)), 145.7 (CH₂-C-CH-CH), 130.9 (C(NO₂)-CH-CH, C(NO₂)-CH-CH), 124.0 (C-CH-CH, C-CH-CH), 49.3 (CH-C(=O)), 44.1 (N-CH₂-CH₂-N-C(=O)), 41.7 (N-CH₂-CH₂-N-C(=O)), 40.0 (S-CH₂-C), 30.7 (S-CH₂-CH₂-CH), 28.7 (S-CH₂-CH₂-CH), 14.9 (S-CH₃). | | | |
| **IR (ATR):** | v (cm⁻¹) = 2916 (w), 2853 (w), 1650 (m), 1598 (w), 1516 (s), 1469 (m), 1416 (s), 1343 (vs), 1279 (m), 1239 (s), 1158 (m), 1107 (w), 1011 (m), 993 (s), 957 (m), 859 (m), 748 (w), 722 (m), 632 (w), 532 (w), 487 (w). | | | |
| **MS** (ESI+): | m/z (%) = 429 (100, [M-Cl]⁺) | | | |
| **HRMS** (ESI+): | m/z für C₁₇H₂₄N₄O₃S₃ | | | |
| | ber.: | 428.1011 | | gef.: 428.1031 |
| **EA:** | ber.: | C:43.91 | H:5.42 | N:12.05 |
| | gef.: | C:44.01 | H:5.50 | N:12.09 |

### Darstellung von (4-Nitrophenyl)methyl-4-(pyrrolidin-2-carbonyl)piperazin-1-carbodithioathydrochlorid (Schl32347):

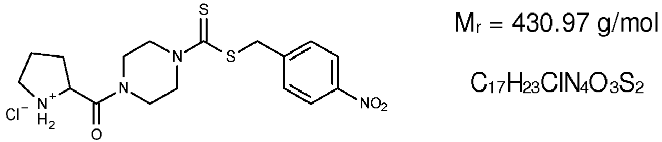

Gemäß **AAV11** wurden 574 mg tert-Butyl-2-[4-[(4-nitrophenyl)methylsulfanyl-carbothioyl]piperazin-1-carbonyl]pyrrolidin-1-carboxylat (**Schl32340,** 1.16 mmol, 1.0 eq) umgesetzt. Es wurden 341 mg von **Schl32347** (68.2%) in Form eines blassgelben Feststoffes erhalten.

| | | | | |
|---|---|---|---|---|
| **Fp.:** | 231 °C | | | |
| **¹H-NMR:** | (DMSO-D₆, 400 MHz), δ [ppm] = 10.30 (*sbr,* 1H, NH*H*⁺), 8.47 (*sbr,* 1H, NH*H*⁺), 8.14 (*d,* ³J = 8.9 Hz, 2H, C-CH-C*H*-C-NO₂, C-CH-C*H*-C-NO₂), 7.64 (*d*, ³J = 8.7 Hz, 2H, C-CH-C*H*-C-NO₂, C-CH-C*H*-C-NO₂), 4.72 (*s,* 2H, S-C*H₂*-C), 4.61-4.53 (*m,* 1 H, N-C*H*-C(=O)), 4.39-4.15 (*m,* 2H, N-CH*H*-CH₂-N-C(=O), N-CH*H-*CH₂-N-C(=O)), 4.10-3.90 (*m*, 2H, N-CH*H*-CH₂-N-C(=O), N-CH*H-*CH₂-N-C(=O)), 3.75-3.68 (*m,* 2H, N-CH₂-CH*H*-N-C(=O), N-CH₂-CH*H*-N-C(=O)), 3.65-3.51 (*m,* 2H, N-CH₂-CH*H*-N-C(=O), N-CH₂-CH*H*-N-C(=O)), 3.27-3.09 (*m,* 2H, N-C*H₂*-CH₂-CH₂-CH), 2.36-2.29 (*m,* 1 H, N-CH₂-CH₂-CH*H*-CH), 1.95-1.74 (*m*, 3H, N-CH₂-CH₂-CH*H*-CH, N-CH₂-C*H₂*-CH₂-CH). | | | |
| **¹³C**-**NMR:** | (DMSO-D₆, 100 MHz), δ [ppm] = 195.2 (C=S), 167.6 (C(=O)-N), 147.2 (C(NO₂)), 145.7 (CH₂-C-CH-CH), 130.9 (C(NO₂)-CH-CH, C(NO₂)-CH-CH), 124.0 (C-CH-CH, C-CH-CH), 57.9 (N-CH-C(=O)), 46.2 (N-CH₂-CH₂-N-C(=O)), 44.0 (N-CH₂-CH₂-CH₂-CH), 41.9 (N-CH₂-CH₂-N-C(=O)), 40.6 (S-CH₂-C), 29.9 (N-CH₂-CH₂-CH₂-CH), 24.2 (N-CH₂-CH₂-CH₂-CH). | | | |
| **IR (ATR):** | v (cm⁻¹) = 2720 (w), 2454 (w), 1646 (s), 1609 (w), 1537 (m), 1459 (m), 1423 (m), 1398 (w), 1346 (vs), 1314 (w), 1280 (m), 1215 (s), 1161 (m), 1107 (w), 1017 (m), 993 (s), 936 (w), 863 (m), 800 (w), 705 (m), 625 (w), 550 (w), 508 (w), 492 (m). | | | |
| **MS** (ESI+): | m/z (%) = 395 (100, [M-Cl]⁺) | | | |
| **HRMS** (ESI+): | m/z für C₁₇H₂₂N₄O₃S₂ | | | |
| | ber.: | 394.1133 | | gef.: 394.1158 |
| **EA:** | ber.: | C: 47.38 | H: 5.38 | N: 13.00 |
| | gef.: | C: 47.31 | H: 5.33 | N: 13.07 |

### Darstellung von (4-Nitrophenyl)methyl-4-[2-amino-3-(4-hydroxyphenyl)-propanoyl]piperazin-1-carbodithioathydrochlorid (Schl32348):

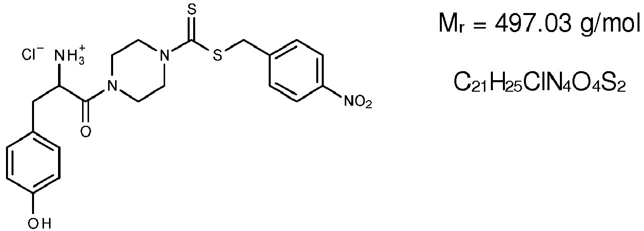

Gemäß **AAV11** wurden 537 mg (4-Nitrophenyl)methyl-4-[2-(*tert-*butoxycarbonylamino)-3-(4-hydroxyphenyl)propanoyl]piperazin-1-carbodithioat **(Schl32341,** 0.96 mmol, 1.0 eq) umgesetzt. Es wurden 388 mg von **Schl32348** (81.3%) in Form eines gelben Feststoffes erhalten.

| | | | | |
|---|---|---|---|---|
| **Fp.:** | 146 °C | | | |
| **¹H-NMR:** | (DMSO-D₆, 400 MHz), δ [ppm] = 9.44 *(sbr,* 1H, O*H*), 8.39 (*sbr,* 3H, N*H₃*⁺), 8.14 (*d*, ³J = 8.7 Hz, 2H, C-CH-C*H*-C-NO₂, C-CH-C*H*-C-NO₂), 7.63 (*d*, ³J = 8.7 Hz, 2H, C-CH-C*H*-C-NO₂, C-CH-C*H*-C-NO₂), 6.99 (*d,* ³J = 8.2 Hz, 2H, OH-C-CH-CH-C, HO-C-CH-C*H*-C), 6.69 (*d,* ³J = 8.5 Hz, 2H, HO-C-C*H*-CH-C, HO-C-CH-C*H*-C), 4.69 (s, 2H, S-C*H₂*-C), 4.49-4.43 (*m,* 1H, C*H-*C(=O)), 3.60-3.40 (*m,* 8H, N-C*H₂*-C*H₂*-N-C(=O), N-C*H₂*-C*H₂*-N-C(=O)), 3.10-2.94 (*m,* 2H, CH-C*H₂*-C). | | | |
| **¹³C**-**NMR:** | (DMSO-D₆, 100 MHz), δ [ppm] = 195.0 (C=S), 167.9 (C(=O)-N), 157.3 (C-OH), 147.1 (C(NO₂)), 145.6 (S-CH₂-C-CH-CH), 131.2 (CH-CH-C-OH, CH-CH-C-OH), 130.9 (C(NO₂)-CH-CH, C(NO₂)-CH-CH), 124.8 (CH-CH₂-C), 124.0 (C-CH-CH, C-CH-CH), 115.9 (CH-C-OH, CH-C-OH), 50.7 (CH-C(=O)), 44.2 (N-CH₂-CH₂-N-C(=O)), 41.6 (N-CH₂-CH₂-N-C(=O)), 40.6 (S-CH₂-C), 36.6 (CH-CH₂-C). | | | |
| **IR (ATR):** | v (cm⁻¹) = 2916 (w), 2853 (w), 1650 (m), 1598 (w), 1516 (s), 1469 (m), 1416 (s), 1343 (vs), 1279 (m), 1239 (s), 1216 (w), 1158 (m), 1107 (w), 1011 (m), 993 (s), 957 (m), 859 (m), 802 (w), 748 (w), 722 (m), 632 (w), 532 (w), 487 (w). | | | |
| **MS** (ESI+): | m/z (%) = 461 (100, [M-Cl]⁺) | | | |
| **HRMS** (ESI+): | m/z für C₂₁H₂₄N₄O₄S₂ | | | |
| | ber.: | 460.1239 | | gef.: 460.1268 |
| **EA:** | ber.: | C: 50.75 | H: 5.07 | N: 11.27 |
| | gef.: | C: 50.62 | H: 5.04 | N: 11.19 |

### Darstellung von (4-Nitrophenyl)methyl-4-(3-aminopropanoyl)piperazin-1-carbodithioathydrochlorid (Schl32349):

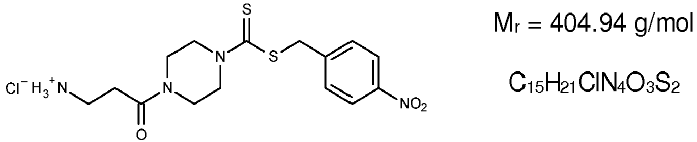

Gemäß **AAV11** wurden 445 mg (4-Nitrophenyl)methyl-4-[3-(*tert*-butoxycarbonylamino)-propanoyl]-piperazin-1-carbodithioat **(Schl32342,** 0.95 mmol, 1.0 eq) umgesetzt. Es wurden 375 mg von **Schl32349** (97.4%) in Form eines blassgelben Feststoffes erhalten.

| | | | | |
|---|---|---|---|---|
| **Fp.:** | 187 °C | | | |
| **¹H-NMR:** | (DMSO-D₆, 400 MHz), δ [ppm] = 8.14 (*d*, ³J = 8.7 Hz, 2H, C-CH-C*H*-C-NO₂, C-CH-C*H*-C-NO₂), 7.64 (*d*, ³J = 8.9 Hz, 2H, C-CH-C*H*-C-NO₂, C-CH-C*H*-C-NO₂), 6.44 (*sbr,* 3H, N*H₃*⁺), 4.72 (*s,* 2H, S-C*H₂*-C), 4.31-4.16 (*m,* 2H, N-CH*H*-CH₂-N-C(=O), N-CH*H-*CH₂-N-C(=O)), 4.07-3.87 (m, 2H, N-CH*H*-CH₂-N-C(=O), N-CH*H*-CH₂-N-C(=O)), 3.64-3.52 (*m,* 3H, N-CH₂-C*H₂*-N-C(=O), N-CH₂-C*H₂*-N-C(=O)), 3.00-2.89 (m*,* 2H, C*H₂*-CH₂-C(=O)), 2.72 (*t*, ³J = 6.5 Hz, 2H, CH₂-C*H₂*-C(=O)). | | | |
| **¹³C**-**NMR:** | (DMSO-D₆, 100 MHz), δ [ppm] = 194.9 (C=S), 169.2 (C(=O)-N), 147.2 (C(NO₂)), 145.7 (CH₂-C-CH-CH), 130.9 (C(NO₂)-CH-CH, C(NO₂)-CH-CH), 124.0 (C-CH-CH, C-CH-CH), 44.0 (N-CH₂-CH₂-N-C(=O)), 40.4 (N-CH₂-CH₂-N-C(=O)), 39.9 (S-CH₂-C), 35.6 (CH₂-CH₂-C(=O)), 30.4 (CH₂-CH₂-C(=O)). | | | |
| **IR (ATR):** | v (cm⁻¹) = 2906 (w), 2824 (w), 2733 (w), 1739 (m), 1607 (w), 1597 (s), 1525 (w), 1471 (w), 1439 (vs), 1411 (w), 1389 (m), 1359 (vs), 1311 (s), 1265 (w), 1251 (s), 1215 (w), 1156 (m), 1095 (w), 1017 (m), 997 (s), 956 (m), 860 (w), 816 (w), 747 (m), 705 (w), 666 (w), 561 (w), 496 (w). | | | |
| **MS** (ESI+): | m/z (%) = 369 (100, [M-Cl]⁺) | | | |
| **HRMS** (ESI+): | m/z für C₁₅H₂₀N₄O₃S₂ | | | |
| | ber.: | 368.0977 | | gef.: 368.1005 |
| **EA:** | ber.: | C: 44.49 | H: 5.23 | N:13.84 |
| | gef.: | C:44.19 | H: 5.26 | N:13.74 |

### Darstellung von (4-Nitrophenyl)methyl-4-(2-amino-3-hydroxypropanoyl)piperazin-1-carbodithioathydrochlorid (Schl32355):

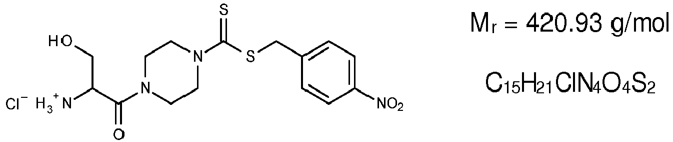

Gemäß **AAV11** wurden 408 mg (4-Nitrophenyl)methyl-4-[2-(*tert-*butoxycarbonylamino)-3-hydroxypropanoyl]piperazin-1-carbodithioat **(Schl32354,** 0.84 mmol, 1.0 eq) umgesetzt. Es wurden 303 mg von **Schl32355** (85.6%) in Form eines farblosen Feststoffes erhalten.

| | | | | |
|---|---|---|---|---|
| **Fp.:** | 148 °C | | | |
| **¹H-NMR:** | (DMSO-D₆, 400 MHz), δ [ppm] = 8.26 (*sbr,* 3H, N*H₃*⁺), 8.14 (*d*, ³J = 8.7 Hz, 2H, C-CH-C*H*-C-NO₂, C-CH-C*H*-C-NO₂), 7.64 (*d*, ³J = 8.4 Hz, 2H, C-CH-C*H*-C-NO₂, C-CH-C*H*-C-NO₂), 5.58 (*sbr,* 1H, O*H*), 4.72 (*s,* 2H, S-C*H₂*-C), 4.40-4.36 (*m,* 1H, C*H-*C(=O)), 4.31-4.17 (*m,* 2H, N-CH*H*-CH₂-N-C(=O), N-CH*H-*CH₂-N-C(=O)), 4.05-3.88 (*m*, 2H, N-CH*H*-CH₂-N-C(=O), N-CH*H-*CH₂-N-C(=O)), 3.80-3.71 (*m,* 2H, CH-C*H₂*-OH), 3.68-3.51 (*m,* 4H, N-CH₂-C*H₂*-N-C(=O), N-CH₂-C*H₂*-N-C(=O)). | | | |
| **¹³C**-**NMR:** | (DMSO-D₆, 100 MHz), δ [ppm] = 195.1 (C=S), 166.6 (C(=O)-N), 147.2 (C(NO₂)), 145.7 (CH₂-C-CH-CH), 130.9 (C(NO₂)-CH-CH, C(NO₂)-CH-CH), 124.0 (C-CH-CH, C-CH-CH), 60.3 (CH-CH₂-OH), 52.7 (CH-C(=O)), 41.9 (N-CH₂-CH₂-N-C(=O)), 40.6 (S-CH₂-C). | | | |
| **IR (ATR):** | v (cm⁻¹) = 3002 (w), 2912 (w), 1651 (vs), 1599 (w), 1526 (s), 1498 (m), 1469 (m), 1421 (s), 1385 (vs), 1313 (w), 1285 (m), 1216 (w), 1160 (w), 1063 (s), 1016 (w), 958 (w), 869 (m), 815 (w), 728 (s), 687 (w), 624 (w), 551 (m), 521 (w), 496 (w), 435 (w). | | | |
| **MS** (ESI+): | m/z (%) = 385 (100, [M-Cl]⁺) | | | |
| **HRMS** (ESI+): | m/z für C₁₅H₂₀N₄O₄S₂ | | | |
| | ber.: | 384.0926 | | gef.: 384.0954 |
| **EA:** | ber.: | C:42.80 | H:5.03 | N:13.31 |
| | gef.: | C:42.96 | H:5.15 | N:13.23 |

### Darstellung von (4-Nitrophenyl)methyl-4-(2-hydroxyethyl)piperazin-1-carbodithioat (Schl32327):

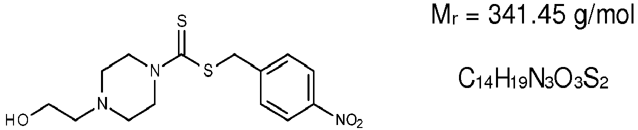

Gemäß **AAV8** wurden 0.25 mL 2-Piperazin-1-ylethanol (2.0 mmol, 1.0 eq), 0.84 mL Triethylamin (6.0 mmol, 3.0 eq), 0.24 mL Kohlenstoffdisulfid (6.0 mmol, 3.0 eq) und 432 mg 1-(Brommethyl)-4-nitrobenzen (2.0 mmol, 1.0 eq) umgesetzt. Säulenchromatographie an Kieselgel (DCM/ MeOH 90:10) ergab 660 mg von **Schl32327** (95.5%) in Form eines blassgelben Feststoffes.

| | | | | |
|---|---|---|---|---|
| **DC:** | R_{f} = 0.51 (DCM/ MeOH 90:10). | | | |
| **Fp.:** | 169 °C | | | |
| **¹H-NMR:** | (DMSO-D₆, 400 MHz), δ [ppm] = 8.14 (*d*, ³J = 8.7 Hz, 2H, C-CH-C*H*-C-NO₂, C-CH-C*H*-C-NO₂), 7.63 (*d*, ³J = 8.9 Hz, 2H, C-CH-C*H*-C-NO₂, C-CH-C*H*-C-NO₂), 4.69 (*s,* 2H, S-C*H₂*-C), 4.45 (*sbr,* 1H, O*H*), 4.24-4.11 (*m,* 2H, N-CH*H*-CH₂-N-CH₂, N-CH*H*-CH₂-N-CH₂), 3.93-3.81 (*m,* 2H, N-CH*H*-CH₂-N-CH₂, N-CH*H*-CH₂-N-CH₂), 3.48 (*t*, ³J = 6.0 Hz, 2H, HO-C*H₂*), 3.39-3.23 (*m,* 4H, N-CH₂-C*H₂*-N-CH₂, N-CH₂-C*H₂*-N-CH₂), 2.41 (*t*, ³J = 6.0 Hz, 2H, HO-CH₂-CH₂). | | | |
| **¹³C**-**NMR:** | (DMSO-D₆, 100 MHz), δ [ppm] = 194.2 (C=S), 147.1 (C(NO₂)), 145.9 (CH₂-C-CH-CH), 130.8 (C(NO₂)-CH-CH, C(NO₂)-CH-CH), 124.0 (C-CH-CH, C-CH-CH), 59.9 (N-CH₂-CH₂-OH), 58.9 (N-CH₂-CH₂-OH), 53.1 (N-CH₂-CH₂-N-CH₂), 50.3 (N-CH₂-CH₂-N-CH₂), 46.1 (S-CH₂-C). | | | |
| **IR (ATR):** | v (cm⁻¹) = 2978 (w), 2882 (w), 2821 (w), 2740 (w), 2676 (w), 2492 (w), 1695 (w), 1599 (w), 1516 (s), 1473 (w), 1434 (w), 1416 (m), 1394 (m), 1346 (s), 1280 (w), 1228 (w), 1191 (s), 1150 (w), 1108 (w), 1051 (w), 1036 (m), 1012 (w), 995 (s), 933 (w), 861 (w), 803 (m), 780 (w), 759 (m), 710 (w), 687 (m), 623 (w), 528 (w), 469 (w). | | | |
| **MS** (ESI+): | m/z (%) = 342 (100, [M+H]⁺) | | | |
| **HRMS** (ESI+): | m/z für C₁₄H₁₉N₃O₃S₂ | | | |
| | ber.: | 341.0868 | | gef.: 341.0876 |
| **EA:** | ber.: | C: 49.25 | H: 5.61 | N: 12.31 |
| | gef.: | C: 49.11 | H: 5.69 | N: 12.37 |

### Darstellung von (4-Nitrophenyl)methyl-4-(2-dimethylaminoethyl)piperazin-1-carbodithioat (Schl32328):

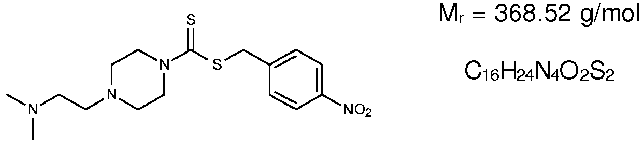

Gemäß **AAV8** wurden 0.21 mL *N,N-*Dimethyl-2-piperazin-1-yl-ethanamin (2.0 mmol, 1.0 eq), 0.84 mL Triethylamin (6.0 mmol, 3.0 eq), 0.24 mL Kohlenstoffdisulfid (6.0 mmol, 3.0 eq) und 432 mg 1-(Brommethyl)-4-nitrobenzen (2.0 mmol, 1.0 eq) umgesetzt. Säulenchromatographie an Kieselgel (DCM/ MeOH 90:10) ergab 642 mg von **Schl32328** (87.1 %) in Form eines blassgelben Feststoffes.

| | | | | |
|---|---|---|---|---|
| **DC:** | R_{f} = 0.55 (DCM/ MeOH 90:10). | | | |
| **Fp.:** | 175 °C | | | |
| **¹H-NMR:** | (DMSO-D₆, 400 MHz), δ [ppm] = 8.14 (*d*, ³J = 8.7 Hz, 2H, C-CH-C*H*-C-NO₂, C-CH-C*H*-C-NO₂), 7.63 (*d*, ³J = 8.7 Hz, 2H, C-CH-C*H*-C-NO₂, C-CH-C*H*-C-NO₂), 4.70 (*s,* 2H, S-C*H₂*-C), 4.30-4.16 (*m,* 2H, N-CH*H*-CH₂-N-CH₂, N-CH*H*-CH₂-N-CH₂), 4.01-3.83 (*m,* 2H, N-CH*H*-CH₂-N-CH₂, N-CH*H*-CH₂-N-CH₂), 3.20 (*t*, ³J = 6.0 Hz, 2H, N-C*H₂*-CH₂-N(CH₃)₂), 2.76 (*s,* 6H, N(C*H₃*)₂), 2.64 (*t,* ³J = 6.0 Hz, 2H, N-CH₂-C*H₂*-N(CH₃)₂), 2.53 (*t*, ³J = 7.2 Hz, 4H, N-CH₂-C*H₂*-N-CH₂, N-CH₂-C*H₂*-N-CH₂). | | | |
| **¹³C**-**NMR:** | (DMSO-D₆, 100 MHz), δ [ppm] = 194.3 (C=S), 147.1 (C(NO₂)), 145.8 (CH₂-C-CH-CH), 130.9 (C(NO₂)-CH-CH, C(NO₂)-CH-CH), 124.0 (C-CH-CH, C-CH-CH), 53.3 (N-CH₂-CH₂-N(CH₃)₂), 52.3 (N-CH₂-CH₂-N-CH₂, N-CH₂-CH₂-N-CH₂), 51.3 (N-CH₂-CH₂-N(CH₃)₂), 46.1 (S-CH₂-C), 43.1 (N-CH₃). | | | |
| **IR (ATR):** | v (cm⁻¹ ) = 3008 (w), 2977 (w), 2949 (w), 2902 (w), 1514 (s), 1473 (s), 1429 (s), 1401 (w), 1340 (w), 1289 (s), 1267 (w), 1231 (m), 1217 (m), 1174 (s), 1160 (w), 1146 (m), 1107 (w), 1064 (w), 1032 (m), 1002 (m), 991 (s), 934 (w), 916 (s), 858 (w), 823 (m), 784 (w), 757 (m), 689 (w), 622 (w), 562 (w), 526 (m). | | | |
| **MS** (ESI+): | m/z (%) = 369 (100, [M+H]⁺) | | | |
| **HRMS** (ESI+): | m/z für C₁₆H₂₄N₄O₂S₂ | | | |
| | ber.: | 368.1341 | | gef.: 368.1334 |
| **EA:** | ber.: | C:52.15 | H: 6.56 | N: 15.20 |
| | gef.: | C:52.40 | H: 6.67 | N: 15.23 |

### Darstellung von (4-Nitrophenyl)methyl-4-methylsulfonylpiperazin-1-carbodithioat (Schl32329):

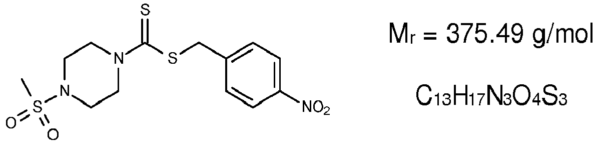

Gemäß **AAV8** wurden 328 mg 1-Methylsulfonylpiperazin (2.0 mmol, 1.0 eq), 0.84 mL Triethylamin (6.0 mmol, 3.0 eq), 0.24 mL Kohlenstoffdisulfid (6.0 mmol, 3.0 eq) und 432 mg 1-(Brommethyl)-4-nitrobenzen (2.0 mmol, 1.0 eq) umgesetzt. Säulenchromatographie an Kieselgel (Cyclohexan/ EtOAc 1:1) ergab 672 mg von **Schl32329** (89.5%) in Form eines gelben Feststoffes.

| | | | | |
|---|---|---|---|---|
| **DC**: | R_{f} = 0.31 (Cyclohexan/ EtOAc 1:1). | | | |
| **Fp.:** | 182 °C | | | |
| **¹H-NMR:** | (CDCl₃, 400 MHz), δ [ppm] = 8.14 (*d*, ³J = 8.9 Hz, 2H, C-CH-C*H*-C-NO₂, C-CH-C*H*-C-NO₂), 7.64 (*d*, ³J = 8.7 Hz, 2H, C-CH-C*H*-C-NO₂, C-CH-C*H*-C-NO₂), 4.71 (*s,* 2H, S-C*H₂*-C), 4.37-4.22 (*m*, 2H, N-CH*H*-CH₂-N-SO₂Me, N-CH*H*-CH₂-N-SO₂Me), 4.13-3.94 (*m*, 2H, N-CH*H*-CH₂-N-SO₂Me, N-CH*H*-CH₂-N-SO₂Me), 2.52 (*t*, ³J = 5.1 Hz, 4H, N-CH₂-C*H₂*-N-SO₂Me, N-CH₂-C*H₂*-N-SO₂Me), 2.89 (*s,* 3H, C*H₃*). | | | |
| **¹³C**-**NMR:** | (CDCl₃, 100 MHz), δ [ppm] = 195.3 (C=S), 147.1 (C(NO₂)), 145.6 (CH₂-C-CH-CH), 130.9 (C(NO₂)-CH-CH, C(NO₂)-CH-CH), 124.0 (C-CH-CH, C-CH-CH), 51.1 (N-CH₂-CH₂-N-SO₂Me), 49.8 (N-CH₂-CH₂-N-SO₂Me), 45.4 (S-CH₂-C), 34.9 (CH₃). | | | |
| **IR (ATR):** | v (cm⁻¹) = 3107 (w), 3080 (w), 3010 (w), 1596 (w), 1520 (s), 1490 (w), 1435 (m), 1420 (m), 1369 (w), 1338 (s), 1327 (s), 1320 (s), 1275 (vs), 1226 (w), 1206 (s), 1191 (w), 1176 (vs), 1154 (s), 1140 (m), 1088 (w), 1034 (m), 1003 (m), 968 (m), 927 (s), 894 (w), 858 (s), 801 (w), 777 (vs), 724 (s), 639 (w), 550 (m), 537 (w), 515 (vs), 495 (m), 475 (w), 456 (vs). | | | |
| **MS** (ESI+): | m/z (%) = 393 (100, [M+NH₄]⁺) | | | |
| **HRMS** (ESI+): | m/z für C₁₃H₁₇N₃O₄S₃ | | | |
| | ber.: | 375.0381 | | gef.: 375.0358 |
| **EA:** | ber.: | C:41.58 | H:4.56 | N:11.19 |
| | gef.: | C:41.77 | H:4.70 | N:11.06 |

### Darstellung von (4-Nitrophenyl)methyl-4-(benzensulfonyl)piperazin-1-carbodithioat (Schl32330):

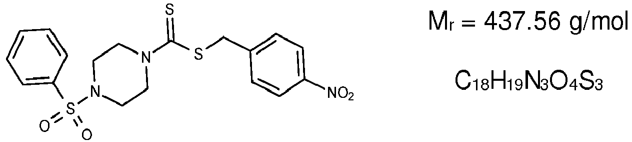

Gemäß **AAV12** wurden 421 mg (4-Nitrophenyl)methylpiperazin-1-carbodithioathydrochlorid (**Schl32319**, 1.25 mmol, 1.0 eq) und 0.24 mL Benzensulfonylchlorid (1.88 mmol, 1.5 eq) umgesetzt. Säulenchromatographie an Kieselgel (Cyclohexan/ EtOAc 3:1) ergab 421 mg von **Schl32330** (77.0%) in Form eines blassgelben Feststoffes.

| | | | | |
|---|---|---|---|---|
| **DC:** | R_{f} = 0.18 (Cyclohexan/ EtOAc 3:1). | | | |
| **Fp.:** | 184 °C | | | |
| **¹H-NMR:** | (CDCl₃, 400 MHz), δ [ppm] = 8.11 (*d*, ³J = 8.7 Hz, 2H, C-CH-C*H*-C-NO₂, C-CH-C*H*-C-NO₂), 7.74 (*d*, ³J = 8.7 Hz, 2H, C*H*-C-SO₂, C*H*-C-SO₂), 7.64 (*t,* ³J = 7.5 Hz, 1 H, C*H*-CH-CH-C-SO₂), 7.55 (*t*, ³J = 7.6 Hz, 2H, C*H*-CH-C-SO₂, C*H*-CH-C-SO₂), 7.49 (*d*, ³J = 8.7 Hz, 2H, C-CH-C*H*-C-NO₂, C-CH-C*H*-C-NO₂), 4.60 (*s,* 2H, S-C*H₂*-C), 4.47-4.25 (*m,* 2H, N-CH*H*-CH₂-N-SO₂Ph, N-CH*H*-CH₂-N-SO₂Ph), 4.20-3.92 (*m,* 2H, N-CH*H*-CH₂-N-SO₂Ph, N-CH*H*-CH₂-N-SO₂Ph), 3.11 (*t*, ³J = 4.9 Hz, 4H, N-CH₂-C*H₂*-N-SO₂Ph, N-CH₂-C*H₂*-N-SO₂Ph). | | | |
| **¹³C**-**NMR:** | (CDCl₃, 100 MHz), δ [ppm] = 196.2 (C=S), 147.1 (C(NO₂)), 144.1 (CH₂-C-CH-CH), 135.2 (C-SO₂), 133.5 (CH-C-SO₂), 130.2 (C(NO₂)-CH-CH, C(NO₂)-CH-CH), 129.5 (CH-CH-C-SO₂, CH-CH-C-SO₂), 127.8 (CH-CH-CH-C-SO₂), 123.8 (C-CH-CH, C-CH-CH), 45.6 (S-CH₂-C), 40.7 (N-CH₂-CH₂-N-SO₂Ph, N-CH₂-CH₂-N-SO₂Ph). | | | |
| **IR (ATR):** | v (cm⁻¹) = 1599 (w), 1510 (m), 1467 (w), 1438 (m), 1398 (w), 1385 (vs), 1366 (w), 1314 (s), 1277 (w), 1237 (m), 1173 (w), 1138 (s), 1118 (w), 1108 (m), 1073 (w), 1053 (m), 999 (w), 942 (s), 932 (s), 856 (w), 800 (w), 762 (s), 715 (w), 690 (s), 624 (w), 571 (vs), 498 (w), 479 (m). | | | |
| **MS** (ESI+): | m/z (%) = 455 (100, [M+NH₄]⁺) | | | |
| **HRMS** (ESI+): | m/z für C₁₈H₁₉N₃O₄S₃ | | | |
| | ber.: | 437.0538 | | gef.: 437.0516 |
| **EA:** | ber.: | C: 49.41 | H:4.38 | N: 9.60 |
| | gef.: | C: 49.50 | H: 4.43 | N: 9.57 |

### Darstellung von (4-Nitrophenyl)methyl-4-[[5-(dimethylamino)-1-naphthyl]sulfonyl]-piperazin-1-carbodithioat (Schl32331):

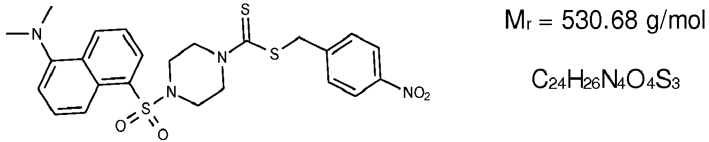

Gemäß **AAV12** wurden 421 mg (4-Nitrophenyl)methylpiperazin-1-carbodithioathydrochlorid (**Schl32319**, 1.25 mmol, 1.0 eq) und 507 mg Dansylchlorid (1.88 mmol, 1.5 eq) umgesetzt. Säulenchromatographie an Kieselgel (Cyclohexan/ EtOAc 2:1) ergab 534 mg von **Schl32331** (80.5%) in Form eines gelben Feststoffes.

| | | | | |
|---|---|---|---|---|
| **DC:** | R_{f} = 0.39 (Cyclohexan/ EtOAc 2:1). | | | |
| **Fp**.**:** | 205 °C | | | |
| **¹H-NMR:** | (CDCl₃, 400 MHz), δ [ppm] = 8.61 (*d*, ³J = 8.0 Hz, 1H, C*H*-CH-CH-C-SO₂), 8.37 (*d*, ³J = 8.5 Hz, 1H, C*H*-C-C-SO₂), 8.20 (*d*, ³J = 6.4 Hz, 1H, C*H*-CH-CH-C-SO₂), 8.11 (*d*, ³J = 8.7 Hz, 2H, C-CH-C*H*-C-NO₂, C-CH-C*H*-C-NO₂), 7.55 (*t*, ³J = 8.3 Hz, 2H, CH-C*H*-CH-C-SO₂, C*H*-CH-C-C-SO₂), 7.48 (*d*, ³J = 8.7 Hz, 2H, C-CH-C*H*-C-NO₂, C-CH-C*H*-C-NO₂), 7.20 (*d*, ³J = 7.3 Hz, 1H, CH-C*H*-CH-C-C-SO₂), 4.60 (*s*, 2H, S-C*H₂*-C), 4.41-4.22 (*m*, 2H, N-CH*H*-CH₂-N-SO₂, N-CH*H*-CH₂-N-SO₂), 4.16-3.94 (*m*, 2H, N-CH*H*-CH₂-N-SO₂, N-CH*H*-CH₂-N-SO₂), 3.29 (*t*, ³J = 4.9 Hz, 4H, N-CH₂-C*H₂*-N-SO₂Me, N-CH₂-C*H₂*-N-SO₂Me), 2.90 (*s*, 6H, C*H₃*)*.* | | | |
| **¹³C**-**NMR:** | (CDCl₃, 100 MHz), δ [ppm] = 196.1 (C=S), 147.3 (C(NO₂)) 144.2 (CH₂-C-CH-CH), 132.1 (Ct _{(Naphthyl)}), 131.3 (Ct _{(Naphthyl)}), 131.0 (Ct _{(Naphthyl)}), 130.4 (Cq _{(Naphthyl)}), 130.2 (C(NO₂)-CH-CH, C(NO₂)-CH-CH), 128.5 (Ct _{(Naphthyl)}), 123.8 (C-CH-CH, C-CH-CH), 123.4 (Ct _{(Naphthyl)}), 115.6 (Ct _{(Naphthyl)}), 45.6 (CH₃), 45.2 (N-CH₂-CH₂-N-SO₂), 40.6 (S-CH₂-C). | | | |
| **IR (ATR):** | v (cm⁻¹) = 1591 (m), 1573 (m), 1514 (m), 1462 (m), 1425 (w), 1399 (w), 1362 (s), 1339 (s), 1328 (s), 1307 (w), 1274 (m), 1253 (m), 1211 (s), 1160 (w), 1114 (vs), 1074 (m), 1026 (w), 993 (m), 930 (s), 858 (w), 822 (w), 799 (m), 785 (vs), 708 (vs), 644 (vs), 618 (w), 582 (m), 561 (m), 546 (vs), 499 (w), 460 (s), 451 (s). | | | |
| **MS** (ESI+): | m/z (%) = 531 (100, [M+H]⁺) | | | |
| **HRMS** (ESI+): | m/z für C₂₄H₂₆N₄O₄S₃ | | | |
| | ber.: | 530.1116 | | gef.: 530.1113 |
| **EA:** | ber.: | C: 54.32 | H:4.94 | N: 10.56 |
| | gef.: | C: 54.30 | H: 5.05 | N: 10.36 |

### Darstellung von (4-Nitrophenyl)methyl-4-(1-naphthylsulfonyl)piperazin-1-carbodithioat (Schl32332):

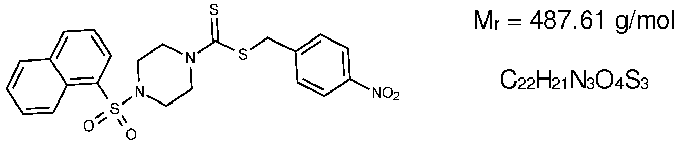

Gemäß **AAV12** wurden 421 mg (4-Nitrophenyl)methylpiperazin-1-carbodithioat-hydrochlorid (**Schl32319**, 1.25 mmol, 1.0 eq) und 426 mg Naphthalen-1-sulfonylchlorid (1.88 mmol, 1.5 eq) umgesetzt. Säulenchromatographie an Kieselgel (Cyclohexan/ EtOAc 2:1) ergab 469 mg von **Schl32332** (77.0%) in Form eines blassgelben Feststoffes.

| | | | | |
|---|---|---|---|---|
| **DC:** | R_{f} = 0.31 (Cyclohexan/ EtOAc 2:1). | | | |
| **Fp**.**:** | 160 °C | | | |
| **¹H-NMR:** | (CDCl₃, 400 MHz), δ [ppm] = 8.71 (*d*, ³J = 8.5 Hz, 1H, C*H*-C-C-SO₂), 8.20 (*d*, ³J = 8.5 Hz, 1H, C*H*-CH-CH-C-SO₂), 8.11 (*d*, ³J = 9.2 Hz, 1H, C*H*-CH-CH-C-SO₂), 8.09 (*d*, ³J = 8.7 Hz, 2H, C-CH-C*H*-C-NO₂, C-CH-C*H*-C-NO₂), 7.95 (*d*, ³J = 7.6 Hz, 1H, C*H*-CH-CH-CH-C-C-SO₂), 7.69-7.53 (*m*, 3H, CH-C*H*-C*H*-CH-C-C-SO₂, C*H*-CH-CH-C-SO₂), 7.47 (*d*, ³J = 8.7 Hz, 2H, C-CH-C*H*-C-NO₂, C-CH-C*H*-C-NO₂), 4.59 (*s*, 2H, S-C*H₂*-C), 4.48-4.24 *(m,* 2H, N-CH*H*-CH₂-N-SO₂, N-CH*H*-CH₂-N-SO₂), 4.16-3.90 (*m*, 2H, N-CH*H*-CH₂-N-SO₂, N-CH*H*-CH₂-N-SO₂), 3.28 (*t*, ³J = 5.0 Hz, 4H, N-CH₂-C*H₂*-N-SO₂Me, N-CH₂-C*H₂*-N-SO₂Me). | | | |
| **¹³C**-**NMR:** | (CDCl₃, 100 MHz), δ [ppm] = 196.1 (C=S), 147.3 (C(NO₂)) 144.2 (CH₂-C-CH-CH), 135.2 (Ct _{(Naphthyl)}), 134.5 (C-SO₂), 131.9 (Cq _{(Naphthyl)}), 131.0 (Ct _{(Naphthyl)}), 130.2 (C(NO₂)-CH-CH, C(NO₂)-CH-CH), 129.3 (Ct _{(Naphthyl)}), 128.9 (Cq _{(Naphthyl)}), 128.6 (Ct _{(Naphthyl)}), 127.2 (Ct _{(Naphthyl)}), 124.8 (Ct _{(Naphthyl)}), 124.3 (C_{t (Naphthyl)}), 123.8 (C-CH-CH, C-CH-CH), 45.2 (N-CH₂-CH₂-N-SO₂), 40.6 (S-CH₂-C). | | | |
| **IR (ATR):** | v (cm⁻¹) = 3107 (w), 2985 (w), 2925 (w), 2860 (w), 1605 (w), 1596 (w), 1520 (s), 1490 (w), 1435 (m), 1420 (m), 1338 (s), 1327 (s), 1320 (s), 1275 (vs), 1226 (w), 1206 (s), 1191 (w), 1176 (vs), 1154 (s), 1140 (m), 1088 (w), 1034 (m), 1003 (m), 968 (m), 927 (s), 858 (s), 801 (w), 777 (vs), 724 (s), 639 (w), 550 (m), 537 (w), 515 (vs), 495 (m), 475 (w), 456 (vs). | | | |
| **MS** (ESI+): | m/z (%) = 488 (100, [M+H]⁺) | | | |
| **HRMS** (ESI+): | m/z für C₂₂H₂₁N₃O₄S₃ | | | |
| | ber.: | 487.0694 | | gef.: 487.0686 |
| **EA:** | ber.: | C: 54.19 | H:4.34 | N: 8.62 |
| | gef.: | C:54.30 | H: 4.48 | N:8.47 |

### Darstellung von (4-Nitrophenyl)methyl-4-(2,4-dichlor-5-sulfamoyl-benzoyl)piperazin-1-carbodithioat (Schl32333):

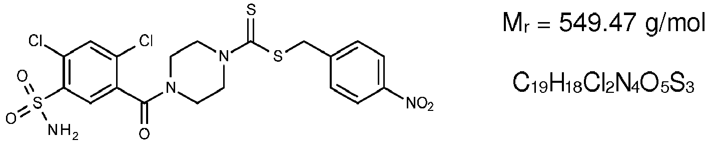

Gemäß **AAV4** wurden 405 mg 2,4-Dichlor-5-sulfamoyl-benzoesäure (1.5 mmol, 1.0 eq), 505 mg (4-Nitrophenyl)methylpiperazin-1-carbodithioathydrochlorid (**Schl32319**, 1.5 mmol, 1.0 eq), 1.01 mL Triethylamin (5.25 mmol, 3.5 eq), 338 mg HOBt (2.25 mmol, 1.5 eq) und 479 mg EDC-HCl (2.25 mmol, 1.5 eq) umgesetzt. Säulenchromatographie an Kieselgel (Cyclohexan/ EtOAc 1:2) ergab 423 mg von **Schl32333** (51.3%) in Form eines blassgelben Feststoffes.

| | | | | |
|---|---|---|---|---|
| **DC:** | R_{f} = 0.35 (Cyclohexan/ EtOAc 1:2). | | | |
| **Fp**.**:** | 134 °C | | | |
| **¹H-NMR:** | (CDCl₃, 400 MHz), δ [ppm] = 8.15 (*d*, ³J = 8.9 Hz, 2H, C-CH-C*H*-C-NO₂, C-CH-C*H*-C-NO₂), 8.06 (*s*, 1H, C-C*H*-C-SO₂NH₂), 7.61 (*s*, 1H, Cl-C-C*H*-C-Cl), 7.55 (*d*, ³J = 8.7 Hz, 2H, C-CH-C*H*-C-NO₂, C-CH-C*H*-C-NO₂), 5.56 (*sbr,* 2H, N*H₂*), 4.68 (*s*, 2H, S-C*H₂-*C), 4.55-4.28 (*m*, 2H, N-CH*H*-CH₂-N-C(=O), N-CH*H*-CH₂-N-C(=O)), 4.21-3.98 (*m*, 2H, N-CH*H*-CH₂-N-C(=O), N-CH*H*-CH₂-N-C(=O)), 3.95-3.82 (*m*, 2H, N-CH₂-C*H*H-N-(=O), N-CH₂-C*H*H-N-C(=O)), 3.42-3.33 (*m*, 2H, N-CH₂-C*H*H-N-(=O), N-CH₂-C*H*H-N-C(=O)). | | | |
| **¹³C**-**NMR:** | (CDCl₃, 100 MHz), δ [ppm] = 196.4 (C=S), 165.1 (C=O), 147.3 (C(NO₂)) 144.2 (CH₂-C-CH-CH), 139.3 (C(Cl)-C-C(=O)), 135.2 (C-SO₂-NH₂), 134.0 (C(Cl)-C-SO₂-NH₂), 133.2 (C-C(=O)), 132.4 (Cl-C-CH-C-Cl), 130.2 (C(NO₂)-CH-CH, C(NO₂)-CH-CH), 129.2 (C(=O)-C-CH), 123.8 (C-CH-CH, C-CH-CH), 45.9 (N-CH₂-CH₂-N-C(=O)), 41.5 N-CH₂-CH₂-N-C(=O)), 40.7 (S-CH₂-C). | | | |
| **IR (ATR):** | v (cm⁻¹) = 1730 (w), 1631 (m), 1585 (w), 1543 (m), 1470 (w), 1416 (m), 1342 (s), 1282 (w), 1260 (m), 1219 (w), 1159 (s), 1108 (w), 1042 (w), 994 (s), 859 (w), 818 (w), 762 (w), 693 (w), 654 (m), 628 (w), 549 (m), 521 (s), 480 (w), 427 (w). | | | |
| **MS** (ESI+): | m/z (%) = 550 (100, [M+H]⁺) | | | |
| **HRMS** (ESI+): | m/z für C₁₉H₁₈Cl₂N₄O₅S₃ | | | |
| | ber.: | 549.9816 | | gef.: 549.9816 |
| **EA:** | ber.: | C: 41.53 | H: 3.30 | N: 10.20 |
| | gef.: | C: 41.47 | H: 3.38 | N: 10.03 |

### Darstellung von (4-Nitrophenyl)methyl-4-(2-hydroxybenzoyl)piperazin-1-carbodithioat (Schl32334):

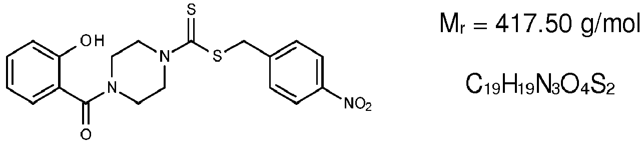

Gemäß **AAV4** wurden 207 mg Salicylsäure (1.5 mmol, 1.0 eq), 505 mg (4-Nitrophenyl)methylpiperazin-1-carbodithioathydrochlorid (**Schl32319**, 1.5 mmol, 1.0 eq), 1.01 mL Triethylamin (5.25 mmol, 3.5 eq), 338 mg HOBt (2.25 mmol, 1.5 eq) und 479 mg EDC-HCl (2.25 mmol, 1.5 eq) umgesetzt. Säulenchromatographie an Kieselgel (Cyclohexan/ EtOAc 1:1) ergab 453 mg von **Schl32334** (72.4%) in Form eines farblosen Feststoffes.

| | | | | |
|---|---|---|---|---|
| **DC:** | R_{f} = 0.34 (Cyclohexan/ EtOAc 1:1). | | | |
| **Fp**.**:** | 255 °C | | | |
| **¹H-NMR:** | (DMSO-D₆, 400 MHz), δ [ppm] = 9.17 (*sbr,* 1H, O*H*), 8.14 (*d*, ³J = 8.9 Hz, 2H, C-CH-C*H*-C-NO₂, C-CH-C*H*-C-NO₂), 7.89 (*d*, ³J = 7.2 Hz, 1H, C-CH-C*H*-CH-CH-OH), 7.65 (*d*, ³J = 8.7 Hz, 2H, C-CH-C*H*-C-NO₂, C-CH-C*H*-C-NO₂), 7.33-7.18 (*m*, 3H, C*H*_{(Phenyl)}), 4.67 (*s*, 2H, S-C*H₂*-C), 4.28-4.17 (*m*, 2H, N-CH*H*-CH₂-N-C(=O), N-CH*H*-CH₂-N-C(=O)), 4.11-3.85 (*m*, 2H, N-CH*H*-CH₂-N-C(=O), N-CH*H*-CH₂-N-C(=O)), 3.41-3.26 (m, 4H, N-CH₂-C*H₂*-N-C(=O), N-CH₂-C*H₂*-N-C(=O)). | | | |
| **¹³C**-**NMR:** | (DMSO-D₆, 100 MHz), δ [ppm] = 196.1 (C=S), 166.8 (C=O), 159.1 (C-OH)), 147.3 (C(NO₂)) 145.7 (CH₂-C-CH-CH), 131.1 (HO-CH-CH-CH-CH-C), 130.9 (C(NO₂)-CH-CH, C(NO₂)-CH-CH), 128.6 (HO-CH-CH-CH-CH-C), 124.0 (C-CH-CH, C-CH-CH), 121.1 (HO-CH-CH-CH-CH-C), 120.8 (C-C(O)), 117.5 (HO-CH-CH-CH-CH-C), 46.1 (N-CH₂-CH₂-N-C(=O)), 39.9 (S-CH₂-C). | | | |
| **IR (ATR):** | v (cm⁻¹ ) = 3062 (w), 3019 (w), 2922 (w), 2856 (w), 1641 (w), 1598 (w), 1576 (s), 1512 (w), 1475 (vs), 1452 (m), 1425 (w), 1367 (vs), 1325 (w), 1287 (vs), 1272 (w), 1261 (m), 1243 (m), 1216 (vs), 1153 (w), 1107 (w), 1064 (w), 996 (vs), 893 (w), 839 (m), 806 (w), 780 (m), 767 (vs), 723 (vs), 644 (w), 580 (w), 511 (s), 489 (s), 416 (w). | | | |
| **MS** (ESI-): | m/z (%) = 416 (100, [M-H]⁻) | | | |
| **HRMS** (ESI+): | m/z für C₁₉H₁₉N₃O₄S₂ | | | |
| | ber.: | 417.0817 | | gef.: 417.0803 |
| **EA:** | ber.: | C: 54.66 | H:4.59 | N: 10.06 |
| | gef.: | C: 54.52 | H:4.84 | N:10.19 |

### Darstellung von [2-[4-[(4-Nitrophenyl)methylsulfanylcarbothioyl]-piperazin-1-carbonyl]-phenyl]acetat (Schl32335):

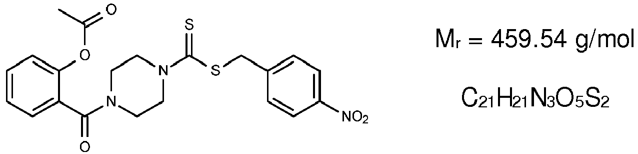

Gemäß **AAV4** wurden 270 mg Acetylsalicylsäure (1.5 mmol, 1.0 eq), 505 mg (4-Nitrophenyl)methylpiperazin-1-carbodithioathydrochlorid (**Schl32319**, 1.5 mmol, 1.0 eq), 1.01 mL Triethylamin (5.25 mmol, 3.5 eq), 338 mg HOBt (2.25 mmol, 1.5 eq) und 479 mg EDC-HCl (2.25 mmol, 1.5 eq) umgesetzt. Säulenchromatographie an Kieselgel (Cyclohexan/ EtOAc 1:1) ergab 310 mg von **Schl32335** (45.0%) in Form eines farblosen Feststoffes.

| | | | | |
|---|---|---|---|---|
| **DC:** | R_{f} = 0.29 (Cyclohexan/ EtOAc 1:1). | | | |
| **Fp**.**:** | 62 °C | | | |
| **¹H-NMR:** | (DMSO-D₆, 400 MHz), δ [ppm] = 8.14 (*d*, ³J = 8.7 Hz, 2H, C-CH-C*H*-C-NO₂, C-CH-C*H*-C-NO₂), 7.64 (*d*, ³J = 8.7 Hz, 2H, C-CH-C*H*-C-NO₂, C-CH-C*H*-C-NO₂), 7.51-7.22 (*m*, 4H, C*H*_{(Phenyl)}), 4.71 (*s*, 2H, S-C*H₂*-C), 4.35-4.21 (*m*, 2H, N-CH*H*-CH₂-N-C(=O), N-CH*H*-CH₂-N-C(=O)), 4.18-3.92 (*m*, 2H, N-CH*H*-CH₂-N-C(=O), N-CH*H*-CH₂-N-C(=O)), 3.41-3.31 (*m*, 4H, N-CH₂-C*H₂*-N-C(=O), N-CH₂-C*H₂*-N-C(=O)), 3.29 (*s*, 3H, C*H₃*)*.* | | | |
| **¹³C**-**NMR:** | (DMSO-D₆, 100 MHz), δ [ppm] = 195.1 (C=S), 169.5 (C(=O)-CH₃), 166.2 (C-C(=O)), 147.1 (C(NO₂)) 145.7 (CH₂-C-CH-CH), 143.9 (C(=O)-CH₃), 131.1 (O-CH-CH-CH-CH-C), 130.9 (C(NO₂)-CH-CH, C(NO₂)-CH-CH), 129.5 (C-C(=O)-N), 128.4 (O-CH-CH-CH-CH-C), 126.7 (O-CH-CH-CH-CH-C), 124.0 (C-CH-CH, C-CH-CH), 123.7 (O-CH-CH-CH-CH-C), 45.9 (N-CH₂-CH₂-N-C(=O)), 41.3 (S-CH₂-C), 21.2 (CH₃). | | | |
| **IR (ATR):** | v (cm⁻¹) = 3062 (w), 3019 (w), 2922 (w), 2856 (w), 1641 (w), 1598 (w), 1576 (s), 1512 (w), 1475 (vs), 1452 (m), 1425 (w), 1367 (vs), 1325 (w), 1287 (vs), 1272 (w), 1261 (m), 1243 (m), 1216 (vs), 1153 (w), 1107 (w), 1064 (w), 996 (vs), 893 (w), 839 (m), 806 (w), 780 (m), 767 (vs), 723 (vs), 644 (w), 580 (w), 511 (s), 489 (s), 416 (w). | | | |
| **MS** (ESI+): | m/z (%) = 477 (100, [M+NH4]⁺) | | | |
| **HRMS** (ESI+): | m/z für C₂₁H₂₁N₃O₅S₂ | | | |
| | ber.: | 459.0923 | | gef.: 459.0919 |
| **EA:** | ber.: | C: 54.89 | H:4.61 | N:9.14 |
| | gef.: | C: 54.69 | H: 4.68 | N: 9.23 |

### Darstellung von (4-Nitrophenyl)methyl-4-[2-(dimethylamino)acetyl]piperazin-1-carbodithioat (Schl32351):

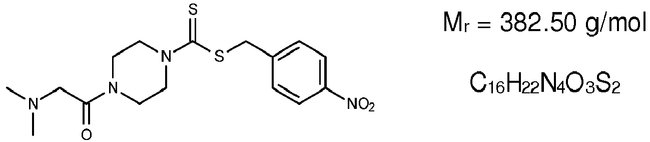

Gemäß **AAV4** wurden 155 mg *N,N-*Dimethylglycin (1.5 mmol, 1.0 eq), 505 mg (4-Nitrophenyl)methylpiperazin-1-carbodithioathydrochlorid (**Schl32319**, 1.5 mmol, 1.0 eq), 1.01 mL Triethylamin (5.25 mmol, 3.5 eq), 338 mg HOBt (2.25 mmol, 1.5 eq) und 479 mg EDC-HCl (2.25 mmol, 1.5 eq) umgesetzt. Säulenchromatographie an Kieselgel (Cyclohexan/ EtOAc 1:4) ergab 425 mg von **Schl32351** (74.0%) in Form eines blassgelben Feststoffes.

| | | | | |
|---|---|---|---|---|
| **DC:** | R_{f} = 0.23 (Cyclohexan/ EtOAc 1:4). | | | |
| **Fp**.**:** | 93 °C | | | |
| **¹H-NMR:** | (CDCl₃, 400 MHz), δ [ppm] = 8.14 (*d*, ³J = 8.7 Hz, 2H, C-CH-C*H*-C-NO₂, C-CH-C*H*-C-NO₂), 7.55 (*d*, ³J = 8.7 Hz, 2H, C-CH-C*H*-C-NO₂, C-CH-C*H*-C-NO₂), 4.68 (*s*, 2H, S-C*H₂*-C), 4.45-4.27 (*m*, 2H, N-CH*H*-CH₂-N-C(=O), N-CH*H*-CH₂-N-C(=O)), 4.16-3.94 (*m*, 2H, N-CH*H*-CH₂-N-C(=O), N-CH*H*-CH₂-N-C(=O)), 3.77 (*t*, ³J = 5.2 Hz, 2H, N-CH₂-CH*H*-N-C(=O), N-CH₂-CH*H*-N-C(=O)), 3.71 (*t*, ³J = 5.3 Hz, 2H, N-CH₂-CH*H*-N-C(=O), N-CH₂-CH*H*-N-C(=O)), 3.14 (s, 2H, N-C*H₂*-C(=O)), 2.27 (*s*, 6H, C*H₃*)*.* | | | |
| **¹³C**-**NMR:** | (CDCl₃, 100 MHz), δ [ppm] = 196.0 (C=S), 168.9 (C(=O)-NH), 147.3 (C(NO₂)) 144.4 (CH₂-C-CH-CH), 130.2 (C(NO₂)-CH-CH, C(NO₂)-CH-CH), 123.8 (C-CH-CH, C-CH-CH), 62.8 (N-CH₂-C(=O), 45.5 (CH₃), 44.6 (N-CH₂-CH₂-N-C(=O)), 41.2 N-CH₂-CH₂-N-C(=O)), 40.6 (S-CH₂-C). | | | |
| **IR (ATR):** | v (cm⁻¹ ) = 2956 (w), 2897 (w), 2860 (w), 1633 (vs), 1608 (w), 1600 (w), 1517 (s), 1457 (s), 1440 (m), 1422 (vs), 1399 (w), 1338 (vs), 1282 (w), 1245 (m), 1219 (vs), 1158 (s), 1143 (m), 1107 (m), 1046 (w), 1024 (m), 1003 (m), 986 (s), 923 (w), 869 (s), 856 (s), 841 (m), 801 (w), 707 (w), 685 (s), 624 (w), 549 (w), 517 (w), 496 (m), 467 (w). | | | |
| **MS** (ESI+): | m/z (%) = 383 (100, [M+H]⁺) | | | |
| **HRMS** (ESI+): | m/z für C₁₆H₂₂N₄O₃S₂ | | | |
| | ber.: | 382.1133 | | gef.: 382.1158 |
| **EA:** | ber.: | C: 50.24 | H: 5.80 | N: 14.65 |
| | gef.: | C: 50.45 | H: 5.82 | N: 14.44 |

### Darstellung von (4-Nitrophenyl)methyl-4-cyclopropylsulfonylpiperazin-1-carbodithioat (Schl32360):

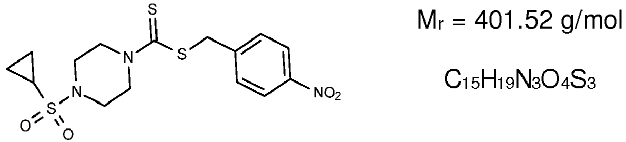

Gemäß **AAV12** wurden 421 mg (4-Nitrophenyl)methylpiperazin-1-carbodithioathydrochlorid (**Schl32319**, 1.25 mmol, 1.0 eq) und 0.19 mL Cyclopropylsulfonylchlorid (1.88 mmol, 1.5 eq) umgesetzt. Säulenchromatographie an Kieselgel (Cyclohexan/ EtOAc 1:1) ergab 379 mg von **Schl32360** (75.6%) in Form eines blassgelben Feststoffes.

| | | | | |
|---|---|---|---|---|
| **DC:** | R_{f} = 0.47 (Cyclohexan/ EtOAc 1:1). | | | |
| **Fp**.**:** | 175 °C | | | |
| **¹H-NMR:** | (CDCl₃, 400 MHz), δ [ppm] = 8.15 (*d*, ³J = 8.0 Hz, 2H, C-CH-C*H*-C-NO₂, C-CH-C*H*-C-NO₂), 7.55 (*d*, ³J = 8.2 Hz, 2H, C-CH-C*H*-C-NO₂, C-CH-C*H*-C-NO₂), 4.68 (*s*, 2H, S-C*H₂*-C), 4.51-4.30 (*m*, 2H, N-CH*H*-CH₂-N-SO₂, N-CH*H*-CH₂-N-SO₂), 4.22-3.95 (*m*, 2H, N-CH*H*-CH₂-N-SO₂, N-CH*H*-CH₂-N-SO₂), 3.40 (*t*, ³J = 4.7 Hz, 4H, N-CH₂-C*H₂*-N-SO₂, N-CH₂-C*H₂*-N-SO₂), 2.28-2.19 (*m*, 1H, SO₂-C*H*), 1.18 (*q*, ³J = 5.7 Hz, 2H, C*H*_{*2*(Cyclopropyl)}), 1.01 (*q*, ³J = 6.4 Hz, 2H, C*H*_{*2*(Cyclopropyl)}). | | | |
| **¹³C**-**NMR:** | (CDCl₃, 100 MHz), δ [ppm] = 196.3 (C=S), 147.3 (C(NO₂)) 144.2 (CH₂-C-CH-CH), 130.3 (C(NO₂)-CH-CH, C(NO₂)-CH-CH), 123.8 (C-CH-CH, C-CH-CH), 45.7 (S-CH₂-C), 40.7 (N-CH₂-CH₂-N-SO₂, N-CH₂-CH₂-N-SO₂), 25.9 (CH_{2(Cyclopropyl)}-CH_{2(Cyclopropyl)}), 4.7 (CH_{(Cyclopropyl)}). | | | |
| **IR (ATR):** | v (cm⁻¹) = 2986 (w), 2855 (w), 1517 (s), 1462 (m), 1364 (w), 1339 (vs), 1327 (m), 1311 (m), 1275 (s), 1229 (m), 1190 (w), 1152 (s), 1112 (m), 1049 (m), 1032 (m), 934 (s), 889 (s), 858 (m), 801 (w), 740 (vs), 722 (vs), 639 (w), 572 (m), 542 (s), 496 (w), 463 (s). | | | |
| **MS** (ESI+): | m/z (%) = 419 (100, [M+NH4]⁺) | | | |
| **HRMS** (ESI+): | m/z für C₁₅H₁₉N₃O₄S₃ | | | |
| | ber.: | 401.0538 | | gef.: 401.0533 |
| **EA:** | ber.: | C: 44.87 | H: 4.77 | N: 10.47 |
| | gef.: | C: 45.07 | H: 4.88 | N: 10.32 |

### Darstellung von (4-Nitrophenyl)methyl-4-(dimethylsulfamoyl)piperazin-1-carbodithioat (Schl32361):

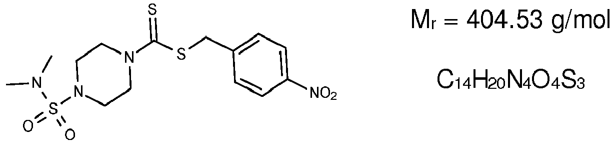

Gemäß **AAV12** wurden 590 mg (4-Nitrophenyl)methylpiperazin-1-carbodithioathydrochlorid (**Schl32319**, 1.75 mmol, 1.0 eq) und 0.23 mL *N,N-*Dimethylsulfamoylchlorid (2.19 mmol, 1.5 eq) umgesetzt. Säulenchromatographie an Kieselgel (Cyclohexan/ EtOAc 1:1) ergab 550 mg von **Schl32361** (77.7%) in Form eines blassgelben Feststoffes.

| | | | | |
|---|---|---|---|---|
| **DC:** | R_{f} = 0.40 (Cyclohexan/ EtOAc 1:1). | | | |
| **Fp**.**:** | 136 °C | | | |
| **¹H-NMR:** | (DMSO-D₆, 400 MHz), δ [ppm] = 8.14 (*d*, ³J = 8.7 Hz, 2H, C-CH-C*H*-C-NO₂, C-CH-C*H*-C-NO₂), 7.64 (*d*, ³J = 8.7 Hz, 2H, C-CH-C*H*-C-NO₂, C-CH-C*H*-C-NO₂), 4.70 (s, 2H, S-C*H₂*-C), 4.37-4.19 (*m*, 2H, N-CH*H*-CH₂-N-SO₂, N-CH*H*-CH₂-N-SO₂), 4.15-3.84 (*m*, 2H, N-CH*H*-CH₂-N-SO₂, N-CH*H*-CH₂-N-SO₂), 3.26 (*t*, ³J = 5.2 Hz, 4H, N-CH₂-C*H₂*-N-SO₂, N-CH₂-C*H₂*-N-SO₂), 2.75 (s, 6H, C*H₃*)*.* | | | |
| **¹³C**-**NMR:** | (DMSO-D₆, 100 MHz), δ [ppm] = 195.3 (C=S), 147.1 (C(NO₂)) 145.6 (CH₂-C-CH-CH), 130.9 (C(NO₂)-CH-CH, C(NO₂)-CH-CH), 124.0 (C-CH-CH, C-CH-CH), 46.1 (S-CH₂-C), 41.7 (N-CH₂-CH₂-N-SO₂, N-CH₂-CH₂-N-SO₂), 38.4 (CH₃). | | | |
| **IR (ATR):** | v (cm⁻¹ ) = 1604 (w), 1516 (w), 1422 (w), 1393 (vs), 1345 (w), 1311 (m), 1244 (w), 1178 (w), 1145 (s), 1104 (w), 1014 (w), 992 (w), 936 (s), 854 (w), 814 (w), 758 (w), 733 (vs), 704 (vs), 664 (m), 574 (w), 521 (s). | | | |
| **MS** (ESI+): | m/z (%) = 422 (100, [M+NH4]⁺) | | | |
| **HRMS** (ESI+): | m/z für C₁₄H₂₀N₄O₄S₃ | | | |
| | ber.: | 404.0647 | | gef.: 404.0645 |
| **EA:** | ber.: | C: 41.57 | H: 4.98 | N: 13.85 |
| | gef.: | C: 41.63 | H: 4.99 | N: 13.70 |

### Darstellung von (4-Nitrophenyl)methyl-4-(4-acetamidophenyl)sulfonylpiperazin-1-carbodithioat (Schl32362):

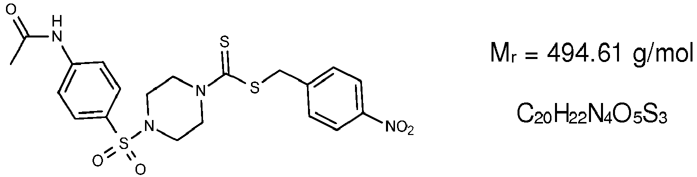

Gemäß **AAV12** wurden 421 mg (4-Nitrophenyl)methylpiperazin-1-carbodithioathydrochlorid (**Schl32319**, 1.25 mmol, 1.0 eq) und 439 mg 4-Acetamidobenzensulfonylchlorid (1.88 mmol, 1.5 eq) umgesetzt. Säulenchromatographie an Kieselgel (Cyclohexan/ EtOAc 1:1) ergab 475 mg von **Schl32362** (76.9%) in Form eines blassgelben Feststoffes.

| | | | | |
|---|---|---|---|---|
| **DC:** | R_{f} = 0.11 (Cyclohexan/ EtOAc 1:1). | | | |
| **Fp**.**:** | 204 °C | | | |
| **¹H-NMR:** | (DMSO-D₆, 400 MHz), δ [ppm] = 10.34 (*sbr,* 1H, N*H*), 8.10 (*d*, ³J = 8.7 Hz, 2H, C-CH-C*H*-C-NO₂, C-CH-C*H*-C-NO₂), 7.78 (*d*, ³J = 8.7 Hz, 2H, C*H*-C-SO₂, C*H*-C-SO₂), 7.64 (*d*, ³J = 8.9 Hz, 2H, C-CH-C*H*-C-NO₂, C-CH-C*H*-C-NO₂), 7.57 (*d*, ³J = 8.5 Hz, 2H, C*H*-CH-C-SO₂, C*H*-CH-C-SO₂), 4.63 (*s*, 2H, S-C*H₂*-C), 4.39-4.20 (*m*, 2H, N-CH*H*-CH₂-N-SO₂, N-CH*H*-CH₂-N-SO₂), 4.12-3.86 (*m*, 2H, N-CH*H*-CH₂-N-SO₂, N-CH*H*-CH₂-N-SO₂), 2.96 (*t*, ³J = 4.6 Hz, 4H, N-CH₂-C*H₂*-N-SO₂, N-CH₂-C*H₂*-N-SO₂), 2.06 *(s,* 3H, C*H₃*)*.* | | | |
| **¹³C**-**NMR:** | (DMSO-D₆, 100 MHz), δ [ppm] = 195.4 (C=S), 169.7 (C(=O)-NH), 147.1 (C(NO₂)) 145.4 (C-NH), 144.3 (CH₂-C-CH-CH), 130.8 (CH-C-SO₂), 129.3 (C(NO₂)-CH-CH, C(NO₂)-CH-CH), 128.5 (C-SO₂), 124.0 (C-CH-CH, C-CH-CH), 119.3 (CH-CH-C-SO₂, CH-CH-C-SO₂), 45.9 (S-CH₂-C), 40.9 (N-CH₂-CH₂-N-SO₂, N-CH₂-CH₂-N-SO₂), 24.7 (CH₃). | | | |
| **IR (ATR):** | v (cm⁻¹ ) = 3306 (w), 2923 (w), 1591 (s), 1512 (w), 1429 (w), 1396 (s), 1342 (w), 1324 (m), 1313 (w), 1275 (m), 1234 (w), 1186 (w), 1166 (s), 1136 (m), 1118 (m), 1054 (w), 995 (m), 941 (s), 924 (s), 829 (m), 758 (w), 736 (s), 696 (s), 632 (s), 610 (vs), 570 (vs), 546 (vs), 493 (w), 456 (m). | | | |
| **MS** (ESI+): | m/z (%) = 512 (100, [M+NH4]⁺) | | | |
| **HRMS** (ESI+): | m/z für C₂₀H₂₂N₄O₅S₃ | | | |
| | ber.: | 494.0752 | | gef.: 494.0742 |
| **EA:** | ber.: | C: 48.57 | H: 4.48 | N: 11.33 |
| | gef.: | C: 48.78 | H: 4.67 | N: 11.00 |

### Darstellung von (4-Nitrophenyl)methyl-4-ethylsulfonylpiperazin-1-carbodithioat (Schl32363):

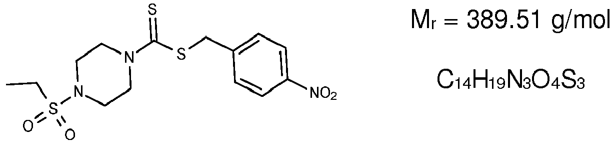

Gemäß **AAV12** wurden 421 mg (4-Nitrophenyl)methylpiperazin-1-carbodithioathydrochlorid (**Schl32319**, 1.25 mmol, 1.0 eq) und 0.18 mL Ethansulfonylchlorid (1.88 mmol, 1.5 eq) umgesetzt. Säulenchromatographie an Kieselgel (Cyclohexan/ EtOAc 1:1) ergab 382 mg von **Schl32363** (78.4%) in Form eines blassgelben Feststoffes.

| | | | | |
|---|---|---|---|---|
| **DC:** | R_{f} = 0.47 (Cyclohexan/ EtOAc 1:1) | | | |
| **Fp**.**:** | 151 °C | | | |
| **¹H-NMR:** | (DMSO-D₆, 400 MHz), δ [ppm] = 8.14 (*d*, ³J = 8.9 Hz, 2H, C-CH-C*H*-C-NO₂, C-CH-C*H*-C-NO₂), 7.64 (*d*, ³J = 8.7 Hz, 2H, C-CH-C*H*-C-NO₂, C-CH-C*H*-C-NO₂), 4.71 (s, 2H, S-C*H₂*-C), 4.37-4.19 (*m*, 2H, N-CH*H*-CH₂-N-SO₂, N-CH*H*-CH₂-N-SO₂), 4.14-3.91 (*m*, 2H, N-CH*H*-CH₂-N-SO₂, N-CH*H*-CH₂-N-SO₂), 3.18 (*t*, ³J = 4.9 Hz, 4H, N-CH₂-C*H₂*-N-SO₂, N-CH₂-C*H₂*-N-SO₂), 3.11 (q, ³J = 7.3 Hz, 2H, C*H₂*-CH₃), 1.18 (*t*, ³J = 7.3 Hz, 3H, CH₂-C*H₃*). | | | |
| **¹³C**-**NMR:** | (DMSO-D₆, 100 MHz), δ [ppm] = 195.3 (C=S), 147.1 (C(NO₂)) 145.6 (CH₂-C-CH-CH), 130.9 (C(NO₂)-CH-CH, C(NO₂)-CH-CH), 124.0 (C-CH-CH, C-CH-CH), 50.2 (SO₂-CH₂-CH₃), 45.2 (S-CH₂-C), 43.3 (N-CH₂-CH₂-N-SO₂, N-CH₂-C*H₂*-N-SO₂), 7.9 (SO₂-CH₂-CH₃). | | | |
| **IR (ATR):** | v (cm⁻¹) = 1605 (w), 1597 (w), 1517 (s), 1460 (w), 1424 (m), 1363 (w), 1324 (vs), 1275 (s), 1225 (s), 1176 (w), 1139 (s), 1111 (s), 1084 (m), 998 (m), 931 (s), 858 (m), 800 (w), 749 (vs), 723 (s), 687 (m), 573 (m), 528 (w), 511 (vs), 470 (w), 456 (s). | | | |
| **MS** (ESI+): | m/z (%) = 407 (100, [M+NH4]⁺) | | | |
| **HRMS** (ESI+): | m/z für C₁₄H₁₉N₃O₄S₃ | | | |
| | ber.: | 389.0538 | | gef.: 389.0527 |
| **EA:** | ber.: | C: 43.17 | H: 4.92 | N: 10.79 |
| | gef.: | C: 43.25 | H: 4.96 | N: 10.74 |

### Darstellung von (4-Nitrophenyl)methyl-4-butylsulfonylpiperazin-1-carbodithioat (Schl32364):

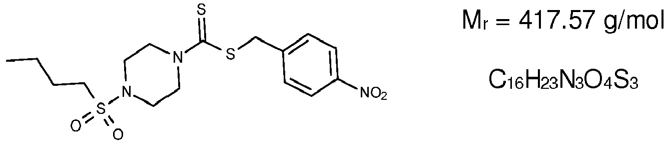

Gemäß **AAV12** wurden 421 mg (4-Nitrophenyl)methylpiperazin-1-carbodithioathydrochlorid (**Schl32319**, 1.25 mmol, 1.0 eq) und 0.24 mL Butansulfonylchlorid (1.88 mmol, 1.5 eq) umgesetzt. Säulenchromatographie an Kieselgel (Cyclohexan/ EtOAc 1:1) ergab 397 mg von **Schl32364** (76.1 %) in Form eines blassgelben Feststoffes.

| | | | | |
|---|---|---|---|---|
| **DC:** | R_{f} = 0.57 (Cyclohexan/ EtOAc 1:1). | | | |
| **Fp**.**:** | 125 °C | | | |
| **¹H-NMR:** | (DMSO-D₆, 400 MHz), δ [ppm] = 8.14 (d, ³J = 8.7 Hz, 2H, C-CH-C*H*-C-NO₂, C-CH-C*H*-C-NO₂), 7.64 (*d*, ³J = 8.7 Hz, 2H, C-CH-C*H*-C-NO₂, C-CH-C*H*-C-NO₂), 4.71 (s, 2H, S-C*H₂*-C), 4.39-4.17 (*m*, 2H, N-CH*H*-CH₂-N-SO₂, N-CH*H*-CH₂-N-SO₂), 4.12-3.89 (*m*, 2H, N-CH*H*-CH₂-N-SO₂, N-CH*H*-CH₂-N-SO₂), 3.18 (*t*, ³J = 4.9 Hz, 4H, N-CH₂-C*H₂*-N-SO₂, N-CH₂-C*H₂*-N-SO₂), 3.03 (*t*, ³J = 7.3 Hz, 2H, C*H*₂-CH₂-CH₂-CH₃), 1.60 (*quin,* ³J = 7.6 Hz, 2H, C*H*₂-CH₂-CH₃), 1.35 *(sex,* ³J = 7.3 Hz, 2H, C*H*₂-CH₃), 0.85 *(t,* ³J = 7.3 Hz, 3H, CH₂-C*H*₃). | | | |
| **¹³C**-**NMR:** | (DMSO-D₆, 100 MHz), δ [ppm] = 195.3 (C=S), 147.1 (C(NO₂)) 145.7 (CH₂-C-CH-CH), 130.9 (C(NO₂)-CH-CH, C(NO₂)-CH-CH), 124.0 (C-CH-CH, C-CH-CH), 48.2 (SO₂-CH₂), 45.2 (S-CH₂-C), 40.3 (N-CH₂-CH₂-N-SO₂, N-CH₂-C*H₂*-N-SO₂), 25.0 (SO₂-CH₂-CH₂), 21.5 (SO₂-CH₂-CH₂-CH₂), 14.0 (CH₃). | | | |
| **IR (ATR):** | v (cm⁻¹ ) = 3109 (w), 2942 (w), 1610 (w), 1599 (w), 1539 (s), 1492 (w), 1421 (m), 1347 (w), 1327 (vs), 1300 (s), 1272 (m), 1220 (vs), 1138 (vs), 1108 (m), 1035 (w), 953 (s), 936 (m), 833 (m), 785 (m), 740 (w), 702 (vs), 624 (w), 578 (m), 532 (s), 498 (w), 458 (m). | | | |
| **MS** (ESI+): | m/z (%) = 435 (100, [M+NH4]⁺) | | | |
| **HRMS** (ESI+): | m/z für C₁₆H₂₃N₃O₄S₃ | | | |
| | ber.: | 417.0851 | | gef.: 417.0849 |
| **EA:** | ber.: | C: 46.02 | H: 5.55 | N: 10.06 |
| | gef.: | C: 46.17 | H: 5.56 | N: 10.04 |

### Darstellung von 2-(1,3-Dioxan-2-yl)ethylmorpholin-4-carbodithioat (Schl32020):

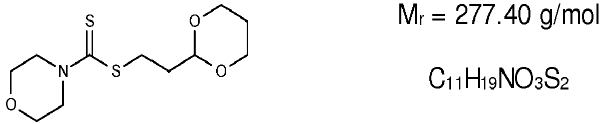

Gemäß **AAV5** wurden 0.41 mL 2-(2-Bromethyl)-1,3-dioxan (3.0 mmol, 1.0 eq), 0.36 mL Kohlenstoffdisulfid (6.0 mmol, 2.0 eq) und 0.52 mL Morpholin (6.0 mmol, 2.0 eq) umgesetzt. Säulenchromatographie an Kieselgel (Cyclohexan/ EtOAc 3:2) ergab 327 mg von **Schl32020** (37.2%) in Form eines gelben Feststoffes.

| | | | | |
|---|---|---|---|---|
| **DC:** | R_{f} = 0.28 (Cyclohexan/ EtOAc 3:2). | | | |
| **Fp**.**:** | 80 °C | | | |
| **¹H-NMR:** | (CDCl₃, 400 MHz), δ [ppm] = 4.69 (*t*, ³J=5.2 Hz, 1H, CH₂-C*H-*O), 4.12-4.05 (*m*, 6H, N-C*H₂*-CH₂, N-C*H₂*-CH₂, CH-O-C*H*H-CH₂, CH-O-C*H*H-CH₂), 4.03-3.96 (*m*, 2H, CH-O-C*H*H-CH₂, CH-O-C*H*H-CH₂), 3.80-3.65 (*m*, 4H, O-C*H₂*-CH₂-N, O-C*H₂*-CH₂-N), 3.36-3.32 (*m*, 2H, S-C*H₂*-CH₂), 2.11-2.01 (*m*, 1H, O-CH₂-C*H*H-CH₂-O), 2.01-1.94 (*m*, 2H, S-CH₂-C*H*₂), 1.35-1.29 (*m*, 1H, O-CH₂-CH*H*-CH₂-O). | | | |
| **¹³C**-**NMR:** | (CDCl₃, 100 MHz), δ [ppm] = 195.5 (C=S), 101.1 (CH₂-CH-O), 67.0 (O-CH₂-CH₂), 66.6 (O-CH₂-CH₂-N, O-CH₂-CH₂-N), 36.7 (N-CH₂-CH₂-O, N-CH₂-CH₂-O), 34.4 (S-CH₂), 31.8 (S-CH₂-CH₂), 25.9 (O-CH₂-CH₂-CH₂). | | | |
| **IR (ATR):** | v (cm⁻¹) = 2859 (m), 1422 (s), 1266 (s), 1231 (m), 1118 (vs), 1072 (s), 997 (vs), 922 (m), 871 (s), 844 (s), 638 (m), 541 (s), 405 (m). | | | |
| **MS** (ESI+): | m/z (%) = 278 (100, [M+H]⁺) | | | |
| **HRMS** (ESI+): | m/z für C₁₁H₁₉NO₃S₂ | | | |
| | ber.: | 277.0806 | | gef.: 277.0807 |
| **EA:** | ber.: | C: 47.63 | H: 6.90 | N: 5.05 |
| | gef.: | C: 47.76 | H: 6.99 | N: 4.99 |

### Darstellung von Methyl-2-(4-bromphenyl)acetat (Schl32034):

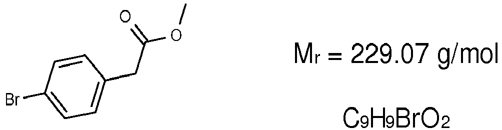

3.0 g 4-Bromphenylessigsäure (13.95 mmol, 1.0 eq) wurden in 50 mL Methanol gelöst, mit 2 mL konzentrierter Schwefelsäure versetzt und 14 Stunden unter Rückfluss erhitzt. Das Lösungsmittel wurde am Rotavapor entfernt, der verbleibende Rückstand in ca. 100 mL Diethylether aufgenommen, 3 Mal gegen 50 mL ges. NaHCO₃-Lösung ausgeschüttelt und abschließend mit ges. NaCl-Lösung gewaschen. Nach Entfernung des Lösungsmittels am Rotavapor wurden 2.56 g von **Schl32034** (80.0%) in Form eines blassgelbes Öls erhalten.

| | | | | |
|---|---|---|---|---|
| **¹H-NMR:** | (CDCl₃, 400 MHz), δ [ppm] = 7.44 (*d*, ³J = 8.2 Hz, 2H, CH₂-C-CH-C*H*-C, CH₂-C-CH-C*H*-C), 7.15 (*d*, ³J = 8.2 Hz, 2H, CH₂-C-C*H*-CH-C, CH₂-C-C*H*-CH-C), 3.69 (s, 3H, O-C*H₃*), 3.57 (s, 2H, C-C*H₂*-COOMe). | | | |
| **¹³C**-**NMR:** | (CDCl₃, 100 MHz), δ [ppm] = 171.6 (C=O), 133.0 (CH-C-CH₂), 131.7 (Br-C-CH, Br-C-CH), 131.0 (Br-C-CH-CH, Br-C-CH-CH), 121.2 (Br-C-CH), 52.2 (O-CH₃), 40.5 (CH₂-COOMe). | | | |
| **IR (ATR):** | v (cm⁻¹ ) = 3125 (w), 3027 (w), 2951 (w), 1733 (vs), 1628 (w), 1589 (w), 1552 (w), 1511 (w), 1488 (m), 1435 (w), 1407 (w), 1340 (m), 1299 (m), 1253 (m), 1218 (m), 1192 (w), 1159 (s), 1107 (w), 1071 (w), 1050 (m), 1011 (s), 929 (w), 894 (w), 836 (w), 801 (s), 784 (m), 756 (w), 729 (w), 679 (w), 652 (w), 571 (w), 526 (w), 489 (m), 449 (w), 423 (w). | | | |
| **MS** (ESI+): | m/z (%) = 251 (100, [M^{79Br}+Na]⁺), 253 (95, [M^{81Br}+Na]⁺) | | | |
| **HRMS** (ESI+): | m/z für C₉H₉BrO₂ | | | |
| | ber.: | 227.9786 | | gef.: 227. 9791 |
| **EA:** | ber.: | C: 47.19 | H: 3.96 | N: 0.00 |
| | gef.: | C: 46.70 | H: 3.82 | N: 0.10 |

### Darstellung von 2-(1,3-Dioxan-2-yl)ethyl 4-methylpiperazin-1-carbodithioat (Schl32141):

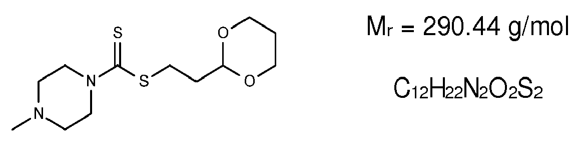

Gemäß **AAV7** wurden 0.34 mL *N*-Methylpiperazin (3.0 mmol, 1.0 eq), 640 mg Kaliumphosphat (3.0 mmol, 1.0 eq), 0.54 mL Kohlenstoffdisulfid (9.0 mmol, 3.0 eq) und 0.41 mL 2-(2-Brommethyl)-1,3-dioxan (3.0 mmol, 1.0 eq) umgesetzt. Säulenchromatographie an Kieselgel (DCM/ MeOH 90:10) ergab 575 mg von **Schl32141** (66.0%) in Form eines blassgelben Feststoffs.

| | | | | |
|---|---|---|---|---|
| **DC:** | R_{f} = 0.53 (DCM/ MeOH 90:10). | | | |
| **Fp**.**:** | 77 °C | | | |
| **¹H-NMR:** | (CDCl₃, 400 MHz), δ [ppm] = 4.16 (*t*, ³J = 5.2 Hz, 1H, S-CH₂-CH₂-C*H*), 4.46-3.82 (*m*, 8H, N-C*H₂*-CH₂-N-CH₃, N-C*H₂*-CH₂-N-CH₃, O-C*H₂*-CH₂-C*H₂*-O), 3.74 *(t,* ³J = 12.2 Hz, 2H, S-C*H₂*-CH₂-CH), 3.36 *(q,* ³J = 8.2 Hz, 2H, S-CH₂-C*H₂*-CH), 2.55-2.46 (*m*, 4H, N-CH₂-C*H₂*-N-CH₃, N-CH₂-C*H₂*-N-CH₃), 2.31 (s, 3H, N-C*H₃*), 2.05-1.97 (*m*, 2H, O-CH₂-C*H₂*-CH₂-O). | | | |
| **¹³C**-**NMR:** | (CDCl₃, 100 MHz), δ [ppm] = 197.1 (C=S), 101.0 (S-CH₂-CH₂-CH), 67.0 (O-CH₂-CH₂-CH₂-O), 54.7 (N-CH₂-CH₂-N-CH₃, N-CH₂-CH₂-N-CH₃), 50.9 (N-CH₂-CH₂-N-CH₃, N-CH₂-CH₂-N-CH₃), 45.7 (N-CH₂-CH₂-N-CH₃), 34.3 (S-CH₂-CH₂-CH), 31.8 (S-CH₂-CH₂-CH), 25.8 (O-CH₂-CH₂-CH₂-O). | | | |
| **IR (ATR):** | v (cm⁻¹) = 2795 (w), 1428 (m), 1288 (m), 1228 (m), 1142 (s), 1072 (m), 1043 (m), 994 (vs), 920 (m), 874 (m), 844 (m), 767 (w), 640 (w), 542 (w), 426 (m). | | | |
| **MS** (ESI+): | m/z (%) = 291 (100, [M+H]⁺) | | | |
| **HRMS** (ESI+): | m/z für C₁₂H₂₂N₂O₂S₂ | | | |
| | ber.: | 290.1123 | | gef.: 290.1111 |
| **EA:** | ber.: | C: 49.62 | H: 7.63 | N: 9.64 |
| | gef.: | C: 49.70 | H: 7.70 | N: 9.57 |

### Darstellung von 2-(1,3-Dioxan-2-yl)ethyl-4-acetylpiperazin-1-carbodithioat (Schl32147):

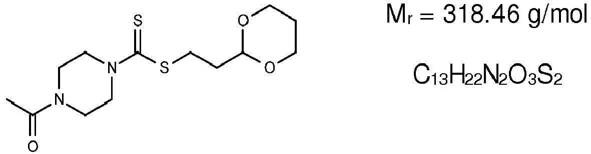

Gemäß **AAV5** wurden 0.41 mL 2-(2-Brommethyl)-1,3-dioxan (3.0 mmol, 1.0 eq), 0.54 mL Kohlenstoffdisulfid (9.0 mmol, 3.0 eq) und 385 mg *N*-Acetylpiperazin (3.0 mmol, 1.0 eq) umgesetzt. Säulenchromatographie an Kieselgel (DCM/ MeOH 90:10) ergab 931 mg von **Schl32147** (95.6%) in Form eines blassgelben Feststoffes.

| | | | | |
|---|---|---|---|---|
| **DC:** | R_{f} = 0.62 (DCM/ MeOH 90:10). | | | |
| **Fp**.**:** | 53 °C | | | |
| **¹H-NMR:** | (CDCl₃, 400 MHz), δ [ppm] = 4.62 (*t*, ³J = 5.0 Hz, 1H, S-CH₂-CH₂-C*H*), 4.55-4.33 (*m*, 4H, O-C*H₂*-CH₂-C*H₂*-O), 4.32-4.11 (*m*, 4H, N-CH₂-C*H₂*-N-C(=O)-CH₃, N-CH₂-C*H₂*-N-C(=O)-CH₃), 3.88-3.60 (*m*, 4H, N-C*H₂*-CH₂-N-C(=O)-CH₃, N-C*H₂*-CH₂-N-C(=O)-CH₃), 3.55 (*t*, ³J = 12.2 Hz, 2H, S-C*H₂*-CH₂-CH), 3.37 (*q*, ³J = 8.2 Hz, 2H, S-CH₂-C*H₂*-CH), 2.11 (*s*, 3H, N-C(=O)-C*H₃*), 2.05-1.97 (*m*, 2H, O-CH₂-C*H₂*-CH₂-O). | | | |
| **¹³C**-**NMR:** | (CDCl₃, 100 MHz), δ [ppm] = 197.7 (C=S), 169.5 (C=O), 100.9 (S-CH₂-CH₂-CH), 67.0 (O-CH₂-CH₂-CH₂-O), 45.3 (N-CH₂-CH₂-N-C(=O)-CH₃, N-CH₂-CH₂-N-C(=O)-CH₃), 40.7 (N-CH₂-CH₂-N-C(=O)-CH₃, N-CH₂-CH₂-N-C(=O)-CH₃), 34.2 (S-CH₂-CH₂-CH), 31.8 (S-CH₂-CH₂-CH), 25.8 (O-CH₂-CH₂-CH₂-O), 21.5 (N-C(=O)-CH₃). | | | |
| **IR (ATR):** | v (cm⁻¹) = 2862 (w), 1639 (s), 1413 (s), 1277 (m), 1244 (m), 1208 (vs), 1128 (s), 1073 (m), 985 (s), 962 (s), 914 (s), 875 (m), 845 (m), 767 (w), 639 (w), 587 (w), 516 (m), 474 (w), 416 (w). | | | |
| **MS** (ESI+): | m/z (%) = 319 (100, [M+H]⁺) | | | |
| **HRMS** (ESI+): | m/z für C₁₃H₂₂N₂O₃S₂ | | | |
| | ber.: | 318.1072 | | gef.: 318.1085 |
| **EA:** | ber.: | C: 49.03 | H: 6.96 | N: 8.80 |
| | gef.: | C: 48.95 | H: 6.58 | N: 9.01 |

### Darstellung von 3-Chlor-N-phenethylacetamid (Schl32179):

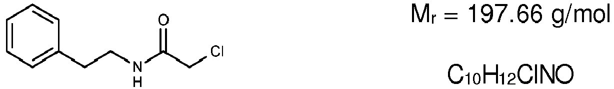

2.52 mL Phenethylamin (20.0 mmol, 1.0 eq) und 2016 mg Natriumhydrogencarbonat (24.0 mmol, 1.2 eq) wurden in 50 mL Dichlormethan gelöst, auf 0 °C gekühlt, 5 Minuten gerührt und mit 1.90 mL Chloracetylchlorid (24.0 mmol, 1.2 eq) versetzt. Anschließen wurde auf Raumtemperatur erwärmt, 4 Stunden bei Raumtemperatur gerührt, die Reaktion mit 50 mL Wasser beendet, mit Dichlormethan extrahiert (5 x 50 mL), die vereinigten organischen Phasen über MgSO₄ getrocknet, filtriert und das Lösungsmittel am Rotavapor entfernt. Säulenchromatographie an Kieselgel (DCM/ MeOH/ NEt₃ 90:10:0.1) ergab 3905 mg von **Schl32179** (98.8%) in Form eines farblosen Feststoffes.

| | | | | |
|---|---|---|---|---|
| **DC:** | R_{f} = 0.62 (DCM/ MeOH/ NEt₃ 90:10:0.1). | | | |
| **Fp**.**:** | 68 °C | | | |
| **¹H-NMR:** | (CDCl₃, 400 MHz), δ [ppm] = 7.35-7.19 (*m*, 5H, C*H*_{(Phenyl)}), 6.61 (*sbr,* 1H, CH₂-N*H-*C(=O)), 4.02 (*s*, 2H, C(=O)-C*H₂*-Cl), 3.57 (*q*, ³J = 6.9 Hz, 2H, C-CH₂-C*H₂*-NH), 2.85 (*t*, ³J = 7.1 Hz, 2H, C-C*H₂*-CH₂-NH). | | | |
| **¹³C**-**NMR:** | (CDCl₃, 100 MHz), δ [ppm] = 165.9 (C=O), 138.4 (C-CH-CH-CH), 128.9 (C-CH-CH-CH, C-CH-CH-CH), 128.8 (C-CH-CH-CH, C-CH-CH-CH), 126.8 (C-CH-CH-CH), 42.8 (C(=O)-CH₂-Cl), 41.0 (C-CH₂-CH₂-NH), 35.6 (C-CH₂-CH₂-NH). | | | |
| **IR (ATR):** | v (cm⁻¹) =3830 (w), 3653 (w), 3335 (w), 3084 (w), 2926 (w), 2860 (w), 1644 (m), 1539 (s), 1494 (w), 1451 (w), 1436 (w), 1399 (w), 1364 (w), 1291 (w), 1259 (m), 1185 (w), 1151 (w), 1087 (w), 1039 (w), 1018 (w), 925 (w), 905 (w), 776 (w), 751 (s), 696 (vs), 578 (w), 556 (s), 498 (s), 444 (w), 425 (s), 408 (w). | | | |
| **MS** (ESI+): | m/z (%) = 198 (100, [M+H]⁺) | | | |
| **HRMS** (ESI+): | m/z für C₁₀H₁₂ClNO | | | |
| | ber.: | 197.0607 | | gef.: 197.0614 |
| **EA:** | ber.: | C:60.76 | H:6.12 | N: 7.09 |
| | gef.: | C:60.89 | H:6.14 | N: 7.01 |

### Darstellung von 3-(2,2-Dimethoxyethylamino)-N-phenethylpropanamid (Schl32180):

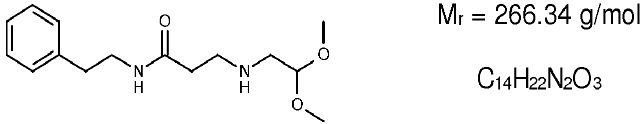

3905 mg 3-Chlor-*N*-phenethylacetamid (19.7 mmol, 1.0 eq) wurden in 50 mL Toluol gelöst, 5 Minuten gerührt und anschließend langsam mit 1.90 mL Chloracetylchlorid (24.0 mmol, 1.2 eq) versetzt, 4 Stunden unter Rückfluss erhitzt und anschließend auf 0 °C gekühlt. Der resultierende Niederschlag wurde abgesaugt, mit Toluol gewaschen und das Filtrat wurde eingeengt. Es wurden 4900 mg von **Schl32180** (93.4%) in Form eines dunkelbraunen Öls erhalten.

| | | | | |
|---|---|---|---|---|
| **¹H-NMR :** | (CDCl₃, 400 MHz), δ [ppm] = 7.33-7.15 (*m*, 5H, *CH*_{(Phenyl)}), 4.23 (t*,*³J = 5.3 Hz, 1H, NH-CH₂-*C̅H*-OMe), 3.46 *(quin,* ³J = 6.4 Hz, 2H, C-CH₂-*CH₂*-NH), 3.27 (*s*, 6H, O*-CH₃,* O*-CH₃),* 3.18 (*t,* ³J = 6.9 Hz, 2H, C(=O)-*CH₂-*NH), 2.76 (*q*, ³J = 7.1 Hz, 2H, C-C*H₂*-CH₂-NH), 2.57 (*q*, ³J = 5.3 Hz, 2H, O-CH-*CH₂-*NH), 2.19 (*sbr*,1H, CH₂-N*H*-CH₂). | | | |
| **¹³C**-**NMR :** | (CDCl₃, 100 MHz), δ [ppm] = 171.4 (C=O), 139.0 (C-CH-CH-CH), 128.8 (C-CH-CH-CH, C-CH-CH-CH), 128.6 (C-CH-CH-CH, C-CH-CH-CH), 126.5 (C-CH-CH-CH), 103.5 (NH-CH₂-CH-O), 54.1 (O-CH₃, O-CH₃), 52.3 (C(=O)-CH₂-NH), 50.9 (NH-CH₂-CH), 40.1 (C-CH₂-CH₂-NH), 35.6 (C-CH₂-CH₂-NH). | | | |
| **IR (ATR) :** | v (cm⁻¹) = 3315 (w), 3062 (w), 3026 (w), 1652 (s), 1603 (w), 1525 (m), 1497 (m), 1453 (m), 1363 (w), 1334 (w), 1248 (w), 1192 (w), 1125 (s), 1056 (s), 969 (w), 917 (w), 827 (w), 748 (m), 698 (s), 568 (m), 494 (m). | | | |
| **MS** (ESI+) : | m/z (%) = 267 (100, [M+H]⁺) | | | |
| **HRMS** (ESI+) : | m/z für C₁₄H₂₂N₂O₃ | | | |
| | ber.: | 266.1630 | | gef.: 266.1645 |
| **EA:** | ber.: | C:63.13 | H:8.33 | N:10.52 |
| | gef.: | C:62.67 | H:8.03 | N:10.27 |

### Darstellung von 3-(2,2-Dimethoxyethylamino)-N-phenethylpropanamidhydrochlorid (Sch132181):

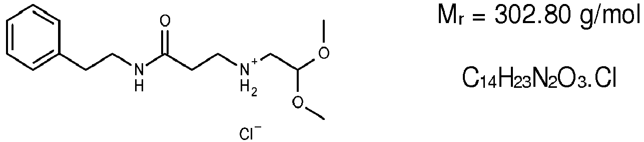

4171 mg 3-(2,2-Dimethoxyethylamino)-*N-*phenethylpropanamid (15.7 mmol, 1.0 eq) wurden in 50 mL Dichlormethan gelöst, auf 0 °C gekühlt und langsam mit 18.8 mL 1M HCl in Diethylether (18.8 mmol, 1.2 eq) versetzt. Der resultierende Niederschlag wurde abgesaugt und mit Diethylether und Cyclohexan gewaschen. Es wurden 2593 mg von **Schl32181** (54.5%) in Form eines hellbraunen Feststoffes erhalten.

| | | | | |
|---|---|---|---|---|
| **Fp.:** | 143 °C | | | |
| **¹H-NMR :** | (CDCl₃, 400 MHz), δ [ppm] = 9.00 (sbr,2H, N*H₂*+), 8.56 (*sbr,* 1H, N*H*), 7.27-7.13 (m, 5H, C*H*_{(Phenyl)}), 4.84 (*t,* ³J = 5.3 Hz, 1 H, NH-CH₂-C*H*-OMe), 3.98 (*quin,* ³J = 6.4 Hz, 2H, C-CH₂-C*H₂*-NH), 3.49 (t, ³J=6.9Hz,2H,C(=O)-C*H₂*NH), 3.40 (s, 6H, O*-*C*H₃,* O*-*C*H₃),* 3.14 *(q,* ³J=7.1Hz, 2H, C-C*H₂*-CH₂-NH), 2.84 (q, ³J = 5.3 Hz, 2H, O-CH-C*H₂-*NH). | | | |
| **¹³C**-**NMR :** | (CDCl₃, 100 MHz), δ [ppm] = 164.6 (C=O), 138.7 (C-CH-CH-CH), 128.9 (C-CH-CH-CH, C-CH-CH-CH), 128.6 (C-CH-CH-CH, C-CH-CH-CH), 126.5 (C-CH-CH-CH), 99.6 (NH-CH₂-CH-O), 54.9 (O-CH₃, O-CH₃), 48.8 (C(=O)-CH₂-NH), 48.6 (NH-CH₂-CH), 41.3 (C-CH₂-CH₂-NH), 35.4 (C-CH₂-CH₂-NH). | | | |
| **IR (ATR):** | v (cm⁻¹) = 3324 (w), 3061 (w), 3027 (w), 2993 (w), 2760 (m), 1667 (vs), 1560 (s), 1496 (m), 1445 (m), 1365 (w), 1293 (w), 1257 (m), 1191 (w), 1140 (m), 1111 (w), 1071 (vs), 1040 (s), 1009 (w), 966 (vs), 888 (m), 831 (w), 749 (m), 699 (s), 630 (w), 558 (m), 494 (m), 449 (m). | | | |
| **MS** (ESI+) : | m/z (%)=267 (100,[M-Cl]⁺) | | | |
| **HRMS** (ESI+) : | m/z für C₁₄H₂₂N₂O₃ | | | |
| | ber.: | 266.1630 | | gef.: 266.1645 |
| **EA:** | ber.: | C: 55.53 | H: 7.66 | N: 9.25 |
| | gef.: | C: 55.83 | H: 7.56 | N: 9.24 |

### Darstellung von 1,2,3,6,7,11 b-Hexahydropyrazino[2,1-a]isochinolin-4-on (Schl32182):

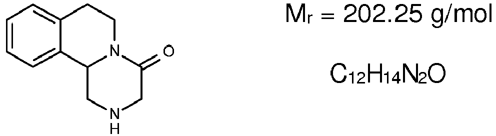

20 mL konzentrierte Schwefelsäure wurden auf 0 °C gekühlt, portionsweise mit 2453 mg 3-(2,2-Dimethoxyethylamino)-*N*-phenethylpropanamidhydrochlorid (8.1 mmol, 1.0 eq) versetzt, auf RT erwärmt und 12 Stunden gerührt. Das Reaktionsgemisch wurde vorsichtig in Eiswasser gegossen, mit 6M NaOH-Lösung auf pH=12 eingestellt und anschließend 5 Mal mit Dichlormethan (je 75 mL) extrahiert. Die vereinigten organischen Phasen wurden über MgSO₄ getrocknet, filtriert und das Lösungsmittel am Rotavapor entfernt. Säulenchromatographie an Kieselgel (DCM/ MeOH/ NEt3 90:10:0.1) ergab 486 mg von **Sch132182** (30.1 %) in Form eines orangen Feststoffes.

| | | | | |
|---|---|---|---|---|
| **DC:** | Rf=0.27 (DCM/ MeOH/ NEt390:10:0.1). | | | |
| **Fp.:** | 114 °C | | | |
| **¹H-NMR :** | (CDCl₃, 400 MHz), δ [ppm] = 7.21-7.06 (*m*, 4H, C*H*_{*(*Aromat)}), 4.83-4.74 (*m*, 1H, N-C*H*-C), 3.74-3.59 (*m*, 2H, C-CH*H*-N-C(=O)-CH*H*), 3.51-3.44 (*m*, 2H, C-CH*H*-N-C(=O)-CH*H*), 2.99-2.61 (m, 4H, C-C*H₂*-CH₂-N, N-CH-C*H*₂-NH), 2.59 (sbr,1H, N*H*). | | | |
| **¹³C**-**NMR :** | (CDCl₃, 100 MHz), δ [ppm] = 167.3 (C=O), 134.9 (C-CH), 134.1 (C-CH), 129.5 (CH_{(Aromat)}), 127.2 (CH_{(Aromat)}), 126.8 (CH_{(Aromat)}), 124.8 (CH_{(Aromat)}), 56.7 (NH-CH₂-CH), 49.9 (NH-CH₂-C(=O)), 49.5 (N-CH₂-CH₂-C), 38.9 (NH-CH₂-CH), 28.9 (C-CH₂-CH₂-N). | | | |
| **IR (ATR) :** | v (cm⁻¹) =3305 (w), 2910 (w), 1629 (vs), 1428 (m), 1424 (m), 1412 (m), 1359 (w), 1327 (w), 1293 (m), 1273 (w), 1248 (w), 1219 (w), 1190 (w), 1147 (w), 1101 (w), 1039 (w), 971 (w), 856 (w), 757 (s), 722 (vs), 668 (m), 644 (m), 593 (w), 570 (w), 522 (w), 487 (w), 461 (w), 442 (w), 410 (s). | | | |
| **MS** (ESI+) : | m/z (%)=203 (100, [M+H-Cl]⁺) | | | |
| **HRMS** (ESI+) : | m/z für C₁₂H₁₄N₂O | | | |
| | ber.: | 202.1106 | | gef.: 202.1137 |
| **EA:** | ber.: | C:71.26 | H: 6.98 | N:13.85 |
| | gef.: | C:70.89 | H: 6.86 | N:13.16 |

### Darstellung von 2-(1,3-Dioxan-2-yl)ethylthiomorpholin-4-carbodithioat (SchI32213):

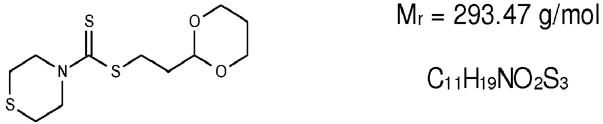

Gemäß **AAV5** wurden 0.41 mL 2-(2-Bromethyl)-1,3-dioxan (3.0 mmol, 1.0 eq), 0.36 mL Kohlenstoffdisulfid (6.0 mmol, 2.0 eq) und 0.60 mL Thiomorpholin (6.0 mmol, 2.0 eq) umgesetzt. Säulenchromatographie an Kieselgel (Cyclohexan/ EtOAc 5:1) ergab 312 mg von **Schl32213** (35.5%) in Form eines gelben Feststoffes.

| | | | | |
|---|---|---|---|---|
| **DC:** | R_{f}=0.21 (Cyclohexan/EtOAc 6:1). | | | |
| **Fp.:** | 84°C | | | |
| **¹H-NMR :** | (CDCl₃, 400 MHz), δ [ppm] = 4.60 (*t*,³J=5.2 Hz, 1H, CH₂-C*H-*O), 4.49-4.11 (*m*, 6H, N-C*H₂-*CH₂, N-C*H₂*-CH₂, CH-O-C*H*H-CH₂, CH-O-C*H*H-CH₂), 4.09-4.02 (*m*, 2H, CH-O-C*H*H-CH₂, CH-O-C*H*H-CH₂), 3.45-3.32 (*m*, 2H, S-C*H₂*-CH₂-CH), 2.77-2.61 (*m*, 4H, S-C*H₂*-CH₂-N, S-C*H₂*-CH₂-N), 2.13-1.92 (*m*, 3H, O-CH₂-C*H*H-CH₂-O, S-CH₂-C*H₂*-CH), 1.39-1.25 (*m*, 1H, O-CH₂-C*H*H-CH₂-O). | | | |
| **¹³C**-**NMR :** | (CDCl₃, 100 MHz), δ [ppm] = 196.9 (C=S), 100.2 (CH₂-CH-O), 67.0 (O-CH₂-CH₂), 56.6 (N-CH₂-CH₂-S, N-CH₂-CH₂-S), 38.2 (S-CH₂-CH₂-CH), 27.3 (S-CH₂-CH₂-N, S-CH₂-CH₂-N), 31.8 (S-CH₂-CH₂-CH), 25.8 (O-CH₂-CH₂-CH₂). | | | |
| **IR (ATR) :** | v (cm⁻¹) = 2920 (w), 2849 (w), 1442 (m), 1424 (s), 1411 (m), 1397 (m), 1378 (w), 1359 (w), 1284 (m), 1242 (m), 1220 (m), 1186 (s), 1163 (w), 1143 (vs), 1122 (s), 1071 (s), 1039 (s), 1020 (s), 1000 (s), 952 (w), 946 (w), 936 (vs), 922 (vs), 895 (m), 875 (s), 846 (s), 816 (w), 803 (w), 763 (w), 642 (w), 495 (w), 484 (w), 473 (w), 418 (m), 401 (m). | | | |
| **MS** (ESI+) : | m/z (%)=294 (100, [M+H]⁺) | | | |
| **HRMS** (ESI+) : | m/z für C₁₁H₁₉NO₂S₃ | | | |
| | ber.: | 293.0578 | | gef.: 293.0591 |
| **EA:** | ber.: | C:45.02 | H: 6.53 | N: 4.77 |
| | gef.: | C:45.17 | H: 6.62 | N:4.82 |

### Darstellung von Benzyl-4-oxo-3,6,7,11 b-tetrahydro-1H-pyrazino[2,1-a]isochinolin-2-carbodithioat (Schl32223):

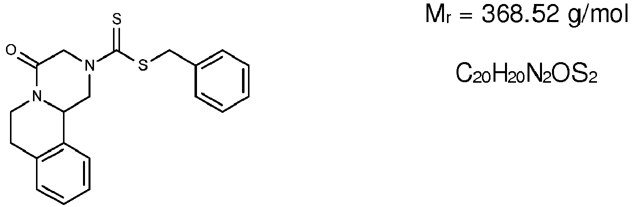

Gemäß **AAV8** wurden 506 mg 1,2,3,6,7,11*b*-Hexahydropyrazino[2,1-*a*]isochinolin-4-on **(Schl32182,** 2.5 mmol, 1.0 eq), 0.30 mL Kohlenstoffdisulfid (5.0 mmol, 2.0 eq) und 0.30 mL Benzylbromid (2.5 mmol, 1.0 eq) umgesetzt. Säulenchromatographie an Kieselgel (Cyclohexan/EtOAc 4:1) ergab 770 mg von **Schl32223** (83.6%) in Form eines gelbbraunen Feststoffes.

| | | | | |
|---|---|---|---|---|
| **DC:** | R_{f}=0.14 (Cyclohexan/ EtOAc 6:1). | | | |
| **Fp.:** | 135 °C | | | |
| **¹H-NMR :** | (DMSO-D₆, 400 MHz), δ [ppm] = 7.42-7.36 (*m*, 2H, C*H*_{(Benzyl)}), 7.34-7.16 *(m,* 7H, C*H*_{*(*Aromat)}, C*H*_{(Benzyl)}), 5.51-5.42 (*m*, 1H, C-C*H*-N), 4.75-4.63 (*m*,1H, N-CH*H-*C(=O)), 4.56 (*s*, 2H, S-C*H*₂-C), 4.54-4.40 (*m*, 2H, N-CH*H-*C(=O)), 3.69-3.55 (*m*, 1H, C-CH₂-CH*H*-N), 3.50-3.39 (*m*, 1H, C-CH₂-CH*H*-N), 2.91-2.71 (*m*, 4H, C-C*H₂*-CH₂-N, N-C*H₂*-CH). | | | |
| **¹³C**-**NMR :** | (DMSO-D₆, 100 MHz), δ [ppm] = 195.3 (C=S), 163.2 (C=O), 136.5 (Cq _{(Aromat)}), 135.9 (Cq _{(Aromat)}), 132.3 (Cq _{(Aromat)}), 129.8 (Ct _{(Aromat)}), 129.6 (Ct _{(Aromat)}), 129.0 (Ct _{(Aromat)}), 128.0 (Ct _{(Aromat)}), 127.9 (Ct (Aromat)), 127.3 (Ct (Aromat)), 126.6 (Ct (Aromat)), 54.9 (N-CH₂-C(=O)), 53.5 (C-CH-N), 41.4 (S-CH₂-C), 40.9 (N-CH-CH₂-N), 38.7 (C-CH₂-CH₂-N), 28.6 (C-CH₂-CH₂-N). | | | |
| **IR (ATR):** | v (cm⁻¹) = 3023 (w), 2964 (w), 2896 (w), 1645 (vs), 1492 (w), 1444 (w), 1423 (w), 1409 (vs), 1362 (w), 1351 (w), 1327 (w), 1308 (w), 1289 (s), 1252 (w), 1242 (w), 1215 (m), 1185 (w), 1155 (w), 1118 (w), 1078 (w), 1033 (w), 1017 (m), 978 (w), 966 (w), 923 (w), 893 (w), 854 (w), 806 (w), 775 (w), 763 (s), 738 (w), 705 (s), 678 (w), 637 (w), 597 (w), 581 (w), 564 (w), 513 (w), 494 (w), 474 (w), 459 (w), 446 (w), 405 (w). | | | |
| **MS** (ESI+) : | m/z (%) = 369 (100,[M+H]⁺) | | | |
| **HRMS** (ESI+) : | m/z für C₂₀H₂₀N₂OS₂ | | | |
| | ber.: | 368.1017 | | gef.: 368.1008 |
| **EA:** | ber.: | C:65.18 | H: 5.47 | N:7.60 |
| | gef.: | C:65.00 | H:5.32 | N:7.37 |

### Darstellung von Benzyl-(2R)-2-[(R)-[2,8-bis(trifluormethyl)-4-chinolyl]-hydroxy-methyl]piperidin-1-carbodithioat (SchI32228):

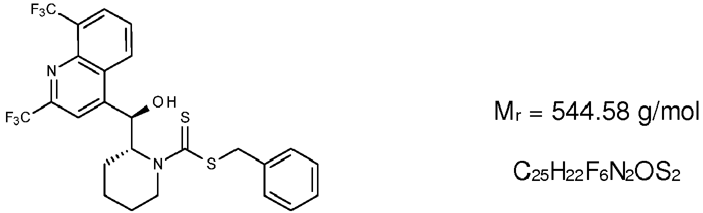

Gemäß **AAV7** wurden 500 mg Mefloquinhydrochlorid (1.2 mmol, 1.0 eq), 561 mg Kaliumphosphat (2.6 mmol, 1.2 eq), 0.15 mL Kohlenstoffdisulfid (2.4 mmol, 2.0 eq) und 0.14 mL Benzylbromid (1.2 mmol, 1.0 eq) umgesetzt. Säulenchromatographie an Kieselgel (Cyclohexan/EtOAc 5:1) ergab 210 mg von **Schl32228** (32.2%) in Form eines farblosen Feststoffes.

| | | | | |
|---|---|---|---|---|
| **DC**: | R_{f}=0.16 (Cyclohexan/ EtOAc 5:1). | | | |
| **Fp.:** | 210°C | | | |
| **¹H-NMR :** | (DMSO-D₆, 400 MHz), δ [ppm] = 9.16 (d, ³J=8.7 Hz, 1H, C(-CF₃)-C*H*-CH-CH-C), 8.32 (*d,* ³J=7.3 Hz, 1H, C(-CF₃)-CH-CH-C*H*-C), 8.12 (s, 1H, C(-CF₃)-C*H*-C), 7.92 (*t*, ³J=7.8 Hz, 1H, C(-CF₃)-CH-C*H*-CH-C), 7.28-7.14 (m, 5H, C*H*_{(Benzyl)}), 6.49 (sbr,1H, O*H*), 6.03-5.96 (m, 1H, C*H*-OH), 5.81-5.72 (m, 1H, N-C*H*), 4.33 (s, 2H, S-C*H₂*-C), 4.06-3.91 (m, 1H, N-CH*H*-CH₂), 3.85-3.69 (*m*, 1H, N-CH*H*-CH₂), 2.09-1.79 (m, 3H, CH-CH*H*-CH*H*-CH*H*-CH₂-N), 1.58-1.22 (m, 3H, CH-CH*H*-CH*H*-CH*H*-CH₂-N). | | | |
| **¹³C**-**NMR :** | (DMSO-D₆, 100 MHz), δ [ppm] = 196.4 (C=S), 153.2 (Cq _{(Aromat)}), 153.0 (Cq _{(Aromat)}), 143.4 (Cq _{(Aromat)}), 136.5 (Cq _{(Benzyl)}), 130.7 (Ct _{(Benzyl)}), 130.2 (Ct _{(Benzyl)}), 129.5 (Ct _{(Aromat)}), 129.2 (Cq_{(Aromat)}), 128.9 (Ct_{(Aromat)}), 128.1 (Ct_{(Benzyl)}), 127.8 (Ct _{(Aromat)}), 126.8 (*q*,¹J_{C,F} = 225.4 Hz, CF₃), 124.4 (Cq _{(Aromat)}), 121.7 (q, ¹J_{C},_{F} = 251.4 Hz, CF₃), 116.5 (Ct (Aromat)), 68.5 (CH-OH), 63.5 (N-CH), 47.7 (N-CH₂-CH₂-CH₂-CH₂-CH), 40.0 (S-CH₂-C), 23.4 (N-CH₂-CH₂-CH₂-CH₂-CH), 18.6 (N-CH₂-CH₂-CH₂-CH₂-CH). | | | |
| **IR (ATR) :** | v (cm⁻¹) = 3357 (w), 2964 (w), 2870 (w), 1598 (w), 1515 (w), 1497 (w), 1486 (m), 1422 (w), 1308 (vs), 1248 (w), 1213 (s), 1178 (s), 1145 (vs), 1106 (vs), 1071 (s), 1043 (m), 984 (w), 956 (s), 928 (m), 906 (m), 863 (m), 805 (w), 768 (s), 736 (m), 717 (s), 676 (m), 613 (w), 570 (w), 523 (w), 497 (s), 485 (m), 429 (w). | | | |
| **MS** (ESI+) : | m/z (%)=545 (100, [M+H]⁺) | | | |
| **HRMS** (ESI+) : | m/z für C₂₅H₂₂F₆N₂OS₂ | | | |
| | ber.: | 544.1078 | | gef.: 544.1086 |
| **EA:** | ber.: | C:55.14 | H:4.07 | N:5.14 |
| | gef.: | C:55.01 | H:4.02 | N:5.04 |

Die biologischen Testungen der vorstehend beschriebenen Verbindungen wurden beispielhaft für parasitäre Helminthen an *Schistosoma mansoni* (Liberianischer Stamm) durchgeführt. Sie belegen beispielhaft die gute Wirksamkeit der erfindungsgemäßen Verbindungen.

Basierend auf den vorstehend offenbarten Ausführungsbeispielen kann der Gegenstand der Erfindung wie folgt definiert werden:
Bei der Erfindung handelt es sich um Verbindungen, die dadurch gekennzeichnet sind, daß sie in ihrem chemischen Aufbau mindestens eine Teilstruktur gemäß der allgemeinen Formel I aufweisen.

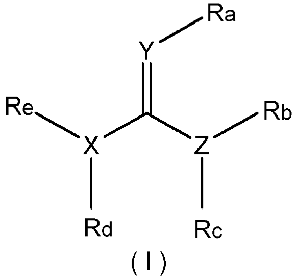

Bei X, Y und Z handelt es sich um Atome der chemischen Elemente Stickstoff und/oder Sauerstoff und/oder Schwefel und/oder Selen, die unabhängig voneinander ausgewählt sind. Die Reste Rₐ und R_{b} sind ausgewählt aus der Liste umfassend "kein Rest", H, Alkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, wobei diese und alle weiteren hier offenbarten Listen nicht einschränkend zu verstehen sind. Der Rest R_{c} ist entweder a) ausgewählt aus der Liste umfassend "kein Rest", H, Alkyl, Aryl, Heteroaryl, Acyl, SOₙR_{f}, SOₙH, POₘR_{f}, POₘH, SO_{z}N(R_{f})₂, POm(NRf)x, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl oder es handelt sich b) bei R_{c} um mindestens einen mindestens ein Atom umfassenden Rest, der mit Z über eine Doppelbindung verknüpft ist.

R_{d} und Rₑ sind entweder i) unabhängig voneinander ausgewählt aus der Liste umfassend "kein Rest", H, Alkyl, Cycloalkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl oder ii) R_{d} und Rₑ sind zweibindige Reste, die miteinander verbunden sind und dadurch zusammen mit Atom X eine cyclische Teilstruktur bilden oder iii) R_{d} ist ausgewählt aus der Liste umfassend "kein Rest", H, Alkyl, Cycloalkyl, Aryl, Heteroaryl, Acyl, SOₙR_{f}, SOₙH, POₘR_{f}, POₘH, SO_{z}N(R_{f})₂; POₘ(NR_{f})ₓ, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl. Im Falle iii) ist Rₑ dann entweder Teil einer cyclischen Teilstruktur oder es handelt sich bei Rₑ um ein Heteroatom, das ausgewählt ist aus der Liste umfassend die chemischen Elemente Stickstoff, Sauerstoff, Schwefel, und dieses Heteroatom ist dann entweder Bestandteil einer cyclischen Teilstruktur, oder es trägt zwei Reste, die unabhängig voneinander ausgewählt sind aus der Liste umfassend "kein Rest", H, Alkyl, Cycloalkyl, Aryl, Heteroaryl, Acyl, SOₙR_{f}, SOₙH, POₘR_{f}, POₘH, SO_{z}N(R_{f})₂; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl.

R_{f} ist ausgewählt aus der Liste umfassend H, Alkyl, Aryl, Heteroaryl, substituiertes Aryl, substituiertes Heteroaryl, Alkylaryl, Alkylheteroaryl. Die Substituenten der substituierten Aryl- und Heteroarylreste sind ausgewählt aus der Liste umfassend F, Cl, Br, OH, Phenyl, NH₂, NO₂, OR_{f}, O-COR_{f}, COR_{f}, N(R_{f})₂, NR_{f}COR_{f}, O-CSR_{f}, CSR_{f}, NR_{f}CSR_{f}, O-CSeR_{f}, CSeR_{f}, NR_{f}CSeR_{f}, O-CN(R_{f})R_{f}, CN(R_{f})R_{f}, NR_{f}CN(R_{f})R_{f}, N(R_{f})SO₂R_{f}, N(R_{f})SO₂N(R_{f})₂, SSR_{f}, SR_{f}, SOₓR_{f}, SOₙN(R_{f})₂, Nitril, Amidin, Guanidin, COOR_{f}, CON(R_{f})₂, CON(R_{f})N(R_{f})₂, COSR_{f}, CSOR_{f}, CSSR_{f}, NR_{f}COR_{f}, N(R_{f})N(R_{f})₂.

Der Formelindex x kann Werte von 1 bis 2 annehmen, der Formelindex y kann ebenfalls Werte von 1 bis 2 annehmen, der Formelindex z kann Werte von 0 bis 1 annehmen, der Formelindex n kann Werte von 0 bis 4 annehmen und der Formelindex m kann Werte von 1 bis 4 annehmen.

Eine vorteilhafte Ausgestaltung der Erfindung umfasst Verbindungen, die in ihrem chemischen Aufbau mindestens eine Teilstruktur gemäß der allgemeinen Formel II aufweisen.

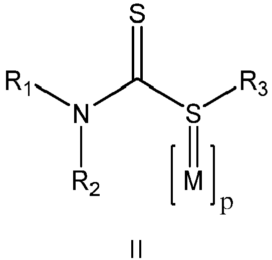

Dabei sind R₁ und R₂ entweder a) unabhängig voneinander ausgewählt aus der Liste umfassend H, Alkyl, Cycloalkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, oder b) es handelt sich bei R₁ und R₂ um mindestens zweiwertige Reste, die über mindestens eine chemische Bindung miteinander verknüpft sind, so daß die Reste R₁ und R₂ zusammen mit dem Stickstoffatom einen Heterozyklus bilden.

R₃ ist ausgewählt aus der Liste umfassend Alkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl. M ist ausgewählt aus der Liste umfassend O, NH, NR_{f}. R_{f} ist ausgewählt aus der Liste umfassend H, Alkyl, Aryl, Heteroaryl, substituiertes Aryl, substituiertes Heteroaryl, Alkylaryl, Alkylheteroaryl.

Der Formelindex x kann Werte von 1 bis 2 annehmen, der Formelindex y kann ebenfalls Werte von 1 bis 2 annehmen, der Formelindex z kann Werte von 0 bis 1 annehmen , der Formelindex n kann Werte von 0 bis 4 annehmen, der Formelindex m kann Werte von 1 bis 4 annehmen und der Formelindex p kann Werte von 0 bis 2 annehmen.

Eine weitere vorteilhafte Ausgestaltung der Erfindung umfasst Verbindungen, die in ihrem chemischen Aufbau mindestens eine Teilstruktur gemäß der allgemeinen Formel III aufweisen.

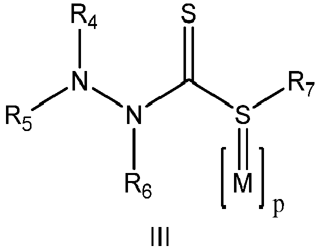

Dabei sind R₄ und R₅ entweder a) unabhängig voneinander ausgewählt aus der Liste umfassend H, Alkyl, Cycloalkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, oder b) es handelt sich bei R₄ und R₅ um mindestens zweiwertige Reste, die über mindestens eine chemische Bindung miteinander verknüpft sind, so daß die Reste R₄ und R₅ zusammen mit dem Stickstoffatom einen Heterozyklus bilden.
R₆ und R₇ sind ausgewählt aus der Liste umfassend H, Alkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl. M ist ausgewählt aus der Liste umfassend O,NH, NR_{f} und R_{f} ist ausgewählt aus der Liste umfassend H, Alkyl, Aryl, Heteroaryl, substituiertes Aryl, substituiertes Heteroaryl, Alkylaryl, Alkylheteroaryl.

Der Formelindex x kann Werte von 1 bis 2 annehmen, der Formelindex y kann ebenfalls Werte von 1 bis 2 annehmen, der Formelindex z kann Werte von 0 bis 1 annehmen, der Formelindex n kann Werte von 0 bis 4 annehmen, der Formelindex m kann Werte von 1 bis 4 annehmen und der Formelindex p kann Werte von 0 bis 2 annehmen.

Eine weitere vorteilhafte Ausgestaltung der Erfindung umfasst Verbindungen, die in ihrem chemischen Aufbau mindestens eine Teilstruktur gemäß der allgemeinen Formel IV aufweisen.

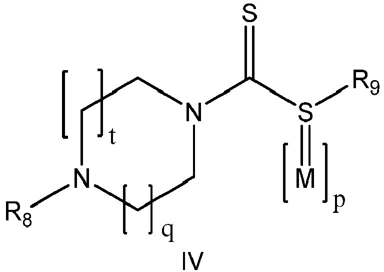

Dabei sind R₈ und R₉ unabhängig voneinander ausgewählt aus der Liste umfassend H, Alkyl, Cycloalkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl. M ist ausgewählt aus der Liste umfassend O,NH, NR, und R_{f} ist ausgewählt aus der Liste umfassend H, Alkyl, Aryl, Heteroaryl, substituiertes Aryl, substituiertes Heteroaryl, Alkylaryl, Alkylheteroaryl.
Der Formelindex t kann Werte von 0 bis 4 annehmen, der Formelindex x kann Werte von 1 bis 2 annehmen, der Formelindex y kann Werte von 1 bis 2 annehmen, der Formelindex z kann Werte von 0 bis 1 annehmen, der Formelindex n kann Werte von 0 bis 4 annehmen, der Formelindex m kann Werte von 1 bis 4 annehmen, der Formelindex p kann Werte von 0 bis 2 annehmen und der Formelindex q kann Werte von 0 bis 4 annehmen.

Eine weitere vorteilhafte Ausgestaltung der Erfindung umfasst Verbindungen, die in ihrem chemischen Aufbau mindestens eine Teilstruktur gemäß der allgemeinen Formel V aufweisen.

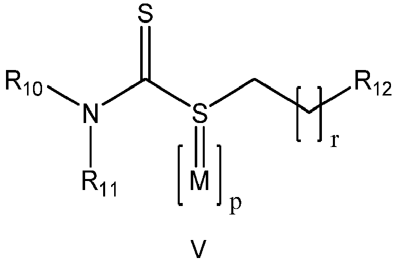

Dabei sind R₁₀ und R₁₁ entweder a) unabhängig voneinander ausgewählt aus der Liste umfassend H, Alkyl, Cycloalkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, oder b) es handelt sich bei R₁₀ und R₁₁ um mindestens zweiwertige Reste, die über mindestens eine chemische Bindung miteinander verknüpft sind, so daß die Reste R₁₀ und R₁₁ zusammen mit dem Stickstoffatom einen Heterozyklus bilden.
R₁₂ ist ausgewählt aus der Liste umfassend Alkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, NHR_{f}, NR_{f}R_{g}, COR_{f}, COOR_{f}, CONHR_{f}, CONR_{f}R_{g}, COR_{f}, OCOR_{f}, NHCOR_{f}, NR_{f}COR_{g}, CSR_{f}, CSOR_{f}, CSNHR_{f}, CSNR_{f}R_{g}, CSR_{f}, OCSR_{f}, NR_{f}CSR_{f}, NR_{f}CSR_{g}, COSR_{f}, COR_{f}, CSSR_{f}, CSNR_{f}N(R_{f})₂, CONR_{f}N(R_{f})₂, NR_{f}N(R_{f})₂ und M ist ausgewählt aus der Liste umfassend O, NH, NR_{f}. R_{f} und R_{g} sind unabhängig voneinander ausgewählt aus der Liste umfassend H, Alkyl, Aryl, Heteroaryl, substituiertes Aryl, substituiertes Heteroaryl, Alkylaryl, Alkylheteroaryl.
Der Formelindex x kann Werte von 1 bis 2 annehmen, der Formelindex y kann ebenfalls Werte von 1 bis 2 annehmen, der Formelindex z kann Werte von 0 bis 1 annehmen, der Formelindex n kann Werte von 0 bis 4 annehmen, der Formelindex m kann Werte von 1 bis 4 annehmen, der Formelindex p kann Werte von 0 bis 2 annehmen und der Formelindex r kann Werte von 0 bis 4 annehmen.

Eine weitere vorteilhafte Ausgestaltung der Erfindung umfasst Verbindungen, die in ihrem chemischen Aufbau mindestens eine Teilstruktur gemäß der allgemeinen Formel VI aufweisen.

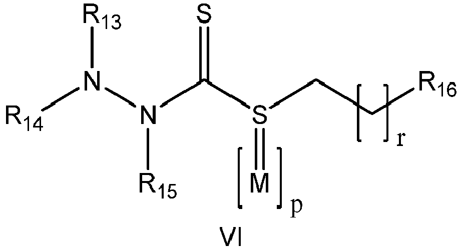

Dabei sind R₁₃ und R₁₄ entweder a) unabhängig voneinander ausgewählt aus der Liste umfassend H, Alkyl, Cycloalkyl, Aryl, Heteroaryl, Acyl, SOₙR_{f}, SOₙH, POₘR_{f}, POₘH, SOzN(Rf)2; POₘ(NR_{f})ₓ, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, oder b) es handelt sich bei R₁₃ und R₁₄ um mindestens zweiwertige Reste, die über mindestens eine chemische Bindung miteinander verknüpft sind, so daß die Reste R₁₃ und R₁₄ zusammen mit dem Stickstoffatom einen Heterozyklus bilden,
R₁₅ und R₁₆ sind ausgewählt sind aus der Liste umfassend Alkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, NHR_{f}, NR_{f}R_{g}, COR_{f}, COOR_{f}, CONHR_{f}, CONR_{f}R_{g}, COR_{f}, OCOR_{f}, NHCOR_{f}, NR_{f}COR_{g}, CSR_{f}, CSOR_{f}, CSNHR_{f}, CSNR_{f}R_{g}, CSR_{f}, OCSR_{f}, NR_{f}CSR_{f}, NR_{f}CSR_{g}, COSR_{f}, COR_{f}, CSSR_{f}, CSNR_{f}N(R_{f})₂, CONR_{f}N(R_{f})₂, NR_{f}N(R_{f})₂ und M ist ausgewählt aus der Liste umfassend O,NH, NR_{f}.
R_{f} und R_{g} sind unabhängig voneinander ausgewählt aus der Liste umfassend H, Alkyl, Aryl, Heteroaryl, substituiertes Aryl, substituiertes Heteroaryl, Alkylaryl, Alkylheteroaryl. Der Formelindex x kann Werte von 1 bis 2 annehmen, der Formelindex y kann Werte von 1 bis 2 annehmen, der Formelindex z kann Werte von 0 bis 1 annehmen, der Formelindex n kann Werte von 0 bis 4 annehmen, der Formelindex m kann Werte von 1 bis 4 annehmen, der Formelindex p kann Werte von 0 bis 2 annehmen und der Formelindex r kann Werte von 0 bis 4 annehmen.

Eine weitere vorteilhafte Ausgestaltung der Erfindung umfasst Verbindungen, die in ihrem chemischen Aufbau mindestens eine Teilstruktur gemäß der allgemeinen Formel VII aufweisen.

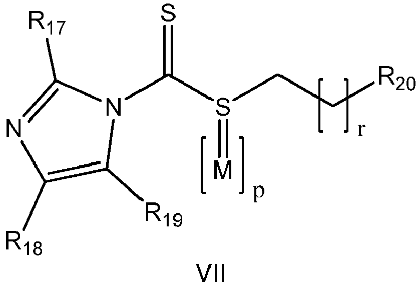

Dabei sind R₁₇ und R₁₈ unabhängig voneinander ausgewählt aus der Liste umfassend H, Alkyl, Cycloalkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl. R₁₉ und R₂₀ sind ausgewählt aus der Liste umfassend H, Alkyl, Aryl, Heteroaryl, Acyl, SOₙR_{f}, SOₙH, POₘR_{f}, POₘH, SO_{z}N(R_{f})₂; POₘ(NR_{f})ₓ, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, NHR_{f}, NR_{f}R_{g}, COR_{f}, COOR_{f}, CONHR_{f}, CONR_{f}R_{g}, COR_{f}, OCOR_{f}, NHCOR_{f}, NR_{f}COR_{g}, CSR_{f}, CSOR_{f}, CSNHR_{f}, CSNR_{f}R_{g}, CSR_{f}, OCSR_{f}, NR_{f}CSR_{f}, NR_{f}CSR_{g}, COSR_{f}, COR_{f}, CSSR_{f}, CSNR_{f}N(R_{f})₂, CONR_{f}N(R_{f})₂, NR_{f}N(R_{f})₂.
M ist ausgewählt aus der Liste umfassend O,NH, NR_{f} und R_{f} und R_{g} sind unabhängig voneinander ausgewählt aus der Liste umfassend H, Alkyl, Aryl, Heteroaryl, substituiertes Aryl, substituiertes Heteroaryl, Alkylaryl, Alkylheteroaryl.
Der Formelindex x kann Werte von 1 bis 2 annehmen, der Formelindex y kann ebenfalls Werte von 1 bis 2 annehmen, der Formelindex z kann Werte von 0 bis 1 annehmen, der Formelindex n kann Werte von 0 bis 4 annehmen, der Formelindex m kann Werte von 1 bis 4 annehmen, der Formelindex p kann Werte von 0 bis 2 annehmen und der Formelindex r kann Werte von 0 bis 4 annehmen.

Eine weitere vorteilhafte Ausgestaltung der Erfindung umfasst Verbindungen, die in ihrem chemischen Aufbau mindestens eine Teilstruktur gemäß der allgemeinen Formel VIII aufweisen.

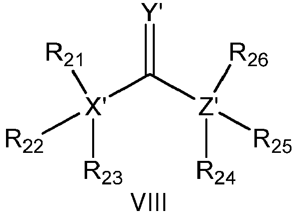

Dabei handelt es sich bei Y' entweder um ein Sauerstoffatom oder ein Schwefelatom oder um ein Selen-Atom. Bei X' und Z' handelt es sich unabhängig voneinander jeweils um ein Atom der chemischen Elemente Stickstoff, Sauerstoff, Kohlenstoff, Schwefel.
Weiterhin sind bei dieser Ausgestaltung der Erfindung entweder a) R₂₁, R₂₂, R₂₃, R₂₄, R₂₅, R₂₆ unabhängig voneinander ausgewählt aus der Liste umfassend "kein Rest", H, OH, Alkyl, Aryl, Heteroaryl, substituiertes Alkyl, substituiertes Aryl, substituiertes Heteroaryl, Alkylaryl, Alkylheteroaryl, wobei die Substituenten der substituierten Alkyl-, Aryl- und Heteroaryl-Reste ausgewählt sind aus der Liste umfassend F, Cl, Br, OH, Phenyl, NH₂, NO₂, OR_{f}, O-COR_{f}, COR_{f}, N(R_{f})₂, NR_{f}COR_{f}, O-CSR_{f}, CSR_{f}, NR_{f}CSR_{f}, O-CSeR_{f}, CSeR_{f}, NR_{f}CSeR_{f}, O-CN(R_{f})R_{f}, CN(R_{f})R_{f}, NR_{f}CN(R_{f})R_{f}, N(R_{f})SO₂R_{f}, N(R_{f})SO₂N(R_{f})₂, SSR_{f}, SR_{f}, SOₓR_{f}, SOₙN(R_{f})₂, Nitril, Amidin, Guanidin, COOR_{f}, CON(R_{f})₂, CON(R_{f})N(R_{f})₂, COSR_{f}, CSOR_{f}, CSSR_{f}, NR_{f}COR_{f}, N(R_{f})N(R_{f})₂, oder b) mindestens zwei der an X' und/oder Z' hängenden Reste R₂₁ bis R₂₆ bilden zusammen mit X' und/oder Z' eine gesättigte oder ungesättigte cyclische oder heterocyclische Ringstruktur, und die nicht in diesen mindestens einen Cyclus eingebundenen verbliebenen Reste R₂₁ oder R₂₂ oder R₂₃ oder R₂₄ oder R₂₅ oder R₂₆ sind unabhängig voneinander ausgewählt aus der Liste umfassend "kein Rest", H, OH, Alkyl, Aryl, Heteroaryl, substituiertes Alkyl, substituiertes Aryl, substituiertes Heteroaryl, Alkylaryl, Alkylheteroaryl, wobei die Substituenten der substituierten Alkyl-, Aryl- und Heteroaryl-Reste ausgewählt sind aus der Liste umfassend F, Cl, Br, OH, Phenyl, NH₂, NO₂, OR_{f}, O-COR_{f}, COR_{f}, N(R_{f})₂, NR_{f}COR_{f}, O-CSR_{f}, CSR_{f}, NR_{f}CSR_{f}, O-CSeR_{f}, CSeR_{f}, NR_{f}CSeR_{f}, O-CN(R_{f})R_{f}, CN(R_{f})R_{f}, NR_{f}CN(R_{f})R_{f}, N(R_{f})SO₂R_{f}, N(R_{f})SO₂N(R_{f})₂, SSR_{f}, SR_{f}, SOₓR_{f}, SOₙN(R_{f})₂, Nitril, Amidin, Guanidin, COOR_{f}, CON(R_{f})₂, CON(R_{f})N(R_{f})₂, COSR_{f}, CSOR_{f}, CSSR_{f}, NR_{f}COR_{f}, N(R_{f})N(R_{f})₂.
Weiterhin umfasst die Erfindung Verbindungen gemäß der vorstehend beschriebenen Ausgestaltungen, die als Ion in mindestens einfach protonierter Form oder in mindestens einfach deprotonierter Form vorliegen, jeweils in Kombination mit einem beliebigen Gegenion. Das heißt, die vorstehend beschriebenen erfindungsgemäßen Verbindungen fallen auch in Form von Salzen in den Umfang der Erfindung.

Weiterhin umfasst die Erfindung Verbindungen gemäß einer beliebigen der vorstehend beschriebenen Ausgestaltungen, dadurch gekennzeichnet, daß sie in dimerer Form vorliegen, d.h. erfindungsgemäße Verbindungen der vorstehend beschriebenen Art, die über mindestens eine chemische Bindung miteinander verknüpft sind, sind ebenfalls erfindungsgemäß.

Die Erfindung beinhaltet weiterhin ein Verfahren zur Herstellung von Carbamodithioat-Derivaten, umfassend die Umsetzung von Aminen mit Kohlenstoffdisulfid und Alkyl(aryl)halogeniden in einem Lösungsmittel enthaltend mindestens eines der Lösungsmittel aus der Liste umfassend Aceton, Acetonitril, Ethanol, Tetrahydrofuran, das dadurch gekennzeichnet ist, dass a) entweder auf die Temperierung der Reaktionsmischung verzichtet wird, oder b) auf den Zusatz einer weiteren Base zusätzlich zu dem umzusetzenden Amin verzichtet wird.

Dieses erfindungsgemäße Verfahren bewirkt somit eine deutliche Reduzierung der Herstellungskosten der erfindungsgemäßen Verbindungen durch Reduktion des Energieaufwandes sowie des Materialeinsatzes.

Weiterhin umfasst die Erfindung ein medizinisches Mittel enthaltend mindestens eine der vorstehend offenbarten erfiundungsgemäßen Verbindungen zur Behandlung von Menschen und/oder Tieren mit Erkrankungen, die durch parasitäreHelminthen verursacht wurden.

Weiterhin umfasst die Erfindung ein medizinisches Mittel enthaltend mindestens eine der vorstehend offenbarten erfindungsgemäßen Verbindungen, das dadurch gekennzeichnet ist, dass es zusätzlich mindestens einen pharmakologisch verträglichen Träger, Hilfsstoff und/oder Lösungsmittel umfasst.

Weiterhin umfasst die Erfindung ein medizinisches Mittel wie vorstehend beschrieben, das mindestens eine der vorstehend offenbarten erfindungsgemäßen Verbindungen enthält und dadurch gekennzeichnet ist, dass es als Pour-on Formulierung vorliegt.

Weiterhin umfasst die Erfindung die Verwendung einer oder mehrerer der vorstehend offenbarten erfindungsgemäßen Verbindungen zur Behandlung von Menschen und/oder Tieren mit Erkrankungen, die durch parasitäre Helminthen verursacht wurden.

### Abbildungslegenden

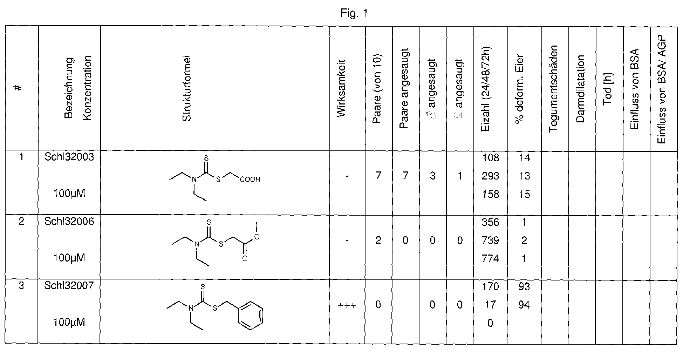

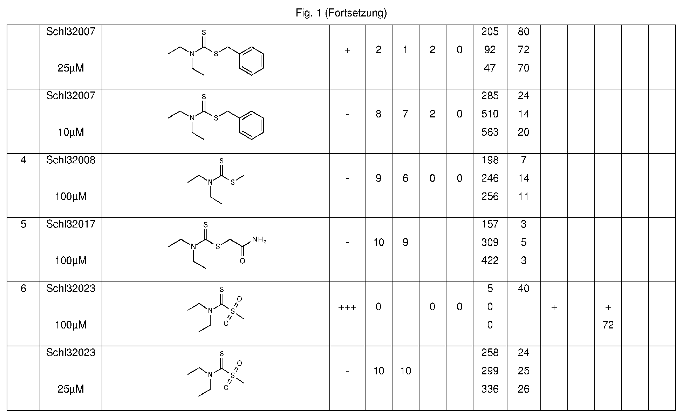

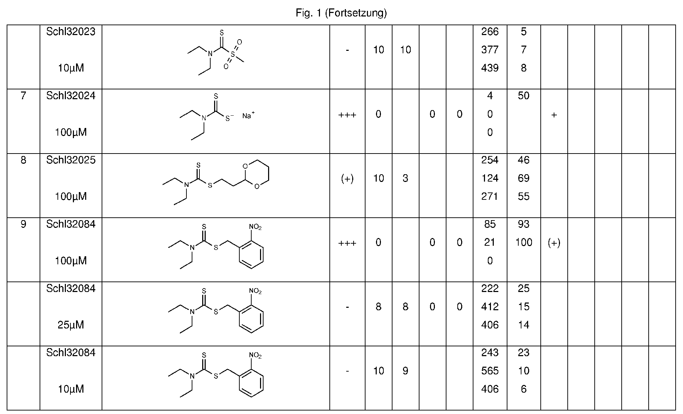

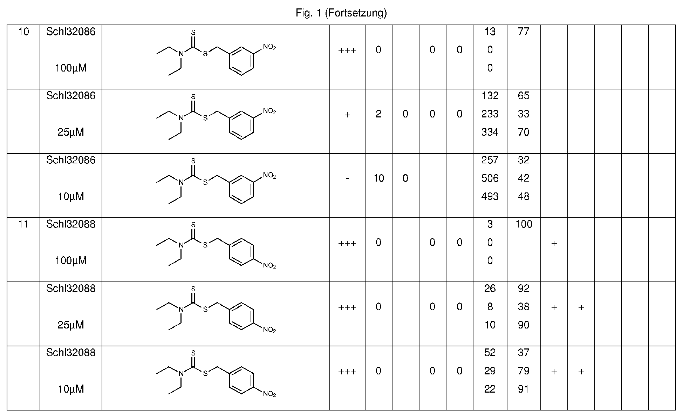

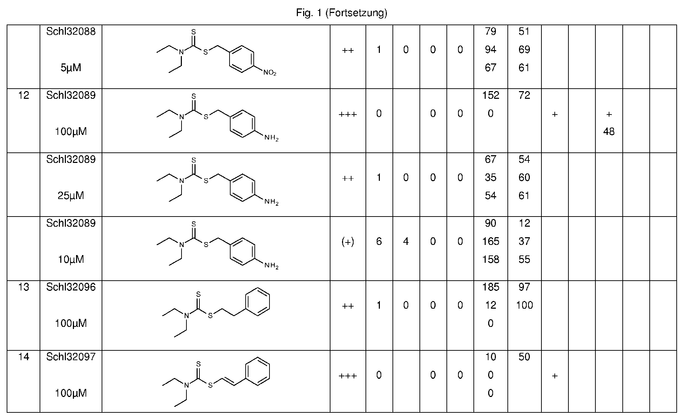

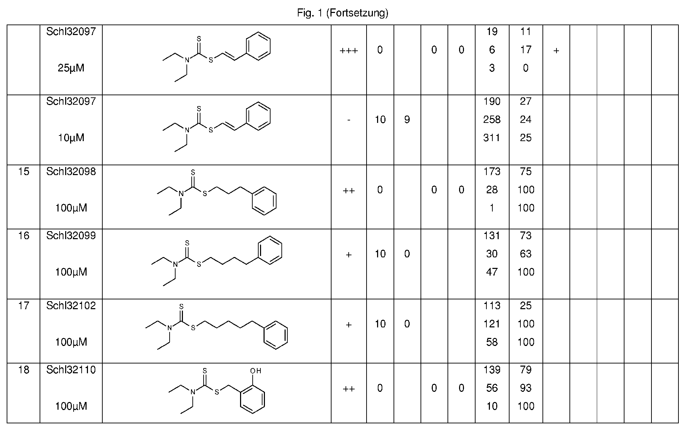

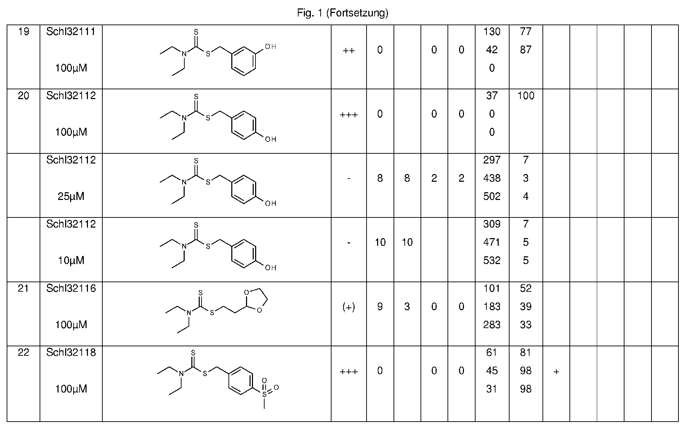

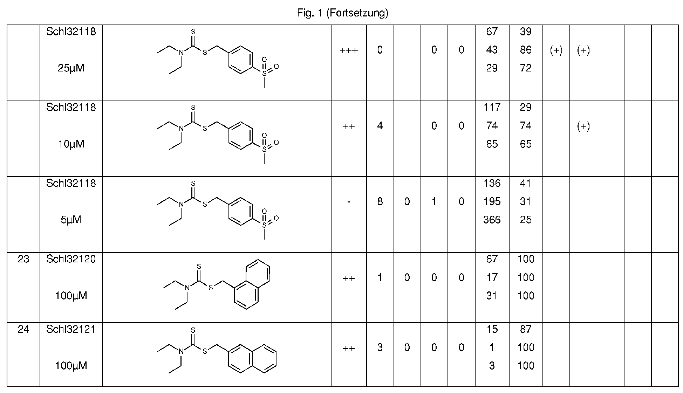

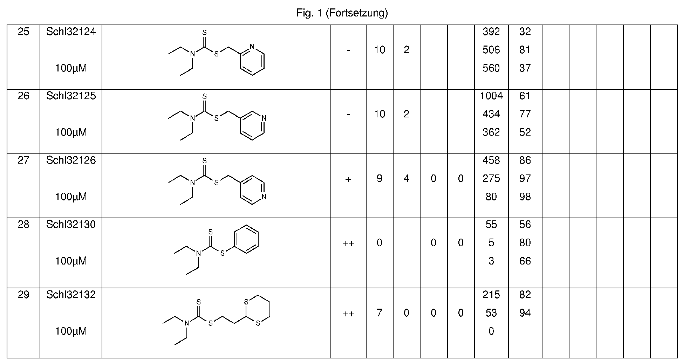

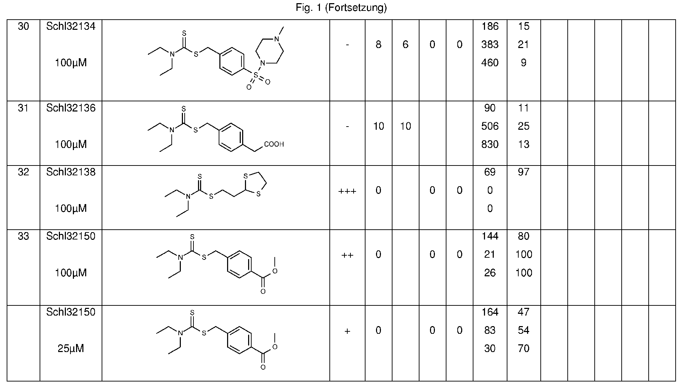

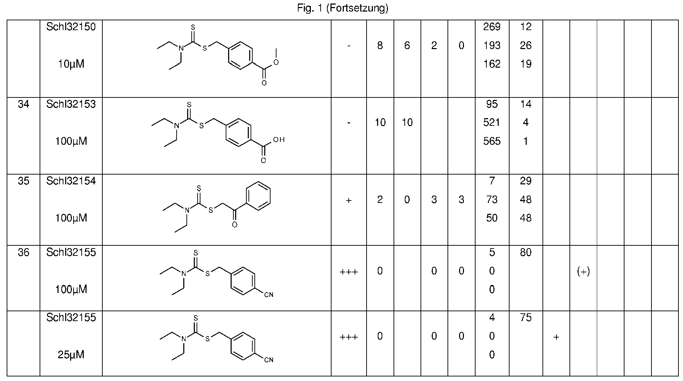

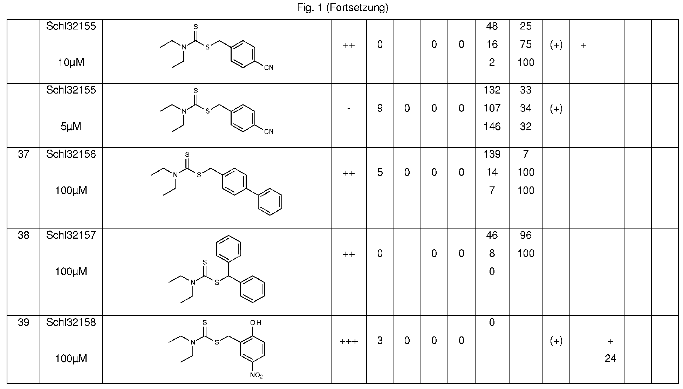

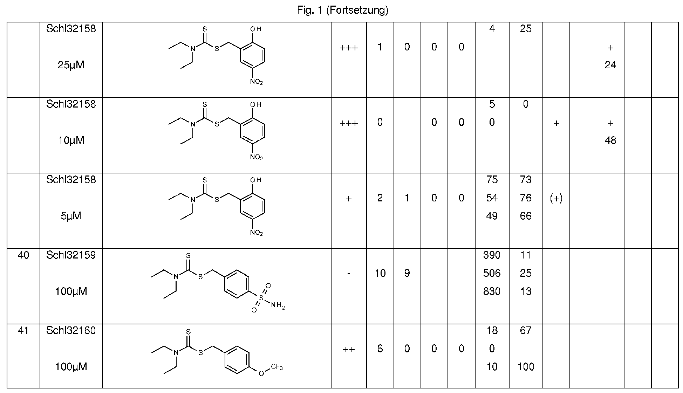

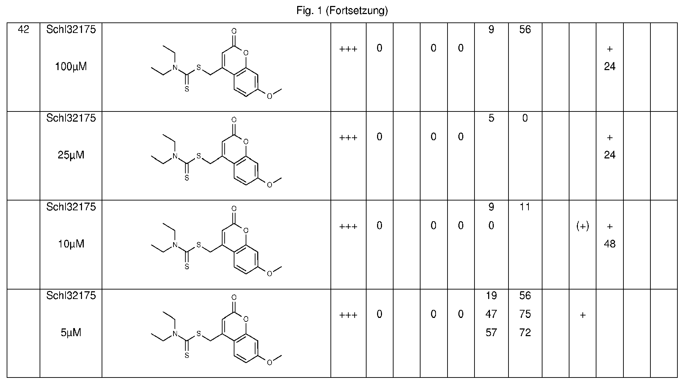

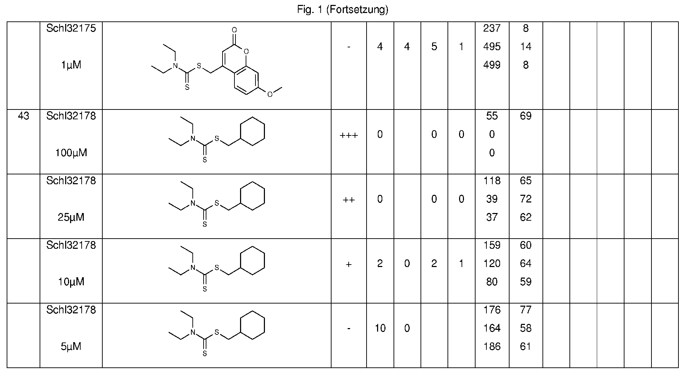

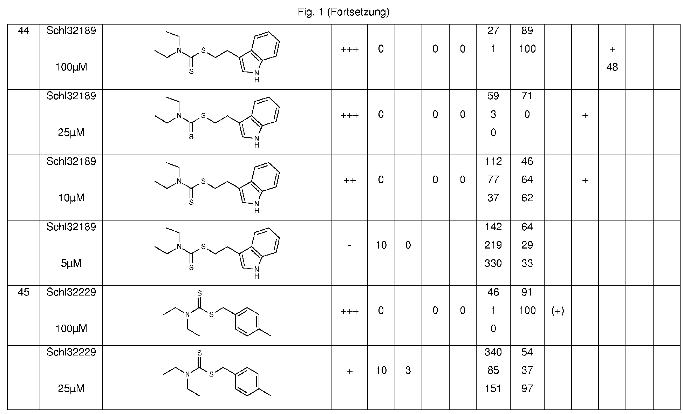

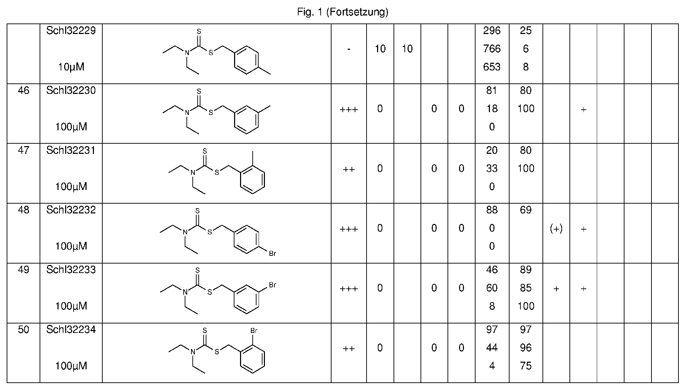

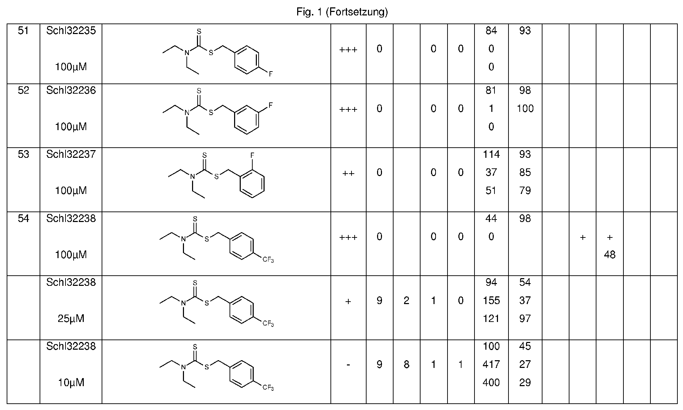

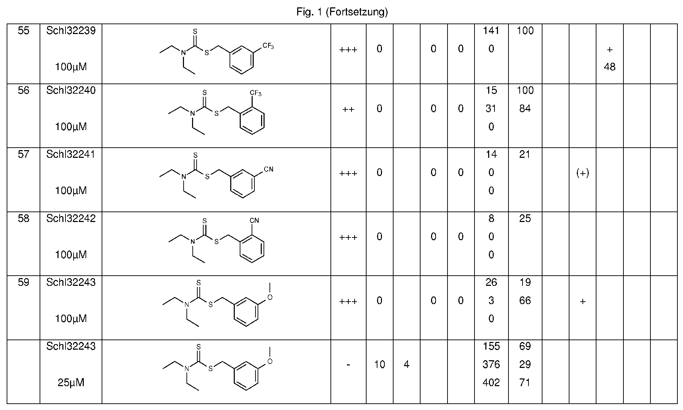

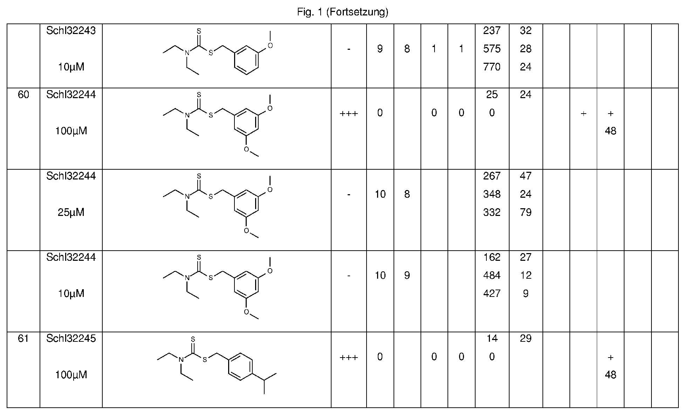

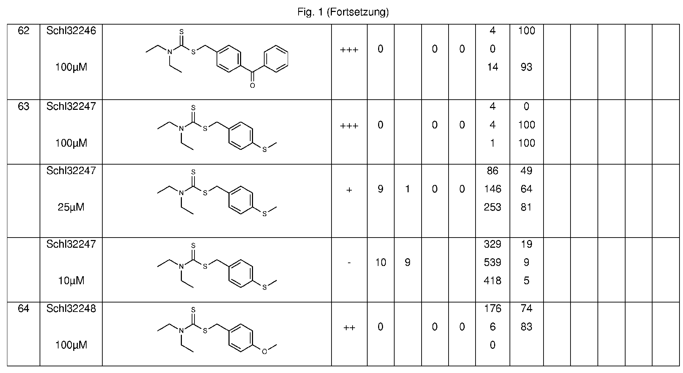

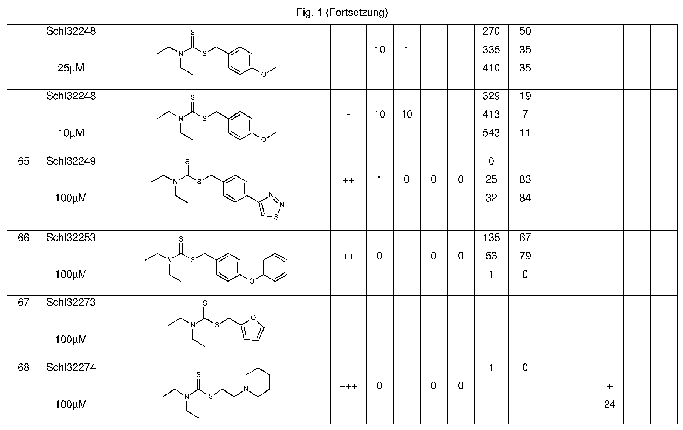

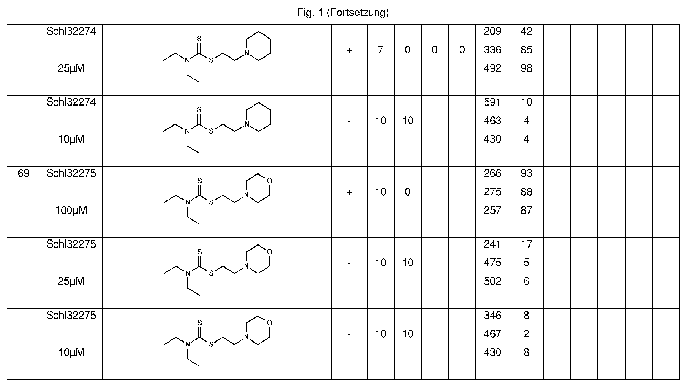

- Fig. 1: Tabellarische Ergebnisübersicht der biologischen Testungen von Diethylamin-Derivaten

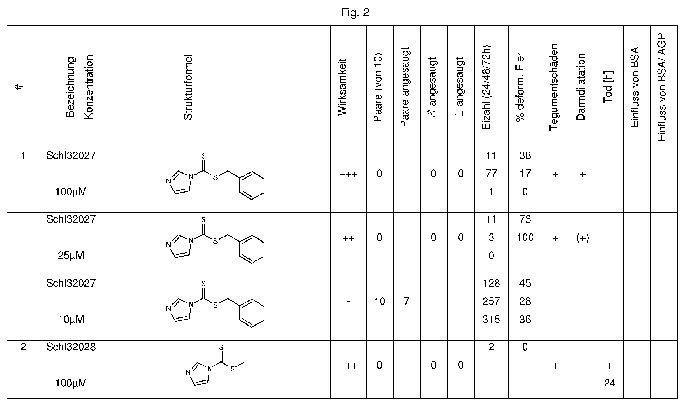

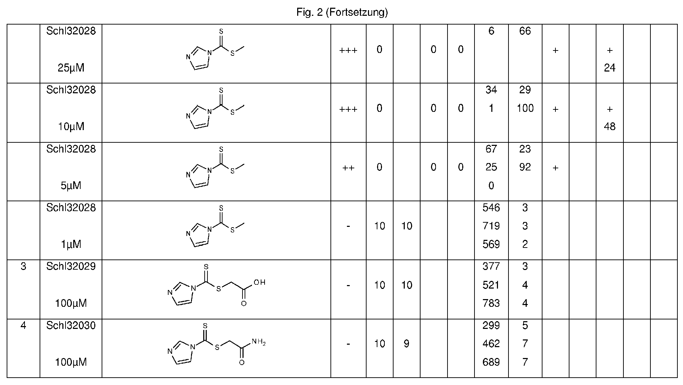

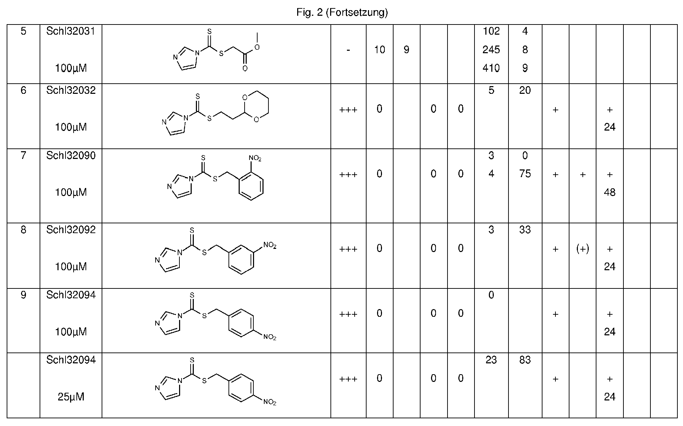

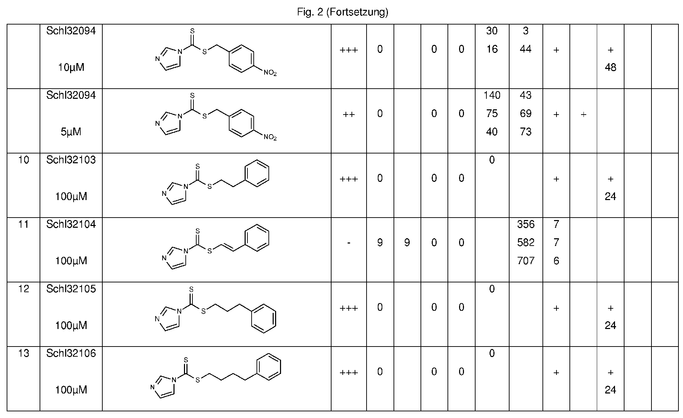

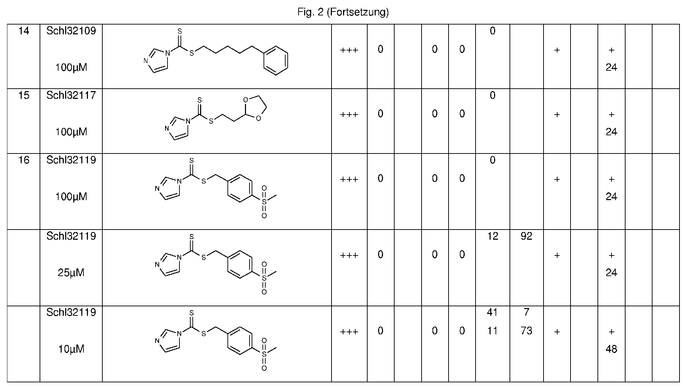

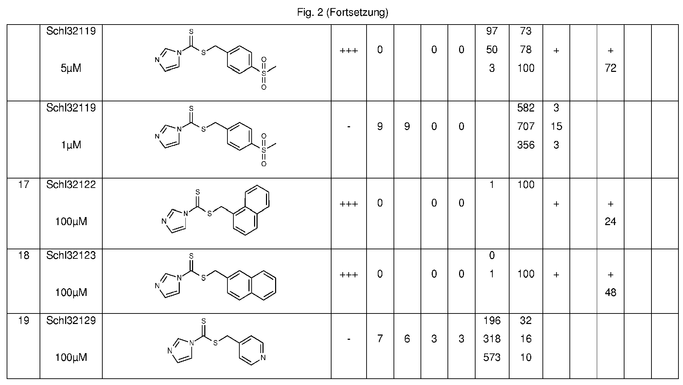

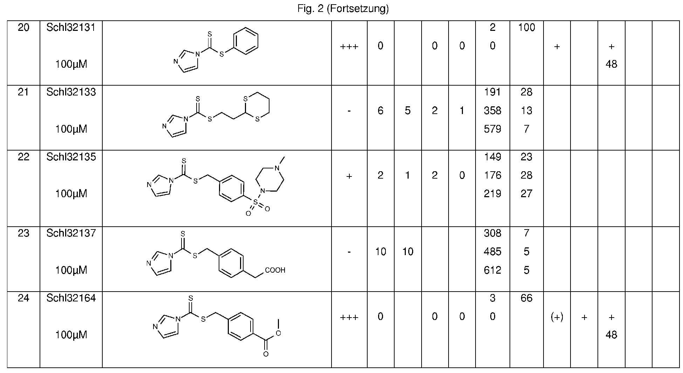

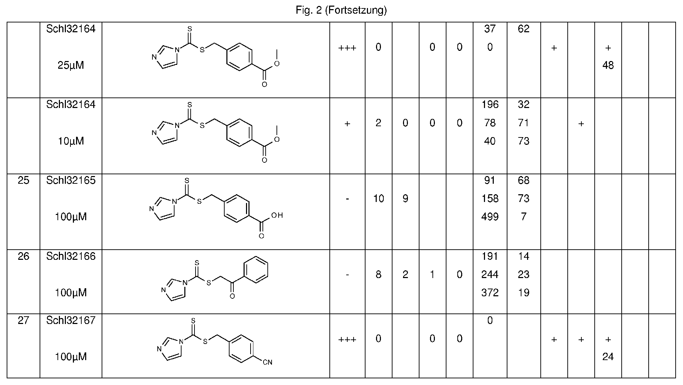

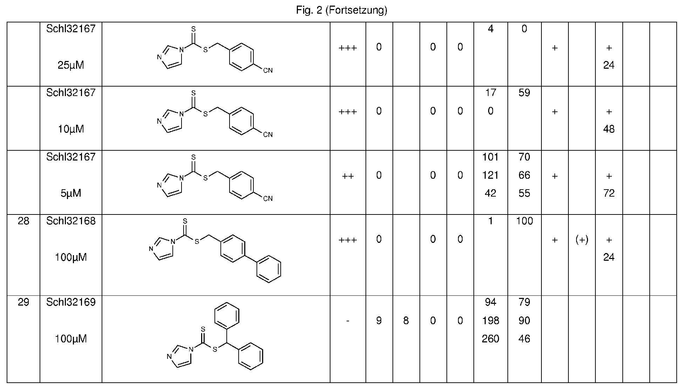

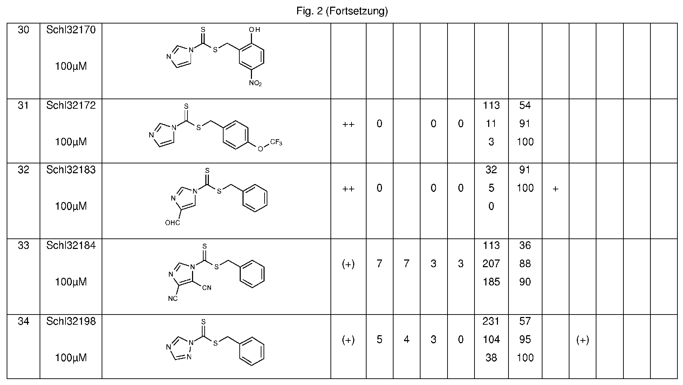

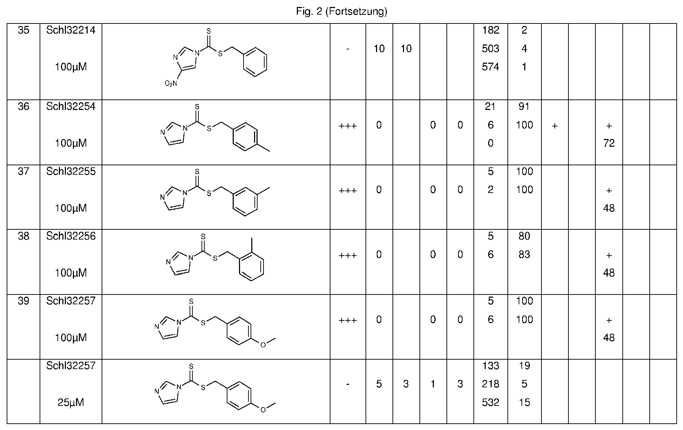

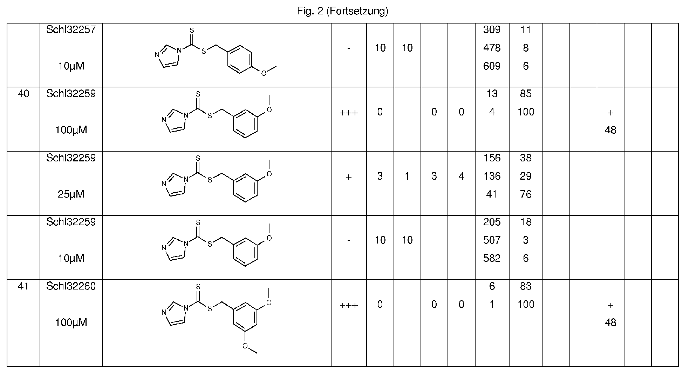

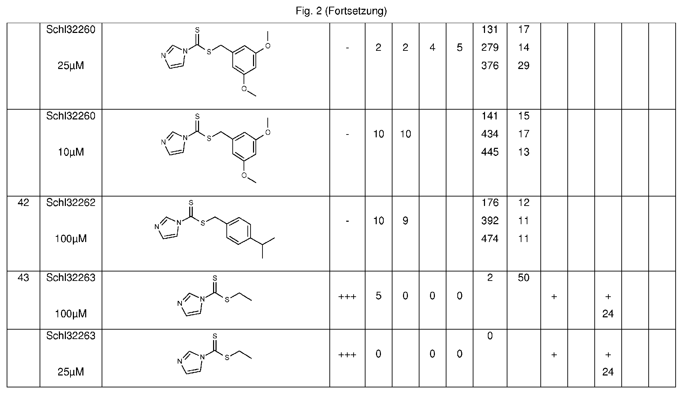

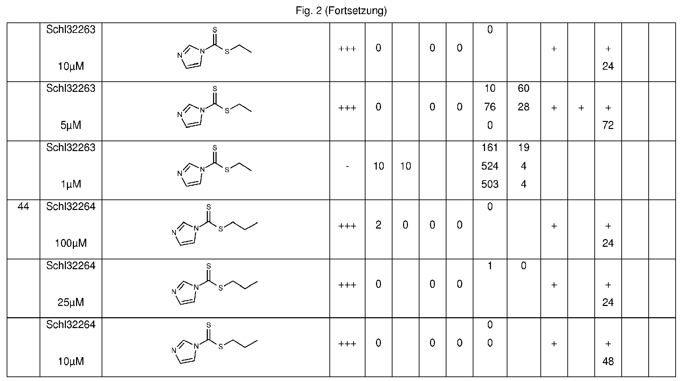

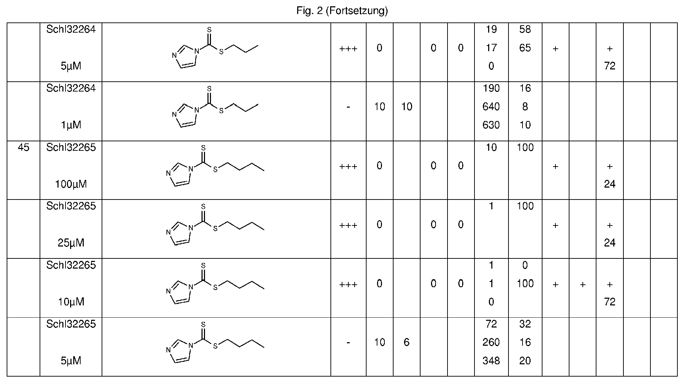

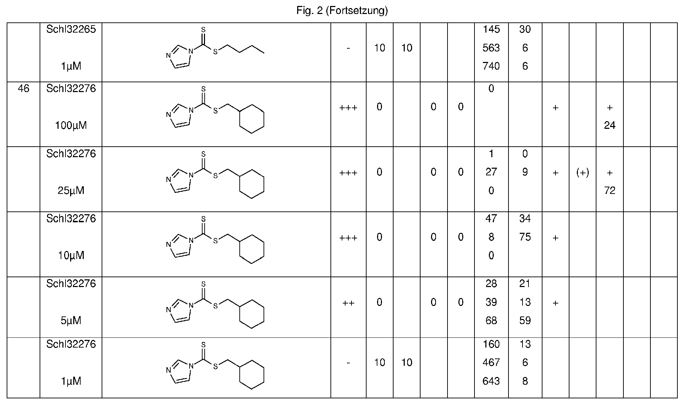

- Fig. 2: Tabellarische Ergebnisübersicht der biologischen Testungen von Imidazol-Derivaten

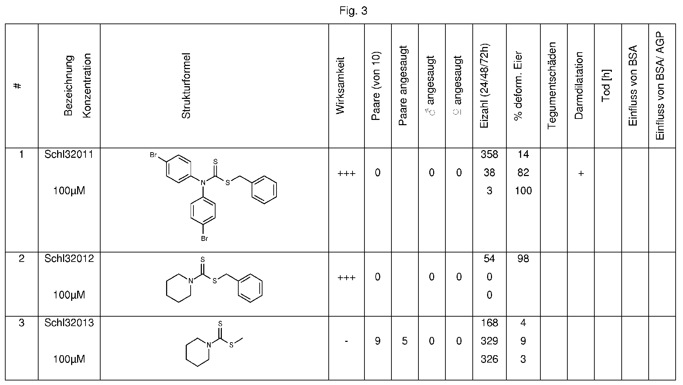

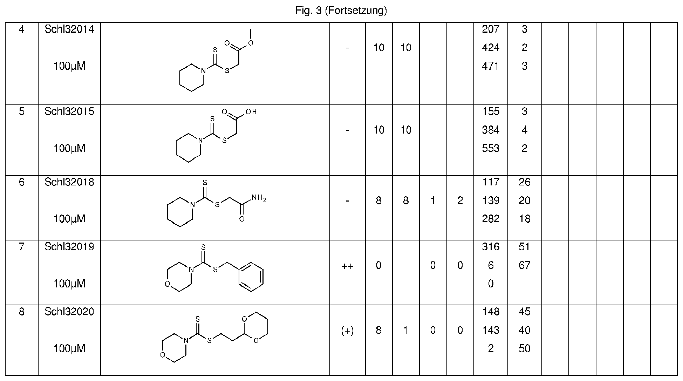

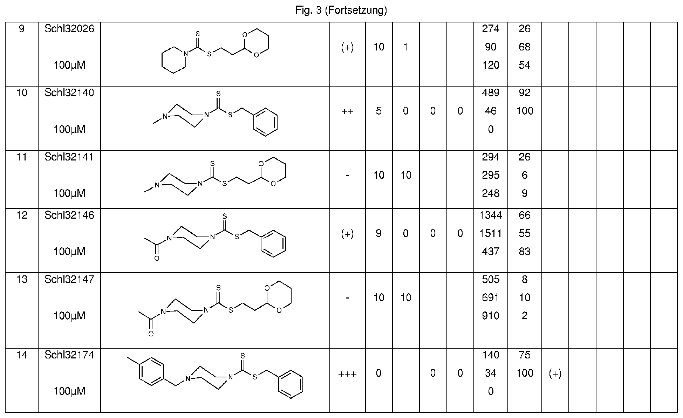

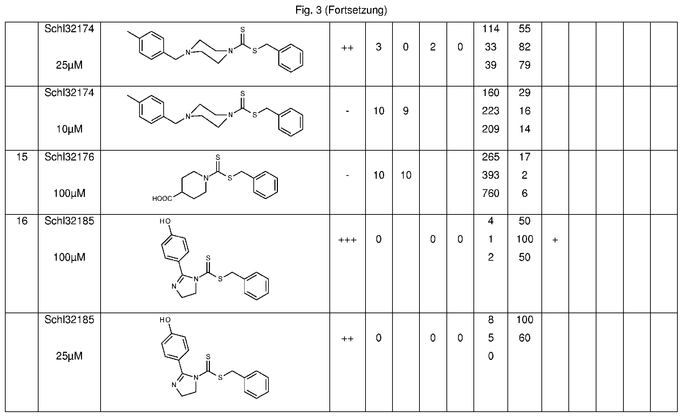

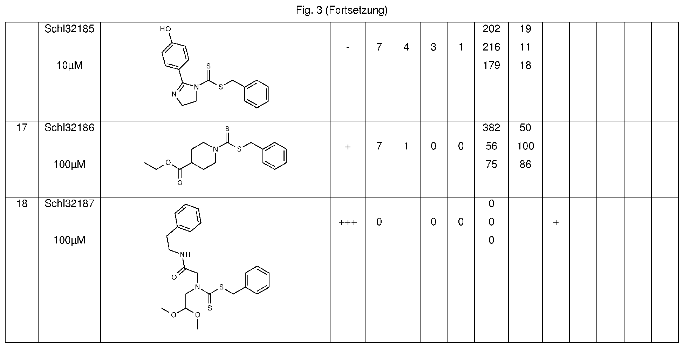

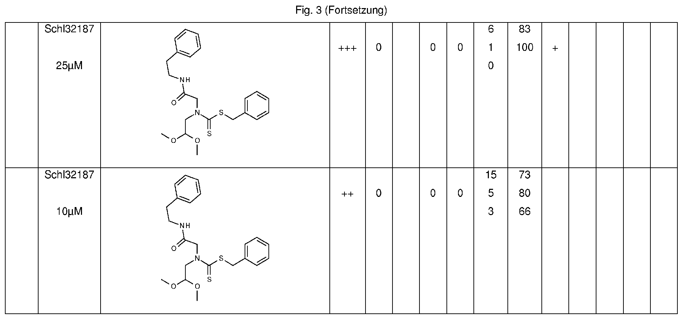

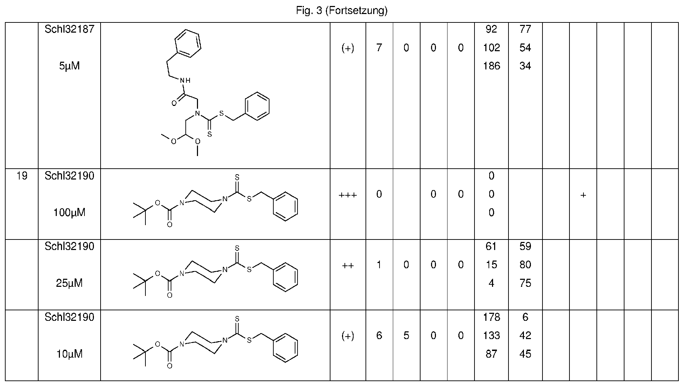

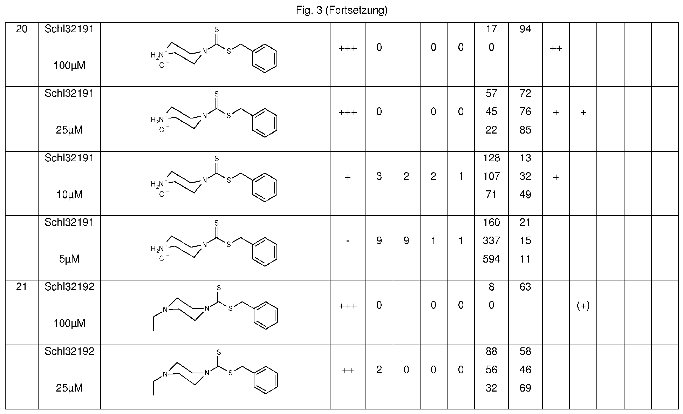

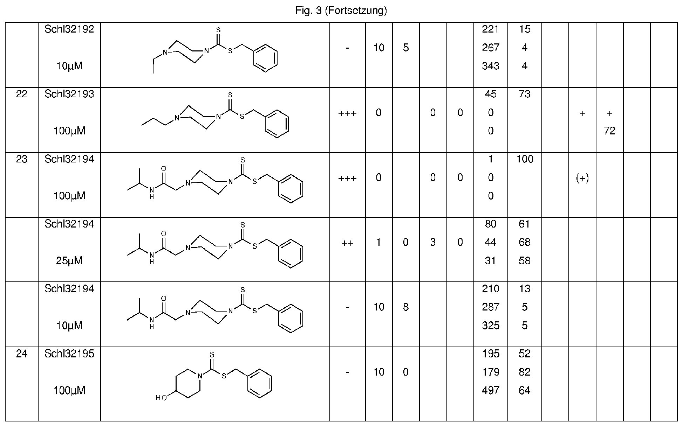

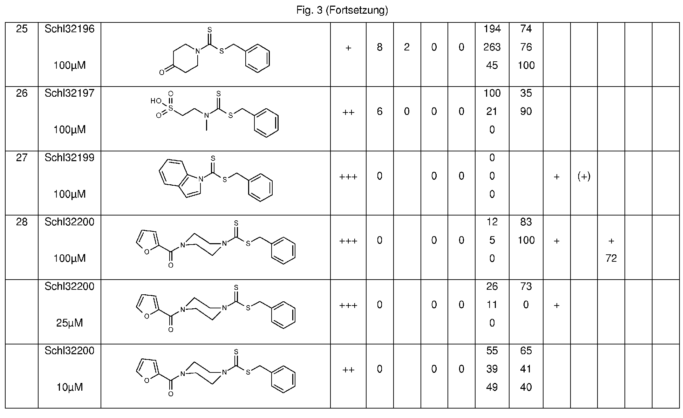

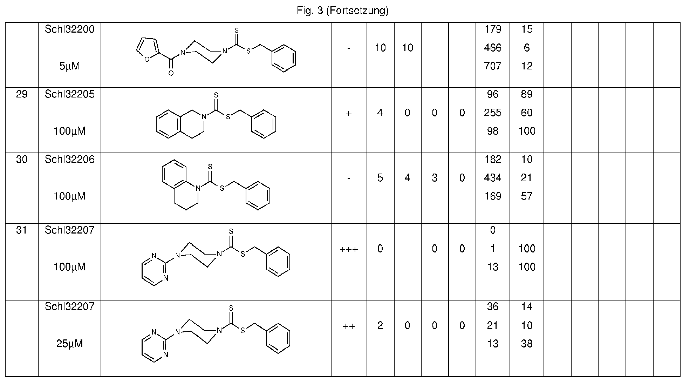

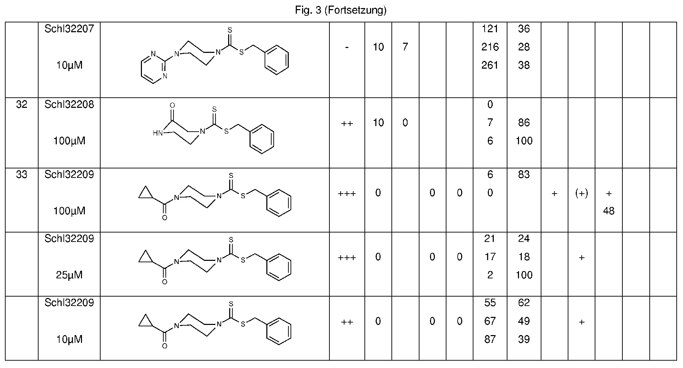

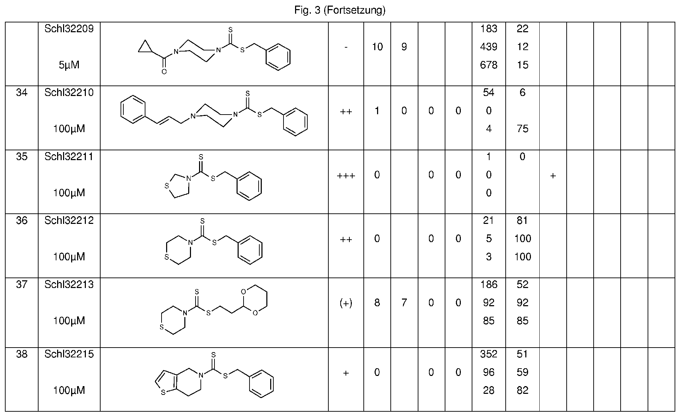

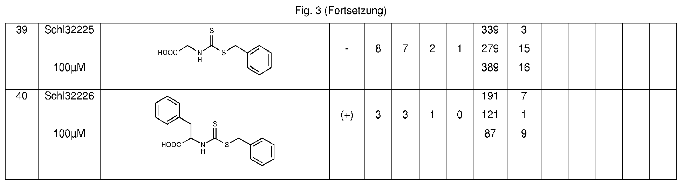

- Fig. 3: Tabellarische Ergebnisübersicht der biologischen Testungen von Verbindungen, die durch Aminaustausch erhalten wurden.

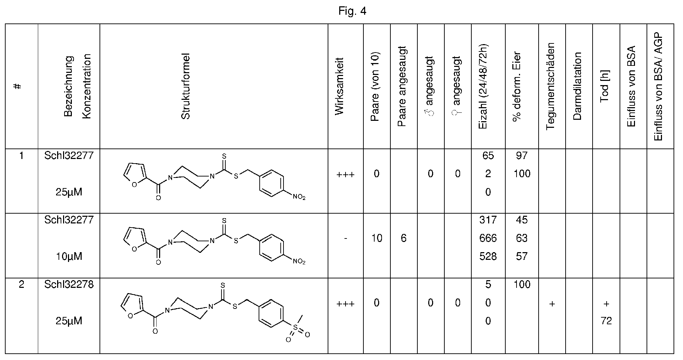

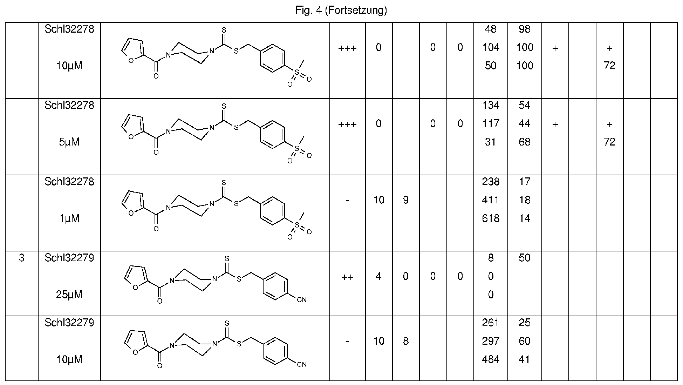

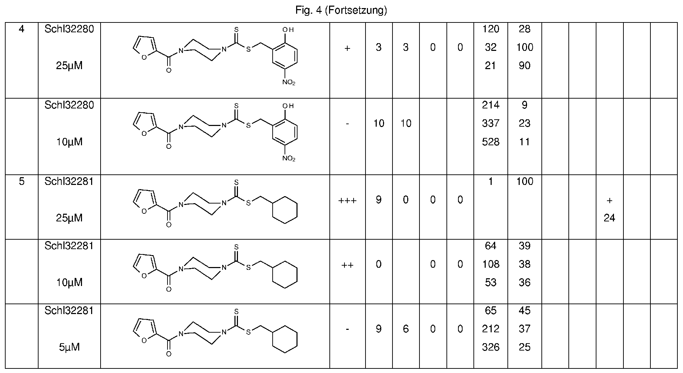

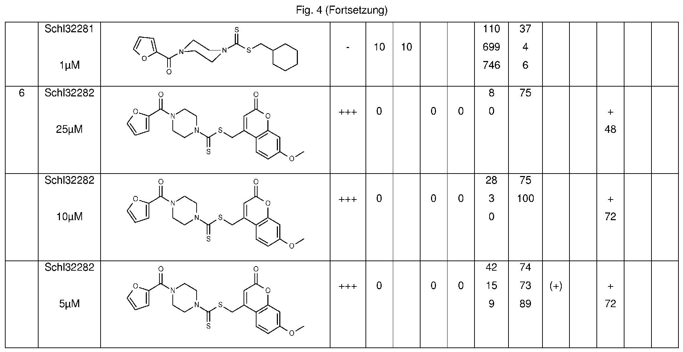

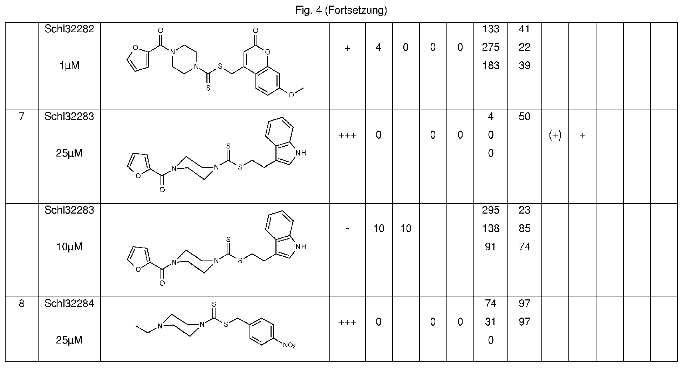

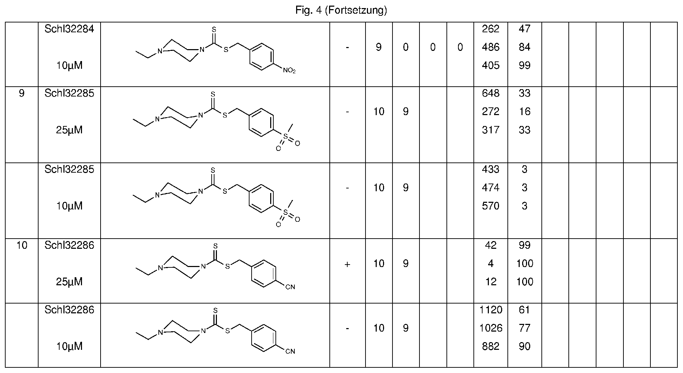

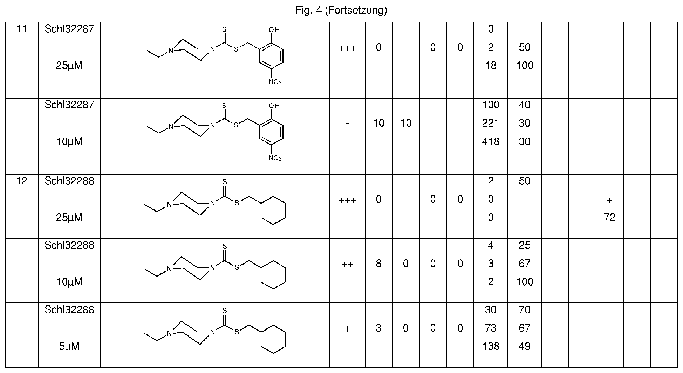

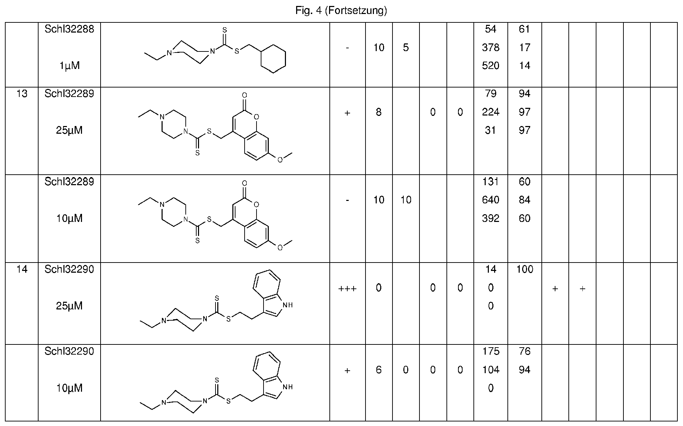

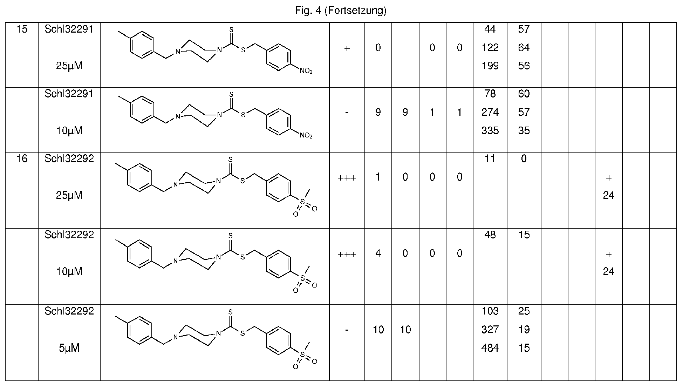

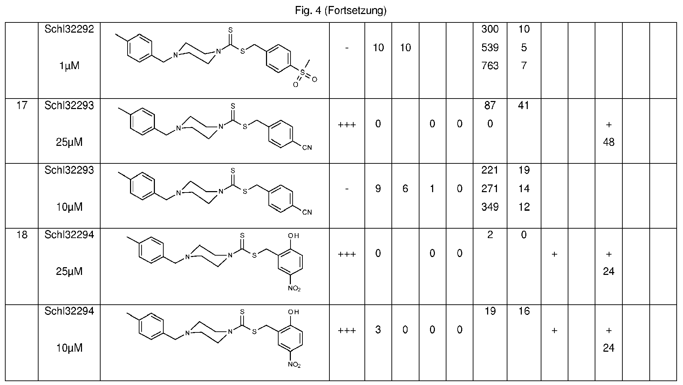

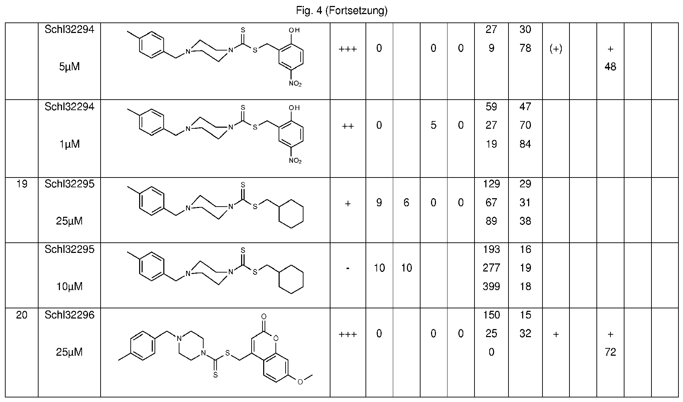

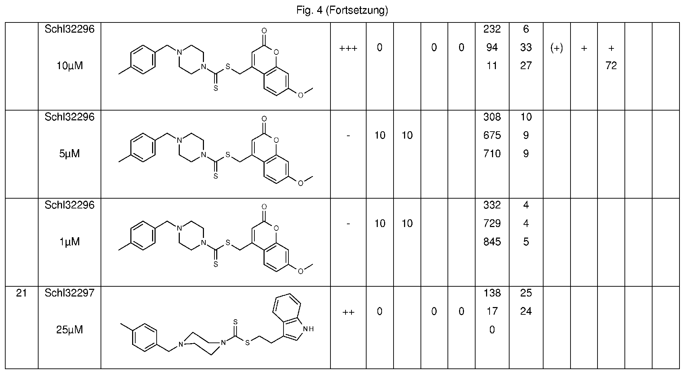

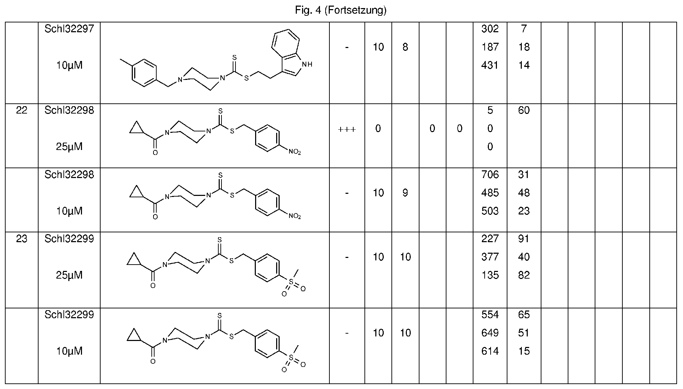

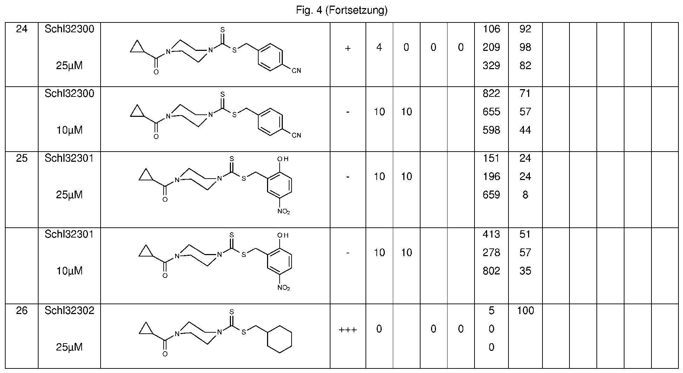

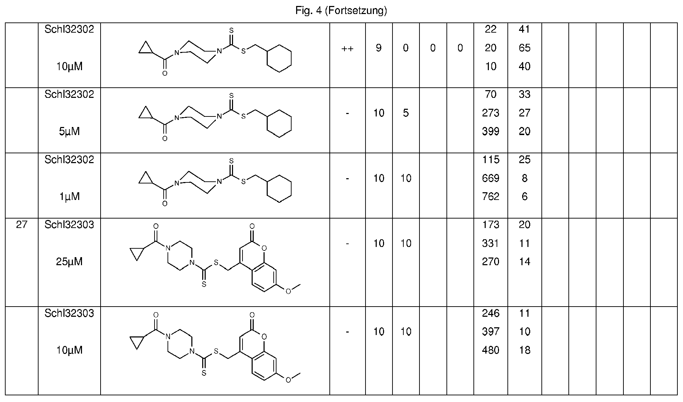

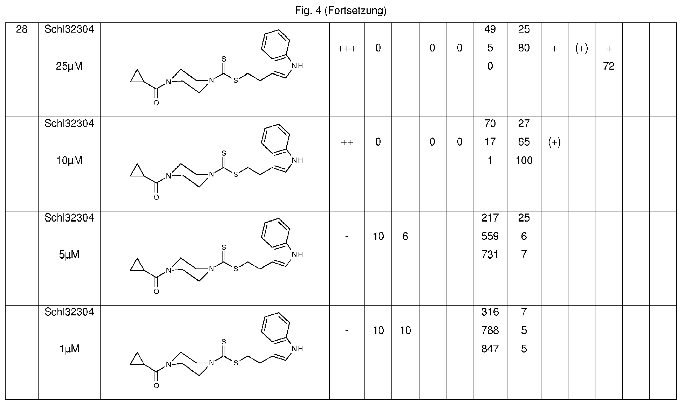

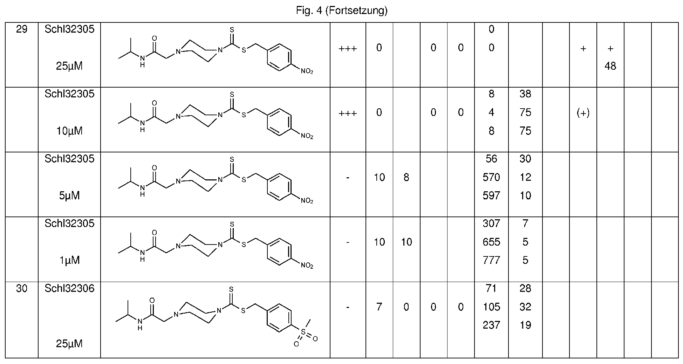

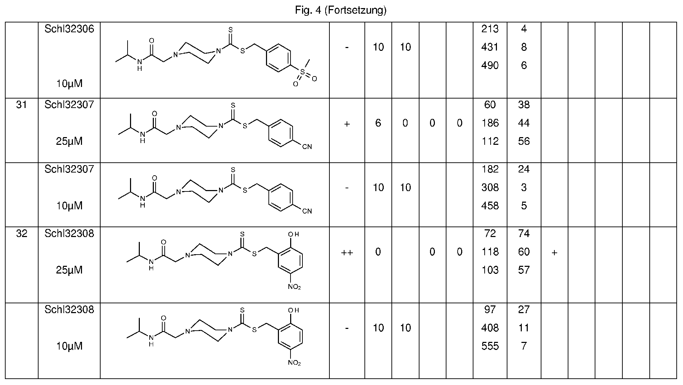

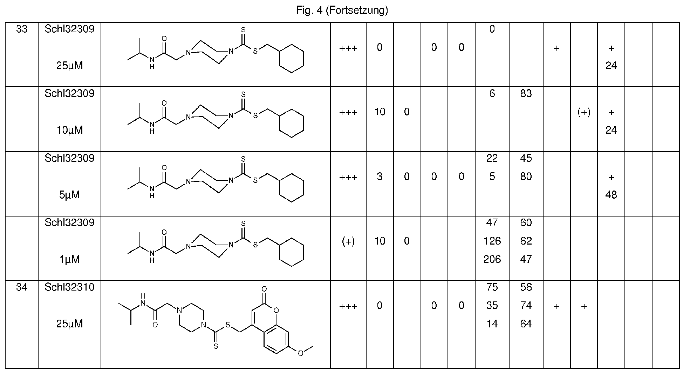

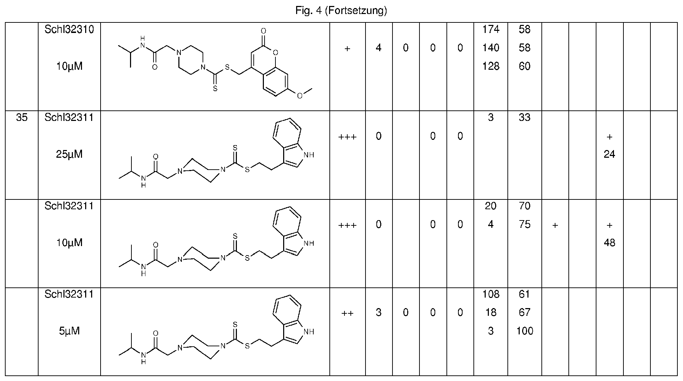

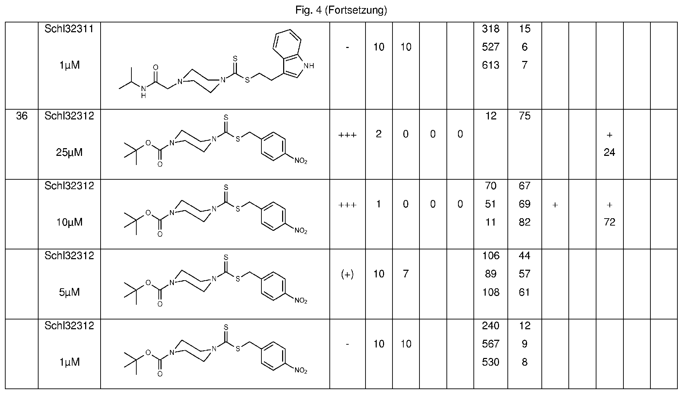

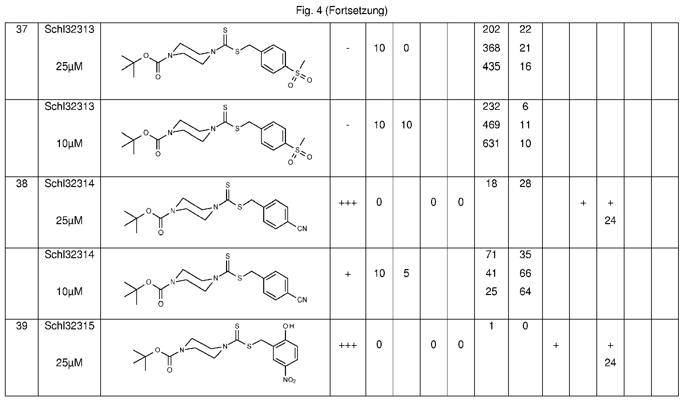

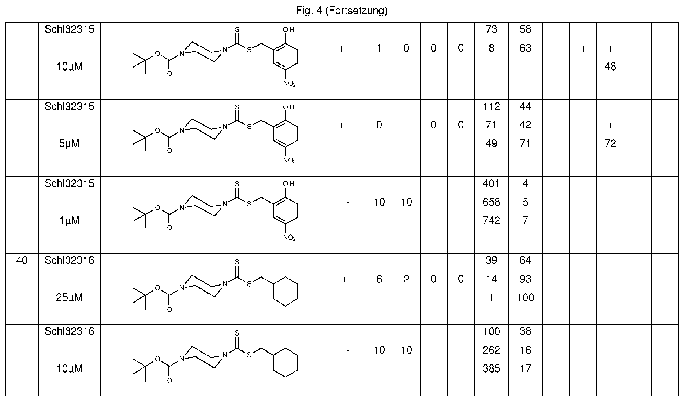

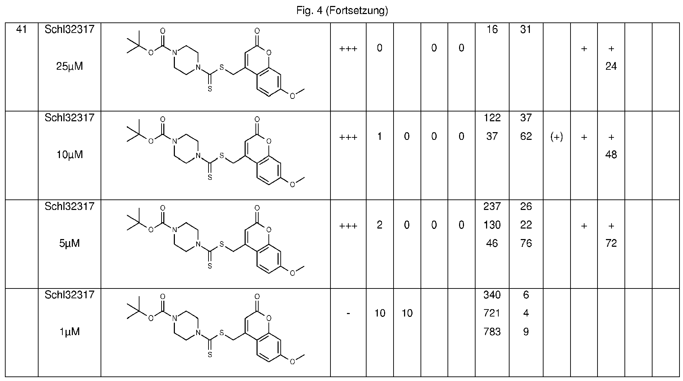

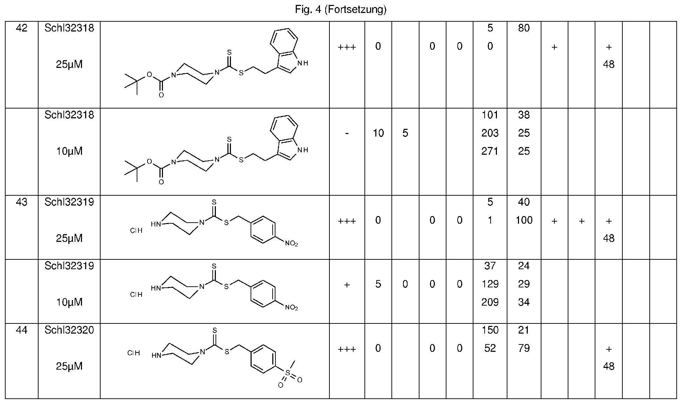

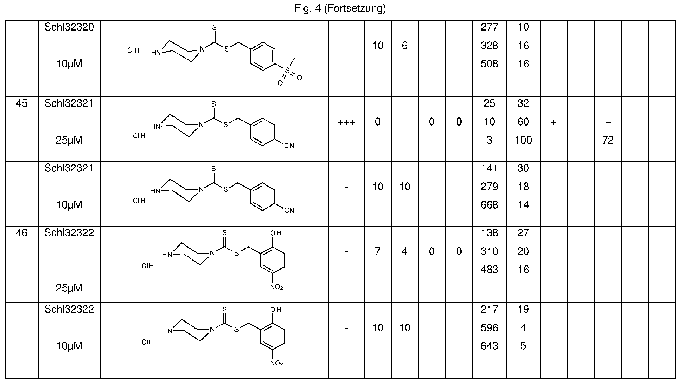

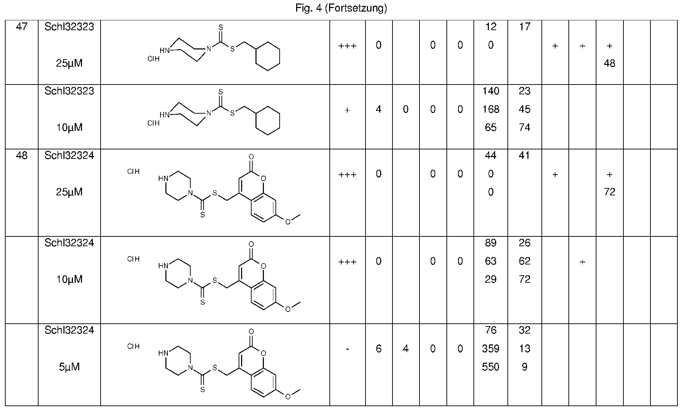

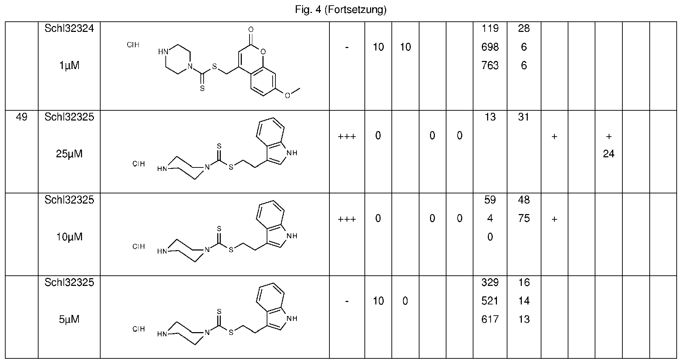

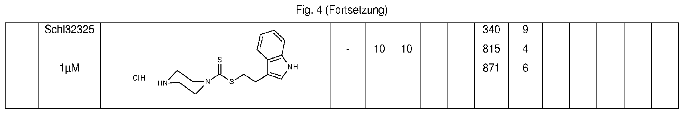

- Fig. 4: Tabellarische Ergebnisübersicht der biologischen Testungen von Verbindungen der Kombinationsserie 1

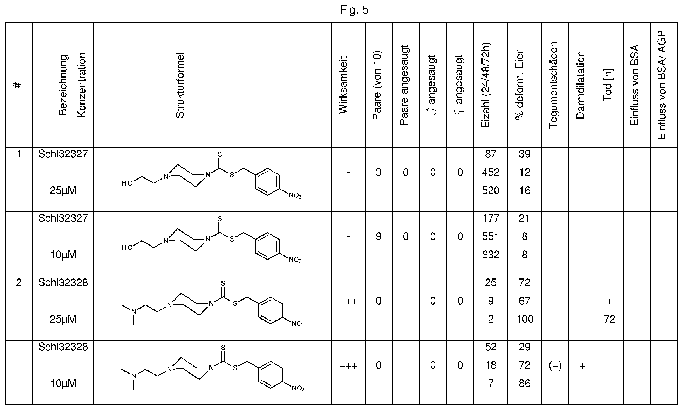

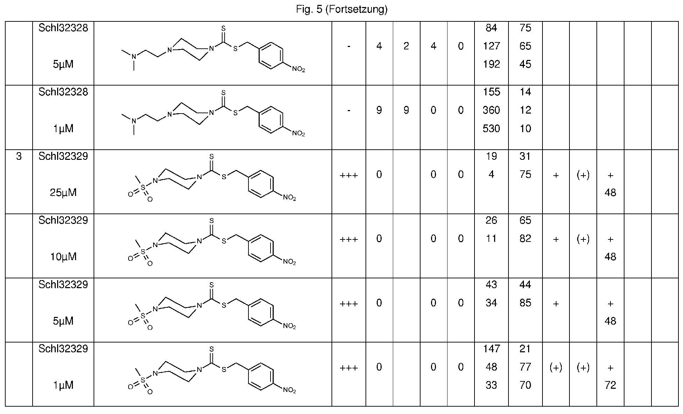

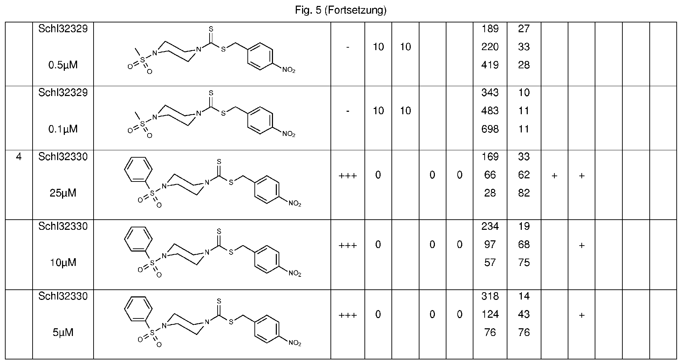

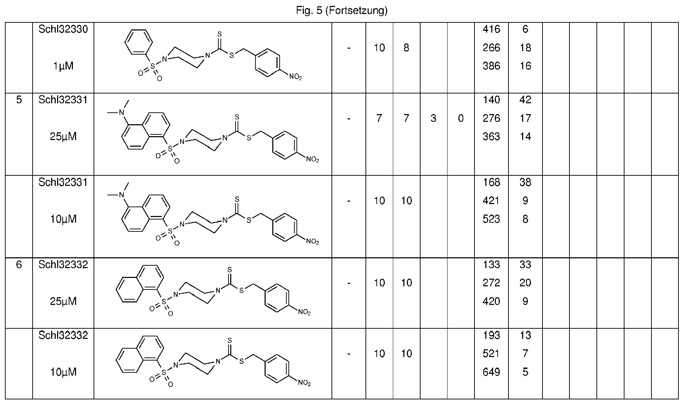

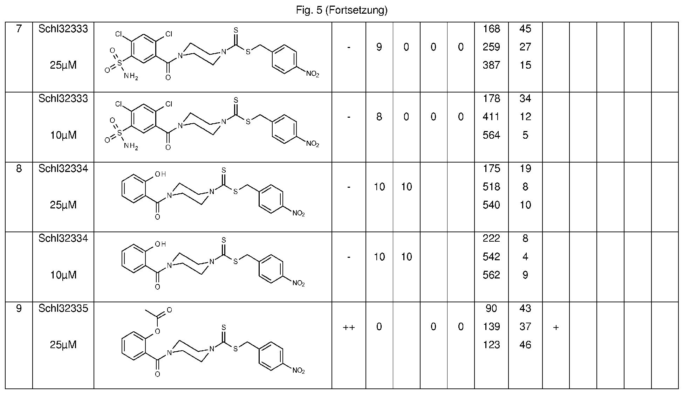

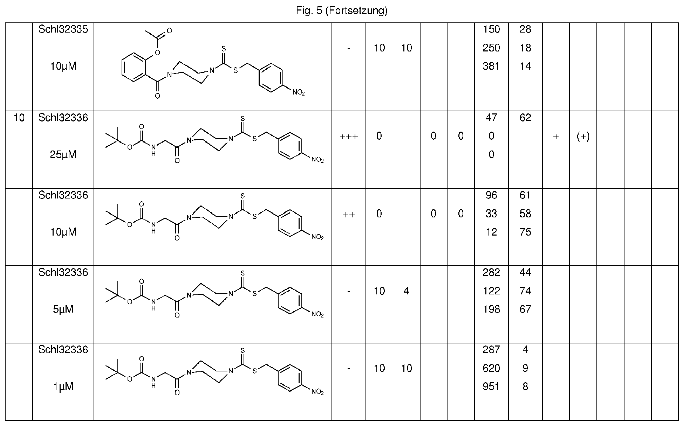

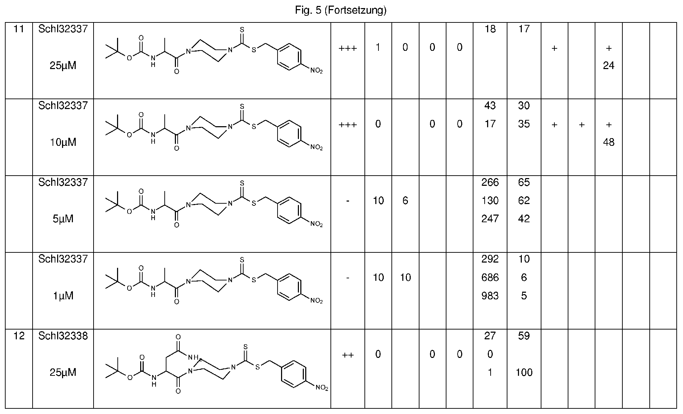

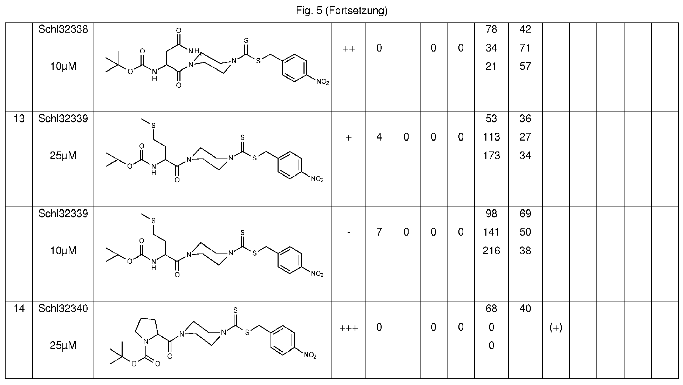

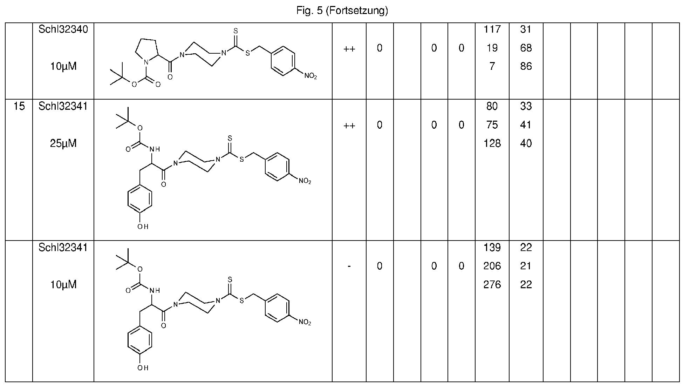

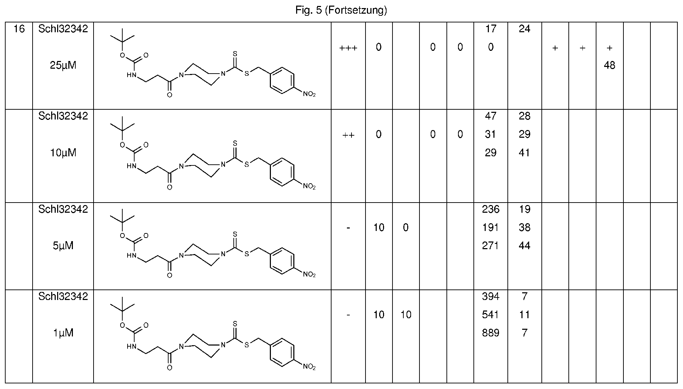

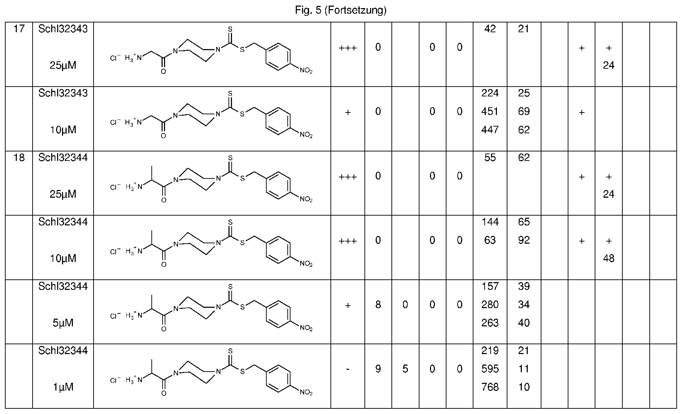

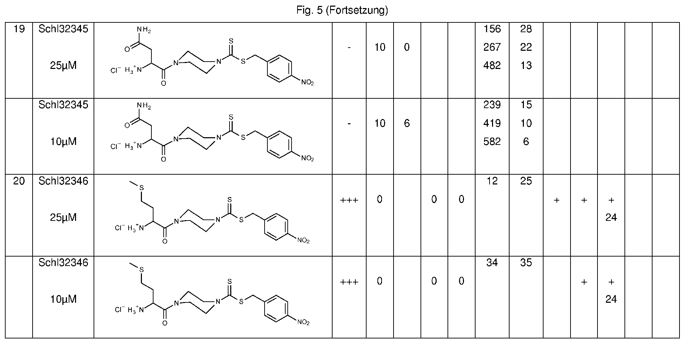

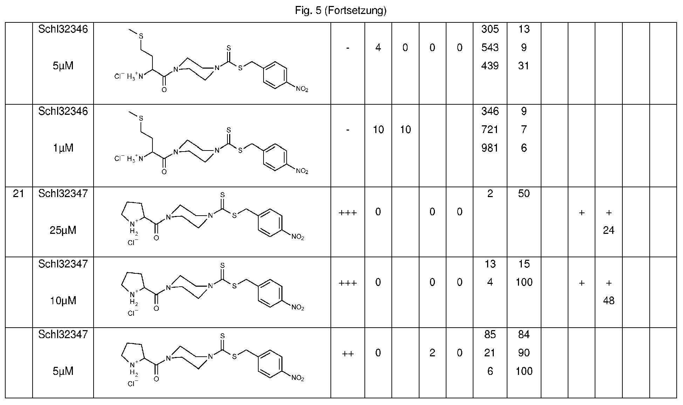

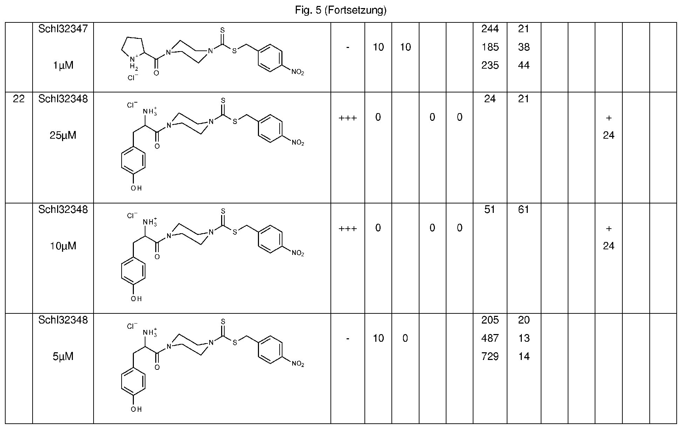

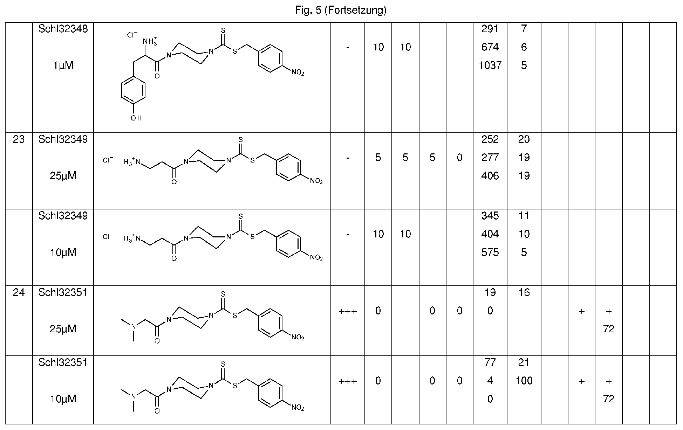

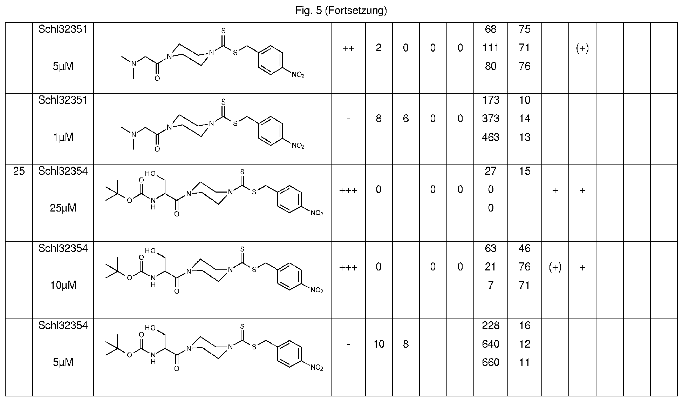

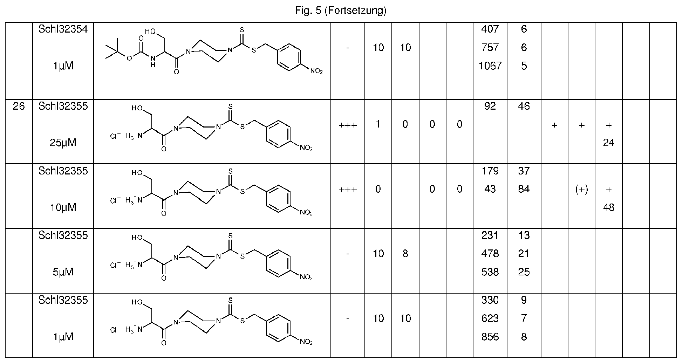

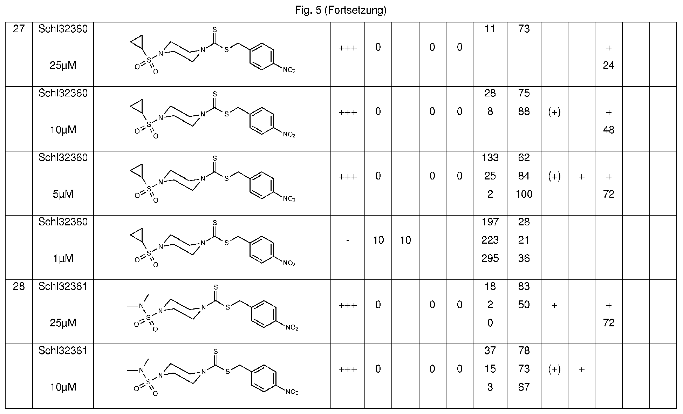

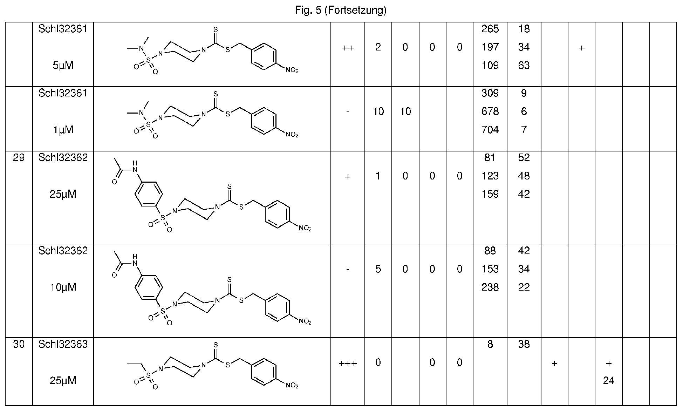

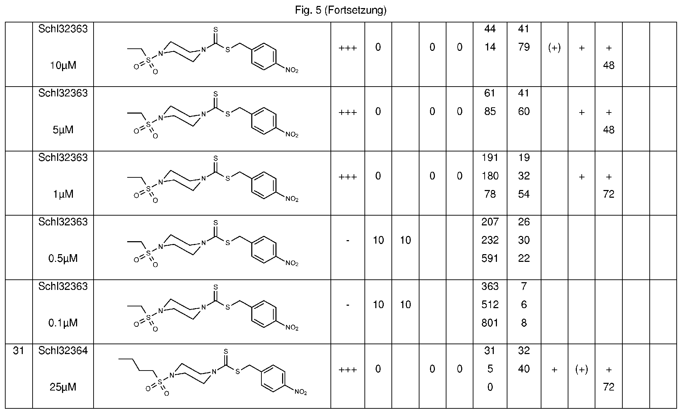

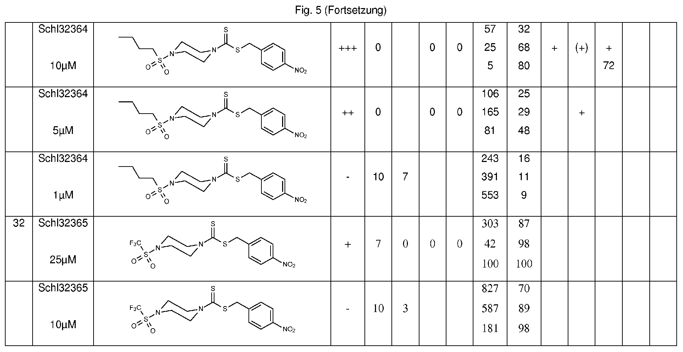

- Fig. 5: Tabellarische Ergebnisübersicht der biologischen Testungen von Verbindungen der Kombinationsserie 2 (Optimierung)

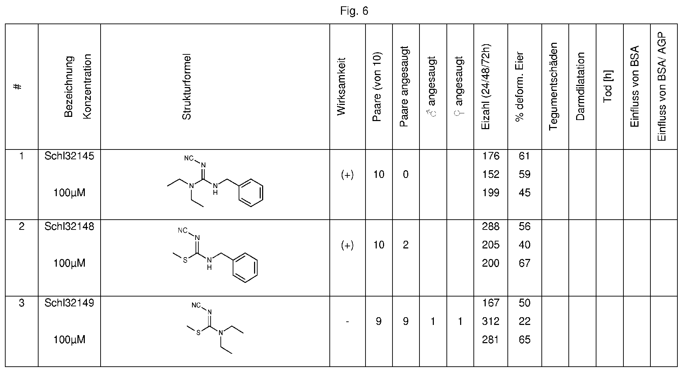

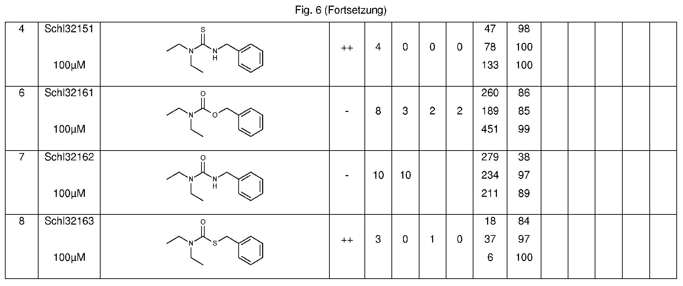

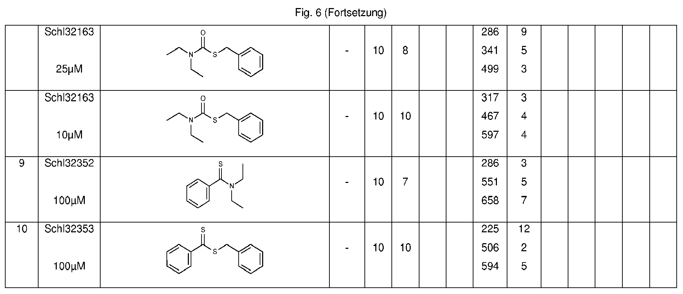

- Fig. 6: Tabellarische Ergebnisübersicht der biologischen Testungen von Verbindungen, die durch einen Heteroatomaustausch aus Dithiocarbamat hervorgegangen sind.

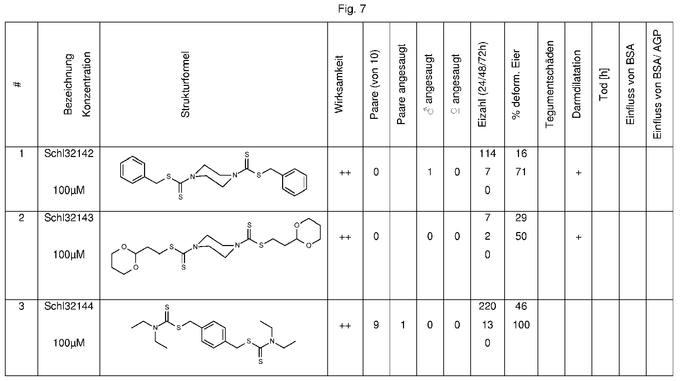

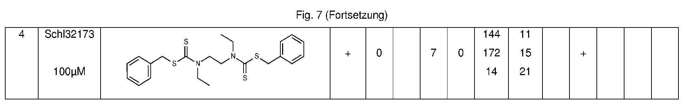

- Fig. 7: Tabellarische Ergebnisübersicht der biologischen Testungen von dimeren Dithiocarbamat- und Dithiocarbazat-Derivaten

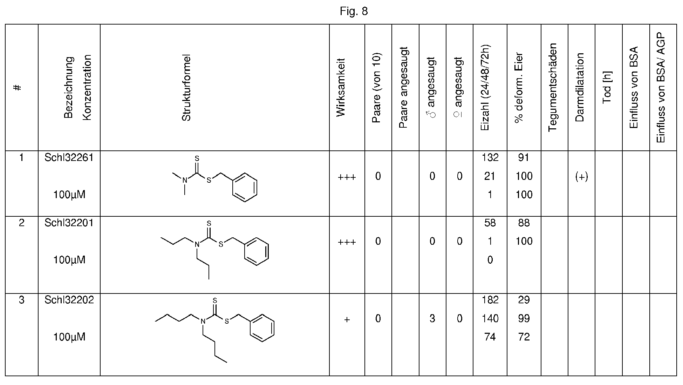

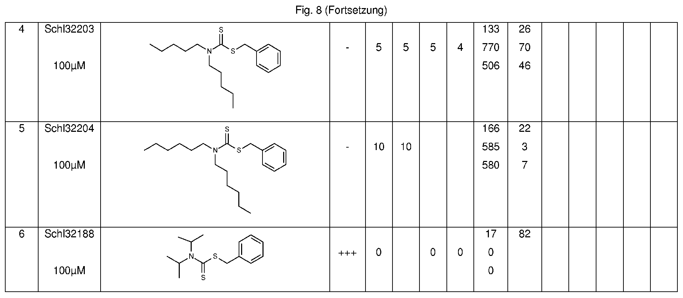

- Fig. 8: Tabellarische Ergebnisübersicht der biologischen Testungen von Benzyl-Derivaten mit veränderten Alkylketten

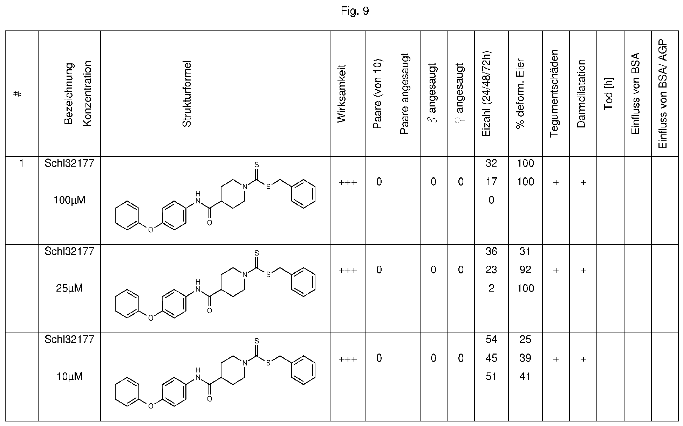

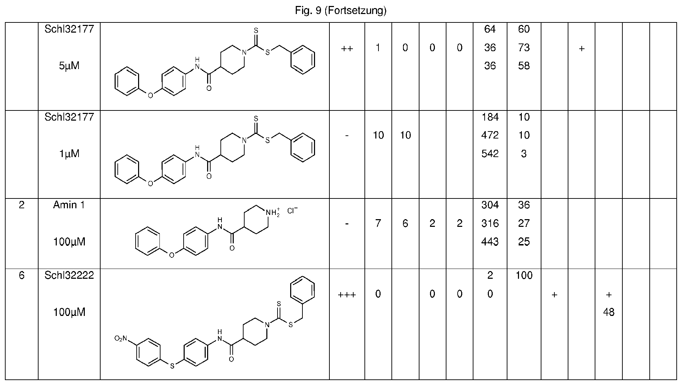

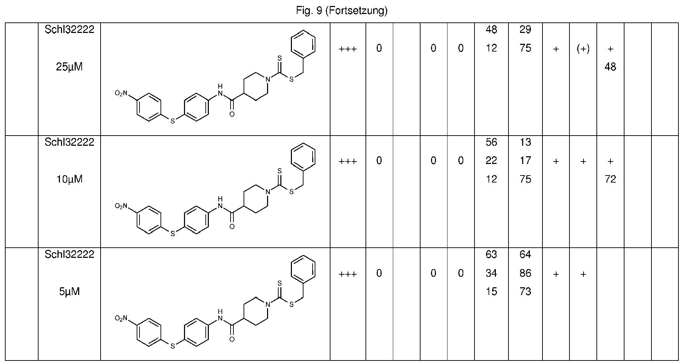

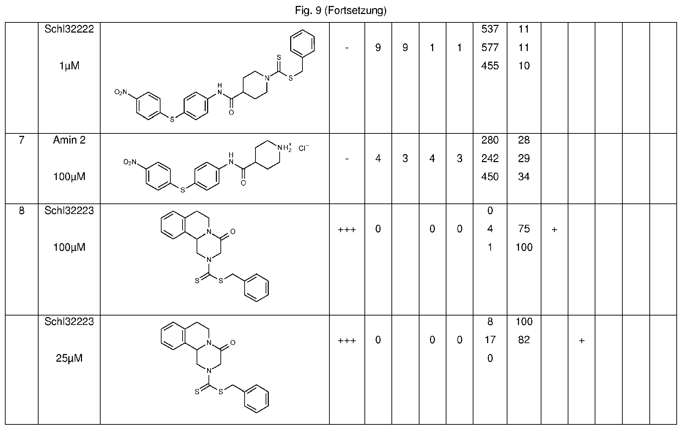

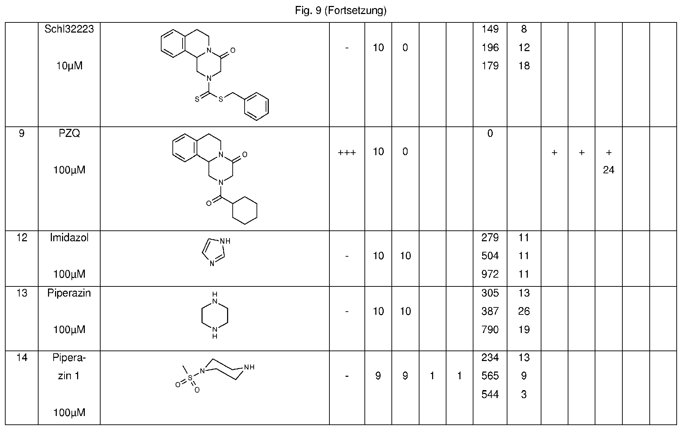

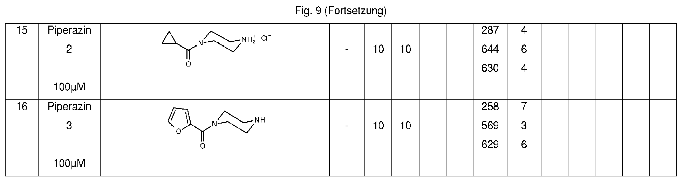

- Fig. 9: Tabellarische Ergebnisübersicht der biologischen Testungen weiterer erfindungsgemäßer Substanzen

Die Spaltenbezeichnungen der in den Abbildungen gezeigten Tabellen haben folgende Bedeutungen:
Spalte "Wirksamkeit":
   +++ ausgezeichnete antischistosomale Aktivität: nahezu vollständige Inhibition der Eiproduktion (weniger als 100 Eier/ 24 Stunden), deutliche Reduktion der Paarungsstabilität (Entpaarung von mindestens 8 der 10 Schistosomenpärchen), Vitalität und Motilität, letaler Effekt (falls aufgetreten), Auftreten von mehr als einem Phänotyp (zum Beispiel Tegumentschäden, Darmdilatationen, Verlust von inneren Strukturen, Anisocytose des Ovars, tumorähnliche Ausstülpungen, ödematöses Aufquellen der Schistosomen, Eiakkumulation im Uterus)
   ++ gute antischistosomale Aktivität: deutliche Inhibition der Eiproduktion (weniger als 200 Eier/ 24 Stunden), deutliche Reduktion der Paarungsstabilität (Entpaarung von mindestens 5 der 10 Schistosomenpärchen), Vitalität und Motilität, Auftreten von mindestens einem Phänotyp (zum Beispiel Tegumentschäden, Darmdilatationen, Verlust von inneren Strukturen, Anisocytose des Ovars, tumorähnliche Ausstülpungen, ödematöses Aufquellen der Schistosomen, Eiakkumulation im Uterus)
   + akzeptable antischistosomale Aktivität: leichte Inhibition der Eiproduktion (Reduktion um maximal 40%, bezogen auf DMSO als Referenz), geringgradige Reduktion der Paarungsstabilität (Entpaarung von maximal 4 der 10 Schistosomenpärchen), Vitalität und Motilität, keine weiteren Phänotypen erkennbar
   - keine antischistosomale Aktivität: keine Inhibition der Eiproduktion, keine Reduktion der Paarungsstabilität, Vitalität und Motilität, Schistosomen erscheinen morphologisch unauffällig und vital

- Spalte "Paare (von 10)":: Anzahl der gepaarten Schistosomenpärchen (von insgesamt 10 eingesetzten S. *mansoni-Pärchen*)*;* je weniger Schistosomenpärchen gepaart vorlagen, desto schlechter wurde die Vitalität der Schistosomen bewertet
- Spalte "Paare angesaugt":: Anzahl der gepaarten S. mansoni-Pärchen, die nach 72 Stunden am Schalenboden angesaugt waren; je weniger Schistosomenpärchen am Schalenboden angesaugt waren, desto schlechter wurde die Vitalität der Schistosomen bewertet
- Spalte ,,♂ angesaugt":: Anzahl der entpaarten Schistosomenmännchen, die nach 72 Stunden am Schalenboden angesaugt waren; je weniger Schistosomenmännchen am Schalenboden angesaugt waren, desto schlechter wurde die Vitalität der Schistosomen bewertet
- Spalte"♀ angesaugt":: Anzahl der entpaarten Schistosomenweibchen, die nach 72 Stunden am Schalenboden angesaugt waren; je weniger Schistosomenweibchen am Schalenboden angesaugt waren, desto schlechter wurde die Vitalität der Schistosomen bewertet
- Spalte "Eizahlen (24/48/72h)":: Absolute Anzahl von Schistosomeneiern, die 24 Stunden, 48 Stunden und 72 Stunden nach Beginn der Testung bei der angegebenen Konzentration gezählt wurden; bei Mehrfachmessungen wurde der Mittelwert angegeben (gerundet auf ganze Zahlen)
- Spalte "% deformierte Eier":: Prozentsatz von deformierten Schistosomeneiern, bezogen auf die absolute Anzahl von Schistosomeneiern; bei Mehrfachmessungen wurde der Mittelwert angegeben (gerundet auf ganze Zahlen)
- Spalte "Tegumentschäden": +: Auftreten von Tegumentschäden wurde bei der angegebenen Konzentration beobachtet
- Spalte "Darmdilatationen": +: Auftreten von Darmdilatationen wurde bei der angegebenen Konzentration beobachtet
- Spalte "Tod [h]": +: Auftreten eines letalen Effekts auf die Schistosomen wurde beobachtet; Angabe der Zeit, bis der letale Effekt bei der angegebenen Konzentration eintrat

## Patentansprüche

1. Verbindungen, **dadurch gekennzeichnet, daß** sie in ihrem chemischen Aufbau mindestens eine Teilstruktur gemäß der allgemeinen Formel I 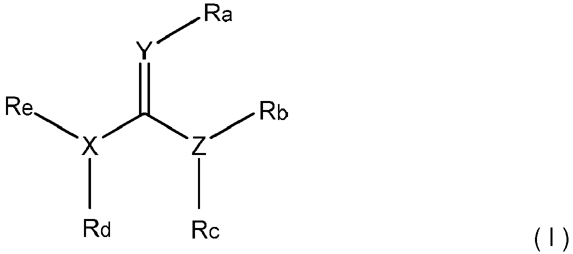 aufweisen, wobei es sich bei X, Y und Z unabhängig voneinander um Atome der chemischen Elemente Stickstoff, Sauerstoff, Schwefel, Selen handelt,
- Rₐ und R_{b} ausgewählt sind aus der Liste umfassend "kein Rest", H, Alkyl, Aryl, Heteroaryl, Acyl, SOₙR_{f}, SOₙH, POₘR_{f}, POₘH, SO_{z}N(R_{f})₂; POm(NRf)x, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl,
substituiertes Heteroaryl, Alkylaryl, Alkylheteroaryl,
- R_{c} entweder
a) ausgewählt ist aus der Liste umfassend "kein Rest", H, Alkyl, Aryl, Heteroaryl, Acyl, SOₙR_{f}, SOₙH, POₘR_{f}, POₘH, SO_{z}N(R_{f})₂; POm(NRf)x, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl,
substituiertes Heteroaryl, Alkylaryl, Alkylheteroaryl oder
b) es sich bei R_{c} um mindestens einen mindestens ein Atom umfassenden Rest handelt, der mit Z über eine Doppelbindung verknüpft ist,
- R_{d} und Rₑ entweder
i) unabhängig voneinander ausgewählt sind aus der Liste umfassend "kein Rest", H, Alkyl, Cycloalkyl, Aryl, Heteroaryl, Acyl, SOₙR_{f}, SOₙH, POₘR_{f}, POₘH, SO_{z}N(R_{f})₂; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl , oder
ii) R_{d} und Rₑ zweibindige Reste sind, die miteinander verbunden sind und dadurch zusammen mit Atom X eine cyclische Teilstruktur bilden, oder
iii) R_{d} ausgewählt ist aus der Liste umfassend "kein Rest", H, Alkyl, Cycloalkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl und Rₑ entweder
α) Teil einer cyclischen Teilstruktur ist, oder
β) es sich bei Rₑ um ein Heteroatom handelt, ausgewählt aus der Liste umfassend die chemischen Elemente Stickstoff, Sauerstoff, Schwefel, und das entweder Bestandteil einer cyclischen Teilstruktur ist, oder das zwei Reste trägt, die unabhängig voneinander ausgewählt sind aus der Liste umfassend "kein Rest", H, Alkyl, Cycloalkyl, Aryl, Heteroaryl, Acyl, SOnRf, SOₙH, POₘR_{f}, POₘH, SOzN(Rf)2; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl,
substituiertes Heteroaryl, Alkylaryl, Alkylheteroaryl,
- R_{f} ausgewählt ist aus der Liste umfassend H, Alkyl, Aryl, Heteroaryl, substituiertes Aryl, substituiertes Heteroaryl, Alkylaryl, Alkylheteroaryl,
- die Substituenten der substituierten Aryl- und Heteroarylreste ausgewählt sind aus der Liste umfassend F, Cl, Br, OH, Phenyl, NH₂, NO₂, OR_{f}, O-COR_{f}, COR_{f}, N(R_{f})₂, NR_{f}COR_{f}, O-CSR_{F}, CSR_{f}, NR_{f}CSR_{f}, O-CSeR_{f}, CSeR_{f}, NR_{f}CSeR_{f}, O-CN(R_{f})R_{f}, CN(R_{f})R_{f}, NR_{f}CN(R_{f})R_{f}, N(R_{f})SO₂R_{f}, N(R_{f})SO₂N(R_{f})₂, SSR_{f}, SR_{f}, SOₓR_{f}, SOₙN(R_{f})₂, Nitril, Amidin, Guanidin, COOR_{f}, CON(R_{f})₂, CON(R_{f})N(R_{f})₂, COSR_{f}, CSOR_{f}, CSSR_{f}, NR_{f}COR_{f}, N(R_{f})N(R_{f})₂,
- der Formelindex x Werte von 1 bis 2 annehmen kann,
- der Formelindex y Werte von 1 bis 2 annehmen kann,
- der Formelindex z Werte von 0 bis 1 annehmen kann,
- der Formelindex n Werte von 0 bis 4 annehmen kann und
- der Formelindex m Werte von 1 bis 4 annehmen kann.

2. Verbindungen gemäß Anspruch 1, **dadurch gekennzeichnet, daß** sie in ihrem chemischen Aufbau mindestens eine Teilstruktur gemäß der allgemeinen Formel II 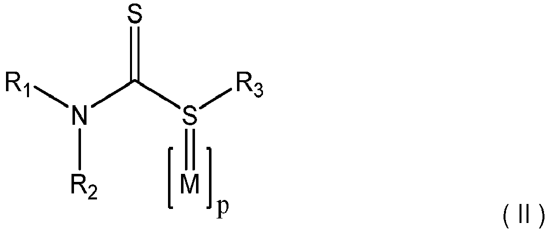 aufweisen, wobei
- R₁ und R₂ entweder
a) unabhängig voneinander ausgewählt sind aus der Liste umfassend H, Alkyl, Cycloalkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, oder
b) es sich bei R₁ und R₂ um mindestens zweiwertige Reste handelt, die über mindestens eine chemische Bindung miteinander verknüpft sind, so daß die Reste R₁ und R₂ zusammen mit dem Stickstoffatom einen Heterozyklus bilden, und
- R₃ ausgewählt ist aus der Liste umfassend Alkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl,
substituiertes Heteroaryl, Alkylaryl, Alkylheteroaryl,
- M ausgewählt ist aus der Liste umfassend O,NH, NR_{f} und
- R_{f} ausgewählt ist aus der Liste umfassend H, Alkyl, Aryl, Heteroaryl, substituiertes Aryl, substituiertes Heteroaryl, Alkylaryl, Alkylheteroaryl,
- der Formelindex x Werte von 1 bis 2 annehmen kann,
- der Formelindex y Werte von 1 bis 2 annehmen kann,
- der Formelindex z Werte von 0 bis 1 annehmen kann,
- der Formelindex n Werte von 0 bis 4 annehmen kann,
- der Formelindex m Werte von 1 bis 4 annehmen kann und
- der Formelindex p Werte von 0 bis 2 annehmen kann.

3. Verbindungen gemäß Anspruch 1, **dadurch gekennzeichnet, daß** sie in ihrem chemischen Aufbau mindestens eine Teilstruktur gemäß der allgemeinen Formel III 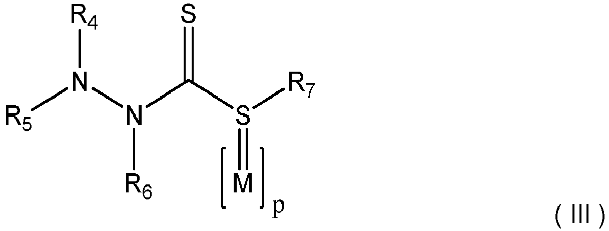 aufweisen, wobei
- R₄ und R₅ entweder
a) unabhängig voneinander ausgewählt sind aus der Liste umfassend H, Alkyl, Cycloalkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, oder
b) es sich bei R₄ und R₅ um mindestens zweiwertige Reste handelt, die über mindestens eine chemische Bindung miteinander verknüpft sind, so daß die Reste R₄ und R₅ zusammen mit dem Stickstoffatom einen Heterozyklus bilden, und
- R₆ und R₇ ausgewählt sind aus der Liste umfassend H, Alkyl, Aryl, Heteroaryl, Acyl, SOₙR_{f}, SOₙH, POₘR_{f}, POₘH, SO_{z}N(R_{f})₂; POₘ(NR_{f})ₓ, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl,
substituiertes Heteroaryl, Alkylaryl, Alkylheteroaryl,
- M ausgewählt ist aus der Liste umfassend O,NH, NR_{f} und
- R_{f} ausgewählt ist aus der Liste umfassend H, Alkyl, Aryl, Heteroaryl, substituiertes Aryl, substituiertes Heteroaryl, Alkylaryl, Alkylheteroaryl,
- der Formelindex x Werte von 1 bis 2 annehmen kann,
- der Formelindex y Werte von 1 bis 2 annehmen kann,
- der Formelindex z Werte von 0 bis 1 annehmen kann,
- der Formelindex n Werte von 0 bis 4 annehmen kann,
- der Formelindex m Werte von 1 bis 4 annehmen kann und
- der Formelindex p Werte von 0 bis 2 annehmen kann.

4. Verbindungen gemäß Anspruch 1, **dadurch gekennzeichnet, daß** sie in ihrem chemischen Aufbau mindestens eine Teilstruktur gemäß der allgemeinen Formel IV 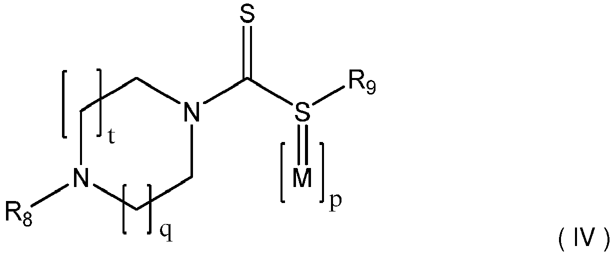 aufweisen, wobei
- R₈ und R₉ unabhängig voneinander ausgewählt sind aus der Liste umfassend H, Alkyl, Cycloalkyl, Aryl, Heteroaryl, Acyl, SOₙR_{f}, SOₙH, POₘR_{f}, POₘH, SO_{z}N(R_{f})₂; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl,
- M ausgewählt ist aus der Liste umfassend O, NH, NR_{f},
- R_{f} ausgewählt ist aus der Liste umfassend H, Alkyl, Aryl, Heteroaryl, substituiertes Aryl, substituiertes Heteroaryl, Alkylaryl, Alkylheteroaryl,
- der Formelindex t Werte von 0 bis 4 annehmen kann,
- der Formelindex x Werte von 1 bis 2 annehmen kann,
- der Formelindex y Werte von 1 bis 2 annehmen kann,
- der Formelindex z Werte von 0 bis 1 annehmen kann,
- der Formelindex n Werte von 0 bis 4 annehmen kann,
- der Formelindex m Werte von 1 bis 4 annehmen kann,
- der Formelindex p Werte von 0 bis 2 annehmen kann und
- der Formelindex q Werte von 0 bis 4 annehmen kann.

5. Verbindungen gemäß Anspruch 1, **dadurch gekennzeichnet, daß** sie in ihrem chemischen Aufbau mindestens eine Teilstruktur gemäß der allgemeinen Formel V 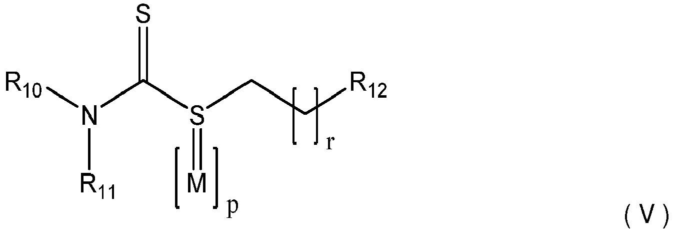 aufweisen, wobei
- R₁₀ und R₁₁ entweder
a) unabhängig voneinander ausgewählt sind aus der Liste umfassend H, Alkyl, Cycloalkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂ , B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, oder
b) es sich bei R₁₀ und R₁₁ um mindestens zweiwertige Reste handelt, die über mindestens eine chemische Bindung miteinander verknüpft sind, so daß die Reste R₁₀ und R₁₁ zusammen mit dem Stickstoffatom einen Heterozyklus bilden,
- R₁₂ ausgewählt ist aus der Liste umfassend Alkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂,
substituiertes Aryl, substituiertes Heteroaryl, Alkylaryl, Alkylheteroaryl, NHR_{f}, NR_{f}R_{g}, COR_{f}, COOR_{f}, CONHR_{f}, CONR_{f}R_{g}, COR_{f}, OCOR_{f}, NHCOR_{f}, NR_{f}COR_{g}, CSR_{f}, CSOR_{f}, CSNHR_{f}, CSNR_{f}R_{g}, CSR_{f}, OCSR_{f}, NR_{f}CSR_{f}, NR_{f}CSR_{g}, COSR_{f}, COR_{f}, CSSR_{f}, CSNR_{f}N(R_{f})₂, CONR_{f}N(R_{f})₂, NR_{f}N(R_{f})₂,
- M ausgewählt ist aus der Liste umfassend O, NH, NR_{f} ,
- R_{f} und R_{g} unabhängig voneinander ausgewählt sind aus der Liste umfassend H, Alkyl, Aryl, Heteroaryl,
substituiertes Aryl, substituiertes Heteroaryl, Alkylaryl, Alkylheteroaryl,
- der Formelindex x Werte von 1 bis 2 annehmen kann,
- der Formelindex y Werte von 1 bis 2 annehmen kann,
- der Formelindex z Werte von 0 bis 1 annehmen kann,
- der Formelindex n Werte von 0 bis 4 annehmen kann,
- der Formelindex m Werte von 1 bis 4 annehmen kann,
- der Formelindex p Werte von 0 bis 2 annehmen kann und
- der Formelindex r Werte von 0 bis 4 annehmen kann.

6. Verbindungen gemäß Anspruch 1, **dadurch gekennzeichnet, daß** sie in ihrem chemischen Aufbau mindestens eine Teilstruktur gemäß der allgemeinen Formel VI 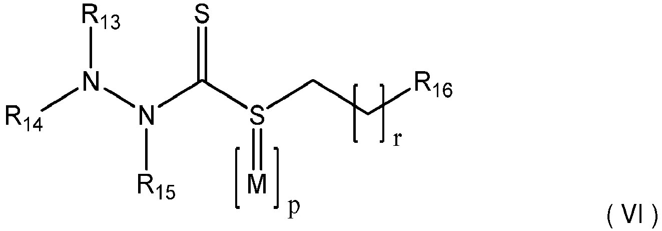 aufweisen, wobei
- R₁₃ und R₁₄ entweder
a) unabhängig voneinander ausgewählt sind aus der Liste umfassend H, Alkyl, Cycloalkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, oder
b) es sich bei R₁₃ und R₁₄ um mindestens zweiwertige Reste handelt, die über mindestens eine chemische Bindung miteinander verknüpft sind, so daß die Reste R₁₃ und R₁₄ zusammen mit dem Stickstoffatom einen Heterozyklus bilden,
- R₁₅ und R₁₆ ausgewählt sind aus der Liste umfassend Alkyl, Aryl, Heteroaryl, Acyl, SOnRf, SOₙH, POₘR_{f}, POₘH, SO_{z}N(R_{f})₂; POm(NRf)x, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, NHR_{f}, NR_{f}R_{g}, COR_{f}, COOR_{f}, CONHR_{f}, CONR_{f}R_{g}, COR_{f}, OCOR_{f}, NHCOR_{f}, NR_{f}COR_{g}, CSR_{f}, CSOR_{f}, CSNHR_{f}, CSNR_{f}R_{g}, CSR_{f}, OCSR_{f}, NR_{f}CSR_{f}, NR_{f}CSR_{g}, COSR_{f}, COR_{f}, CSSR_{f}, CSNR_{f}N(R_{f})₂, CONR_{f}N(R_{f})₂, NR_{f}N(R_{f})₂,
- M ausgewählt ist aus der Liste umfassend O, NH, NR_{f} ,
- R_{f} und R_{g} unabhängig voneinander ausgewählt sind aus der Liste umfassend H, Alkyl, Aryl, Heteroaryl,
substituiertes Aryl, substituiertes Heteroaryl, Alkylaryl, Alkylheteroaryl,
- der Formelindex x Werte von 1 bis 2 annehmen kann,
- der Formelindex y Werte von 1 bis 2 annehmen kann,
- der Formelindex z Werte von 0 bis 1 annehmen kann,
- der Formelindex n Werte von 0 bis 4 annehmen kann,
- der Formelindex m Werte von 1 bis 4 annehmen kann,
- der Formelindex p Werte von 0 bis 2 annehmen kann und
- der Formelindex r Werte von 0 bis 4 annehmen kann.

7. Verbindungen gemäß Anspruch 1, **dadurch gekennzeichnet, daß** sie in ihrem chemischen Aufbau mindestens eine Teilstruktur gemäß der allgemeinen Formel VII 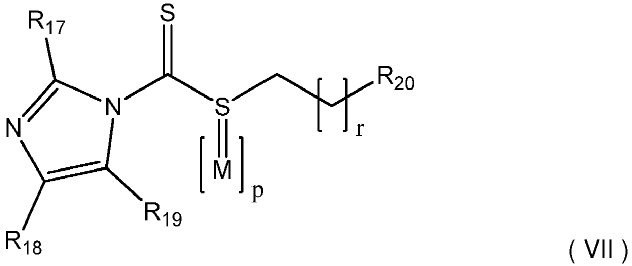 aufweisen, wobei
- R₁₇ und R₁₈ unabhängig voneinander ausgewählt sind aus der Liste umfassend H, Alkyl, Cycloalkyl, Aryl, Heteroaryl, Acyl, SOₙR_{f}, SOₙH, POₘR_{f}, POₘH, SOzN(Rf)2; POm(NRf)x, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl,
substituiertes Heteroaryl, Alkylaryl, Alkylheteroaryl,
- R₁₉ und R₂₀ ausgewählt sind aus der Liste umfassend H, Alkyl, Aryl, Heteroaryl, Acyl, SOₙR_{f}, SOₙH, POₘR_{f}, POₘH, SO_{z}N(R_{f})₂; POₘ(NR_{f})ₓ, SO_{z}(NR_{f})_{y}, B(OR_{f})₂, OB(OR_{f})₂, B(OR_{f})(NR_{f}), B(NR_{f})₂, OB(OR_{f})(NR_{f}), OB(NR_{f})₂, substituiertes Aryl, substituiertes Heteroaryl, Alkylaryl, Alkylheteroaryl, NHR_{f}, NR_{f}R_{g}, COR_{f}, COOR_{f}, CONHR_{f}, CONR_{f}R_{g}, COR_{f}, OCOR_{f}, NHCOR_{f}, NR_{f}COR_{g}, CSR_{f}, CSOR_{f}, CSNHR_{f}, CSNR_{f}R_{g}, CSR_{f}, OCSR_{f}, NR_{f}CSR_{f}, NR_{f}CSR_{g}, COSR_{f}, COR_{f}, CSSR_{f}, CSNR_{f}N(R_{f})₂, CONR_{f}N(R_{f})₂, NR_{f}N(R_{f})₂,
- M ausgewählt ist aus der Liste umfassend O, NH, NR_{f} ,
- R_{f} und R_{g} unabhängig voneinander ausgewählt sind aus der Liste umfassend H, Alkyl, Aryl, Heteroaryl,
substituiertes Aryl, substituiertes Heteroaryl, Alkylaryl, Alkylheteroaryl,
- der Formelindex x Werte von 1 bis 2 annehmen kann,
- der Formelindex y Werte von 1 bis 2 annehmen kann,
- der Formelindex z Werte von 0 bis 1 annehmen kann,
- der Formelindex n Werte von 0 bis 4 annehmen kann,
- der Formelindex m Werte von 1 bis 4 annehmen kann,
- der Formelindex p Werte von 0 bis 2 annehmen kann und
- der Formelindex r Werte von 0 bis 4 annehmen kann.

8. Verbindungen gemäß Anspruch 1, **dadurch gekennzeichnet, daß** sie in ihrem chemischen Aufbau mindestens eine Teilstruktur gemäß der allgemeinen Formel VIII 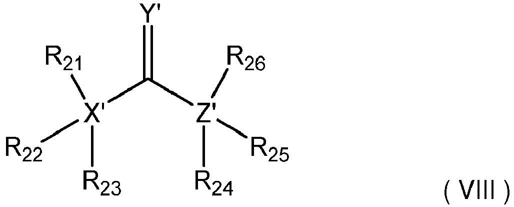 aufweisen, wobei
- es sich bei Y' entweder um ein Sauerstoffatom oder ein Schwefelatom oder um ein Selen-Atom handelt und
- es sich bei X' und Z' unabhängig voneinander jeweils um ein Atom der chemischen Elemente Stickstoff, Sauerstoff, Kohlenstoff, Schwefel handelt und entweder
a) R₂₁, R₂₂, R₂₃, R₂₄, R₂₅, R₂₆ unabhängig voneinander ausgewählt sind aus der Liste umfassend "kein Rest", H, OH, Alkyl, Aryl, Heteroaryl, substituiertes Alkyl, substituiertes Aryl, substituiertes Heteroaryl, Alkylaryl, Alkylheteroaryl, wobei die Substituenten der substituierten Alkyl-, Aryl- und Heteroaryl-Reste ausgewählt sind aus der Liste umfassend F, Cl, Br, OH, Phenyl, NH₂, NO₂, OR_{f}, O-COR_{f}, COR_{f}, N(R_{f})₂, NR_{f}COR_{f}, O-CSR_{f}, CSR_{f}, NR_{f}CSR_{f}, O-CSeR_{f}, CSeR_{f}, NR_{f}CSeR_{f}, O-CN(R_{f})R_{f}, CN(R_{f})R_{f}, NR_{f}CN(R_{f})R_{f}, N(R_{f})SO₂R_{f}, N(R_{f})SO₂N(R_{f})₂, SSR_{f}, SR_{f}, SOₓR_{f}, SOₙN(R_{f})₂, Nitril, Amidin, Guanidin, COOR_{f}, CON(R_{f})₂, CON(R_{f})N(R_{f})₂, COSR_{f}, CSOR_{f}, CSSR_{f}, NR_{f}COR_{f}, N(R_{f})N(R_{f})₂ , oder
b) mindestens zwei der an X' und/oder Z' hängenden Reste R₂₁ bis R₂₆ zusammen mit X' und/oder Z' eine gesättigte oder ungesättigte cyclische oder heterocyclische Ringstruktur bilden, und die nicht in diesen mindestens einen Cyclus eingebundenen verbliebenen Reste R₂₁ oder R₂₂ oder R₂₃ oder R₂₄ oder R₂₅ oder R₂₆ unabhängig voneinander ausgewählt sind aus der Liste umfassend "kein Rest", H, OH, Alkyl, Aryl, Heteroaryl, substituiertes Alkyl, substituiertes Aryl, substituiertes Heteroaryl, Alkylaryl, Alkylheteroaryl, wobei die Substituenten der substituierten Alkyl-, Aryl- und Heteroaryl-Reste ausgewählt sind aus der Liste umfassend F, Cl, Br, OH, Phenyl, NH₂, NO₂, OR_{f}, O-COR_{f}, COR_{f}, N(R_{f})₂, NR_{f}COR_{f}, O-CSR_{f}, CSR_{f}, NR_{f}CSR_{f}, O-CSeR_{f}, CSeR_{f}, NR_{f}CSeR_{f}, O-CN(R_{f})R_{f}, CN(R_{f})R_{f}, NR_{f}CN(R_{f})R_{f}, N(R_{f})SO₂R_{f}, N(R_{f})SO₂N(R_{f})₂, SSR_{f}, SR_{f}, SOₓR_{f}, SOₙN(R_{f})₂, Nitril, Amidin, Guanidin, COOR_{f}, CON(R_{f})₂, CON(R_{f})N(R_{f})₂, COSR_{f}, CSOR_{f}, CSSR_{f}, NR_{f}COR_{f}, N(R_{f})N(R_{f})₂.

9. Verbindungen gemäß einem der vorangegangenen Ansprüche 1 bis 8, **dadurch gekennzeichnet, daß** sie als Ion in mindestens einfach protonierter Form oder in mindestens einfach deprotonierter Form, jeweils in Kombination mit einem beliebigen Gegenion, also in Form eines Salzes, vorliegen.

10. Verbindungen gemäß einem der vorangegangenen Ansprüche 1 bis 9, **dadurch gekennzeichnet, daß** sie in dimerer Form vorliegen.

11. Medizinisches Mittel enthaltend mindestens eine der Verbindungen gemäß einem der vorangegangenen Ansprüche 1 bis 10 zur Behandlung von Menschen und/oder Tieren mit Erkrankungen, die durch parasitäre Helminthen verursacht wurden.

12. Medizinisches Mittel gemäß Anspruch 11 enthaltend mindestens eine der Verbindungen gemäß einem der vorangegangenen Ansprüche 1 bis 10 **dadurch gekennzeichnet, dass** es zusätzlich mindestens einen pharmakologisch verträglichen Träger, Hilfsstoff und/oder Lösungsmittel umfasst.

13. Medizinisches Mittel gemäß Anspruch 11 oder 12 enthaltend mindestens eine der Verbindungen gemäß einem der vorangegangenen Ansprüche 1 bis 10 **dadurch gekennzeichnet, dass** es als Pour-on Formulierung vorliegt.

14. Verwendung einer oder mehrerer Verbindungen gemäß einem der Ansprüche 1 bis 10 zur Behandlung von Menschen und/oder Tieren mit Erkrankungen, die durch parasitäre Helminthen verursacht wurden.

15. Verfahren zur Herstellung von Carbamodithioat-Derivaten gemäß einem der Ansprüche 1 bis 10, umfassend die Umsetzung von Aminen mit Kohlenstoffdisulfid und Alkyl(aryl)halogeniden in einem Lösungsmittel enthaltend mindestens eines der Lösungsmittel aus der Liste umfassend Aceton, Acetonitril, Ethanol, Tetrahydrofuran, **dadurch gekennzeichnet, dass**
a) entweder auf die Temperierung der Reaktionsmischung verzichtet wird, oder
b) auf den Zusatz einer weiteren Base zusätzlich zu dem umzusetzenden Amin verzichtet wird.
